# EUROPEAN PATENT APPLICATION

(11) **EP 4 582 422 A1**
(43) Date of publication of application: **09.07.2025**
(21) Application number: 23864827.3
(22) Date of filing: 18.09.2023
(51) Int. Cl.: C07D 471/04, C07D 519/00, A61P 35/00, A61P 35/02, A61P 25/00, A61P 29/00, A61P 31/00, A61K 31/437, A61K 31/4375

(54) **METTL3 INHIBITOR COMPOUND, AND PHARMACEUTICAL COMPOSITION AND USE THEREOF**

(30) Priority: 16.09.2022 CN 202211132234; 20.02.2023 CN 202310138424
(71) Applicant: Global Health Drug Discovery Institute, Beijing 100192 (CN)
(72) Inventor: LI, Zizhong, Beijing 100192 (CN); GAO, Hui, Beijing 100192 (CN); WANG, Ting, Beijing 100192 (CN); CHANG, Lei, Beijing 100192 (CN); DING, Sheng, Beijing 100192 (CN)
(74) Representative: Ipside
(86) International application number: PCT/CN2023/119505
(87) International publication number: WO 2024/056099

(57) **Abstract**

Provided is use of a compound shown in formula I, formula I' or formula I", and tautomers, stereoisomers, or pharmaceutically acceptable salts of the three compounds as METTL3 inhibitors. The compounds have good regulatory and inhibitory effects on METTL3/METTL14 and AML related cell lines. In terms of enzyme and cell activity, they have obvious advantages compared with compounds disclosed in the prior art (under the same Assay, the enzyme and cell activity of the compounds provided by the present invention are significantly improved compared with similar compounds in the prior art). Therefore, the compounds provided by the present invention can be used for treating symptoms and diseases associated with the methyltransferase activity of METTL3/METTL14. In addition, preparation methods of the compounds provided by the present invention are simple and have good application prospects.

## Description

**METTL3 Inhibitor Compound, and Pharmaceutical Composition and Use Thereof [0001]** The present application claims the priority to the earlier application with Patent Application No. 202211132234.2, entitled "METTL3 Inhibitor Compound, and Pharmaceutical Composition and Use Thereof" and filed in the China National Intellectual Property Administration on September 16, 2022, and to the earlier application with Patent Application No. 202310138424.3, entitled "METTL3 Inhibitor Compounds and Preparation Methods Therefor and Applications Thereof" and filed in the China National Intellectual Property Administration on February 20, 2023. The full texts of these two applications are incorporated into the present application by reference.

### Technical Field

The present invention belongs to the technical field of medicine, and specifically relates to an METTL3 inhibitor compound, and a pharmaceutical composition and use thereof.

### Background

N6 methyladenosine (m6A) is the most common and abundant covalent modification of messenger RNA (mRNA). Methyltransferase can modify specific sites on mRNA to synthesize m6A. Approximately 0.1% to 0.5% of all mRNAs are m6A modified. Data from cell experiments indicate that m6A modification will affects various aspects of mRNA biology, mainly mRNA expression, splicing, stability, localization, and translation. M6A modifications are tissue-specific, with significant differences in their occurrence in non-pathological tissues such as the brain, heart and kidney, pathological tissues and cells. M6A-related proteins, such as FTO ALKBH5, methyltransferase 3 (METTL3) and methyltransferase 14 (METTL14) are associated with major diseases such as solid organ cancers, leukemia, type 2 diabetes, neuropsychiatric behavior and depression.

METTL3, the main enzyme catalyzing the modification of RNA m6A, exists as a heterotrimer complex with METTL 14 and Wilm's tumor-associated protein (WTAP). METTL3 has catalytic activity. It can transfer a methyl group of the cofactor adenosylmethionine to a substrate RNA, METTL14 promotes the binding of the substrate RNA, and WTAP localizes the complex to a specific RNA site. Currently, METTL3 has been reported to play an important role in many aspects of cancer development. Knockdown of METTL3 expression in lung cancer cell lines (A549, H1299 and H1792) and HeLa cells inhibits the growth, survival and invasion of human lung cancer cells. In human bladder cancer, researchers observed that expression level of METTL3 was significantly up-regulated. Knocking down the expression of an METTL3 gene can significantly reduce the proliferation, invasion, survival in vitro and tumorigenicity in vivo of bladder cancer cells.

In addition, METTL3 plays an important role in controlling the myeloid differentiation of normal hematopoietic and leukemic cells in mammals. High expression of METTL3 can promote the proliferation of CD34+ hematopoietic stem cells from human umbilical cord blood and inhibit cell differentiation. Knockdown of METTL3 will promote the differentiation of such cells and inhibit cell proliferation. When METTL3 is deleted, down-regulation of m6A levels on interferon-stimulated gene (ISG) mRNA leads to up-regulation of the protein level of this gene, which inhibits viral reproduction. Therefore, METTL3 inhibitors may provide a new therapeutic approach for a range of infectious and inflammatory diseases. The m6A modification affects the division and differentiation of embryonic stem cells and different tissue progenitors/stem cells through NANOG, SOX2, NR5A2 and MYC.

METTL3 inhibitors have been reported in relevant literature, but the activity of these inhibitors needs to be further improved.

### Summary

In order to solve the above technical problems, the present invention provides a compound shown in formula I, formula I' or formula I", and tautomers, stereoisomers, or pharmaceutically acceptable salts of the three compounds: 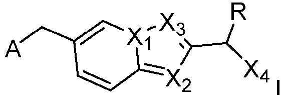
in formula I, A is selected from the following groups unsubstituted or optionally substituted by one, two or more Ra: -NH-(C₁₋₁₂alkyl)-C₃₋₂₀cycloalkyl, 3-20 membered heterocyclic groups, -3-20 membered heterocyclic groups-3-20 membered heterocyclic groups, -5-20 membered heteroaryl-5-20 membered heteroaryl, -NH-(C₁₋₁₂alkyl)-3-20 membered heterocyclic groups, C₃₋₂₀cycloalkyl or -N(Z)CO-5-20 membered heteroaryl;
X₁, X₂, and X₃ are the same or different, independently selected from C, O, or N, provided that X₁, X₂, and X₃ are not simultaneously heteroatoms (i.e., not simultaneously N and/or O);
X₄ is selected from the following groups unsubstituted or optionally substituted by one, two or more Rb: -5-20 membered heteroaryl-5-20 membered heteroaryl, -5-20 membered heteroaryl-C₆₋₂₀aryl, -N(Z)CO-5-20 membered heteroaryl, -CON(Z)-5-20 membered heteroaryl, - 5-20 membered heteroaryl-CO-C₁₋₁₂alkyl, -NH-(C₁₋₁₂alkyl)-C₃₋₂₀cycloalkyl, 3-20 membered heterocyclic groups or -COO-5-20 membered heteroaryl;
R is selected from H or C₁₋₁₂alkyl;
Ra is selected from nitro, amino, halogen, -CH₂N(C₁₋₁₂alkyl)₂, -N(C₁₋₁₂alkyl)₂, -O-(C₁₋₁₂alkyl-O)₂-C₁₋₁₂alkyl, 3-20 membered heterocyclic groups, C₁₋₁₂alkyl substituted 3-20 membered heterocyclic groups, -Se-C₁₋₁₂alkyl, -S-C₁₋₁₂alkyl, 5-20 membered heteroaryl, C₁₋₁₂alkoxy or C₁₋₁₂alkyl;
Rb is selected from H, halogen, NO₂, -CHO, OH, NH₂, =O, and the following groups unsubstituted or optionally substituted by one, two or more Rd: -S-C₁₋₁₂alkyl, -NH-C₁₋₁₂alkyl, - N(C₁₋₁₂alkyl)₂, 3-20 membered heterocyclic groups, C₁₋₁₂alkoxy, -O-(C₁₋₁₂alkyl-O)₂-C₁₋₁₂alkyl, - S(O)2-C₁₋₁₂alkyl, -Se-C₁₋₁₂alkyl, -N(C₁₋₁₂alkyl-OH)₂, -N(C₁₋₁₂alkyl-Cl)₂, C₁₋₁₂alkyl, -NHC(O)C₁₋₁₂alkyl, -N(Z)CO-5-20 membered heteroaryl, -OC(O)C₁₋₁₂alkyl, -N(CD₃)₂, and 5-20 membered heteroaryl;
Z is selected from H or C₁₋₁₂alkyl;
Rd is selected from halogen, cyano, C₁₋₁₂alkyl, -S-C₁₋₁₂alkyl, -NH-C₁₋₁₂alkyl, -N(C₁₋₁₂alkyl)₂; 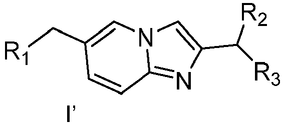
in formula I', R₁ is selected from the following groups: -NH-(C₁₋₁₂alkyl)-bicyclo[1.1.1]pentane-(Ra)n, -NH-(C₁₋₁₂alkyl)-C₆₋₂₀aryl-(Ra)n, -NH-(C₁₋₁₂alkyl)-(Ra)n, -NH-(C₁₋₁₂alkyl)-C₃₋₂₀cycloalkyl-(Ra)n, -NH-(C₁₋₁₂alkyl)-3-20 membered heterocyclic groups-(Ra)n, -3-20 membered heterocyclic groups-(Ra)n, -NH-(C₁₋₁₂alkyl)-5-20 membered heteroaryl-(Ra)n, -NH-(C₁₋₁₂alkyl)-bicyclo[2.2.2]octane-(Ra)n, and -NH-(C₁₋₁₂alkyl)-cubane-(Ra)n;
Ra is selected from H, OH, halogen, halogenated C₁₋₁₂alkyl, C₁₋₁₂alkyl, CN or C₂₋₁₂alkynyl;
n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₁₂alkyl, or CN;
R₃ is selected from -5-20 membered heteroaryl-Rb, -OC(O)-5-20 membered heteroaryl-Rb or -NC(O)-5-20 membered heteroaryl-Rb;
Rb is selected from 5-20 membered heteroaryl-Rc, 3-20 membered heterocyclic groups or halogen;
m is 1, 2 or 3;
Rc is selected from -3-20 membered heterocyclic groups-Rd, -5-20 membered heteroaryl-Rd, -O(C₁₋₁₂alkyl-O)m-C₁₋₁₂alkyl;
Rd is selected from H, deuterium, halogen, OH, CN, halogenated C₁₋₁₂alkyl, C₁₋₁₂alkyl or =O; 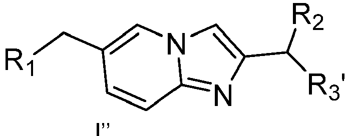
in formula I", R₁ is selected from the following groups: -NH-(C₁₋₁₂alkyl)-bicyclo[1.1.1]pentane-(Ra)n, -NH-(C₁₋₁₂alkyl)-C₆₋₂₀aryl-(Ra)n, -NH-(C₁₋₁₂alkyl)-(Ra)n, -NH-(C₁₋₁₂alkyl)-C₃₋₂₀cycloalkyl-(Ra)n, -NH-(C₁₋₁₂alkyl)-3-20 membered heterocyclic groups-(Ra)n, -3-20 membered heterocyclic groups-(Ra)n, -NH-(C₁₋₁₂alkyl)-5-20 membered heteroaryl-(Ra)n, -NH-(C₁₋₁₂alkyl)-bicyclo[2.2.2]octane-(Ra)n, and -NH-(C₁₋₁₂alkyl)-cubane-(Ra)n;
Ra is selected from H, OH, halogen, halogenated C₁₋₁₂alkyl, C₁₋₁₂alkyl, CN or C₂₋₁₂alkynyl;
n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₁₂alkyl, or CN; and
R₃ is selected from -OC(O)-5-12 membered heteroaryl-Rm or -NC(O)-5-12 membered heteroaryl-Rm, and Rm is selected from -NH-(C₁₋₁₂alkyl), -N(C₁₋₁₂alkyl)₂ or 3-12 membered heterocyclic groups.

According to the embodiments of the present invention, the compound shown in formula I does not include the following specific compounds:

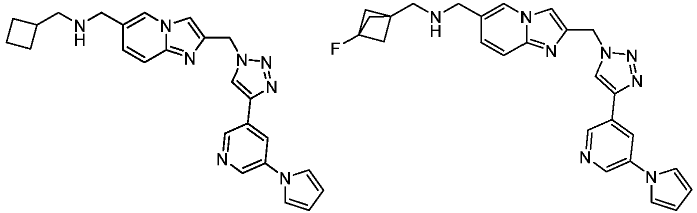

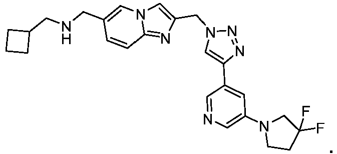

According to the embodiments of the present invention, the compound shown in formula I' is not a compound as follows:

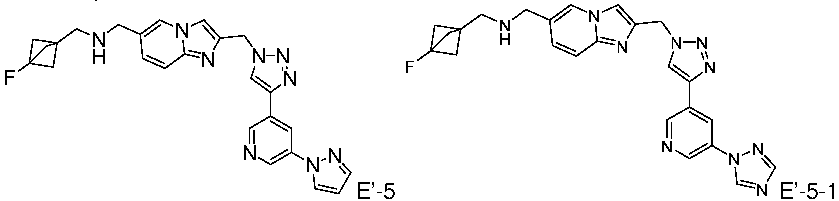

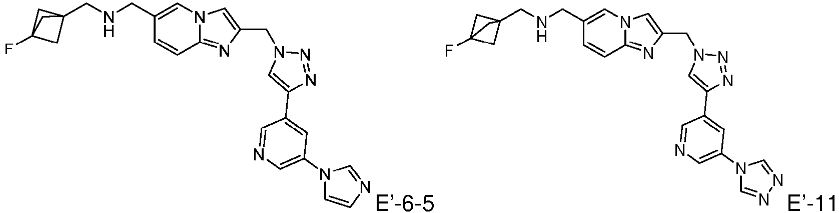

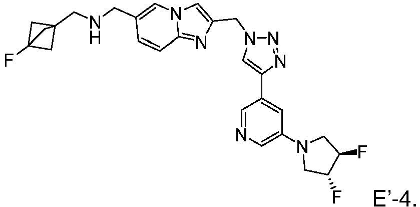

According to the embodiments of the present invention, in the compound shown in formula I, A is selected from the following groups unsubstituted or optionally substituted by one, two or more Ra: -NH-(C₁₋₁₂alkyl)-C₃₋₂₀cycloalkyl, 3-20 membered heterocyclic groups, -3-20 membered heterocyclic groups-3-20 membered heterocyclic groups, -5-20 membered heteroaryl-5-20 membered heteroaryl, -NH-(C₁₋₁₂alkyl)-3-20 membered heterocyclic groups, C₃₋₂₀cycloalkyl or -N(Z)CO-5-20 membered heteroaryl;
X₁, X₂, and X₃ are the same or different, independently selected from C, O, or N, provided that X₁, X₂, and X₃ are not simultaneously heteroatoms (i.e., not simultaneously N and/or O);
X₄ is selected from the following groups unsubstituted or optionally substituted by one, two or more Rb: -5-20 membered heteroaryl-5-20 membered heteroaryl, -5-20 membered heteroaryl-C₆₋₂₀aryl, -N(Z)CO-5-20 membered heteroaryl, -CON(Z)-5-20 membered heteroaryl, - 5-20 membered heteroaryl-CO-C₁₋₁₂alkyl, -NH-(C₁₋₁₂alkyl)-C₃₋₂₀cycloalkyl, 3-20 membered heterocyclic groups or -COO-5-20 membered heteroaryl;
R is selected from H or C₁₋₁₂alkyl;
Ra is selected from nitro, amino, halogen, -CH₂N(C₁₋₁₂alkyl)₂, -N(C₁₋₁₂alkyl)₂, -O-(C₁₋₁₂alkyl-O)₂-C₁₋₁₂alkyl, 3-20 membered heterocyclic groups, C₁₋₁₂alkyl substituted 3-20 membered heterocyclic groups, -Se-C₁₋₁₂alkyl, -S-C₁₋₁₂alkyl, 5-20 membered heteroaryl, C₁₋₁₂alkoxy or C₁₋₁₂alkyl;
Rb is selected from H, halogen, NO₂, -CHO, OH, NH₂, =O, and the following groups unsubstituted or optionally substituted by one, two or more Rd: -S-C₁₋₁₂alkyl, -NH-C₁₋₁₂alkyl, - N(C₁₋₁₂alkyl)₂, 3-20 membered heterocyclic groups, C₁₋₁₂alkoxy, -O-(C₁₋₁₂alkyl-O)₂-C₁₋₁₂alkyl, - S(O)2-C₁₋₁₂alkyl, -Se-C₁₋₁₂alkyl, -N(C₁₋₁₂alkyl-OH)₂, -N(C₁₋₁₂alkyl-Cl)₂, C₁₋₁₂alkyl, -NHC(O)C₁₋₁₂alkyl, -N(Z)CO-5-20 membered heteroaryl, -OC(O)C₁₋₁₂alkyl, -N(CD₃)₂, and 5-20 membered heteroaryl;
Z is selected from H or C₁₋₁₂alkyl;
Rd is selected from halogen, cyano, C₁₋₁₂alkyl, -S-C₁₋₁₂alkyl, -NH-C₁₋₁₂alkyl, -N(C₁₋₁₂alkyl)₂;
moreover, when A is selected from -NH-(C₁₋₁₂alkyl)-C₃₋₂₀cycloalkyl, X₁ and X₂ are N, and X₃ is C; when R is selected from H, X₄ is not 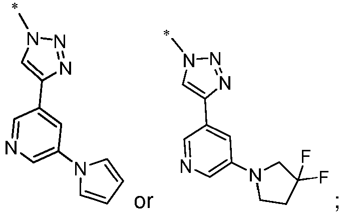
alternatively, when Ra is selected from halogen and A is selected from -NH-(C₁₋₁₂alkyl)-C₃₋₂₀cycloalkyl, X₁ and X₂ are N, and X₃ is C; and when R is selected from H, X₄ is not

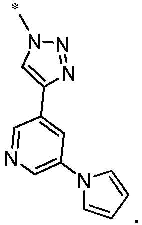

According to the preferred embodiment of the present invention, in formula I, A is selected from the following groups unsubstituted or optionally substituted by one, two or more Ra: -NH-(C₁₋₆alkyl)-C₃₋₁₂cycloalkyl, 3-12 membered heterocyclic groups, -3-12 membered heterocyclic groups-3-12 membered heterocyclic groups, -5-12 membered heteroaryl-5-12 membered heteroaryl, -NH-(C₁₋₆alkyl)-3-12 membered heterocyclic groups, C₃₋₁₂cycloalkyl or - N(Z)CO-5-12 membered heteroaryl;
X₁, X₂, and X₃ are the same or different, independently selected from C, O, or N, provided that X₁, X₂, and X₃ are not simultaneously heteroatoms;
X₄ is selected from the following groups unsubstituted or optionally substituted by one, two or more Rb: -5-12 membered heteroaryl-5-12 membered heteroaryl, -5-12 membered heteroaryl-C₆₋₁₂ aryl, -N(Z)CO-5-12 membered heteroaryl, -CON(Z)-5-12 membered heteroaryl, -5-12 membered heteroaryl-CO-C₁₋₆alkyl, -NH-(C₁₋₃alkyl)-C₃₋₁₂cycloalkyl, 3-12 membered heterocyclic groups or -COO-5-12 membered heteroaryl;
R is selected from H or C₁₋₆alkyl;
Ra is selected from NO₂, NH₂, halogen, -CH₂N(C₁₋₃alkyl)₂, -N(C₁₋₆alkyl)₂, -O-(C₁₋₆alkyl-O)₂-C₁₋₆alkyl, 3-12 membered heterocyclic groups, C₁₋₆alkyl substituted 3-12 membered heterocyclic groups, -Se-C₁₋₆alkyl, -S-C₁₋₆alkyl, 5-12 membered heteroaryl, C₁₋₆alkoxy or C₁₋₆alkyl;
Rb is selected from H, halogen, NO₂, -CHO, OH, NH₂, =O, and the following groups unsubstituted or optionally substituted by one, two or more Rd: -S-C₁₋₆alkyl, -NH-C₁₋₆alkyl, -N(C₁₋₆alkyl)₂, 3-12 membered heterocyclic groups, C₁₋₆alkoxy, -O-(C₁₋₆alkyl-O)₂-C₁₋₆alkyl, -S(O)₂-C₁₋₆alkyl, -Se-C₁₋₆alkyl, -N(C₁₋₆alkyl-OH)₂, -N(C₁₋₆alkyl-Cl)₂, C₁₋₆alkyl, -NHC(O)C₁₋₆alkyl, -N(Z)CO-5-12 membered heteroaryl, -OC(O)C₁₋₆alkyl, -N(CD₃)₂, and 5-12 membered heteroaryl;
Z is selected from H or C₁₋₆alkyl; and
Rd is selected from halogen, CN, C₁₋₆alkyl, -S-C₁₋₆alkyl, -NH-C₁₋₆alkyl, -N(C₁₋₆alkyl)₂.

According to the preferred embodiment of the present invention, in formula I, A is selected from 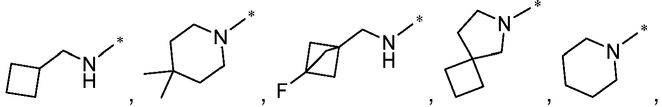 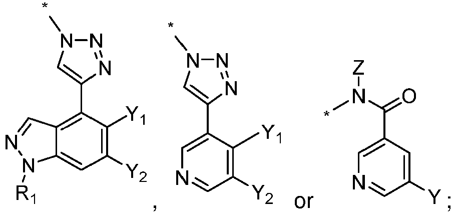
X₄ is selected from 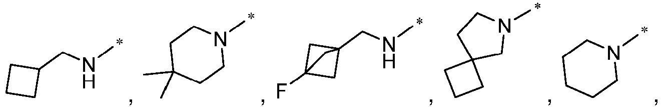 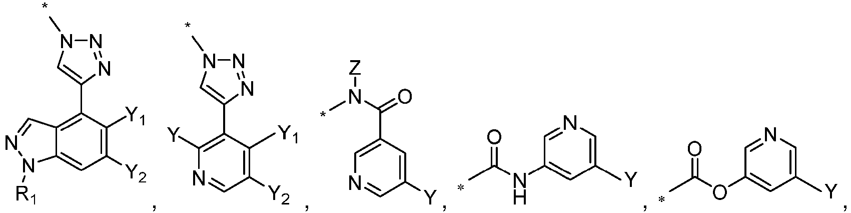 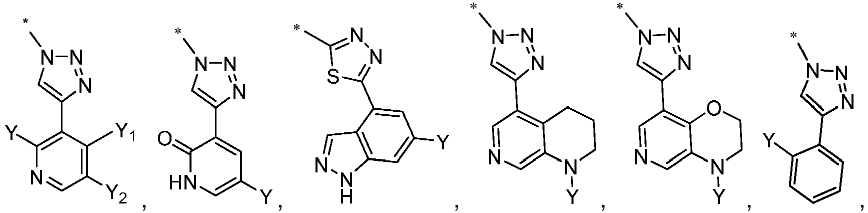 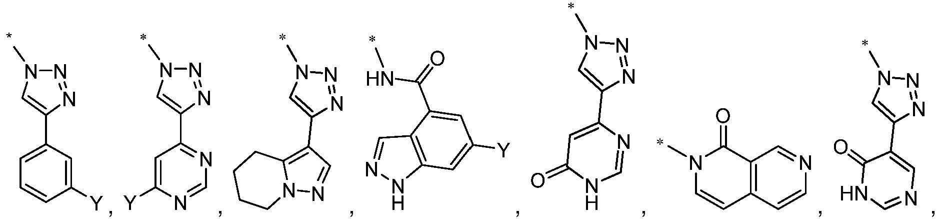 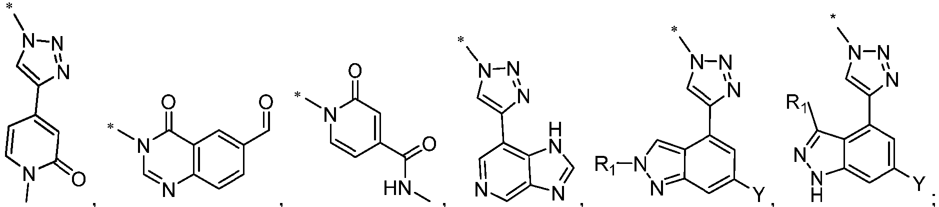
Y, Y₁ and Y₂ are the same or different, independently selected from H, NH₂, CN, CHO, OH, =O, -S-C₁₋₆alkyl, I, NH₂, -NH-C₁₋₆alkyl, -N(C₁₋₆alkyl)₂, 3-6 membered heterocyclic groups, Cl, Br, C₁₋₆alkoxy, -O-(C₁₋₆alkyl-O)₂-C₁₋₆alkyl, -S(O)₂-C₁₋₆alkyl, -Se-C₁₋₆alkyl, -N(C₁₋₆alkyl-OH)₂, -N(C₁₋₆alkyl-Cl)₂, C₁₋₆alkyl, -NHC(O)C₁₋₆alkyl, -OC(O)C₁₋₆alkyl, -CHO, OH, -N(CD₃)₂, 5-6 membered heteroaryl, NO₂, F; the 3-6 membered heterocyclic groups or 5-6 membered heteroaryl is optionally substituted with one, two, or three groups selected from F, Cl, Br, and I;
R₁ and R₂ are the same or different, independently selected from H, F or C₁₋₆alkyl; and
Z is the same or different, independently selected from H or C₁₋₆alkyl.

According to the preferred embodiment of the present invention, in formula I, when A is selected from 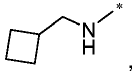 X₁, X₂, and X₃ are the same or different, independently selected from C or N, provided that X₁, X₂, and X₃ are not simultaneously heteroatoms; X₄ is selected from 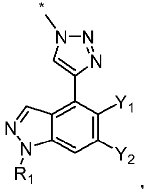 where Y₁ and Y₂ are the same or different, independently selected from H, - S-C₁₋₆alkyl, I, Cl, Br, NH₂, -NH-C₁₋₆alkyl, -N(C₁₋₆alkyl)₂, 3-6 membered heterocyclic groups, -Se-C₁₋₆alkyl, C₁₋₆alkyl, 3-6 membered heterocyclic groups; and R₁ is selected from H, F, or C₁₋₆alkyl.

According to the preferred embodiment of the present invention, in formula I, when A is selected from 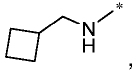 X₁, X₂, and X₃ are the same or different, independently selected from C or N, provided that X₁, X₂, and X₃ are not simultaneously heteroatoms; and X₄ is selected from 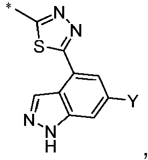 where Y is selected from Cl, Br, C₁₋₆alkyl or C₁₋₆alkoxy.

According to the preferred embodiment of the present invention, in formula I, when X₄ is selected from 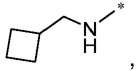 X₁, X₂, and X₃ are the same or different, independently selected from C or N, provided that X₁, X₂, and X₃ are not simultaneously heteroatoms; A is selected from 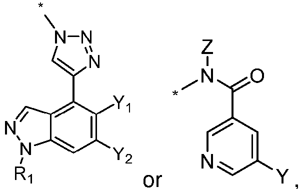 where Y₁ and Y₂ are the same or different, independently selected from H, NH₂, -NH-C₁₋₆alkyl, -N(C₁₋₆alkyl)₂, C₁₋₆alkyl; R₁ is selected from H, F, or C₁₋₆alkyl, where Y is selected from H or 3-6 membered heterocyclic groups, and Z is selected from H or C₁₋₆alkyl; and

Y is selected from 3-6 membered heterocyclic groups, and Z is selected from H or C₁₋₆alkyl.

According to the preferred embodiment of the present invention, in formula I, when A is selected from 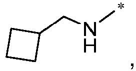 X₁, X₂, and X₃ are the same or different, independently selected from C or N, provided that X₁, X₂, and X₃ are not simultaneously heteroatoms; and X₄ is selected from 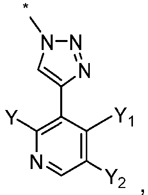 and Y, Y₁ and Y₂ are the same or different, independently selected from H, 3-6 membered heterocyclic groups, C₁₋₆alkyl substituted 3-6 membered heterocyclic groups.

According to the preferred embodiment of the present invention, in formula I, when A is selected from 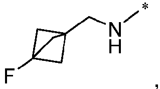 X₁, X₂, and X₃ are the same or different, independently selected from C or N, provided that X₁, X₂, and X₃ are not simultaneously heteroatoms; X₄ is selected from 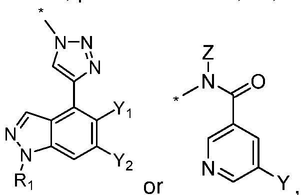 where Y₁ and Y₂ are the same or different, independently selected from H, I, -S- C₁₋₆alkyl, -NH-C₁₋₆alkyl, -N(C₁₋₆alkyl)₂, -Se-C₁₋₆alkyl; R₁ is selected from H, F, or C₁₋₆alkyl; Y is selected from 3-6 membered heterocyclic groups; and Z is selected from H or C₁₋₆alkyl.

According to the preferred embodiment of the present invention, in formula I, when X₄ is selected from 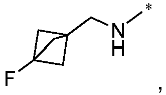 X₁, X₂, and X₃ are the same or different, independently selected from C or N, provided that X₁, X₂, and X₃ are not simultaneously heteroatoms; A is selected from 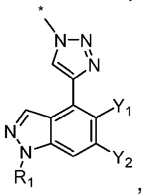 where Y₁ and Y₂ are the same or different, independently selected from H, 3-6 membered heterocyclic groups, -NH-C₁₋₆alkyl, -N(C₁₋₆alkyl)₂, -Se-C₁₋₆alkyl; and R₁ is selected from H, F, or C₁₋₆alkyl.

According to the preferred embodiment of the present invention, in formula I, when A is selected from 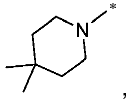 X₁, X₂, and X₃ are the same or different, independently selected from C or N, provided that X₁, X₂, and X₃ are not simultaneously heteroatoms; and X₄ is selected from 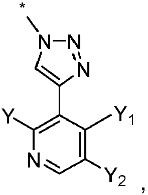 and Y, Y₁ and Y₂ are the same or different, independently selected from H, 3-6 membered heterocyclic groups, C₁₋₆alkyl substituted 3-6 membered heterocyclic groups.

According to the preferred embodiment of the present invention, in formula I, when X₄ is selected from 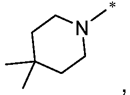 X₁, X₂, and X₃ are the same or different, independently selected from C or N, provided that X₁, X₂, and X₃ are not simultaneously heteroatoms; A is selected from 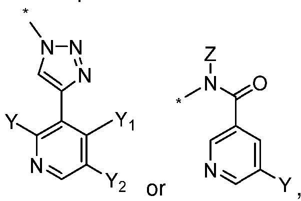 where Y, Y₁ and Y₂ are the same or different, independently selected from H, 3-6 membered heterocyclic groups, C₁₋₆alkyl substituted 3-6 membered heterocyclic groups, -NH-C₁₋₆alkyl, -N(C₁₋₆alkyl)₂; and Z is selected from H or C₁₋₆alkyl.

According to the preferred embodiment of the present invention, in formula I, when A is selected from 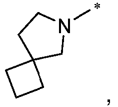 X₁, X₂, and X₃ are the same or different, independently selected from C or N provided that X₁, X₂, and X₃ are not simultaneously heteroatoms; X₄ is selected from 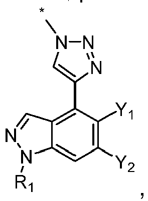 where Y₁ and Y₂ are the same or different, independently selected from H, I, NH₂, -S-C₁₋₆alkyl, -NH-C₁₋₆alkyl, -N(C₁₋₆alkyl)₂, -Se-C₁₋₆alkyl, 3-6 membered heterocyclic groups; and R₁ is selected from H, F, or C₁₋₆alkyl.

According to the preferred embodiment of the present invention, in formula I, when X₄ is selected from 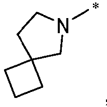 X₁, X₂, and X₃ are the same or different, independently selected from C or N, provided that X₁, X₂, and X₃ are not simultaneously heteroatoms; A is selected from 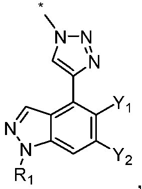 where Y₁ and Y₂ are the same or different, independently selected from H, I, NH₂, -S-C₁₋₆alkyl, -NH-C₁₋₆alkyl, -N(C₁₋₆alkyl)₂, -Se-C₁₋₆alkyl, 3-6 membered heterocyclic groups; and R₁ is selected from H, F, or C₁₋₆alkyl.

According to the preferred embodiment of the present invention, in formula I, when X₄ is selected from 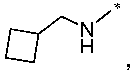 X₁, X₂, and X₃ are the same or different, independently selected from C or N, provided that X₁, X₂, and X₃ are not simultaneously heteroatoms; A is selected from 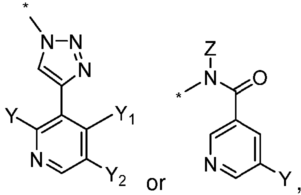 where Y, Y₁ and Y₂ are the same or different, independently selected from H, 3-6 membered heterocyclic groups; and Z is selected from H or C₁₋₆alkyl.

According to the preferred embodiment of the present invention, in formula I, when A is selected from 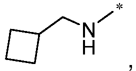 X₁, X₂, and X₃ are the same or different, independently selected from C or N, provided that X₁, X₂, and X₃ are not simultaneously heteroatoms; and X₄ is selected from 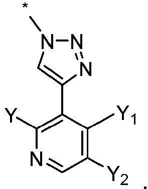 where Y, Y₁ and Y₂ are the same or different, independently selected from H, 3-6 membered heterocyclic groups.

According to the preferred embodiment of the present invention, in formula I, when A is selected from 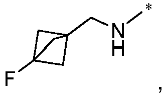 X₁, X₂, and X₃ are the same or different, independently selected from C or N, provided that X₁, X₂, and X₃ are not simultaneously heteroatoms; X₄ is selected from 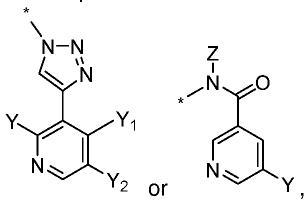 where Y, Y₁ and Y₂ are the same or different, independently selected from H, 3-6 membered heterocyclic groups, -NH-C₁₋₆alkyl, -N(C₁₋₆alkyl)₂; the 3-6 membered heterocyclic groups is optionally substituted with one, two, or three groups selected from F, Cl, Br, and I; and Z is selected from H or C₁₋₆alkyl.

According to the preferred embodiment of the present invention, in formula I, when X₄ is selected from 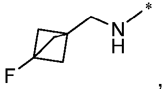 X₁, X₂, and X₃ are the same or different, independently selected from C or N, provided that X₁, X₂, and X₃ are not simultaneously heteroatoms; A is selected from 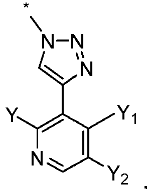 where Y, Y₁ and Y₂ are the same or different, independently selected from H, 3-6 membered heterocyclic groups, -NH-C₁₋₆alkyl, -N(C₁₋₆alkyl)₂; and the 3-6 membered heterocyclic groups is optionally substituted with one, two, or three groups selected from F, Cl, Br, and I.

In one embodiment, the compound shown in formula I is selected from the following structures: 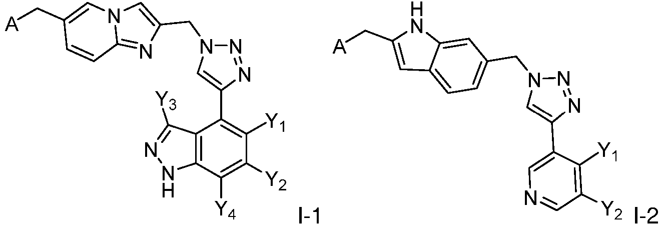 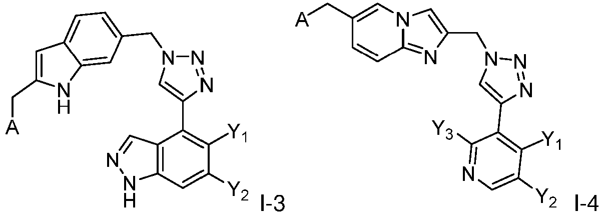 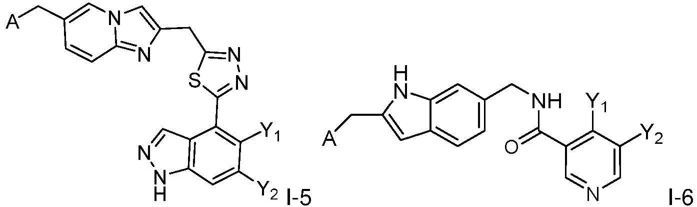 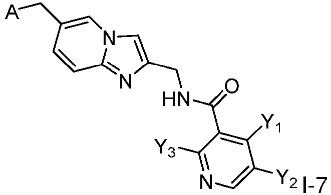
where A is selected from 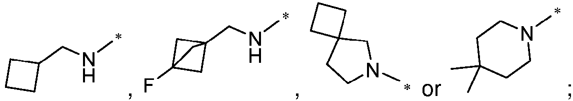 and
Y₁, Y₂, Y₃, and Y₄ are the same or different, independently selected from H, I, Br, Cl, F, NH₂, 3-6 membered heterocyclic groups, -Se-C₁₋₆alkyl, -S-C₁₋₆alkyl, -N(C₁₋₆alkyl)₂, -NH-C₁₋₆alkyl, -N(C₁₋₃alkyl-Cl)₂, difluoro 3-6 membered heterocyclic groups (e.g., 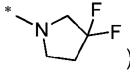 , fluoro 3-6 membered heterocyclic groups (e.g., 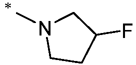 ), 5-6 membered heteroaryl, C₁₋₆alkyl substituted 3-6 membered heterocyclic groups (e.g., 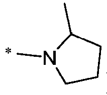 ), C₁₋₆alkyl or C₁₋₆alkoxy.

In one embodiment, 3-6 membered heterocyclic groups are selected from

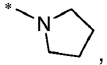

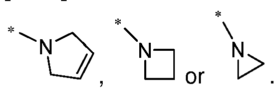

As an example, the compound shown in formula I is selected from the following:

| | | | |
|---|---|---|---|
| E-1 | 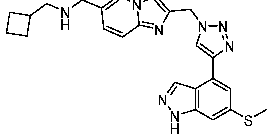 | E-73 | 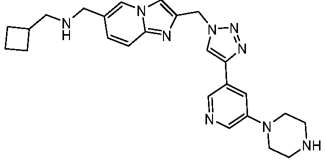 |
| E-2 | 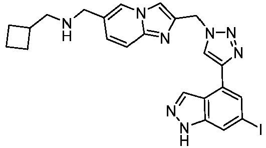 | E-74 | 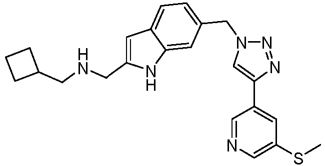 |
| E-3 | 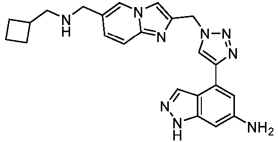 | E-75 | 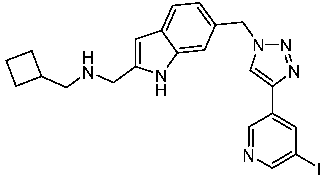 |
| E-4 | 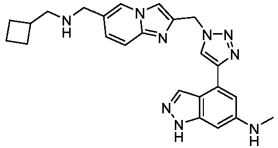 | E-76 | 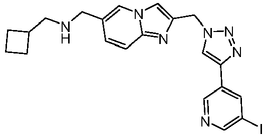 |
| E-5 | 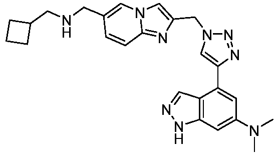 | E-77 | 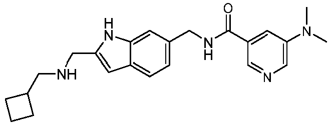 |
| E-6 | 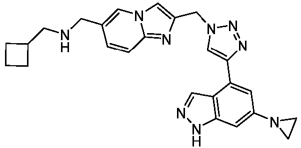 | E-78 | 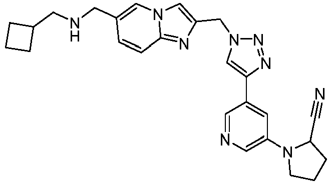 |
| E-7 | 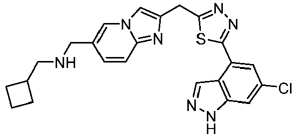 | E-79 | 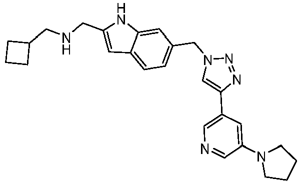 |
| E-8 | 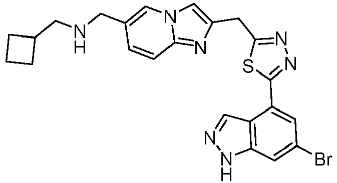 | E-80 | 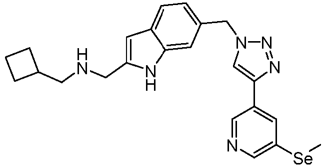 |
| E-9 | 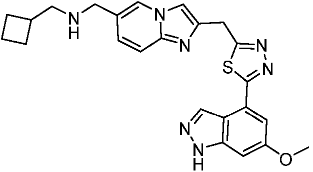 | E-81 | 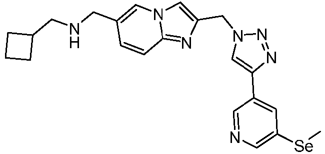 |
| E-10 | 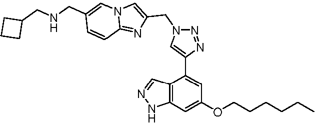 | E-82 | 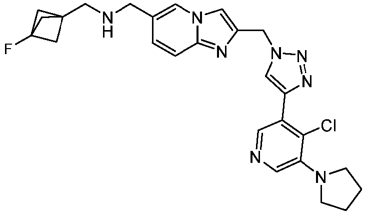 |
| E-11 | 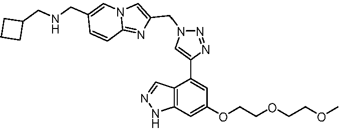 | E-83 | 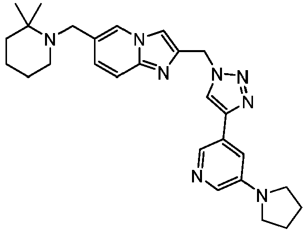 |
| E-12 | 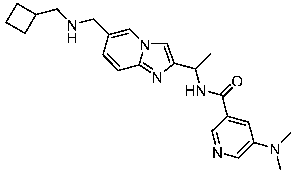 | E-84 | 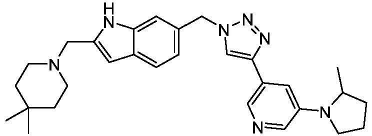 |
| E-13 | 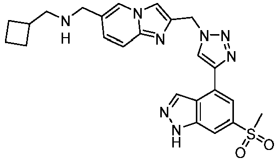 | E-85 | 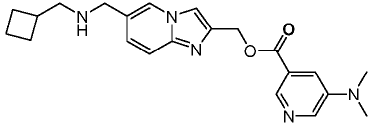 |
| E-14 | 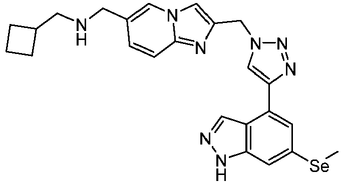 | E-86 | 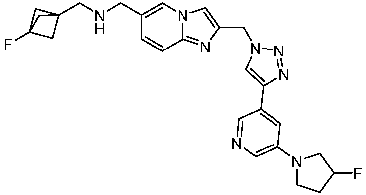 |
| E-15 | 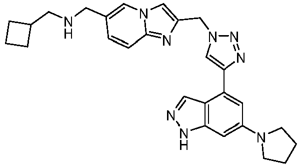 | E-87 | 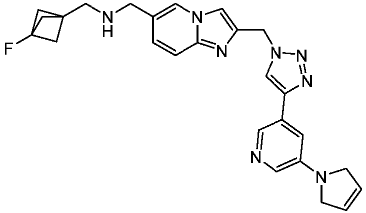 |
| E-16 | 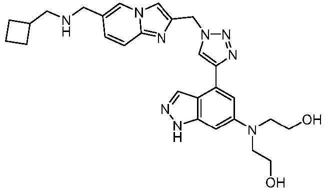 | E-88 | 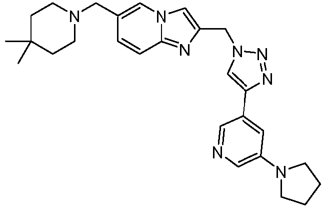 |
| E-17 | 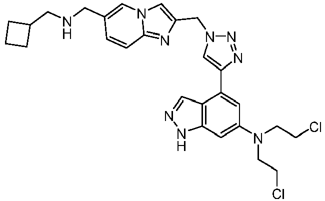 | E-89 | 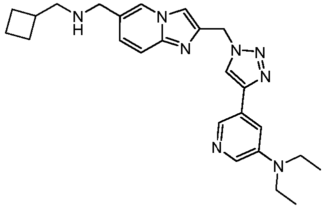 |
| E-18 | 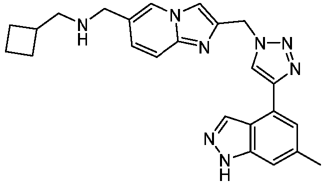 | E-90 | 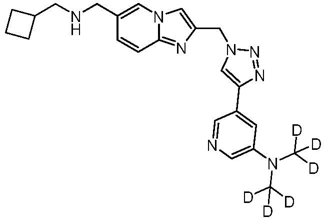 |
| E-19 | 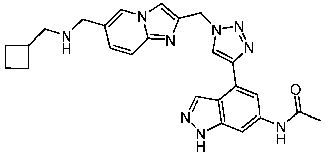 | E-91 | 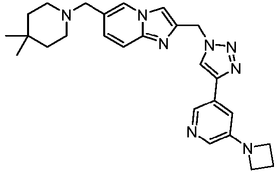 |
| E-20 | 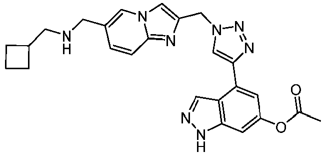 | E-92 | 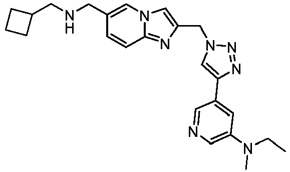 |
| E-21 | 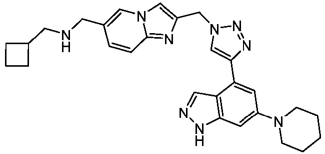 | E-93 | 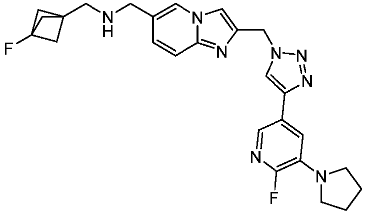 |
| E-22 | 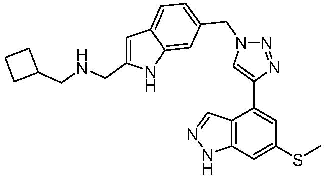 | E-94 | 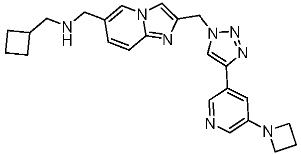 |
| E-23 | 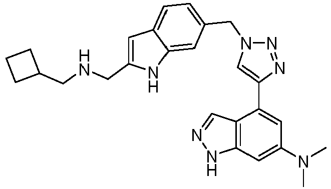 | E-95 | 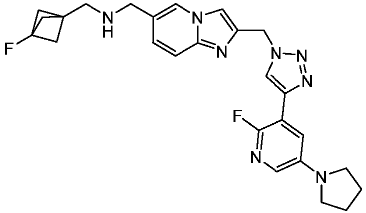 |
| E-24 | 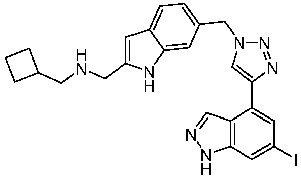 | E-96 | 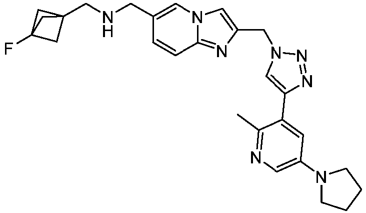 |
| E-25 | 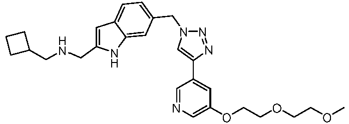 | E-97 | 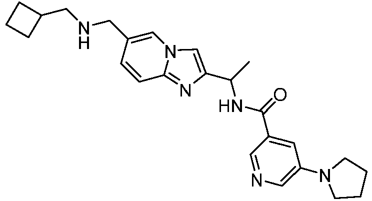 |
| E-26 | 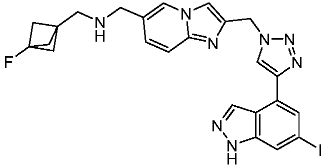 | | |
| E-27 | 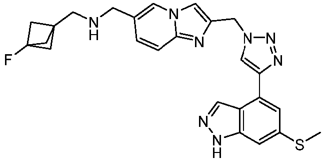 | E-99 | 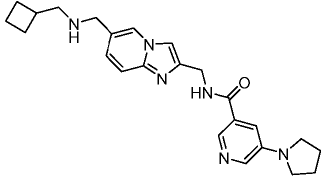 |
| E-28 | 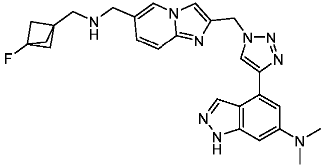 | E-100 | 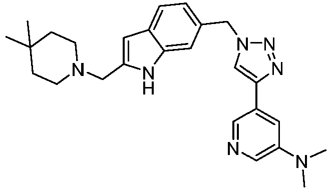 |
| E-29 | 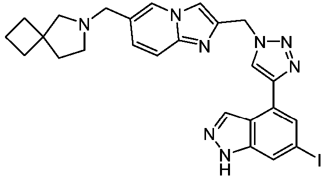 | E-101 | 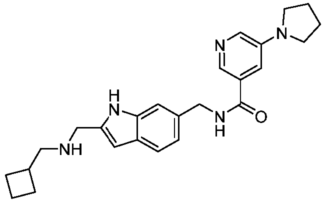 |
| E-30 | 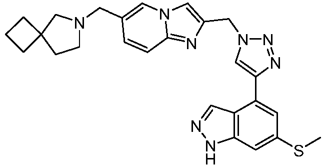 | E-102 | 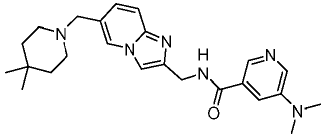 |
| E-31 | 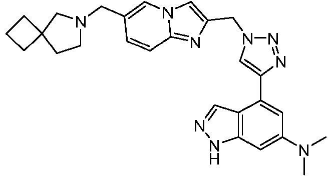 | E-103 | 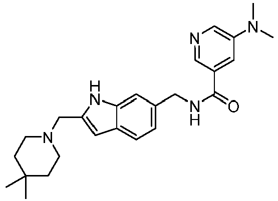 |
| E-32 | 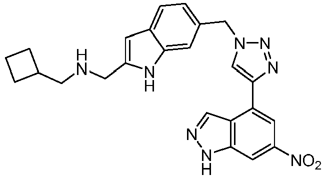 | E-104 | 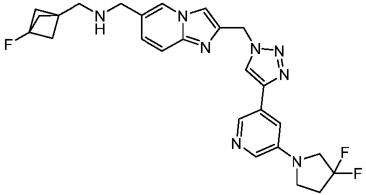 |
| E-33 | 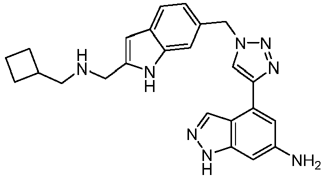 | E-105 | 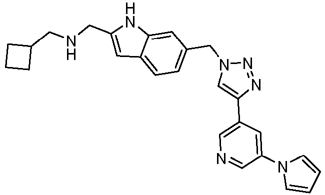 |
| E-34 | 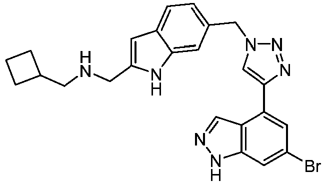 | E-106 | 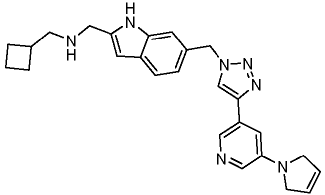 |
| E-35 | 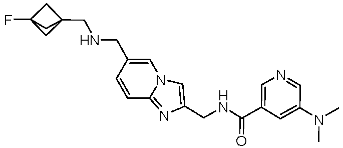 | E-107 | 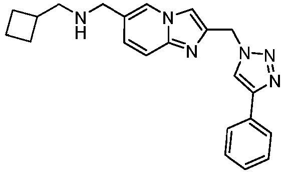 |
| E-36 | 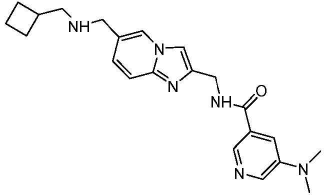 | E-108 | 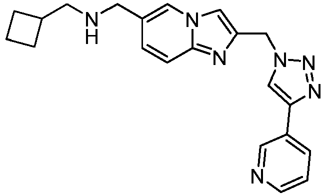 |
| E-37 | 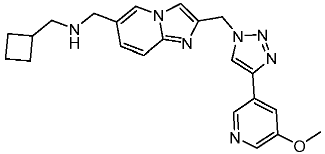 | E-109 | 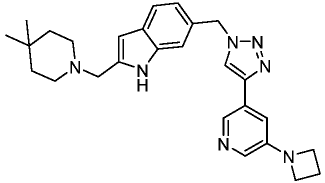 |
| E-38 | 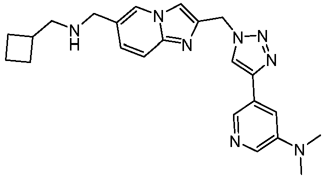 | E-110 | 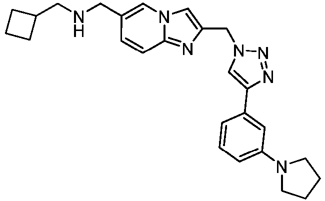 |
| E-39 | 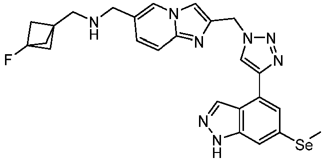 | E-111 | 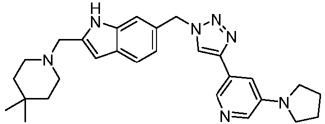 |
| E-40 | 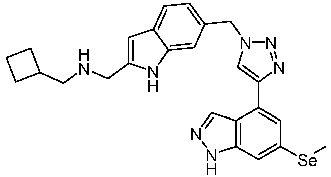 | E-112 | 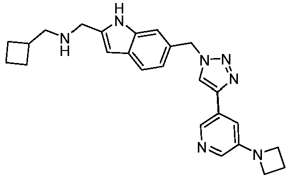 |
| E-41 | 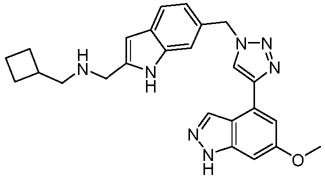 | E-113 | 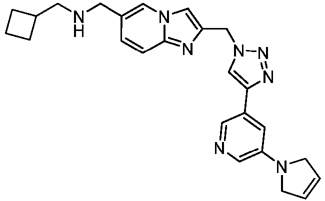 |
| E-42 | 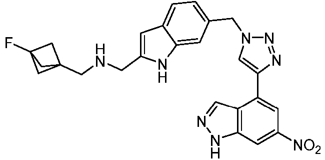 | | |
| E-43 | 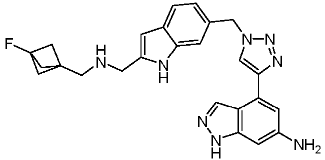 | E-116 | 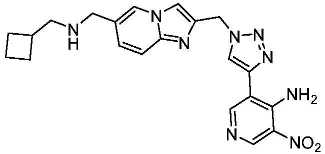 |
| E-44 | 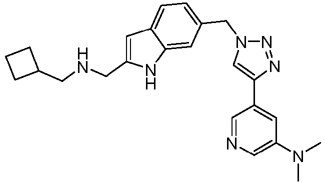 | E-119 | 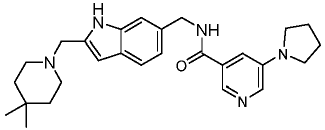 |
| E-45 | 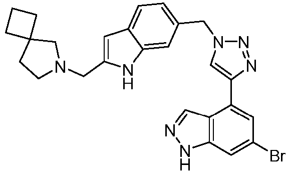 | E-120 | 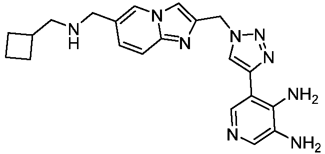 |
| E-46 | 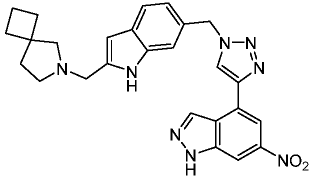 | E-121 | 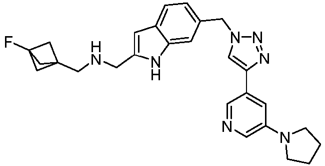 |
| E-47 | 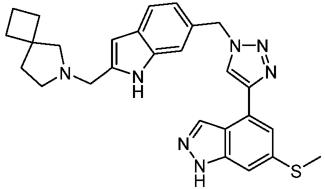 | E-122 | 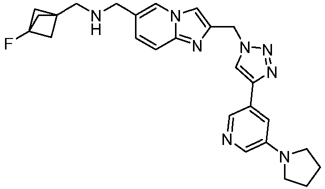 |
| E-48 | 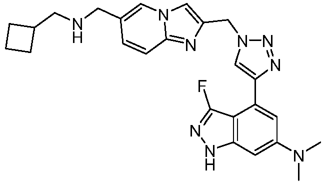 | E-123 | 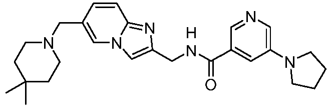 |
| E-49 | 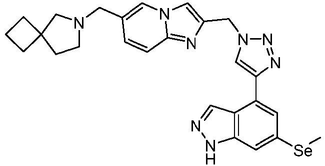 | E-124 | 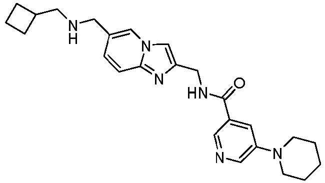 |
| E-50 | 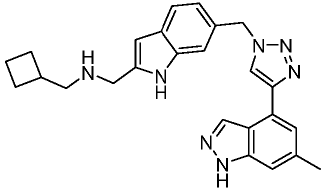 | E-125 | 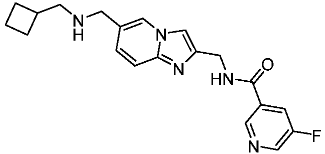 |
| E-51 | 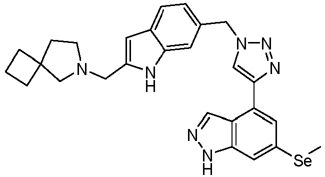 | E-127 | 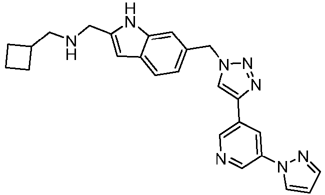 |
| E-52 | 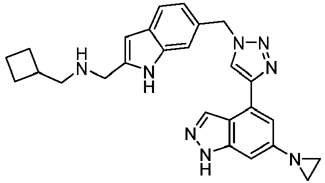 | E-129 | 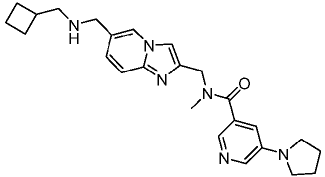 |
| E-53 | 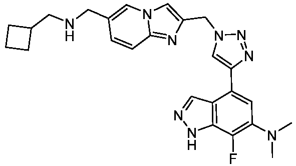 | E-130 | 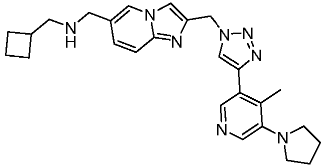 |
| E-54 | 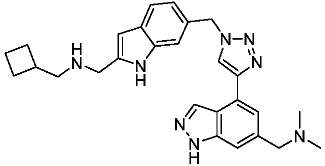 | | |
| E-55 | 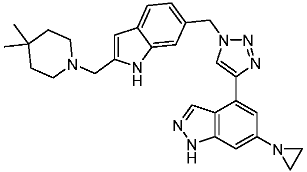 | E-132 | 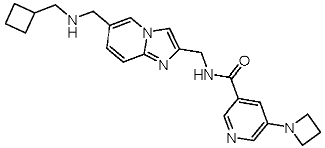 |
| E-56 | 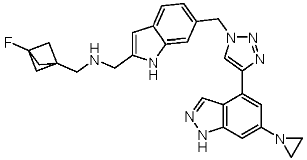 | E-133 | 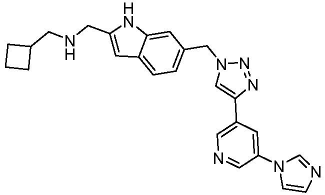 |
| E-57 | 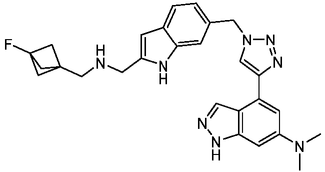 | E-134 | 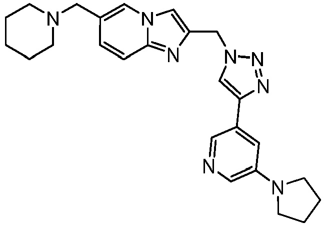 |
| E-58 | 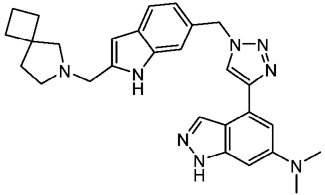 | E-136 | 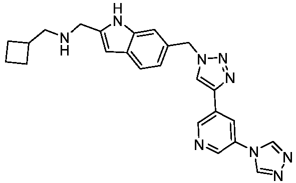 |
| E-59 | 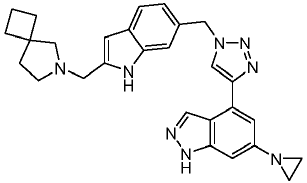 | E-139 | 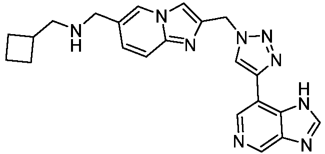 |
| E-60 | 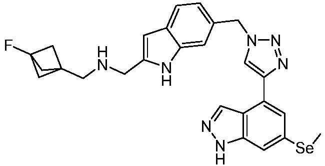 | E-140 | 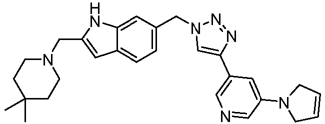 |
| E-61 | 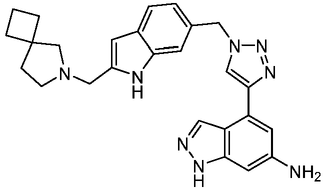 | E-141 | 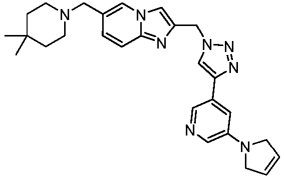 |
| E-62 | 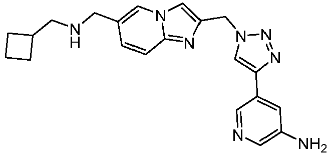 | E-143 | 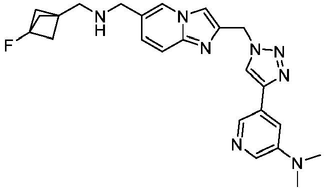 |
| E-63 | 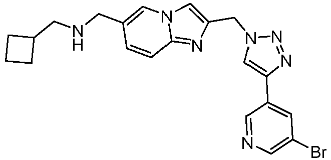 | E-68 | 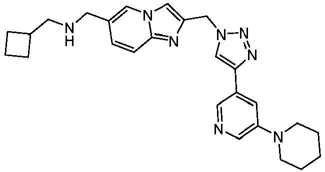 |
| E-64 | 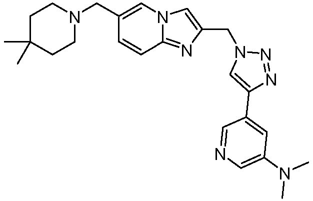 | E-69 | 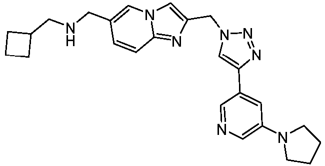 |
| E-65 | 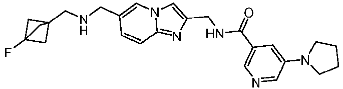 | E-70 | 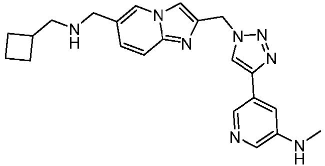 |
| E-66 | 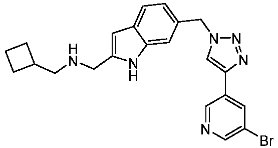 | E-71 | 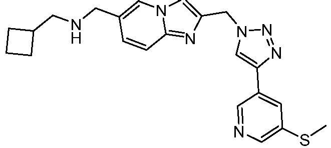 |
| E-67 | 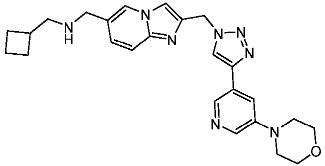 | E-72 | 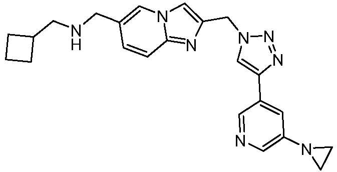 |

According to the embodiments of the present invention, the pharmaceutically acceptable salts are HCl or TFA salts.

As an example, the compound shown in formula I is selected from TFA salts or HCl salts of the following compounds:

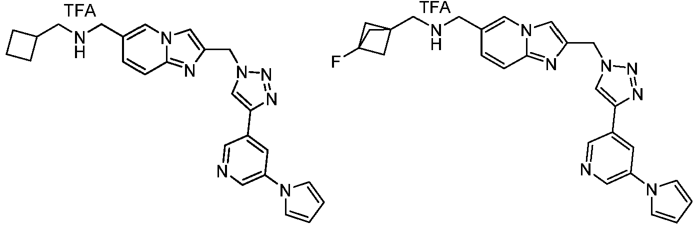

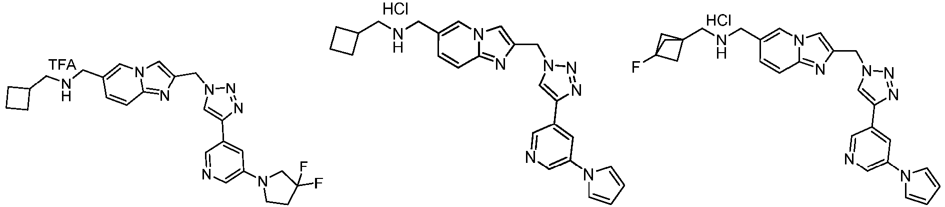

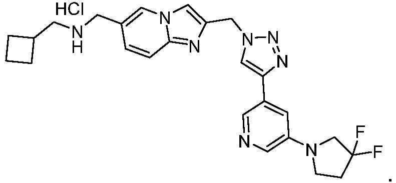

According to the embodiments of the present invention, in formula I', R₁ is selected from the following groups: -NH-(C₁₋₃alkyl)-bicyclo[1.1.1]pentane-(Ra)n, -NH-(C₁₋₃alkyl)-C₆₋₁₂ aryl-(Ra)n, -NH-(C₁₋₃alkyl)-(Ra)n, -NH-(C₁₋₃alkyl)-C₃₋₁₂cycloalkyl-(Ra)n, -NH-(C₁₋₃alkyl)-3-12 membered heterocyclic groups-(Ra)n, -3-12 membered heterocyclic groups-(Ra)n, -NH-(C₁₋₃alkyl)-5-12 membered heteroaryl-(Ra)n, -NH-(C₁₋₃alkyl)-bicyclo[2.2.2]octane-(Ra)n, and -NH-(C₁₋₃alkyl)-cubane-(Ra)n;
Ra is selected from H, OH, halogen, halogenated C₁₋₃alkyl, C₁₋₃alkyl, CN or C₂₋₆alkynyl;
n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₃alkyl, or CN;
R₃ is selected from -5-12 membered heteroaryl-Rb, -OC(O)-5-12 membered heteroaryl-Rb or -NC(O)-5-12 membered heteroaryl-Rb;
Rb is selected from 5-12 membered heteroaryl-Rc, 3-12 membered heterocyclic groups or halogen;
m is 1, 2 or 3;
Rc is selected from -3-20 membered heterocyclic groups-Rd, -5-12 membered heteroaryl-Rd, or -O(C₁₋₃alkyl-O)m-C₁₋₃alkyl; and
Rd is selected from H, deuterium, halogen OH, CN, halogenated C₁₋₃alkyl, C₁₋₃alkyl or =O.

In one embodiment, in formula I', R₁ is selected from the following groups: 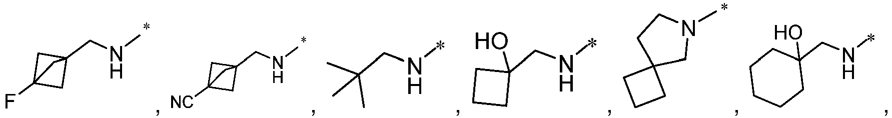 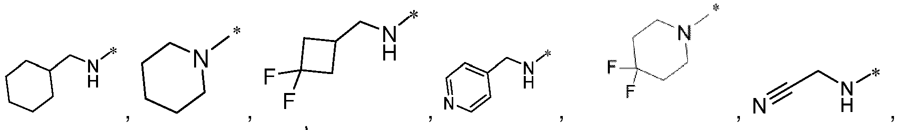 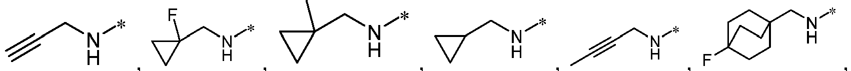 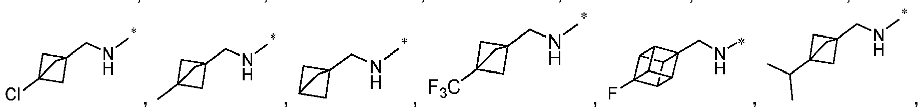 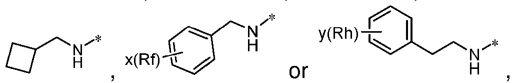 where Rf and Rh are the same or different, independently selected from H, F, Cl, methyl, trifluoromethyl, or CN; and m and n are the same or different, independently selected from 1, 2, 3, 4, or 5.

In one embodiment in formula I', 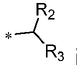 is selected from the following groups:

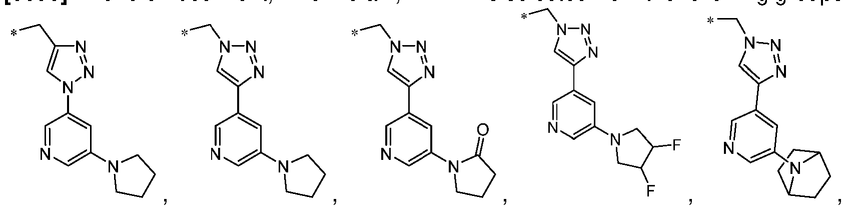

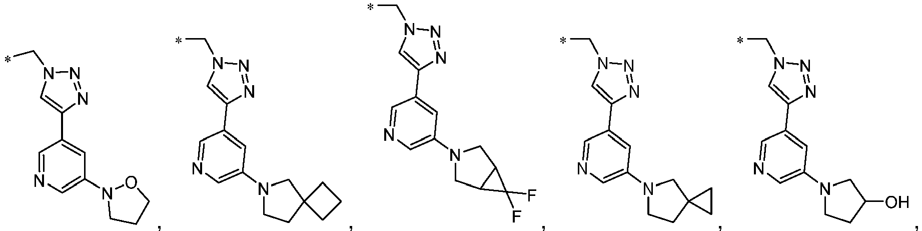

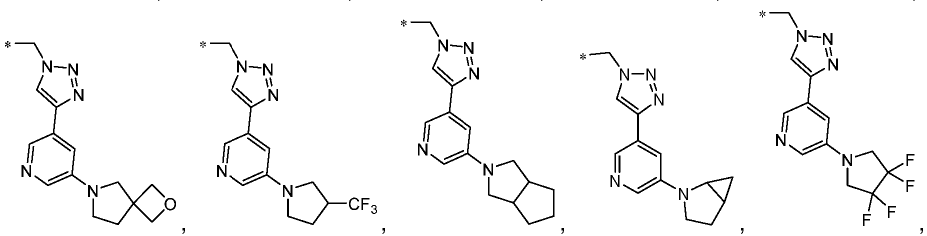

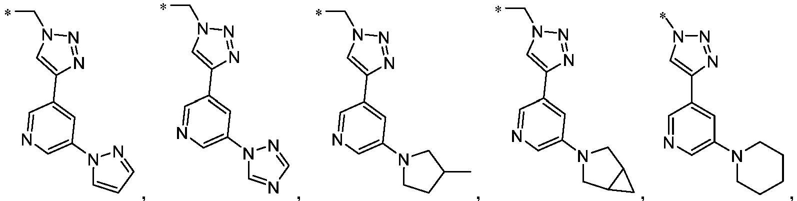

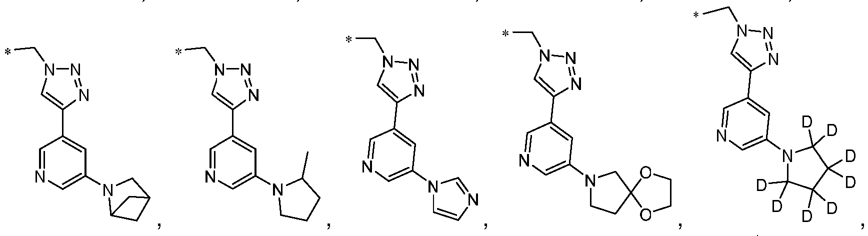

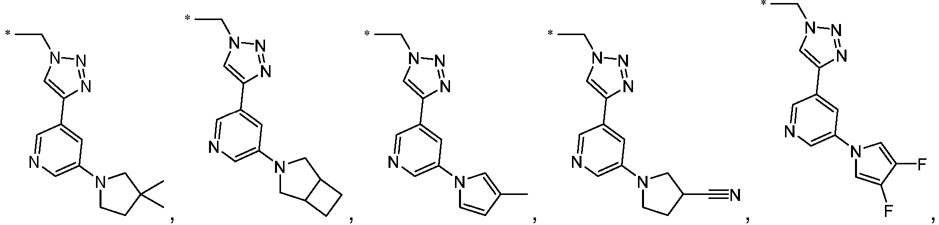

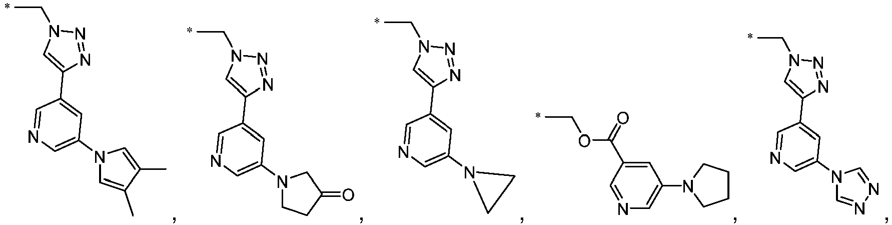

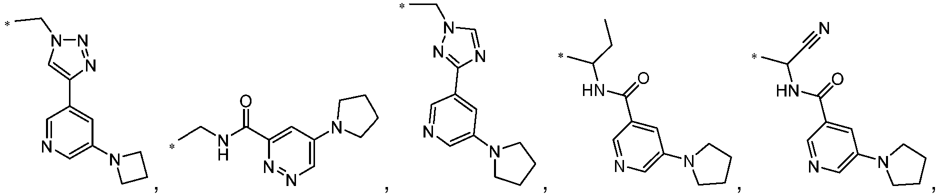

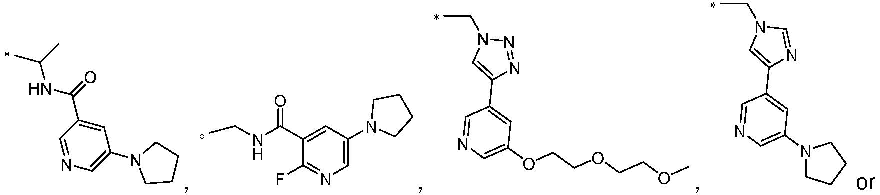

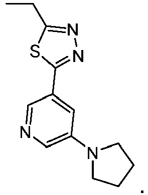

In a preferred embodiment, the compound shown in formula I' is selected from the following structures: 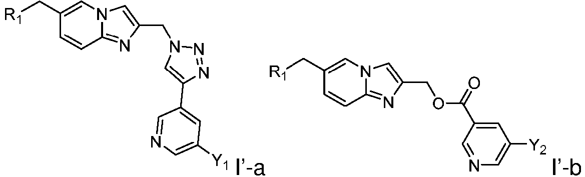 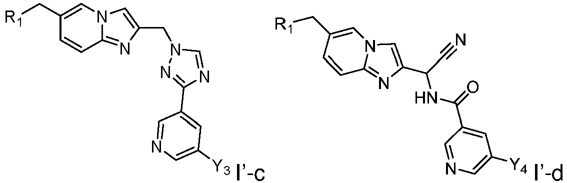 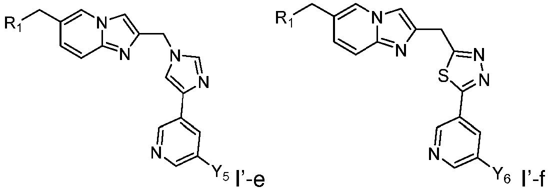 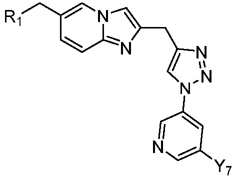
where A is selected from 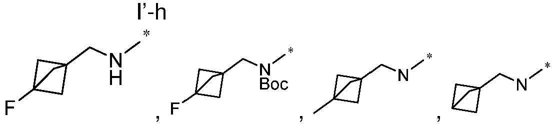 or 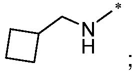 and
Y₁, Y₂, Y₃, Y₄, Y₅, Y₆, and Y₇ are the same or different, independently selected from

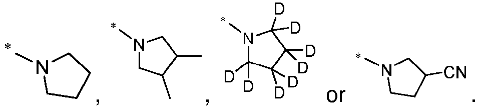

In some embodiments of the present invention, the compound shown in I'-a is prepared using the following method: 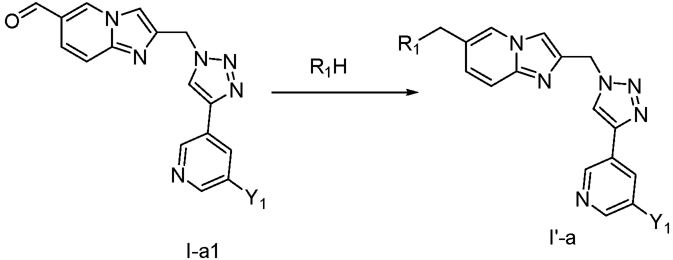
compound I-a1 reacts with compound R₁H to obtain the compound shown in formula I'-a; and
Y₁ and R₁ have the definitions as described above.

In some embodiments of the present invention, the compound shown in I'-b is prepared using the following method: 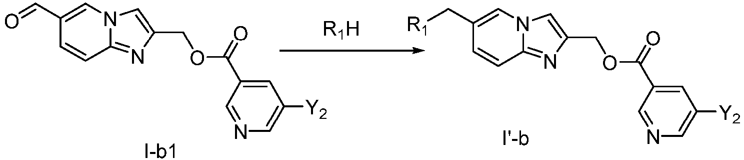
compound I-b1 reacts with compound R₁H to obtain the compound shown in formula I'-b; and
Y₂ and R₁ have the definitions as described above.

In some embodiments of the present invention, the compound shown in I'-c is prepared using the following method: 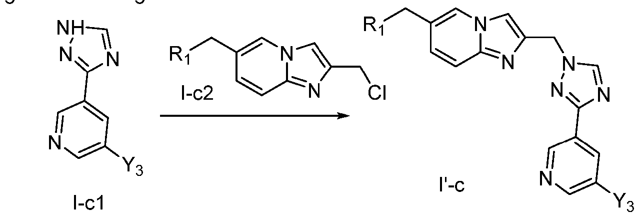
compound I-c1 reacts with compound I-c2 to obtain the compound shown in formula I'-c; and
Y₃ and R₁ have the definitions as described above.

In some embodiments of the present invention, the compound shown in I'-d is prepared using the following method: 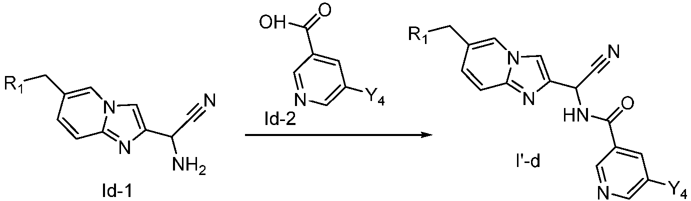
compound I-d1 reacts with compound I-d2 to obtain the compound shown in formula I'-d; and
Y₄ and R₁ have the definitions as described above.

In some embodiments of the present invention, the compound shown in I'-e is prepared using the following method: 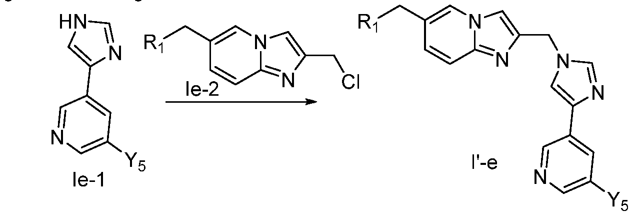
compound IE'-1 reacts with compound IE'-2 to obtain the compound shown in formula I'-e; and
Y₅ and R₁ have the definitions as described above.

In some embodiments of the present invention, the compound shown in I'-f is prepared using the following method: 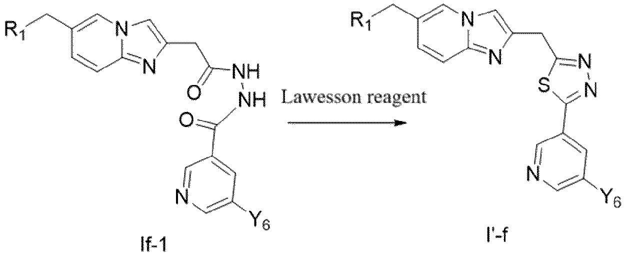
compound If-1 reacts with a lawesson reagent to obtain the compound shown in formula I'-f; and
Y₆ and R₁ have the definitions as described above.

In some embodiments of the present invention, the compound shown in I'-h is prepared using the following method: 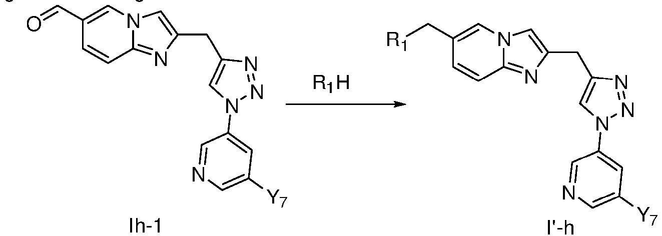
compound Ih-1 reacts with compound R₁H to obtain the compound shown in formula I'-h; and
Y₇ and R₁ have the definitions as described above.

As an example, the compound shown in formula I' is selected from the following:

| | | | |
|---|---|---|---|
| E'-1 | 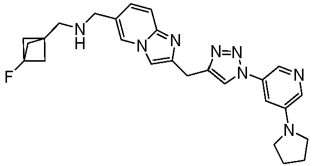 | E'-4-11 | |
| E'-2 | 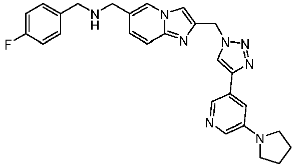 | E'-4-12 | |
| E'-2-1 | 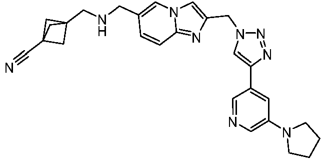 | | |
| E'-2-2 | 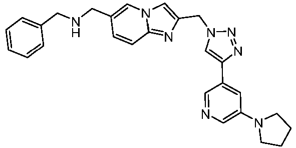 | | |
| E'-2-3 | 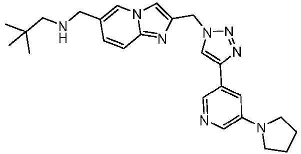 | E'-6 | 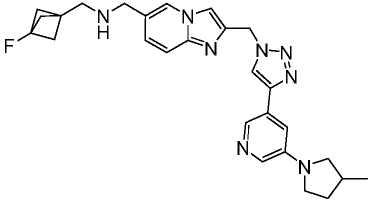 |
| E'-2-4 | 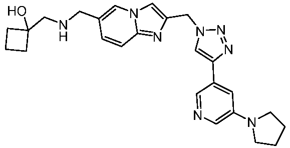 | E'-6-1 | 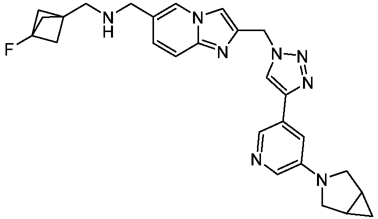 |
| E'-2-5 | 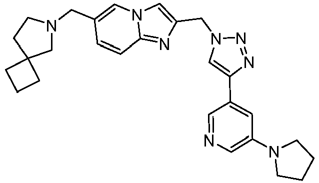 | E'-6-2 | 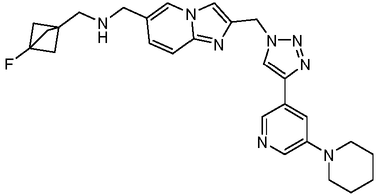 |
| E'-2-6 | 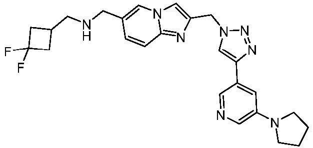 | E'-6-3 | 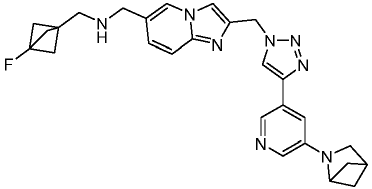 |
| E'-2-7 | 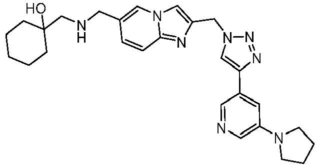 | E'-6-4 | 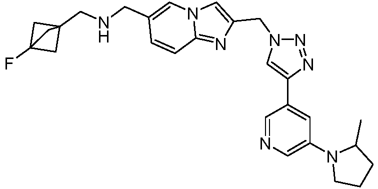 |
| E'-2-8 | 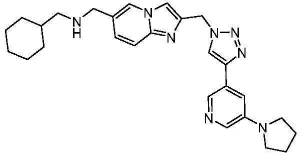 | | 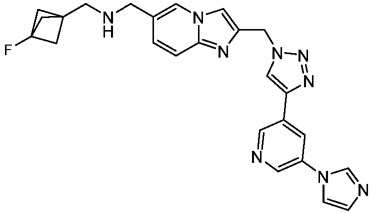 |
| E'-2-9 | 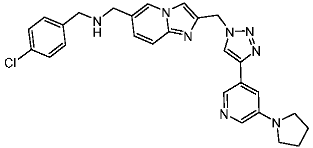 | E'-6-6 | 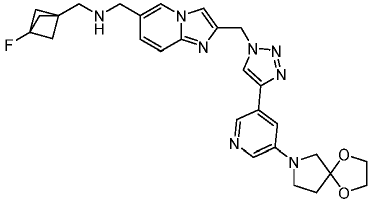 |
| E'-2-10 | 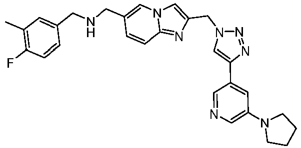 | E'-6-7 | 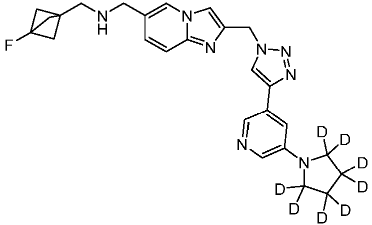 |
| E'-2-11 | 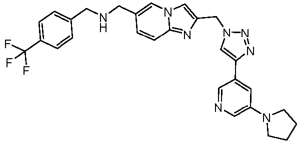 | E'-6-8 | 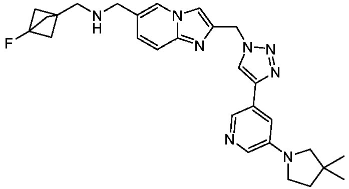 |
| E'-2-12 | 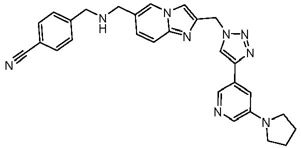 | E'-6-9 | 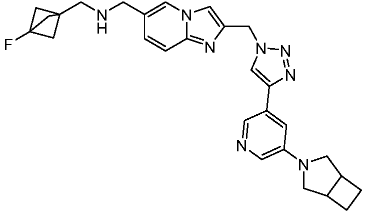 |
| E'-2-13 | 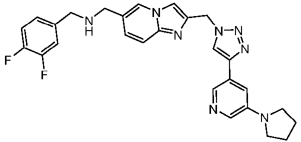 | E'-6-10 | 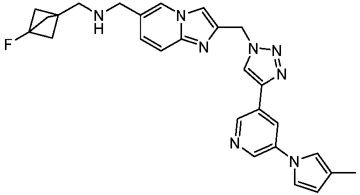 |
| E'-2-14 | 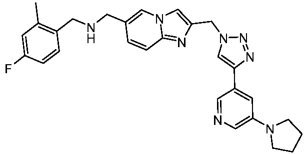 | E'-6-11 | 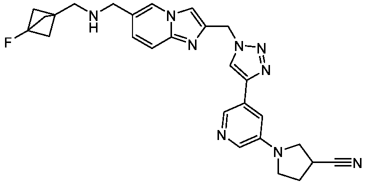 |
| E'-2-15 | 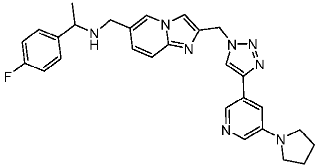 | E'-6-12 | 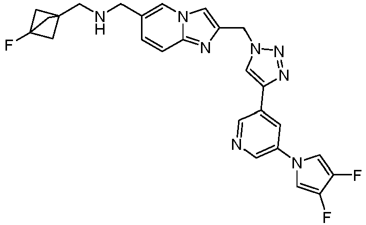 |
| E'-2-16 | 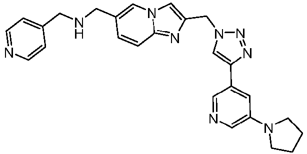 | E'-6-13 | 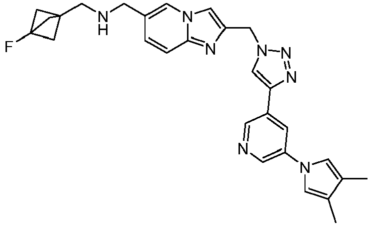 |
| E'-2-17 | 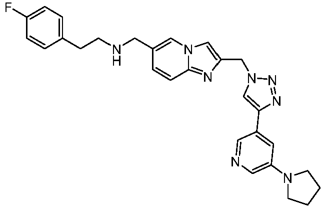 | E'-7 | 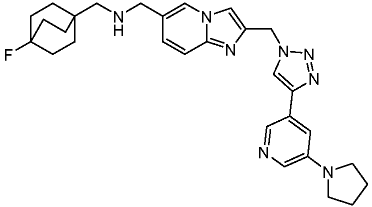 |
| E'-2-18 | 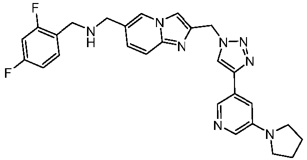 | E'-8 | 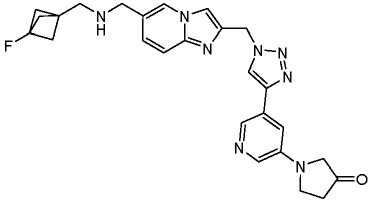 |
| E'-2-19 | 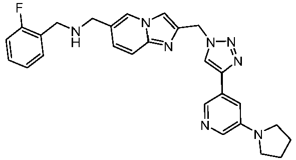 | E'-9 | 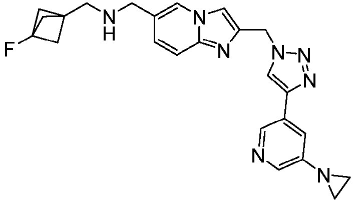 |
| E'-2-20 | 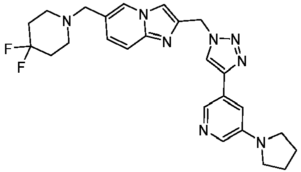 | E'-10 | 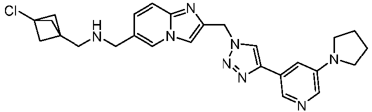 |
| E'-2-21 | 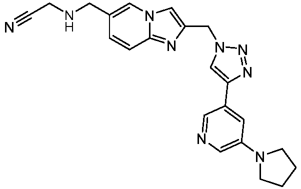 | E'-10-1 | 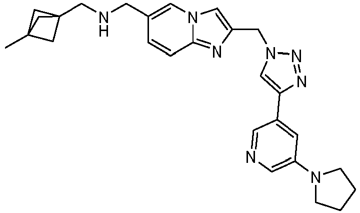 |
| E'-2-22 | 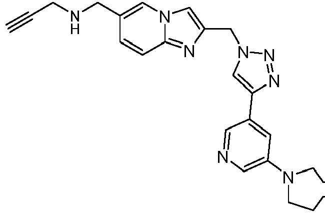 | E'-10-2 | 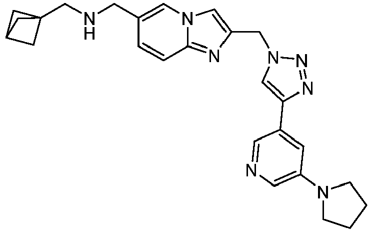 |
| E'-2-23 | 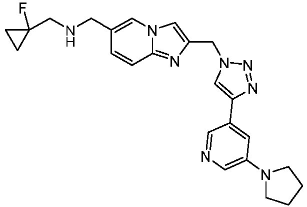 | E'-10-3 | 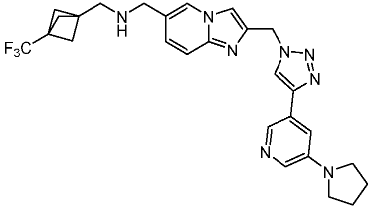 |
| E'-2-24 | 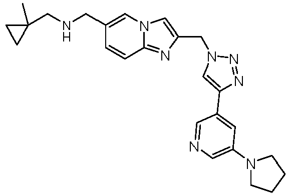 | E'-10-4 | 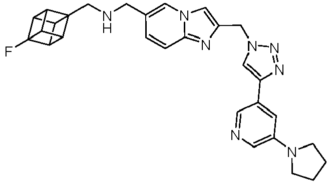 |
| E'-2-25 | 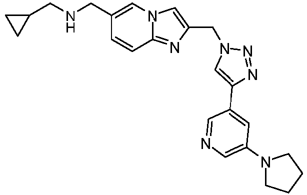 | E'-10-5 | 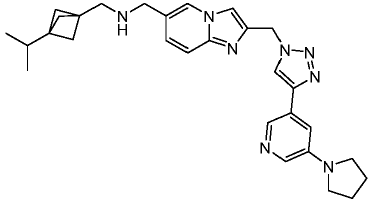 |
| E'-2-26 | 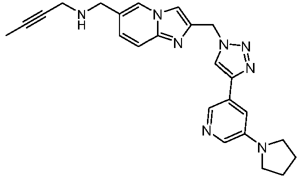 | | |
| E'-3 | 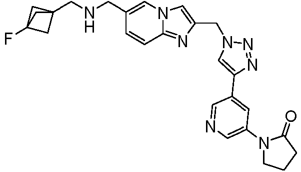 | E'-12 | 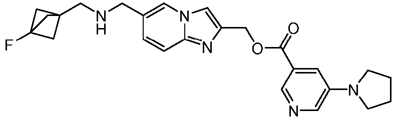 |
| - | | E'-13 | 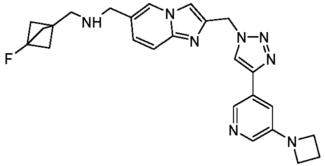 |
| E'-4-1 | 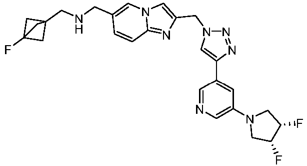 | E'-14 | 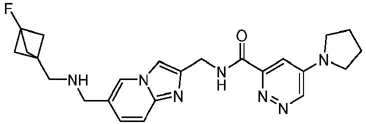 |
| E'-4-2 | 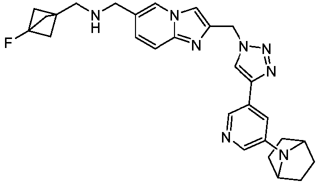 | E'-15 | 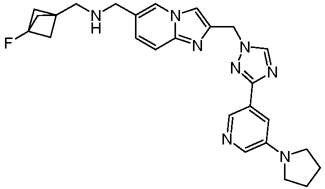 |
| E'-4-3 | 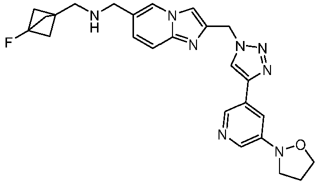 | E'-16 | 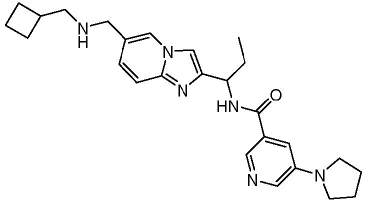 |
| E'-4-4 | 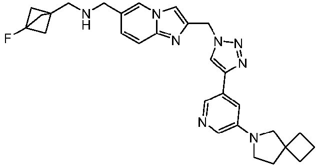 | E'-16-1 | 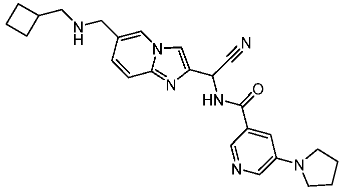 |
| E'-4-5 | 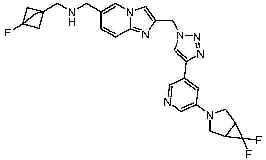 | E'-16-2 | 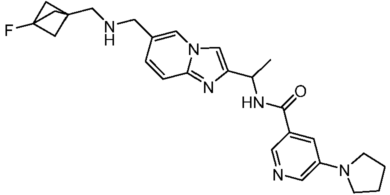 |
| E'-4-6 | 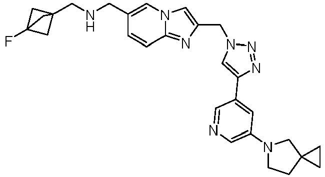 | E'-17 | 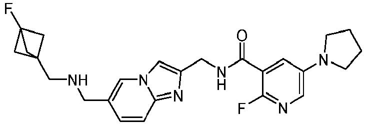 |
| E'-4-7 | 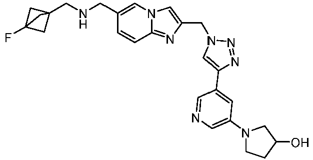 | E'-18 | 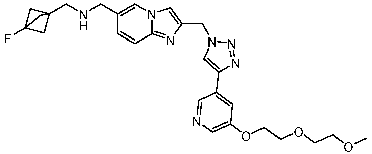 |
| E'-4-8 | 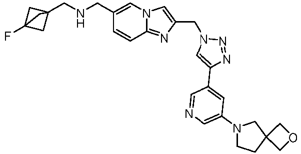 | E'-19 | 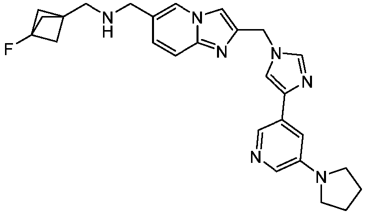 |
| E'-4-9 | 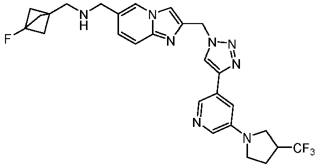 | E'-20 | 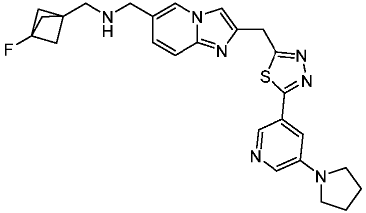 |
| E'-4-10 | 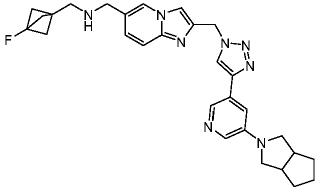 | | |
| | | | |

According to the embodiments of the present invention, the compound shown in formula I' is selected from TFA salts or HCl salts of the following compounds:

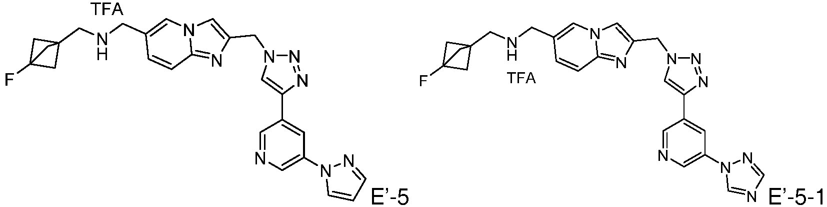

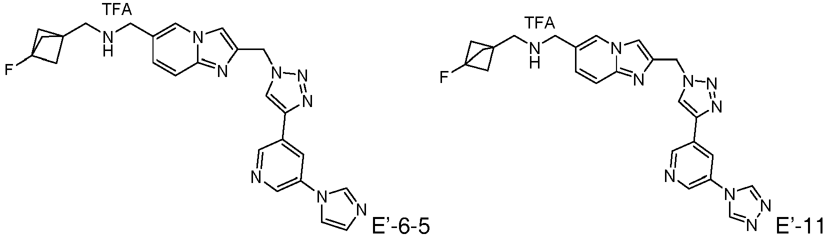

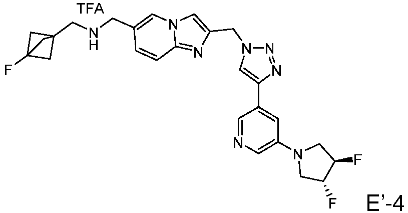

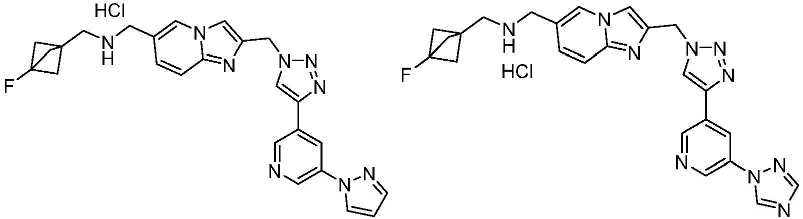

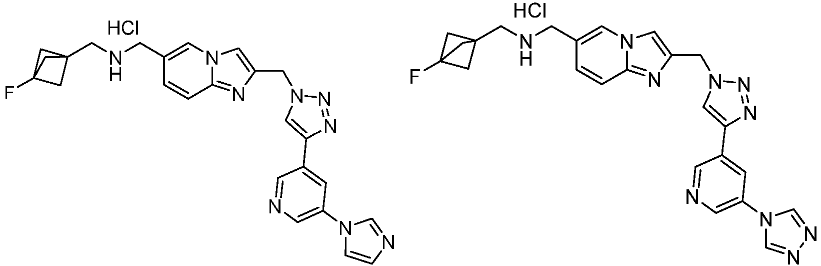

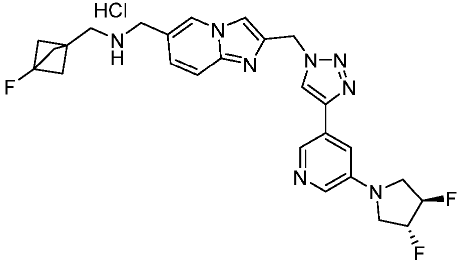

According to the embodiments of the present invention, in formula I", R₁ is selected from 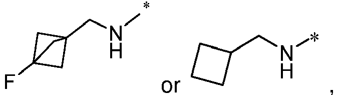 R₂ is selected from C₁₋₆alkyl, R₃ is selected from -OC(O)-5-6 membered heteroaryl-Rm or -NC(O)-5-6 membered heteroaryl-Rm, and Rm is selected from -NH-(C₁₋₃alkyl), -N(C₁₋₃alkyl)₂ or 3-6 membered heterocyclic groups.

According to the embodiments of the present invention, in formula I", R₁ is selected from 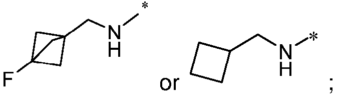 R₂ is selected from C₁₋₃alkyl, such as methyl or ethyl; and R₃ is selected from

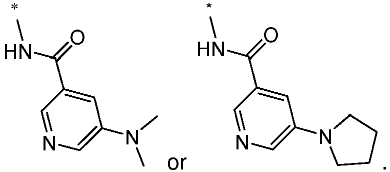

According to the embodiments of the present invention, the compound shown in formula I" is selected from the following compounds:

| | | | |
|---|---|---|---|
| E'-16-2A | 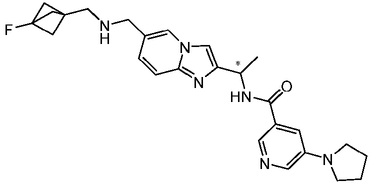 | E'-16-2B | 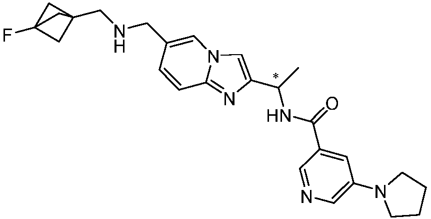 |
| E'-16-3 | 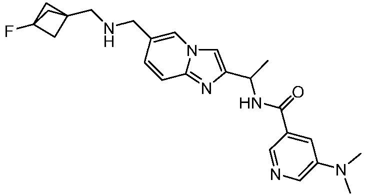 | E'-16-3A | 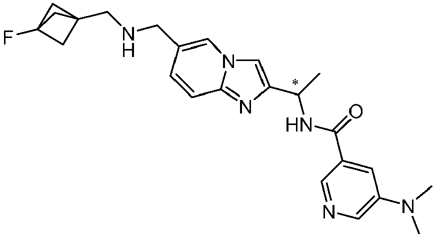 |
| E'-16-3B | 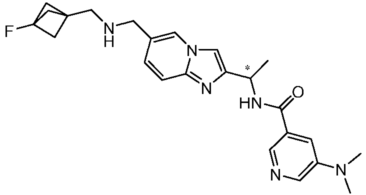 | E'-16-4 | 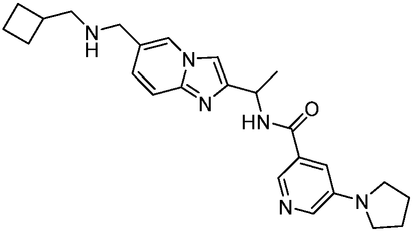 |
| E'-16-5 | 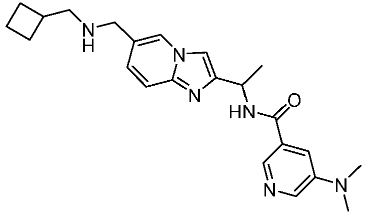 | E'-16-6 | 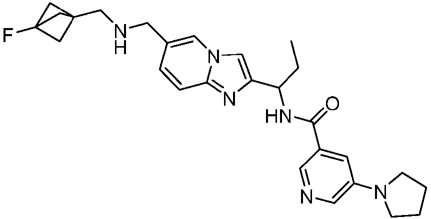 |
| E'-16-6A | 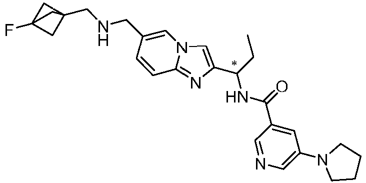 | E'-16-6B | 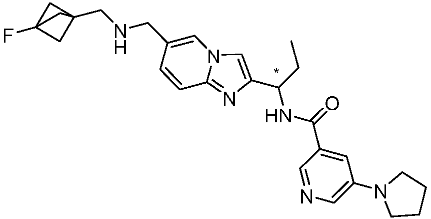 |
| E'-16-7 | 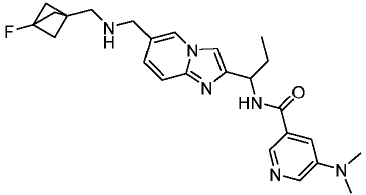 | E'-16-7A | 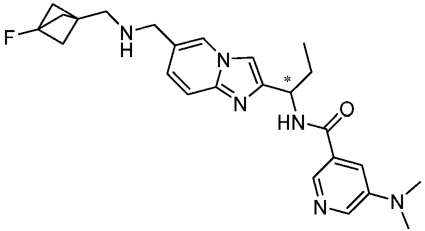 |
| E'-16-7B | 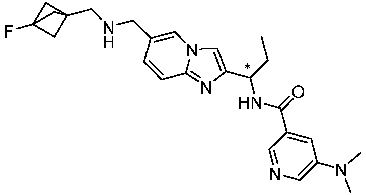 | | |

According to the embodiments of the present invention, the pharmaceutically acceptable salts are HCl or TFA salts.

According to the embodiments of the present invention, it can be seen from the compounds listed above and the general structures of the compounds provided in the present application that the compounds provided in the present application also have chiral centers. Therefore, the enantiomers of the compounds given above are also within the scope of protection of the present application. The present invention also provides use of at least one of the compound shown in formula I, formula I' or formula I", or the tautomers, stereoisomers, or pharmaceutically acceptable salts of the three compounds in preparation of METTL3 inhibitors.

According to the embodiments of the present invention, diseases, symptoms or conditions prevented, alleviated and/or treated by the METTL3 inhibitors include solid tumors, leukemia, autoimmune diseases, neurological diseases, inflammatory diseases or infectious diseases.

As an example, the diseases, symptoms or conditions prevented, alleviated and/or treated by the METTL3 inhibitors include hematological malignant tumors, AML leukemia, chronic myeloid leukemia, solid tumors or diseases caused by viruses.

The present invention also provides a pharmaceutical composition, including the compound shown in formula I, formula I' or formula I", or the tautomers, stereoisomers, or pharmaceutically acceptable salts of the three compounds.

According to the embodiments of the present invention, the pharmaceutical composition is used for treating, alleviating, and/or preventing diseases, symptoms or conditions related to METTL3 dysfunction.

According to the embodiments of the present invention, the pharmaceutical composition may be administered by oral, rectal, topical, buccal, parenteral, intramuscular, intradermal, intravenous and transdermal administration.

The present invention also provides a method for treating, alleviating, and/or preventing METTL3 dysfunction, including administering the compound as described above to individuals in need thereof.

According to the embodiments of the present invention, the diseases, symptoms or conditions in which METTL3 is dysfunctional include solid tumors, leukemia, autoimmune diseases, neurological diseases, inflammatory diseases or infectious diseases.

According to the embodiments of the present invention, the diseases, symptoms or conditions include hematological malignant tumors, AML leukemia, chronic myeloid leukemia, solid tumors or diseases caused by viruses.

The present invention also provides a method for treating, alleviating, and/or preventing the following diseases, symptoms or conditions, including: administering the compound as described above to individuals in need thereof, where the diseases, symptoms or conditions include solid tumors, leukemia, autoimmune diseases, neurological diseases, inflammatory diseases or infectious diseases.

According to the embodiments of the present invention, the diseases, symptoms or conditions include hematological malignant tumors, AML leukemia, chronic myeloid leukemia, solid tumors or diseases caused by viruses.

### Beneficial effects

The compounds provided by the present invention have good regulatory and inhibitory effects on METTL3/METTL14 and AML related cell lines. In terms of enzyme and cell activity, they have obvious advantages compared with compounds disclosed in the prior art for example, in WO2022074379A1 (under the same Assay, the enzyme and cell activity of the compounds provided by the present invention are significantly improved compared with similar compounds in the prior art). Therefore, the compounds provided by the present invention can be used for treating symptoms and diseases associated with the methyltransferase activity of METTL3/METTL14. In addition, the preparation methods of the compounds provided by the present invention are simple and have good application prospects.

### Definition and Explanation of Terms

Unless otherwise stated, definitions of groups and terms set forth in the description and claims of the present application, including their definitions as examples, exemplary definitions, preferred definitions, definitions set forth in tables, definitions of specific compounds described in the examples etc., may be arbitrarily combined and bonded with each other. The group definitions and compound structures after such combinations and bindings shall fall within the scope contained in the description of the present application.

As used herein, the terms "comprise", "include" and/or "contain" are open-ended expressions, i.e., they include what is specified in the present invention, but do not exclude other aspects.

As used herein, when describing one, two, or more in term of number or species, "more" shall refer to situations greater than 2, such as integers greater than or equal to 3, such as 3, 4, 5, 6, 7, 8, 9, or 10.

As used herein, the term "optional/optionally" indicates both the presence or absence of the described feature, which means that the subsequently described event may, but does not necessarily, occur, thus including both cases where the event occurs or does not occur. For example, "heterocyclic groups optionally substituted with alkyl" means that the alkyl may be, but are not necessarily, present, thus including the case of heterocyclic groups substituted with alkyl and heterocyclic groups not substituted with alkyl.

In the present application, the "*" position of some substituents is a linking site, for example, if A in formula I is selected from 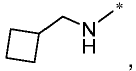 the formula I is structured as follows: 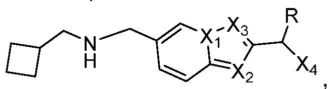 and so on for the rest of the groups.

As used herein, the term "halogen" denotes fluorine, chlorine, bromine and/or iodine. Accordingly, the term "halo" refers to fluoro, chloro, bromo and/or iodo. Within the scope herein, where an atom, residue, group, or moiety is halogenated, the atom at the halogenated position may be mono-substituted, di-substituted, or poly-substituted up to full-substituted with a halogen atom.

The term "C₁₋₁₂alkyl" should be understood to refer to straight or branched-chain saturated monovalent hydrocarbyl groups having 1 to 12 carbon atoms, preferably "C₁₋₆alkyl". The "C₁₋₆alkyl" should be understood to refer to straight or branched-chain saturated monovalent hydrocarbyl groups having 1, 2, 3, 4, 5, or 6 carbon atoms. The alkyl are, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl or the like, or isomers thereof. In particular, the groups have 1, 2 or 3 carbon atoms ("C₁₋₃alkyl"), for example, methyl, ethyl, n-propyl or isopropyl.

The term "C₂₋₁₂alkynyl" should be understood to refer to straight or branched-chain monovalent hydrocarbyl groups, containing one or more triple bonds and having 2 to 12 carbon atoms, preferably "C₂-C₁₀alkynyl". The term "C₂-C₁₀alkynyl" should be understood to refer to straight or branched-chain monovalent hydrocarbyl groups, containing one or more triple bonds and having 2, 3, 4, 5, 6, 7, 8, 9 or 10 carbon atoms, in particular 2 or 3 carbon atoms ("C₂-C₃ alkynyl"). The alkynyl groups are, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpentyl-1-alkynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl group is ethynyl, prop-1-ynyl or prop-2-ynyl. The term "C₃₋₂₀cycloalkyl" should be understood to refer to saturated monovalent monocyclic, bicyclic or polycyclic hydrocarbon rings (also referred to as a fused polycyclic hydrocarbon rings) with 3-20 carbon atoms. The bicyclic or polycyclic cycloalkyl include concyclic cycloalkyl, bridged cycloalkyl, and spirocyclic alkyl; and the concyclic ring refers to a fused ring structure formed by two or more cyclic structures sharing two adjacent ring atoms (i.e., sharing a bond) with each other. The bridged ring refers to a fused ring structure formed by two or more cyclic structures sharing two non-adjacent ring atoms with each other. The spirocyclic ring refers to a fused ring structure formed by two or more cyclic structures sharing one ring atom with each other. For example, the C₃₋₂₀cycloalkyl may be C₃₋₈ monocyclic cycloalkyl, such as cyclopropyl, cyclobutyl, cyclopentyl, cyclohexyl, cycloheptyl, and cyclooctyl, or C₇₋₁₂ concyclic cycloalkyl, such as decahydronaphthalene; and they may also be C₇₋₁₂ bridged cycloalkyl or spirocyclic alkyl, such as

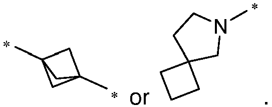

The term "3-20 membered heterocyclic groups" refers to saturated monovalent monocyclic or bicyclic hydrocarbon rings, which include 1-5 heteroatoms independently selected from N, O, and S, preferably "3-12 membered heterocyclic groups". The term "3-12 membered heterocyclic groups" refers to saturated monovalent monocyclic or bicyclic hydrocarbon rings, which include 1-5, preferably 1-3 heteroatoms selected from N, O, and S. The heterocyclic groups may be attached to the rest of a molecule through any of the carbon atoms or a nitrogen atom (if present). In particular, the heterocyclic groups may include, but are not limited to: 4-membered rings, such as azetidinyl, and oxetanyl; 5-membered rings, such as tetrahydrofuranyl, dioxolenyl, pyrrolidinyl, imidazolidinyl, pyrazolidyl, and pyrrolinyl; 6-membered rings, such as tetrahydropyranyl, piperidinyl, morpholinyl, dithianyl, thiomorpholinyl, piperazinyl or trithianyl; or 7-membered rings, such as diazacycloheptyl. Optionally, the heterocyclic groups may be benzo-fused. The heterocyclic groups may be bicyclic, for example, but not limited to, 5,5-membered rings, such as a hexahydrocyclopento[c]pyrrol-2(1H)-yl ring, or a 5,6-membered bicyclic ring, such as a hexahydropyrrolo[1,2-a]pyrazin-2(1H)-yl ring. The nitrogen atom-containing ring may be partially unsaturated, i.e., it may contain one or more double bonds, for example, but not limited to, 2,5-dihydro-1H-pyrrolyl, 4H-[1,3,4]thiadiazinyl, 4,5-dihydrooxazolyl or 4H-[1,4]thiazinyl, or it may be benzo-fused, for example, but not limited to, dihydroisoquinolinyl. According to the present invention, the heterocyclic groups are nonaromatic. According to the present invention, the heterocyclic groups may also be spirocyclic alkyl, such as

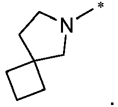

The term "C₆₋₂₀aryl" should be understood to refer to aromatic or partially aromatic monovalent monocyclic, bicyclic or tricyclic hydrocarbon rings having 6 to 20 carbon atoms, preferably "C₆₋₁₄aryl". The term "C₆₋₁₄aryl" should be understood to refer to aromatic or partially aromatic monovalent monocyclic, bicyclic or tricyclic hydrocarbon rings ("C₆₋₁₄aryl") having 6, 7, 8, 9, 10, 11, and 12 carbon atoms, in particular a ring ("C₆aryl") having 6 carbon atoms, such as phenyl; or biphenyl, or a ring ("C₉aryl") having 9 carbon atoms, such as indanyl or indenyl, or a ring ("C₁₀aryl") having 10 carbon atoms, such as tetrahydronaphthyl, dihydronaphthyl or naphthyl, or a ring ("C₁₃aryl") having 13 carbon atoms, such as fluorenyl, or a ring ("C₁₄aryl") having 14 carbon atoms, such as anthryl.

The term "5-20 membered heteroaryl" should be understood to include such monovalent monocyclic, bicyclic or tricyclic aromatic ring systems: they have 5 to 20 ring atoms and contain 1 to 5 heteroatoms independently selected from N, O, and S, such as "5-12 membered heteroaryl". The term "5-12 membered heteroaryl" should be understood to include such monovalent monocyclic, bicyclic or tricyclic aromatic ring systems: they have 5, 6, 7, 8, 9, 10, 11, and 12 ring atoms, in particular 5, 6, 9 or 10 carbon atoms, and contain 1 to 5, preferably, 1 to 3 heteroatoms independently selected from N, O, and S; and in addition, in each case, they may be benzo-fused. In particular, the heteroaryl are selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl, and thi-4H-pyrazolyl, and benzo derivatives thereof, for example, benzofuryl, benzothienyl, benzoxazolyl, benzisoxazolyl, benzimidazolyl, benzotriazolyl, indazolyl, indolyl, isoindolyl, and the like; or pyridyl, pyridazinyl, pyrimidinyl, pyrazinyl, and triazinyl, and benzo derivatives thereof, such as quinolyl, quinazolinyl, and isoquinolyl; or acridyl, indolazinyl, purinyl, and benzo derivatives thereof; or cinolinyl, phthalazinyl, quinazolinyl, quinoxalinyl, naphthyridinyl, pteridyl, carbazolyl, acridinyl, phenazinyl, phenothiazinyl, phenoxazinyl, and the like.

Unless otherwise specified, a heterocyclic group, heteroaryl or sub-heteroaryl includes all possible isomeric forms thereof, such as positional isomers thereof. Therefore, for some illustrative non-limiting examples, pyridyl or pyridylidene includes pyridine-2-yl, pyridylidene-2-yl, pyridine-3-yl, pyridylidene-3-yl, pyridine-4-yl, and pyridylidene-4-yl; and thiophene or thenylidene includes thiophene-2-yl, thenylidene-2-yl, thiophene-3-yl, and thenylidene-3-yl.

The above definition of the term "C₁₋₁₂alkyl" also applies to other terms containing "C₁₋₁₂alkyl", such as the terms "halogenated C₁₋₁₂alkyl", "C₁₋₁₂alkoxy", "halogenated N(C₁₋₁₂alkyl)₂", and so on.

As used herein, "pharmaceutically acceptable salts" refer to salts of the compounds of the present invention, which are safe and effective for use in mammals, and have due biological activity.

Pharmaceutically acceptable salts include acid addition salts of the compounds of the present invention having nitrogen atoms in chains or rings with sufficient alkalinity. In addition, basic nitrogen-containing groups can be quaternized using the following reagents: lower alkyl halides, such as methyl, ethyl, propyl and butyl chlorides, bromides and iodides; dialkyl sulfates, such as dimethyl sulfate, diethyl sulfate, dibutyl sulfate and diamyl sulfate; long chain halides, such as decyl, lauryl, myristyl and stearyl chlorides, bromides and iodides; and aralkyl halides, such as benzyl and phenethyl bromides. By way of example, physiologically/pharmaceutically acceptable salts include, but are not limited to, hydrochloride, sulfate, nitrate, bisulfate, hydrobromide, acetate, oxalate, citrate, mesylate, formate, or meglumine salts, and the like.

Since the compounds provided by the present invention may have multiple salt-forming sites, the physiologically/pharmaceutically acceptable salts include not only salts formed at one salt-forming sites of the compounds provided by the present invention, but also salts formed at 2^{nd}, 3^{rd} or all salt-forming sites. Therefore, in the physiologically/pharmaceutically acceptable salts, the molar ratio of the compound as shown in formula (I) to the radical ion (anion) of the acid or the cation of the base required for the formation of the salt may vary over a wide range, for example, from 4:1 to 1:4, such as 3:1, 2:1, 1:1, 1:2, and 1:3.

### Detailed Description

The technical solution of the present invention will be described in further detail below with reference to specific examples. It should be understood that the following examples are only exemplary to illustrate and explain the present invention, and should not be construed as limiting the scope of the present invention. All technologies implemented based on the above content of the present invention are covered within the intended protection scope of the present invention.

Unless otherwise specified, the raw materials and reagents used in the following examples are commercially available or can be prepared by known methods.

It should be noted that, in the following Examples, a solution of HCl in 1,4-dioxane is used for deaminating the protecting group. It is well known to those skilled in the art that upon deprotection, free hydrochloric acid will form a salt with an electron-donating amino group in a compound, resulting in the hydrochloride salt of the compound. Therefore, even though some compounds in the following examples are not labeled as hydrochloride salts, it is well known to those skilled in the art that they should have hydrochloride structures. The number of hydrochloride salts is determined by the number of salt-forming amino groups in the hydrochloride structures. Similarly, when other acids such as TFA are used for deprotection, the corresponding salts will be formed.

### 1.1 Preparation of intermediates of compound corresponding to formula I

### Intermediate I-1: Synthesis of tert-butyl ((2-(azidomethyl)imidazo[1,2-a]pyridin-6-yl)methyl)(cyclobutylmethyl)carbamate

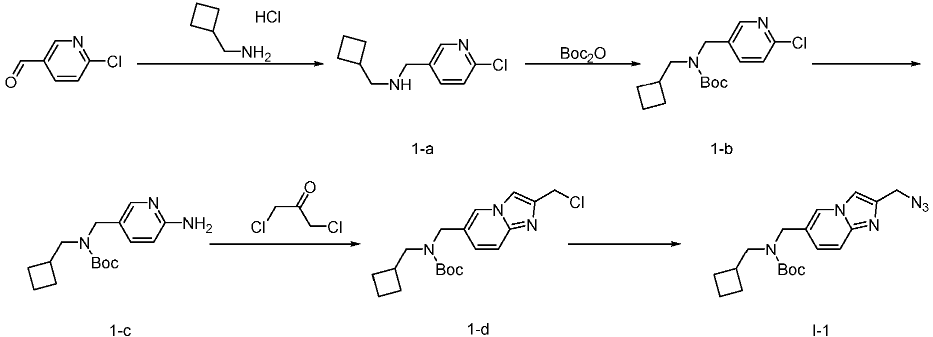

### Intermediate 1-a: 1-(6-chloropyridin-3-yl)-N-(cyclobutylmethyl)methylamine

A solution of sodium borohydride (2.21 g, 55.494 mmol, 3.31 eq) in MeOH (2 mL) was added in batches to a solution of 6-chloronicotinaldehyde (2.5 g, 16.779 mmol, 1.00 eq), 1-cyclobutylmethylamine hydrochloride (1.8 g, 20.082 mmol, 1.20 eq) and magnesium sulfate (1.06 g, 8.366 mmol, 0.50 eq) in 1,2-dichloroethane (30 mL) while stirring, and the resulting mixture was stirred at 40°C for 4 h under nitrogen protection. The mixture was quenched with a saturated sodium bicarbonate solution and extracted with DCM (3 x 30 mL), and the combined organic layers were washed with saline (3 x 10 mL) and dried over anhydrous sodium sulfate. After filtration and concentration, the residue was purified by silica gel column chromatography and eluted with DCM/EtOAc (1:7) to obtain a yellow oily intermediate **1-a** (1.38 g, 37.78%). LC-MS (ESI): m/z 210.7 [M+H]⁺.

### Intermediate 1-b: Tert-butyl ((6-chloropyridin-3-yl)methyl)(cyclobutylmethyl)carbamate

Intermediate **1-a** (1.38 g, 6.340 mmol, 1.00 eq) and di-tert-butyl dicarbonate (1.51 g, 6.573 mmol, 1.04 eq) were dissolved in MeCN (15 mL) at 25°C, and the resulting mixture was stirred at 80°C for 2 h under nitrogen protection. The mixture was concentrated, and the residue was purified by silica gel column chromatography and eluted with petroleum ether/ethyl acetate (7:1) to obtain a yellow oily intermediate **1-b** (1.87 g, 93.19%).

LC-MS (ESI): m/z 310.8 [M+H]⁺.

### Intermediate 1-c: Tert-butyl ((6-aminopyridin-3-yl)methyl)(cyclobutylmethyl)carbamate

Intermediate **1-b** (1.87 g, 5.908 mmol, 1.00 eq), 2-dicyclohexylphosphino-2', 4', 6'-triisopropyl biphenyl (563.29 mg, 1.123 mmol, 0.19 eq) and tris(dibenzylideneacetone)dipalladium (284.74 mg, 0.295 mmol, 0.05 eq) were dissolved in THF (20 mL), and the reaction solution was deoxygenated with nitrogen for 30 min. LiHMDS (45.5 mL, 45.5 mmol, 7.70 eq) was then added dropwise at 0°C under nitrogen protection, and the resulting mixture was stirred at 70°C for 2 h under nitrogen protection. The resulting mixture was concentrated, and the residue was purified by silica gel column chromatography and eluted with petroleum ether/EtOAc (1:3) to obtain a yellow solid intermediate **1-c** (1.0 g, 53.56%).

LC-MS (ESI): m/z 291.4 [M+H]⁺.

### Intermediate 1-d: Tert-butyl ((2-(chloromethyl)imidazo[1,2-a]pyridin-6-yl)methyl)(cyclobutylmethyl)carbamate

Intermediate **1-c** (1.0 g, 3.164 mmol, 1.00 eq) and 1,3-dichloroacetone (537.97 mg, 4.025 mmol, 1.27 eq) were dissolved in 1,2-dimethoxyethane (10 mL), and the resulting mixture was stirred at 80°C for 1.5 h under nitrogen protection. The mixture was quenched with a saturated sodium bicarbonate solution and extracted with EtOAc (3 x 30 mL), and the combined organic layers were washed with saline (3 x 10 mL) and dried over anhydrous sodium sulfate. After filtration and concentration, the residue was purified by silica gel column chromatography and eluted with petroleum ether/EtOAc (1:1) to obtain a yellow solid intermediate **1-d** (420 mg, 32.39%).

LC-MS (ESI): m/z 363.8 [M+H]⁺.

### Intermediate I-1: Tert-butyl ((2-(azidomethyl)imidazo[1,2-a]pyridin-6-yl)methyl)(cyclobutylmethyl)carbamate

Sodium azide (500 mg, 7.307 mmol, 2.64 eq) was added to a solution of intermediate **1-d** (1.12 g, 2.770 mmol, 1 eq) and sodium iodide (60 mg, 0.380 mmol, 0.14 eq) in DMF (10 mL, 129.218 mmol, 46.65 eq), and the resulting mixture was stirred at 25°C for 2 h under nitrogen protection. The mixture was quenched with a saturated sodium bicarbonate solution and extracted with ethyl acetate (3 x 30 mL), and the combined organic layers were washed with saline (3 x 10 mL) and dried over anhydrous sodium sulfate. After filtration and concentration, the residue was purified by silica gel column chromatography and eluted with petroleum ether/EtOAc (3:1) to obtain a purple oily intermediate **I-1** (900 mg, 79.20%).

LC-MS (ESI): m/z 370.46 [M+H]⁺.

### Synthesis of intermediate I-2: 4-ethynyl-6-nitro-1-(tetrahydro-2H-pyran-2-yl)-1H-indazole

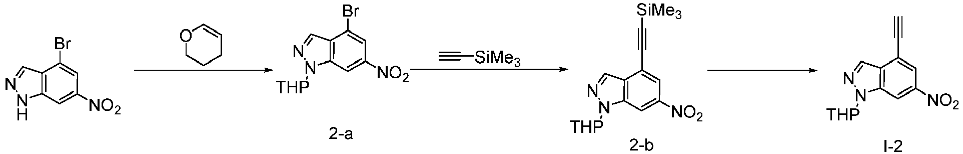

### Intermediate 2-a: 4-bromo-6-nitro-1-(tetrahydro-2H-pyran-2-yl)-1H-indazole

At 25°C, p-toluenesulfonic acid (78.26 mg, 0.432 mmol, 0.11 eq) and dihydropyran (1.10 mL, 12.442 mmol, 3.17 eq) were added dropwise to a solution of 4-bromo-6-nitro-1H-indazole (1 g, 3.925 mmol, 1.00 eq) in DCM (15 mL) while stirring. Under nitrogen protection, the resulting mixture was stirred at 25°C for 2 h. The mixture was quenched with a saturated sodium bicarbonate solution, and the combined organic layers were washed with DCM (2 x 30 mL) and dried over anhydrous sodium sulfate. After filtration and concentration, a yellow oily intermediate **2-a** (1 g, crude product) was obtained.

LC-MS (ESI): m/z 326.15 [M+H]⁺.

### Intermediate 2-b: 6-nitro-1-(tetrahydro-2H-pyran-2-yl)-4-((trimethylsilyl)ethynyl)-1H-indazole

Copper iodide (56.30 mg, 0.281 mmol, 0.10 eq) and bis(triphenylphosphine) palladium (II) chloride (207.51 mg, 0.281 mmol, 0.10 eq) were added into a solution of intermediate **2-a** (1 g, 2.809 mmol, 1.00 eq) and trimethylsilyl acetylene (468.78 mg, 4.534 mmol, 2.00 eq) in DMF (10 mL) while stirring, and the resulting mixture was stirred at 80°C for 3 h under nitrogen protection. The reaction solution was extracted with ethyl acetate (2 x 80 mL), and the combined organic layers were washed with saline (2 x 50 mL) and dried over anhydrous sodium sulfate. After filtration and concentration, the residue was purified by silica gel column chromatography and eluted with petroleum ether/EtOAc (2:1) to obtain a light yellow solid intermediate **2-b** (600 mg, 59%).

LC-MS (ESI): m/z 343.46 [M+H]⁺.

### Intermediate I-2: 4-ethynyl-6-nitro-1-(tetrahydro-2H-pyran-2-yl)-1H-indazole

Potassium carbonate (242 mg, 1.663 mmol, 1.00 eq) was added to a solution of intermediate **2-b** (600 mg, 1.660 mmol, 1 eq) in methanol (8 mL) at 25°C while stirring, and the resulting mixture was stirred at 25°C for 3 h under nitrogen protection. The reaction solution was concentrated, the residue was purified by silica gel column chromatography and eluted with petroleum ether/EtOAc (5:1) to obtain a light yellow solid intermediate **I-2** (390 mg, 84.55%).

LC-MS (ESI): m/z 271.28 [M+H]⁺.

### Synthesis of intermediate I-3: 6-bromo-4-ethynyl-1-(tetrahydro-2H-pyran-2-yl)-1H-indazole

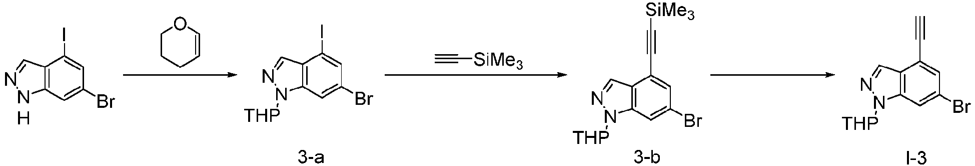

### Intermediate 3-a: 6-bromo-4-iodo-1-(tetrahydro-2H-pyran-2-yl)-1H-indazole

An off-white solid intermediate **3-a** (4.52 g, 93.00%) was prepared from 6-bromo-4-iodo-1H-indazole and 3,4-dihydro-2H-pyran using the method described in intermediate **2-a.**

LC-MS (ESI): m/z 407.05 [M+H]⁺.

### Intermediate 3-b: 6-bromo-1-(tetrahydro-2H-pyran-2-yl)-4-((trimethylsilyl)ethynyl)-1H-indazole

A brown syrup-like intermediate **3-b** (910 mg, 75.80%) was prepared from intermediate **3-a** using the method described in intermediate **2-b.**

LC-MS (ESI): m/z 377.1 [M+H]⁺.

### Intermediate I-3: 6-bromo-4-ethynyl-1-(tetrahydro-2H-pyran-2-yl)-1H-indazole

An off-white solid intermediate **I-3** (540 mg, 95.08%) was prepared from intermediate **3-b** using the method described in intermediate **1-3.**

LC-MS (ESI): m/z 305.0 [M+H]⁺.

### Synthesis of Intermediate I-4: (2-(azidomethyl)imidazo[1,2-a]pyridin-6-yl)methanol

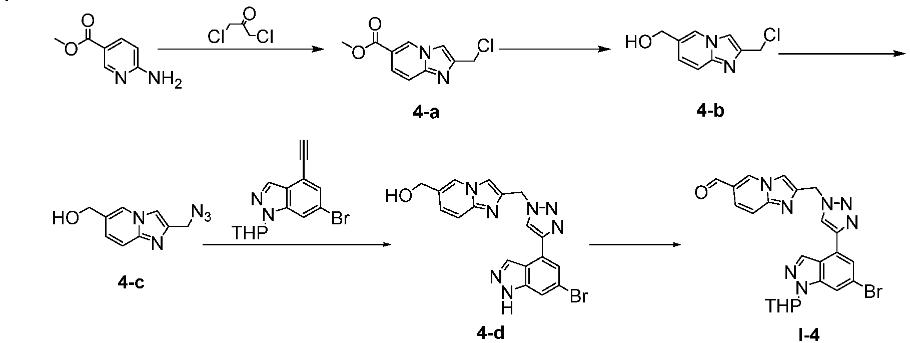

### Intermediate 4-a: Methyl 2-(chloromethyl)imidazo[1,2-a]pyridine-6-carboxylate

A yellow solid intermediate **4-a** (70.0 g, 52.7%) was prepared from methyl 6-aminonicotinate using the method described in intermediate **1-d.**

¹H NMR (400 MHz, CDCl₃): δ 8.89 - 8.91 (m, 1 H) 7.76 - 7.81 (m, 1 H) 7.71 - 7.74 (m, 1 H) 7.59 - 7.63 (m, 1 H) 4.77 - 4.82 (m, 2 H) 3.98 - 4.00 (m, 3 H).

### Intermediate 4-b: (2-(chloromethyl)imidazo[1,2-a]pyridin-6-yl)methanol

DIBAL-H (1.00 M, 445 mL, 2.50 eq) was added to a solution of methyl 2-(chloromethyl)imidazo[1,2-a]pyridine-6-carboxylate (40.0 g, 178 mmol, 1.00 eq) in THF (1.20 L) while stirring under nitrogen protection at 0°C, and the resulting mixture was stirred at 0°C for 2 h. The mixture was quenched with methanol (2 L) and extracted with DCM (4 L), and the combined organic layers were washed with saline (2 x 100 mL) and dried over anhydrous sodium sulfate. After filtration and concentration, the residue was purified with petroleum ether/EtOAc (3:1) using silica gel column chromatography to obtain a yellow solid intermediate **4-b** (10.0 g, 25.7%).

LC-MS (ESI): m/z 196.63 [M+H]⁺.

### Intermediate 4-c: (2-(azidomethyl)imidazo[1,2-a]pyridin-6-yl)methanol

A yellow solid intermediate **4-c** (540 mg, crude product) was prepared from intermediate **4-b** using the method described in intermediate **I-1.**

LC-MS (ESI): m/z 203.21 [M+H]⁺.

### Intermediate 4-d: (2-((4-(6-bromo-1H-indazole-4-yl)-1H-1,2,3-triazol-1-yl)methyl)imidazo[1,2-a]pyridine-6-yl)methanol

Copper (II) sulfate (63.26 mg, 0.396 mmol, 0.21 eq) and sodium ascorbate (435.16 mg, 2.076 mmol, 1.10 eq) were added to a solution of intermediate **4-c** (540 mg, 1.616 mmol, 1.00 eq) and (2-(azidomethyl)imidazo[1,2-a]pyridine-6-yl)methanol (439.47 mg, 2.076 mmol, 1.10 eq) in DMF (18 mL) and water (6 mL) while stirring. The resulting mixture was stirred at 25°C for 1 h under nitrogen protection. The reaction solution was diluted with saturated ammonium chloride (20 mL) and stirred for 35 min. The precipitate was collected by filtration, washed with water (2 x 50 mL), and concentrated to obtain a gray solid intermediate **4-d** (0.87 g, 99.05%).

LC-MS (ESI): m/z 423.3 [M+H]⁺.

### Intermediate 1-4: 2-((4-(6-bromo-1-(tetrahydro-2H-pyran-2-yl)-1H-indazol-4-yl)-1H-1,2,3-triazol-1-yl)methyl)imidazo[1,2-a]pyridine-6-carbaldehyde

A Dess-Martin reagent (1.61 g, 3.602 mmol, 2.25 eq) was added to intermediate 4-d (0.87 g, 1.601 mmol, 1.00 eq) in DCM (58 mL), and the resulting mixture was stirred at 25°C for 30 min. The mixture was diluted with 1 M sodium hydroxide (50 mL) and extracted with DCM (3 × 50 mL), and the combined organic layers were dried over anhydrous sodium sulfate. After filtration and concentration, the residue was purified by silica gel column chromatography and eluted with petroleum ether/EtOAc (1:1) to obtain a yellow solid intermediate **I-4** (720 mg, 84.54%).

LC-MS (ESI): m/z 506.3 [M+H]⁺.

### Synthesis of intermediate 1-5: Tert-butyl (cyclobutylmethyl)((2-(2-hydrazino-2-oxyethyl)imidazo[1,2-a]pyridine-6-yl)methyl)carbamate

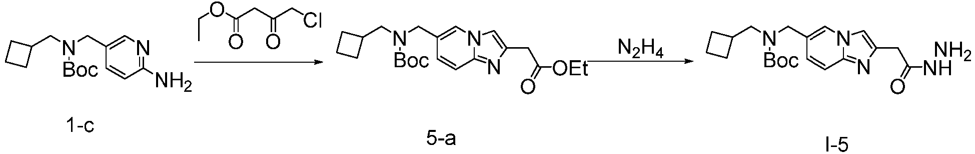

### Intermediate 5-a: Ethyl 2-(6-(((tert-butoxycarbonyl)(cyclobutylmethyl)amino)methyl)imidazo[1,2-a]pyridin-2-yl)acetate

Ethyl 4-chloro-3-oxo-butyrate (237.58 mg, 1.371 mmol, 1.00 eq) was added to a solution of intermediate **1-c** (400 mg, 1.371 mmol, 1 eq) in ethanol (4.44 mL) at 25°C while stirring, and the resulting mixture was stirred at 80°C for 3 h under nitrogen protection. The mixture was concentrated, and the residue was purified by reversed-phase rapid chromatography to obtain a light brown syrup-like intermediate **5-a** (210 mg, 31.56%).

LC-MS (ESI): m/z 402.3 [M+H]⁺.

### Intermediate 1-5: Tert-butyl (cyclobutylmethyl)((2-(2-hydrazino-2-oxoethyl)imidazo[1,2-a]pyridin-6-yl)methyl)carbamate

Hydrazine hydroxide (104.02 mg, 2.598 mmol, 6.00 eq) was added dropwise to a solution of intermediate **5-a** (210 mg, 0.433 mmol, 1 eq) in ethanol (4 mL) at 25°C while stirring, and the resulting mixture was stirred at 100°C for 12 h under nitrogen protection. The mixture was concentrated, and the residue was purified by reversed-phase rapid chromatography to obtain a light brown syrup-like intermediate **I-5** (170 mg, 94.94%).

LC-MS (ESI): m/z 388.4 [M+H]⁺.

### Intermediate 1-6: Synthesis of tert-butyl ((2-((4-(6-bromo-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

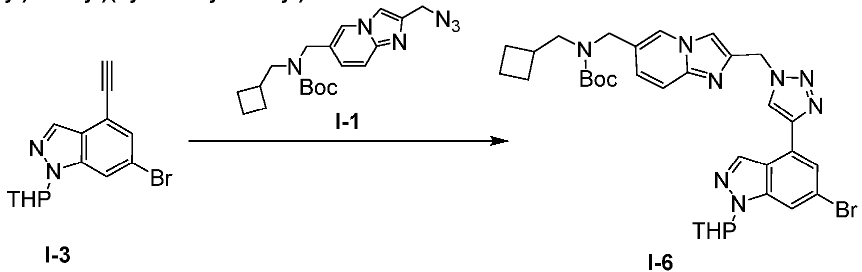

A yellow solid intermediate **I-6** (3.70 g, 83.6%) was prepared from intermediate **I-1** and intermediate **I-3** using the method described in intermediate **4-d.**

LC-MS (ESI): m/z 677.2 [M+H]⁺.

### Synthesis of intermediate I-7: Tert-butyl 6-(azidomethyl)-2-(((tert-butoxycarbonyl)(cyclobutylmethyl)amino)methyl)-1H-indole-1-carboxylate

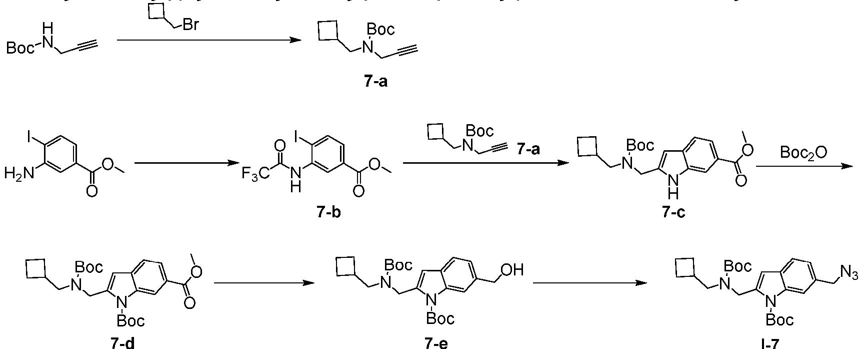

**Intermediate 7-a: Tert-butyl (cyclobutylmethyl)(propyl-2-alkynyl-1-yl)carbamate [00218]** At 0°C, sodium hydride (5.67 g, 141 mmol, 60% purity, 1.10 eq) was added in batches to a solution of tert-butyl propyl-2-alkynyl-1-yl carbamate (20.0 g, 129 mmol, 1.00 eq) in DMF (200 mL) while stirring. Then, (bromomethyl)cyclobutane (21.1 g, 141 mmol, 15.9 mL, 1.10 eq) was added dropwise at 0°C. The resulting mixture was stirred at 20°C for 16 h. The mixture was poured into ice water (1000 mL) and extracted with tert-butyl methyl ether (200 mL x 3), and the combined organic layers were washed with saline (200 mL) and dried over anhydrous sodium sulfate. After filtration and concentration, the residue was purified with silica gel column chromatography (petroleum ether/ethyl acetate=50/1 to 20/1) to obtain a yellow oily intermediate **7-a** (17.0 g, 59.1%).

¹H NMR (400 MHz, chloroform-*d*): *δ* 3.94 (s, 2H), 3.29 (d, *J* = 7.3 Hz, 2H), 2.51 (td, *J* = 7.7, 15.3 Hz, 1H), 2.11 (t, *J* = 2.4 Hz, 1H), 1.97 - 1.91 (m, 2H), 1.86 - 1.75 (m, 2H), 1.72 - 1.61 (m, 2H), 1.40 (s, 9H).

### Intermediate 7-b: Methyl 4-iodo-3-(2,2,2-trifluoroacetamide)benzoate

Under nitrogen protection of at 0°C, trifluoroacetic anhydride (56.8 g, 271 mmol, 37.6 mL, 3.00 eq) was added to a solution of methyl 3-amino-4-iodobenzoate (25.0 g, 90.2 mmol, 1.00 eq) in DCM (250 mL) while stirring, and the mixture was stirred at 25°C for 16 h under nitrogen protection. The pH of the mixture was adjusted to 8 with a saturated sodium bicarbonate solution at 0°C and then extracted with DCM (3 x 200 mL), and the combined organic layers were dried over anhydrous sodium sulfate, filtered and concentrated. The residue was purified by flash column chromatography to obtain a white solid intermediate **7-b** (30.0 g, 89.1%).

LC-MS (ESI): m/z 374.2 [M+H]⁺.

### Intermediate 7-c: Methyl 2-(((tert-butoxycarbonyl)(cyclobutylmethyl)amino)methyl)-1H-indole-6-carboxylate

Triphenylphosphine (422 mg, 1.61 mmol, 0.30 eq), cuprous iodide (153 mg, 804 µmol, 0.15 eq), and tripotassium phosphate (2 eq) were added to a solution of intermediate **7-b** (2.00 g, 5.36 mmol, 1.00 eq) and intermediate **7-a tert-butyl (cyclobutylmethyl)(propyl-2-alkynyl-1-yl)carbamate** (1.20 g, 5.36 mmol, 1.00 eq) in 1,4-dioxane (20 mL), and the mixture was stirred at 110°C for 16 h. The reaction solution was poured into water (100 mL) and extracted with EtOAc (20 mL x 3), and the combined organic layers were washed with saline (20 mL), dried over anhydrous sodium sulfate, filtered and concentrated. The residue was purified by silica gel column chromatography (petroleum ether/ethyl acetate=50/1 to 20/1) to obtain a yellow solid intermediate **7-c** (0.50 g, 25.0%).

LC-MS (ESI): m/z 373.3 [M+H]⁺.

### Intermediate 7-d: 1-(tert-butyl) 6-methyl 2-(((tert-butoxycarbonyl)(cyclobutylmethyl)amino)methyl)-1H-indole-1,6-dicarboxylate

A yellow solid intermediate **7-d** (0.55 g, 86.7%) was prepared from intermediate 7-c using the method described in intermediate **1-b.**

LC-MS (ESI): m/z 473.3 [M+H]⁺.

### Intermediate 7-e: Tert-butyl 2-(((tert-butoxycarbonyl)(cyclobutylmethyl)amino)methyl)-6-(hydroxymethyl)-1H-indole-1-carboxylate

A yellow oily intermediate **7-e** (0.50 g, 77.3%) was prepared from intermediate **7-d** using the method described in intermediate **4-b.**

LC-MS (ESI): m/z 444.3 [M+H]⁺.

### Intermediate I-7: Tert-butyl 6-(azidomethyl)-2-(((tert-butoxycarbonyl)(cyclobutylmethyl)amino)methyl)-1H-indole-1-carboxylate

At 20°C, DBU (54.8 mg, 360 µmol, 54.2 µL, 2.00 eq) was added to a solution of intermediate **7-e** (0.10 g, 180 µmol, 1.00 eq) in DMF (1.00 mL) while stirring. Then, the mixture was cooled to 0°C, and diphenylphosphoryl azide (DPPA) (99.0 mg, 360 µmol, 78.0 µL, 2.00 eq) was added at 0°C, and the mixture was stirred at 20°C for 16 h. The reaction mixture was poured into water (10 mL) and extracted with EtOAc (2 mL x 3), and the combined organic layers were washed with saline (5 mL x 1), dried over anhydrous sodium sulfate, filtered and concentrated. The residue was purified by preparative TLC (petroleum ether/ethyl acetate=5/1) to obtain yellow oily intermediate **I-7** (0.06 g, 71.0%).

LC-MS (ESI): m/z 469.5 [M+H]⁺ . ¹H NMR (400 MHz, chloroform-*d*): δ 8.03 (s, 1H), 7.39 (d, *J =* 7.6 Hz, 1H), 7.09 (s, 1H), 6.22 (d, *J =* 15.8 Hz, 1H), 4.66 (d, *J =* 16.6 Hz, 2H), 4.35 (br s, 2H), 3.31 (d, *J =* 11.1 Hz, 2H), 2.51 (s, 1H), 2.00 - 1.92 (m, 2H), 1.83 - 1.75 (m, 2H), 1.72 - 1.66 (m, 2H), 1.62 (s, 9H), 1.51 - 1.41 (m, 9H).

### Synthesis of intermediate I-8: Tert-butyl 6-((4-(6-bromo-1-(tetrahydro-2H-pyran-2-yl)-1H-indazol-4-yl)-1H-1,2,3-triazol-1-yl)methyl)-2-formyl-1H-indole-1-carboxylate

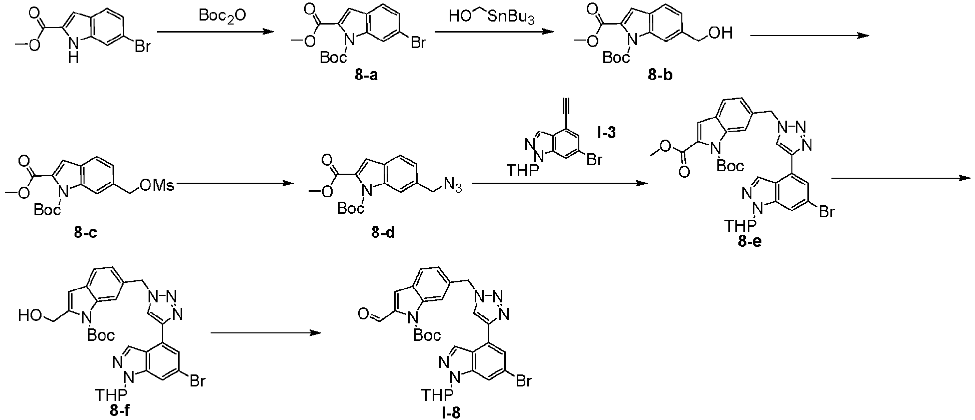

### Intermediate 8-a: 1-(tert-butyl)2-methyl 6-bromo-1H-indole-1,2-dicarboxylate

A colorless oily intermediate **8-a** (6.40 g, 91.8%) was prepared from methyl 6-bromo-1H-indole-2-carboxylate using the method described in intermediate **1-b.**

¹H NMR (400 MHz, chloroform-d): δ 8.23 (d, *J =* 0.6 Hz, 1H), 7.40 - 7.35 (m, 1H), 7.32 - 7.27 (m, 1H), 6.96 (s, 1H), 3.95 - 3.66 (m, 3H), 1.54 (s, 9H).

### Intermediate 8-b: 6-(hydroxymethyl)-1H-indol-1,2-dicarboxylic acid 1-(tert-butyl) 2-methyl ester

Under nitrogen protection, (tributyltin alkyl)methanol (9.07 g, 28.2 mmol, 2 eq) and tetrakis(triphenylphosphine)palladium (1.63 g, 1.41 mmol 0.1 eq) were added to a solution of intermediate **8-a** (5.00 g, 14.1 mmol, 1 eq) in 1,4-dioxane (50 mL), and the mixture was stirred at 100°C for 16 h under nitrogen protection. The mixture was concentrated, and the residue was purified by silica gel column chromatography (petroleum ether/EtOAc=20/1 to 0/1) to obtain a colorless oily intermediate **8-b** (3.60 g, 83.5%).

¹H NMR (400 MHz, DMSO-*d*₆): δ 8.02 (s, 1H), 7.67 (d, *J =* 8.1 Hz, 1H), 7.38 - 7.14 (m, 2H), 4.76 - 4.55 (m, 2H), 3.95 - 3.75 (m, 3H), 1.70 - 1.45 (m, 9H).

### Intermediate 8-c: 1-(tert-butyl)2-methyl 6-(((methylsulfonyl)oxy)methyl)-1H-indole-1,2-dicarboxylate

Under nitrogen protection at 0°C, methylsulfonyl chloride (3.21 g, 28.0 mmol, 2.17 mL, 2.38 eq) was added to a solution of 1-(tert-butyl)2-methyl 6-(hydroxymethyl)-1H-indole-1,2-dicarboxylate (3.60 g, 11.8 mmol, 1.00 eq) and triethylamine (5.97 g, 59.0 mmol, 8.21 mL, 5.00 eq) in DCM (36 mL) while stirring, and the mixture was stirred at 25°C for 3 h under nitrogen protection. The mixture was concentrated, the residue was diluted with ice water (30 mL) and extracted with DCM (40.0 mL x 2), and the combined organic layers were washed with saline (30 mL), dried over anhydrous sodium sulfate, filtered and concentrated. After filtration and concentration, a yellow solid intermediate **8-c** (4.50 g, crude product) was obtained.

LC-MS (ESI): m/z 383.4 [M+H]⁺.

### Intermediate 8-d: 6-(azidomethyl)-1H-indole-1,2-dicarboxylic acid 1-(tert-butyl) 2-methyl ester

At 25°C, sodium azide (1.53 g, 23.5 mmol, 2 eq) was added to a solution of intermediate **8-c** (4.50 g, 11.7 mmol, 1 eq) in DMF (45 mL) while stirring, and the resulting mixture was stirred at 25°C for 16 h. The mixture was diluted with saturated sodium bicarbonate solution (50.0 mL) and extracted with EtOAc (50.0 mL x 3). The organic layers were washed with saline (30 mL) and dried over anhydrous sodium sulfate. After filtration and concentration, a yellow oily intermediate **8-d** (6.70 g, crude product) was obtained.

¹H NMR (400 MHz, DMSO-*d*₆): δ 8.02 (s, 1H), 7.74 (d, *J =* 8.1 Hz, 1H), 7.35 - 7.30 (m, 1H), 7.29 (s, 1H), 4.64 - 4.48 (m, 2H), 3.88 (s, 3H), 1.57 (s, 9H).

### Intermediate 8-e: 1-(tert-butyl)2-methyl 6-((4-(6-bromo-1-(tetrahydro-2H-pyran-2-yl)-1H-indazol-4-yl)-1H-1,2,3-triazol-1-yl)methyl)-1H-indole-1,2-dicarboxylate

A yellow oily intermediate **8-e** (1.50 g, crude product) was prepared from intermediate **8-d** and intermediate **I-3** using the method described in intermediate **4-d.**

¹H NMR (400 MHz, DMSO-*d*₆): δ 9.04 (s, 1H), 8.64 (s, 1H), 8.01 (s, 1H), 7.97 (s, 1H), 7.82 (d, *J =* 1.4 Hz, 1H), 7.75 (d, *J =* 8.1 Hz, 1H), 7.37 (d, *J* = 1.3, 8.1 Hz, 1H), 7.29 (s, 1H), 5.92 (dd, *J =* 2.1, 9.6 Hz, 1H), 5.86 (s, 2H), 3.85 (s, 3H), 3.82 - 3.74 (m, 1H), 2.47 - 2.29 (m, 2H), 2.04 (d, *J* = 4.1 Hz, 1H), 1.96 (d, *J =* 2.8 Hz, 1H), 1.82 - 1.67 (m, 1H), 1.58 (d, *J =* 3.8 Hz, 2H), 1.51 (s, 9H).

### Intermediate 8-f: Tert-butyl 6-((4-(6-bromo-1-(tetrahydro-2H-pyran-2-yl)-1H-indazol-4-yl)-1H-1,2,3-triazol-1-yl)methyl)-2-(hydroxymethyl)-1H-indole-1-carboxylate

A white solid intermediate **8-f** (1.10 g, 76.7%) was prepared from intermediate **8-**e using the method described in intermediate **4-b.**

LC-MS (ESI): m/z 607.5 [M+H]⁺.

### Intermediate I-8: Tert-butyl 6-((4-(6-bromo-1-(tetrahydro-2H-pyran-2-yl)-1H-indazol-4-yl)-1H-1,2,3-triazol-1-yl)methyl)-2-formyl-1H-indole-1-carboxylate

A white solid intermediate **I-8** (0.700 g, 63.9%) was prepared from intermediate **8-f** using the method described in intermediate **I-4.**

LC-MS (ESI): m/z 605.48 [M+H]⁺.

### Synthesis of intermediate I-9: Tert-butyl ((2-((4-(6-amino-1-(tetrahydro-2H-pyran-2-yl)-1H-indazol-4-yl)-1H-1,2,3-triazol-1-yl)methyl)imidazo[1,2-a]pyridin-6-yl)methyl)((3-fluorobicyclo[1.1.1]pentan-1-yl)methyl)carbamate

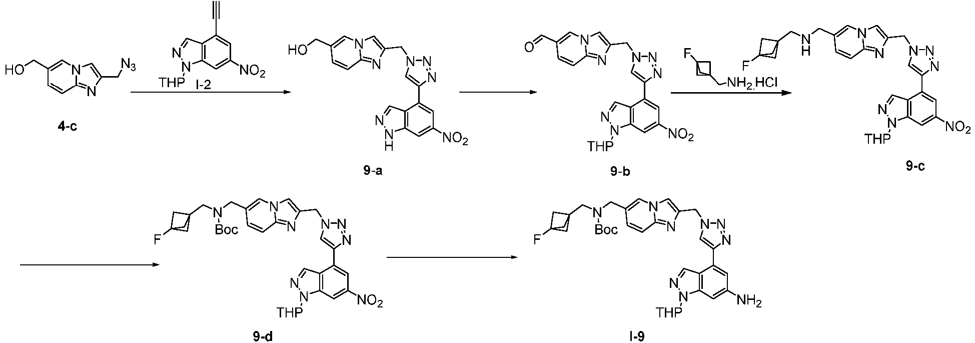

### Intermediate 9-a: (2-((4-(6-nitro-1H-indazole-4-yl)-1H-1,2,3-triazol-1-yl)methyl)imidazo[1,2-a]pyridine-6-yl)methanol

A white solid intermediate **9-a** (1.10 g, crude product) was prepared from intermediate **4-c** using the method described in intermediate **4-d.**

LC-MS (ESI): m/z 390.4 [M+H]⁺.

### Intermediate 9-b: 2-((4-(6-nitro-1-(tetrahydro-2H-pyran-2-yl)-1H-indazol-4-yl)-1H-1,2,3-triazol-1-yl)methyl)imidazo[1,2-a]pyridine-6-formaldehyde

A yellow oily intermediate **9-b** (0.90 g, crude product) was prepared from intermediate **9-a** using the method described in intermediate **I-4.**

LC-MS (ESI): m/z 472.5 [M+H]⁺.

### Intermediate 9-c: 1-(3-fluorobicyclo[1.1.1]pentan-1-yl)-N-((2-((4-(6-nitro-1-(tetrahydro-2H-pyran-2-yl)-1H-indazol-4-yl)-1H-1,2,3-triazol-1-yl)methyl)imidazo[1,2-a]pyridin-6-yl)methyl)methylamine

A yellow solid intermediate **9-c** (650 mg, 54,7%) was prepared from intermediate **9-b** and (3-fluorobicyclo[1.1.1]pentan-1-yl)methylamine using the method described in intermediate **E6-1.**

LC-MS (ESI): m/z 572.2 [M+H]⁺.

### Intermediate 9-d: Tert-butyl ((3-fluorobicyclo[1.1.1]pentan-1-yl)methyl)((2-((4-(6-nitro-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **9-d** (200 mg, 30.9%) was prepared from intermediate 9-c using the method described in intermediate **1-b.**

LC-MS (ESI): m/z 672.4 [M+H]⁺.

### Intermediate I-9: Tert-butyl ((2-((4-(6-amino-1-(tetrahydro-2H-pyran-2-yl)-1H-indazol-4-yl)-1H-1,2,3-triazol-1-yl)methyl)imidazo[1,2-a]pyridin-6-yl)methyl)((3-fluorobicyclo[1.1.1]pentan-1-yl)methyl)carbamate

A yellow solid intermediate **I-9** (220 mg, crude product) was prepared from intermediate **9-d** using the method described in intermediate **E3-2.**

LC-MS (ESI): m/z 642.5 [M+H]⁺.

### Synthesis of intermediate I-10: Tert-butyl ((2-(aminomethyl)imidazo[1,2-a]pyridin-6-yl)methyl)(cyclobutylmethyl)carbamate

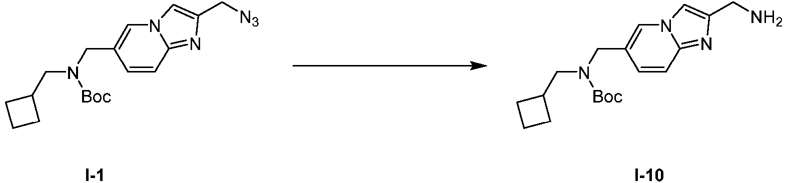

### Intermediate I-10: Tert-butyl ((2-(aminomethyl)imidazo[1,2-a]pyridin-6-yl)methyl)(cyclobutylmethyl)carbamate

At 20°C, triphenylphosphine (850 mg, 3.24 mmol, 3.00 eq) and water (195 mg, 10.8 mmol, 195 µL, 10 eq) were added to a solution of tert-butyl ((2-(azidomethyl)imidazo[1,2-a]pyridin-6-yl)methyl)(cyclobutylmethyl)carbamate (400 mg, 1.08 mmol, 1.00 eq) in THF (8.00 mL), and the mixture was stirred at 20°C for 16 h. The resulting mixture was concentrated, and the residue was purified by preparative TLC (petroleum ether/EtOAc=1/3) to obtain a yellow oily intermediate **I-10** (290 mg, 78.0%).

LC-MS (ESI): m/z 345.3 [M+H]⁺. ¹H NMR (400 MHz, chloroform-d): δ 7.83 - 8.02 (m, 1 H) 7.42 - 7.54 (m, 2 H) 7.01 - 7.14 (m, 1 H) 4.31 - 4.44 (m, 2 H) 3.99 - 4.09 (m, 2 H) 3.14 - 3.38 (m, 2 H) 2.44 - 2.60 (m, 1 H) 1.97 - 2.03 (m, 2 H) 1.80 - 1.89 (m, 2 H) 1.63 - 1.74 (m, 2 H) 1.44 - 1.55 (m, 9 H).

### 1.2 Preparation and activity test of compound corresponding to formula I

### Example 1: Synthesis of E-1: 1-cyclobutyl-N-((2-((4-(6-(methylthio)-1H-indazol-4-yl)-1H-1,2,3-triazol-1-yl)methyl)imidazo[1,2-a]pyridin-6-yl)methyl)methanamine

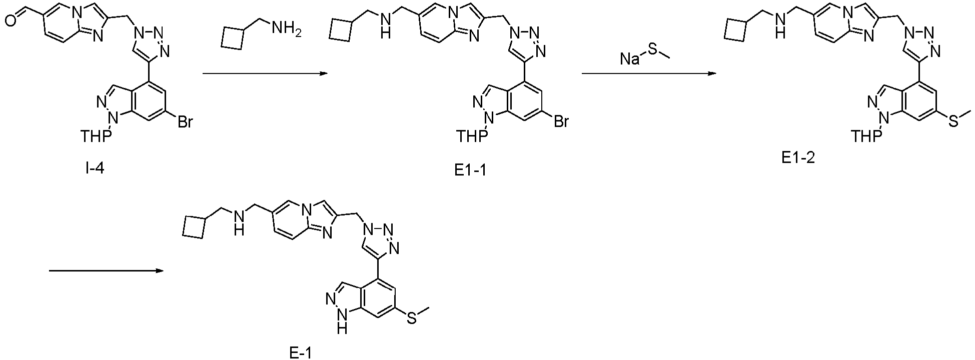

### E1-1: 1-(2-((4-(6-bromo-1-(tetrahydro-2H-pyran-2-yl)-1H-indazol-4-yl)-1H-1,2,3-triazol-1-yl)methyl)imidazo[1,2-a]pyridin-6-yl)-N-(cyclobutylmethyl)methylamine

At 25°C, 1-cyclobutylmethylamine (109.15 mg, 1.218 mmol, 2.00 eq) was added to a solution of intermediate **I-4** (324 mg, 0.609 mmol, 1.00 eq) in DCM (10 mL) under nitrogen protection while stirring. The resulting mixture was stirred at 50°C for 10 min. After cooling to room temperature, sodium triacetoxyborohydride (407.52 mg, 1.827 mmol, 3.00 eq) was added, and the resulting mixture was stirred at 50°C for 3 h under nitrogen protection. The reaction was quenched with a saturated sodium bicarbonate solution (100 mL), and then extraction was performed with DCM (3 × 100 mL). The combined organic layers were washed with saline (100 mL) and dried over anhydrous sodium sulfate. After filtration and concentration, the residue was purified by silica gel column chromatography and eluted with DCM/MeOH (5:1) to obtain a yellow solid intermediate **E1-1** (250 mg, 69.58%).

LC-MS: [M+H]⁺= 575.4.

### E1-2: 1-cyclobutyl-N-((2-((4-(6-(methylthio)-1-(tetrahydro-2H-pyran-2-yl)-1H-indazol-4-yl)-1H-1,2,3-triazol-1-yl)methyl)imidazo[1,2-a]pyridin-6-yl)methyl)methylamine

At 25°C, 4,5-bis(diphenylphosphino)-9,9-dimethylxanthene (35.60 mg, 0.058 mmol, 0.23 eq) and tris(dibenzylideneacetone)dipalladium (56.34 mg, 0.058 mmol, 0.23 eq) were added to a solution of intermediate **E1-1** (150 mg, 0.254 mmol, 1 eq) and (methylthioalkyl)sodium (56.24 mg, 0.762 mmol, 3.00 eq) in DMF (7.5 mL) under nitrogen protection while stirring. The resulting mixture was stirred at 180°C for 1 h under nitrogen protection. The mixture was concentrated, and the residue was purified by silica gel column chromatography and eluted with DCM/MeOH (1:1) to obtain a light brown solid intermediate **E1-2** (120 mg, 68.67%).

LC-MS: [M+H]⁺= 543.6.

### E-1: 1-cyclobutyl-N-((2-((4-(6-(methylthio)-1H-indazol-4-yl)-1H-1,2,3-triazol-1-yl)methyl)imidazo[1,2-a]pyridin-6-yl)methyl)methanamine

At 25°C, a 1,4-dioxane solution (1.20 mL, 4.8 mmol, 27 eq, 4 M) of hydrochloric acid was added to a solution of intermediate **E1-2** (120 mg, 0.175 mmol, 1 eq) in methanol (6 mL) under nitrogen protection while stirring. The resulting mixture was stirred at 25°C for 2 h under nitrogen protection. The mixture was concentrated, and the residue was purified by preparative HPLC to obtain a yellow solid compound **E-1** (50.9 mg, 56.39%).

LC-MS: [M+H]⁺= 459.2. ¹H NMR (300 MHz, DMSO-*d*₆): 9.78 (br s, 2H), 9.20-9.10 (m, 2H), 8.62 (s, 1H), 8.53 (s, 1H), 8.26 (d, *J =* 9.3 Hz, 1H), 8.03 (d, *J =* 9.3 Hz, 1H), 7.52 (s, 1H), 7.31 (s, 1H), 6.11 (s, 2H), 4.31-4.21 (m, 2H), 2.98-2.88 (m, 2H), 2.78-2.68 (m, 1H), 2.58 (s, 3H), 2.15-2.00 (m, 2H), 1.91-1.73 (m, 4H).

### Example 2: Synthesis of E-2: 1-cyclobutyl-N-((2-((4-(6-iodo-1H-indazol-4-yl)-1H-1,2,3-triazol-1-yl)methyl)imidazo[1,2-a]pyridin-6-yl)methyl)methylamine

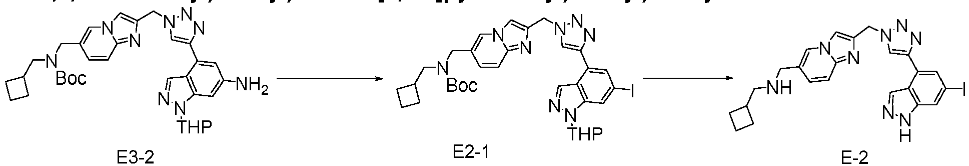

### E2-1: Tert-butyl (cyclobutylmethyl)((2-((4-(6-iodo-1-(tetrahydro-2H-pyran-2-yl)-1H-indazol-4-yl)-1H-1,2,3-triazol-1-yl)methyl)imidazo[1,2-a]pyridin-6-yl)methyl)carbamate

At 0°C, sulfuric acid (220 µL, 6.393 mmol, 20.69 eq) and sodium nitrite (35 mg, 0.482 mmol, 1.56 eq) were added to a solution of intermediate **E3-2** (200 mg, 0.309 mmol, 1 eq) in AcOH (4.00 mL) under argon protection while stirring. After stirring at 0°C for 1 h under argon protection, iodomethane (1 mL, 17.168 mmol, 55.57 eq) was added to the resulting mixture, and stirring was continued at 0°C for 2 h. The pH of the solution was adjusted to 8-9 with a saturated sodium bicarbonate solution, the mixture was diluted with water (70 mL) and extracted with ethyl acetate (3 × 30 mL), and the combined organic layers were washed with saline (3 × 20 mL), dried over anhydrous sodium sulfate, filtered and concentrated. The residue was purified by preparative TLC with petroleum ether/EtOAc (1:4) to obtain a red oily intermediate **E2-1** (310 mg, 87.06%).

LC-MS: [M+H]⁺= 722.6.

### E-2: 1-cyclobutyl-N-((2-((4-(6-iodo-1H-indazol-4-yl)-1H-1,2,3-triazol-1-yl)methyl)imidazo[1,2-a]pyridin-6-yl)methyl)methylamine

A light brown solid compound **E-2** (9.5 mg, 19.73%) was prepared from intermediate **E2-1** using the method described in compound **E-1.**

LC-MS: [M+H]⁺= 538.30. ¹H NMR (300 MHz, DMSO-*d*₆): δ 13.29 (s, 1H), 8.95 (s, 1H), 8.59 (s, 1H), 8.43 (s, 1H), 7.98 (s, 1H), 7.94-7.88 (m, 2H), 7.46 (d, *J* = 9.3 Hz, 1H), 7.26 (d, *J* = 9.3 Hz, 1H), 5.78 (s, 2H), 3.66 (s, 2H), 2.55-2.30 (m, 3H), 2.05-1.89 (m, 2H), 1.88-1.69 (m, 2H), 1.67-1.59 (m, 2H), 1.29-1.18 (m, 1H).

### Example 3: Synthesis of E-3: 4-(1-((6-(((cyclobutylmethyl)amino)methyl)imidazo[1,2-a]pyridin-2-yl)methyl)-1H-1,2,3-triazol-4-yl)-1H-indazole-6-amine

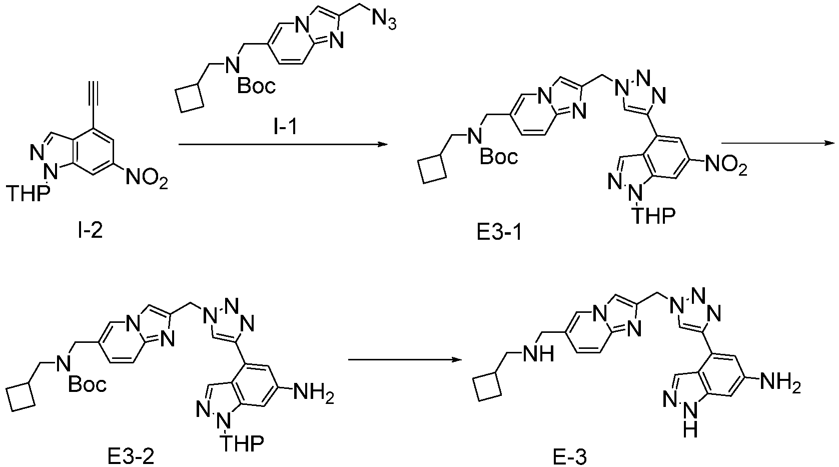

### E3-1: Tert-butyl (cyclobutylmethyl)((2-((4-(6-nitro-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **E3-1** (670 mg, 88.51%) was prepared from intermediate **I-2** using the method described in intermediate **4-d.**

LC-MS: [M+H]⁺= 641.7.

### E3-2: Tert-butyl ((2-((4-(6-amino-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

Ethanol (2 mL) and water (1 mL) were added to intermediate **E3-1** (200 mg, 0.285 mmol, 1 eq), ammonium chloride (83.35 mg, 1.480 mmol, 5.19 eq), and Fe (69.81 mg, 1.188 mmol, 4.16 eq) at 25°C, and the mixture was enabled to react at 80°C for 1 h under nitrogen protection. The mixture was concentrated, and the residue was dissolved in EtOAc (20 mL), diluted with EtOAc (30 mL), washed with saline (3 × 10 mL), and dried over anhydrous sodium sulfate. After filtration and concentration, a yellow solid intermediate **E3-2** (120 mg, crude product) was obtained.

LC-MS: [M+H]⁺= 611.8.

### E-3: 4-(1-((6-(((cyclobutylmethyl)amino)methyl)imidazo[1,2-a]pyridin-2-yl)methyl)-1H-1,2,3-triazol-4-yl)-1H-indazole-6-amine

A solution of HCl in 1,4-dioxane (3 mL, 12 mmol, 4 M) was added dropwise to a solution of intermediate **E3-2** (120 mg, 0.128 mmol, 1 eq) in DCM (2.5 mL) at 25°C while stirring, and the resulting mixture was enabled to react at 25°C for 1 h under nitrogen protection. The resulting mixture was concentrated, and the residue was purified by preparative HPLC to obtain a white solid compound **E-3** (41.8 mg, 52.06%).

LC-MS: [M+H]⁺= 427.20. ¹H NMR (300 MHz, DMSO-*d*₆): δ 12.41 (s, 1H), 8.67 (s, 1H), 8.42 (s, 1H), 8.20 (s, 1H), 7.95 (s, 1H), 7.46 (d, *J =* 9.3 Hz, 1H), 7.25 (d, *J =* 9.3, 1H), 7.06 (s, 1H), 6.51 (s, 1H), 5.76 (s, 2H), 5.31 (s, 2H), 3.65 (s, 2H), 2.55-2.18 (m, 3H), 205-1.50 (m, 6H).

### Example 4: Synthesis of E-4: 4-(1-((6-(((cyclobutylmethyl)amino)methyl)imidazo[1,2-a]pyridin-2-yl)methyl)-1H-1,2,3-triazol-4-yl)-N-methyl-1H-indazole-6-amine

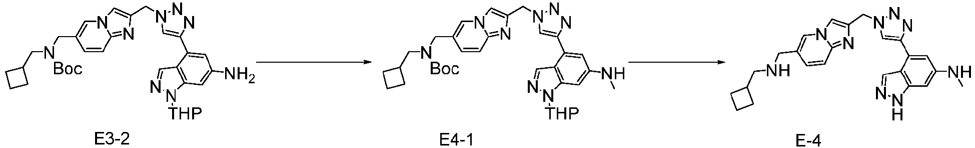

### E4-1: Tert-butyl (cyclobutylmethyl)((2-((4-(6-(methylamino)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

At 25°C, POM (12.27 mg, 0.123 mmol, 1.73 eq) was added to a solution of intermediate **E3-2** (50 mg, 0.071 mmol, 1 eq) and sodium methoxide (44.13 mg, 0.777 mmol, 10.95 eq) in MeOH (2 mL), and the resulting mixture was stirred at 80°C for 3 h under nitrogen protection. Then, sodium borohydride (6.18 mg, 0.155 mmol, 2.19 eq) was added in batches at 0°C under nitrogen protection, and the resulting mixture was stirred at 80°C for 16 h under nitrogen protection. The mixture was diluted with water (10 mL) and extracted with EtOAc (3 x 10 mL), and the combined organic layers were washed with saline (3 x 5 mL) and dried over anhydrous sodium sulfate. After filtration and concentration, a white solid intermediate **E4-1** (50 mg, 98.32%) was obtained.

LC-MS: [M+H]⁺ = 625.8.

### E-4: 4-(1-((6-(((cyclobutylmethyl)amino)methyl)imidazo[1,2-a]pyridin-2-yl)methyl)-1H-1,2,3-triazol-4-yl)-N-methyl-1H-indazole-6-amine

A light brown solid compound **E-4** (11.4 mg, 36.50%) was prepared from intermediate **E4-1** using the method described in compound **E-3.**

LC-MS: [M+H]⁺= 442.30. ¹H NMR (300 MHz, DMSO-*d*₆): δ 12.50 (s, 1H), 8.71 (s, 1H), 8.42 (s, 1H), 8.21 (s, 1H), 7.95 (s, 1H), 7.46 (d, *J =* 9.3 Hz, 1H), 7.25 (dd, *J* = 9.3 Hz, 1H), 7.08 (s, 1H), 6.30 (s, 1H), 6.02-5.92 (m, 1H), 5.76 (s, 2H), 3.65 (s, 2H), 2.78-2.70 (m, 3H), 2.55-2.39 (m, 3H), 2.05-1.55 (m, 6H).

### Example 5: Synthesis of E-5: 4-(1-((6-(((cyclobutylmethyl)amino)methyl)imidazo[1,2-a]pyridin-2-yl)methyl)-1H-1,2,3-triazol-4-yl)-N,n-dimethyl-1H-indazole-6-amine

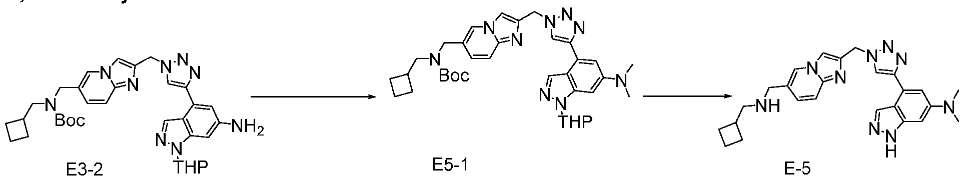

### E5-1: Tert-butyl (cyclobutylmethyl)((2-((4-(6-(dimethylamino)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

Formaldehyde (35.66 mg, 0.476 mmol, 2.05 eq) was added to a solution of intermediate **E3-2** (150 mg, 0.232 mmol, 1 eq) in DCM (5 mL) at 25°C while stirring, and the mixture was enabled to react at 25°C for 30 min under nitrogen protection. Sodium triacetoxyborohydride (19.47 mg, 0.295 mmol, 1.27 eq) was added in batches, and the resulting mixture was stirred at 25°C for 2 h under nitrogen protection. The mixture was concentrated, and the residue was purified by silica gel column chromatography and eluted with EtOAc/MeOH (10:1) to obtain a white solid intermediate **E5-1** (130 mg, 70%).

LC-MS: [M+H]⁺= 639.8.

### E-5: 4-(1-((6-(((cyclobutylmethyl)amino)methyl)imidazo[1,2-a]pyridin-2-yl)methyl)-1H-1,2,3-triazol-4-yl)-N,n-dimethyl-1H-indazole-6-amine

A white solid compound **E-5** (18.0 mg, 34.11%) was prepared from intermediate **E5-1** using the method described in compound **E-1.**

LC-MS: [M+H]⁺= 456.20. ¹H NMR (300 MHz, DMSO-*d*₆): δ 12.60 (s, 1H), 8.85 (s, 1H), 8.42 (s, 1H), 8.32 (s, 1H), 7.96 (s, 1H), 7.46 (d, J = 9.3 Hz, 1H), 7.30-7.20 (m, 2H), 6.54 (s, 1H), 5.77 (s, 2H), 3.64 (s, 2H), 3.00 (s, 6H), 2.55-2.20 (m, 3H), 2.04-1.55 (m, 6H).

### Example 6: Synthesis of E-6: 1-(2-((4-(6-(aziridin-1-yl)-1H-indazol-4-yl)-1H-1,2,3-triazol-1-yl)methyl)imidazo[1,2-a]pyridin-6-yl)-N-(cyclobutylmethyl)methanamine

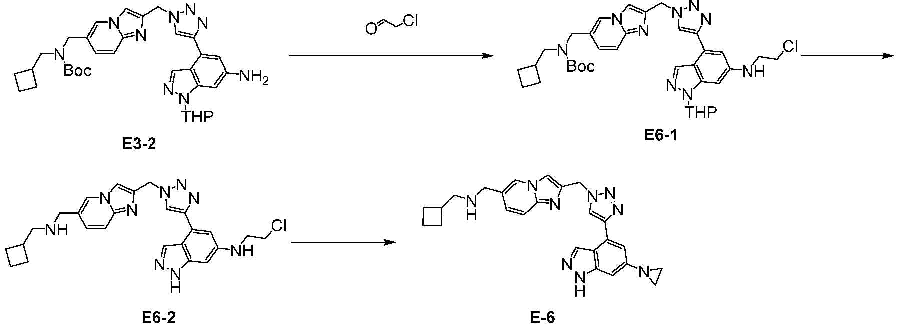

### E6-1: Tert-butyl ((2-((4-((6-((2-chloroethyl)amino)-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

Intermediate **E3-2** (0.50 g, 817 µmol, 1.00 eq), AcOH (226 mg, 81 µmol,1.00 eq), 2-chloroacetate (192 mg, 981 µmol, 158 µL, 1.20 eq) and sodium cyanoborohydride (128 mg, 2.04 mmol, 2.50 eq) were dissolved in MeOH (5.00 mL) at 25°C under nitrogen protection, and the resulting mixture was stirred at 25°C for 16 h. The mixture was diluted with water (5.00 mL) and extracted with EtOAc (3 x 2 mL). The combined organic layers were washed with saline (3.00 mL), dried over anhydrous sodium sulfate, filtered and concentrated. The residue was purified by silica gel column chromatography (petroleum ether/EtOAc (1:10)) to obtain a yellow solid intermediate **E6-1** (0.420 g, 76.2%).

LC-MS: [M+H]⁺= 674.5.

### E6-2: N-(2-chloroethyl)-4-(1-(6-(((cyclobutylmethyl)amino)methyl)imidazo[1,2-a]pyridin-2-yl)methyl)-1H-1,2,3-triazol-4-yl)-1H-indazole-6-amine

A yellow oily intermediate **E6-2** (0.05 g, crude product) was prepared from intermediate **E6-1** using the method described in compound **E-1.**

LC-MS: [M+H]⁺= 490.4.

### E-6: 1-(2-((4-(6-(aziridin-1-yl)-1H-indazol-4-yl)-1H-1,2,3-triazol-1-yl)methyl)imidazo[1,2-a]pyridin-6-yl)-N-(cyclobutylmethyl)methanamine

Sodium hydride (20.4 mg, 510 µmol, 60% purity, 5.00 eq) was added to a solution of intermediate **E6-2** (0.05 g, 102 µ mol, 1.00 eq) in DMF (1.00 mL) at 0°C, and the resulting mixture was stirred at 25°C for 2 h. The mixture was quenched with water at 0°C and concentrated. The residue was purified by preparative HPLC to obtain a white solid compound **E-6** (12.5 mg, 26.2%).

LC-MS: [M+H]⁺= 454.2. ¹H NMR (400 MHz, DMSO-*d*₆): δ 12.90 (s, 1H), 8.86 (s, 1H), 8.43 (s, 2H), 7.97 (s, 1H), 7.47 (d, *J* = 9.2 Hz, 1H), 7.39 (d, *J =* 1.7 Hz, 1H), 7.28 - 7.24 (m, 1H), 6.94 (s, 1H), 5.77 (s, 2H), 3.65 (s, 2H), 2.39 (t, *J =* 7.3, 15.0 Hz, 2H), 2.15 (s, 4H), 1.97 (d, *J* = 7.6 Hz, 3H), 1.86 - 1.73 (m, 2H), 1.67 - 1.59 (m, 2H).

### Example 7: Synthesis of E-7: 1-(2-((5-(6-chloro-1H-indazol-4-yl)-1,3,4-thiadiazol-2-yl)methyl)imidazo[1,2-a]pyridin-6-yl)-N-(cyclobutylmethyl)methylamine

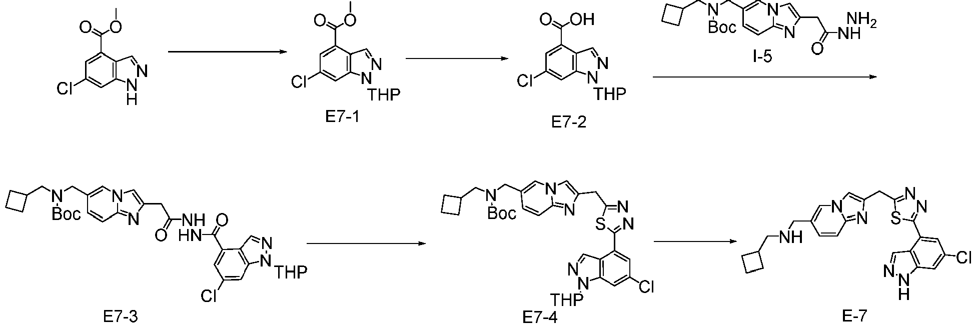

### E7-1: Methyl 6-chloro-1-(tetrahydro-2H-pyran-2-yl)-1H-indazole-4-carboxylate

A light yellow solid intermediate **E7-1** (1.52 g, 83.19%) was prepared from methyl 6-chloro-1H-indazole-4-carboxylate using the method described in intermediate **2-a**.

LC-MS: [M+H]⁺= 295.1.

### E7-2: 6-chloro-1-(tetrahydro-2H-pyran-2-yl)-1H-indazole-4-carboxylic acid

Lithium hydroxide (507.19 mg, 11.481 mmol, 3.06 eq) and water (15 mL) were added to a solution of intermediate **E7-1** (1.52 g, 3.752 mmol, 1 eq) in THF (15 mL) at 25°C, and the resulting mixture was stirred at 60°C for 3 h under nitrogen protection. The pH of the solution was adjusted to 3 with HCl (3 N), the mixture was diluted with water (50 mL) and extracted with EtOAc (3 x 100 mL). The combined organic layers were washed with saline (2 x 100 mL), dried over anhydrous sodium sulfate, filtered and concentrated. The residue was purified using Prep-TLC (petroleum ether/EtOAc=1:1) to obtain an off-white solid intermediate **E7-2** (1.0 g, 94%).

LC-MS: [M+H]⁺= 281.1.

### E7-3: Tert-butyl ((2-(2-(2-(6-chloro-1-(tetrahydro-2H-pyran-2-yl)-1H-indazole-4-carbonyl)hydrazino)-2-oxoethyl)imidazo[1,2-a]pyridin-6-yl)methyl)(cyclobutylmethyl)carbamate

Tert-butyl (cyclobutylmethyl)((2-(2-hydrazino-2-oxoethyl)imidazo[1,2-a]pyridin-6-yl)methyl)carbamate (150 mg, 0.363 mmol, 1 eq) and triethylamine (115.86 mg, 1.089 mmol, 3.00 eq) were added to a stirred solution of intermediate **E7-2** (128.56 mg, 0.436 mmol, 1.20 eq) and 1-[bis(dimethylamino)methylene]-1H-1,2,3-triazolo[4,5-b]pyridinium 3-oxide hexafluorophosphate (217.67 mg, 0.544 mmol, 1.50 eq) in DMF (6 mL) at 25°C under nitrogen protection, and the resulting mixture was stirred at 25°C for 16 h under nitrogen protection. The reaction solution was quenched with a saturated sodium bicarbonate solution (50 mL) and extracted with EtOAc (3 x 100 mL), and the combined organic layers were washed with saline (50 mL), dried over anhydrous sodium sulfate, filtered and concentrated. The residue was purified by silica gel column chromatography and eluted with DCM/MeOH (5:1) to obtain a brown syrup-like intermediate **E7-3** (140 mg, 46.83%).

LC-MS: [M+H]⁺= 650.4.

### E7-4: Tert-butyl ((2-((5-(6-chloro-1-(tetrahydro-2H-pyran-2-yl)-1H-indazol-4-yl)-1,3,4-thiadiazol-2-yl)methyl)imidazo[1,2-a]pyridin-6-yl)methyl)(cyclobutylmethyl)carbamate

A lawesson reagent (73.58 mg, 0.173 mmol, 1.50 eq) was added to a solution of intermediate **E7-3** (95 mg, 0.115 mmol, 1 eq) in toluene (2 mL) at 25°C, and the resulting mixture was stirred at 80°C for 5 h under nitrogen protection. The mixture was concentrated, and the residue was purified by reversed-phase chromatography to obtain a light brown solid intermediate **E7-4** (45 mg, 49.71%).

LC-MS: [M+H]⁺= 648.6.

### E-7: 1-(2-((5-(6-chloro-1H-indazol-4-yl)-1,3,4-thiadiazol-2-yl)methyl)imidazo[1,2-a]pyridin-6-yl)-N-(cyclobutylmethyl)methylamine

A light yellow solid compound **E-7** (7.5 mg, 38.78%) was prepared from intermediate **E7-4** using the method described in compound **E-1.**

LC-MS: [M+H]⁺= 464.1. ¹H NMR (400 MHz, methanol-d₄): 9.03 (s, 1H), 8.63 (s, 1H), 8.39 (s, 1H), 8.13 (d, *J* = 9.2 Hz, 1H), 8.00 (d, *J* = 9.3 Hz, 1H), 7.79 (s, 1H), 7.72 (s, 1H), 4.99 (s, 2H), 4.42 (s, 2H), 3.22-3.16 (m, 2H), 2.81-2.71 (m, 1H), 2.21-2.11 (m, 2H), 1.98-1.85 (m, 4H).

### Example 8: Synthesis of E-8: 1-(2-((5-(6-bromo-1H-indazol-4-yl)-1,3,4-thiadiazol-2-yl)methyl)imidazo[1,2-a]pyridin-6-yl)-N-(cyclobutylmethyl)methylamine

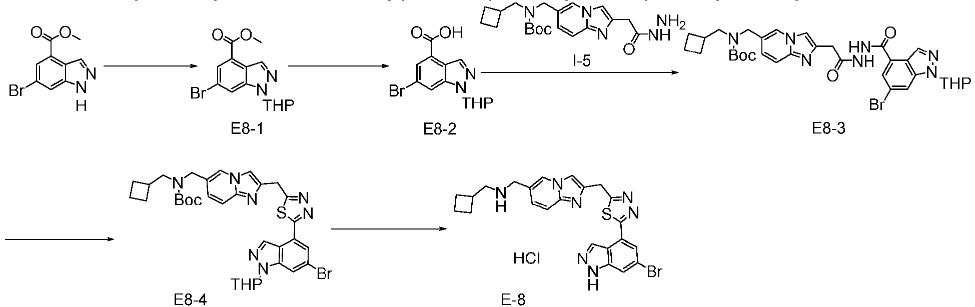

### E8-1: Methyl 6-bromo-1-(tetrahydro-2H-pyran-2-yl)-1H-indazole-4-carboxylate

A white solid intermediate **E8-1** (1.5 g, 72%) was prepared from methyl 6-bromo-1H-indazole-4-carboxylate using the method described in intermediate **2-a.**

LC-MS: [M+H]⁺= 339.2.

### E8-2: 6-bromo-1-(tetrahydro-2H-pyran-2-yl)-1H-indazole-4-carboxylic acid

A white solid intermediate **E8-2** (1.25 g, 96%) was prepared from intermediate **E8-1** using the method described in intermediate **E7-2.**

LC-MS: [M+H]⁺= 325.2.

### E8-3: Tert-butyl ((2-(2-(2-(6-bromo-1-(tetrahydro-2H-pyran-2-yl)-1H-indazole-4-carbonyl)hydrazino)-2-oxoethyl)imidazo[1,2-a]pyridin-6-yl)methyl)(cyclobutylmethyl)carbamate

A light yellow solid intermediate **E8-3** (170 mg, 33.22%) was prepared from intermediate **E8-2** using the method described in intermediate **E7-3.**

LC-MS: [M+H]⁺= 694.5.

### E8-4: Tert-butyl ((2-((5-(6-bromo-1-(tetrahydro-2H-pyran-2-yl)-1H-indazol-4-yl)-1,3,4-thiadiazol-2-yl)methyl)imidazo[1,2-a]pyridin-6-yl)methyl)(cyclobutylmethyl)carbamate

A light brown syrupy-like intermediate **E8-4** (50 mg, 44.19%) was prepared from intermediate **E8-3** using the method described in intermediate **E7-4.**

LC-MS: [M+H]⁺= 692.4.

### E-8: 1-(2-((5-(6-bromo-1H-indazol-4-yl)-1,3,4-thiadiazol-2-yl)methyl)imidazo[1,2-a]pyridin-6-yl)-N-(cyclobutylmethyl)methylamine

An off-white solid compound **E-8** (19.0 mg, 51.53%) was prepared from intermediate **E8-4** using the method described in compound **E-1.**

LC-MS: [M+H]⁺= 508.1. ¹H NMR (300 MHz, DMSO-d₆) 9.77 (s, 1H), 9.13 (s, 1H), 8.60-8.49 (m, 2H), 8.27 (d, *J =* 9.4 Hz, 1H), 8.10-8.01 (m, 2H), 7.82 (s, 1H), 5.02 (s, 2H), 4.27 (s, 2H), 3.01-2.90 (m, 2H), 2.2.80-2.68 (m, 1H), 2.10-1.95 (m, 2H), 1.96-1.75 (m, 4H).

### Example 9: Synthesis of E-9: 1-cyclobutyl-N-((2-((5-(6-methoxy-1H-indazol-4-yl)-1,3,4-thiadiazol-2-yl)methyl)imidazo[1,2-a]pyridin-6-yl)methyl)methylamine

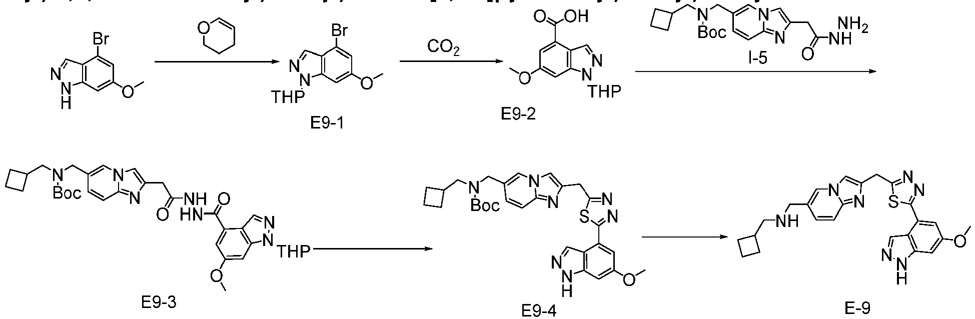

### E9-1: 4-bromo-6-methoxy-1-(tetrahydro-2H-pyran-2-yl)-1H-indazole

A yellow solid intermediate **E9-1** (740 mg, 44.21%) was prepared from 4-bromo-6-methoxy-1H-indazole using the method described in intermediate **2-a.**

LC-MS: [M+H]⁺= 311.2.

### E9-2: 6-methoxy-1-(tetrahydro-2H-pyran-2-yl)-1H-indazole-4-carboxylic acid

At -78°C, n-butyl lithium (0.86 mL, 2.151 mmol, 1.1 eq) was added dropwise to a solution of intermediate **E9-1** (620 mg, 1.955 mmol, 1 eq) in THF (10 mL) under nitrogen protection, and the mixture was stirred at -78°C for 30 min under nitrogen protection. Then, dry carbon dioxide gas was introduced within 10 min, and the resulting mixture was stirred at -78°C for 30 min, followed by stirring at 25°C for 16 h under nitrogen protection. The mixture was concentrated, and the residue was precipitated by addition of diethyl ether to obtain an off-white solid intermediate **E9-2** (220 mg, 35%).

LC-MS: [M+H]⁺= 277.2.

### E9-3: Tert-butyl (cyclobutylmethyl)((2-(2-(2-(6-methoxy-1-(tetrahydro-2H-pyran-2-yl)-1H-indazole-4-carbonyl)hydrazino)-2-oxyethyl)imidazo[1,2-a]pyridin-6-yl)methyl)carbamate

A light yellow syrup-like intermediate **E9-3** (140 mg, 72.76%) was prepared from intermediate **E9-2** using the method described in intermediate **E7-3.**

LC-MS: [M+H]⁺= 646.4.

### E9-4: Tert-butyl (2-((5-(6-methoxy-1H-indazol-4-yl)-1,3,4-thiadiazol-2-yl)methyl)imidazo[1,2-a]pyridin-6-yl)methyl)carbamate

An intermediate **E9-4** (70 mg, 61%) was prepared from intermediate **E9-3** using the method described in intermediate **E7-4.**

LC-MS: [M+H]⁺= 560.4.

### E-9: 1-cyclobutyl-N-((2-((5-(6-methoxy-1H-indazol-4-yl)-1,3,4-thiadiazol-2-yl)methyl)imidazo[1,2-a]pyridin-6-yl)methyl)methylamine

A light yellow solid compound **E-9** (12.7 mg, 35.38%) was prepared from intermediate **E9-4** using the method described in compound **E-1.**

LC-MS: [M+H]⁺= 460.1. ¹H NMR (300 MHz, methanol-d₄) δ 9.08 (s, 1H), 8.71 (s, 1H), 8.41 (s, 1H), 8.18 (d, *J =* 9.1 Hz, 1H), 8.00 (d, *J =* 9.1 Hz, 1H), 7.37 (s, 1H), 7.19 (s, 1H), 4.97 (s, 2H), 4.43 (s, 2H), 3.95 (s, 3H), 3.25-3.15 (m, 2H), 2.90-2.70 (m, 1H), 2.30-2.10 (m, 2H), 2.09-1.91 (m, 4H).

### Example 10: Synthesis of E-10: 1-cyclobutyl-N-((2-((4-(6-(hexoxy)-1H-indazol-4-yl)-1H-1,2,3-triazol-1-yl)methyl)imidazo[1,2-a]pyridin-6-yl)methyl)methylamine hydrochloride

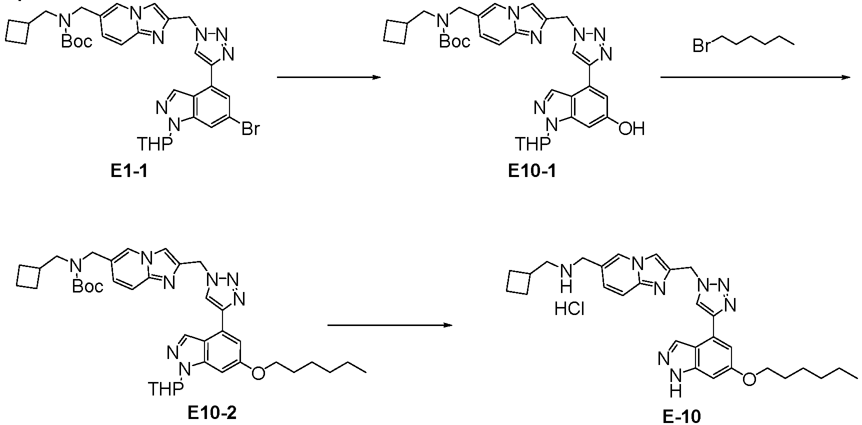

### E10-1: Tert-butyl (cyclobutylmethyl)((2-((4-(6-hydroxy-1-(tetrahydro-2H-pyran-2-yl)-1H-indazol-4-yl)-1H-1,2,3-triazol-1-yl)methyl)imidazo[1,2-a]pyridin-6-yl)methyl)carbamate

At 25°C, potassium hydroxide (166 mg, 2.96 mmol, 4.00 eq), 2-di-tert-butylphosphino-2',4',6'-triisopropyl biphenyl (31.4 mg, 74.0 µmol, 0.10 eq) and tris(dibenzylideneacetone)dipalladium (33.9 mg, 37.0 µmol, 0.05 eq) were added to a solution of intermediate **E1-1** (500 mg, 740 µmol, 1.00 eq) in water (1.00 mL) and dioxane (5.00 mL) while stirring, and the resulting mixture was stirred at 100°C for 16 h under nitrogen protection. The mixture was diluted with water (20 mL) and extracted with tert-butyl methyl ether (3 × 20 mL), and the combined organic layers were washed with saline (20 mL), dried with anhydrous sodium sulfate, filtered, and concentrated. The residue was purified by preparative TLC using petroleum ether/EtOAc (0:1) to obtain a red oily intermediate **E10-1** (40 mg, 88.2%).

LC-MS: [M+H]⁺= 613.5.

### E10-2: Tert-butyl (cyclobutylmethyl)((2-((4-(6-(hexoxy)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

Potassium iodide (2.71 mg, 16.3 µmol, 0.1 eq) and potassium carbonate (45.1 mg, 326 µmol, 2 eq) were added to a solution of intermediate **E10-1** (100 mg, 163 µmol, 1 eq) and 1-bromohexane (40.4 mg, 245 µmol, 34.2 µL, 1.5 eq) in DMF (2 mL), and the resulting mixture was stirred at 80°C for 16 h under nitrogen protection. The mixture was quenched with water (10 mL) and extracted with EtOAc (2 x 20 mL). The combined organic layers were dried with anhydrous sodium sulfate, filtered and concentrated to obtain a white solid intermediate **E10-2** (100 mg, crude product).

LC-MS: [M+H]⁺= 697.5.

### E-10: 1-cyclobutyl-N-((2-((4-(6-(hexoxy)-1H-indazol-4-yl)-1H-1,2,3-triazol-1-yl)methyl)imidazo[1,2-a]pyridin-6-yl)methyl)methylamine hydrochloride

A white solid compound **E-10** (34.0 mg, 45.8%) was prepared from intermediate **E10-2** using the method described in compound **E-1.**

LC-MS: [M+H]⁺= 549.2. ¹H NMR (400 MHz, DMSO-*d*₆): δ 9.56 (brs, 2 H) 9.03 (s, 2 H) 8.51 (s, 1 H) 8.45 (s, 1 H) 8.04 - 8.14 (m, 1 H) 7.96 (d, *J* = 9.29 Hz, 1 H) 7.25 (d, *J =* 1.83 Hz, 1 H) 6.92 (d, *J =* 0.73 Hz, 1 H) 6.03 (s, 2 H) 4.23 (t, *J =* 4.77 Hz, 2 H) 4.05 (t, *J =* 6.42 Hz, 2 H) 2.89 - 2.99 (m, 2 H) 2.64 - 2.74 (m, 1 H) 1.98 - 2.10 (m, 2 H) 1.70 - 1.84 (m, 6 H) 1.40 - 1.50 (m, 2 H) 1.28 - 1.35 (m, 4 H) 0.84 - 0.91 (m, 3 H)

### Example 11: Synthesis of E-11: 1-cyclobutyl-N-((2-((4-(6-(2-(2-methoxyethoxy)ethoxy)-1H-indazol-4-yl)-1H-1,2,3-triazol-1-yl)methyl)imidazo[1,2-a]pyridin-6-yl)methyl)methylamine

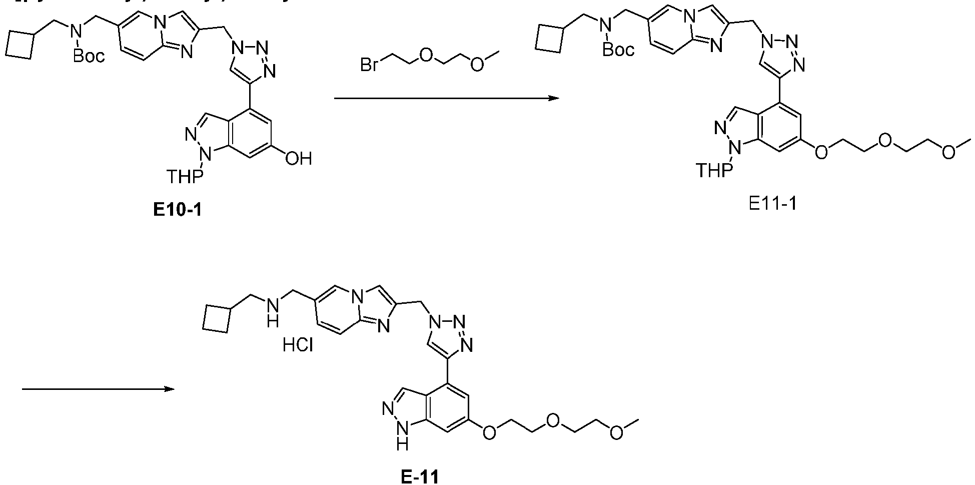

### E11-1: 1-cyclobutyl-N-((2-((4-(6-(hexoxy)-1H-indazol-4-yl)-1H-1,2,3-triazol-1-yl)methyl)imidazo[1,2-a]pyridin-6-yl)methyl)methylamine hydrochloride

A white solid intermediate **E11-1** (100 mg, crude product) was prepared from intermediate **E10-1** using the method described in intermediate **E10-2.**

LC-MS: [M+H]⁺= 715.6.

### E-11: 1-cyclobutyl-N-((2-((4-(6-(2-(2-methoxyethoxy)ethoxy)-1H-indazol-4-yl)-1H-1,2,3-triazol-1-yl)methyl)imidazo[1,2-a]pyridin-6-yl)methyl)methylamine

A yellow solid compound **E-11** (37.4 mg, 49.8%) was prepared from intermediate **E11-1** using the method described in compound **E-1.**

LC-MS: [M+H]⁺= 531.2. ¹H NMR (400 MHz, methanol-d₄): δ 9.02 (s, 1 H) 8.75 (s, 1 H) 8.56 (s, 1 H) 8.48 (s, 1 H) 8.12 (d, *J =* 9.41 Hz, 1 H) 7.99 (d, *J* = 9.29 Hz, 1 H) 7.36 (d, *J* = 1.96 Hz, 1 H) 7.03 (s, 1 H) 6.11 (s, 2 H) 4.40 (s, 2 H) 4.22 - 4.28 (m, 2 H) 3.87 - 3.94 (m, 2 H) 3.69 - 3.75 (m, 2 H) 3.55 - 3.60 (m, 2 H) 3.36 (s, 3 H) 3.18 (d, *J* = 7.46 Hz, 2 H) 2.74 (d, *J* = 15.19, 7.50 Hz, 1 H) 2.15 - 2.25 (m, 2 H) 1.85 - 2.03 (m, 4 H)

### Example 12: Synthesis of E-12: N-(1-(6-(((cyclobutylmethyl)amino)methyl)imidazo[1,2-a]pyridin-2-yl)ethyl)-5-(dimethylamino)nicotinamide

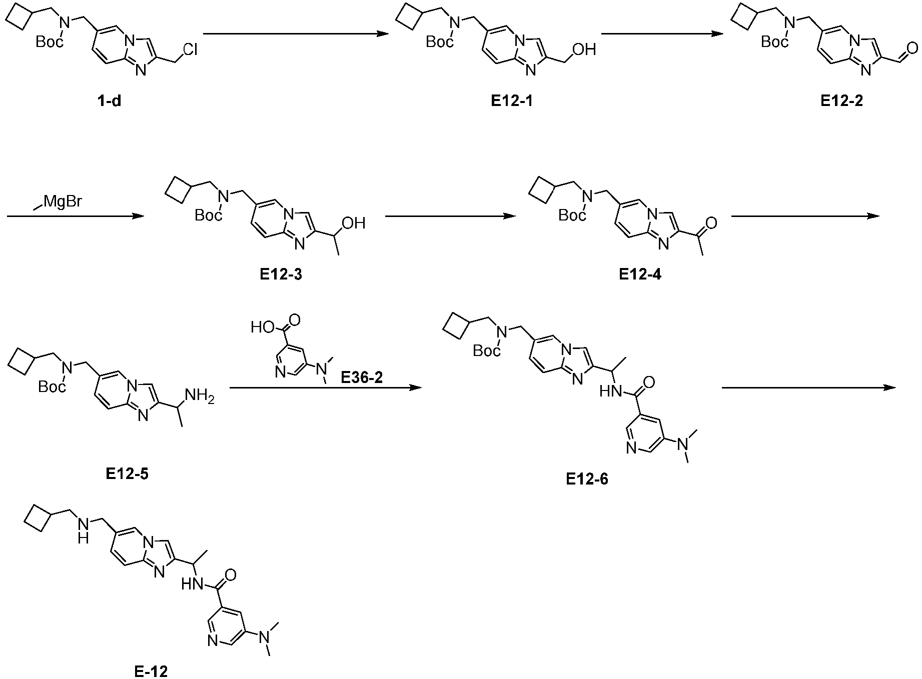

### E12-1: Tert-butyl (cyclobutylmethyl)((2-(hydroxymethyl)imidazo[1,2-a]pyridin-6-yl)methyl)carbamate

Sodium bicarbonate (1.39 g, 16.5 mmol, 641 µL, 3.00 eq) was added to a mixed solution of **1-d** (2.00 g, 5.50 mmol, 1.00 eq) in DMSO (20.0 mL) and water (20.0 mL), and the resulting mixture was stirred at 120°C for 1 h. The reaction mixture was cooled to 20°C, diluted with water (100 mL), and extracted with DCM (30.0 mL × 3). The combined organic layers were dried over anhydrous sodium sulfate, filtered, and concentrated. The residue was purified by silica gel column chromatography (ethyl acetate: methanol=50/1 to 10/1) to obtain a yellow solid **E12-1** (1.20 g, 63.2%).

LC-MS: [M+H]⁺= 346.2.

### E12-2: Tert-butyl (cyclobutylmethyl)((2-formylimidazo[1,2-a]pyridin-6-yl)methyl)carbamate

Yellow solid **E12-2** (940 mg, 78.8%) was prepared from **E12-1** using the method described in **E54-2.**

¹H NMR: (400 MHz, CDCl₃): *δ* 10.14 (s, 1H), 8.11 (s, 1H), 8.00 (d, *J =* 1.3 Hz, 1H), 7.64 (d, *J* = 9.4 Hz, 1H), 7.21 (d, *J =* 8.3 Hz, 1H), 4.40 (s, 2H), 3.26 (br s, 2H), 2.61-2.43 (m, 1H), 2.03-1.94 (m, 2H), 1.91-1.81 (m, 2H), 1.75-1.67 (m, 2H), 1.49 (s, 9H).

### E12-3: Tert-butyl (cyclobutylmethyl)((2-(1-hydroxyethyl)imidazo[1,2-a]pyridin-6-yl)methyl)carbamate

Methyl magnesium bromide (3 M, 1.82 mL, 2.00 eq) was added dropwise to a solution of **E12-2** in THF (20.0 mL) at -60°C under nitrogen protection, and the resulting mixture was stirred at 20°C for 15 h. The mixture was quenched with a saturated ammonium chloride solution (5.00 mL) at 0°C, diluted with water (20.0 mL) and extracted with DCM (10.0 mL x 3). The combined organic layers were dried over anhydrous sodium sulfate, filtered and concentrated. The residue was purified by silica gel column chromatography (ethyl acetate/methanol=50/1 to 10/1) to obtain yellow solid **E12-3** (0.75 g, 76.2%).

LC-MS: [M+H]⁺= 360.2.

### E12-4: Tert-butyl ((2-acetylimidazo[1,2-a]pyridin-6-yl)methyl)(cyclobutylmethyl)carbamate

Brown solid **E12-4** (200 mg, crude product) was prepared from **E12-3** using the method described in **E54-2.**

LC-MS: [M+H]⁺= 358.2.

### E12-5: Tert-butyl (2-(1-aminoethyl)imidazo[1,2-a]pyridin-6-yl)methyl)(cyclobutylmethyl)carbamate

NH₄OAc (431mg, 5.60 mmol, 10.0 eq) was added to a solution of **E12-4** (0.20 g, 560 µmol, 1.00 eq) in MeOH (6.00 mL), and the mixture was stirred at 30°C for 0.5 h. Then, NaBH₃CN (70.3 mg, 1.12 mmol, 2.00 eq) was added and stirred at 30°C for 15.5 h. The reaction mixture was concentrated, and the residue was diluted with water and extracted with ethyl acetate (5.00 mL × 3). The organic layers were washed with saline (5.00 mL), dried over anhydrous sodium sulfate, filtered and concentrated to obtain yellow solid **E12-5** (0.20 g, crude product).

LC-MS: [M+H]⁺= 359.2.

### E12-6: Tert-butyl (cyclobutylmethyl)((2-(1-(5-(dimethylamino)nicotinamido)ethyl)imidazo[1,2-a]pyridin-6-yl)methyl)carbamate

Yellow solid **E12-6** (30 mg, 42.5%) was prepared from **E12-5** using the method described in **E36-2.**

LC-MS: [M+H]⁺= 507.

### E-12: N-(1-(6-(((cyclobutylmethyl)amino)methyl)imidazo[1,2-a]pyridin-2-yl)ethyl)-5-(dimethylamino)nicotinamide hydrochloride

Yellow solid **E-12** (11.0 mg, 40.8%) was prepared from **E12-6** using the method described in **E-1.**

LC-MS: [M+H]⁺= 407.2. ¹H NMR (400 MHz, DMSO-*d*₆): *δ* 9.75 (d, *J =* 7.4 Hz, 1H), 9.53 (br s, 2H), 8.99 (s, 1H), 8.53 (s, 1H), 8.45 (s, 1H), 8.31 (d, *J* = 2.9 Hz, 1H), 8.12 (d, *J* = 9.5 Hz, 1H), 8.07 (br s, 1H), 7.99 (d, *J* = 9.3 Hz, 1H), 5.48 (t, *J* = 7.0 Hz, 1H), 4.26 (t, *J* = 5.0 Hz, 2H), 3.10 (s, 6H), 2.98-2.93 (m, 2H), 2.73-2.67 (m, 1H), 2.54 (s, 1H), 2.08-2.00 (m, 2H), 1.88-1.76 (m, 4H), 1.70 (d, *J =* 7.0 Hz, 3H).

### Example 13: Synthesis of E-13: 1-cyclobutyl-N-((2-((4-(6-(methylsulfonyl)-1H-indazol-4-yl)-1H-1,2,3-triazol-1-yl)methyl)imidazo[1,2-a]pyridin-6-yl)methyl)methylamine

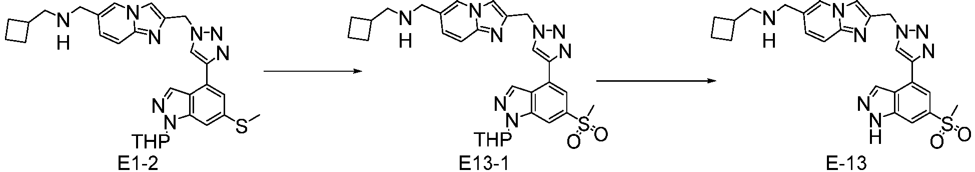

### E13-1: 1-cyclobutyl-N-((2-((4-(6-(methylsulfonyl)-1-(tetrahydro-2H-pyran-2-yl)-1H-indazol-4-yl)-1H-1,2,3-triazol-1-yl)methyl)imidazo[1,2-a]pyridin-6-yl)methyl)methylamine

Intermediate **E1-2** (100 mg, 0.097 mmol, 1 eq) and m-CPBA (53 mg, 0.291 mmol, 3 eq) were dissolved in DCM (2 mL) and stirred at 25°C for 1 h under nitrogen protection. The mixture was diluted with water (10 mL) and extracted with DCM (3 x 20 mL). The combined organic layers were washed with saline, dried over anhydrous sodium sulfate, filtered, and concentrated. The residue was purified by silica gel column chromatography and eluted with DCM/MeOH (8:1) to obtain a yellow solid intermediate **E13-1** (45 mg, 10%).

LC-MS: [M+H]⁺= 575.35.

### E-13: 1-cyclobutyl-N-((2-((4-(6-(methylsulfonyl)-1H-indazol-4-yl)-1H-1,2,3-triazol-1-yl)methyl)imidazo[1,2-a]pyridin-6-yl)methyl)methylamine

A white solid compound **E-13** (3.7 mg, 69%) was prepared from intermediate **E13-1** using the method described in compound **E-1.**

LC-MS: [M+H]⁺= 491.2. ¹H NMR (400 MHz, methanol-d₄): *δ* 9.15-8.95 (m, 2H), 8.75 (s, 1H), 8.54 (s, 1H), 8.22-8.10 (m, 2H), 8.05-7.95 (m, 1H), 6.18 (s, 2H), 4.43 (s, 2H), 3.30-3.10 (m, 5H), 2.82-2.72 (m, 1H), 2.23-2 .15 (m, 2H), 2.06-1.85 (m, 5H), 1.30 (s, 1H).

### Example 14: Synthesis of E-14: 1-cyclobutyl-N-((2-((4-(6-(methylsilyl)-1H-indazol-4-yl)-1H-1,2,3-triazol-1-yl)methl)imidazo[1,2-a]pyridin-6-yl)methyl)methylamine

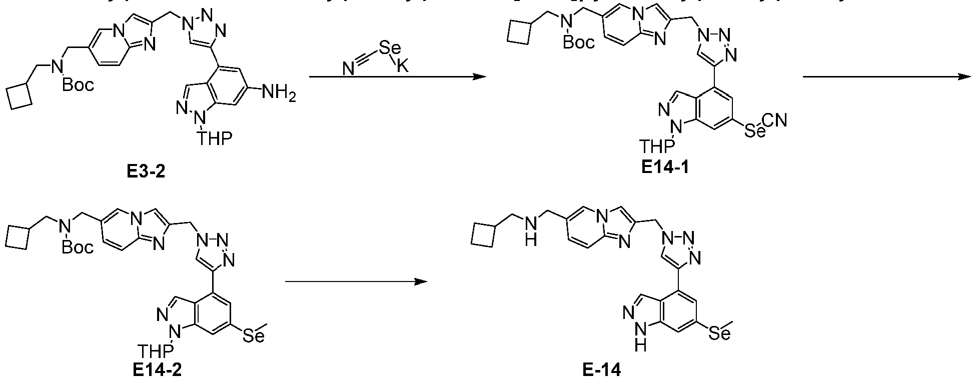

### E14-1: Tert-butyl (2-((4-(6-selenocyano-1-(tetrahydro-2H-pyran-2-yl)-1H-indazol-4-yl)-1H-1,2,3-triazol-1-yl)methyl)imidazo[1,2-a]pyridin-6-yl)methyl)carbamate

Potassium selenocyanate (47.1 mg, 326.94 µmol, 1.00 eq) and tert-butyl nitrite (40.5 mg, 392 µmol, 46.7 µL, 1.20 eq) were added dropwise to a solution of intermediate **E3-2** (0.200 g, 327 µmol, 1.00 eq) in ACN (2.00 mL) at 25°C while stirring, and the mixture was stirred at 50°C for 2 h under nitrogen protection. The mixture was diluted with water (10 mL) and extracted with EtOAc (3 x 10 mL). The combined organic layers were washed with saline (2 x 10 mL), dried over anhydrous sodium sulfate, filtered and concentrated to obtain a yellow solid intermediate **E14-1** (0.220 g, crude product).

LC-MS: [M+H]⁺= 702.2.

### E14-2: Tert-butyl (cyclobutylmethyl)((2-((4-(6-(methylsilyl)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

At 0°C, NaBH₄ (23.8 mg, 628 µmol, 2.00 eq) was added to a solution of intermediate **E14-1** (0.220 g, 314 µmol, 1.00) and CH₃I (89.1 mg, 628 µmol, 39.9) in MeOH (2.2 mL), and the mixture was stirred at 25°C for 2 h. The mixture was quenched with a saturated sodium bicarbonate solution (20 mL) and extracted with petroleum ether (2 x 20 mL). The combined organic layers were washed with saline (3 x 20 mL), dried over anhydrous sodium sulfate, filtered and concentrated. The residue was purified by preparative thin-layer chromatography and eluted with EtOAc/petroleum ether (1:0) to obtain a white solid intermediate **E14-2** (0.0700 g, 32.3%).

LC-MS: [M+H]⁺= 691.2.

### E-14: 1-cyclobutyl-N-((2-((4-(6-(methylsilyl)-1H-indazol-4-yl)-1H-1,2,3-triazol-1-yl)methyl)imidazo[1,2-a]pyridin-6-yl)methyl)methylamine

A white solid compound **E-14** (0.016 g, 35.0%) was prepared from intermediate **E14-2** using the method described in compound **E-1.**

LC-MS: [M+H]⁺= 507.1. ¹H NMR (400 MHz, methanol-d₄): δ 8.65 (s, 1H), 8.49 (s, 1H), 8.36 (s, 1H), 7.98 - 7.90 (m, 1H), 7.68 (d, *J* = 1.1 Hz, 1H), 7.55 (s, 1H), 7.51 (d, *J* = 9.3 Hz, 1H), 7.38 (d, *J =* 1.6, 9.3 Hz, 1H), 5.84 (s, 2H), 3.76 (s, 2H), 2.66 - 2.59 (m, 2H), 2.57 - 2.49 (m, 1H), 2.47 - 2.44 (m, 3H), 2.14 - 2.03 (m, 2H), 1.98 - 1.80 (m, 2H), 1.75 - 1.63 (m, 2H).

### Example 15: Synthesis of E-15: 1-cyclobutyl-N-((2-((4-(6-(pyrrolidin-1-yl)-1H-indazol-4-yl)-1H-1,2,3-triazol-1-yl)methyl)imidazo[1,2-a]pyridin-6-yl)methyl)methylamine hydrochloride

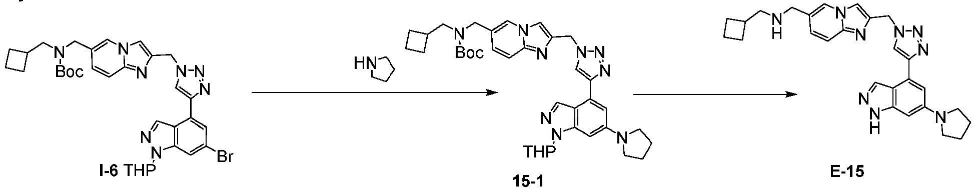

### E15-1: Tert-butyl (2-((4-(6-(pyrrolidin-1-yl)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

Tris(dibenzylideneacetone)dipalladium (27.1 mg, 29.6 µmol, 0.100 eq), 2-dicyclohexylphosphino-2'-(N,N-dimethylamino)biphenyl (11.6 mg, 29.6 µmol, 0.1 eq) and sodium tert-butoxide (42.7 mg, 444 µmol, 1.5 eq) were added to a solution of intermediate **I-6** (200 mg, 296 µmol, 1 eq) and pyrrolidine (421 mg, 5.92 mmol, 494 µL, 20.0 eq) in toluene (4.00 mL) at 20°C under nitrogen protection, and the resulting mixture was stirred at 80°C for 3 h. The mixture was concentrated, and the residue was purified by preparative thin-layer chromatography and eluted with EtOAc/petroleum ether (5:1) to obtain a yellow solid intermediate **E15-1** (140 mg, 210 µmol, 71.0% yield).

LC-MS: [M+H]⁺= 666.4.

### E-15: 1-cyclobutyl-N-((2-((4-(6-(pyrrolidin-1-yl)-1H-indazol-4-yl)-1H-1,2,3-triazol-1-yl)methyl)imidazo[1,2-a]pyridin-6-yl)methyl)methylamine hydrochloride

A light yellow solid compound **E-15** (70.9 mg, 64.9%) was prepared from intermediate **E15-1** using the method described in compound **E-1.**

LC-MS: [M+H]⁺= 482.4. ¹H NMR (400 MHz, DMSO-*d*₆): δ 9.70 (brs, 2H), 9.23 - 9.02 (m, 2H), 8.65 - 8.41 (m, 2H), 8.21 (d, *J =* 1.2, 9.3 Hz, 1H), 8.01 (d, *J =* 9.3 Hz, 1H), 7.27 (s, 1H), 6.59 (s, 1H), 6.09 (s, 2H), 4.27 - 4.24 (m, 2H), 3.41 (s, 4H), 3.02 - 2.87 (m, 2H), 2.80 - 2.62 (m, 1H), 2.17 - 1.95 (m, 6H), 1.90 - 1.73 (m, 4H)

### Example 16: Synthesis of E-16: 2,2'-((4-(1-((6-(((cyclobutylmethyl)amino)methyl)imidazo[1,2-a]pyridin-2-yl)methyl)-1H-1,2,3-triazol-4-yl)-1H-indazol-6-yl)azadiyl)bis(ethyl-1-ol)

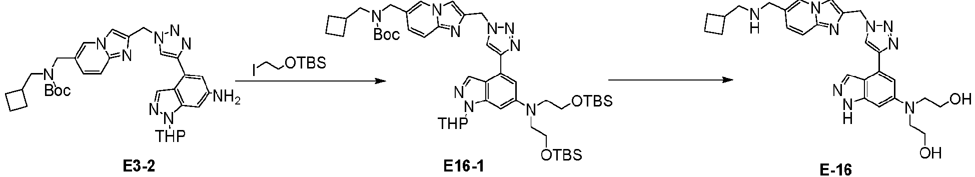

### E16-1: Tert-butyl (2-((4-(6-(bis(2-((tert-butyldimethylsilyl)oxy)ethyl)amino)-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

At 20°C, cesium carbonate (372 mg, 1.14 mmol, 3.50 eq) was added to a solution of intermediate **E3-2** (200 mg, 326 µmol, 1.00 eq) and tert-butyl (2-iodoethoxy)dimethylsilane (2.00 mL) in DMF (2.00 mL) while stirring, and the resulting mixture was stirred at 80°C for 3 h. The mixture was diluted with water (8 mL) and extracted with EtOAc (3 x 10 mL). The combined organic layers were washed with saline (2 x 10 mL), dried over anhydrous sodium sulfate, filtered, and concentrated. The residue was purified by preparative thin-layer chromatography and eluted with EtOAc/petroleum ether (1:0) to obtain a black brown solid intermediate **E16-1** (60.0 mg, 64.6 µmol, 19.7% yield).

LC-MS: [M+H]⁺= 928.7.

### E-16: 2,2'-((4-(1-((6-(((cyclobutylmethyl)amino)methyl)imidazo[1,2-a]pyridin-2-yl)methyl)-1H-1,2,3-triazol-4-yl)-1H-indazol-6-yl)azadiyl)bis(ethyl-1-ol)

A yellow solid compound **E-16** (70.9 mg, 64.9%) was prepared from intermediate **E16-1** using the method described in compound **E-1.**
¹H NMR (400 MHz, DMSO-*d*₆): δ 9.27 - 9.47 (m, 2 H) 9.02 (s, 1 H) 8.93 - 8.96 (m, 1 H) 8.46 (s, 1 H) 8.36 (s, 1 H) 7.94 - 8.00 (m, 1 H) 7.87 - 7.93 (m, 1 H) 7.37 (s, 1 H) 6.66 - 6.96 (m, 1 H) 6.00 (s, 2 H) 4.22 (t, *J =* 5.26 Hz, 2 H) 3.38 (s, 8 H) 2.93 - 2.97 (m, 2 H) 2.63 - 2.66 (m, 1 H) 2.01 (s, 2 H) 1.79 - 1.89 (m, 2 H) 1.75 - 1.79 (m, 2 H)
LC-MS: [M+H]⁺= 516.2.

### Example 17: Synthesis of E-17: N,n-bis(2-chloroethyl)-4-(1-((6-(((cyclobutylmethyl)amino)methyl)imidazo[1,2-a]pyridin-2-yl)methyl)-1H-1,2,3-triazole-4-yl)-1H-indazole-6-amine hydrochloride

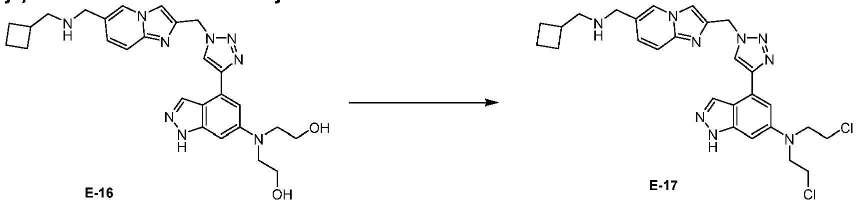

Under nitrogen protection, phosphorus oxychloride (1.5 mL) was added to compound **E-16** (30.0 mg, 58.1 µmol, 1.00 eq), and the resulting mixture was stirred at 80°C for 1 h. The mixture was diluted with ice water (1.5 mL) and purified by Prep-HPLC to obtain a white solid compound **E-17** (4.00 mg, 12.4%).

LC-MS: [M+H]⁺= 552.4. ¹H NMR (400 MHz, DMSO-*d*₆): δ 9.42 (br s, 2 H) 9.01 - 9.05 (m, 1 H) 8.95 - 9.00 (m, 1 H) 8.43 - 8.51 (m, 1 H) 8.30 - 8.37 (m, 1 H) 7.98 - 8.06 (m, 1 H) 7.88 - 7.96 (m, 1 H) 7.26 (s, 1 H) 6.66 (s, 1 H) 6.02 (s, 2 H) 4.22 (s, 2 H) 3.82 - 3.86 (m, 4 H) 3.80 (d, *J* = 5.13 Hz, 4 H) 2.94 (d, *J* = 4.25 Hz, 2 H) 2.64 - 2.70 (m, 1 H) 2.01 - 2.08 (m, 2 H) 1.72 - 1.85 (m, 4 H).

### Example 18: Synthesis of E-18: 1-cyclobutyl-N-((2-((4-(6-methyl-1H-indazol-4-yl)-1H-1,2,3-triazol-1-yl)methyl)imidazo[1,2-a]pyridin-6-yl)methyl)methylamine

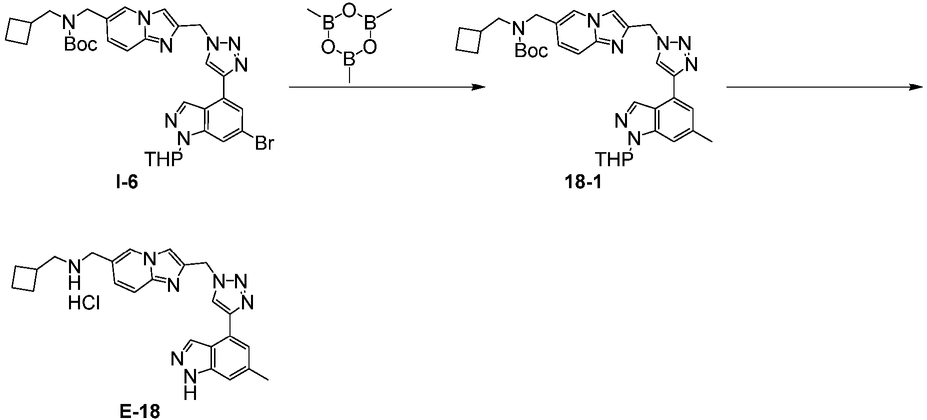

### E18-1: Tert-butyl (cyclobutylmethyl)((2-((4-(6-methyl-1-(tetrahydro-2H-pyran-2-yl)-1H-indazol-4-yl)-1H-1,2,3-triazol-1-yl)methyl)imidazo[1,2-a]pyridin-6-yl)methyl)carbamate

Intermediate **I-6** (100 mg, 148 µmol, 1.00 eq) and trimethylcyclotriboroxane (74.3 mg, 592 µmol, 82.8 µL, 4.00 eq) were dissolved in water (1.00 mL) and 1,4-dioxane (2.00 mL), and cesium carbonate (145 mg, 444 µmol, 3.00 eq) and tetrakis(triphenylphosphine)palladium (17.1 mg, 14.8 µmol, 0.10 eq) were added to the obtained solution under nitrogen protection. The resulting mixture was stirred at 100°C for 16 h. The mixture was diluted with water (10.00 mL) and extracted with EtOAc (3 x 2.00 mL). The combined organic layers were dried over anhydrous sodium sulfate, filtered, and concentrated. The residue was purified by preparative thin-layer chromatography and eluted with EtOAc/petroleum ether (1:0) to obtain a white solid compound **E18-1** (50.0 mg, 27.7%).

LC-MS: [M+H]⁺= 611.3.

### E-18: 1-cyclobutyl-N-((2-((4-(6-methyl-1H-indazol-4-yl)-1H-1,2,3-triazol-1-yl)methyl)imidazo[1,2-a]pyridin-6-yl)methyl)methylamine

A white solid compound **E-18** (70.9 mg, 64.9%) was prepared from intermediate **E18-1** using the method described in compound **E-1.**

LC-MS: [M+H]⁺= 427.4. ¹H NMR (400 MHz, DMSO-*d*₆): δ 8.79 - 8.82 (m, 1 H) 8.46 - 8.50 (m, 1 H) 8.40 - 8.45 (m, 1 H) 7.95 - 7.99 (m, 1 H) 7.45 - 7.50 (m, 2 H) 7.24 - 7.30 (m, 2 H) 5.76 - 5.79 (m, 2 H) 3.64 - 3.67 (m, 2 H) 3.30 - 3.31 (m, 2 H) 2.47 (s, 5 H) 1.94 - 2.00 (m, 2 H) 1.67 - 1.93 (m, 3 H) 1.58 - 1.65 (m, 2 H).

### Example 19: Synthesis of E-19: N-(4-(1-((6-(((cyclobutylmethyl)amino)methyl)imidazo[1,2-a]pyridin-2-yl)methyl)-1H-1,2,3-triazol-4-yl)-1H-indazol-6-yl)acetamide

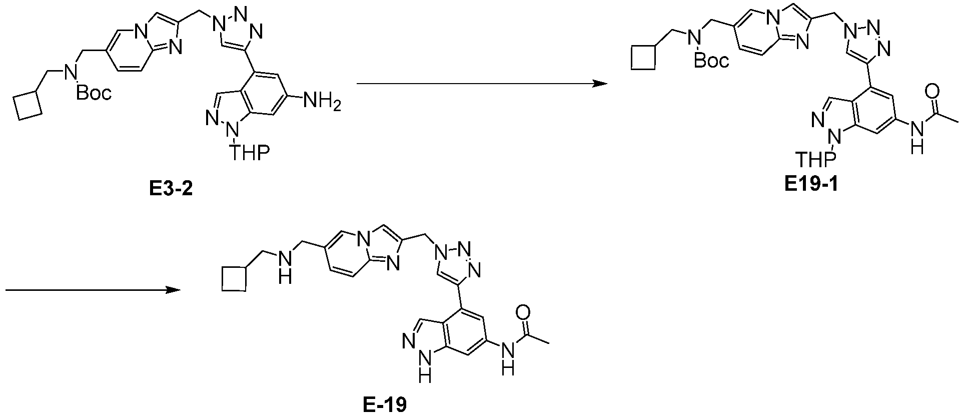

### E19-1: Tert-butyl (2-((4-(6-acetamido-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

Glacial acetic acid (5.01 mg, 49.0 µmol, 4.59 µL, 1.50 eq) was added to a solution of intermediate **E3-2** (0.02 g, 32.7 µmol, 1.00 eq) and triethylamine (6.62 mg, 65.4 µmol, 9.10 µL, 2.00 eq) in DCM (1.00 mL) at 0°C, and the resulting mixture was stirred at 25°C for 2 h. The mixture was diluted with water (1.00 mL) and extracted with DCM (3 x 2.00 mL). The combined organic layers were dried over anhydrous sodium sulfate, filtered and concentrated. The residue was purified by preparative thin-layer chromatography and eluted with EtOAc/petroleum ether (1:0) to obtain a yellow oily intermediate **E19-1** (10.0 mg, 46.8%).

LC-MS: [M+H]⁺= 654.5.

### E-19: N-(4-(1-((6-(((cyclobutylmethyl)amino)methyl)imidazo[1,2-a]pyridin-2-yl)methyl)-1H-1,2,3-triazol-4-yl)-1H-indazol-6-yl)acetamide

A yellow sticky compound **E-19** (70.9 mg, 64.9%) was prepared from intermediate **E19-1** using the method described in compound **E-3.**

LC-MS: [M+H]⁺= 470.0. ¹H NMR (400 MHz, DMSO-*d*₆): *δ* 10.24 (s, 1H), 9.22 (br s, 2H), 8.85 (s, 1H), 8.81 (s, 1H), 8.38 (s, 1H), 8.28 (s, 1H), 8.08 (s, 1H), 7.74 (s, 1H), 7.78 - 7.71 (m, 1H), 7.69 (d, *J* = 1.5 Hz, 1H), 7.70 - 7.68 (m, 1H), 5.91 (s, 2H), 4.14 (t, *J* = 5.3 Hz, 2H), 2.91 (br d, *J* = 4.8 Hz, 2H), 2.65 - 2.61 (m, 1H), 2.06 (s, 3H), 2.03 - 1.97 (m, 2H), 1.78 - 1.68 (m, 4H).

### Example 20: Synthesis of E-20: 4-(1-((6-(((cyclobutylmethyl)amino)methyl)imidazo[1,2-a]pyridin-2-yl)methyl)-1H-1,2,3-triazole-4-yl)-1H-indazole-6-yl acetate

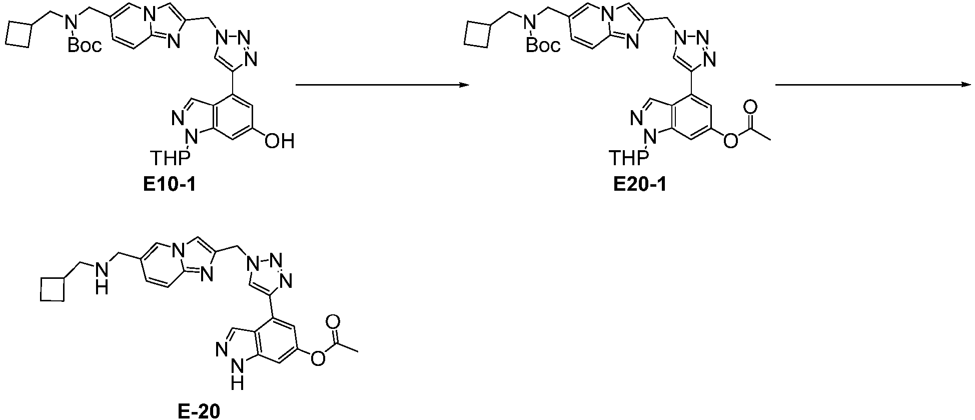

### E20-1: 4-(1-((6-(((tert-butoxycarbonyl)(cyclobutylmethyl)amino)methyl)imidazo[1,2-a]pyridin-2-yl)methyl)-1H-1,2,3-triazol-4-yl)-1-(tetrahydro-2H-pyran-2-yl)-1H-indazol-6-yl acetate

Glacial acetic acid (30.0 mg, 294 µmol, 27.5 µL, 1.50 eq) was added to a solution of intermediate **E10-1** (120 mg, 196 µmol, 1.00 eq) and pyridine (15.5 mg, 196 µmol, 15.8 µL, 1.00 eq) in toluene (6.00 mL) and tetrahydrofuran (1.20 mL) at 0°C, and the resulting mixture was stirred at 20°C for 12 h. The mixture was diluted with water (10.00 mL) and extracted with EtOAc (3 x 3.00 mL). The combined organic layers were washed with saline (5 mL), dried over anhydrous sodium sulfate, filtered and concentrated to obtain a red solid intermediate **E20-1** (0.120 g, crude product).

LC-MS: [M+H]⁺= 655.5.

### E-20: 4-(1-((6-(((cyclobutylmethyl)amino)methyl)imidazo[1,2-a]pyridin-2-yl)methyl)-1H-1,2,3-triazole-4-yl)-1H-indazole-6-yl acetate

TFA (0.35 mL, 2.000 mmol, 20 eq) was added to a solution of intermediate **E20-1** (70.00 mg, 106.91 µmol, 1 eq) in DCM (1.40 mL), and the resulting mixture was stirred at 0-25°C for 2 h. The mixture was concentrated and the residue was purified by Prep-HPLC to obtain a white solid compound **E-20** (0.0011 g, 5.08%).

LC-MS: [M+H]⁺= 471.2. ¹H NMR (400 MHz, DMSO-*d*₆): δ 10.32 (s, 1 H) 8.92 (s, 1 H) 8.74 (s, 1 H) 8.64 (br s, 1 H) 8.11 (br s, 1 H) 7.74 (d, *J* = 1.13 Hz, 1 H) 7.61 (d, *J* = 9.26 Hz, 1 H) 7.33 - 7.38 (m, 2 H) 5.80 - 5.83 (m, 2 H) 4.12 (br s, 2 H) 2.96 (d, *J =* 6.38 Hz, 2 H) 2.70 (s, 3 H) 2.54 (d, *J* = 3.88 Hz, 1 H) 2.04 (d, *J* = 8.13 Hz, 2 H) 1.80 (br s, 2 H) 1.76 (br s, 2 H).

### Example 21: Synthesis of E-21: 1-cyclobutyl-N-((2-((4-(6-(piperidin-1-yl)-1H-indazol-4-yl)-1H-1,2,3-triazol-1-yl)methyl)imidazo[1,2-a]pyridin-6-yl)methyl)methylamine

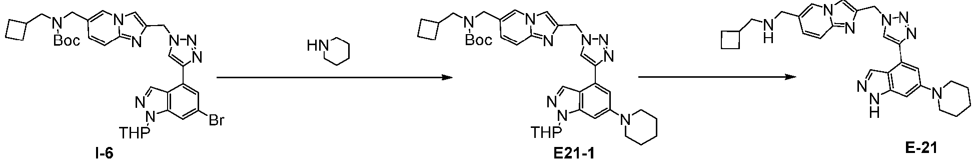

### E21-1: Tert-butyl (2-((4-(6-(piperidin-1-yl)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **E21-1** (120 mg, 79.5%) was prepared from intermediate **I-6** using the method described in intermediate **E15-1.**

LC-MS: [M+H]⁺= 780.4.

### E-21: 1-cyclobutyl-N-((2-((4-(6-(piperidin-1-yl)-1H-indazol-4-yl)-1H-1,2,3-triazol-1-yl)methyl)imidazo[1,2-a]pyridin-6-yl)methyl)methylamine

A white solid compound **E-21** (58.4 mg, 62.2%) was prepared from intermediate **E21-1** using the method described in compound **E-1.**

LC-MS: [M+H]⁺= 496.1. ¹H NMR (400 MHz, DMSO-*d*₆): δ 9.62 (br s, 2H), 9.16 (s, 1H), 9.03 (s, 1H), 8.67 (s, 1H), 8.51 (s, 1H), 8.14 (br s, 2H), 8.07 (d, *J* = 9.4 Hz, 1H), 7.93 (d, *J* = 9.4 Hz, 1H), 6.08 (s, 2H), 4.26 - 4.20 (m, 2H), 3.62 (br s, 4H), 2.98 - 2.90 (m, 2H), 2.76 - 2.64 (m, 1H), 2.17 - 1.93 (m, 6H), 1.87 - 1.65 (m, 6H).

### Example 22: Synthesis of E-22: 1-cyclobutyl-N-((6-((4-(6-(methylthio)-1H-indazol-4-yl)-1H-1,2,3-triazol-1-yl)methyl)-1H-indole-2-yl)methyl)methylamine

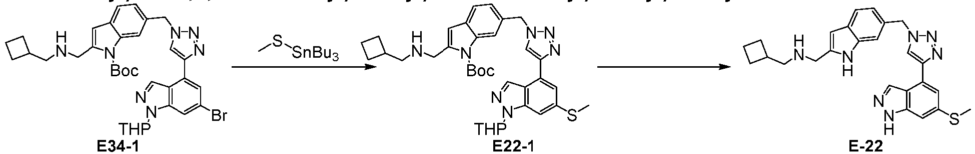

### E22-1: Tert-butyl 2-(((cyclobutylmethyl)amino)methyl)-6-((4-(6-(methylthio)-1-(tetrahydro-2H-pyran-2-yl)-1H-indazol-4-yl)-1H-1,2,3-triazol-1-yl)methyl)-1H-indole-1-carboxylate

Under nitrogen protection, intermediate **E34-1** (0.02 g, 29.7 µmol, 1.00 eq) and tributyl(methylthio)stannane (30.0 mg, 88.9 µmol, 3.00 eq) were dissolved in DMF (1.00 mL), and [1,1'-bis(diphenylphosphino)ferrocene]palladium (II) dichloride (2.17 mg, 2.96 µmol, 0.1 eq) was added to the obtained solution. The mixture was stirred at 100°C for 16 h under nitrogen protection. The mixture was diluted with water (3 mL) and extracted with EtOAc (2 mL * 2). The combined organic layers were dried over anhydrous sodium sulfate, filtered and concentrated. The residue was purified by preparative thin-layer chromatography (petroleum ether/EtOAc=3/1) to obtain a yellow solid intermediate **E22-1** (0.01 g, crude product).

LC-MS: [M+H]⁺= 642.3.

### E-22: 1-cyclobutyl-N-((6-((4-(6-(methylthio)-1H-indazol-4-yl)-1H-1,2,3-triazol-1-yl)methyl)-1H-indole-2-yl)methyl)methylamine

A white solid compound **E-22** (1.2 mg, 15.8%) was prepared from intermediate **E22-1** using the method described in compound **E-34.**

LC-MS: [M+H]⁺= 457.4. ¹H NMR (400 MHz, DMSO-*d*₆): δ 11.38 (s, 1H), 9.09 (s, 2H), 8.98 (s, 1H), 8.51 (s, 1H), 7.58 (d, *J =* 8.3 Hz, 1H), 7.53 - 7.45 (m, 2H), 7.29 (s, 1H), 7.17 - 7.09 (m, 1H), 6.61 (s, 1H), 5.75 (s, 2H), 4.27 (t, *J =* 5.2 Hz, 2H), 2.99 - 2.94 (m, 2H), 2.65 - 2.61 (m, 1H), 2.58 (s, 3H), 2.09 - 2.00 (m, 2H), 1.89 - 1.75 (m, 4H)

### Example 23: Synthesis of E-23: 4-(1-((2-(((cyclobutylmethyl)amino)methyl)-1H-indole-6-yl)methyl)-1H-1,2,3-triazol-4-yl)-N,N-dimethyl-1H-indazole-6-amine

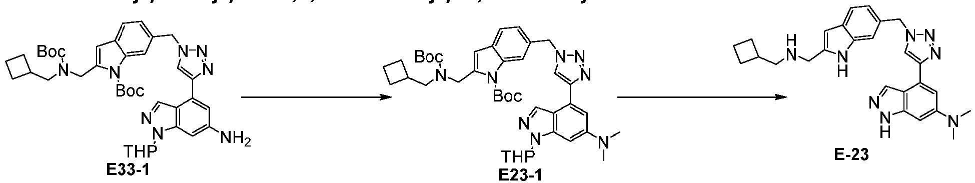

### E23-1: Tert-butyl 2-(((tert-butoxycarbonyl)(cyclobutylmethyl)amino)methyl)-6-((4-(6-(dimethylamino)-1-(tetrahydro-2H-pyran-2-yl)-1H-indazol-4-yl)-1H-1,2,3-triazol-1-yl)methyl)-1H-indole-1-carboxylate

A white solid intermediate **E23-1** (70.0 mg, 77.9%) was prepared from intermediate **E33-1** using the method described in intermediate **E5-1.**

LC-MS: [M+H]⁺= 738.4.

### E-23: 4-(1-((2-(((cyclobutylmethyl)amino)methyl)-1H-indole-6-yl)methyl)-1H-1,2,3-triazol-4-yl)-N,N-dimethyl-1H-indazole-6-amine

A yellow oily compound **E-23** (13.8 mg, 35.6%) was prepared from intermediate **E23-1** using the method described in compound **E-1.**

LC-MS: [M+H]⁺= 455.2. ¹H NMR (400 MHz, DMSO-*d*₆): δ 11.56 (s, 1H), 9.37 (br s, 2H), 8.94 (s, 1H), 8.57 (s, 1H), 7.77 (br s, 1H), 7.54 (d, *J =* 8.3 Hz, 1H), 7.47 (s, 1H), 7.08 (d, *J* = 8.3 Hz, 1H), 6.58 (s, 1H), 5.76 (s, 2H), 4.24 (t, *J* = 5.0 Hz, 2H), 3.12 (s, 6H), 2.94 - 2.86 (m, 2H), 2.63 (td, *J* = 7.4, 14.7 Hz, 1H), 2.05 - 1.96 (m, 2H), 1.86 - 1.77 (m, 1H), 1.77 - 1.70 (m, 2H), 1.77 - 1.70 (m, 1H).

### Example 24: Synthesis of E-24: 1-cyclobutyl-N-((6-((4-(6-iodo-1H-indazol-4-yl)-1H-1,2,3-triazol-1-yl)methyl)-1H-indole-2-yl)methyl)methylamine

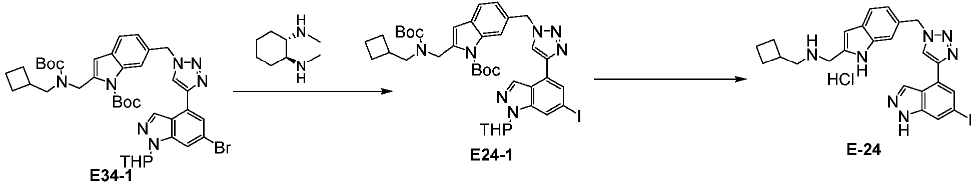

### E24-1: Tert-butyl 2-(((tert-butoxycarbonyl)(cyclobutylmethyl)amino)methyl)-6-((4-(6-iodo-1-(tetrahydro-2H-pyran-2-yl)-1H-indazol-4-yl)-1H-1,2,3-triazol-1-yl)methyl)-1H-indole-1-carboxylate

At 20°C, sodium iodide (58.0 mg, 387 µmol, 3.00 eq), cuprous iodide (12.3 mg, 64.5 µmol, 0.50 eq), and (1S,2S)-N1,N2-dimethylcyclohexane-1,2-diamine (18.3 mg, 129 µmol, 1.00 eq) were added to a solution of intermediate **E34-1** (0.10 g, 129 µmol, 1.00 eq) in 1,4-dioxane (3.00 mL), and the mixture was stirred at 110°C for 16 h under nitrogen protection. The resulting mixture was concentrated, and the residue was purified by preparative thin-layer chromatography (petroleum ether/ethyl acetate=2/1) to obtain a yellow solid intermediate **E24-1** (65.0 mg, 61.2%).

### E-24: 1-cyclobutyl-N-((6-((4-(6-iodo-1H-indazol-4-yl)-1H-1,2,3-triazol-1-yl)methyl)-1H-indole-2-yl)methyl)methylamine

A yellow solid compound **E-24** (32.0 mg, 69.0%) was prepared from intermediate **E24-1** using the method described in compound **E-1.**

LC-MS (ESI): m/z 538.2 [M+H]⁺. ¹H NMR (400 MHz, DMSO-*d*₆): δ 13.31 (s, 1H), 11.35 (s, 1H), 9.10 - 8.98 (m, 3H), 8.59 (s, 1H), 7.91 (d, *J =* 3.2 Hz, 2H), 7.58 (d, *J =* 8.2 Hz, 1H), 7.50 (s, 1H), 7.13 (d, *J* = 7.5 Hz, 1H), 6.61 (s, 1H), 5.75 (s, 2H), 4.30 - 4.23 (m, 2H), 3.01 - 2.94 (m, 2H), 2.65 - 2.58 (m, 1H), 2.09 - 2.00 (m, 2H), 1.91 - 1.83 (m, 1H), 1.82 - 1.73 (m, 3H).

### Example 25: Synthesis of E-25: 1-cyclobutyl-N-((6-((4-(5-(2-(2-methoxyethoxy)ethoxy)pyridin-3-yl)-1H-1,2,3-triazol-1-yl)methyl)-1H-indole-2-yl)methyl)methylamine

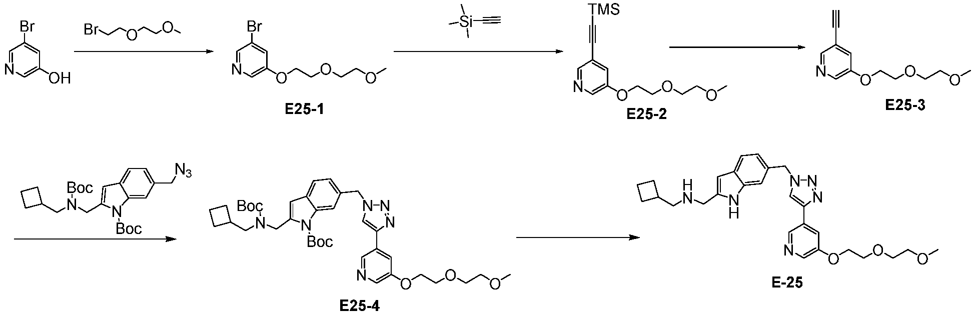

### E25-1: 3-bromo-5-(2-(2-methoxyethoxy)ethoxy)pyridine

A yellow oily intermediate **E25-1** (32.0 mg, 69.0%) was prepared from 5-bromopyridine-3-ol using the method described in intermediate **E10-2.**

LC-MS (ESI): m/z 276.0 [M+H]⁺.

### E25-2: 3-(2-(2-methoxyethoxy)ethoxy)-5-((trimethylsilyl)ethynyl)pyridine

Bis(triphenylphosphine)palladium (II) dichloride (254mg, 362 µmol, 0.05 eq), cuprous iodide (138 mg, 724 µmol, 0.10 eq), and triethylamine (2.93 g, 29.0 mmol, 4.03 mL, *4.00* eq) were added to a solution of 3-bromo-5-(2-(2-methoxyethoxy)ethoxy)pyridine (2.00 g, 7.24 mmol, 1.00 eq) and ethynyl trimethylsilane (854 mg, 8.69 mmol, 1.20 mL, 1.20 eq) in ethyl acetate (20.0 mL), and the mixture was stirred at 75°C for 20 h. The resulting mixture was filtered, washed with EtOAc (20.0 mL * 4) and concentrated. The residue was purified by silica gel column chromatography (petroleum ether/ethyl acetate=100/1 to 0/1) to obtain a black oily intermediate **E25-2** (1.80 g, 84.6%).

LC-MS (ESI): m/z 294.2 [M+H]⁺.

### E25-3: 3-ethynyl-5-(2-(2-methoxyethoxy)ethoxy)pyridine

A yellow oily intermediate **E25-3** (1.00 g, 73.7%) was prepared from intermediate **E25-2** using the method described in intermediate **I-2.**

LC-MS (ESI): m/z 222.1 [M+H]⁺.

### E25-4: Tert-butyl 2-(((tert-butoxycarbonyl)(cyclobutylmethyl)amino)methyl)-6-((4-(5-(2-(2-methoxyethoxy)ethoxy)pyridin-3-yl)-1H-1,2,3-triazol-1-yl)methyl)-1H-indole-1-carboxylate

A yellow solid intermediate **E25-4** (0.08 g, 54.4%) was prepared from intermediate **E25-3** using the method described in intermediate **4-d.**

LC-MS (ESI): m/z 691.6 [M+H]⁺.

### E-25: 1-cyclobutyl-N-((6-((4-(5-(2-(2-methoxyethoxy)ethoxy)pyridin-3-yl)-1H-1,2,3-triazol-1-yl)methyl)-1H-indole-2-yl)methyl)methylamine

A yellow oily compound **E-25** (22.0 mg, 35.8%) was prepared from intermediate **E25-4** using the method described in compound **E-1.**

LC-MS (ESI): m/z 491.2 [M+H]⁺. ¹H NMR (400 MHz, DMSO-*d*₆): δ 11.55 (s, 1H), 9.33 (s, 2H), 8.85 (s, 1H), 8.78 (s, 1H), 8.43 (d, *J* = 2.4 Hz, 1H), 8.14 (s, 1H), 7.54 (d, *J* = 8.1 Hz, 1H), 7.45 (s, 1H), 7.04 (d, *J =* 8.3 Hz, 1H), 6.59 (s, 1H), 5.74 (s, 2H), 4.33 - 4.21 (m, 4H), 3.75 (s, 1H), 3.56 (d, *J =* 5.0 Hz, 3H), 3.45 - 3.39 (m, 2H), 3.20 (s, 3H), 2.91 (d, *J =* 4.8 Hz, 2H), 2.66 - 2.60 (m, 1H), 2.02 (d, *J =* 6.8 Hz, 2H), 1.86 - 1.69 (m, 4H).

### Example 26: Synthesis of E-26: 1-(3-fluorobicyclo[1.1.1]pentan-1-yl)-N-((2-((4-(6-iodo-1H-indazol-4-yl)-1H-1,2,3-triazol-1-yl)methyl)imidazo[1,2-a]pyridin-6-yl)methyl)methylamine

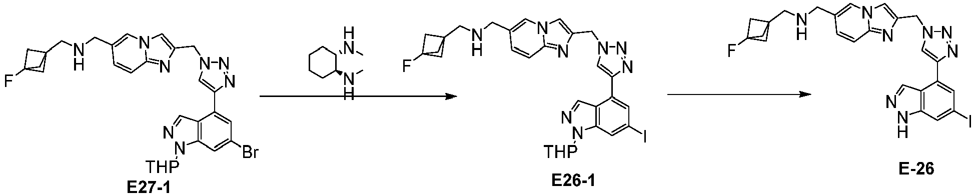

### E26-1: 1-(3-fluorobicyclo[1.1.1]pentan-1-yl)-N-((2-((4-(6-iodo-1-(tetrahydro-2H-pyran-2-yl)-1H-indazol-4-yl)-1H-1,2,3-triazol-1-yl)methyl)imidazo[1,2-a]pyridin-6-yl)methyl)methylamine

A brown solid intermediate **E26-1** (0.06 g, 62.8%) was prepared from intermediate **E27-1** using the method described in intermediate **E24-1.**

LC-MS: [M+H]⁺= 653.5.

### E-26: 1-(3-fluorobicyclo[1.1.1]pentan-1-yl)-N-((2-((4-(6-iodo-1H-indazol-4-yl)-1H-1,2,3-triazol-1-yl)methyl)imidazo[1,2-a]pyridin-6-yl)methyl)methylamine

A yellow solid compound **E-26** (5.20 mg, 15.3%) was prepared from intermediate **E26-1** using the method described in compound **E-1.**

LC-MS: [M+H]⁺= 569.1. ¹H NMR (400 MHz, DMSO-*d*₆): δ 8.95 (s, 1H), 8.78 (s, 1H), 8.57 (s, 1H), 8.40 (s, 1H), 8.01 - 7.91 (m, 4H), 6.08 (s, 2H), 4.43 (s, 2H), 3.50 (s, 2H), 2.19 (d, *J* = 2.3 Hz, 6H).

### Example 27: Synthesis of E-27: 1-(3-fluorobicyclo[1.1.1]pentan-1-yl)-N-((2-((4-(6-(methylthio)-1H-indazol-4-yl)-1H-1,2,3-triazol-1-yl)methyl)imidazo[1,2-a]pyridin-6-yl)methyl)methylamine

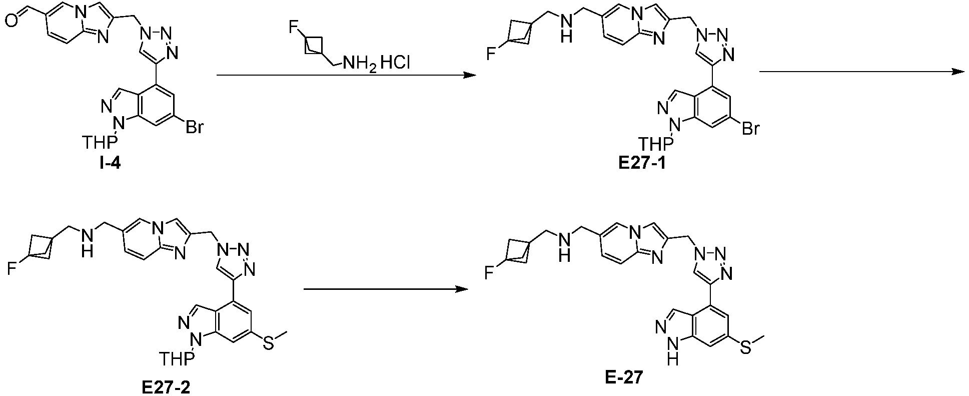

### E27-1: 1-(2-((4-(6-bromo-1-(tetrahydro-2H-pyran-2-yl)-1H-indazol-4-yl)-1H-1,2,3-triazol-1-yl)methyl)imidazo[1,2-a]pyridin-6-yl)-N-((3-fluorobicyclo[1.1.1]pentan-1-yl)methyl)methylamine

A yellow solid intermediate **E27-1** (0.15 g, 41.8%) was prepared from intermediate **I-4** and (3-fluorobicyclo[1.1.1]pentan-1-yl)methylamine hydrochloride using the method described in intermediate **E6-1.**

**LC-MS:** [M+H]⁺= 605.3.

### E27-2: 1-(3-fluorobicyclo[1.1.1]pentan-1-yl)-N-((2-((4-(6-(methylthio)-1-(tetrahydro-2H-pyran-2-yl)-1H-indazol-4-yl)-1H-1,2,3-triazol-1-yl)methyl)imidazo[1,2-a]pyridin-6-yl)methyl)methylamine

A yellow solid intermediate **E27-2** (0.07 g, 52.5%) was prepared from intermediate **E27-1** using the method described in intermediate **E1-2.**

LC-MS: [M+H]⁺=573.3.

### E-27: 1-(3-fluorobicyclo[1.1.1]pentan-1-yl)-N-((2-((4-(6-(methylthio)-1H-indazol-4-yl)-1H-1,2,3-triazol-1-yl)methyl)imidazo[1,2-a]pyridin-6-yl)methyl)methylamine

A yellow solid compound **E-27** (32.8 mg, 50.5%) was prepared from intermediate **E27-2** using the method described in compound **E-1.**

LC-MS: [M+H]⁺= 489.1. ¹H NMR (400 MHz, DMSO-*d*₆): δ 9.67 (s, 2H), 9.05 (s, 1H), 9.00 (s, 1H), 8.49 (s, 2H), 8.06 (d, *J* = 9.0 Hz, 1H), 7.93 (d, *J =* 9.4 Hz, 1H), 7.49 (d, *J* = 1.3 Hz, 1H), 7.28 (s, 1H), 6.02 (s, 2H), 4.26 (s, 2H), 3.29 - 3.20 (m, 2H), 2.55 (s, 3H), 2.10 (d, *J* = 2.4 Hz, 6H).

### Example 28: Synthesis of E-28: 4-(1-((6-((((3-fluorobicyclo[1.1.1]pentan-1-yl)methyl)amino)methyl)imidazo[1,2-a]pyridin-2-yl)methyl)-1H-1,2,3-triazol-4-yl)-N,n-dimethyl-1H-indazole-6-amine

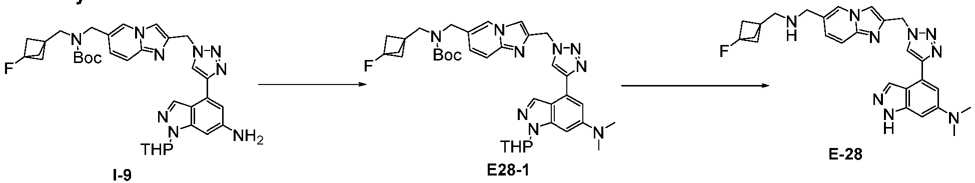

### E28-1: Tert-butyl ((2-((4-(6-(dimethylamino)-1-(tetrahydro-2H-pyran-2-yl)-1H-indazol-4-yl)-1H-1,2,3-triazol-1-yl)methyl)imidazo[1,2-a]pyridin-6-yl)methyl)((3-fluorobicyclo[1.1.1]pentan-1-yl)methyl)carbamate

A yellow solid intermediate **E28-1** (100 g, crude product) was prepared from intermediate **I-9** using the method described in intermediate **E5-1.**

LC-MS: [M+H]⁺= 670.3.

### E-28: 4-(1-((6-((((3-fluorobicyclo[1.1.1]pentan-1-yl)methyl)amino)methyl)imidazo[1,2-a]pyridin-2-yl)methyl)-1H-1,2,3-triazol-4-yl)-N,n-dimethyl-1H-indazole-6-amine

A yellow solid compound **E-28** (35.9 mg, 45.8%) was prepared from intermediate **E28-1** using the method described in compound **E-1.**

LC-MS: [M+H]⁺= 486.3. ¹H NMR (400 MHz, DMSO-*d*₆): δ 9.07 (s, 1 H) 9.00 (s, 1 H) 8.77 (s, 1 H) 8.52 (s, 1 H) 8.15 (dd, J= 9.42, 1.55 Hz, 1 H) 8.00 (d, *J* = 9.30 Hz, 1 H) 7.90 (s, 1 H) 7.67 (br s, 1 H) 6.15 (s, 2 H) 4.46 (s, 2 H) 3.51 (s, 2 H) 3.39 (s, 6 H) 2.19 (d, *J* = 2.38 Hz, 6 H).

### Example 29: Synthesis of E-29: 4-(1-((6-((6-azaspiro[3.4]octan-6-yl)methyl)imidazo[1,2-a]pyridin-2-yl)methyl)-1H-1,2,3-triazol-4-yl)-6-iodo-1H-indazole

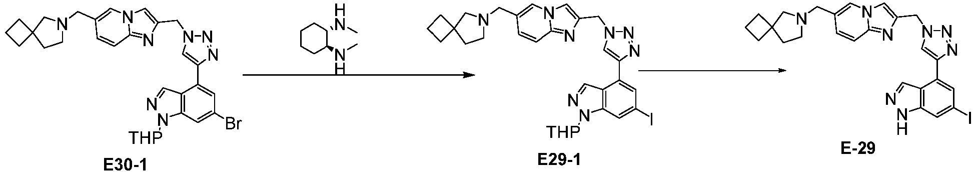

### E29-1: 4-(1-((6-((6-azaspiro[3.4]octan-6-yl)methyl)imidazo[1,2-a]pyridin-2-yl)methyl)-1H-1,2,3-triazol-4-yl)-6-iodo-1-(tetrahydro-2H-pyran-2-yl)-1H-indazole

A yellow solid intermediate **E29-1** (30.0 mg, 34.8%) was prepared from intermediate **E30-1** using the method described in intermediate **E24-1.**

LC-MS: [M+H]⁺= 649.5.

### E-29: 4-(1-((6-((6-azaspiro[3.4]octan-6-yl)methyl)imidazo[1,2-a]pyridin-2-yl)methyl)-1H-1,2,3-triazol-4-yl)-6-iodo-1H-indazole

A yellow solid compound **E-29** (4.00 mg, 15.1%) was prepared from intermediate **E29-1** using the method described in compound **E-3.**

LC-MS: [M+H]⁺= 565.0. ¹H NMR (400 MHz, DMSO-*d*₆): δ (br s, 1 H) 9.09 (s, 1 H) 9.03 (s, 1 H) 8.56 (s, 1 H) 8.45 (s, 1 H) 8.11 (d, *J* = 8.99 Hz, 1 H) 7.90 - 7.94 (m, 2 H) 7.88 (s, 1 H) 6.02 (s, 2 H) 4.44 - 4.50 (m, 1 H) 4.37 - 4.43 (m, 1 H) 3.36 (d, *J =* 11.51, 5.37 Hz, 2 H) 3.10 - 3.17 (m, 1 H) 3.06 (d, *J* = 11.40, 7.02 Hz, 1 H) 2.04 - 2.12 (m, 2 H) 1.93 - 2.04 (m, 3 H) 1.78 - 1.89 (m, 2 H) 1.69 - 1.76 (m, 1 H).

### Example 30: Synthesis of E-30: 4-(1-((6-((6-azaspiro[3.4]octan-6-yl)methyl)imidazo[1,2-a]pyridin-2-yl)methyl)-1H-1,2,3-triazol-4-yl)-6-(methylthio)-1H-indazole

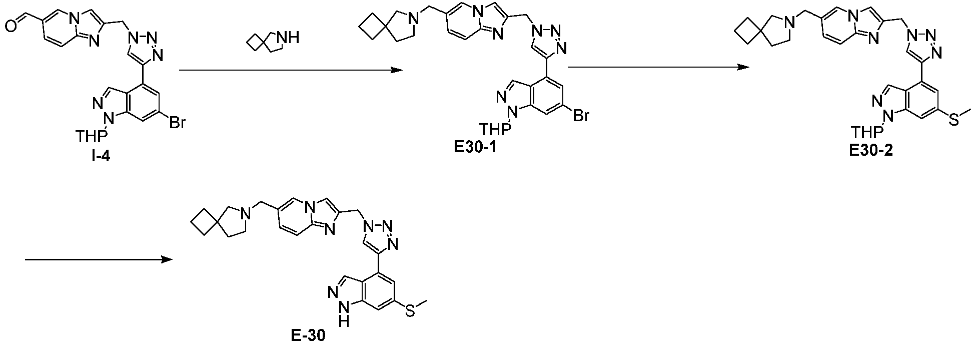

### E30-1: 4-(1-((6-((6-azaspiro[3.4]octan-6-yl)methyl)imidazo[1,2-a]pyridin-2-yl)methyl)-1H-1,2,3-triazol-4-yl)-6-bromo-1-(tetrahydro-2H-pyran-2-yl)-1H-indazole

AcOH (3.56 mg, 59.2 µmol, 3.39 µL, 0.100 eq) was added to a solution of intermediate **I-4** (300 mg, 592 µmol, 1.00 eq) and 6-azaspiro[3.4]octane (131 mg, 1.18 mmol, 2.00 eq) in MeOH (15.0 mL), and the resulting mixture was stirred at 25°C for 30 min under nitrogen protection. Then, sodium triacetoxyborohydride (74.4 mg, 1.18 mmol, 2.00 eq) was added to the mixture at 25°C. The mixture was stirred at 50°C for 2 h under nitrogen protection. The resulting mixture was diluted with water (20 mL) and extracted with DCM (3 x 10 mL). The combined organic layers were dried over anhydrous sodium sulfate, filtered and concentrated. The residue was purified by preparative thin-layer chromatography (DCM/MeOH=10/1) to obtain a yellow oily intermediate **E30-1** (220 mg, 61.7%).

LC-MS: [M+H]⁺= 603.2. ¹H NMR (400 MHz, DMSO-*d*₆): δ 8.95 (s, 1 H) 8.62 (s, 1 H) 8.41 (s, 1 H) 7.98 (s, 1 H) 7.95 (s, 1 H) 7.80 (d, *J =* 1.53 Hz, 1 H) 7.43 (d, *J =* 9.65 Hz, 1 H) 7.18 (dd, *J* = 9.43, 1.32 Hz, 1 H) 5.86 - 5.91 (m, 1 H) 5.75 (s, 2 H) 5.72 (s, 2 H) 3.71 - 3.87 (m, 2 H) 3.48 (d, *J* = 0.88 Hz, 2 H) 3.28 (s, 2 H) 2.49 (d, *J* = 1.75 Hz, 2 H) 2.28 - 2.41 (m, 2 H) 1.86 - 1.88 (m, 2 H) 1.65 - 1.81 (m, 6 H) 1.53 - 1.57 (m, 2 H).

### E30-2: 4-(1-((6-((6-azaspiro[3.4]octan-6-yl)methyl)imidazo[1,2-a]pyridin-2-yl)methyl)-1H-1,2,3-triazol-4-yl)-6-(methylthio)-1-(tetrahydro-2H-pyran-2-yl)-1H-indazole

A yellow solid intermediate **E30-2** (34.0 mg, 35.9%) was prepared from intermediate **E30-1** using the method described in intermediate **E1-2.**

LC-MS: [M+H]⁺= 569.3. ¹H NMR (400 MHz, DMSO-*d*₆): δ 8.94 (s, 1 H) 8.87 (s, 1 H) 8.63 (s, 1 H) 8.56 (s, 1 H) 8.45 (s, 1 H) 7.98 (s, 1 H) 7.58 (d, *J =* 1.31 Hz, 1 H) 7.53 (s, 1 H) 7.45 (s, 1 H) 7.23 (d, *J =* 1.31 Hz, 1 H) 1.91 (d, *J* = 7.51 Hz, 4 H) 1.73 - 1.84 (m, 6 H) 1.60 (d, *J* = 2.86 Hz, 2 H) 1.24 (s, 2 H)

### E-30: 4-(1-((6-((6-azaspiro[3.4]octan-6-yl)methyl)imidazo[1,2-a]pyridin-2-yl)methyl)-1H-1,2,3-triazol-4-yl)-6-(methylthio)-1H-indazole

A colorless oily compound **E-30** (13.2 mg, 45.5%) was prepared from intermediate **E30-2** using the method described in compound **E-3.**

LC-MS: [M+H]⁺= 485.2. ¹H NMR (400 MHz, DMSO-*d*₆): δ 13.11 (s, 1 H) 8.93 (s, 1 H) 8.52 (s, 1 H) 8.45 (s, 1 H) 7.98 (s, 1 H) 7.52 (d, J = 1.34 Hz, 1 H) 7.46 (d, J = 9.29 Hz, 1 H) 7.28 (s, 1 H) 7.22 (dd, J = 9.29, 1.47 Hz, 1 H) 5.78 (s, 2 H) 3.52 (s, 2 H) 3.33 (s, 2 H) 2.58 (s, 3 H) 1.87 - 1.97 (m, 4 H) 1.81 (t, J = 6.97 Hz, 2 H) 1.68 - 1.77 (m, 2 H).

### Example 31: Synthesis of E-31: 4-(1-((6-((6-azaspiro[3.4]octan-6-yl)methyl)imidazo[1,2-a]pyridin-2-yl)methyl)-1H-1,2,3-triazole-4-yl)-N,n-dimethyl-1H-indazole-6-amine

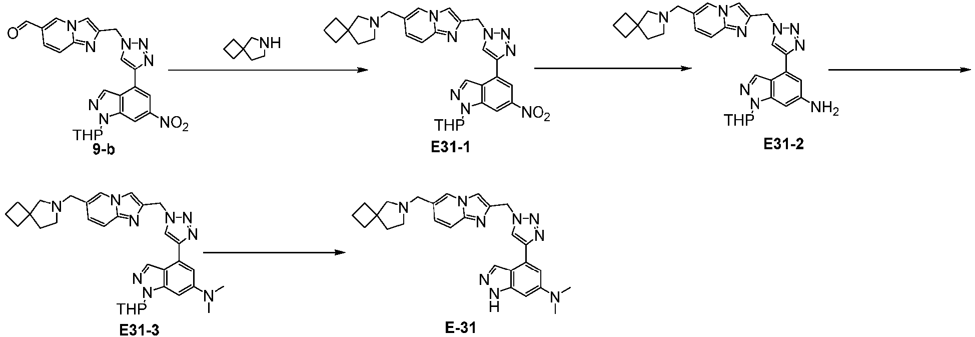

### E31-1: 4-(1-((6-((6-azaspiro[3.4]octan-6-yl)methyl)imidazo[1,2-a]pyridin-2-yl)methyl)-1H-1,2,3-triazol-4-yl)-6-nitro-1-(tetrahydro-2H-pyran-2-yl)-1H-indazole

A yellow solid intermediate **E31-1** (0.29 g, 34.4%) was prepared from intermediate **9-b** using the method described in intermediate **E30-1.**

LC-MS: [M+H]⁺= 568.4.

### E31-2: 4-(1-((6-((6-azaspiro[3.4]octan-6-yl)methyl)imidazo[1,2-a]pyridin-2-yl)methyl)-1H-1,2,3-triazol-4-yl)-1-(tetrahydro-2H-pyran-2-yl)-1H-indazole-6-amine

A yellow solid intermediate **E31-2** (30 mg, 34.4%) was prepared from intermediate **E31-1** using the method described in intermediate **E3-2.**

LC-MS: [M+H]⁺= 538.5.

### E31-3: 4-(1-((6-((6-azaspiro[3.4]octan-6-yl)methyl)imidazo[1,2-a]pyridin-2-yl)methyl)-1H-1,2,3-triazol-4-yl)-N,n-dimethyl-1-(tetrahydro-2H-pyran-2-yl)-1H-indazole-6-amine

A yellow oily intermediate **E31-3** (crude product) was prepared from intermediate **E31-2** using the method described in intermediate **E5-1.**

**LC-MS:** [M+H]⁺= 566.5.

### E-31: 4-(1-((6-((6-azaspiro[3.4]octan-6-yl)methyl)imidazo[1,2-a]pyridin-2-yl)methyl)-1H-1,2,3-triazole-4-yl)-N,n-dimethyl-1H-indazole-6-amine

A yellow oily compound **E-31** (23.3 mg, 20.7%) was prepared from intermediate **E31-3** using the method described in compound **E-3.**

LC-MS: [M+H]⁺= 482.1. ¹H NMR (400 MHz, DMSO-*d*₆): δ 12.52 - 12.80 (m, 1 H) 9.82 - 10.01 (m, 1 H) 8.90 (s, 1 H) 8.73 (s, 1 H) 8.32 (s, 1 H) 8.15 (s, 1 H) 7.67 (d, *J =* 9.38 Hz, 1 H) 7.43 (d, *J =* 9.26 Hz, 1 H) 7.29 (d, *J =* 1.50 Hz, 1 H) 6.58 (br s, 1 H) 5.84 (s, 2 H) 4.32 - 4.40 (m, 2 H) 3.42 (dd, *J* = 11.57, 5.44 Hz, 2 H) 3.16 - 3.23 (m, 1 H) 3.12 (dd, *J* = 11.57, 7.07 Hz, 1 H) 3.01 (s, 6 H) 2.07 - 2.21 (m, 2 H) 2.00 - 2.06 (m, 2 H) 1.95 - 2.00 (m, 1 H) 1.86 - 1.92 (m, 1 H) 1.71 - 1.86 (m, 2 H).

### Example 32: Synthesis of E-32: 1-cyclobutyl-N-((6-((4-(6-nitro-1H-indazol-4-yl)-1H-1,2,3-triazol-1-yl)methyl)-1H-indole-2-yl)methyl)methylamine

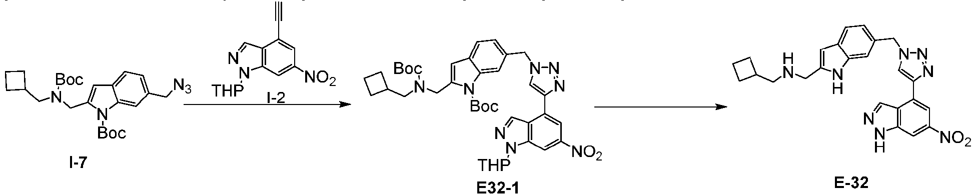

### E32-1: Tert-butyl 2-(((tert-butoxycarbonyl)(cyclobutylmethyl)amino)methyl)-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E32-1** (0.07 g, 73.9%) was prepared from intermediate **I-7** using the method described in intermediate **4-d.**

LC-MS: [M+H]⁺= 741.4.

### E-32: 1-cyclobutyl-N-((6-((4-(6-nitro-1H-indazol-4-yl)-1H-1,2,3-triazol-1-yl)methyl)-1H-indole-2-yl)methyl)methylamine

A yellow solid compound **E-32** (28.1 mg, 52.2%) was prepared from intermediate **E32-1** using the method described in compound **E-1.**

LC-MS: [M+H]⁺= 457.1. ¹H NMR (400 MHz, DMSO-*d*₆): δ 13.96 (s, 1H), 11.35 (s, 1H), 9.20 (s, 1H), 9.03 (s, 2H), 8.80 (s, 1H), 8.39 (s, 2H), 7.55 (d, *J =* 8.3 Hz, 1H), 7.49 (s, 1H), 7.12 (d, *J* = 8.1 Hz, 1H), 6.58 (s, 1H), 5.76 (s, 2H), 4.24 (t, *J =* 5.0 Hz, 2H), 2.93 (d, *J* = 5.5 Hz, 2H), 2.63 - 2.54 (m, 1H), 2.05 - 1.96 (m, 2H), 1.87 - 1.70 (m, 4H).

### Example 33: Synthesis of E-33: 4-(1-((2-(((cyclobutylmethyl)amino)methyl)-1H-indole-6-yl)methyl)-1H-1,2,3-triazol-4-yl)-1H-indazole-6-amine

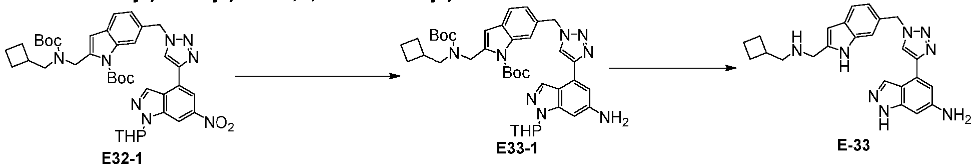

### E33-1: Tert-butyl 6-((4-(6-amino-1-(tetrahydro-2H-pyran-2-yl)-1H-indazol-4-yl)-1H-1,2,3-triazol-1-yl)methyl)-2-(((tert-butoxycarbonyl)(cyclobutylmethyl)amino)methyl)-1H-indole-1-carboxylate

A yellow solid intermediate **E33-1** (0.07 g, 73.9%) was prepared from intermediate **E32-1** using the method described in intermediate **E3-2.**

LC-MS: [M+H]⁺= 711.4.

### E-33: 4-(1-((2-(((cyclobutylmethyl)amino)methyl)-1H-indole-6-yl)methyl)-1H-1,2,3-triazol-4-yl)-1H-indazole-6-amine

A brown solid compound **E-33** (4.00 mg, 12.3%) was prepared from intermediate **E33-1** using the method described in compound **E-1.**

LC-MS: [M+H]⁺= 427.2. ¹H NMR (400 MHz, DMSO-*d*₆): δ 11.44 (s, 1H), 9.18 (s, 2H), 8.95 (s, 1H), 8.49 (s, 1H), 7.54 (d, *J =* 8.3 Hz, 1H), 7.50 - 7.44 (m, 2H), 7.28 (s, 1H), 7.11 (d, *J* = 8.1 Hz, 1H), 6.58 (s, 1H), 5.73 (s, 2H), 4.27 - 4.21 (m, 2H), 2.94 - 2.89 (m, 2H), 2.64 - 2.58 (m, 1H), 2.04 - 1.96 (m, 2H), 1.82 - 1.69 (m, 4H).

### Example 34: Synthesis of E-34: 1-(6-((4-(6-bromo-1H-indazol-4-yl)-1H-1,2,3-triazol-1-yl)methyl)-1H-indole-2-yl)-N-(cyclobutylmethyl)methylamine

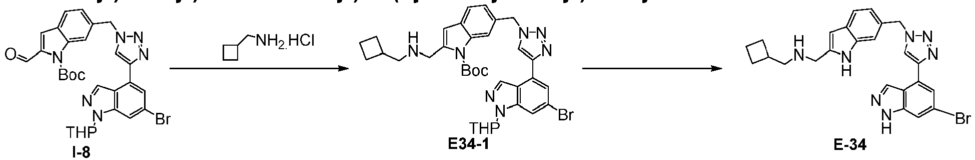

### E34-1: Tert-butyl 6-((4-(6-bromo-1-(tetrahydro-2H-pyran-2-yl)-1H-indazol-4-yl)-1H-1,2,3-triazol-1-yl)methyl)-2-(((cyclobutylmethyl)amino)methyl)-1H-indole-1-carboxylate

A yellow solid intermediate **E34-1** (200 mg, 59.8%) was prepared from intermediate **I-8** using the method described in intermediate **E30-1.**

LC-MS: [M+H]⁺= 674.6.

### E-34: 1-(6-((4-(6-bromo-1H-indazol-4-yl)-1H-1,2,3-triazol-1-yl)methyl)-1H-indole-2-yl)-N-(cyclobutylmethyl)methylamine

1,4-dioxane (4 M, 222 µL, 20 eq) of HCl was added to a solution of intermediate **E34-1** (30.0 mg, 44.5 µmol, 1.00 eq) in 1,4-dioxane (1.00 mL), and the mixture was stirred at 25°C for 2 h. The mixture was concentrated and purified by Prep-HPLC to obtain a yellow oily compound **E-34** (12.2 mg, 50.3%).

LC-MS: [M+H]⁺= 490.4. ¹H NMR (400 MHz, DMSO-*d*₆): δ 13.37 (br s, 1H), 11.41 (s, 1H), 9.14 (br s, 2H), 9.02 (s, 1H), 8.59 (s, 1H), 7.79 - 7.68 (m, 2H), 7.55 (d, *J* = 8.1 Hz, 1H), 7.47 (s, 1H), 7.10 (d, *J =* 8.3 Hz, 1H), 6.58 (s, 1H), 5.73 (s, 2H), 4.31 - 4.18 (m, 2H), 3.00 - 2.86 (m, 2H), 2.62 (d, *J* = 7.9, 15.8 Hz, 1H), 2.05 - 1.97 (m, 2H), 1.88 - 1.79 (m, 1H), 1.78 - 1.69 (m, 3H).

### Example 35: Synthesis of E-35: 5-(dimethylamino)-N-((6-(((((3-fluorobicyclic[1.1.1]pentan-1-yl)methyl)amino)methyl)imidazo[1,2-a]pyridin-2-yl)methyl)nicotinamide

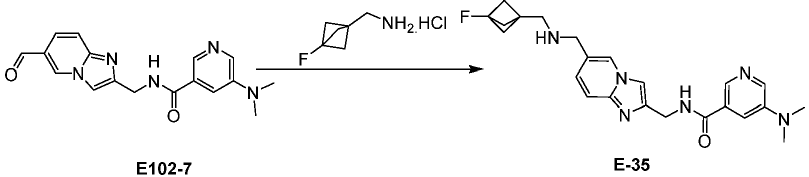

A yellow solid **E-35** (7.0 mg, 26.2 %) was prepared from **E102-7** using the method described in **E30-1.**

LC-MS: [M+H]⁺= 423.1. ¹H NMR (400 MHz, DMSO-*d*₆): *δ* 9.10 (t, *J* = 5.7 Hz, 1H), 8.36 (s, 2H), 8.22 (d, *J =* 2.9 Hz, 1H), 7.76 (s, 1H), 7.50-7.46 (m, 1H), 7.42 (d, *J =* 9.1 Hz, 1H), 7.20 (dd, *J* = 1.4, 9.2 Hz, 1H), 4.56 (d, *J =* 5.8 Hz, 2H), 3.66 (s, 2H), 2.97 (s, 6H), 2.74 (s, 2H), 1.92 (d, *J* = 2.6 Hz, 6H).

### Example 36: Synthesis of E-36: N-((6-(((cyclobutylmethyl)amino)methyl)imidazo[1,2-a]pyridin-2-yl)methyl)-5-(dimethylamino)nicotinamide

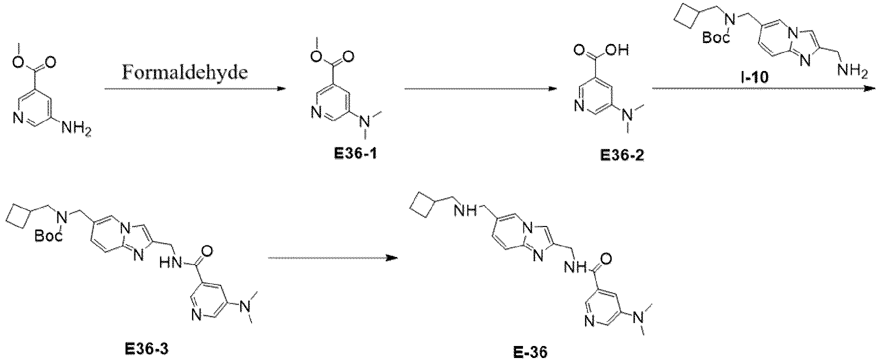

### E36-1: Methyl 5-(dimethylamino)nicotinate

A yellow oily intermediate **E36-1** (2.20 g, 41.3%) was prepared from methyl 5-amino-nicotinate using the method described in intermediate **E5-1.**

LC-MS: [M+H]⁺= 181.0.

### E36-2: 5-(dimethylamino)nicotinic acid

A yellow solid intermediate **E36-2** (200 mg, 68.4%) was prepared from intermediate **E36-1** using the method described in intermediate **E7-2.**

LC-MS: [M+H]⁺= 167.1.

### E36-3: Tert-butyl (cyclobutylmethyl)((2-((5-(dimethylamino)nicotinamido)methyl)imidazo[1,2-a]pyridin-6-yl)methyl)carbamate

A yellow solid intermediate **E36-3** (40.0 mg, 59.4%) was prepared from intermediates **E36-2** and **I-10** using the method described in intermediate **E7-3.**

LC-MS: [M+H]⁺= 493.3.

### E-36: N-((6-(((cyclobutylmethyl)amino)methyl)imidazo[1,2-a]pyridin-2-yl)methyl)-5-(dimethylamino)nicotinamide

A white solid compound **E-36** (9.20 mg, 28.8%) was prepared from intermediate **E36-3** using the method described in compound **E-3.**

LC-MS: [M+H]⁺= 393.2. ¹H NMR (400 MHz, DMSO-*d*₆):δ 9.10 (t, *J* = 5.69 Hz, 1 H) 8.36 (d, *J =* 1.63 Hz, 2 H) 8.21 (d, *J =* 2.88 Hz, 1 H) 7.77 (s, 1 H) 7.46 - 7.50 (m, 1 H) 7.43 (d, *J* = 9.13 Hz, 1 H) 7.21 (d, *J* = 9.26 Hz, 1 H) 4.56 (d, *J* = 5.63 Hz, 2 H) 3.66 (s, 2 H) 2.97 (s, 5 H) 2.94 (br s, 1 H) 2.52 (br d, *J* = 1.75 Hz, 1 H) 2.52 - 2.53 (m, 1 H) 2.33 - 2.44 (m, 1 H) 1.91 - 2.01 (m, 2 H) 1.71 - 1.86 (m, 2 H) 1.54 - 1.68 (m, 2 H).

### Example 37: Synthesis of E-37: 1-cyclobutyl-N-((2-((4-(5-methoxypyridin-3-yl)-1H-1,2,3-triazol-1-yl)methyl)imidazo[1,2-a]pyridin-6-yl)methyl)methylamine

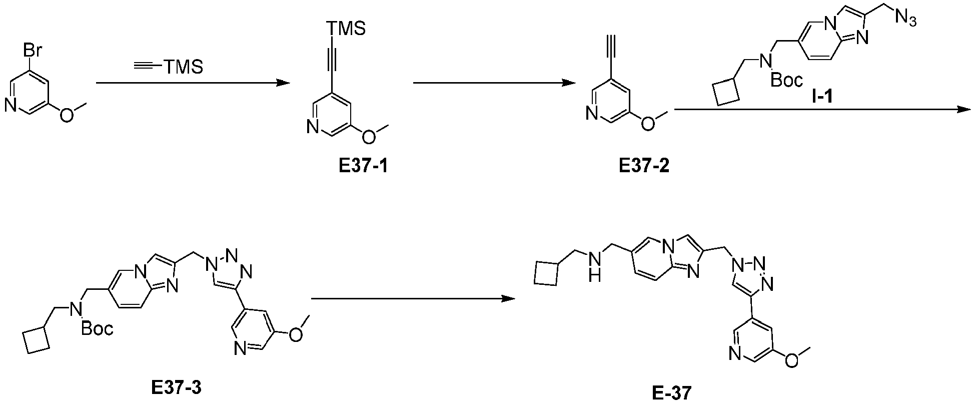

### E37-1: 3-methoxy-5-((trimethylsilyl)ethynyl)pyridine

A red oily intermediate **E37-1** (2.00 g, 91.5%) was prepared from 3-bromo-5-methoxypyridine using the method described in intermediate **2-b.**

LC-MS: [M+H]⁺= 206.1.

### E37-2: 3-ethynyl-5-methoxypyridine

A yellow solid intermediate **E37-2** (0.90 g, 69.0%) was prepared from intermediate **E37-1** using the method described in intermediate **I-2.**

¹H NMR: (400 MHz, CDCl₃-*d*): *δ* 8.32 (dd, *J* = 2.1, 19.0 Hz, 2H), 7.29 - 7.28 (m, 1H), 3.87 (s, 3H), 3.22 (s, 1H).

### E37-3: Tert-butyl (2-((4-(5-methoxypyridin-3-yl)-1H-1,2,3-triazol-1-yl)methyl)imidazo[1,2-a]pyridin-6-yl)methyl)carbamate

A yellow solid intermediate **E37-3** (0.06 g, 44.1%) was prepared from intermediate **E37-2** using the method described in intermediate **4-d.**

LC-MS: [M+H]⁺= 504.4.

### E-37: 1-cyclobutyl-N-((2-((4-(5-methoxypyridin-3-yl)-1H-1,2,3-triazol-1-yl)methyl)imidazo[1,2-a]pyridin-6-yl)methyl)methylamine

A yellow solid compound **E-37** (26.5 mg, 50.4%) was prepared from intermediate **E37-3** using the method described in compound **E-1.**

LC-MS: [M+H]⁺= 404.2. ¹H NMR (400 MHz, DMSO-*d*₆): δ 9.62 (br s, 2H), 9.07 - 8.99 (m, 2H), 8.82 (d, *J =* 1.2 Hz, 1H), 8.48 (s, 2H), 8.15 (br s, 1H), 8.06 (d, *J =* 9.2 Hz, 1H), 7.92 (d, *J* = 9.4 Hz, 1H), 6.04 (s, 2H), 4.23 (br s, 2H), 3.97 (s, 3H), 2.95 (d, *J =* 4.5 Hz, 2H), 2.76 - 2.66 (m, 1H), 2.10 - 2.00 (m, 2H), 1.90 - 1.74 (m, 4H).

### Example 38: Synthesis of E-38: 5-(1-((6-(((cyclobutylmethyl)amino)methyl)imidazo[1,2-a]pyridin-2-yl)methyl)-1H-1,2,3-triazole-4-yl)-N,n-dimethylpyridin-3-amine

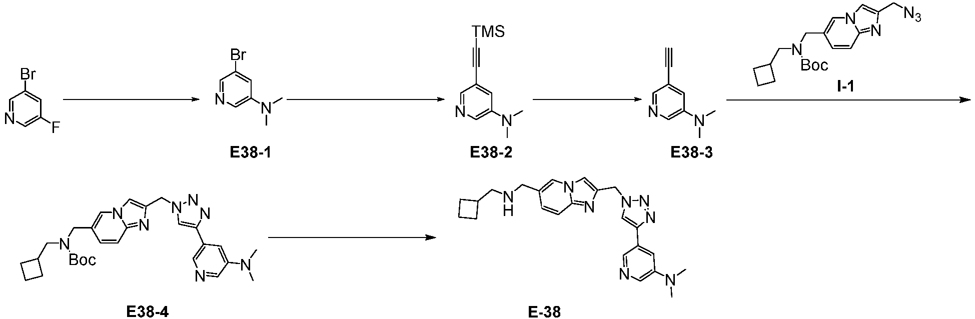

### E38-1: 5-bromo-N,N-dimethylpyridine-3-amine

A yellow solid and red oily intermediate **E38-1** (4.34 g, 76.0%) was prepared from 3-bromo-5-fluoropyridine using the method described in intermediate **E16-1.**

LC-MS: [M+H]⁺= 203.0.

### E38-2: N,N-dimethyl-5-((trimethylsilyl)ethynyl)pyridine-3-amine

A brown solid intermediate **E38-2** (1.37 g, 63.1%) was prepared from intermediate **E38-1** using the method described in intermediate **2-b.**

LC-MS: [M+H]⁺= 219.2.

### E38-3: 5-ethynyl-N,N-dimethylpyridine-3-amine

A light yellow solid intermediate **E38-3** (800 mg, 87.2%) was prepared from intermediate **E38-2** using the method described in intermediate **I-2.**

LC-MS: [M+H]⁺= 147.2.

### E38-4: Tert-butyl (2-((4-(5-(dimethylamino)pyridin-3-yl)-1H-1,2,3-triazol-1-yl)methyl)imidazo[1,2-a]pyridin-6-yl)methyl)carbamate

A black solid intermediate **E38-4** (0.22 g, crude product) was prepared from intermediate **E38-3** using the method described in intermediate **4-d.**

LC-MS: [M+H]⁺= 517.5.

### E-38: 5-(1-((6-(((cyclobutylmethyl)amino)methyl)imidazo[1,2-a]pyridin-2-yl)methyl)-1H-1,2,3-triazole-4-yl)-N,n-dimethylpyridin-3-amine

A yellow solid compound **E-38** (26.2 mg, 36.0%) was prepared from intermediate **E38-4** using the method described in compound **E-3.**

LC-MS: [M+H]⁺= 417.5. ¹H NMR (400 MHz, DMSO-*d*₆): δ 9.08 (s, 1 H) 9.00 (s, 1 H) 8.54 (br s, 1 H) 8.50 (s, 1 H) 8.15 - 8.21 (m, 2 H) 8.12 (d, *J* = 2.50 Hz, 1 H) 8.01 (d, *J* = 9.38 Hz, 1 H) 6.14 (s, 2 H) 4.41 - 4.42 (m, 1 H) 4.42 (s, 1 H) 3.20 (s, 6 H) 3.18 (s, 2 H) 2.76 (dt, *J* = 15.26, 7.75 Hz, 1 H) 2.18 - 2.24 (m, 2 H) 1.93 - 2.05 (m, 2 H) 1.87 - 1.92 (m, 2 H).

### Example 39: Synthesis of E-39: 1-(3-fluorobicyclo[1.1.1]pentan-1-yl)-N-((2-((4-(6-(methylsilyl)-1H-indazol-4-yl)-1H-1,2,3-triazol-1-yl)methyl)imidazo[1,2-a]pyridin-6-yl)methyl)methylamine

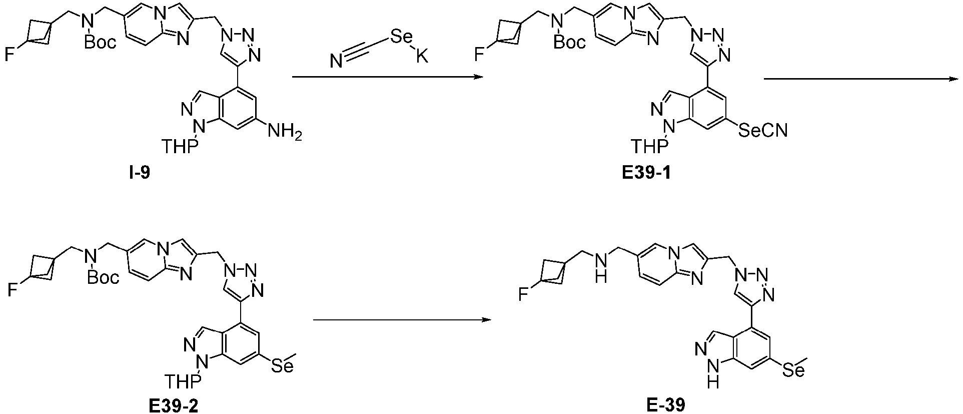

### E39-1: Tert-butyl ((3-fluorobicyclo[1.1.1]pentan-1-yl)methyl)((2-((4-(6-selecyano-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A red solid intermediate **E39-1** (220 mg, crude product) was prepared from intermediate **I-9** using the method described in intermediate **E14-1.**

LC-MS: [M+H]⁺= 732.2.

### E39-2: Tert-butyl ((3-fluorobicyclo[1.1.1]pentan-1-yl)methyl)((2-((4-(6-(methylsilyl)-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **E39-2** (40.0 mg, 14.3%) was prepared from intermediate **E39-1** using the method described in intermediate **E14-2.**

LC-MS: [M+H]⁺= 721.2.

### E-39: 1-(3-fluorobicyclo[1.1.1]pentan-1-yl)-N-((2-((4-(6-(methylsilyl)-1H-indazol-4-yl)-1H-1,2,3-triazol-1-yl)methyl)imidazo[1,2-a]pyridin-6-yl)methyl)methylamine

A yellow solid compound **E-39** (10.2 mg, 27.6%) was prepared from intermediate **E39-2** using the method described in compound **E-3.**

LC-MS: [M+H]⁺= 537.0. ¹H NMR (400 MHz, DMSO-*d*₆): δ 9.81 (br s, 2 H) 9.03 - 9.14 (m, 2 H) 8.53 (d, *J* = 2.13 Hz, 2 H) 8.14 (d, *J* = 9.26 Hz, 1 H) 7.97 (d, *J* = 9.26 Hz, 1 H) 7.65 (s, 1 H) 7.49 (s, 1 H) 6.06 (s, 2 H) 4.28 (br s, 2 H) 3.24 - 3.29 (m, 2 H) 2.46 (s, 3 H) 2.12 (d, *J* = 2.38 Hz, 6 H).

### Example 40: Synthesis of E-40: 1-cyclobutyl-N-((6-((4-(6-(methylsilyl)-1H-indazol-4-yl)-1H-1,2,3-triazol-1-yl)methyl)-1H-indole-2-yl)methyl)methylamine

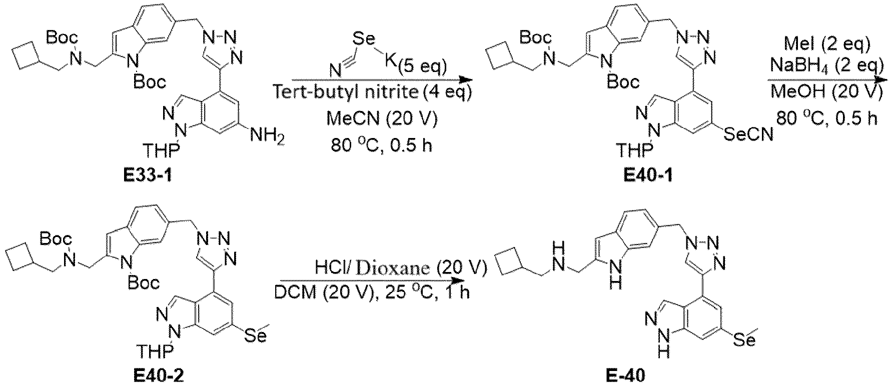

### E40-1: Tert-butyl 2-(((tert-butoxycarbonyl)(cyclobutylmethyl)amino)methyl)-6-((4-(6-selecyano-1-(tetrahydro-2H-pyran-2-yl)-1H-indazol-4-yl)-1H-1,2,3-triazol-1-yl)methyl)-1H-indole-1-carboxylate

A red solid intermediate **E40-1** (220 mg, crude product) was prepared from intermediate **E33-1** using the method described in intermediate **E14-1.**

LC-MS: [M+H]⁺= 801.3.

### E40-2: Tert-butyl 2-(((tert-butoxycarbonyl)(cyclobutylmethyl)amino)methyl)-6-((4-(6-(methylsilyl)-1-(tetrahydro-2H-pyran-2-yl)-1H-indazol-4-yl)-1H-1,2,3-triazol-1-yl)methyl)-1H-indole-1-carboxylate

A yellow solid intermediate **E40-2** (30.0 mg, 15.2%) was prepared from intermediate **E40-1** using the method described in intermediate **E14-2.**

LC-MS: [M+H]⁺=790.5.

### E-40: 1-cyclobutyl-N-((6-((4-(6-(methylsilyl)-1H-indazol-4-yl)-1H-1,2,3-triazol-1-yl)methyl)-1H-indole-2-yl)methyl)methylamine

A yellow solid compound **E-40** (11.8 mg, 55.6%) was prepared from intermediate **E40-2** using the method described in compound **E-3.**

LC-MS: [M+H]⁺= 505.9. ¹H NMR (400 MHz, DMSO-*d*₆): δ 12.98 - 13.32 (m, 1 H) 11.29 - 11.47 (m, 1 H) 8.99 - 9.15 (m, 2 H) 8.97 (s, 1 H) 8.52 (s, 1 H) 7.63 (d, *J* = 1.07 Hz, 1 H) 7.57 (d, *J* = 8.23 Hz, 1 H) 7.47 (d, *J* = 12.52 Hz, 2 H) 7.12 (d, *J* = 8.23 Hz, 1 H) 6.60 (s, 1 H) 5.75 (s, 2 H) 4.26 (t, *J* = 5.07 Hz, 2 H) 2.96 (d, *J* = 4.77 Hz, 2 H) 2.58 - 2.64 (m, 1 H) 2.40 - 2.46 (m, 3 H) 1.99 - 2.11 (m, 2 H) 1.70 - 1.89 (m, 4 H).

### Example 41: Synthesis of E-41: 1-cyclobutyl-N-((6-((4-(6-methoxy-1H-indazol-4-yl)-1H-1,2,3-triazol-1-yl)methyl)-1H-indole-2-yl)methyl)methylamine

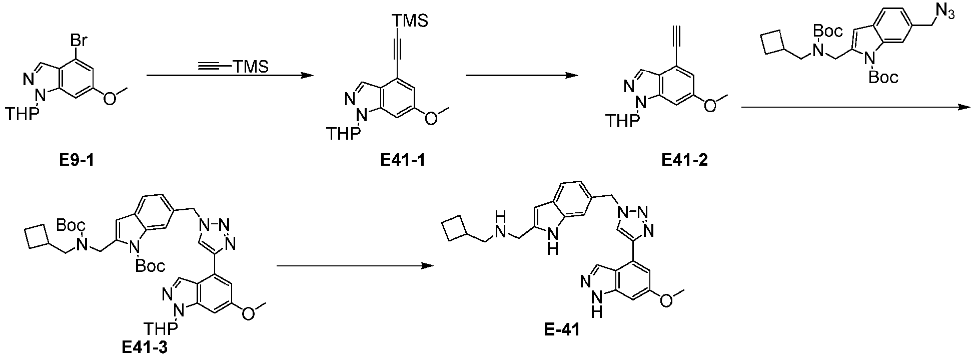

### E41-1: 6-methoxy-1-(tetrahydro-2H-pyran-2-yl)-4-((trimethylsilyl)ethynyl)-1H-indazole

A red oily intermediate **E41-1** (0.06 g, 56.8%) was prepared from intermediate **E9-1** using the method described in intermediate **2-b.**

LC-MS: [M+H]⁺= 329.2.

### E41-2: 4-ethynyl-6-methoxy-1-(tetrahydro-2H-pyran-2-yl)-1H-indazole

A yellow solid intermediate **E41-2** (20.0 mg, 42.7%) was prepared from intermediate **E41-1** using the method described in intermediate **I-2.**

¹H NMR: (400 MHz, CDCl₃): δ 7.95 (d, *J =* 6.8 Hz, 1H), 6.91 (dd, *J* = 5.6, 12.2 Hz, 2H), 5.57 (s, 1H), 3.91 (s, 1H), 3.81 (d, *J* = 7.4 Hz, 3H), 3.66 (s, 1H), 3.24 (d, *J =* 7.4 Hz, 1H), 2.48 (br s, 1H), 2.08 (br s, 1H), 2.14 - 2.05 (m, 1H), 1.99 (d, *J =* 12.6 Hz, 1H), 1.18 (d, *J =* 5.5 Hz, 2H).

### E41-3: Tert-butyl 2-(((tert-butoxycarbonyl)(cyclobutylmethyl)amino)methyl)-6-((4-(6-methoxy-1-(tetrahydro-2H-pyran-2-yl)-1H-indazol-4-yl)-1H-1,2,3-triazol-1-yl)methyl)-1H-indole-1-carboxylate

A yellow oily intermediate **E41-3** (0.04 g, 64.7%) was prepared from intermediate **E41-2** using the method described in intermediate **4-d.**

LC-MS: [M+H]⁺= 726.5.

### E-41: 1-cyclobutyl-N-((6-((4-(6-methoxy-1H-indazol-4-yl)-1H-1,2,3-triazol-1-yl)methyl)-1H-indole-2-yl)methyl)methylamine

A yellow solid compound **E-41** (18.6 mg, 70.1%) was prepared from intermediate **E41-3** using the method described in compound **E-1.**

LC-MS: [M+H]⁺= 442.2. ¹H NMR (400 MHz, DMSO-*d*₆): δ 11.43 (s, 1H), 9.16 (br s, 2H), 8.94 (s, 1H), 8.45 (s, 1H), 7.57 (d, *J =* 8.2 Hz, 1H), 7.49 (s, 1H), 7.26 (d, *J =* 2.0 Hz, 1H), 7.12 (d, *J* = 8.1 Hz, 1H), 6.92 (s, 1H), 6.61 (s, 1H), 5.75 (s, 2H), 4.30 - 4.24 (m, 1H), 4.27 (t, *J* = 5.1 Hz, 1H), 3.85 (s, 3H), 2.99 - 2.92 (m, 2H), 2.66 - 2.59 (m, 1H), 2.09 - 2.00 (m, 2H), 1.92 - 1.82 (m, 1H), 1.81 - 1.72 (m, 3H).

### Example 42: Synthesis of E-42: 1-(3-fluorobicyclo[1.1.1]pentan-1-yl)-N-((6-((4-(6-nitro-1H-indazol-4-yl)-1H-1,2,3-triazol-1-yl)methyl)-1H-indole-2-yl)methyl)methanamine

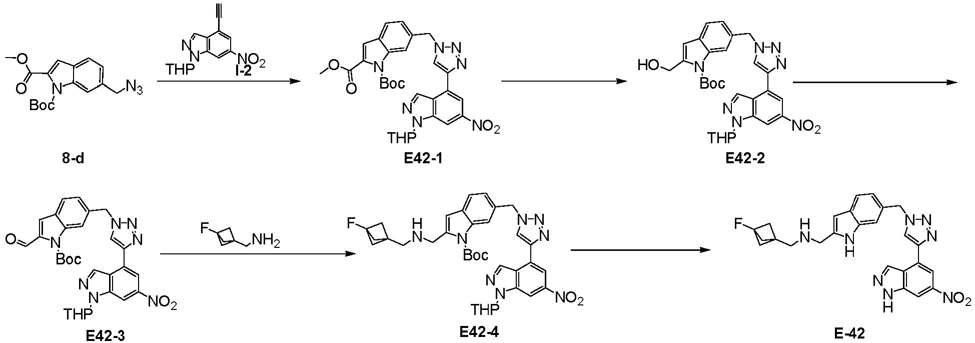

### E42-1: 1-(tert-butyl)2-methyl 6-((4-(6-nitro-1-(tetrahydro-2H-pyran-2-yl)-1H-indazol-4-yl)-1H-1,2,3-triazol-1-yl)methyl)-1H-indole-1,2-dicarboxylate

A yellow solid intermediate **E42-1** (1.50 g, 78.4%) was prepared from intermediate **8-d** using the method described in intermediate **4-d.**

LC-MS: [M+H]⁺= 602.5.

### E42-2: Tert-butyl 2-(hydroxymethyl)-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E42-2** (0.650 g, 48.8%) was prepared from intermediate **E42-1** using the method described in intermediate **4-b.**

¹H NMR (400 MHz, DMSO-*d*₆): δ 9.22 (s, 1 H) 8.86 (s, 1 H) 8.72 (s, 1 H) 8.47 (d, *J* = 1.75 Hz, 1 H) 8.09 (s, 1 H) 7.59 (d, *J =* 7.88 Hz, 1 H) 7.32 (dd, *J* = 8.00, 1.38 Hz, 1 H) 6.67 (s, 1 H) 6.18 (dd, *J* = 9.38, 2.13 Hz, 1 H) 5.83 (s, 2 H) 5.34 (t, *J* = 5.75 Hz, 1 H) 4.76 (d, *J* = 5.25 Hz, 2 H) 3.82 - 3.93 (m, 2 H) 2.45 (br s, 2 H) 2.02 - 2.08 (m, 2 H) 1.61 (br s, 2 H) 1.57 (s, 9 H).

### E42-3: Tert-butyl 2-formyl-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E42-3** (500 mg, 77.2%) was prepared from intermediate **E42-2** using the method described in intermediate **I-4.**

LC-MS: [M+H]⁺= 572.5.

### E42-4: Tert-butyl 2-((((3-fluorobicyclo[1.1.1]pentan-1-yl)methyl)amino)methyl)-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E42-4** (0.290 g, 49.4%) was prepared from intermediate **E42-3** using the method described in intermediate **E30-1.**

LC-MS: [M+H]⁺= 671.3.

### E-42: 1-(3-fluorobicyclo[1.1.1]pentan-1-yl)-N-((6-((4-(6-nitro-1H-indazol-4-yl)-1H-1,2,3-triazol-1-yl)methyl)-1H-indole-2-yl)methyl)methanamine

A yellow solid compound **E-42** (16.2 mg, 44.1%) was prepared from intermediate **E42-4** using the method described in compound **E-3.**

LC-MS: [M+H]⁺= 487.1. ¹H NMR (400 MHz, DMSO-*d*₆): δ 13.92 - 14.07 (m, 1 H) 11.42 (s, 1 H) 9.32 (br s, 2 H) 9.24 (s, 1 H) 8.82 (s, 1 H) 8.42 (s, 2 H) 7.58 (d, *J* = 8.19 Hz, 1 H) 7.52 (s, 1 H) 7.12 - 7.17 (m, 1 H) 6.61 (s, 1 H) 5.78 (s, 2 H) 4.31 (t, *J* = 4.71 Hz, 2 H) 3.24 - 3.30 (m, 2 H) 2.10 (d, *J* = 2.57 Hz, 6 H).

### Example 43: Synthesis of E-43: 4-(1-((2-((((3-fluorobicyclo[1.1.1]pentan-1-yl)methyl)amino)methyl)-1H-indole-6-yl)methyl)-1H-1,2,3-triazol-4-yl)-1H-indazole-6-amine

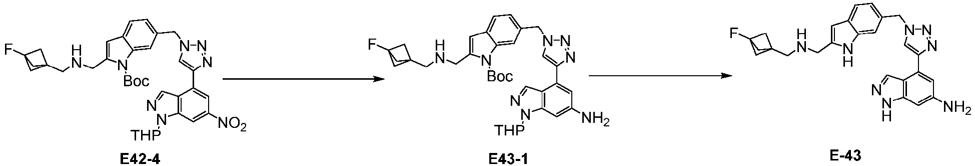

### E43-1: Tert-butyl 6-((4-(6-amino-1-(tetrahydro-2H-pyran-2-yl)-1H-indazol-4-yl)-1H-1,2,3-triazol-1-yl)methyl)-2-((((3-fluorobicyclo[1.1.1]pentan-1-yl)methyl)amino)-methyl)-1H-indole-1-carboxylate

A yellow solid intermediate **E43-1** (56.0 mg, 58.6%) was prepared from intermediate **E42-4** using the method described in intermediate **E3-2.**

LC-MS: [M+H]⁺= 641.5.

### E-43: 4-(1-((2-((((3-fluorobicyclo[1.1.1]pentan-1-yl)methyl)amino)methyl)-1H-indole-6-yl)methyl)-1H-1,2,3-triazol-4-yl)-1H-indazole-6-amine

A yellow oily compound **E-43** (12.4 mg, 30.1%) was prepared from intermediate **E43-1** using the method described in compound **E-3.**

LC-MS: [M+H]⁺= 457.1. ¹H NMR (400 MHz, DMSO-*d*₆): δ 11.57 (s, 1 H) 9.55 (br s, 2 H) 9.05 (s, 1 H) 8.60 (s, 1 H) 7.55 - 7.60 (m, 2 H) 7.50 - 7.55 (m, 2 H) 7.14 (dd, *J* = 8.19, 1.10 Hz, 1 H) 6.61 (s, 1 H) 5.77 (s, 2 H) 4.31 (br s, 2 H) 3.21 - 3.29 (m, 2 H) 2.11 (d, *J* = 2.57 Hz, 6 H).

### Example 44: Synthesis of E-44: 5-(1-((2-(((cyclobutylmethyl)amino)methyl)-1H-indole-6-yl)methyl)-1H-1,2,3-triazol-4-yl)-N,n-dimethylpyridine-3-amine

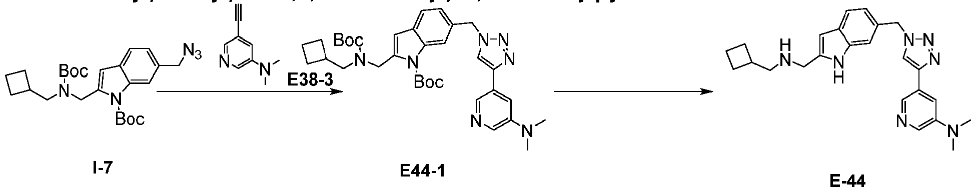

### E44-1: Tert-butyl 2-(((tert-butoxycarbonyl)(cyclobutylmethyl)amino)methyl)-6-((4-(5-(dimethylamino)pyridin-3-yl)-1H-1,2,3-triazol-1-yl)methyl)-1H-indole-1-carboxylate

A yellow solid intermediate **E44-1** (0.10 g, 76.2%) was prepared from intermediates **I-7** and **E38-3** using the method described in intermediate **4-d.**

LC-MS: [M+H]⁺= 616.6.

### E-44: 5-(1-((2-(((cyclobutylmethyl)amino)methyl)-1H-indole-6-yl)methyl)-1H-1,2,3-triazol-4-yl)-N,n-dimethylpyridine-3-amine

A yellow solid compound **E-44** (55.5 mg, 72.0%) was prepared from intermediate **E44-1** using the method described in compound **E-1.**

LC-MS: [M+H]⁺= 416.3. ¹H NMR (400 MHz, DMSO-*d*₆): δ 11.67 (s, 1H), 9.47 (s, 2H), 9.00 (s, 1H), 8.51 (s, 1H), 8.15 (d, *J =* 2.6 Hz, 1H), 8.06 (s, 1H), 7.58 (d, *J =* 8.2 Hz, 1H), 7.48 (s, 1H), 7.07 (d, *J =* 8.2 Hz, 1H), 6.62 (s, 1H), 5.79 (s, 2H), 4.28 (t, *J =* 5.0 Hz, 2H), 3.11 (s, 6H), 2.97 - 2.89 (m, 2H), 2.72 - 2.64 (m, 1H), 2.09 - 2.00 (m, 2H), 1.89 - 1.73 (m, 4H).

### Example 45: Synthesis of E-45: 4-(1-((2-((6-azaspiro[3.4]octan-6-yl)methyl)-1H-indole-6-yl)methyl)-1H-1,2,3-triazol-4-yl)-6-bromo-1H-indazole

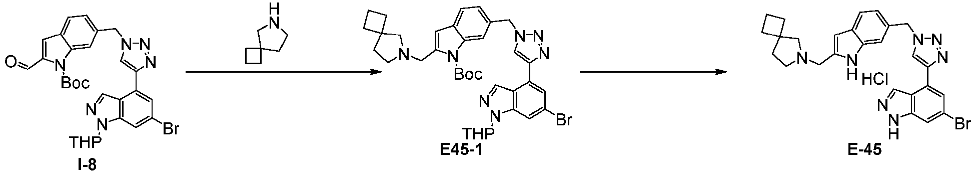

### E45-1: Tert-butyl 2-((6-azaspiro[3.4]octan-6-yl)methyl)-6-((4-(6-bromo-1-(tetrahydro-2H-pyran-2-yl)-1H-indazol-4-yl)-1H-1,2,3-triazol-1-yl)methyl)-1H-indole-1-carboxylate

A yellow solid intermediate **E45-1** (140 mg, 60.5%) was prepared from intermediate **I-8** using the method described in intermediate **E5-1.**

¹H NMR (400 MHz, chloroform-*d*): δ 8.59 (s, 1H), 8.09 (s, 1H), 7.80 (s, 1H), 7.71 (s, 1H), 7.54 (s, 1H), 7.50 (d, *J=* 8.3 Hz, 1H), 7.19 (d, *J= 8.3* Hz, 1H), 6.73 - 6.51 (m, 1H), 5.70 (s, 2H), 5.69 - 5.65 (m, 1H), 4.01 (d, *J =* 11.6 Hz, 1H), 3.80 - 3.67 (m, 1H), 2.68 (br s, 2H), 2.51 (d, *J* = 7.5 Hz, 2H), 2.13 (s, 1H), 2.00 - 1.87 (m, 7H), 1.76 (d, *J* = 9.4 Hz, 1H), 1.25 - 1.22 (m, 9H), 0.89 - 0.81 (m, 7H).

### E-45: 4-(1-((2-((6-azaspiro[3.4]octan-6-yl)methyl)-1H-indole-6-yl)methyl)-1H-1,2,3-triazol-4-yl)-6-bromo-1H-indazole

A white solid compound **E-45** (12.4 mg, 51.1%) was prepared from intermediate **E45-1** using the method described in compound **E-34.**

LC-MS: [M+H]⁺= 516.2. ¹H NMR (400 MHz, DMSO-*d*₆): δ 11.53 (s, 1H), 11.18 (s, 1H), 9.06 (s, 1H), 8.62 (d, *J =* 0.6 Hz, 1H), 7.81 - 7.70 (m, 2H), 7.59 (d, *J =* 8.1 Hz, 1H), 7.50 (s, 1H), 7.14 (dd, *J =* 1.2, 8.2 Hz, 1H), 6.68 (s, 1H), 5.77 (s, 2H), 4.54 - 4.38 (m, 2H), 3.48 - 3.33 (m, 2H), 3.20 - 3.11 (m, 2H), 2.15 - 2.05 (m, 2H), 2.04 - 1.92 (m, 3H), 1.89 - 1.70 (m, 3H).

### Example 46: Synthesis of E-46: 4-(1-((2-((6-azaspiro[3.4]octan-6-yl)methyl)-1H-indole-6-yl)methyl)-1H-1,2,3-triazole-4-yl)-6-nitro-1H-indazole

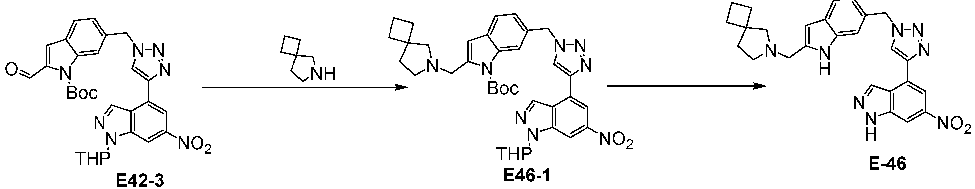

### E46-1: Tert-butyl 2-((6-azaspiro[3.4]octan-6-yl)methyl)-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E46-1** (0.907 g, 51.8%) was prepared from intermediate **E42-3** using the method described in intermediate **E30-1.**

**LC-MS:** [M+H]⁺= 667.5.

### E-46: 4-(1-((2-((6-azaspiro[3.4]octan-6-yl)methyl)-1H-indole-6-yl)methyl)-1H-1,2,3-triazole-4-yl)-6-nitro-1H-indazole

A yellow solid compound **E-46** (32.5 mg, 41.1%) was prepared from intermediate **E46-1** using the method described in compound **E-3.**

LC-MS: [M+H]⁺= 483.3. ¹H NMR (400 MHz, DMSO-*d*₆): δ 13.96 (s, 1 H) 11.36 (s, 1 H) 10.26 - 10.44 (m, 1 H) 9.23 (s, 1 H) 8.82 (s, 1 H) 8.42 (s, 2 H) 7.57 - 7.64 (m, 1 H) 7.50 (s, 1 H) 7.13 - 7.20 (m, 1 H) 6.66 (s, 1 H) 5.79 (s, 2 H) 4.46 (s, 2 H) 3.43 (d, *J* = 11.44, 5.44 Hz, 2 H) 3.22 (br s, 2 H) 2.05 - 2.11 (m, 2 H) 1.98 - 2.04 (m, 2 H) 1.88 (d, *J =* 5.63 Hz, 2 H) 1.85 (br s, 2 H).

### Example 47: Synthesis of E-47: 4-(1-((2-((6-azaspiro[3.4]octan-6-yl)methyl)-1H-indole-6-yl)methyl)-1H-1,2,3-triazole-4-yl)-6-(methylthio)-1H-indazole

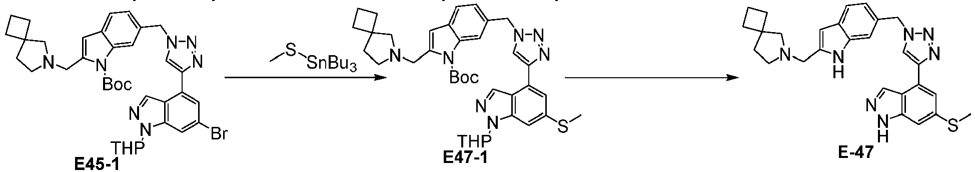

### E47-1: Tert-butyl 2-((6-azaspiro[3.4]octan-6-yl)methyl)-6-((4-(6-(methylthio)-1-(tetrahydro-2H-pyran-2-yl)-1H-indazol-4-yl)-1H-1,2,3-triazol-1-yl)methyl)-1H-indole-1-carboxylate

A yellow solid intermediate **E47-1** (10 mg, 15.0%) was prepared from intermediate **E45-1** using the method described in intermediate **E22-1.**

LC-MS: [M+H]⁺= 668.3.

### E-47: 4-(1-((2-((6-azaspiro[3.4]octan-6-yl)methyl)-1H-indole-6-yl)methyl)-1H-1,2,3-triazole-4-yl)-6-(methylthio)-1H-indazole

A white solid compound **E-47** (1.3 mg, 16.6%) was prepared from intermediate **E47-1** using the method described in compound **E-34.**

LC-MS: [M+H]⁺= 484.1. ¹H NMR (400 MHz, DMSO-*d*₆): δ 13.14 (br s, 1H), 11.41 (s, 1H), 10.62 (br s, 1H), 8.99 (s, 1H), 8.51 (s, 1H), 7.60 (d, *J* = 8.3 Hz, 1H), 7.51 (d, *J* = 1.1 Hz, 1H), 7.49 (s, 1H), 7.29 (s, 1H), 7.15 (d, *J=* 8.3 Hz, 1H), 6.67 (s, 1H), 5.76 (s, 2H), 4.54 - 4.40 (m, 2H), 3.44 (d, *J* = 6.1 Hz, 2H), 3.27 - 3.11 (m, 2H), 2.58 (s, 3H), 2.14 - 1.96 (m, 5H), 1.91 - 1.68 (m, 3H).

### Example 48: Synthesis of E-48: 4-(1-((6-(((cyclobutylmethyl)amino)methyl)imidazo[1,2-a]pyridin-2-yl)methyl)-1H-1,2,3-triazol-4-yl)-3-fluoro-N,n-dimethyl-1H-indazole-6-amine

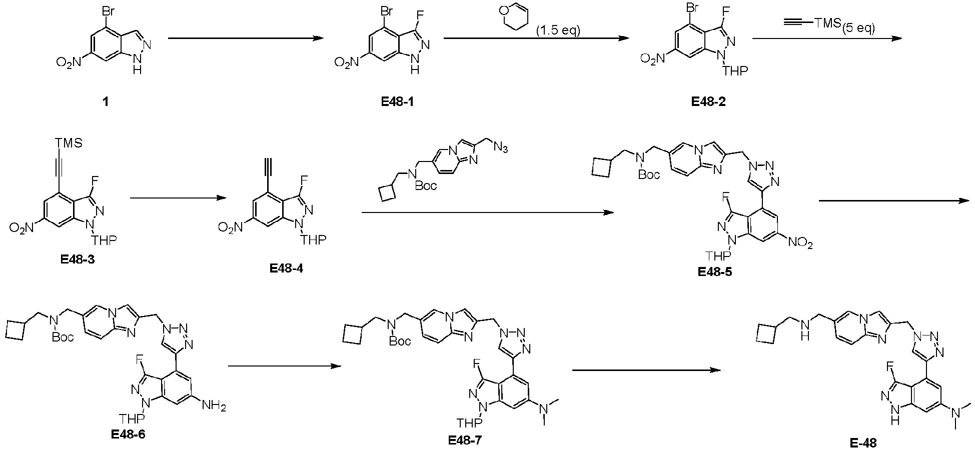

### E48-1: 4-bromo-3-fluoro-6-nitro-1H-indazole

A selective fluorine reagent (7.32 g, 20.7 mmol, 1.00 eq) was added to a solution of 4-bromo-6-nitro-1H-indazole (5.00 g, 20.7 mmol, 1.00 eq) in MeCN (50.0 mL) and AcOH (10.0 mL), the resulting mixture was stirred at 100°C for 16 h under nitrogen protection. The mixture was concentrated, and the residue was diluted with water (30.0 mL) and extracted with ethyl acetate (10.0 mL * 3). The combined organic layers were washed with saline (30 mL), dried over anhydrous sodium sulfate, filtered and concentrated. The residue was purified by silica gel column chromatography (petroleum ether/ethyl acetate (50/1 to 3/1)) to obtain a yellow solid intermediate **E48-1** (2.00 g, 37.2%).

LC-MS: [M-H]⁻= 259.0.

### E48-2: 4-bromo-3-fluoro-6-nitro-1-(tetrahydro-2H-pyran-2-yl)-1H-indazole

A yellow solid intermediate **E48-2** (1.10 g, 41.6%) was prepared from intermediate **E48-1** using the method described in intermediate **2-a.**

¹H NMR (400 MHz, chloroform-d): δ 8.45 (t, *J=* 1.75 Hz, 1 H) 8.19 (d, *J=* 1.63 Hz, 1 H) 5.68 (dt, *J* = 8.60, 2.20 Hz, 1 H) 3.96 - 4.01 (m, 1 H) 3.73 - 3.80 (m, 1 H) 2.36 - 2.45 (m, 1 H) 2.09 - 2.18 (m, 2 H) 1.68 - 1.76 (m, 3 H).

### E48-3: 3-fluoro-6-nitro-1-(tetrahydro-2H-pyran-2-yl)-4-((trimethylsilyl)ethynyl)-1H-indazole

A brown solid intermediate **E48-3** (200 mg, 54.0%) was prepared from intermediate **E48-2** using the method described in intermediate **2-b.**

LC-MS: [M+H]⁺= 362.1.

### E48-4: 4-ethynyl-3-fluoro-6-nitro-1-(tetrahydro-2H-pyran-2-yl)-1H-indazole

A yellow solid intermediate **E48-4** (130 mg, 81.2%) was prepared from intermediate **E48-3** using the method described in intermediate **I-2.**

LC-MS: [M+H]⁺= 290.0.

### E48-5: Tert-butyl (2-((4-(3-fluoro-6-nitro-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **E48-5** (150 mg, 50.6%) was prepared from intermediate **E48-4** using the method described in intermediate **4-d.**

LC-MS: [M+H]⁺= 660.3.

### E48-6: Tert-butyl (2-((4-(6-amino-3-fluoro-1-(tetrahydro-2H-pyran-2-yl)-1H-indazol-4-yl)-1H-1,2,3-triazol-1-yl)methyl)imidazo[1,2-a]pyridin-6-yl)methyl)(cyclobutylmethyl)carbamate

A yellow solid intermediate **E48-6** (100 mg, 69.8%) was prepared from intermediate **E48-5** using the method described in intermediate **E3-2.**

LC-MS: [M+H]⁺= 630.4.

### E48-7: Tert-butyl (cyclobutylmethyl)((2-((4-(6-(dimethylamino)-3-fluoro-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A yellow solid intermediate **E48-7** (60 mg, 57.4%) was prepared from intermediate **E48-6** using the method described in intermediate **E5-1.**

LC-MS: [M+H]⁺= 658.4.

### E-48: 4-(1-((6-(((cyclobutylmethyl)amino)methyl)imidazo[1,2-a]pyridin-2-yl)methyl)-1H-1,2,3-triazol-4-yl)-3-fluoro-N,n-dimethyl-1H-indazole-6-amine

A white solid compound **E-48** (24.6 mg, 52.5%) was prepared from intermediate **E48-7** using the method described in compound **E-1.**

LC-MS: [M+H]⁺= 474.3. ¹H NMR (400 MHz, DMSO-*d*₆): δ 12.44 (brs, 1 H) 9.68 (br s, 2 H) 9.07 (s, 1 H) 8.75 (s, 1 H) 8.52 (s, 1 H) 8.15 - 8.22 (m, 1 H) 7.99 (d, *J* = 9.26 Hz, 1 H) 7.50 (br s, 1 H) 6.66 - 6.98 (m, 1 H) 6.10 (s, 2 H) 4.24 (t, *J = 5.25* Hz, 2 H) 3.06 (s, 6 H) 2.90 - 2.97 (m, 2 H) 2.65 - 2.76 (m, 1 H) 2.00 - 2.09 (m, 2 H) 1.71 - 1.90 (m, 4 H).

### Example 49: Synthesis of E-49: 4-(1-((6-(6-(6-azaspiro[3.4]octan-6-yl)methyl)imidazo[1,2-a]pyridin-2-yl)methyl)-1H-1,2,3-triazole

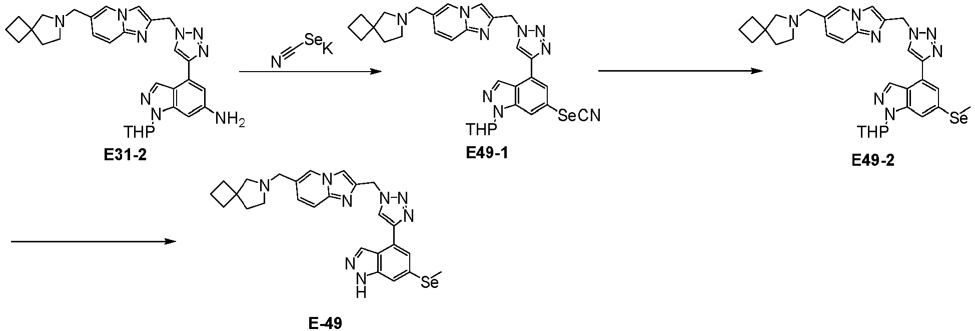

### E49-1: 4-(1-((6-((6-azaspiro[3.4]octan-6-yl)methyl)imidazo[1,2-a]pyridin-2-yl)methyl)-1H-1,2,3-triazol-4-yl)-6-selecyano-1-(tetrahydro-2H-pyran-2-yl)-1H-indazole

Intermediate **E31-2** (0.03 g, 55.8 µmol, 1.00 eq) was dissolved in HCl (8 M, 1.00 mL), and NaNO₂ (3 M, 37.2 µL, 2.00 eq) was added to the mixture. The resulting mixture was stirred at 0°C for 1 h. Then, saturated sodium acetate was added dropwise to the mixture, the pH was adjusted to 6 at 0°C, and potassium selenocyanate (32.2 mg, 223 µmol, 4.00 eq) was added. Next, the obtained mixture was stirred at 0°C for 10 min. The mixture was quenched with water (1.00 mL) at 0°C and extracted with EtOAc (2.00 mL * 3). The combined organic layers were dried over anhydrous sodium sulfate, filtered and concentrated to obtain a brown oily intermediate **E49-1** (0.03 g, crude product).

LC-MS: [M+H]⁺= 628.

### E49-2: 4-(1-((6-((6-azaspiro[3.4]octan-6-yl)methyl)imidazo[1,2-a]pyridin-2-yl)methyl)-1H-1,2,3-triazol-4-yl)-6-(methylsilyl)-1-(tetrahydro-2H-pyran-2-yl)-1H-indazole

A yellow solid intermediate **E49-2** (100 mg, 69.8%) was prepared from intermediate **E49-1** using the method described in intermediate **E14-2.**

LC-MS: [M+H]⁺= 616.

### E-49: 4-(1-((6-((6-azaspiro[3.4]octan-6-yl)methyl)imidazo[1,2-a]pyridin-2-yl)methyl)-1H-1,2,3-triazol-4-yl)-6-(methylsilyl)-1H-indazole

A yellow solid compound **E-49** (100 mg, 69.8%) was prepared from intermediate **E49-2** using the method described in compound **E-1.**

LC-MS: [M+H]⁺= 533.1. ¹H NMR (400 MHz, DMSO-*d*₆): δ 11.83 (br, J= 5.6 Hz, 1H), 9.28 - 9.02 (m, 2H), 8.68 - 8.47 (m, 2H), 8.28 (dd, *J* = 1.1, 9.4 Hz, 1H), 8.02 (d, *J* = 9.3 Hz, 1H), 7.64 (d, *J* = 1.1 Hz, 1H), 7.49 (s, 1H), 6.10 (s, 2H), 4.49 (br, *J* = 5.6, 13.9 Hz, 2H), 3.51 - 3.32 (m, 2H), 3.24 - 3.04 (m, 2H), 2.47 - 2.44 (m, 3H), 2.19 - 2.08 (m, 2H), 2.07 - 1.96 (m, 3H), 1.91 - 1.84 (m, 1H), 1.83 - 1.67 (m, 2H).

### Example 50: Synthesis of E-50: 1-cyclobutyl-N-((6-((4-(6-methyl-1H-indazol-4-yl)-1H-1,2,3-triazol-1-yl)methyl)-1H-indole-2-yl)methyl)methylamine

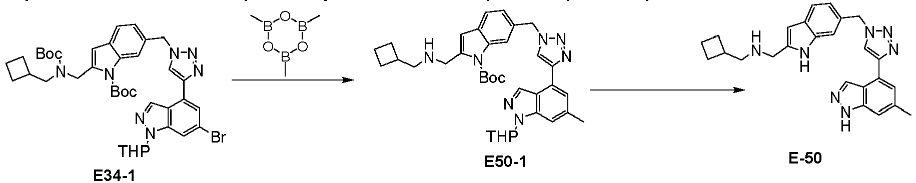

### E50-1: Tert-butyl 2-(((cyclobutylmethyl)amino)methyl)-6-((4-(6-methyl-1-(tetrahydro-2H-pyran-2-yl)-1H-indazol-4-yl)-1H-1,2,3-triazol-1-yl)methyl)-1H-indole-1-carboxylate

A white solid intermediate **E50-1** (0.053 g, 67.3%) was prepared from intermediate **E34-1** using the method described in intermediate **E18-1.**

LC-MS: [M+H]⁺= 610.3.

### E-50: 1-cyclobutyl-N-((6-((4-(6-methyl-1H-indazol-4-yl)-1H-1,2,3-triazol-1-yl)methyl)-1H-indole-2-yl)methyl)methylamine

A yellow solid compound **E-50** (16.2 mg, 40.2%) was prepared from intermediate **E50-1** using the method described in compound **E-3.**

LC-MS: [M+H]⁺= 426.2. ¹H NMR (400 MHz, DMSO-*d*₆): *δ*11.49 (s, 1 H) 9.27 (br s, 2 H) 8.88 (s, 1 H) 8.49 (d, *J* = 0.75 Hz, 1 H) 7.57 (d, *J* = 8.25 Hz, 1 H) 7.49 (d, *J* = 12.51 Hz, 2 H) 7.29 (s, 1 H) 7.08 - 7.16 (m, 1 H) 6.61 (s, 1 H) 5.75 (s, 2 H) 4.27 (t, *J* = 4.94 Hz, 2 H) 2.91 - 2.97 (m, 2 H) 2.61 - 2.70 (m, 1 H) 2.47 (s, 3 H) 1.99 - 2.08 (m, 2 H) 1.82 - 1.89 (m, 1 H) 1.74 - 1.81 (m, 3 H).

### Example 51: Synthesis of E-51: 4-(1-((2-((6-azaspiro[3.4]octan-6-yl)methyl)-1H-indole-6-yl)methyl)-1H-1,2,3-triazole-4-yl)-6-(methylsilyl)-1H-indazole

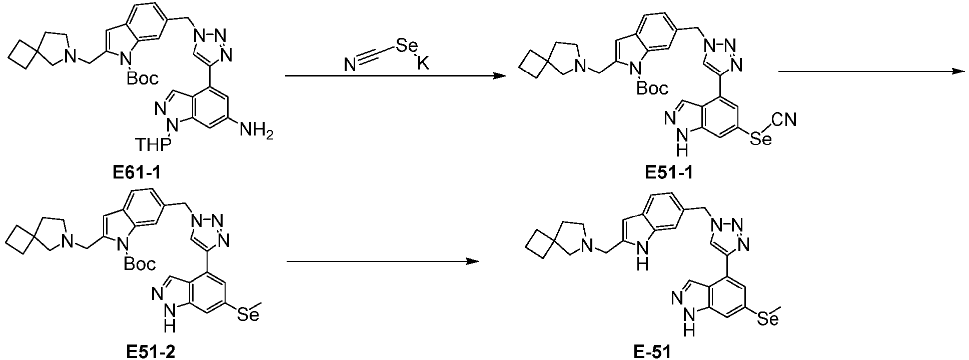

### E51-1: Tert-butyl 2-((6-azaspiro[3.4]octan-6-yl)methyl)-6-((4-(6-selenocyano-1H-indazol-4-yl)-1H-1,2,3-triazol-1-yl)methyl)-1H-indole-1-carboxylate

A pink solid intermediate **E51-1** (0.14 g, crude product) was prepared from intermediate **E61-1** using the method described in intermediate **E49-1.**

LC-MS: [M+H]⁺= 643.2.

### E51-2: Tert-butyl 2-((6-azaspiro[3.4]octan-6-yl)methyl)-6-((4-(6-(methylsilyl)-1H-indazol-4-yl)-1H-1,2,3-triazol-1-yl)methyl)-1H-indole-1-carboxylate

A yellow solid intermediate **E51-2** (50 mg, 36.3 %) was prepared from intermediate **E51-1** using the method described in intermediate **E14-2.**

LC-MS: [M+H]⁺= 632.2.

### E-51: 4-(1-((2-((6-azaspiro[3.4]octan-6-yl)methyl)-1H-indole-6-yl)methyl)-1H-1,2,3-triazole-4-yl)-6-(methylsilyl)-1H-indazole

A yellow solid compound **E-51** (11.8 mg, 26.2 %) was prepared from intermediate **E51-2** using the method described in compound **E-1.**

LC-MS: [M+H]⁺= 532.1. ¹H NMR (400 MHz, DMSO-*d*₆): δ 11.44 (s, 1 H) 10.72 - 10.85 (m, 1 H) 8.99 (s, 1 H) 8.53 (s, 1 H) 7.65 (d, *J* = 1.10 Hz, 1 H) 7.60 (d, *J* = 8.19 Hz, 1 H) 7.48 (d, *J* = 8.07 Hz, 2 H) 7.11 - 7.19 (m, 1 H) 6.67 (s, 1 H) 5.77 (s, 2 H) 4.46 (s, 2 H) 3.42 (dd, J = 11.80, 5.93 Hz, 2 H) 3.10 - 3.25 (m, 2 H) 2.42 - 2.48 (m, 3 H) 2.06 - 2.14 (m, 2 H) 2.05 (br s, 2 H) 1.97 (s, 2 H) 1.84 (d, *J* = 6.85 Hz, 2 H).

### Example 52: Synthesis of E-52: 1-(6-((4-(6-(aziridine-1-yl)-1H-indazol-4-yl)-1H-1,2,3-triazol-1-yl)methyl)-1H-indole-2-yl)-N-(cyclobutylmethyl)methylamine

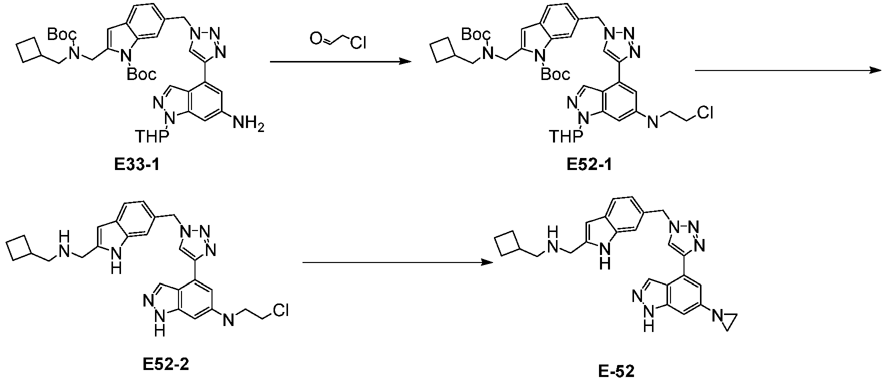

### E52-1: Tert-butyl 2-(((tert-butoxycarbonyl)(cyclobutylmethyl)amino)methyl)-6-((4-(6-((2-chloroethyl)amino)-1-(tetrahydro-2H-pyran-2-yl)-1H-indazol-4-yl)-1H-1,2,3-triazol-1-yl)methyl)-1H-indole-1-carboxylate

A yellow solid intermediate **E52-1** (90 mg, 82.7%) was prepared from intermediate **E33-1** using the method described in intermediate **E6-1.**

LC-MS: [M+H]⁺= 773.6.

### E52-2: N-(2-chloroethyl)-4-(1-((2-(((cyclobutylmethyl)amino)methyl)-1H-indole-6-yl)methyl)-1H-1,2,3-triazol-4-yl)-1H-indazole-6-amine

A pink solid intermediate **E52-2** (15 mg, 29.3%) was prepared from intermediate **E52-1** using the method described in intermediate **E-1.**

LC-MS: [M+H]⁺= 489.2

### E-52: 1-(6-((4-(6-(aziridine-1-yl)-1H-indazol-4-yl)-1H-1,2,3-triazol-1-yl)methyl)-1H-indole-2-yl)-N-(cyclobutylmethyl)methylamine

A white solid compound **E-52** (2.6 mg, 27.8%) was prepared from intermediate **E52-2** using the method described in compound **E-6.**

LC-MS: [M+H]⁺= 453.3. ¹H NMR (400 MHz, DMSO-*d*₆): δ 12.90 (s, 1H), 11.00 (s, 1H), 8.88 (s, 1H), 8.50 - 8.24 (m, 1H), 7.43 (d, *J* = 8.1 Hz, 1H), 7.36 (d, *J* = 1.7 Hz, 2H), 7.03 (d, *J=* 8.1 Hz, 1H), 6.92 (s, 1H), 6.23 (s, 1H), 5.70 (s, 2H), 3.77 (s, 2H), 2.52 - 2.51 (m, 2H), 2.42 - 2.35 (m, 1H), 2.13 (s, 4H), 1.96 (d, *J =* 7.8 Hz, 2H), 1.81 - 1.75 (m, 2H), 1.64 - 1.57 (m, 2H).

### Example 53: Synthesis of E-53: 4-(1-((6-(((cyclobutylmethyl)amino)methyl)imidazo[1,2-a]pyridin-2-yl)methyl)-1H-1,2,3-triazol-4-yl)-7-fluoro-N,n-dimethyl-1H-indazole-6-amine hydrochloride

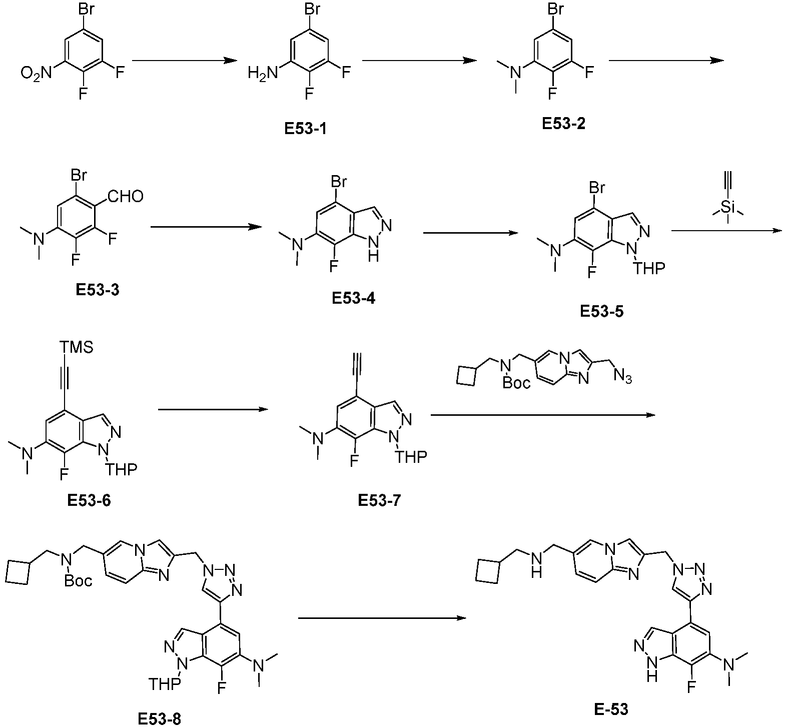

### E53-1: 5-bromo-2,3-difluoroaniline

A yellow oily intermediate **E53-1** (7.8 g, 52.5%) was prepared from 5-bromo-1,2-difluoro-3-nitrobenzene using the method described in intermediate **E3-2.**

LC-MS: [M+H]⁺= 208.1.

### E53-2: 5-bromo-2,3-difluoro-N,N-dimethylaniline

A brown oily intermediate **E53-2** (6.4 g, 72.3%) was prepared from intermediate **E53-1** using the method described in compound **E-6.**

LC-MS: [M+H]⁺= 236.1.

### E53-3: 6-bromo-4-(dimethylamino)-2,3-difluorobenzaldehyde

Under the protection of nitrogen atmosphere, DMF (2.58 g, 35.3 mmol, 2.71 mL, 1.30 eq) and phosphorus oxychloride (4.99 g, 32.5 mmol, 3.02 mL, 1.20 eq) were added to a solution of intermediate **E53-2** (6.40 g, 27.1 mmol, 1.00 eq) in toluene (64.0 mL), and the resulting mixture was stirred at 100°C for 20 h. The reaction solution was quenched by adding 2 N NaOH (60.0 mL) at 0°C, diluted with ice water (40 mL) and extracted with ethyl acetate (40.0 mL*3). The combined organic layers were washed with saline (30 mL), dried over anhydrous sodium sulfate, filtered and concentrated. The residue was purified by silica gel column chromatography (petroleum ether/ethyl acetate (100/1 to 10/1)) to obtain a white solid intermediate **E53-3** (1.4 g, 19.6%).

LC-MS: [M+H]⁺= 264.1.

### E53-4: 4-bromo-7-fluoro-N,N-dimethyl-1H-indazole-6-amine

Hydrazine hydrate (1.44 g, 28.8 mmol, 1.40 mL, 4.22 eq) was added to a solution of intermediate **E53-3** (1.80 g, 6.82 mmol, 1.00 eq) in 1,4-dioxane (18.0 mL), and the resulting mixture was stirred at 110°C for 48 h. The mixture was cooled to room temperature and concentrated, and the residue was diluted with water (50.0 mL) and extracted with ethyl acetate (20.0 mL * 3). The combined organic layers were dried over anhydrous sodium sulfate, filtered and concentrated. The residue was purified by column chromatography (petroleum ether/ethyl acetate=100/1 to 5/1) to obtain a yellow solid intermediate **E53-4** (1.40 g, 79.6%).

LC-MS: [M+H]⁺= 257.8.

### E53-5: 4-bromo-7-fluoro-N,n-dimethyl-1-(tetrahydro-2H-pyran-2-yl)-1H-indazole-6-amine

A green oily intermediate **E53-5** (300 mg, 82.9%) was prepared from intermediate **E53-4** using the method described in intermediate **2-a.**

LC-MS: [M+H]⁺= 342.2.

### E53-6: 7-fluoro-N,n-dimethyl-1-(tetrahydro-2H-pyran-2-yl)-4-((trimethylsilyl)ethynyl)-1H-indazole-6-amine

At 25°C, copper iodide (16.1 mg, 84.7 µmol, 0.10 eq) and [1,1'-bis(diphenylphosphino)ferrocene]palladium (II) dichloride (62.0 mg, 84.7 µmol, 0.10 eq) were added to a solution of intermediate **E53-5** (330 mg, 847 µmol, 1 eq) and trimethylsilylacetylene (416 mg, 4.24 mmol, 587 µL, 5 eq) in triethylamine (3.3 mL), and the resulting mixture was stirred at 90°C for 16 h under nitrogen protection. The mixture was quenched with water (5.00 mL) and extracted with EtOAc (3 x 3 mL). The combined organic layers were washed with saline (3 mL), dried over anhydrous sodium sulfate, filtered and concentrated. The residue was purified by silica gel column chromatography and eluted with petroleum ether/EtOAc (3:1) to obtain a brown oily intermediate **E53-6** (280 mg, 80.7%).

LC-MS: [M+H]⁺= 360.2.

### E53-7: 4-ethynyl-7-fluoro-N,n-dimethyl-1-(tetrahydro-2H-pyran-2-yl)-1H-indazole-6-amine

A brown oily intermediate **E53-7** (200 mg, 89.4%) was prepared from intermediate **E53-6** using the method described in intermediate **I-2.**

LC-MS: [M+H]⁺= 288.1.

### E53-8: Tert-butyl (cyclobutylmethyl)((2-((4-(6-(dimethylamino)-7-fluoro-1-(tetrahydro-2H-pyran-2-yl)-1H-indazol-4-yl)-1H-1,2,3-triazol-1-yl)methyl)imidazo[1,2-a]pyridin-6-yl)methyl)carbamate

A brown oily intermediate **E53-8** (90 mg, 86.5%) was prepared from intermediate **E53-7** using the method described in intermediate **4-d.**

LC-MS: [M+H]⁺= 658.3.

### E-53: 4-(1-((6-(((cyclobutylmethyl)amino)methyl)imidazo[1,2-a]pyridin-2-yl)methyl)-1H-1,2,3-triazol-4-yl)-7-fluoro-N,n-dimethyl-1H-indazole-6-amine hydrochloride

A white solid intermediate **E-53** (14.4 mg, 99.4%) was prepared from intermediate **E53-8** using the method described in compound **E-1.**

LC-MS: [M+H]⁺= 474.2. ¹H NMR (400 MHz, DMSO-*d*₆): δ 9.29 (d, *J* = 4.00 Hz, 2 H) 8.98 (s, 1 H) 8.90 (s, 1 H) 8.49 (d, *J* = 3.38 Hz, 1 H) 8.39 (s, 1 H) 7.86 (br s, 2 H) 7.41 (d, *J* = 6.88 Hz, 1 H) 5.97 (s, 2 H) 4.20 (t, *J* = 5.25 Hz, 4 H) 2.95 (br s, 1 H) 2.93 (s, 6 H) 2.63 - 2.70 (m, 1 H) 2.01 - 2.07 (m, 2 H) 1.75 - 1.86 (m, 4 H).

### Example 54: Synthesis of E-54: 1-(4-(1-((2-(((cyclobutylmethyl)amino)methyl)-1H-indole-6-yl)methyl)-1H-1,2,3-triazol-4-yl)-1H-indazole-6-yl)-N,N-dimethylformamide

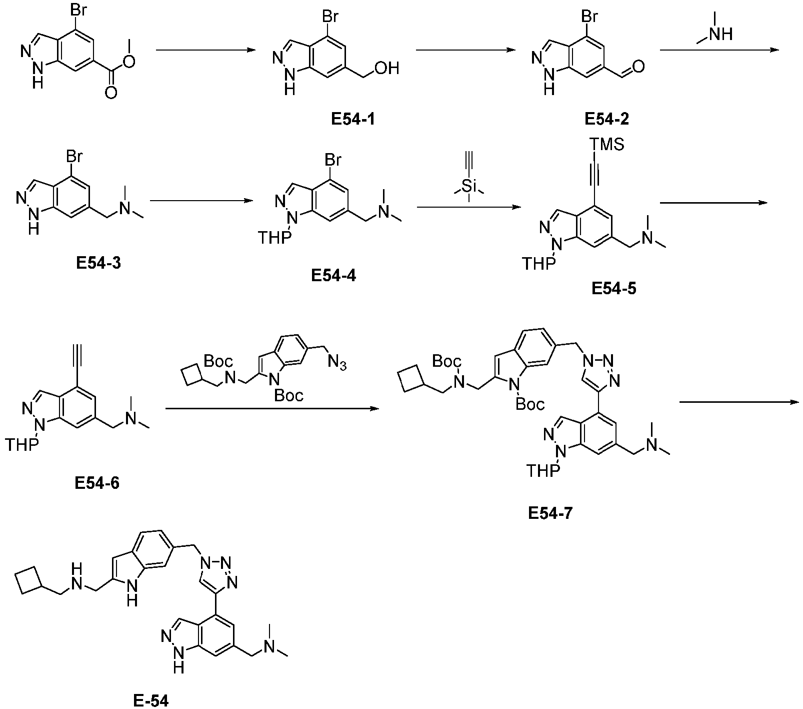

### E54-1: (4-bromo-1H-indazole-6-yl)methanol

Under nitrogen protection at 0°C, a solution of LAH (1.34 g, 35.3 mmol, 1.50 eq) in THF (30.0 mL) was added to a solution of methyl 4-bromo-1H-indazole-6-carboxylate (6.00 g, 23.5 mmol, 1.00 eq) in THF (60.0 mL), and the resulting mixture was stirred at 25°C for 0.5 h. The mixture was quenched with a 15% NaOH aqueous solution (4.20 mL) and water (4.2 mL) at 0°C. The resulting mixture was filtered and concentrated to obtain a white solid intermediate **E54-1** (4.80 g, crude product).

LC-MS: [M+H]⁺= 226.9.

### E54-2: 4-bromo-1H-indazole-6-formaldehyde

Manganese dioxide (16.5 g, 189 mmol, 10.0 eq) was added to a solution of intermediate **E54-1** (4.30 g, 18.9 mmol, 1.00 eq) in DCM (43.0 mL) while stirring, and the resulting mixture was stirred at 25°C for 16 h. The mixture was filtered and washed with ethyl acetate (50.0 mL * 4). The combined filtrate was concentrated to obtain a white solid intermediate **E54-2** (2.50 g, crude product).

LC-MS: [M+H]⁺= 225.0.

### E54-3: 1-(4-bromo-1H-indazole-6-yl)-N,N-dimethylformamide

Dimethylamine (541 mg, 4.80 mmol, 608 µL, 40% purity, 1.20 eq) was added to a solution of intermediate **E54-2** (900 mg, 4.00 mmol, 1.00 eq) in DCM (9.00 mL) while stirring, and the mixture was stirred at 25°C for 0.5 h under nitrogen protection. Then, sodium cyanoborohydride (319 mg, 5.08 mmol, 1.27 eq) was added, and the resulting mixture was stirred at 25°C for 2 h. The mixture was quenched with water (10.0 mL) and extracted with DCM (5.00 mL * 3). The combined organic layers were washed with saline (5.00 mL), dried over anhydrous sodium sulfate, filtered and concentrated. The residue was purified by silica gel column chromatography (DCM/MeOH (5:1)) to obtain a white solid intermediate **E54-3** (270 mg, 26.6%).

LC-MS: [M+H]⁺= 254.0.

### E54-4: 1-(4-bromo-1-(tetrahydro-2H-pyran-2-yl)-1H-indazol-6-yl)-N,N-dimethylformamide

A yellow solid intermediate **E54-4** (90 mg, 86.5%) was prepared from intermediate **E54-3** using the method described in intermediate **2-a.**

LC-MS: [M+H]⁺= 338.0.

**E54-5: N,N-dimethyl-1-(1-(tetrahydro-2H-pyran-2-yl)-4-((trimethylsilyl)ethynyl)-1H-indazol-6-yl)methylamine**

A brown oily intermediate **E54-5** (90 mg, 86.5%) was prepared from intermediate **E54-4** using the method described in intermediate **E53-6.**

LC-MS: [M+H]⁺= 356.3.

### E54-6: 1-(4-ethynyl-1-(tetrahydro-2H-pyran-2-yl)-1H-indazol-6-yl)-N,n-dimethylformamide

A brown oily intermediate **E54-6** (37 mg, 46.4%) was prepared from intermediate **E54-5** using the method described in intermediate **I-2.**

LC-MS: [M+H]⁺= 284.1.

### E54-7: Tert-butyl 2-(((tert-butoxycarbonyl)(cyclobutylmethyl)amino)methyl)-6-((4-(6-((dimethylamino)methyl)-1-(tetrahydro-2H-pyran-2-yl)-1H-indazol-4-yl)-1H-1,2,3-triazol-1-yl)methyl)-1H-indole-1-carboxylate

A brown oily intermediate **E54-7** (30 mg, 30.5%) was prepared from intermediate **E54-6** using the method described in intermediate **4-d.**

LC-MS: [M+H]⁺= 753.5.

### E-54: 1-(4-(1-((2-(((cyclobutylmethyl)amino)methyl)-1H-indole-6-yl)methyl)-1H-1,2,3-triazol-4-yl)-1H-indazole-6-yl)-N,N-dimethylformamide

A yellow solid intermediate **E-54** (7.80 mg, 38.1%) was prepared from intermediate **E54-7** using the method described in compound **E-1.**

LC-MS: [M+H]⁺= 469.0. ¹H NMR (400 MHz, DMSO-*d*₆):*δ* 13.50 - 13.60 (m, 1 H) 11.63 (br s, 1 H) 10.62 - 10.77 (m, 1 H) 9.44 (br s, 2 H) 8.85 (s, 1 H) 8.61 (br s, 1 H) 7.84 (s, 1 H) 7.76 (s, 1 H) 7.56 (d, *J* = 8.07 Hz, 1 H) 7.49 (s, 1 H) 7.10 (d, *J* = 8.07 Hz, 1 H) 6.60 (s, 1 H) 5.77 (s, 2 H) 4.42 (d, *J* = 2.57 Hz, 2 H) 4.26 (br s, 2 H) 2.91 (d, *J* = 3.30 Hz, 2 H) 2.71 (d, *J* = 2.69 Hz, 6 H) 2.66 (br s, 1 H) 2.00 - 2.06 (m, 2 H) 1.74 - 1.84 (m, 4 H).

### Example 55: Synthesis of E-55: 6-(aziridin-1-yl)-4-(1-((2-((4,4-dimethylpiperidine-1-yl)methyl)-1H-indole-6-yl)methyl)-1H-1,2,3-triazol-4-yl)-1H-indazole

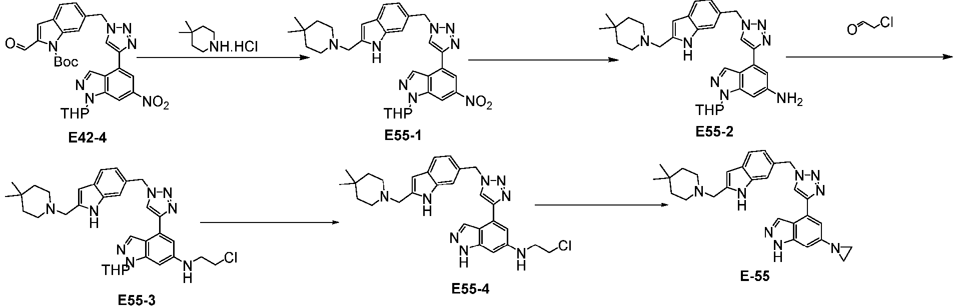

### E55-1: 4-(1-((2-((4,4-dimethylpiperidine-1-yl)methyl)-1H-indole-6-yl)methyl)-1H-1,2,3-triazol-4-yl)-6-nitro-1-(tetrahydro-2H-pyran-2-yl)-1H-indazole

A brown oily intermediate **E55-1** (180 mg, 18.1%) was prepared from intermediate **E42-4** using the method described in intermediate **E30-1.**

LC-MS: [M+H]⁺= 569.4.

### E55-2: 4-(1-((2-((4,4-dimethylpiperidine-1-yl)methyl)-1 H-indole-6-yl)methyl)-1H-1,2,3-triazol-4-yl)-1-(tetrahydro-2H-pyran-2-yl)-1H-indazole-6-amine

A brown oily intermediate **E55-2** (66 mg, 53.6%) was prepared from intermediate **E55-1** using the method described in intermediate **E3-2.**

**LC-MS:** [M+H]⁺= 539.4.

### E55-3: N-(2-chloroethyl)-4-(1-((2-((4,4-dimethylpiperidine-1-yl)methyl)-1H-indole-6-yl)methyl)-1H-1,2,3-triazol-4-yl)-1-(tetrahydro-2H-pyran-2-yl)-1H-indazole-6-amine

A yellow solid intermediate **E55-3** (46 mg, 62.5%) was prepared from intermediate **E55-2** using the method described in intermediate **E6-1.**

LC-MS: [M+H]⁺= 601.4.

### E55-4: N-(2-chloroethyl)-4-(1-((2-((4,4-dimethylpiperidine-1-yl)methyl)-1H-indole-6-yl)methyl)-1H-1,2,3-triazol-4-yl)-1-(tetrahydro-2H-pyran-2-yl)-1H-indazole-6-amine

A red oily intermediate **E55-4** (39 mg, crude product) was prepared from intermediate **E55-3** using the method described in compound **E-1.**

LC-MS: [M+H]⁺= 517.3.

### E-55: 6-(aziridin-1-yl)-4-(1-((2-((4,4-dimethylpiperidine-1-yl)methyl)-1H-indole-6-yl)methyl)-1H-1,2,3-triazol-4-yl)-1H-indazole

A yellow solid intermediate **E-55** (5.8 mg, 17%) was prepared from intermediate **E55-4** using the method described in compound **E-6.**

LC-MS: [M+H]⁺= 481.4. ¹H NMR (400 MHz, DMSO-*d*₆): δ 12.78 - 12.96 (m, 1 H) 11.05 (s, 1 H) 8.88 (s, 1 H) 8.42 (s, 1 H) 7.44 (d, *J* = 8.13 Hz, 1 H) 7.35 - 7.38 (m, 2 H) 7.03 (dd, *J =* 8.19, 1.31 Hz, 1 H) 6.92 (s, 1 H) 6.24 (d, *J* = 0.75 Hz, 1 H) 5.70 (s, 2 H) 3.57 (s, 2 H) 2.33 (d, *J =* 4.00 Hz, 4 H) 2.13 (s, 4 H) 1.31 (t, *J* = 5.44 Hz, 4 H) 0.87 (s, 6 H).

### Example 56: Synthesis of E-56: 1-(6-((4-(6-(aziridine-1-yl)-1H-indazol-4-yl)-1H-1,2,3-triazol-1-yl)methyl)-1H-indole-2-yl)-N-((3-fluorobicyclo[1.1.1]pentan-1-yl)methyl)methylamine

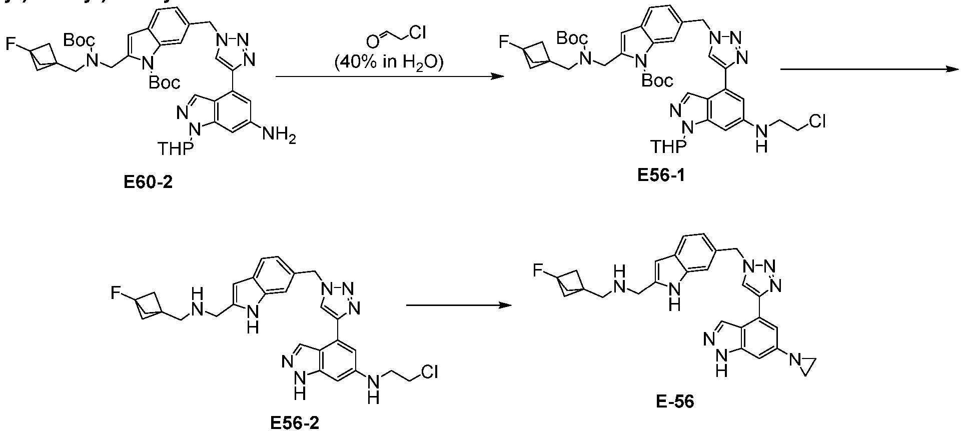

### E56-1: Tert-butyl 2-(((tert-butoxycarbonyl)((3-fluorobicyclo[1.1.1]pentan-1-yl)methyl)amino)-6-((4-(6-((2-chloroethyl)amino)-1-(tetrahydro-2H-pyran-2-yl)-1H-indazol-4-yl)-1H-1,2,3-triazol-1-yl)methyl)-1H-indole-1-carboxylate

A yellow solid intermediate **E56-1** (80 mg, 73.8%) was prepared from intermediate **E60-2** using the method described in intermediate **E6-1.**

LC-MS: [M+H]⁺= 803.5.

### E56-2: N-(2-chloroethyl)-4-(1-((2-(((((3-fluorobicyclo[1.1.1]pentan-1-yl)methyl)amino)methyl)-1H-indole-6-yl)methyl)-1H-1,2,3-triazol-4-yl)-1H-indazole-6-amine

A yellow solid intermediate **E56-2** (50 mg, crude product) was prepared from intermediate **E56-1** using the method described in compound **E-1.**

LC-MS: [M+H]⁺= 519.4.

### E-56: 1-(6-((4-(6-(aziridine-1-yl)-1H-indazol-4-yl)-1H-1,2,3-triazol-1-yl)methyl)-1H-indole-2-yl)-N-((3-fluorobicyclo[1.1.1]pentan-1-yl)methyl)methylamine

A yellow solid compound **E-56** (5.7 mg, 12.9%) was prepared from intermediate **E56-2** using the method described in compound **E-6.**

LC-MS: [M+H]⁺= 483.3. ¹H NMR (400 MHz, DMSO-*d*₆): δ 12.89 (s, 1 H) 11.00 (s, 1 H) 8.87 (s, 1 H) 8.42 (s, 1 H) 7.44 (d, *J=* 8.13 Hz, 1 H) 7.36 (s, 2 H) 7.03 (d, *J* = 8.25 Hz, 1 H) 6.92 (s, 1 H) 6.24 (s, 1 H) 5.70 (s, 2 H) 3.81 (br s, 2 H) 2.75 (br s, 2 H) 2.42 - 2.47 (m, 1 H) 2.13 (s, 4 H) 1.92 (d, *J* = 2.63 Hz, 6 H).

### Example 57: Synthesis of E-57: 4-(1-((2-((((3-fluorobicyclo[1.1.1]pentan-1-yl)methyl)amino)methyl)-1H-indole-6-yl)methyl)-1H-1,2,3-triazol-4-yl)-N,n-dimethyl-1H-indazole-6-amine

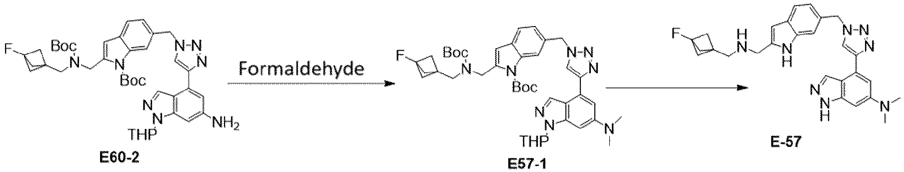

### E57-1: Tert-butyl 2-(((tert-butoxycarbonyl)((3-fluorobicyclo[1.1.1]pentan-1-yl)methyl)amino)methyl)-6-((4-(6-(dimethylamino)-1-(tetrahydro-2H-pyran-2-yl)-1H-indazol-4-yl)-1H-1,2,3-triazol-1-yl)methyl)-1H-indole-1-carboxylate

A white solid intermediate **E57-1** (50 mg, 48.2%) was prepared from intermediate **E60-2** using the method described in intermediate **E5-1.**

LC-MS: [M+H]⁺= 769.6.

### E-57: 4-(1-((2-((((3-fluorobicyclo[1.1.1]pentan-1-yl)methyl)amino)methyl)-1H-indole-6-yl)methyl)-1H-1,2,3-triazol-4-yl)-N,n-dimethyl-1H-indazole-6-amine

A yellow solid compound **E-57** (6.7 mg, 21.3%) was prepared from intermediate **E57-1** using the method described in compound **E-1.**

LC-MS: [M+H]⁺= 485.3. ¹H NMR (400 MHz, DMSO-*d*₆): δ 12.59 (s, 1 H) 10.99 (s, 1 H) 8.86 (s, 1 H) 8.32 (s, 1 H) 7.44 (d, *J* = 8.13 Hz, 1 H) 7.35 (s, 1 H) 7.25 (d, *J* = 1.88 Hz, 1 H) 6.98 - 7.09 (m, 1 H) 6.53 (s, 1 H) 6.24 (s, 1 H) 5.70 (s, 2 H) 3.81 (s, 2 H) 2.99 (s, 6 H) 2.74 (s, 2 H) 1.98 - 2.13 (m, 1 H) 1.92 (d, J= 2.75 Hz, 6 H).

### Example 58: Synthesis of E-58: 4-(1-((2-((6-azaspiro[3.4]octan-6-yl)methyl)-1H-indole-6-yl)methyl)-1H-1,2,3-triazol-4-yl)-N,n-dimethyl-1H-indazole-6-amine

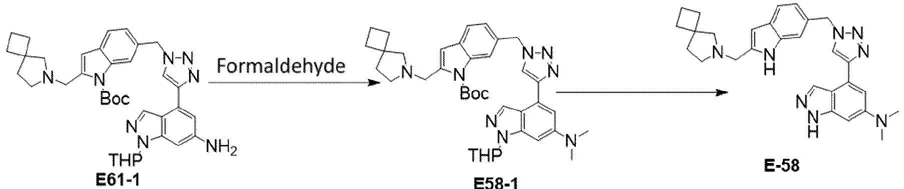

### E58-1: 4-(1-((2-((6-azaspiro[3.4]octan-6-yl)methyl)-1H-indole-6-yl)methyl)-1H-1,2,3-triazol-4-yl)-N,n-dimethyl-1-(tetrahydro-2H-pyran-2-yl)-1H-indazole-6-amine

A yellow solid intermediate **E58-1** (15 mg, 19.7%) was prepared from intermediate **E61-1** using the method described in intermediate **E5-1.**

LC-MS: [M+H]⁺= 665.3.

### E-58: 4-(1-((2-((6-azaspiro[3.4]octan-6-yl)methyl)-1H-indole-6-yl)methyl)-1H-1,2,3-triazol-4-yl)-N,n-dimethyl-1H-indazole-6-amine

A yellow solid compound **E-58** (2.7 mg, 20.7%) was prepared from intermediate **E58-1** using the method described in compound **E-1.**

LC-MS: [M+H]⁺= 481.2. ¹H NMR (400 MHz, DMSO-*d*₆): *δ* 11.28 - 11.67 (m, 1 H) 10.78 - 11.16 (m, 1 H) 8.85 - 9.05 (m, 1 H) 8.27 - 8.53 (m, 1 H) 7.54 - 7.66 (m, 1 H) 7.37 - 7.54 (m, 2 H) 7.08 - 7.17 (m, 1 H) 6.63 - 6.70 (m, 1 H) 5.68 - 5.84 (m, 2 H) 4.43 - 4.48 (m, 2 H) 3.34 - 3.46 (m, 2 H) 3.11 - 3.23 (m, 2 H) 3.10 (s, 6 H) 1.97 - 2.12 (m, 4 H) 1.65 - 1.95 (m, 4 H).

### Example 59: Synthesis of E-59: 4-(1-((2-((6-azaspiro[3.4]octan-6-yl)methyl)-1H-indole-6-yl)methyl)-1H-1,2,3-triazol-4-yl)-6-(aziridin-1-yl)-1H-indazole

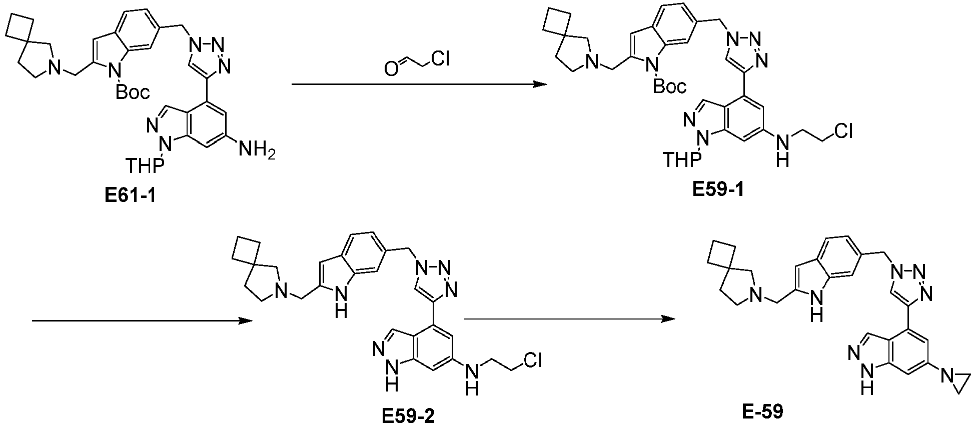

### E59-1: 4-(1-((2-((6-azaspiro[3.4]octan-6-yl)methyl)-1H-indole-6-yl)methyl)-1H-1,2,3-triazol-4-yl)-N-(2-chloroethyl)-1-(tetrahydro-2H-pyran-2-yl)-1H-indazole-6-amine

A yellow solid intermediate **E59-1** (60 mg, 54.9%) was prepared from intermediate **E61-1** using the method described in intermediate **E6-1.**

LC-MS: [M+H]⁺= 699.4.

### E59-2: 4-(1-((2-((6-azaspiro[3.4]octan-6-yl)methyl)-1H-indole-6-yl)methyl)-1H-1,2,3-triazol-4-yl)-N-(2-chloroethyl)-1H-indazole-6-amine

A yellow solid intermediate **E59-2** (50 mg, crude product) was prepared from intermediate **E59-1** using the method described in compound **E-1.**

LC-MS: [M+H]⁺= 515.4.

### E-59: 4-(1-((2-((6-azaspiro[3.4]octan-6-yl)methyl)-1H-indole-6-yl)methyl)-1H-1,2,3-triazol-4-yl)-6-(aziridin-1-yl)-1H-indazole

A yellow solid compound **E-59** (5.4 mg, 12.6%) was prepared from intermediate **E59-2** using the method described in compound **E-6.**

LC-MS: [M+H]⁺= 479.3. ¹H NMR (400 MHz, DMSO-*d*₆): *δ* 12.93 (s, 1 H) 11.12 - 11.15 (m, 1 H) 8.91 (s, 1 H) 8.44 - 8.46 (m, 1 H) 7.46 (d, *J* = 7.82 Hz, 1 H) 7.38 (d, *J* = 4.52 Hz, 2 H) 7.06 (d, *J* = 7.95 Hz, 1 H) 6.94 (s, 1 H) 6.26 (s, 1 H) 5.72 (s, 2 H) 3.67 (s, 2 H) 2.16 (s, 4 H) 1.94 (s, 2 H) 1.90 - 1.93 (m, 2 H) 1.85 1.90 (m, 2 H) 1.82 - 1.85 (m, 2 H) 1.76 1.81 (m, 2 H) 1.65 - 1.76 (m, 2 H).

### Example 60: Synthesis of E-60: 1-(3-fluorobicyclo[1.1.1]pentan-1-yl)-N-((6-((4-(6-(methylsilyl)-1H-indazol-4-yl)-1H-1,2,3-triazol-1-yl)methyl)-1H-indole-2-yl)methyl)methylamine

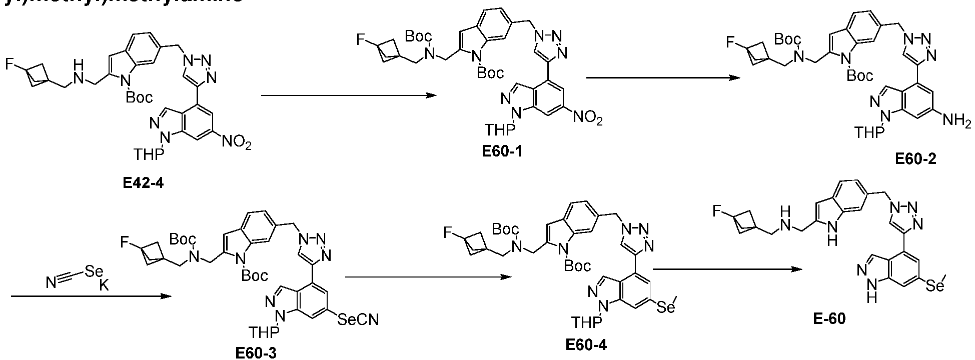

### E60-1: Tert-buty 2-(((tert-butoxycarbonyl)((3-fluorobicyclo[1.1.1]pentan-1-yl)methyl)amino)methyl)-6-((4-(6-nitro-1-(tetrahydro-2H-pyran-2-yl)-1H-indazol-4-yl)-1H-1,2,3-triazol-1-yl)methyl)-1H-indole-1-carboxylate

A yellow solid intermediate **E60-1** (90 mg, 65.3 %) was prepared from intermediate **E42-4** using the method described in intermediate **1-b.**

LC-MS: [M+H]⁺= 771.4.

### E60-2: Tert-butyl 6-((4-(6-amino-1-(tetrahydro-2H-pyran-2-yl)-1H-indazol-4-yl)-1H-1,2,3-triazol-1-yl)methyl)-2-(((tert-butoxycarbonyl)((3-fluorobicyclo[1.1.1]pentan-1-yl)methyl)amino)-1H-indole-1-carboxylate

A white solid intermediate **E60-2** (75 mg, 86.7%) was prepared from intermediate **E60-1** using the method described in intermediate **E3-2.**

LC-MS: [M+H]⁺= 741.4.

### E60-3: Tert-butyl 2-(((tert-butoxycarbonyl)((3-fluorobicyclo[1.1.1]pentan-1-yl)methyl)amino)methyl)-6-((4-(6-selenocyano-1-(tetrahydro-2H-pyran-2-yl)-1H-indazol-4-yl)-1H-1,2,3-triazol-1-yl)methyl)-1H-indole-1-carboxylate

A brown solid intermediate **E60-3** (100 mg, crude product) was prepared from intermediate **E60-2** using the method described in intermediate **E14-1.**

LC-MS: [M+H]⁺= 831.3.

### E60-4: Tert-butyl 2-(((tert-butoxycarbonyl)((3-fluorobicyclo[1.1.1]pentan-1-yl)methyl)amino)methyl)-6-((4-(6-(methylsilyl)-1-(tetrahydro-2H-pyran-2-yl)-1H-indazol-4-yl)-1H-1,2,3-triazol-1-yl)methyl)-1H-indole-1-carboxylate

A yellow solid intermediate **E60-4** (50.0 mg, 50.7%) was prepared from intermediate **E60-3** using the method described in intermediate **E14-2.**

LC-MS: [M+H]⁺= 820.3.

### E-60: 1-(3-fluorobicyclo[1.1.1]pentan-1-yl)-N-((6-((4-(6-(methylsilyl)-1H-indazol-4-yl)-1H-1,2,3-triazol-1-yl)methyl)-1H-indole-2-yl)methyl)methylamine

A yellow solid compound **E-60** (7.8 mg, 22.1%) was prepared from intermediate **E60-4** using the method described in compound **E-3.**

LC-MS: [M+H]⁺= 536.1. ¹H NMR (400 MHz, DMSO-*d*₆): *δ* 11.40 (s, 1 H) 9.31 (br s, 2 H) 8.99 (s, 1 H) 8.53 (s, 1 H) 7.64 (d, *J* = 1.10 Hz, 1 H) 7.58 (d, *J* = 8.19 Hz, 1 H) 7.50 (s, 1 H) 7.46 (s, 1 H) 7.13 (dd, *J* = 8.19, 1.22 Hz, 1 H) 6.62 (s, 1 H) 5.63 - 5.88 (m, 2 H) 4.21 - 4.38 (m, 2 H) 3.20 - 3.32 (m, 2 H) 2.45 (s, 3 H) 2.11 (d, *J* = 2.57 Hz, 6 H).

### Example 61: Synthesis of E-61: 4-(1-((2-((6-azaspiro[3.4]octan-6-yl)methyl)-1H-indole-6-yl)methyl)-1H-1,2,3-triazole-4-yl)-1H-indazole-6-amine

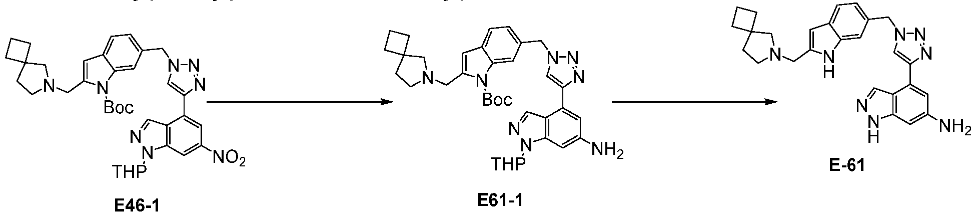

### E61-1: Tert-butyl 2-((6-azaspiro[3.4]octan-6-yl)methyl)-6-((4-(6-amino-1-(tetrahydro-2H-pyran-2-yl)-1H-indazol-4-yl)-1H-1,2,3-triazol-1-yl)methyl)-1H-indole-1-carboxylate

A yellow solid intermediate **E61-1** (60.0 mg, 62.8%) was prepared from intermediate **E46-1** using the method described in intermediate **E3-2.**

LC-MS: [M+H]⁺= 637.5.

### E-61: 4-(1-((2-((6-azaspiro[3.4]octan-6-yl)methyl)-1H-indole-6-yl)methyl)-1H-1,2,3-triazole-4-yl)-1H-indazole-6-amine

A black brown oily compound **E-61** (31.7 mg, 68.8%) was prepared from intermediate **E61-1** using the method described in compound **E-3.**

LC-MS: [M+H]⁺= 453.3. ¹H NMR (400 MHz, DMSO-*d*₆): *δ* 11.61 (s, 1 H) 11.33 (d, *J=* 5.38 Hz, 1 H) 9.11 (s, 1 H) 8.65 (s, 1 H) 7.59 - 7.66 (m, 3 H) 7.53 (s, 1 H) 7.18 (dd, *J* = 8.25, 1.16 Hz, 1 H) 6.69 (s, 1 H) 5.80 (s, 2 H) 4.48 (t, *J* = 5.75 Hz, 2 H) 3.40 (dd, *J =* 10.15, 4.52 Hz, 2 H) 3.10 - 3.24 (m, 2 H) 2.06 - 2.16 (m, 2 H) 1.98 - 2.05 (m, 2 H) 1.86 - 1.98 (m, 2 H) 1.74 - 1.85 (m, 2 H).

### Example 62: Synthesis of E-62: 5-(1-((6-(((cyclobutylmethyl)amino)methyl)imidazo[1,2-a]pyridin-2-yl)methyl)-1H-1,2,3-triazole-4-yl)pyridin-3-amine

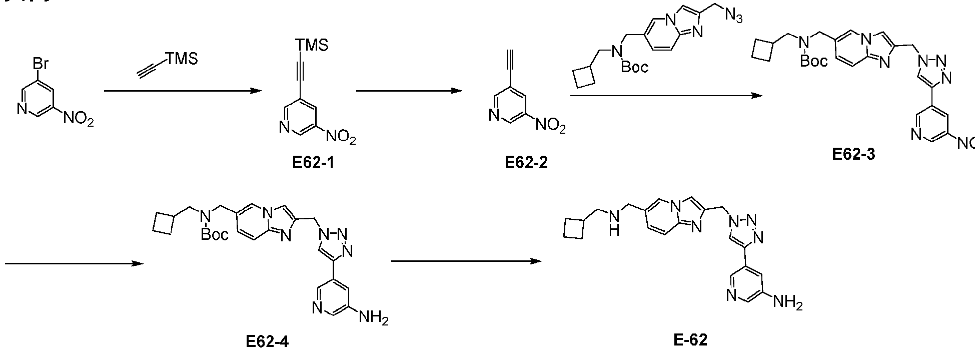

### E62-1: 3-nitro-5-((trimethylsilyl)ethynyl)pyridine

A black solid intermediate **E62-1** (9.8 g, 45.2%) was prepared from 3-bromo-5-nitropyridine using the method described in intermediate **2-b.**

**LC-MS (ESI):** m/z 221.1 [M+H]⁺.

### E62-2: 3-ethynyl-5-nitropyridine

A yellow solid intermediate **E62-2** (5.1 g, 77.4%) was prepared from intermediate **E62-1** using the method described in intermediate **1-2.**

**LC-MS (ESI):** m/z 149.2 [M+H]⁺.

### E62-3: Tert-butyl (2-((4-(5-nitropyridin-3-yl)-1H-1,2,3-triazol-1-yl)methyl)imidazo[1,2-a]pyridin-6-yl)methyl)carbamate

A yellow solid intermediate **E62-3** (1.2 g, 76.2%) was prepared from intermediate **E62-2** using the method described in intermediate **4-d.**

**LC-MS (ESI):** m/z 519.4 [M+H]⁺.

### E62-4: Tert-butyl (2-((4-(5-aminopyridin-3-yl)-1H-1,2,3-triazol-1-yl)methyl)imidazo[1,2-a]pyridin-6-yl)methyl)(cyclobutylmethyl)carbamate

A yellow solid intermediate **E62-4** (0.18 g, crude product) was prepared from intermediate **E62-3** using the method described in intermediate **E3-2.**

**¹H NMR** (400 MHz, chloroform-d): *δ* 8.27 (s, 1H), 7.97 (d, *J* = 5.3 Hz, 2H), 7.87 (s, 1H), 7.56 - 7.42 (m, 3H), 7.09 (s, 1H), 5.67 (s, 2H), 4.31 (s, 2H), 3.71 (s, 2H), 3.16 (s, 2H), 2.44 (s, 1H), 1.90 (s, 2H), 1.77 (d, *J =* 7.5 Hz, 2H), 1.62 (d, *J* = 8.0 Hz, 2H), 1.41 (s, 9H).

### E-62: 5-(1-((6-(((cyclobutylmethyl)amino)methyl)imidazo[1,2-a]pyridin-2-yl)methyl)-1H-1,2,3-triazole-4-yl)pyridin-3-amine

A white solid compound **E-62** (0.18 g, crude product) was prepared from intermediate **E62-4** using the method described in compound **E-1.**

LC-MS (ESI): m/z 389.2 [M+H]⁺. ¹H NMR (400 MHz, DMSO-*d*₆): *δ* 9.70 - 9.58 (m, 1H), 9.64 (br s, 1H), 9.06 - 8.99 (m, 2H), 8.46 (d, *J* = 6.6 Hz, 2H), 8.12 (s, 1H), 8.08 - 8.01 (m, 2H), 7.91 (d, *J* = 9.4 Hz, 1H), 6.04 (s, 2H), 4.26 - 4.20 (m, 1H), 4.26 - 4.19 (m, 1H), 2.99 - 2.92 (m, 2H), 2.78 - 2.70 (m, 1H), 2.10 - 2.01 (m, 2H), 1.91 - 1.77 (m, 4H).

### Example 63: Synthesis of E-63: 1-(2-((4-(5-bromopyridine-3-yl)-1H-1,2,3-triazol-1-yl)methyl)imidazo[1,2-a]pyridin-6-yl)-N-(cyclobutylmethyl)methylamine

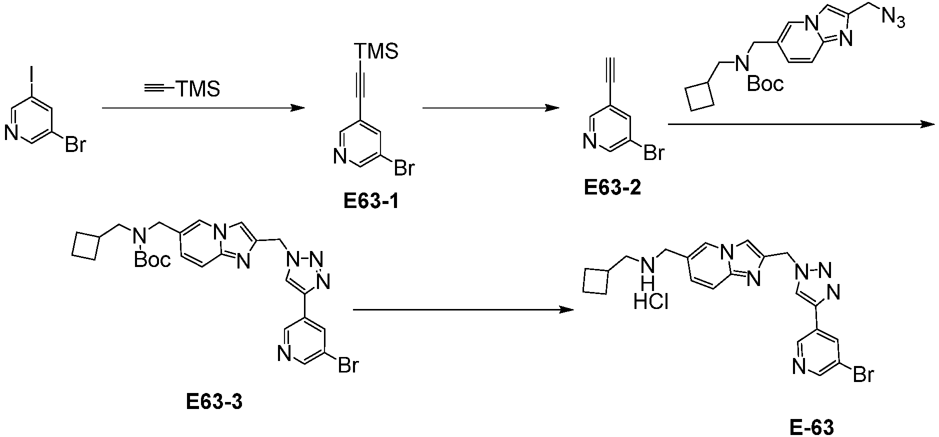

### E63-1: 3-bromo-5-((trimethylsilyl)ethynyl)pyridine

A black solid intermediate E63-1 (20.0 g, 89.4%) was prepared from 3-bromo-5-iodopyridine using the method described in intermediate 2-b.

**LC-MS (ESI):** m/z 256.0 [M+H]⁺.

### E63-2: 3-bromo-5-ethynylpyridine

A yellow solid intermediate E63-2 (5.1 g, 77.4%) was prepared from intermediate **E63-1** using the method described in intermediate **1-2.**

**LC-MS (ESI):** m/z 181.9 [M+H]⁺.

### E63-3: Tert-butyl (2-((4-(5-bromopyridine-3-yl)-1H-1,2,3-triazol-1-yl)methyl)imidazo[1,2-a]pyridin-6-yl)methyl)(cyclobutylmethyl)carbamate

A purple solid intermediate **E63-3** (3.3 g, 82.0%) was prepared from intermediate **E63-2** using the method described in intermediate **4-d.**

**LC-MS (ESI):** m/z 552.1 [M+H]⁺.

### E-63: 1-(2-((4-(5-bromopyridine-3-yl)-1H-1,2,3-triazol-1-yl)methyl)imidazo[1,2-a]pyridin-6-yl)-N-(cyclobutylmethyl)methylamine

A white solid compound **E-63** (35.0 mg, 49.5%) was prepared from intermediate **E63-3** using the method described in compound **E-1.**

**LC-MS (ESI):** m/z 452.0 [M+H]⁺ . **¹H NMR** (400 MHz, DMSO-*d*₆): *δ* 9.01 (s, 1 H) 8.92 (s, 1 H) 8.82 (s, 1 H) 8.66 (d, *J =* 1.88 Hz, 1 H) 8.44 (t, *J* = 2.00 Hz, 1 H) 8.42 (s, 1 H) 7.96 - 8.00 (m, 1 H) 7.88 - 7.92 (m, 1 H) 6.01 (s, 2 H) 4.24 (s, 2 H) 2.99 (d, *J* = 7.50 Hz, 2 H) 2.60 (dt, *J=* 15.17, 7.61 Hz, 1 H) 1.98 - 2.06 (m, 2 H) 1.76 - 1.88 (m, 2 H) 1.70 - 1.75 (m, 2 H).

### Example 64: Synthesis of E-64: 5-(1-((6-((4,4-dimethylpiperidine-1-yl)methyl)imidazo[1,2-a]pyridin-2-yl)methyl)-1H-1,2,3-triazole-4-yl)-N,n-dimethylpyridine-3-amine

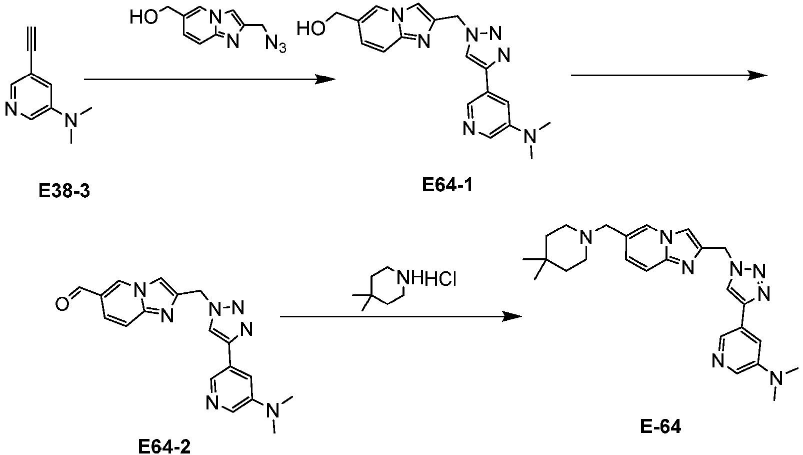

### E64-1: (2-((4-(5-(dimethylamino)pyridin-3-yl)-1H-1,2,3-triazol-1-yl)methyl)imidazo[1,2-a]pyridin-6-yl)methanol

A yellow solid intermediate **E64-1** (0.60 g, 41.8%) was prepared from intermediate **E38-3** using the method described in intermediate **4-d.**

**LC-MS (ESI):** m/z 350.2 [M+H]⁺.

### E64-2: 2-((4-(5-(dimethylamino)pyridin-3-yl)-1H-1,2,3-triazol-1-yl)methyl)imidazo[1,2-a]pyridine-6-formaldehyde

A yellow solid intermediate **E64-2** (180 mg, 60.3%) was prepared from intermediate **E64-1** using the method described in intermediate **E54-2.**

**LC-MS (ESI):** m/z 348.1 [M+H]⁺.

### E-64: 5-(1-((6-((4,4-dimethylpiperidine-1-yl)methyl)imidazo[1,2-a]pyridin-2-yl)methyl)-1H-1,2,3-triazole-4-yl)-N,n-dimethylpyridine-3-amine

A brown solid compound **E-64** (47.0 mg, 38.3%) was prepared from intermediate **E64-2** using the method described in intermediate **E30-1.**

LC-MS (ESI): m/z 445.3 [M+H]⁺ . ¹H NMR (400 MHz, DMSO-*d*₆): *δ* 8.70 (s, 1H), 8.44 (s, 1H), 8.37 (d, *J* = 1.6 Hz, 1H), 8.07 (d, *J* = 2.8 Hz, 1H), 7.96 (s, 1H), 7.49 - 7.44 (m, 2H), 7.21 (dd, J= 1.5, 9.4 Hz, 1H), 5.74 (s, 2H), 3.44 (s, 2H), 2.98 (s, 6H), 2.34 (d, *J* = 3.9 Hz, 4H), 1.31 (t, *J* = 5.4 Hz, 4H), 0.89 (s, 6H).

### Example 65: Synthesis of E-65: N-((6-((((3-fluorobicyclo[1.1.1]pentan-1-yl)methyl)amino)methyl)imidazo[1,2-a]pyridin-2-yl)methyl)-5-(pyrrolidine-1-yl)nicotinamide

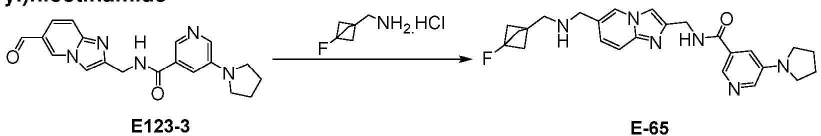

White solid **E-65** (15.1 mg, 39.4%) was prepared from **E123-3** using the method described in **E30-1.**

LC-MS: [M+H]⁺= 449.1. ¹H NMR (400 MHz, DMSO-*d*₆): *δ* 9.07 (t, *J* = 5.7 Hz, 1H), 8.36 (s, 1H), 8.31 (d, *J* = 1.6 Hz, 1H), 8.04 (d, *J* = 2.8 Hz, 1H), 7.75 (s, 1H), 7.46-7.37 (m, 1H), 7.33-7.28 (m, 1H), 7.20 (dd, *J* = 1.6, 9.3 Hz, 1H), 4.56 (d, *J* = 5.8 Hz, 2H), 3.66 (s, 2H), 3.31-3.28 (m, 4H), 2.74 (s, 2H), 2.00-1.95 (m, 4H), 1.94-1.90 (m, 6H).

### Example 66: Synthesis of E-66: 1-(6-((4-(5-bromopyridine-3-yl)-1H-1,2,3-triazol-1-yl)methyl)-1H-indole-2-yl)-N-(cyclobutylmethyl)methylamine hydrochloride

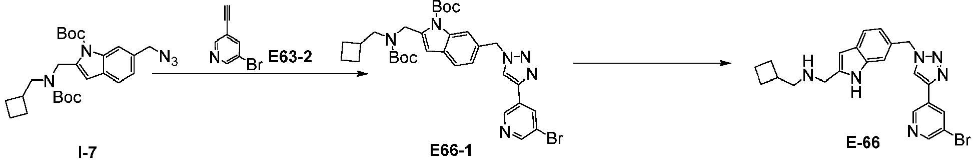

### E66-1: Tert-butyl 6-((4-(5-bromopyridine-3-yl)-1H-1,2,3-triazol-1-yl)methyl)-2-(((tert-butoxycarbonyl)(cyclobutylmethyl)amino)methyl)-1H-indole-1-carboxylate

A white solid intermediate **E66-1** (320 mg, 76.9%) was prepared from intermediate **I-7** using the method described in intermediate **4-d.**

LC-MS (ESI): m/z 652.2 [M+H]⁺.

### E-66: 1-(6-((4-(5-bromopyridine-3-yl)-1H-1,2,3-triazol-1-yl)methyl)-1H-indole-2-yl)-N-(cyclobutylmethyl)methylamine hydrochloride

A white solid compound **E-66** (20.3 mg, 38.3%) was prepared from intermediate **E66-1** using the method described in compound **E-3.**

LC-MS (ESI): m/z 453.0 [M+H]⁺ . ¹H NMR (400 MHz, DMSO-*d*₆): *δ* 11.36 (br s, 1 H) 9.05 (d, *J* = 1.75 Hz, 2 H) 8.83 (s, 1 H) 8.66 (d, *J* = 2.13 Hz, 1 H) 8.46 (t, *J =* 1.94 Hz, 1 H) 7.57 (d, *J* = 8.00 Hz, 1 H) 7.46 (s, 1 H) 7.07 (d, *J* = 8.25 Hz, 1 H) 6.60 (s, 1 H) 5.74 (s, 2 H) 4.26 (t, *J =* 5.19 Hz, 2 H) 2.94 - 2.99 (m, 2 H) 2.64 (br s, 1 H) 1.99 - 2.10 (m, 2 H) 1.78 - 1.90 (m, 2 H) 1.72 - 1.78 (m, 2 H).

### Example 67: Synthesis of E-67: 1-cyclobutyl-N-((2-((4-(5-morpholinopyridine-3-yl)-1H-1,2,3-triazol-1-yl)methyl)imidazo[1,2-a]pyridin-6-yl)methyl)methylamine

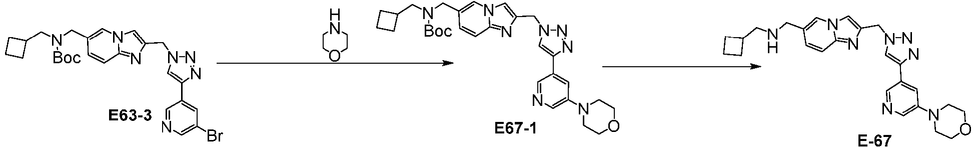

### E67-1: Tert-butyl (2-((4-(5-morpholinopyridine-3-yl)-1H-1,2,3-triazol-1-yl)methyl)imidazo[1,2-a]pyridin-6-yl)methyl)carbamate

A yellow solid intermediate **E67-1** (70 mg, 69.2%) was prepared from intermediate **E63-3** using the method described in intermediate **E15-1.**

LC-MS (ESI): m/z 559.3 [M+H]⁺.

### E-67: 1-cyclobutyl-N-((2-((4-(5-morpholinopyridine-3-yl)-1H-1,2,3-triazol-1-yl)methyl)imidazo[1,2-a]pyridin-6-yl)methyl)methylamine

A white solid compound **E-67** (41.4 mg, 66.3%) was prepared from intermediate **E67-1** using the method described in compound **E-34.**

LC-MS (ESI): m/z 459.2 [M+H]⁺ . ¹H NMR (400 MHz, DMSO-*d*₆): *δ* 9.66 (br s, 2H), 9.14 (s, 1H), 9.01 (s, 1H), 8.64 (s, 1H), 8.51 - 8.41 (m, 2H), 8.35 (s, 1H), 8.00 (d, *J=* 9.3 Hz, 1H), 7.87 (d, *J* = 9.3 Hz, 1H), 6.02 (s, 2H), 4.21 (t, *J* = 5.2 Hz, 2H), 3.81 - 3.72 (m, 4H), 3.48 - 3.40 (m, 4H), 3.00 - 2.88 (m, 2H), 2.72 (td, *J* = 7.5, 14.9 Hz, 1H), 2.11 - 2.01 (m, 2H), 1.89 - 1.74 (m, 4H).

### Example 68: Synthesis of E-68: 1-cyclobutyl-N-((2-((4-(5-(piperidin-1-yl)pyridin-3-yl)-1H-1,2,3-triazol-1-yl)methyl)imidazo[1,2-a]pyridin-6-yl)methyl)methylamine

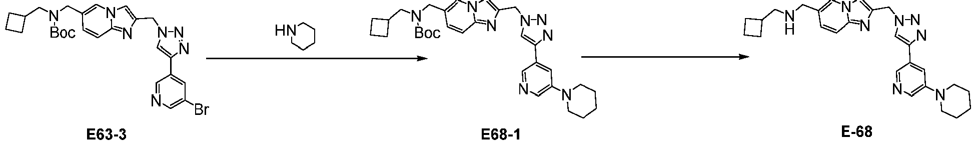

### E68-1: Tert-butyl (2-((4-(5-(piperidin-1-yl)pyridin-3-yl)-1H-1,2,3-triazol-1-yl)methyl)imidazo[1,2-a]pyridin-6-yl)methyl)carbamate

A yellow solid intermediate **E68-1** (50 mg, 49.6%) was prepared from intermediate **E63-3** using the method described in intermediate **E15-1.**

LC-MS (ESI): m/z 557.3 [M+H]⁺.

### E-68: 1-cyclobutyl-N-((2-((4-(5-(piperidin-1-yl)pyridin-3-yl)-1H-1,2,3-triazol-1-yl)methyl)imidazo[1,2-a]pyridin-6-yl)methyl)methylamine

A yellow solid compound **E-68** (22.4 mg, 50.6%) was prepared from intermediate **E68-1** using the method described in compound **E-1.**

**¹H NMR** (400 MHz, DMSO-*d*₆): *δ* 9.58 (br s, 2 H) 9.12 (s, 1 H) 8.95 (s, 1 H) 8.53 (s, 1 H) 8.37 - 8.43 (m, 2 H) 8.30 (s, 1 H) 7.88 - 7.95 (m, 1 H) 7.79 - 7.85 (m, 1 H) 5.98 (s, 2 H) 4.19 (t, *J =* 5.25 Hz, 2 H) 3.48 (br s, 4 H) 2.89 - 2.98 (m, 2 H) 2.65 - 2.76 (m, 1 H) 2.00 - 2.10 (m, 2 H) 1.75 - 1.88 (m, 4 H) 1.62 (br s, 6 H).

**LC-MS (ESI):** m/z 457.2 [M+H]⁺.

### Example 69: Synthesis of E-69: 1-cyclobutyl-N-((2-((4-(5-(pyrrolidin-1-yl)pyridin-3-yl)-1H-1,2,3-triazol-1-yl)methyl)imidazo[1,2-a]pyridin-6-yl)methyl)methylamine

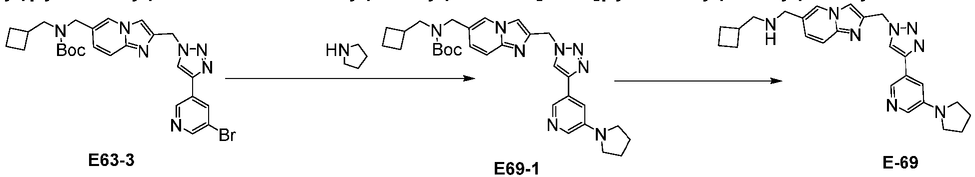

### E69-1: Tert-butyl (2-((4-(5-(pyrrolidine-1-yl)pyridin-3-yl)-1H-1,2,3-triazol-1-yl)methyl)imidazo[1,2-a]pyridin-6-yl)methyl)carbamate

A yellow solid intermediate **E69-1** (70 mg, 71.3%) was prepared from intermediate **E63-3** using the method described in intermediate **E15-1.**

LC-MS (ESI): m/z 543.3 [M+H]⁺.

### E-69: 1-cyclobutyl-N-((2-((4-(5-(pyrrolidin-1-yl)pyridin-3-yl)-1H-1,2,3-triazol-1-yl)methyl)imidazo[1,2-a]pyridin-6-yl)methyl)methylamine

A white solid compound **E-69** (26.6 mg, 42.7%) was prepared from intermediate **E69-1** using the method described in compound **E-34.**

LC-MS (ESI): m/z 443.3 [M+H]⁺ . ¹H NMR (400 MHz, DMSO-*d*₆): δ 9.62 (br s, 2H), 9.14 (s, 1H), 9.00 (s, 1H), 8.46 (d, *J* = 9.9 Hz, 2H), 8.06 (d, *J =* 2.5 Hz, 1H), 7.99 (d, *J =* 9.1 Hz, 1H), 7.94 (s, 1H), 7.87 (d, *J* = 9.4 Hz, 1H), 6.02 (s, 2H), 4.21 (t, *J* = 5.3 Hz, 2H), 3.42 (br t, *J* = 6.4 Hz, 4H), 2.98 - 2.91 (m, 2H), 2.80 - 2.64 (m, 1H), 2.09 - 2.00 (m, 6H), 1.89 - 1.76 (m, 4H).

### Example 70: Synthesis of E-70: 5-(1-((6-(((cyclobutylmethyl)amino)methyl)imidazo[1,2-a]pyridin-2-yl)methyl)-1H-1,2,3-triazole-4-yl)-N-methylpyridine-3-amine

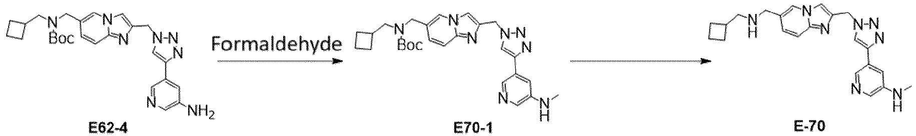

### E70-1: Tert-butyl (2-((4-(5-(methylamino)pyridin-3-yl)-1H-1,2,3-triazol-1-yl)methyl)imidazo[1,2-a]pyridin-6-yl)methyl)carbamate

A yellow solid intermediate **E70-1** (60 mg, 29.1%) was prepared from intermediate **E62-4** using the method described in intermediate **E5-1.**

**LC-MS (ESI):** m/z 503.3 [M+H]⁺.

### E-70: 5-(1-((6-(((cyclobutylmethyl)amino)methyl)imidazo[1,2-a]pyridin-2-yl)methyl)-1H-1,2,3-triazole-4-yl)-N-methylpyridine-3-amine

A white solid compound **E-70** (40.9 mg, 78.0%) was prepared from intermediate **E70-1** using the method described in compound **E-1.**

LC-MS (ESI): m/z 403.2 [M+H]⁺ . ¹H NMR (400 MHz, DMSO-*d*₆): *δ* 9.65 (br s, 2 H), 9.07 (s, 1 H), 9.00 (s, 1 H), 8.45 (d, *J* = 6.65 Hz, 2 H), 7.98 - 8.07 (m, 3 H), 7.87 (d, *J* = 9.29 Hz, 1 H), 6.02 (s, 2 H), 4.21 (t, *J* = 5.21 Hz, 2 H), 2.91 - 2.98 (m, 2 H), 2.85 (s, 3 H), 2.72 (dt, *J* = 15.00, 7.43 Hz, 1 H), 2.00 - 2.10 (m, 2 H), 1.73 - 1.89 (m, 4 H).

### Example 71: Synthesis of E-71: 1-cyclobutyl-N-((2-((4-(5-(methylthio)pyridin-3-yl)-1H-1,2,3-triazol-1-yl)methyl)imidazo[1,2-a]pyridin-6-yl)methyl)methylamine

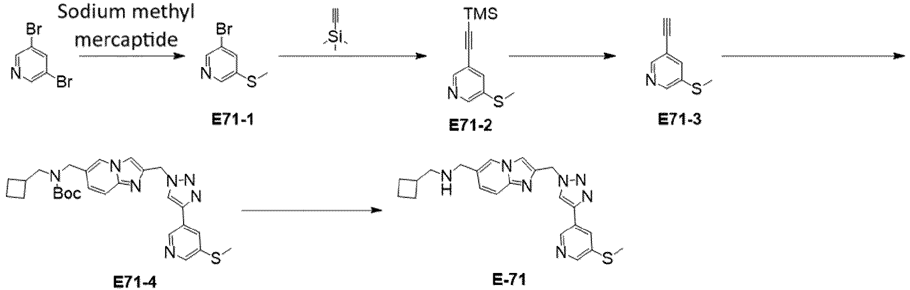

### E71-1: 3-bromo-5-(methylthio)pyridine

A yellow solid intermediate **E71-1** (8.30 g, crude product) was prepared from 3,5-dibromopyridine using the method described in intermediate **E1-2.**

LC-MS (ESI): m/z 204.0 [M+H]⁺.

### E71-2: 3-(methylthio)-5-((trimethylsilyl)ethynyl)pyridine

A yellow oily intermediate **E71-2** (4.9 g, 61.8%) was prepared from intermediate **E71-1** using the method described in intermediate **2-b.**

LC-MS (ESI): m/z 222.1 [M+H]⁺.

### E71-3: 3-ethynyl-5-(methylthio)pyridine

A colorless oily intermediate **E71-3** (2.9 g, 76.8%) was prepared from intermediate **E71-2** using the method described in intermediate **1-2.**

LC-MS (ESI): m/z 150.0 [M+H]⁺.

### E71-4: Tert-butyl (2-((4-(5-(methylthio)pyridin-3-yl)-1H-1,2,3-triazol-1-yl)methyl)imidazo[1,2-a]pyridin-6-yl)methyl)carbamate

A yellow solid intermediate **E71-4** (110 mg, 78.4%) was prepared from intermediate **E71-3** using the method described in intermediate **4-d.**

LC-MS (ESI): m/z 520.3 [M+H]⁺ . ¹H NMR (400 MHz, DMSO-*d*₆): *δ* 9.32 (br s, 2 H) 8.88 - 8.92 (m, 2 H) 8.86 (d, *J =* 1.75 Hz, 1 H) 8.48 (d, *J =* 2.25 Hz, 1 H) 8.36 (s, 1 H) 8.17 (t, *J* = 2.

### E-71: 1-cyclobutyl-N-((2-((4-(5-(methylthio)pyridin-3-yl)-1H-1,2,3-triazol-1-yl)m1-cyclobutyl-N-((2-((4-(5-(methylthio)pyridin-3-yl)-1H-1,2,3-triazol-1-yl)methyl)imidazo[1,2-a]pyridin-6-yl)methyl)methylaminomethyl)imidazo[1,2-a]pyridin-6-yl

A white solid compound **E-71** (63.9 mg, 60.1%) was prepared from intermediate **E71-4** using the method described in compound **E-1.**

LC-MS (ESI): m/z 420.1 [M+H]⁺ . ¹H NMR (400 MHz, DMSO-*d*₆): *δ* 9.32 (br s, 2 H) 8.88 - 8.92 (m, 2 H) 8.86 (d, *J =* 1.75 Hz, 1 H) 8.48 (d, *J* = 2.25 Hz, 1 H) 8.36 (s, 1 H) 8.17 (t, *J* = 2.00 Hz, 1 H) 7.80 - 7.89 (m, 2 H) 5.95 (s, 2 H) 4.19 (t, *J* = 5.00 Hz, 2 H) 2.91 - 2.97 (m, 2 H) 2.66 - 2.71 (m, 1 H) 2.59 - 2.60 (m, 3 H) 2.00 - 2.08 (m, 2 H) 1.81 - 1.88 (m, 1 H) 1.74 - 1.81 (m, 3 H).

### Example 72: Synthesis of E-72: 1-(2-((4-(5-(aziridin-1-yl)pyridin-3-yl)-1H-1,2,3-triazol-1-yl)methyl)imidazo[1,2-a]pyridin-6-yl)-N-(cyclobutylmethyl)methylamine

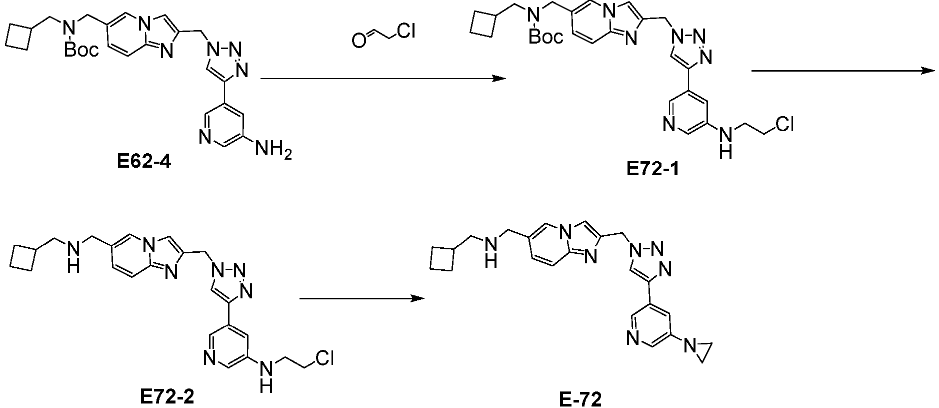

### E72-1: Tert-butyl (2-((4-(5-((2-chloroethyl)amino)pyridin-3-yl)-1H-1,2,3-triazol-1-yl)methyl)imidazo[1,2-a]pyridin-6-yl)methyl)(cyclobutylmethyl)carbamate

A yellow solid intermediate E72-1 (0.15 g, 26.6%) was prepared from intermediate **E62-4** using the method described in intermediate **E6-1.**

LC-MS (ESI): m/z 551.3 [M+H]⁺.

### E72-2: N-(2-chloroethyl)-5-(1-((6-(((cyclobutylmethyl)amino)methyl)imidazo[1,2-a]pyridin-2-yl)methyl)-1H-1,2,3-triazole-4-yl)pyridin-3-amine

A yellow solid intermediate **E72-2** (0.12 g, 90.4%) was prepared from intermediate **E72-1** using the method described in compound **E-1.**

LC-MS (ESI): m/z 451.2 [M+H]⁺.

### E-72: 1-(2-((4-(5-(aziridin-1-yl)pyridin-3-yl)-1H-1,2,3-triazol-1-yl)methyl)imidazo[1,2-a]pyridin-6-yl)-N-(cyclobutylmethyl)methylamine

A yellow solid intermediate **E-72** (33.0 mg, 44.7%) was prepared from intermediate **E72-2** using the method described in compound **E-6.**

LC-MS (ESI): m/z 415.2 [M+H]⁺ . ¹H NMR (400 MHz, DMSO-*d*₆): *δ* 8.71 (s, 1H), 8.66 (d, *J* = 1.6 Hz, 1H), 8.42 (s, 1H), 8.25 (d, *J* = 2.5 Hz, 1H), 7.97 (s, 1H), 7.81 (d, *J* = 1.9 Hz, 1H), 7.46 (d, *J* = 9.1 Hz, 1H), 7.26 (d, *J* = 9.3 Hz, 1H), 5.75 (s, 2H), 3.65 (s, 2H), 2.49 (br s, 2H), 2.44 - 2.37 (m, 1H), 2.17 (s, 4H), 2.01 - 1.93 (m, 2H), 1.86 - 1.77 (m, 2H), 1.66 - 1.58 (m, 1H).

### Example 73: Synthesis of E-73: 1-cyclobutyl-N-((2-((4-(5-(piperazin-1-yl)pyridin-3-yl)-1H-1,2,3-triazol-1-yl)methyl)imidazo[1,2-a]pyridin-6-yl)methyl)methylamine hydrochloride

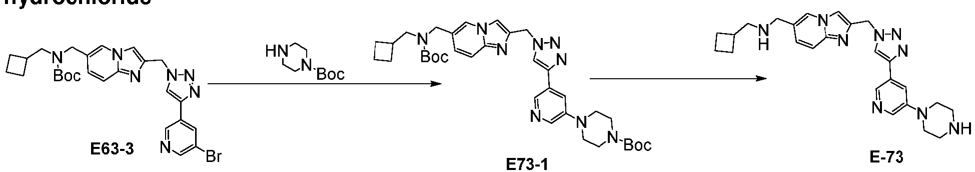

### E73-1: Tert-butyl 4-(5-(1-((6-(((tert-butoxycarbonyl)(cyclobutylmethyl)amino)methyl)imidazo[1,2-a]pyridin-2-yl)methyl)-1H-1,2,3-triazol-4-yl)pyridin-3-yl)piperazine-1-carboxylate

A yellow solid intermediate **E73-1** (100 mg, 42.0%) was prepared from intermediate **E63-3** using the method described in intermediate **E15-1.**

LC-MS (ESI): m/z 658.4 [M+H]⁺.

### E-73: 1-cyclobutyl-N-((2-((4-(5-(piperazin-1-yl)pyridin-3-yl)-1H-1,2,3-triazol-1-yl)methyl)imidazo[1,2-a]pyridin-6-yl)methyl)methylamine

A yellow solid compound **E-73** (19.3 mg, 25.7%) was prepared from intermediate **E73-1** using the method described in compound **E-1.**

LC-MS (ESI): m/z 458.2 [M+H]⁺ . ¹H NMR (400 MHz, DMSO-*d*₆): *δ* 9.59 - 9.79 (m, 4 H) 9.19 (s, 1 H) 9.06 (s, 1 H) 8.66 (s, 1 H) 8.51 (s, 2 H) 8.42 (s, 1 H) 8.09 (d, *J* = 9.18 Hz, 1 H) 7.92 (d, *J= 9.42* Hz, 1 H) 6.06 (s, 2 H) 4.22 (t, *J* = 5.19 Hz, 2 H) 3.70 - 3.82 (m, 4 H) 3.22 (br s, 4 H) 2.90 - 2.98 (m, 2 H) 2.66 - 2.78 (m, 1 H) 2.00 - 2.09 (m, 2 H) 1.72 - 1.91 (m, 4 H).

### Example 74: Synthesis of E-74: 1-cyclobutyl-N-((6-((4-(5-(methylthio)pyridin-3-yl)-1H-1,2,3-triazol-1-yl)methyl)-1H-indole-2-yl)methyl)methylamine

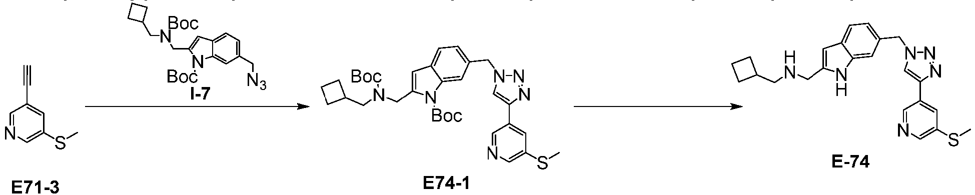

### E74-1: Tert-butyl 2-(((tert-butoxycarbonyl)(cyclobutylmethyl)amino)methyl)-6-((4-(5-(methylthio)pyridin-3-yl)-1H-1,2,3-triazol-1-yl)methyl)-1H-indole-1-carboxylate

A yellow solid intermediate **E74-1** (50 mg, 37.9%) was prepared from intermediate **E71-3** using the method described in intermediate **4-d.**

LC-MS (ESI): m/z 619.3 [M+H]⁺.

### E-74: 1-cyclobutyl-N-((6-((4-(5-(methylthio)pyridin-3-yl)-1H-1,2,3-triazol-1-yl)methyl)-1H-indole-2-yl)methyl)methylamine

A white solid compound **E-74** (23.8 mg, 64.3%) was prepared from intermediate **E74-1** using the method described in compound **E-1.**

LC-MS (ESI): m/z 419.1 [M+H]⁺ . ¹H NMR (400 MHz, DMSO-*d*₆): *δ* 9.32 (br s, 2 H) 8.88 - 8.92 (m, 2 H) 8.86 (d, *J* =1.75 Hz, 1 H) 8.48 (d, *J* =2.25 Hz, 1 H) 8.36 (s, 1 H) 8.17 (t, *J* =2.00 Hz, 1 H) 7.80 - 7.89 (m, 2 H) 5.95 (s, 2 H) 4.19 (t, J=5.00 Hz, 2 H) 2.91 - 2.97 (m, 2 H) 2.66 - 2.71 (m, 1 H) 2.59 - 2.60 (m, 3 H) 2.00 - 2.08 (m, 2 H) 1.81 - 1.88 (m, 1 H) 1.74 - 1.81 (m, 3 H).

### Example 75: Synthesis of E-75: 1-cyclobutyl-N-((6-((4-(5-iodopyridin-3-yl)-1H-1,2,3-triazol-1-yl)methyl)-1H-indole-2-yl)methyl)methylamine

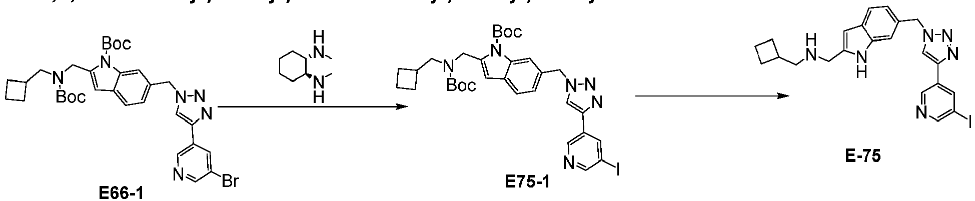

### E75-1: Tert-butyl 2-(((tert-butoxycarbonyl)(cyclobutylmethyl)amino)methyl)-6-((4-(5-iodopyridin-3-yl)-1H-1,2,3-triazol-1-yl)methyl)-1H-indole-1-carboxylate

A yellow oily intermediate **E75-1** (80 mg, 74.6%) was prepared from intermediate **E66-1** using the method described in intermediate **E24-1.**

**LC-MS (ESI):** m/z 699.3 [M+H]⁺.

### E-75: 1-cyclobutyl-N-((6-((4-(5-iodopyridine-3-yl)-1H-1,2,3-triazol-1-yl)methyl)-1H-indole-2-yl)methyl)methylamine

A yellow solid compound **E-75** (26.7 mg, 43.6%) was prepared from intermediate **E75-1** using the method described in compound **E-3.**

LC-MS (ESI): m/z 499.0 [M+H]⁺ . ¹H NMR (400 MHz, DMSO-*d*₆): *δ* 11.40 (s, 1 H) 9.12 (br s, 1 H) 9.04 (d, *J* = 1.75 Hz, 1 H) 8.81 (s, 1 H) 8.76 (d, *J* = 1.88 Hz, 1 H) 8.60 (t, *J* = 1.88 Hz, 1 H) 7.57 (d, *J* = 8.25 Hz, 1 H) 7.46 (s, 1 H) 7.06 (d, *J* = 8.25 Hz, 1 H) 6.60 (s, 1 H) 5.73 (s, 2 H) 4.25 - 4.28 (m, 2 H) 2.92 - 2.99 (m, 2 H) 2.59 - 2.64 (m, 1 H) 2.00 - 2.07 (m, 2 H) 1.79 - 1.91 (m, 2 H) 1.74 - 1.78 (m, 2 H).

### Example 76: Synthesis of E-76: 1-cyclobutyl-N-((2-((4-(5-iodopyridine-3-yl)-1H-1,2,3-triazol-1-yl)methyl)imidazo[1,2-a]pyridin-6-yl)methyl)methylamine

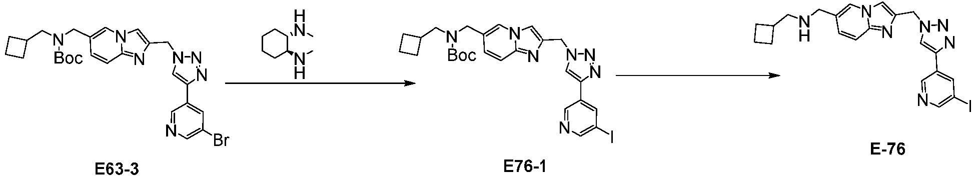

### E76-1: Tert-butyl (2-((4-(5-iodopyridin-3-yl)-1H-1,2,3-triazol-1-yl)methyl)imidazo[1,2-a]pyridin-6-yl)methyl)carbamate

A yellow solid intermediate **E76-1** (80 mg, 73.7%) was prepared from intermediate **E63-3** using the method described in intermediate **E24-1.**

LC-MS (ESI): m/z 600.2 [M+H]⁺.

### E-76: 1-cyclobutyl-N-((2-((4-(5-iodopyridine-3-yl)-1H-1,2,3-triazol-1-yl)methyl)imidazo[1,2-a]pyridin-6-yl)methyl)methylamine

A white solid compound **E-76** (59.6 mg, 83.3%) was prepared from intermediate **E76-1** using the method described in compound **E-1.**

LC-MS (ESI): m/z 500.1 [M+H]⁺ . ¹H NMR (400 MHz, DMSO-*d*₆): *δ* 9.60 (br s, 2 H) 9.06 (d, *J* = 1.83 Hz, 1 H) 9.01 (s, 1 H) 8.94 (s, 1 H) 8.79 (d, *J* = 1.96 Hz, 1 H) 8.61 (t, *J* = 1.96 Hz, 1 H) 8.45 (s, 1 H) 8.05 (d, *J* = 9.29 Hz, 1 H) 7.91 (d, *J* = 9.29 Hz, 1 H) 6.00 (s, 2 H) 4.22 (t, *J* = 5.38 Hz, 2 H) 2.90 - 2.98 (m, 2 H) 2.65 - 2.77 (m, 1 H) 1.99 - 2.10 (m, 2 H) 1.71 - 1.91 (m, 4 H).

### Example 77: Synthesis of E-77: N-((2-(((cyclobutylmethyl)amino)methyl)-1H-indole-6-yl)methyl)-5-(dimethylamino)nicotinamide

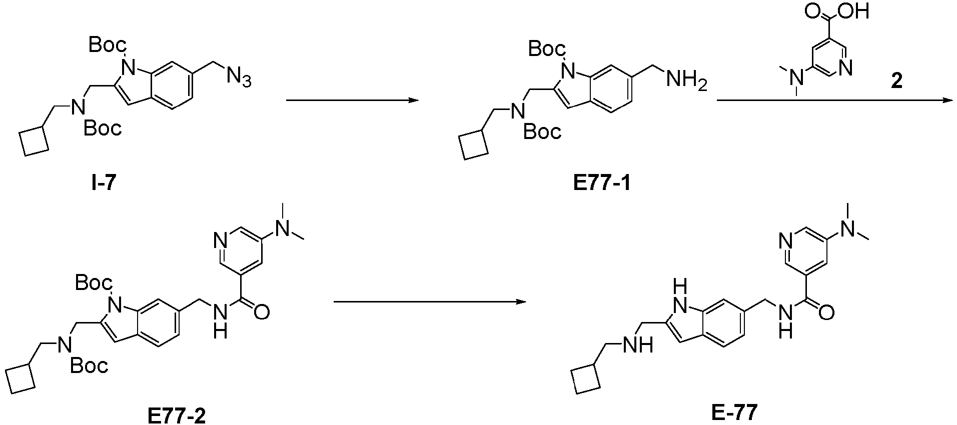

### E77-1: Tert-butyl 6-(aminomethyl)-2-(((tert-butoxycarbonyl)(cyclobutylmethyl)amino)methyl)-1H-indole-1-carboxylate

A yellow oily intermediate **E77-1** (60 mg, 63.5%) was prepared from intermediate **I-7** using the method described in intermediate **1-10.**

LC-MS (ESI): m/z 442.6 [M+H]⁺.

### E77-2: Tert-butyl 2-(((tert-butoxycarbonyl)(cyclobutylmethyl)amino)methyl)-6-((5-(dimethylamino)nicotinamido)methyl)-1H-indole-1-carboxylate

A yellow oily intermediate **E77-2** (80 mg, 99.9%) was prepared from intermediate **E77-1** using the method described in intermediate **E7-3.**

LC-MS (ESI): m/z 592.3 [M+H]⁺.

### E-77: N-((2-(((cyclobutylmethyl)amino)methyl)-1H-indole-6-yl)methyl)-5-(dimethylamino)nicotinamide

A white solid compound **E-77** (14.6 mg, 25.0%) was prepared from intermediate **E77-2** using the method described in compound **E-3.**

LC-MS (ESI): m/z 392.2 [M+H]⁺ . ¹H NMR (400 MHz, DMSO-*d*₆): *δ* 8.28 (s, 1H), 8.15 (br s, 1H), 7.54 (s, 1H), 7.46 (d, *J* = 8.1 Hz, 1H), 7.35 (s, 1H), 7.03 (d, *J* = 7.5 Hz, 1H), 6.36 (s, 1H), 4.66 (s, 2H), 3.94 (s, 2H), 3.04 (s, 6H), 2.71 (d, *J* = 7.3 Hz, 2H), 2.53 (td, *J* = 7.7, 15.3 Hz, 1H), 2.15 - 2.04 (m, 2H), 1.98 - 1.78 (m, 2H), 1.76 - 1.64 (m, 2H).

### Example 78: Synthesis of E-78: 1-(5-(1-((6-(((cyclobutylmethyl)amino)methyl)imidazo[1,2-a]pyridin-2-yl)methyl)-1H-1,2,3-triazol-4-yl)pyridin-3-yl)pyrrolidine-2-nitrile

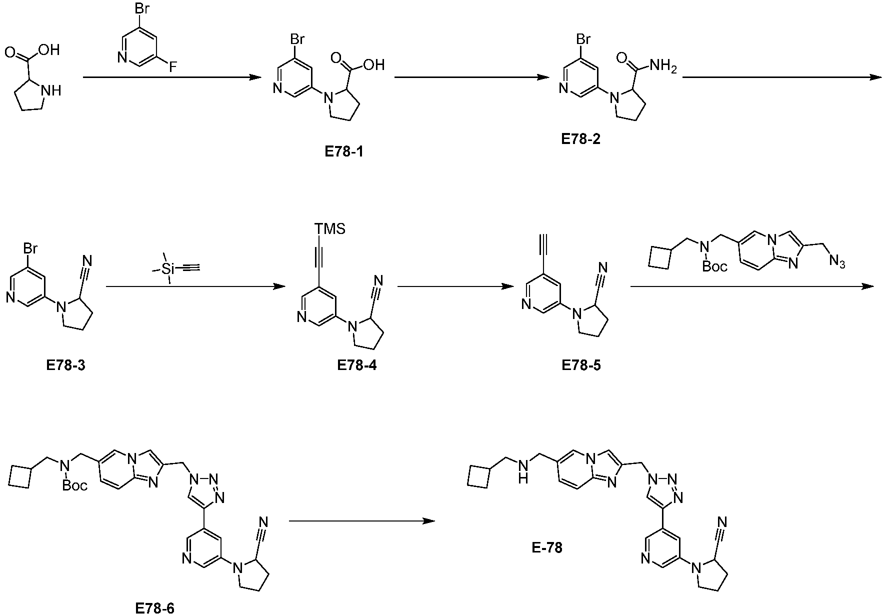

### E78-1: (5-bromopyridin-3-yl)proline

White solid **E78-1** (1.4 g, 59.5%) was prepared from proline using the method described in **E16-1.**

LC-MS: [M+H]⁺= 271.0.

### E78-2: 1-(5-bromopyridin-3-yl)pyrrolidine-2-carboxamide

EDCI (1.98 g, 10.3 mmol, 2.00 eq) was added to a solution of **E78-1** (1.40 g, 5.16 mmol, 1.00 eq) and ammonium chloride (414 mg, 7.75 mmol, 1.50 eq) in pyridine (14.0 mL), and the resulting mixture was stirred at 25°C for 16 h. The reaction mixture was quenched by adding water (50.0 mL) and extracted with ethyl acetate (20.0 mL × 3). The merged organic layers were dried over anhydrous sodium sulfate, filtered and concentrated. The residue was purified by column chromatography (petroleum ether/ethyl acetate=50/1 to 0/1) to obtain yellow solid **E78-2** (0.82 g, 58.8%).

LC-MS: [M+H]⁺= 271.9.

### E78-3: 1-(5-bromopyridin-3-yl)pyrrolidine-2-nitrile

Triethylamine (408 mg, 1.94 mmol, 270 µL, 1.50 eq) was added to a solution of **E78-2** (0.35 g, 1.30 mmol, 1.00 eq) in THF (7.00 mL) at 0°C, and the resulting mixture was stirred at 25°C for 3 h. Then, sodium carbonate (549 mg, 5.18 mmol, 4.00 eq) was added to the reaction mixture at 0°C and stirred at 0°C for 1 h. The reaction mixture was concentrated, and the residue was diluted with water (10.0 mL) and extracted with ethyl acetate (3.00 mL × 3). The combined organic layers were dried over anhydrous sodium sulfate, filtered and concentrated. The residue was purified by preparative thin-layer chromatography (petroleum ether: ethyl acetate=1:1) to obtain yellow solid **E78-3** (0.30 g, 91.8%).

LC-MS: [M+H]⁺= 253.9.

### E78-4: 1-(5-((trimethylsilyl)ethynyl)pyridin-3-yl)pyrrolidine-2-nitrile

Yellow solid **E78-4** (270 mg, 84.2%) was prepared from **E78-3** using the method described in **E53-6.**

LC-MS: [M+H]⁺= 270.2.

### E78-5: 1-(5-ethynylpyridine-3-yl)pyrrolidine-2-nitrile

Yellow solid **E78-5** (40 mg, crude product) was prepared from **E78-4** using the method described in **1-2.**

LC-MS: [M+H]⁺= 198.2.

### E78-6: Tert-butyl (2-((4-(5-(2-cyanopyrrolidin-1-yl)pyridin-3-yl)-1H-1,2,3-triazol-1-yl)methyl)imidazo[1,2-a]pyridin-6-yl)methyl)(cyclobutylmethyl)carbamate

Yellow solid **E78-6** (50 mg, 43.4%) was prepared from **E78-5** using the method described in **4-d.**

LC-MS: [M+H]⁺= 568.4.

### E-78: 1-(5-(1-((6-(((cyclobutylmethyl)amino)methyl)imidazo[1,2-a]pyridin-2-yl)methyl)-1H-1,2,3-triazol-4-yl)pyridin-3-yl)pyrrolidine-2-nitrile

Yellow solid **E-78** (6.40 mg, 37.0 %) was prepared from **E78-6** using the method described in **E-94.**

LC-MS: [M+H]⁺= 468.2. ¹H NMR (400 MHz, DMSO-*d*₆): *δ* 8.72 (s, 1H), 8.50 (d, *J* = 1.4 Hz, 1H), 8.42 (s, 1H), 8.07 (d, *J* = 2.8 Hz, 1H), 7.97 (s, 1H), 7.55-7.52 (m, 1H), 7.46 (d, *J*= 9.3 Hz, 1H), 7.26 (dd, *J* = 1.4, 9.3 Hz, 1H), 5.75 (s, 2H), 5.01 (dd, *J* = 2.7, 6.7 Hz, 1H), 3.64 (s, 2H), 3.50-3.38 (m, 2H), 2.48 (br s, 2H), 2.42 (s, 1H), 2.36-2.25 (m, 2H), 2.20-2.04 (m, 2H), 2.02-1.89 (m, 2H), 1.84-1.70 (m, 2H), 1.66-1.54 (m, 2H).

### Example 79: Synthesis of E-79: 1-cyclobutyl-N-((6-((4-(5-(pyrrolidine-1-yl)pyridin-3-yl)-1H-1,2,3-triazol-1-yl)methyl)-1H-indole-2-yl)methyl)methylamine hydrochloride

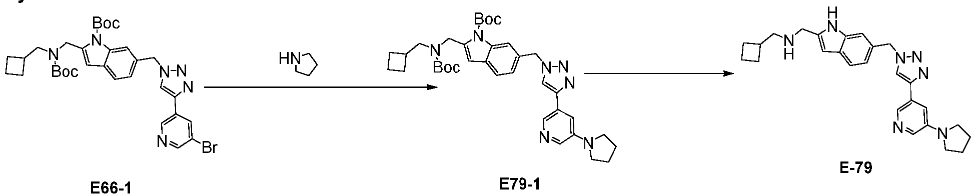

### E79-1: Tert-butyl 2-(((tert-butoxycarbonyl)(cyclobutylmethyl)amino)methyl)-6-((4-(5-(pyrrolidine-1-yl)pyridin-3-yl)-1H-1,2,3-triazol-1-yl)methyl)-1H-indole-1-carboxylate

A white solid intermediate **E79-1** (30 mg, 30.5%) was prepared from intermediate **E66-1** using the method described in intermediate **E15-1.**

LC-MS (ESI): m/z 642.2 [M+H]⁺.

### E-79: 1-cyclobutyl-N-((6-((4-(5-(pyrrolidine-1-yl)pyridin-3-yl)-1H-1,2,3-triazol-1-yl)methyl)-1H-indole-2-yl)methyl)methylamine hydrochloride

A yellow solid compound **E-79** (1.3 mg, 6.18%) was prepared from intermediate **E79-1** using the method described in compound **E-1.**

LC-MS (ESI): m/z 442.1 [M+H]⁺ . ¹H NMR (400 MHz, DMSO-*d*₆): δ 11.55 (s, 1 H) 9.30 (d, *J* = 3.00 Hz, 2 H) 8.94 (s, 1 H) 8.45 (br s, 1 H) 8.01 (br s, 1 H) 7.86 (s, 1 H) 7.57 (d, *J =* 8.13 Hz, 1 H) 7.48 (s, 1 H) 7.06 (dd, *J* = 8.19, 1.19 Hz, 1 H) 6.60 (s, 1 H) 5.77 (s, 2 H) 4.27 (t, *J* = 5.07 Hz, 2 H) 3.38 - 3.41 (m, 4 H) 2.89 - 2.97 (m, 2 H) 2.61 - 2.70 (m, 1 H) 1.98 - 2.08 (m, 6 H) 1.73 - 1.88 (m, 4 H).

### Example 80: Synthesis of E-80: 1-cyclobutyl-N-((6-((4-(5-(methylsilyl)pyridin-3-yl)-1H-1,2,3-triazol-1-yl)methyl)-1H-indole-2-yl)methyl)methylamine

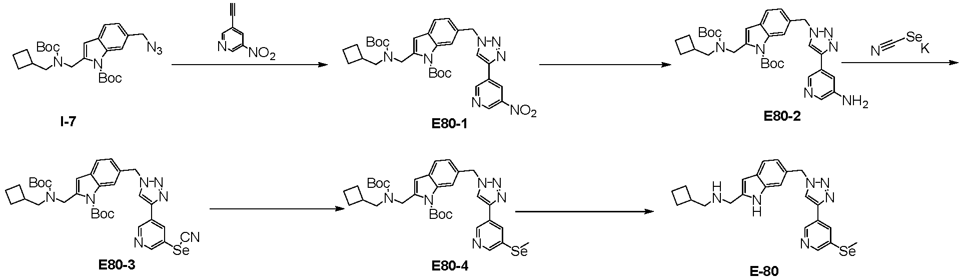

### E80-1: Tert-butyl 2-(((tert-butoxycarbonyl)(cyclobutylmethyl)amino)methyl)-6-((4-(5-nitropyridin-3-yl)-1H-1,2,3-triazol-1-yl)methyl)-1H-indole-1-carboxylate

A yellow solid intermediate **E80-1** (1.10 g, 83.6%) was prepared from intermediate **I-7** using the method described in intermediate **4-d.**

¹H NMR: (400 MHz, CDCl₃): *δ* 9.17 - 9.32 (m, 2 H), 8.79 (t, *J=* 2.13 Hz, 1 H), 8.13 (br s, 1 H), 7.72 (br s, 1 H), 7.43 (d, *J* = 7.25 Hz, 1 H), 7.13 (br s, 1 H), 6.22 (br s, 1 H), 5.64 (br s, 2 H), 4.59 - 4.72 (m, 2 H), 3.30 (d, *J* = 6.25 Hz, 2 H), 2.43 - 2.57 (m, 1 H), 1.95 (br s, 2 H), 1.74 - 1.84 (m, 2 H), 1.63 - 1.72 (m, 2 H), 1.59 (br s, 9 H), 1.29 - 1.46 (m, 9 H).

### E80-2: Tert-butyl 6-((4-(5-aminopyridin-3-yl)-1H-1,2,3-triazol-1-yl)methyl)-2-(((tert-butoxycarbonyl)(cyclobutylmethyl)amino)methyl)-1H-indole-1-carboxylate

A yellow solid intermediate **E80-2** (1 g, 86.0%) was prepared from intermediate **E80-1** using the method described in intermediate **E3-2.**

¹H NMR: (400 MHz, CDCl₃): *δ* 8.17 - 8.24 (m, 1 H), 8.06 (br s, 1 H), 7.95 (d, *J=* 2.38 Hz, 1 H), 7.58 (br s, 1 H), 7.48 (br s, 1 H), 7.41 (d, *J* = 6.00 Hz, 1 H), 7.05 - 7.16 (m, 1 H), 6.23 (d, *J* = 14.26 Hz, 1 H), 5.60 (br s, 2 H), 4.65 (d, *J* = 16.76 Hz, 2 H), 3.69 (br s, 2 H), 3.29 (d, *J* = 6.50 Hz, 2 H), 2.51 (br s, 1 H), 1.95 (br s, 2 H), 1.74 - 1.83 (m, 2 H), 1.63 - 1.71 (m, 2 H), 1.57 (s, 9 H), 1.28 - 1.46 (m, 9 H).

### E80-3: Tert-butyl 2-(((tert-butoxycarbonyl)(cyclobutylmethyl)amino)methyl)-6-((4-(5-selenocyanopyridin-3-yl)-1H-1,2,3-triazol-1-yl)methyl)-1H-indole-1-carboxylate

A red solid intermediate **E80-3** (0.11 g, crude product) was prepared from intermediate **E80-2** using the method described in intermediate **E14-1.**

LC-MS (ESI): m/z 677.2 [M+H]⁺.

### E80-4: Tert-butyl 2-(((tert-butoxycarbonyl)(cyclobutylmethyl)amino)methyl)-6-((4-(5-(methylsilyl)pyridin-3-yl)-1H-1,2,3-triazol-1-yl)methyl)-1H-indole-1-carboxylate

A yellow oily intermediate **E80-4** (70 mg, 64.7%) was prepared from intermediate **E80-3** using the method described in intermediate **E14-2.**

¹H NMR: (400 MHz, CDCl₃): *δ* 8.66 (br s, 1 H), 8.48 (br s, 1 H), 8.16 (br s, 1 H), 8.03 - 8.10 (m, 1 H), 7.58 - 7.71 (m, 1 H), 7.41 (d, *J* = 7.38 Hz, 1 H), 7.06 - 7.18 (m, 2 H), 6.18 - 6.31 (m, 1 H), 5.61 (br s, 2 H), 4.58 - 4.71 (m, 2 H), 3.29 (d, *J* = 6.00 Hz, 2 H), 2.45 - 2.56 (m, 1 H), 2.32 - 2.37 (m, 3 H), 2.15 - 2.16 (m, 1 H), 1.94 (d, *J* = 5.63 Hz, 2 H), 1.73 - 1.84 (m, 2 H), 1.63 - 1.70 (m, 2 H), 1.58 (s, 9 H), 1.29 - 1.47 (m, 7 H).

### E-80: 1-cyclobutyl-N-((6-((4-(5-(methylsilyl)pyridin-3-yl)-1H-1,2,3-triazol-1-yl)methyl)-1H-indole-2-yl)methyl)methylamine

A white solid compound **E-80** (17.5 mg, 32.6%) was prepared from intermediate **E80-4** using the method described in compound **E-1.**

LC-MS (ESI): m/z 467.0 [M+H]⁺ . ¹H NMR (400 MHz, DMSO-*d*₆): *δ* 11.59 (s, 1 H), 9.39 (br s, 2 H), 8.96 (d, *J* = 1.88 Hz, 1 H), 8.87 (s, 1 H), 8.63 (d, *J* = 2.13 Hz, 1 H), 8.44 (t, *J* = 1.88 Hz, 1 H), 7.57 (d, *J* = 8.13 Hz, 1 H), 7.47 (s, 1 H), 7.07 (dd, *J* = 8.13, 1.38 Hz, 1 H), 6.61 (d, *J* = 1.00 Hz, 1 H), 5.76 (s, 2 H), 4.27 (t, *J* = 5.32 Hz, 2 H), 2.89 - 2.98 (m, 2 H), 2.62 - 2.72 (m, 1 H), 2.50 (br s, 3 H), 1.98 - 2.11 (m, 2 H), 1.72 - 1.90 (m, 4 H).

### Example 81: Synthesis of E-81: 1-cyclobutyl-N-((2-((4-(5-(methylsilyl)pyridin-3-yl)-1H-1,2,3-triazol-1-yl)methyl)imidazo[1,2-a]pyridin-6-yl)methyl)methylamine

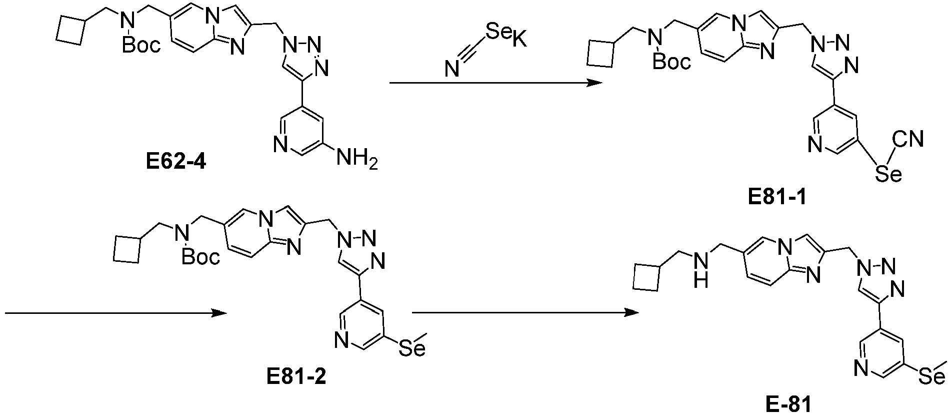

### E81-1: Tert-butyl (2-((4-(5-selenocyanopyridin-3-yl)-1H-1,2,3-triazol-1-yl)methyl)imidazo[1,2-a]pyridin-6-yl)methyl)carbamate

A yellow solid intermediate **E81-1** (30 mg, 8.46%) was prepared from intermediate **E62-4** using the method described in intermediate **E14-1.**

LC-MS (ESI): m/z 579.2 [M+H]⁺.

### E81-2: Tert-butyl (2-((4-(5-(methylsilyl)pyridin-3-yl)-1H-1,2,3-triazol-1-yl)methyl)imidazo[1,2-a]pyridin-6-yl)methyl)carbamate

A yellow solid intermediate **E81-2** (20 mg, 67.9%) was prepared from intermediate **E81-1** using the method described in intermediate **E14-2.**

LC-MS (ESI): m/z 568.1 [M+H]⁺.

### E-81: 1-cyclobutyl-N-((2-((4-(5-(methylsilyl)pyridin-3-yl)-1H-1,2,3-triazol-1-yl)methyl)imidazo[1,2-a]pyridin-6-yl)methyl)methylamine

A white solid compound **E-81** (11.5 mg, 61.0%) was prepared from intermediate **E81-2** using the method described in compound **E-1.**

LC-MS (ESI): m/z 468.1 [M+H]⁺ . ¹H NMR (400 MHz, DMSO-*d*₆): *δ* 9.27 (s, 2 H) 8.92 (d, *J* = 1.67 Hz, 1 H) 8.89 (s, 1 H) 8.87 (s, 1 H) 8.59 (d, *J =* 1.91 Hz, 1 H) 8.33 (d, J=2.03 Hz, 2 H) 7.80 (s, 2 H) 5.93 (s, 2 H) 4.19 (t, *J* = 5.36 Hz, 2 H) 2.96 (d, *J* = 5.13 Hz, 2 H) 2.62 - 2.74 (m, 1 H) 2.48 (s, 3 H) 2.02 - 2.09 (m, 2 H) 1.80 (s, 2 H) 1.78 (s, 2 H).

### Example 82: Synthesis of E-82: 1-(2-((4-(4-chloro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

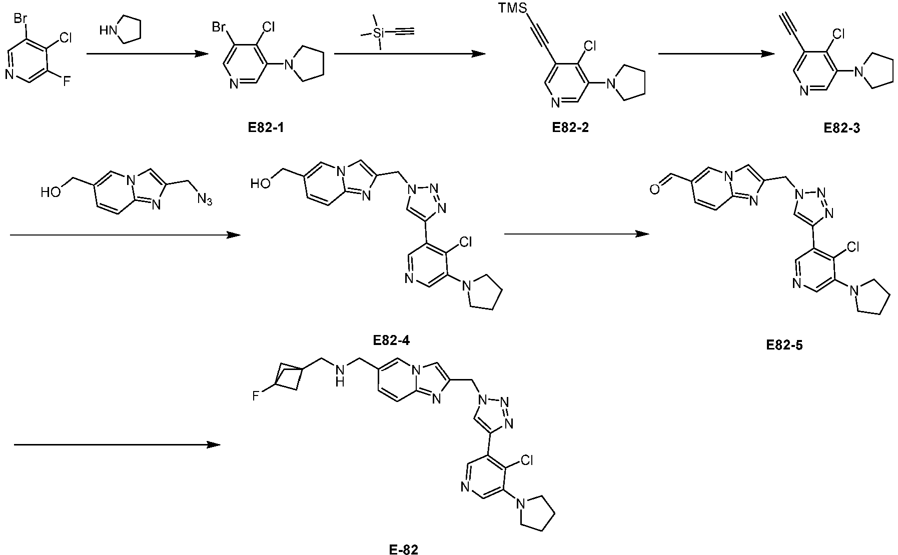

### E82-1: 3-bromo-4-chloro-5-(pyrrolidin-1-yl)pyridine

White solid **E82-1** (500 mg, 44.6%) was prepared from 3-bromo-4-chloro-5-fluoropyridine using the method described in **E16-1.**

LC-MS: [M+H]⁺= 262.9.

### E82-2: 4-chloro-3-(pyrrolidin-1-yl)-5-((trimethylsilyl)ethynyl)pyridine

Brown solid **E82-2** (180 mg, 60.8%) was prepared from **E82-1** using the method described in the **E53-6.**

LC-MS: [M+H]⁺= 279.0.

### E82-3: 4-chloro-3-ethynyl-5-(pyrrolidin-1-yl)pyridine

A solution of **E82-3** (140 mg, crude product) was prepared from **E82-2** using the method described in **1-2.**

LC-MS: [M+H]⁺= 207.1.

### E82-4: (2-((4-(4-chloro-5-(pyrrolidin-1-yl)pyridin-3-yl)-1H-1,2,3-triazol-1-yl)methyl)imidazo[1,2-a]pyridin-6-yl)methanol

Yellow solid **E82-4** (60 mg, 81.8%) was prepared from **E82-3** using the method described in **4-d.**

LC-MS: [M+H]⁺= 410.1.

### E82-5: 2-((4-(4-chloro-5-(pyrrolidin-1-yl)pyridin-3-yl)-1H-1,2,3-triazol-1-yl)methyl)imidazo[1,2-a]pyridine-6-formaldehyde

Gray solid **E82-5** (15 mg, 37.6%) was prepared from **E82-4** using the method described in **E143-2.**

LC-MS: [M+H]⁺= 408.1.

### E-82: 1-(2-((4-(4-chloro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

Yellow oily **E-82** (1.40 mg, 7.45%) was prepared from **E82-5** using the method described in **E6-1.**

LC-MS: [M+H]⁺= 507.2. ¹H NMR (400 MHz, DMSO-*d*₆): *δ* 8.71 (s, 1H), 8.52 (s, 1H), 8.42 (s, 1H), 8.20 (s, 1H), 7.94 (s, 1H), 7.46 (d, *J* = 9.4 Hz, 1H), 7.26 (d, *J* = 7.9 Hz, 1H), 5.78 (s, 2H), 3.67 (s, 2H), 3.43-3.39 (m, 4H), 2.74 (s, 2H), 1.97-1.87 (m, 10H).

### Example 83: Synthesis of E-83: 6-((2,2-dimethylpiperidin-1-yl)methyl)-2-((4-(5-(pyrrolidin-1-yl)pyridin-3-yl)-1H-1,2,3-triazol-1-yl)methyl)imidazo[1,2-a]pyridine hydrochloride

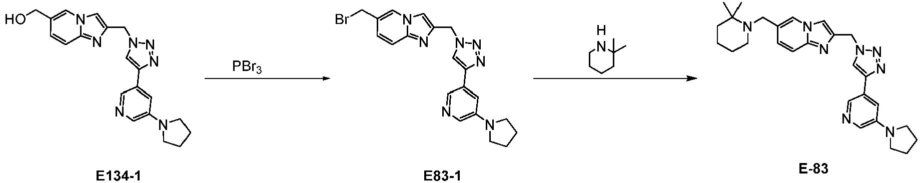

### E83-1: 6-(bromomethyl)-2-((4-(5-(pyrrolidin-1-yl)pyridin-3-yl)-1H-1,2,3-triazol-1-yl)methyl)imidazo[1,2-a]pyridine

Brown solid **E83-1** (400 mg, crude product) was prepared from **E134-1** using the method described in **E141-2.**

LC-MS: [M+H]⁺= 438.1.

### E-83: 6-((2,2-dimethylpiperidin-1-yl)methyl)-2-((4-(5-(pyrrolidin-1-yl)pyridin-3-yl)-1H-1,2,3-triazol-1-yl)methyl)imidazo[1,2-a]pyridine hydrochloride

Yellow solid **E-83** (0.80 mg, 1.38%) was prepared from **E83-1** using the method described in **E16-1.**

LC-MS: [M+H]⁺= 471.3. ¹H NMR (400 MHz, DMSO-*d*₆): *δ* 9.88-9.57 (m, 1H), 8.96 (s, 1H), 8.83 (s, 1H), 8.50-8.44 (m, 1H), 8.17 (s, 1H), 8.02 (d, *J* = 2.6 Hz, 1H), 7.83 (br s, 1H), 7.69-7.60 (m, 1H), 7.52 (d, *J* = 9.3 Hz, 1H), 5.85 (s, 2H), 4.68-4.60 (m, 1H), 3.96 (dd, *J* = 8.7, 13.3 Hz, 1H), 3.11-3.05 (m, 2H), 2.96-2.88 (m, 2H), 2.56-2.54 (m, 2H), 2.02 (t, *J* = 6.6 Hz, 4H), 1.94-1.87 (m, 1H), 1.77-1.63 (m, 3H), 1.61 (s, 3H), 1.59 (br s, 2H), 1.40 (s, 3H).

### Example 84: Synthesis of E-84: 2-((4,4-dimethylpiperidin-1-yl)methyl)-6-((4-(5-(2-methylpyrrolidin-1-yl)pyridin-3-yl)-1H-1,2,3-triazol-1-yl)methyl)-1H-indole

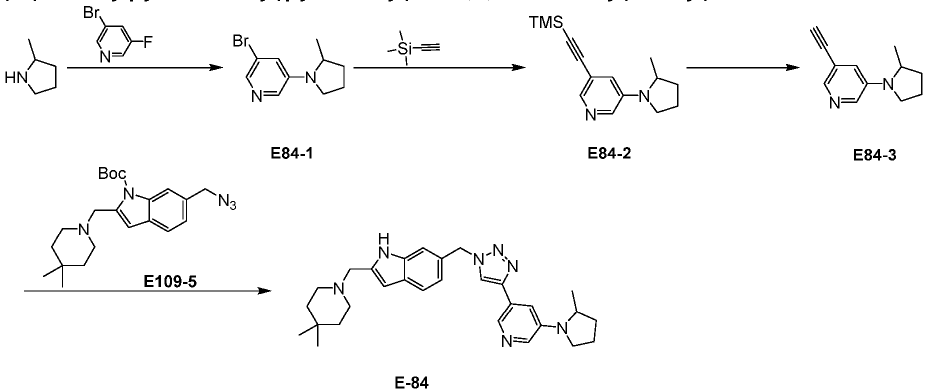

### E84-1: 3-bromo-5-(2-methylpyrrolidin-1-yl)pyridine

White solid **E84-1** (250 mg, 18.0%) was prepared from 2-methylpyrrolidine and 3-bromo-5-fluoropyridine using the method described in **E16-1.**

LC-MS: [M+H]⁺= 240.9.

### E84-2: 3-(2-methylpyrrolidin-1-yl)-5-((trimethylsilyl)ethynyl)pyridine

Brown solid **E84-2** (70 mg, 40.8%) was prepared from **E84-1** using the method described in **E53-6.**

LC-MS: [M+H]⁺= 259.3.

### E84-3: 3-ethynyl-5-(2-methylpyrrolidin-1-yl)pyridine

Brown solid **E84-3** (50.4 mg, crude product) was prepared from **E84-2** using the method described in **1-2.**

LC-MS: [M+H]⁺= 187.4.

### E-84: 2-((4,4-dimethylpiperidin-1-yl)methyl)-6-((4-(5-(2-methylpyrrolidin-1-yl)pyridin-3-yl)-1H-1,2,3-triazol-1-yl)methyl)-1H-indole

Yellow solid **E-84** (3.60 mg, 2.66%) was prepared from **E84-3** using the method described in **4-d.**

LC-MS: [M+H]⁺= 484.3. ¹H NMR (400 MHz, DMSO-*d*₆): *δ* 11.05 (s, 1H), 8.67 (s, 1H), 8.26 (s, 1H), 7.88 (d, *J* = 2.8 Hz, 1H), 7.43 (d, *J* = 8.1 Hz, 1H), 7.32 (s, 1H), 7.27 (s, 1H), 6.98 (d, *J* = 7.3 Hz, 1H), 6.25 (s, 1H), 5.67 (s, 2H), 3.97 (br s, 1H), 3.57 (s, 2H), 3.47-3.40 (m, 1H), 3.21-3.13 (m, 1H), 2.34 (br s, 4H), 2.10-1.92 (m, 3H), 1.69 (br s, 1H), 1.31 (t, *J* = 5.5 Hz, 4H), 1.12 (d, *J =* 6.1 Hz, 3H), 0.87 (s, 6H).

### Example 85: Synthesis of E-85: (6-(((cyclobutylmethyl)amino)methyl)imidazo[1,2-a]pyridin-2-yl)methyl 5-(dimethylamino)nicotinic acid

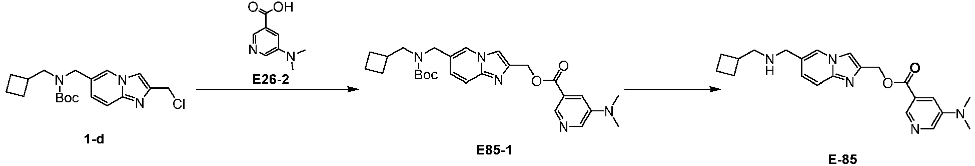

### E85-1: (6-(((tert-butoxycarbonyl)(cyclobutylmethyl)amino)methyl)imidazo[1,2-a]pyridin-2-yl)methyl 5-(dimethylamino)nicotinate

Yellow oily **E85-1** (30.0 mg, 44.2%) was prepared from **1-d** using the method described in **E16-1.**

LC-MS: [M+H]⁺= 494.3.

### E-85: (6-(((cyclobutylmethyl)amino)methyl)imidazo[1,2-a]pyridin-2-yl)methyl 5-(dimethylamino)nicotinic acid

Yellow solid **E-85** (18.3 mg, 75.2%) was prepared from **E85-1** using the method described in **E-1.**

LC-MS: [M+H]⁺= 394.1. ¹H NMR (400 MHz, DMSO-*d*₆): *δ* 9.67 (d, *J* = 1.6 Hz, 2H), 9.07 (s, 1H), 8.57 (s, 1H), 8.52 (s, 1H), 8.39 (d, *J* = 2.8 Hz, 1H), 8.17 (d, *J* = 9.4 Hz, 1H), 8.02 (d, *J =* 9.3 Hz, 1H), 7.86 (br s, 1H), 5.68 (s, 2H), 4.27-4.23 (m, 2H), 3.08 (s, 6H), 2.98-2.92 (m, 2H), 2.71 (td, *J* = 7.5, 15.0 Hz, 1H), 2.09-2.01 (m, 2H), 1.88-1.75 (m, 4H).

### Example 86: Synthesis of E-86: 1-(3-fluorobicyclo[1.1.1]pentan-1-yl)-N-((2-((4-(5-(3-fluoropyrrolidin-1-yl)pyridin-3-yl)-1H-1,2,3-triazol-1-yl)methyl)imidazo[1,2-a]pyridin-6-yl)methyl)methylamine

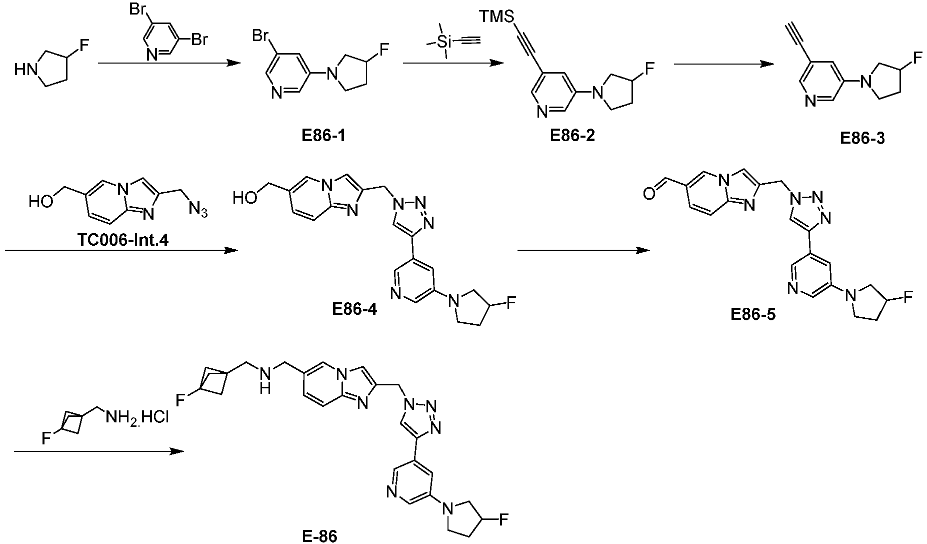

### E86-1: 3-bromo-5-(3-fluoropyrrolidin-1-yl)pyridine

Gray solid **E86-1** (300.0 mg, 48.3%) was prepared from 3-fluoropyrrolidine and 3,5-dibromopyridine using the method described **E130-1.**

LC-MS: [M+H]⁺= 247.0.

### E86-2: 3-(3-fluoropyrrolidin-1-yl)-5-((trimethylsilyl)ethynyl)pyridine

Black solid **E86-2** (250.0 mg, 77.8%) was prepared from **E86-1** using the method described in **E53-6.**

LC-MS: [M+H]⁺= 263.7.

### E86-3: 3-ethynyl-5-(3-fluoropyrrolidin-1-yl)pyridine

Yellow oily **E86-3** (181.0 mg, crude product) was prepared from **E86-2** using the method described in **1-2.**

LC-MS: [M+H]⁺= 191.2.

### E86-4: (2-((4-(5-(3-fluoropyrrolidin-1-yl)pyridin-3-yl)-1H-1,2,3-triazol-1-yl)methyl)imidazo[1,2-a]pyridin-6-yl)methanol

Yellow solid **E86-4** (84.0 mg, 45.1%) was prepared from **E86-3** using the method described in **4-d.**

LC-MS: [M+H]⁺= 394.4.

### E86-5: 2-((4-(5-(3-fluoropyrrolidin-1-yl)pyridin-3-yl)-1H-1,2,3-triazol-1-yl)methyl)imidazo[1,2-a]pyridine-6-formaldehyde

Yellow solid **E86-5** (14.0 mg, crude product) was prepared from **E86-4** using the method described in **E54-2.**

LC-MS: [M+H]⁺= 392.2.

### E-86: 1-(3-fluorobicyclo[1.1.1]pentan-1-yl)-N-((2-((4-(5-(3-fluoropyrrolidin-1-yl)pyridin-3-yl)-1H-1,2,3-triazol-1-yl)methyl)imidazo[1,2-a]pyridin-6-yl)methyl)methylamine

Yellow solid **E-86** (4.20 mg, 23.8%) was prepared from **E86-5** using the method described in **E6-1.**

LC-MS: [M+H]⁺= 491.5. ¹H NMR (400 MHz, DMSO-*d*₆): *δ* 8.69 (s, 1H), 8.46-8.33 (m, 2H), 7.95 (d, *J* = 12.9 Hz, 2H), 7.47 (d, *J* = 9.4 Hz, 1H), 7.35 (br s, 1H), 7.26 (d, *J* = 9.3 Hz, 1H), 5.75 (s, 2H), 5.59-5.40 (m, 1H), 3.68 (s, 2H), 3.65-3.61 (m, 1H), 3.55 (br s, 2H), 3.42 (d, *J=* 7.6 Hz, 2H), 2.74 (s, 2H), 2.35-2.24 (m, 2H), 1.93 (d, *J =* 1.3 Hz, 6H).

### Example 87: Synthesis of E-87: 1-(2-((4-(5-(2,5-dihydro-1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine hydrochloride

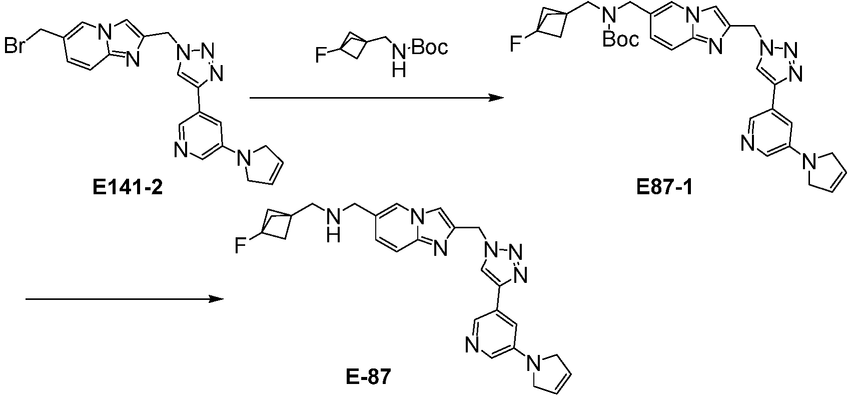

### E87-1: Tert-butyl (2-((4-(5-(2,5-dihydro-1H-pyrrol-1-yl)pyridin-3-yl)-1H-1,2,3-triazol-1-yl)methyl)imidazo[1,2-a]pyridin-6-yl)methyl)((3-fluorobicyclo[1.1.1]pentan-1-yl)methyl)carbamate

White solid **E87-1** (12.0 mg, 18.4%) was prepared from **E141-2** using the method described in **7-a.**

LC-MS: [M+H]⁺= 571.3.

### E-87: 1-(2-((4-(5-(2,5-dihydro-1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine hydrochloride

White solid **E-87** (3.50 mg, 31.1%) was prepared from **E87-1** using the method described in **E-34.**

LC-MS: [M+H]⁺= 471.2. ¹H NMR (400 MHz, DMSO-*d*₆): *δ* 9.53 (d, *J* = 1.6 Hz, 2H), 9.05 (s, 1H), 8.85 (s, 1H), 8.54 (s, 1H), 8.29 (s, 1H), 8.05 (d, *J* = 2.5 Hz, 1H), 7.92 (s, 1H), 7.73 (s, 2H), 6.09 (s, 2H), 5.92 (s, 2H), 4.26 (s, 4H), 4.21 (br s, 2H), 3.30-3.25 (m, 2H), 2.12 (d, *J* = 2.4 Hz, 6H).

### Example 88: Synthesis of E-88: 6-((4,4-dimethylpiperidin-1-yl)methyl)-2-((4-(5-(pyrrolidin-1-yl)pyridin-3-yl)-1H-1,2,3-triazol-1-yl)methyl)imidazo[1,2-a]pyridine

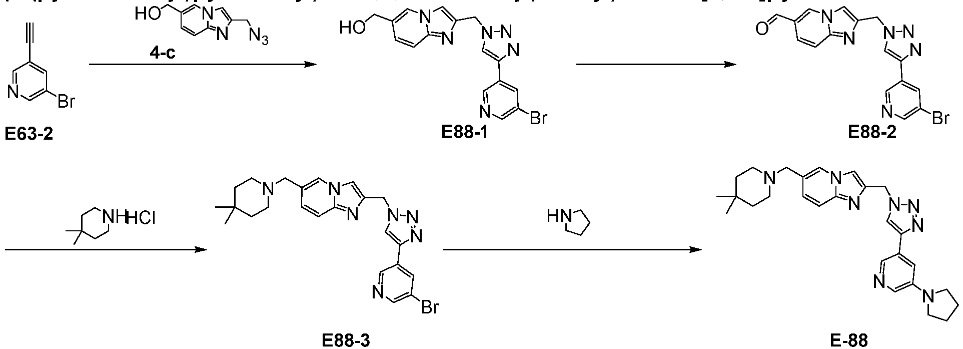

### E88-1: (2-((4-(5-bromopyridin-3-yl)-1H-1,2,3-triazol-1-yl)methyl)imidazo[1,2-a]pyridin-6-yl)methanol

A yellow solid intermediate **E88-1** (1.30 g, 61.3%) was prepared from intermediate **E63-2** using the method described in intermediate **4-d.**

LC-MS (ESI): m/z 385.2 [M+H]⁺.

### E88-2: 2-((4-(5-bromopyridin-3-yl)-1H-1,2,3-triazol-1-yl)methyl)imidazo[1,2-a]pyridin-6-formaldehyde

A yellow solid intermediate **E88-2** (0.80 g, 73.1%) was prepared from intermediate **E88-1** using the method described in intermediate **E54-2.**

LC-MS (ESI): m/z 383.2 [M+H]⁺.

### E88-3: 2-((4-(5-bromopyridin-3-yl)-1H-1,2,3-triazol-1-yl)methyl)-6-((4,4-dimethylpiperidin-1-yl)methyl)imidazo[1,2-a]pyridine

A brown solid intermediate **E88-3** (0.22 g, 58.5%) was prepared from intermediate **E88-2** using the method described in intermediate **E30-1.**

LC-MS (ESI): m/z 480.3 [M+H]⁺.

### E-88: 6-((4,4-dimethylpiperidin-1-yl)methyl)-2-((4-(5-(pyrrolidin-1-yl)pyridin-3-yl)-1H-1,2,3-triazol-1-yl)methyl)imidazo[1,2-a]pyridine

A yellow solid intermediate **E-88** (6.4 mg, 12.7%) was prepared from intermediate **E88-3** using the method described in intermediate **E15-1.**

LC-MS (ESI): m/z 471.2 [M+H]⁺ . ¹H NMR (400 MHz, DMSO-*d*₆): δ 8.68 (s, 1 H), 8.44 (s, 1 H), 8.31 (d, *J* = 1.50 Hz, 1 H), 8.18 (s, 1 H), 7.96 (s, 1 H), 7.89 (d, *J =* 2.75 Hz, 1 H), 7.47 (d, *J* = 9.26 Hz, 1 H), 7.26 - 7.33 (m, 1 H), 7.21 (dd, *J* = 9.26, 1.50 Hz, 1 H), 5.74 (s, 2 H), 3.44 - 3.46 (m, 2 H), 3.29 - 3.33 (m, 4 H), 2.32 - 2.40 (m, 4 H), 1.94 - 2.01 (m, 4 H), 1.32 (t, *J=* 5.44 Hz, 4 H), 0.89 (s, 6 H).

### Example 89: Synthesis of E-89: 5-(1-((6-(((cyclobutylmethyl)amino)methyl)imidazo[1,2-a]pyridin-2-yl)methyl)-1H-1,2,3-triazole-4-yl)-N,n-diethylpyridine-3-amine

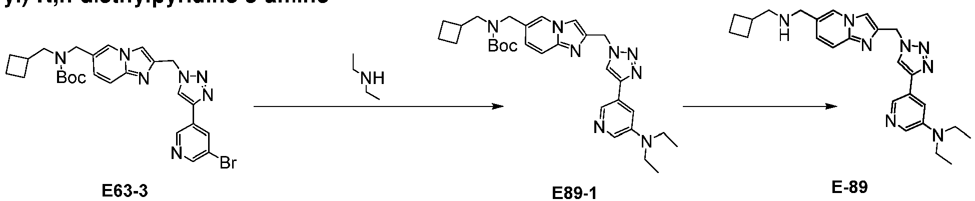

### E89-1: Tert-butyl (2-((4-(5-(diethylamino)pyridin-3-yl)-1H-1,2,3-triazol-1-yl)methyl)imidazo[1,2-a]pyridin-6-yl)methyl)carbamate

A white solid intermediate **E89-1** (70 mg, 71.0%) was prepared from intermediate **E63-3** using the method described in intermediate **E15-1.**

LC-MS (ESI): m/z 545.3 [M+H]⁺.

### E-89: 5-(1-((6-(((cyclobutylmethyl)amino)methyl)imidazo[1,2-a]pyridin-2-yl)methyl)-1H-1,2,3-triazole-4-yl)-N,n-diethylpyridine-3-amine

A white solid compound **E-89** (26.6 mg, 64.3%) was prepared from intermediate **E89-1** using the method described in compound **E-1.**

LC-MS (ESI): m/z 445.4 [M+H]⁺ . ¹H NMR (400 MHz, DMSO-*d*₆): δ 9.62 (br s, 2H), 9.18 (s, 1H), 9.00 (s, 1H), 8.44 (d, *J* = 2.3 Hz, 2H), 8.17 (d, *J* = 2.6 Hz, 1H), 8.08 - 7.96 (m, 2H), 7.87 (d, *J* = 9.4 Hz, 1H), 6.02 (s, 2H), 4.20 (t, *J* = 5.2 Hz, 2H), 3.53 (q, *J* = 7.0 Hz, 4H), 3.00 - 2.87 (m, 2H), 2.79 - 2.62 (m, 1H), 2.12 - 1.96 (m, 2H), 1.92 - 1.68 (m, 4H), 1.14 (t, *J =* 7.0 Hz, 6H).

### Example 90: Synthesis of E-90: 5-(1-((6-(((cyclobutylmethyl)amino)methyl)imidazo[1,2-a]pyridin-2-yl)methyl)-1H-1,2,3-triazol-4-yl)-N,n-bis(methyl-d3)pyridin-3-amine hydrochloride

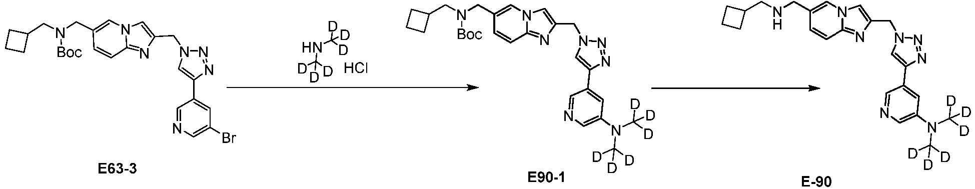

### E90-1: Tert-butyl (2-((4-(5-(bis(methyl-d3)amino)pyridin-3-yl)-1H-1,2,3-triazol-1-yl)methyl)imidazo[1,2-a]pyridin-6-yl)methyl)(cyclobutylmethyl)carbamate

A white solid intermediate **E90-1** (30 mg, 31.7%) was prepared from intermediate **E63-3** using the method described in intermediate **E15-1.**

LC-MS (ESI): m/z 523.3 [M+H]⁺.

### E-90: 5-(1-((6-(((cyclobutylmethyl)amino)methyl)imidazo[1,2-a]pyridin-2-yl)methyl)-1H-1,2,3-triazol-4-yl)-N,n-bis(methyl-d3)pyridin-3-amine hydrochloride

A white solid compound **E-90** (8.30 mg, 31.2%) was prepared from intermediate **E90-1** using the method described in compound **E-1.**

LC-MS (ESI): m/z 423.2 [M+H]⁺ . ¹H NMR (400 MHz, DMSO-*d*₆): δ 9.37 (br s, 2H), 9.07 (s, 1H), 8.86 (s, 1H), 8.52 (s, 1H), 8.31 (s, 1H), 8.16 (d, *J* = 2.7 Hz, 1H), 8.05 (s, 1H), 7.75 (br s, 2H), 5.93 (s, 2H), 4.17 (br s, 2H), 2.94 (d, *J* = 4.8 Hz, 2H), 2.75 - 2.62 (m, 1H), 2.05 (d, *J*= 5.7 Hz, 2H), 1.93 - 1.68 (m, 4H).

### Example 91: Synthesis of E-91: 2-((4-(5-(azetidine-1-yl)pyridin-3-yl)-1H-1,2,3-triazol-1-yl)methyl)-6-((4,4-dimethylpiperidin-1-yl)methyl)imidazo[1,2-a]pyridine

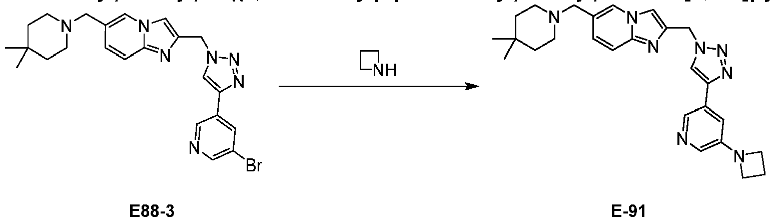

### E-91: 2-((4-(5-(azetidine-1-yl)pyridin-3-yl)-1H-1,2,3-triazol-1-yl)methyl)-6-((4,4-dimethylpiperidin-1-yl)methyl)imidazo[1,2-a]pyridine

A yellow solid compound **E-91** (2.00 mg, 2.08%) was prepared from intermediate **E88-3** using the method described in intermediate **E15-1.**

LC-MS (ESI): m/z 457.2 [M+H]⁺ . ¹H NMR (400 MHz, DMSO-*d*₆): *δ* 8.68 (s, 1 H), 8.36 - 8.47 (m, 2 H), 7.96 (s, 1 H), 7.75 (d, *J* = 2.75 Hz, 1 H), 7.46 (d, *J* = 9.26 Hz, 1 H), 7.18 - 7.26 (m, 2 H), 5.74 (s, 2 H), 3.92 (t, *J* = 7.25 Hz, 4 H), 3.42 - 3.47 (m, 2 H), 2.30 - 2.42 (m, 5 H), 1.32 (t, *J* = 5.44 Hz, 4 H), 0.89 (s, 6 H).

### Example 92: Synthesis of E-92: 5-(1-((6-(((cyclobutylmethyl)amino)methyl)imidazo[1,2-a]pyridin-2-yl)methyl)-1H-1,2,3-triazole-4-yl)-N-ethyl-N-methylpyridin-3-amine

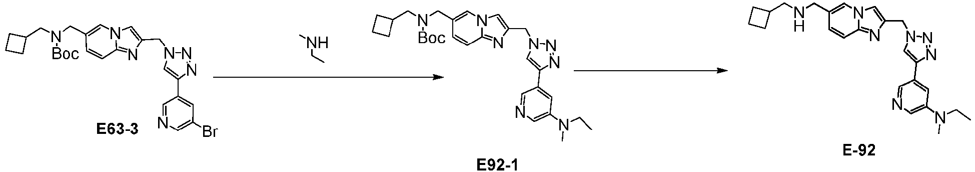

### E92-1: Tert-butyl (2-((4-(5-(ethyl(methyl)amino)pyridin-3-yl)-1H-1,2,3-triazol-1-yl)methyl)imidazo[1,2-a]pyridin-6-yl)methyl)carbamate

A white solid intermediate **E92-1** (70 mg, crude product) was prepared from intermediate **E63-3** using the method described in intermediate **E15-1.**

LC-MS (ESI): m/z 531.3 [M+H]⁺.

### E-92: 5-(1-((6-(((cyclobutylmethyl)amino)methyl)imidazo[1,2-a]pyridin-2-yl)methyl)-1H-1,2,3-triazole-4-yl)-N-ethyl-N-methylpyridin-3-amine

A white solid compound **E-92** (5.70 mg, 9.00%) was prepared from intermediate **E92-1** using the method described in compound **E-1.**

LC-MS (ESI): m/z 431.2 [M+H]⁺ . ¹H NMR (400 MHz, DMSO-*d*₆): δ 9.53 (br s, 2H), 9.12 (s, 1H), 8.93 (s, 1H), 8.48 (s, 1H), 8.37 (s, 1H), 8.18 (d, *J* = 2.3 Hz, 1H), 8.07 (s, 1H), 7.86 (br s, 1H), 7.83 - 7.76 (m, 1H), 5.97 (s, 2H), 4.18 (br s, 2H), 3.57 (br s, 2H), 3.06 (s, 3H), 2.94 (d, *J* = 4.8 Hz, 2H), 2.77 - 2.63 (m, 1H), 2.11 - 1.99 (m, 2H), 1.89 - 1.74 (m, 4H), 1.11 (t, *J* = 7.0 Hz, 3H).

### Example 93: Synthesis of E-93: 1-(2-((4-(6-fluoro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

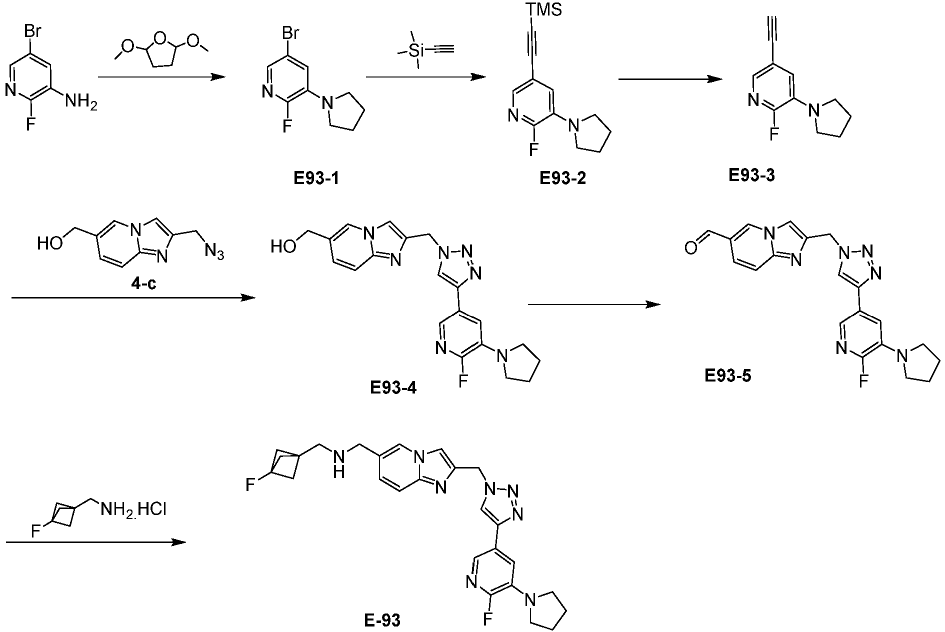

### E93-1: 5-bromo-2-fluoro-3-(pyrrolidin-1-yl)pyridine

A solution of sulfuric acid (408 mg, 4.08 mmol, 222 µL, 98% purity, 3.90 eq) in water (1.78 g, 98.8 mmol, 1.78 mL, 95.0 eq) was added to a solution of 5-bromo-2-fluoropyridine-3-amine (200 mg, 1.05 mmol, 1.00 eq) in THF (4.00 mL) and MeOH (4.00 mL). Then, 2,5-dimethoxy-tetrahydrofuran (416 mg, 3.15 mmol, 405 µL, 3.00 eq) and NaBH₄ (119 mg, 3.15 mmol, 3.00 eq) were added in batches at 0°C, and the resulting mixture was stirred at 25°C for 16 h. The reaction mixture was quenched with water (3.00 mL) at 0°C, the pH was adjusted to about 8 with saturated sodium bicarbonate at 0°C, and the mixture was extracted with ethyl acetate (5.00 mL × 3). The combined organic layers were washed with saline (2.00 mL), dried over anhydrous sodium sulfate, filtered and concentrated. The residue was purified by preparative thin-layer chromatography (petroleum ether: ethyl acetate=20:1) to obtain white solid **E93-1** (0.10 g, 39.0%).

LC-MS: [M+H]⁺= 245.0.

### E93-2: 2-fluoro-3-(pyrrolidin-1-yl)-5-((trimethylsilyl)ethynyl)pyridine

Yellow solid **E93-2** (70.0 mg, 26.2%) was prepared from **E93-1** using the method described in **E53-6.**

LC-MS: [M+H]⁺= 263.2.

### E93-3: 5-ethynyl-2-fluoro-3-(pyrrolidin-1-yl)pyridine

Yellow solid **E93-3** (20.0 mg, crude product) was prepared from **E93-2** using the method described in **1-2.**

LC-MS: [M+H]⁺= 191.1.

### E93-4: (2-((4-(6-fluoro-5-(pyrrolidin-1-yl)pyridin-3-yl)-1H-1,2,3-triazol-1-yl)methyl)imidazo[1,2-a]pyridin-6-yl)methanol

Brown solid **E93-4** (130.0 mg, crude product) was prepared from **E93-3** using the method described in **4-d.**

LC-MS: [M+H]⁺= 394.1.

### E93-5: 2-((4-(6-fluoro-5-(pyrrolidin-1-yl)pyridin-3-yl)-1H-1,2,3-triazol-1-yl)methyl)imidazo[1,2-a]pyridine-6-formaldehyde

Brown solid **E93-5** (70.0 mg, 43.3%) was prepared from **E93-4** using the method described in **E54-2.**

LC-MS: [M+H]⁺= 392.1.

### E-93: 1-(2-((4-(6-fluoro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

Yellow solid **E-93** (4.20 mg, 10.3%) was prepared from **E93-5** using the method described in **E6-1.**

LC-MS: [M+H]⁺= 491.1. ¹H NMR (400 MHz, DMSO-*d*₆): *δ* 8.67 (s, 1H), 8.42 (s, 1H), 7.96 (s, 1H), 7.88 (s, 1H), 7.46 (d, *J* = 9.8 Hz, 2H), 7.30-7.22 (m, 1H), 5.73 (s, 2H), 3.68 (s, 2H), 3.40 (d, *J* = 2.0 Hz, 4H), 2.74 (s, 2H), 1.99-1.87 (m, 10H).

### Example 94: Synthesis of E-94: 1-(2-((4-(5-(azetidine-1-yl)pyridin-3-yl)-1H-1,2,3-triazol-1-yl)methyl)imidazo[1,2-a]pyridin-6-yl)-N-(cyclobutylmethyl)methylamine

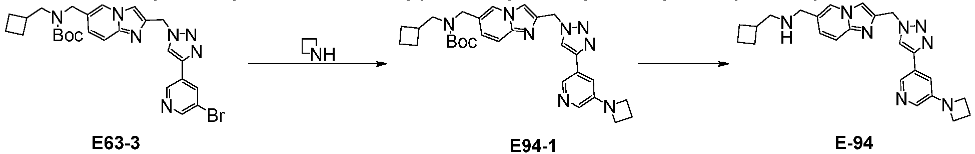

### E94-1: Tert-butyl (2-((4-(5-(azetidin-1-yl)pyridin-3-yl)-1H-1,2,3-triazol-1-yl)methyl)imidazo[1,2-a]pyridin-6-yl)methyl)(cyclobutylmethyl)carbamate

A yellow solid intermediate **E94-1** (50 mg, 52.3%) was prepared from intermediate **E63-3** using the method described in intermediate **E15-1.**

LC-MS (ESI): m/z 529.3 [M+H]⁺.

### E-94: 1-(2-((4-(5-(azetidine-1-yl)pyridin-3-yl)-1H-1,2,3-triazol-1-yl)methyl)imidazo[1,2-a]pyridin-6-yl)-N-(cyclobutylmethyl)methylamine

At 25°C, TFA/DCM (V/V=1/50, 35.0 eq) was added to a solution of intermediate **E94-1** (30.0 mg, 56.8 µmol, 1.00 eq) in DCM (0.60 mL) while stirring, and the resulting mixture was stirred at 25°C for 16 h. The mixture was concentrated, and the residue was purified by preparative HPLC to obtain a yellow solid compound **E-94** (3.30 mg, 13.3%).

LC-MS (ESI): m/z 429.2 [M+H]⁺ . ¹H NMR (400 MHz, DMSO-*d*₆): *δ* 8.67 (s, 1 H) 8.41 (s, 1 H) 8.38 (d, *J* = 1.43 Hz, 1 H) 7.95 (s, 1 H) 7.90 - 8.01 (m, 1 H) 7.74 (d, *J* = 2.50 Hz, 1 H) 7.45 (d, *J =* 9.18 Hz, 1 H) 7.25 (dd, *J* = 9.30, 1.43 Hz, 1 H) 7.20 (t, *J* = 2.15 Hz, 1 H) 5.73 (s, 2 H) 3.91 (t, *J* = 7.27 Hz, 4 H) 3.64 (s, 2 H) 3.29 (s, 2 H) 2.37 - 2.41 (m, 3 H) 1.90 - 2.03 (m, 3 H) 1.71 - 1.87 (m, 3 H) 1.55 - 1.66 (m, 2 H).

### Example 95: Synthesis of E-95: 1-(2-((4-(2-fluoro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

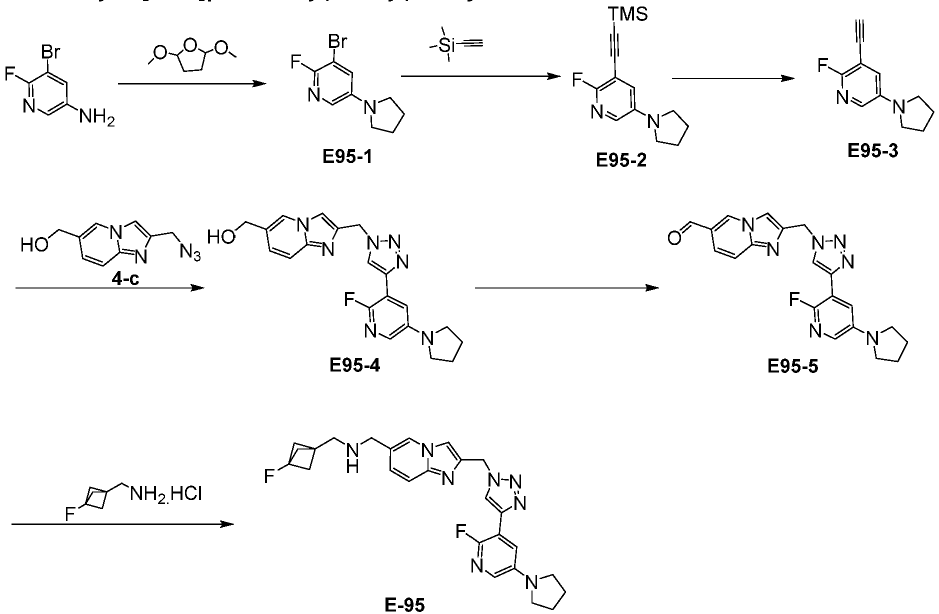

### E95-1: 3-bromo-2-fluoro-5-(pyrrolidin-1-yl)pyridine

White solid **E95-1** (100.0 mg, 39.1%) was prepared from 5-bromo-6-fluoropyridine-3-amine using the method described in **E93-1.**

LC-MS: [M+H]⁺= 264.9.

### E95-2: 2-fluoro-5-(pyrrolidin-1-yl)-3-((trimethylsilyl)ethynyl)pyridine

Gray solid **E95-2** (70.0 mg, 81.7%) was prepared from **E95-1** using the method described in **E53-6.**

LC-MS: [M+H]⁺= 263.2.

### E95-3: 3-ethynyl-2-fluoro-5-(pyrrolidin-1-yl)pyridine

Yellow oily **E95-3** (50.7 mg, crude product) was prepared from **E95-2** using the method described in **1-2.**

LC-MS: [M+H]⁺= 191.1.

### E95-4: (2-((4-(2-fluoro-5-(pyrrolidin-1-yl)pyridin-3-yl)-1H-1,2,3-triazol-1-yl)methyl)imidazo[1,2-a]pyridin-6-yl)methanol

Yellow solid **E95-4** (70.0 mg, 58.8%) was prepared from **E95-3** using the method described in **4-d.**

LC-MS: [M+H]⁺= 394.4.

### E95-5: 2-((4-(2-fluoro-5-(pyrrolidin-1-yl)pyridin-3-yl)-1H-1,2,3-triazol-1-yl)methyl)imidazo[1,2-a]pyridine-6-formaldehyde

Brown solid **E95-5** (30.0 mg, 30.2%) was prepared from **E95-4** using the method described in **E143-2.**

LC-MS: [M+H]⁺= 392.2.

### E-95: 1-(2-((4-(2-fluoro-5-(pyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

Yellow solid **E-95** (1.80 mg, 9.34%) was prepared from **E95-5** using the method described in **E6-1.**

LC-MS: [M+H]⁺= 491.2. ¹H NMR (400 MHz, DMSO-*d*₆): *δ* 8.47 (d, *J* = 3.6 Hz, 1H), 8.41 (s, 1H), 7.94 (s, 1H), 7.65 (dd, *J* = 2.9, 8.2 Hz, 1H), 7.46 (d, *J* = 9.4 Hz, 1H), 7.43 (br s, 1H), 7.25 (d, *J* = 9.4 Hz, 1H), 5.78 (s, 2H), 3.67 (s, 2H), 3.30 (d, *J* = 6.6 Hz, 4H), 2.73 (s, 2H), 1.98 (t, *J* = 6.4 Hz, 4H), 1.92 (d, *J* = 2.6 Hz, 6H).

### Example 96: Synthesis of E-96: 1-(3-fluorobicyclo[1.1.1]pentan-1-yl)-N-((2-((4-(2-methyl-5-(pyrrolidin-1-yl)pyridin-3-yl)-1H-1,2,3-triazol-1-yl)methyl)imidazo[1,2-a]pyridin-6-yl)methyl)methylamine

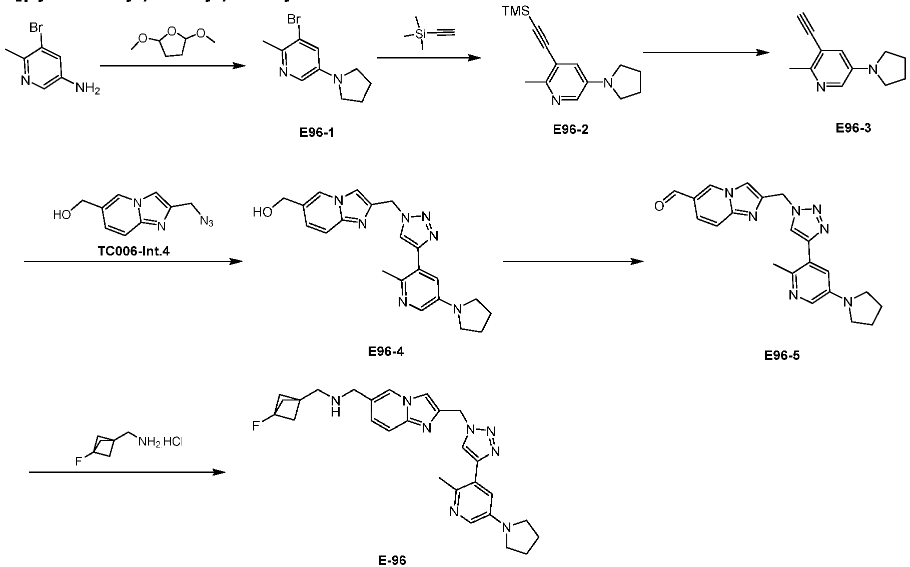

### E96-1: 3-bromo-2-methyl-5-(pyrrolidin-1-yl)pyridine

Yellow solid **E96-1** (400 mg, 31.6%) was prepared from 5-bromo-6-methylpyridine-3-amine using the method described in **E93-1.**

LC-MS: [M+H]⁺= 241.1.

### E96-2: 2-methyl-5-(pyrrolidin-1-yl)-3-((trimethylsilyl)ethynyl)pyridine

Brown solid **E96-2** (150.0 mg, 73.7%) was prepared from **E96-1** using the method described in **E53-6.**

LC-MS: [M+H]⁺= 259.2.

### E96-3: 3-ethynyl-2-methyl-5-(pyrrolidin-1-yl)pyridine

Yellow oily **E96-3** (108 mg, crude product) was prepared from **E96-2** using the method described in **1-2.**

LC-MS: [M+H]⁺= 187.3.

### E96-4: (2-((4-(2-methyl-5-(pyrrolidin-1-yl)pyridin-3-yl)-1H-1,2,3-triazol-1-yl)methyl)imidazo[1,2-a]pyridin-6-yl)methanol

Brown solid **E96-4** (200 mg, crude product) was prepared from **E96-3** using the method described in **4-d.**

LC-MS: [M+H]⁺= 390.1.

### E96-5: 2-((4-(2-methyl-5-(pyrrolidin-1-yl)pyridin-3-yl)-1H-1,2,3-triazol-1-yl)methyl)imidazo[1,2-a]pyridine-6-formaldehyde

Yellow solid **E96-5** (25.0 mg, 10.9%) was prepared from **E96-4** using the method described in **E143-2.**

LC-MS: [M+H]⁺= 388.2.

### E-96: 1-(3-fluorobicyclo[1.1.1]pentan-1-yl)-N-((2-((4-(2-methyl-5-(pyrrolidin-1-yl)pyridin-3-yl)-1H-1,2,3-triazol-1-yl)methyl)imidazo[1,2-a]pyridin-6-yl)methyl)methylamine

Yellow solid **E-96** (11.40 mg, 22.7%) was prepared from **E96-5** using the method described in **E6-1.**

LC-MS: [M+H]⁺= 487.2. ¹H NMR (400 MHz, DMSO-*d*₆): *δ* 8.50 (s, 1H), 8.41 (s, 1H), 7.93 (s, 1H), 7.83 (d, *J* = 2.4 Hz, 1H), 7.46 (d, *J* = 9.4 Hz, 1H), 7.25 (d, *J* = 9.3 Hz, 1H), 7.22 (d, *J* = 2.4 Hz, 1H), 5.75 (s, 2H), 3.67 (s, 2H), 3.31 (br s, 3H), 3.26 (br s, 4H), 2.74 (s, 2H), 1.96 (br s, 4H), 1.93 (d, *J* = 2.4 Hz, 6H).

### Example 97: Synthesis of E-97: N-(1-(6-(((cyclobutylmethyl)amino)methyl)imidazo[1,2-a]pyridin-2-yl)ethyl)-5-(pyrrolidin-1-yl)nicotinamide

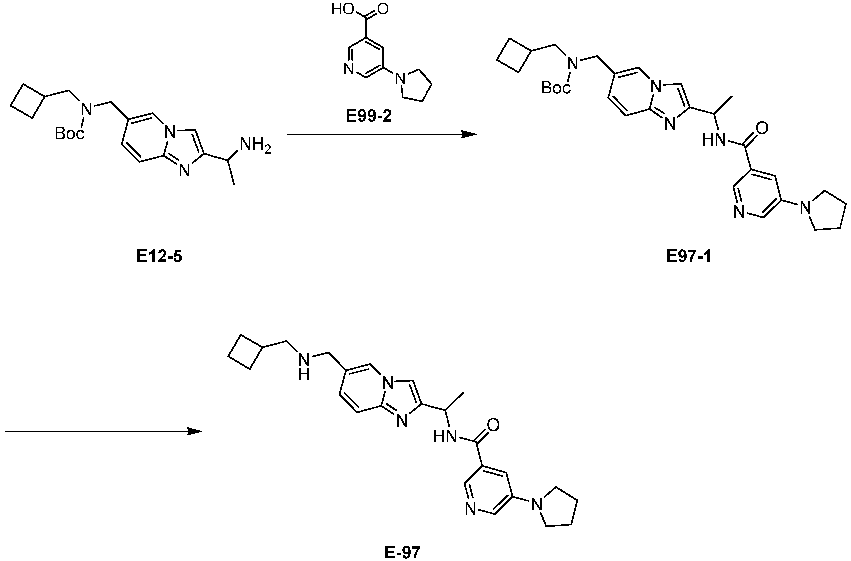

### E97-1: Tert-butyl (cyclobutylmethyl)((2-(1-(5-(pyrrolidin-1-yl)nicotinamido)ethyl)imidazo[1,2-a]pyridin-6-yl)methyl)carbamate

White solid **E97-1** (15.0 mg, 19.6%) was prepared from **E12-5** using the method described in **E101-2.**

LC-MS: [M+H]⁺= 533.3.

### E-97: N-(1-(6-(((cyclobutylmethyl)amino)methyl)imidazo[1,2-a]pyridin-2-yl)ethyl)-5-(pyrrolidin-1-yl)nicotinamide

Yellow solid **E-97** (4.50 mg, 33.8%) was prepared from **E97-1** using the method described in **E-3.**

LC-MS: [M+H]⁺= 433.2. ¹H NMR (400 MHz, DMSO-*d*₆): *δ* 9.78 (d, *J* = 7.3 Hz, 1H), 9.61 (br s, 2H), 9.00 (s, 1H), 8.49 (s, 1H), 8.47-8.42 (m, 1H), 8.17 (d, *J* = 2.0 Hz, 1H), 8.13 (d, *J* = 9.1 Hz, 1H), 7.98 (d, *J* = 9.4 Hz, 1H), 7.95 (br s, 1H), 5.48 (t, *J* = 7.1 Hz, 1H), 4.25 (br s, 2H), 3.45-3.43 (m, 4H), 2.95 (d, *J* = 4.4 Hz, 2H), 2.73-2.66 (m, 1H), 2.10-2.03 (m, 2H), 2.02-1.97 (m, 4H), 1.90-1.76 (m, 4H), 1.70 (d, *J* = 7.0 Hz, 3H).

### Example 98: Synthesis of E-98: 1-(2-((4-(5-(1H-pyrrol-1-yl)pyridin-3-yl)-1H-1,2,3-triazol-1-yl)methyl)imidazo[1,2-a]pyridin-6-yl)-N-(cyclobutylmethyl)methylamine

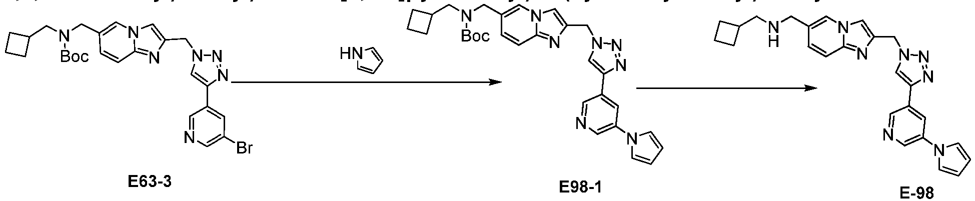

### E98-1: Tert-butyl (2-((4-(5-(1H-pyrrol-1-yl)pyridin-3-yl)-1H-1,2,3-triazol-1-yl)methyl)imidazo[1,2-a]pyridin-6-yl)methyl)(cyclobutylmethyl)carbamate

A white solid intermediate **E98-1** (0.07 g, 72.6%) was prepared from intermediate **E63-3** using the method described in intermediate **E15-1.**

LC-MS (ESI): m/z 539.2 [M+H]⁺.

### E-98: 1-(2-((4-(5-(1H-pyrrol-1-yl)pyridin-3-yl)-1H-1,2,3-triazol-1-yl)methyl)imidazo[1,2-a]pyridin-6-yl)-N-(cyclobutylmethyl)methylamine

A white solid compound **E-98** (25.6 mg, 39.5%) was prepared from intermediate **E98-1** using the method described in compound **E-1.**

LC-MS (ESI): m/z 439.1 [M+H]⁺ . ¹H NMR (400 MHz, DMSO-*d*₆): *δ* 8.96 (s, 1 H) 8.92 (s, 1 H) 8.89 - 8.91 (m, 1 H) 8.87 - 8.89 (m, 1 H) 8.44 - 8.47 (m, 1 H) 8.38 - 8.40 (m, 1 H) 7.85 (s, 2 H) 7.55 (s, 2 H) 6.36 (s, 2 H) 5.99 (s, 2 H) 4.21 - 4.24 (m, 2 H) 2.98 (s, 2 H) 2.62 (br s, 1 H) 2.04 (s, 2 H) 1.73 - 1.84 (m, 4 H).

### Example 99: Synthesis of E-99: N-((6-(((cyclobutylmethyl)amino)methyl)imidazo[1,2-a]pyridin-2-yl)methyl)-5-(pyrrolidin-1-yl)nicotinamide hydrochloride

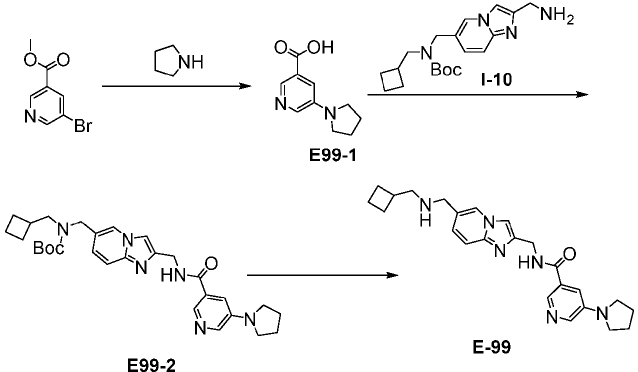

### E99-1: 5-(pyrrolidin-1-yl)nicotinic acid

A yellow solid intermediate **E99-1** (0.40 g, crude product) was prepared from methyl 5-bromonicotinate using the method described in intermediate **E15-1.**

LC-MS (ESI): m/z 193.0 [M+H]⁺.

### E99-2: Tert-butyl (2-((5-(pyrrolidin-1-yl)nicotinamido)methyl)imidazo[1,2-a]pyridin-6-yl)methyl)carbamate

A yellow solid intermediate **E99-2** (35.0 mg, 23.2%) was prepared from intermediates **E99-1** and **I-10** using the method described in intermediate **E7-3.**

LC-MS (ESI): m/z 519.5 [M+H]⁺.

### E-99: N-((6-(((cyclobutylmethyl)amino)methyl)imidazo[1,2-a]pyridin-2-yl)methyl)-5-(pyrrolidin-1-yl)nicotinamide hydrochloride

A white solid compound **E-99** (5.60 mg, 18.2%) was prepared from intermediate **E99-2** using the method described in compound **E-1.**

LC-MS (ESI): m/z 419.4 [M+H]⁺ . ¹H NMR (400 MHz, DMSO-*d*₆): *δ* 9.99 (br s, 1H), 9.55 (br s, 2H), 9.01 (s, 1H), 8.46 (s, 1H), 8.40 (s, 1H), 8.18 (d, *J* = 2.5 Hz, 1H), 8.12 (d, *J* = 9.4 Hz, 1H), 8.01 - 7.92 (m, 2H), 4.76 (d, *J* = 5.4 Hz, 2H), 4.24 (br s, 2H), 3.42 - 3.39 (m, 4H), 3.01 - 2.92 (m, 2H), 2.76 - 2.68 (m, 1H), 2.07 - 1.99 (m, 6H), 1.87 - 1.76 (m, 4H).

### Example 100: Synthesis of E-100: 5-(1-((2-((4,4-dimethylpiperidin-1-yl)methyl)-1H-indole-6-yl)methyl)-1H-1,2,3-triazole-4-yl)-N,n-dimethylpyridine-3-amine

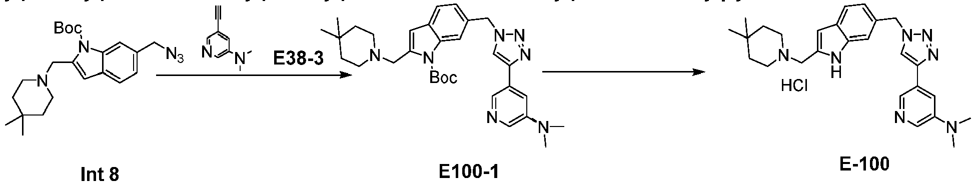

### E100-1: Tert-butyl 6-((4-(5-(dimethylamino)pyridin-3-yl)-1H-1,2,3-triazol-1-yl)methyl)-2-((4,4-dimethylpiperidin-1-yl)methyl)-1H-indole-1-carboxylate

A white solid intermediate **E100-1** (0.10 g, 28.5%) was prepared from intermediate **E38-3** using the method described in intermediate **E7-3.**

LC-MS (ESI): m/z 544.4 [M+H]⁺.

### E-100: 5-(1-((2-((4,4-dimethylpiperidin-1-yl)methyl)-1 H-indole-6-yl)methyl)-1H-1,2,3-triazole-4-yl)-N,n-dimethylpyridine-3-amine

A yellow solid compound **E-100** (14.5 mg, 16.4%) was prepared from intermediate **E100-1** using the method described in compound **E-1.**

LC-MS (ESI): m/z 444.1 [M+H]⁺ . ¹H NMR (400 MHz, DMSO-*d*₆): *δ* 11.58 - 11.67 (m, 1 H) 10.69 - 10.85 (m, 1 H) 8.98 - 9.01 (m, 1 H) 8.49 - 8.53 (m, 1 H) 8.14 - 8.18 (m, 1 H) 8.04 - 8.07 (m, 1 H) 7.57 - 7.63 (m, 1 H) 7.47 - 7.51 (m, 1 H) 7.07 - 7.12 (m, 1 H) 6.64 - 6.68 (m, 1 H) 5.76 - 5.81 (m, 2 H) 4.49 (s, 2 H) 3.16 - 3.21 (m, 2 H) 3.11 (s, 6 H) 3.03 - 3.08 (m, 2 H) 1.67 - 1.75 (m, 2 H) 1.48 - 1.54 (m, 2 H) 0.93 - 0.99 (m, 6 H).

### Example 101: Synthesis of E-101: N-((2-(((cyclobutylmethyl)amino)methyl)-1H-indole-6-yl)methyl)-5-(pyrrolidin-1-yl)nicotinamide hydrochloride

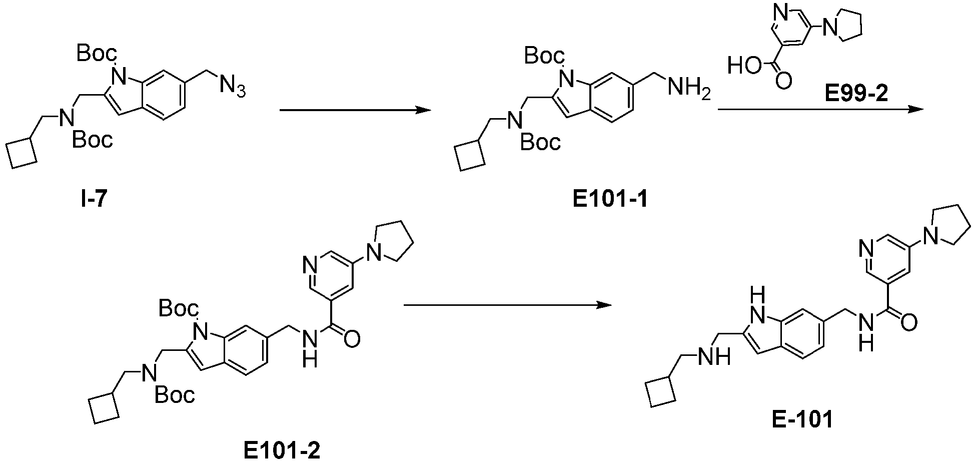

### E101-1: Tert-butyl 6-(aminomethyl)-2-(((tert-butoxycarbonyl)(cyclobutylmethyl)amino)methyl)-1H-indole-1-carboxylate

A yellow oily intermediate **E101-1** (840 mg, 59.3%) was prepared from intermediate **I-7** using the method described in intermediate **I-10.**

LC-MS (ESI): m/z 443.7 [M+H]⁺.

### E101-2: Tert-butyl 2-(((tert-butoxycarbonyl)(cyclobutylmethyl)amino)methyl)-6-((5-(pyrrolidin-1-yl)nicotinamido)methyl)-1H-indole-1-carboxylate

EDCI (64.8 mg, 338 µmol, 1.50 eq) was added to a solution of intermediates **E99-2** (43.3 mg, 225 µmol, 1.00 eq) and **E101-1** (100 mg, 225 µmol, 1.00 eq) in pyridine (2.00 mL) while stirring. The resulting mixture was stirred at 25°C for 16 h under nitrogen protection. The mixture was quenched by adding water (5.00 mL) and extracted with ethyl acetate (2.00 mL * 3). The merged organic layers were washed with saline (2.00 mL), dried over anhydrous sodium sulfate, filtered and concentrated. The residue was purified by silica gel pre-thin-layer chromatography and eluted with DCM: MeOH (10:1) to obtain brown oily intermediate **E101-2** (0.07 g, 50.3%).

LC-MS (ESI): m/z 618.5 [M+H]⁺.

### E-101: N-((2-(((cyclobutylmethyl)amino)methyl)-1H-indole-6-yl)methyl)-5-(pyrrolidin-1-yl)nicotinamide hydrochloride

A yellow oily compound **E-101** (37.6 mg, 67.2%) was prepared from intermediate **E101-2** using the method described in compound **E-1.**

LC-MS (ESI): m/z 418.2 [M+H]⁺ . ¹H NMR (400 MHz, DMSO-*d*₆): *δ* 11.46 (s, 1 H) 9.70 (t, *J* = 5.92 Hz, 1 H) 9.35 - 9.45 (m, 2 H) 8.44 (s, 1 H) 8.17 (d, *J* = 2.63 Hz, 1 H) 7.99 (s, 1 H) 7.50 (d, *J* = 8.11 Hz, 1 H) 7.38 (s, 1 H) 7.04 (dd, *J* = 8.11, 1.32 Hz, 1 H) 6.57 (s, 1 H) 4.60 (d, *J* = 5.92 Hz, 2 H) 4.25 (t, *J* = 5.26 Hz, 2 H) 3.40 (t, *J* = 6.36 Hz, 4 H) 2.88 - 2.95 (m, 2 H) 2.62 - 2.70 (m, 1 H) 1.97 - 2.05 (m, 6 H) 1.73 - 1.84 (m, 4 H).

### Example 102: Synthesis of E-102: 5-(dimethylamino)-N-((6-((4,4-dimethylpiperidin-1-yl)methyl)imidazo[1,2-a]pyridin-2-yl)methyl)nicotinamide

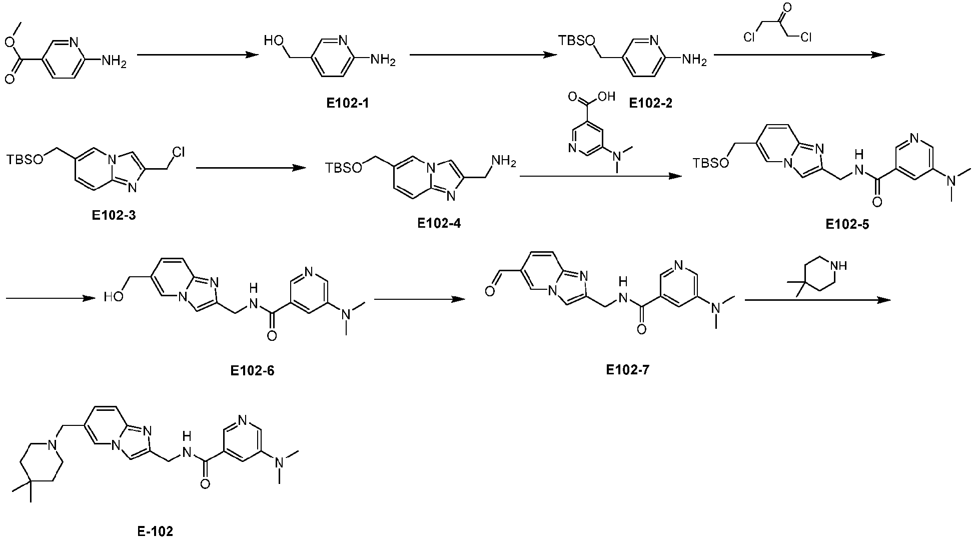

### E102-1: (6-aminopyridin-3-yl)methanol

A yellow solid intermediate **E102-1** (9.50 g, crude product) was prepared from methyl 6-amino-nicotinate using the method described in intermediate **E54-1.**

**LC-MS (ESI):** m/z 125.0 [M+H]⁺.

### E102-2: 5-((tert-butyl dimethyloxy)methyl)pyridine-2 amine

Imidazole (5.73 g, 84.1 mmol, 1.10 eq) and TBSCI (11.5 g, 76.5 mmol, 9.38 mL, 1.00 eq) were added to a solution of intermediate **E102-1** (9.50 g, 76.5 mmol, 1.00 eq) in DCM (250 mL). The resulting mixture was stirred at 25°C for 16 h. The mixture was quenched with water (100 mL) and extracted with DCM (30.0 mL * 3). The combined organic layers were washed with saline (2.00 mL), dried over anhydrous sodium sulfate, filtered and concentrated. The residue was purified by column chromatography (petroleum ether/ethyl acetate=50/1 to 1/1) to obtain a white solid intermediate **E102-2** (8.00 g, 43.8%).

**LC-MS (ESI):** m/z 239.0 [M+H]⁺.

### E102-3: 6-(((tert-butyl dimethylsiloxy)methyl)-2-(chloromethyl)imidazo[1,2-a]pyridine

1,3-dichloropropan-2-one (8.52 g, 67.1 mmol, 8.35 mL, 2.00 eq) was added to a suspension of intermediate **E102-2** (8.00 g, 33.6 mmol, 1.00 eq) in MeCN (80.0 mL) at 20°C, and the resulting mixture was stirred at 80°C for 16 h. The mixture was quenched with 2 M sodium bicarbonate (100.0 mL) and extracted with DCM (30.0 mL *. The combined organic layers were washed with saline (2.00 mL), dried over anhydrous sodium sulfate, filtered and concentrated. The residue was purified by column chromatography (petroleum ether/ethyl acetate=50/1 to 0/1) to obtain a pink solid intermediate **E102-3** (1.00 g, 9.60%).

**LC-MS (ESI):** m/z 311.1 [M+H]⁺.

### E102-4: (6-(((tert-butyl dimethylsilyl)oxy)methyl)imidazo[1,2-a]pyridin-2-yl)methylamine

A solution of NH₃ in MeOH(7M, 30.0 mL) was added to intermediate **E102-3** (1.00 g, 3.22 mmol, 1.00 eq), and the mixture was stirred at 50°C for 12 h. The solution was concentrated. The residue was purified by column chromatography (DCM/MeOH=50/1 to 5/1) to obtain a yellow solid intermediate **E102-4** (0.50 g, 53.3%).

**LC-MS (ESI):** m/z 292.1 [M+H]⁺.

### E102-5: N-((6-(((tert-butyl dimethylsilyl)oxy)methyl)imidazo[1,2-a]pyridin-2-yl)methyl)-5-(dimethylamino)nicotinamide

A yellow solid intermediate **E102-5** (0.32 g, 42.4%) was prepared from intermediate **E102-4** using the method described in intermediate **E7-3.**

**LC-MS (ESI):** m/z 440.2 [M+H]⁺.

### E102-6: 5-(dimethylamino)-N-((6-(hydroxymethyl)imidazo[1,2-a]pyridin-2-yl)methyl)nicotinamide

TBAF (1 M, 1.23 mL, 2.00 eq) was added to a solution of intermediate **E102-5** (0.27 g, 614 µmol, 1.00 eq) in THF (5.50 mL) at 20°C. The resulting mixture was stirred at 25°C for 2 h. The mixture was concentrated. The residue was purified by preparative thin-layer chromatography (DCM/MeOH=5/1) to obtain a brown solid intermediate **E102-6** (0.120 g, 60.0%).

**LC-MS (ESI):** m/z 326.2 [M+H]⁺.

### E102-7: 5-(dimethylamino)-N-((6-formylimidazo[1,2-a]pyridin-2-yl)methyl)nicotinamide

A red solid intermediate **E102-7** (30 mg, crude product) was prepared from intermediate **E102-6** using the method described in intermediate **I-4.**

**LC-MS (ESI):** m/z 323.4 [M+H]⁺.

### E-102: 5-(dimethylamino)-N-((6-((4,4-dimethylpiperidin-1-yl)methyl)imidazo[1,2-a]pyridin-2-yl)methyl)nicotinamide

A yellow solid compound **E-102** (2.80 mg, 7.00%) was prepared from intermediate **E102-7** using the method described in Intermediate **E30-1.**

LC-MS (ESI): m/z 421.2 [M+H]⁺ . ¹H NMR (400 MHz, DMSO-*d*₆): *δ* 9.08 - 9.14 (m, 1 H), 8.34 - 8.41 (m, 2 H), 8.20 - 8.25 (m, 1 H), 7.76 - 7.80 (m, 1 H), 7.47 - 7.49 (m, 1 H), 7.43 (d, *J* = 9.38 Hz, 1 H), 7.13 - 7.18 (m, 1 H), 4.52 - 4.60 (m, 2 H), 3.39 - 3.47 (m, 2 H), 2.96 - 3.01 (m, 6 H), 2.32 - 2.37 (m, 4 H), 1.29 - 1.36 (m, 4 H), 0.87 - 0.91 (m, 6 H).

### Example 103: Synthesis of E-103: 5-(dimethylamino)-N-((2-((4,4-dimethylpiperidine-1-yl)methyl)-1H-indole-6-yl)methyl)nicotinamide

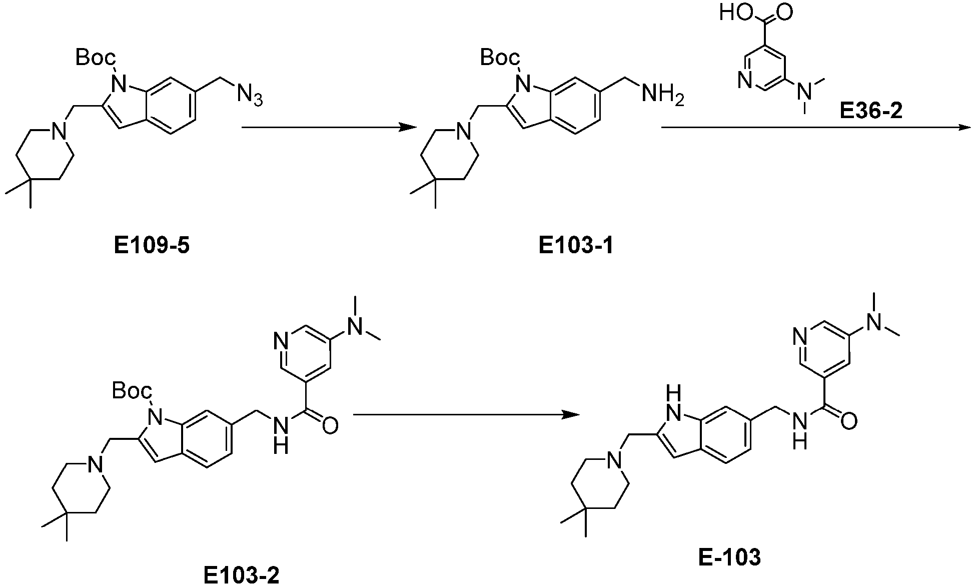

### E103-1: Tert-butyl 6-(aminomethyl)-2-((4,4-dimethylpiperidin-1-yl)methyl)-1H-indole-1-carboxylate

A white solid intermediate **E103-1** (26.0 mg, 37.0%) was prepared from intermediate **E109-5** using the method described in intermediate **I-10.**

LC-MS (ESI): m/z 372.2 [M+H]⁺.

### E103-2: Tert-butyl 6-((5-(dimethylamino)nicotinamido)methyl)-2-((4,4-dimethylpiperidin-1-yl)methyl)-1H-indole-1-carboxylate

A white solid intermediate **E103-2** (30.0 mg, 18.8%) was prepared from intermediate **E103-1** using the method described in intermediate **E7-3.**

LC-MS (ESI): m/z 520.5 [M+H]⁺.

### E-103: 5-(dimethylamino)-N-((2-((4,4-dimethylpiperidin-1-yl)methyl)-1H-indole-6-yl)methyl)nicotinamide

A white solid compound **E-103** (16.4 mg, 30.7%) was prepared from intermediate **E103-2** using the method described in intermediate **E-1.**

LC-MS (ESI): m/z 420.3 [M+H]⁺ . ¹H NMR (400 MHz, DMSO-*d*₆): *δ* 11.42 (s, 1 H) 10.54 - 10.67 (m, 1 H) 9.59 - 9.66 (m, 1 H) 8.47 (s, 1 H) 8.30 (s, 1 H) 8.04 - 8.07 (m, 1 H) 7.52 - 7.56 (m, 1 H) 7.40 (s, 1 H) 7.05 - 7.09 (m, 1 H) 6.61 - 6.63 (m, 1 H) 4.60 (s, 2 H) 4.47 (s, 2 H) 3.16 (s, 2 H) 3.10 (s, 6 H) 3.03 - 3.08 (m, 2 H) 1.65 - 1.72 (m, 2 H) 1.52 (s, 2 H) 0.96 (d, *J=* 10.38 Hz, 6 H).

### Example 104: Synthesis of E-104: 1-(2-((4-(5-(3,3-difluoropyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

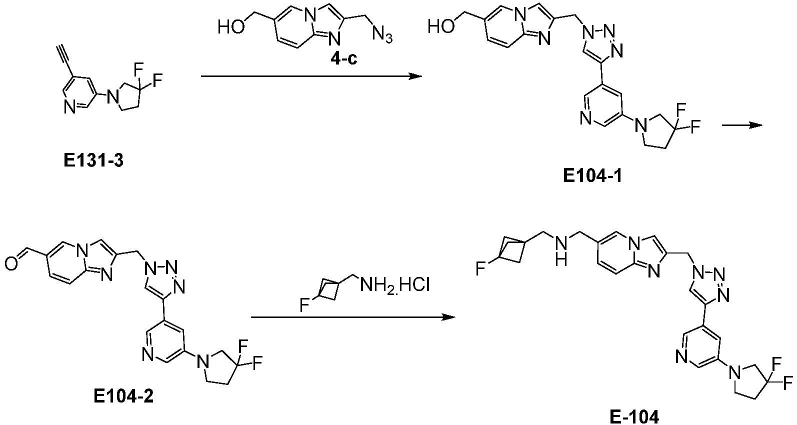

### E104-1: (2-((4-(5-(3,3-difluoropyrrolidin-1-yl)pyridin-3-yl)-1H-1,2,3-triazol-1-yl)methyl)imidazo[1,2-a]pyridin-6-yl)methanol

Yellow solid **E104-1** (70.0 mg, 42.5%) was prepared from **E131-4** and **4-c** using the method described in **4-d.**

LC-MS: [M+H]⁺= 412.3.

### E104-2: 2-((4-(5-(3,3-difluoropyrrolidin-1-yl)pyridin-3-yl)-1H-1,2,3-triazol-1-yl)methyl)imidazo[1,2-a]pyridine-6-formaldehyde

White solid **E104-2** (49 mg, crude product) was prepared from **E104-1** using the method described in **E54-2.**

LC-MS: [M+H]⁺= 410.2.

### E-104: 1-(2-((4-(5-(3,3-difluoropyrrolidin-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

Yellow solid **E-104** (37.5 mg, 59.2%) was prepared from **E104-2** using the method described in **E6-1.**

LC-MS: [M+H]⁺= 509.2. ¹H NMR (400 MHz, DMSO-*d*₆): *δ* 8.69 (s, 1H), 8.47-8.41 (m, 2H), 7.98-7.95 (m, 2H), 7.47 (d, *J* = 9.3 Hz, 1H), 7.40 (s, 1H), 7.28 (s, 1H), 5.75 (s, 2H), 3.80 (s, 2H), 3.68 (s, 2H), 3.58 (s, 2H), 2.74 (s, 2H), 2.61-2.56 (m, 2H), 1.93 (d, *J* = 2.6 Hz, 6H).

### Example 105: Synthesis of E-105: 1-(6-((4-(5-(1H-pyrrol-1-yl)pyridin-3-yl)-1H-1,2,3-triazol-1-yl)methyl)-1H-indole-2-yl)-N-(cyclobutylmethyl)methylamine

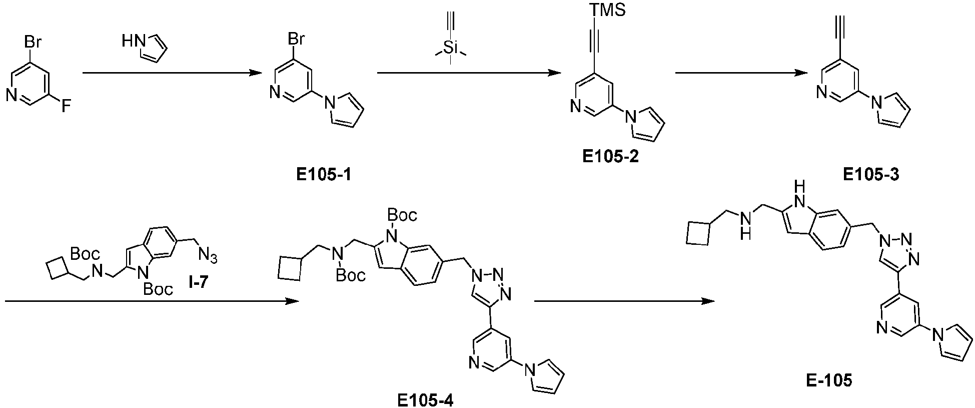

### E105-1: 3-bromo-5-(1H-pyrrol-1-yl)pyridine

A white solid intermediate **E105-1** (1.10 g, 86.8%) was prepared from 3-bromo-5-fluoropyridine using the method described in intermediate **E16-1.**

LC-MS (ESI): m/z 223.2 [M+H]⁺.

### E105-2: 3-(1H-pyrrole-1-yl)-5-((trimethylsilyl)ethynyl)pyridine

A brown solid intermediate **E105-2** (0.90 g, 83.5%) was prepared from intermediate **E105-1** using the method described in intermediate **2-b.**

LC-MS (ESI): m/z 241.3 [M+H]⁺.

### E105-3: 3-ethynyl-5-(1H-pyrrol-1-yl)pyridine

A brown solid intermediate **E105-3** (0.12 g, 85.8%) was prepared from intermediate **E105-2** using the method described in intermediate **I-2.**

LC-MS (ESI): m/z 169.1 [M+H]⁺.

### E105-4: Tert-butyl 6-((4-(5-(1H-pyrrol-1-yl)pyridin-3-yl)-1H-1,2,3-triazol-1-yl)methyl)-2-(((tert-butoxycarbonyl)(cyclobutylmethyl)amino)methyl)-1H-indole-1-carboxylate

A white solid intermediate **E105-4** (0.08 g, 70.3%) was prepared from intermediate **E105-3** using the method described in intermediate **4-d.**

LC-MS (ESI): m/z 638.6 [M+H]⁺.

### E-105: 1-(6-((4-(5-(1H-pyrrol-1-yl)pyridin-3-yl)-1H-1,2,3-triazol-1-yl)methyl)-1H-indole-2-yl)-N-(cyclobutylmethyl)methylamine

A white solid compound **E-105** (26.3 mg, 44.2%) was prepared from intermediate **E105-4** using the method described in intermediate **E-1.**

LC-MS (ESI): m/z 438.1 [M+H]⁺ . ¹H NMR (400 MHz, DMSO-*d*₆): *δ* 11.55 (d, *J =* 0.66 Hz, 1 H) 9.27 - 9.35 (m, 2 H) 8.98 (s, 1 H) 8.92 (s, 1 H) 8.87 (s, 1 H) 8.49 (t, *J* = 1.86 Hz, 1 H) 7.55 - 7.60 (m, 3 H) 7.48 (s, 1 H) 7.07 (dd, *J =* 8.22, 1.21 Hz, 1 H) 6.61 (s, 1 H) 6.35 (t, *J =* 2.08 Hz, 2 H) 5.78 (s, 2 H) 4.27 (t, *J =* 5.26 Hz, 2 H) 2.90 - 2.97 (m, 2 H) 2.61 - 2.70 (m, 1 H) 2.00 - 2.08 (m, 2 H) 1.73 - 1.85 (m, 4 H).

### Example 106: Synthesis of E-106: 1-cyclobutyl-N-((6-((4-(5-(2,5-dihydro-1H-pyrrol-1-yl)pyridin-3-yl)-1H-1,2,3-triazol-1-yl)methyl)-1H-indole-2-yl)methyl)methylamine

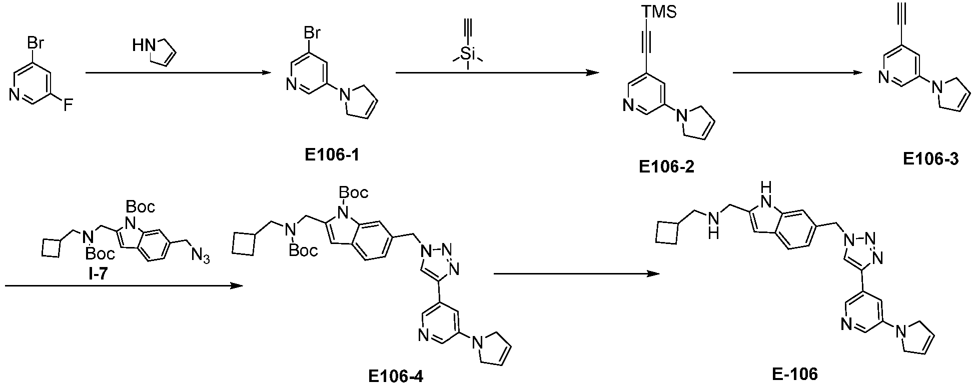

### E106-1: 3-bromo-5-(2,5-dihydro-1H-pyrrol-1-yl)pyridine

A black solid intermediate **E106-1** (1.00 g, 33.4%) was prepared from 3-bromo-5-fluoropyridine using the method described in intermediate **E16-1.**

LC-MS (ESI): m/z 224.9 [M+H]⁺.

### E106-2: 3-(2,5-dihydro-1H-pyrrol-1-yl)-5-((trimethylsilyl)ethynyl)pyridine

A black solid intermediate **E106-2** (0.95 g, 67.8%) was prepared from intermediate **E106-1** using the method described in intermediate **2-b.**

LC-MS (ESI): m/z 243.2 [M+H]⁺.

### E106-3: 3-(2,5-dihydro-1H-pyrrol-1-yl)-5-ethynylpyridine

A black solid intermediate **E106-3** (55.0 mg, 63.8%) was prepared from intermediate **E106-2** using the method described in intermediate **I-2.**

LC-MS (ESI): m/z 171.0 [M+H]⁺.

### E106-4: Tert-butyl 2-(((tert-butoxycarbonyl)(cyclobutylmethyl)amino)methyl)-6-((4-(5-(2,5-dihydro-1H-pyrrol-1-yl)pyridin-3-yl)-1H-1,2,3-triazol-1-yl)methyl)-1H-indole-1-carboxylate

A yellow solid intermediate **E106-4** (0.08 g, 41.1%) was prepared from intermediate **E106-3** using the method described in intermediate **4-d.**

LC-MS (ESI): m/z 638.5 [M+H]⁺.

### E-106: 1-cyclobutyl-N-((6-((4-(5-(2,5-dihydro-1H-pyrrol-1-yl)pyridin-3-yl)-1H-1,2,3-triazol-1-yl)methyl)-1H-indole-2-yl)methyl)methylamine

A yellow solid compound **E-106** (21.6 mg, 31.3%) was prepared from intermediate **E106-4** using the method described in intermediate **E-1.**

LC-MS (ESI): m/z 440.2 [M+H]⁺ . ¹H NMR (400 MHz, DMSO-*d*₆): *δ* 11.62 (s, 1H), 9.39 (s, 2H), 8.96 (s, 1H), 8.51 (s, 1H), 8.03 (d, *J =* 2.15 Hz, 1H), 7.88 (s, 1H), 7.58 (d, *J =* 8.11 Hz, 1H), 7.49 (s, 1H), 7.07 (d, *J =* 8.23 Hz, 1H), 6.62 (s, 1 H), 6.09 (s, 2H), 5.78 (s, 2H), 4.28 (s, 2H), 4.25 (s, 4H), 2.98-2.89 (m, 2H), 2.67 (s, 1H), 2.09-2.02 (m, 2H), 1.83-1.72 (m, 4H).

### Example 107: Synthesis of E-107: 1-cyclobutyl-N-((2-((4-phenyl-1H-1,2,3-triazol-1-yl)methyl)imidazo[1,2-a]pyridin-6-yl)methyl)methylamine

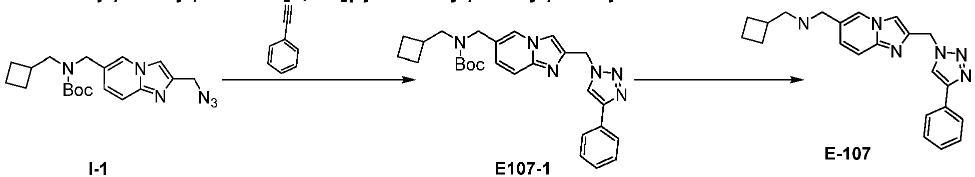

### E107-1: Tert-butyl (2-((4-phenyl-1H-1,2,3-triazol-1-yl)methyl)imidazo[1,2-a]pyridin-6-yl)methyl)carbamate

A yellow solid intermediate **E107-1** (0.08 g, 41.1%) was prepared from intermediate **I-1** using the method described in intermediate **4-d.**

LC-MS (ESI): m/z 473.3 [M+H]⁺.

### E-107: 1-cyclobutyl-N-((2-((4-phenyl-1H-1,2,3-triazol-1-yl)methyl)imidazo[1,2-a]pyridin-6-yl)methyl)methylamine

A white solid compound **E-107** (34.6 mg, 49.8%) was prepared from intermediate **E107-1** using the method described in intermediate **E-1.**

LC-MS (ESI): m/z 373.2 [M+H]⁺ . ¹H NMR (400 MHz, DMSO-*d*₆): *δ* 9.72-9.64 (m, 2H), 9.05-9.02 (m, 1H), 8.79 (s, 1H), 8.47 (s, 1H), 8.13-8.08 (m, 1H), 7.96-7.91 (m, 1H), 7.84 (s, 2H), 7.46 (s, 2H), 7.38-7.32 (m, 1H), 6.00 (s, 2H), 4.22 (d, *J =* 4.88 Hz, 2H), 2.97-2.91 (m, 2H), 2.71 (s, 1H), 2.05 (d, *J =* 6.63 Hz, 2H), 1.83-1.74 (m, 4H).

### Example 108: Synthesis of E-108: 1-cyclobutyl-N-((2-((4-(pyridin-3-yl)-1H-1,2,3-triazol-1-yl)methyl)imidazo[1,2-a]pyridin-6-yl)methyl)methylamine

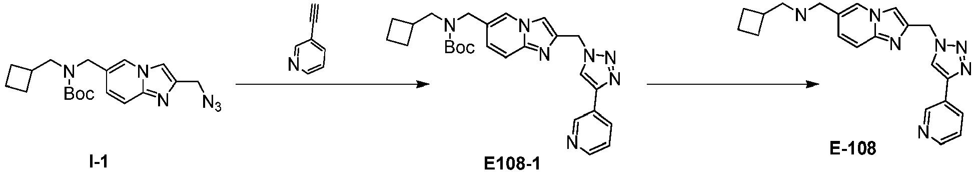

### E108-1: Tert-butyl (cyclobutylmethyl)((2-((4-(pyridin-3-yl)-1H-1,2,3-triazol-1-yl)methyl)imidazo[1,2-a]pyridin-6-yl)methyl)carbamate

A white solid intermediate **E108-1** (42.0 mg, 46.9%) was prepared from intermediate **I-1** using the method described in intermediate **4-d.**

LC-MS (ESI): m/z 474.4 [M+H]⁺.

### E-108: 1-cyclobutyl-N-((2-((4-(pyridin-3-yl)-1H-1,2,3-triazol-1-yl)methyl)imidazo[1,2-a]pyridin-6-yl)methyl)methylamine

A white solid compound **E-108** (21.1 mg, 62.9%) was prepared from intermediate **E108-1** using the method described in intermediate **E-1.**

LC-MS (ESI): m/z 374.1 [M+H]⁺ . ¹H NMR (400 MHz, DMSO-*d*₆): *δ* 9.07 (d, *J* = 1.25 Hz, 1H), 8.74 (s, 1H), 8.53 (dd, *J =* 4.75, 1.50 Hz, 1H), 8.42 (s, 1H), 8.23 (t, *J =* 7.91, 1.80 Hz, 1H), 7.97 (s, 1H), 7.50-7.44 (m, 2H), 7.26 (dd, *J =* 9.26, 1.25 Hz, 1H), 5.76 (s, 2H), 3.65 (s, 2H), 3.33 (s, 2H), 2.45-2.35 (m, 1H), 2.02-1.95 (m, 2H), 1.80 (d, *J =* 8.13 Hz, 2H), 1.63 (d, *J =* 9.51 Hz, 2H).

### Example 109: Synthesis of E-109: 6-((4-(5-(azetidine-1-yl)pyridin-3-yl)-1H-1,2,3-triazol-1-yl)methyl)-2-((4,4-dimethylpiperidin-1-yl)methyl)-1H-indole

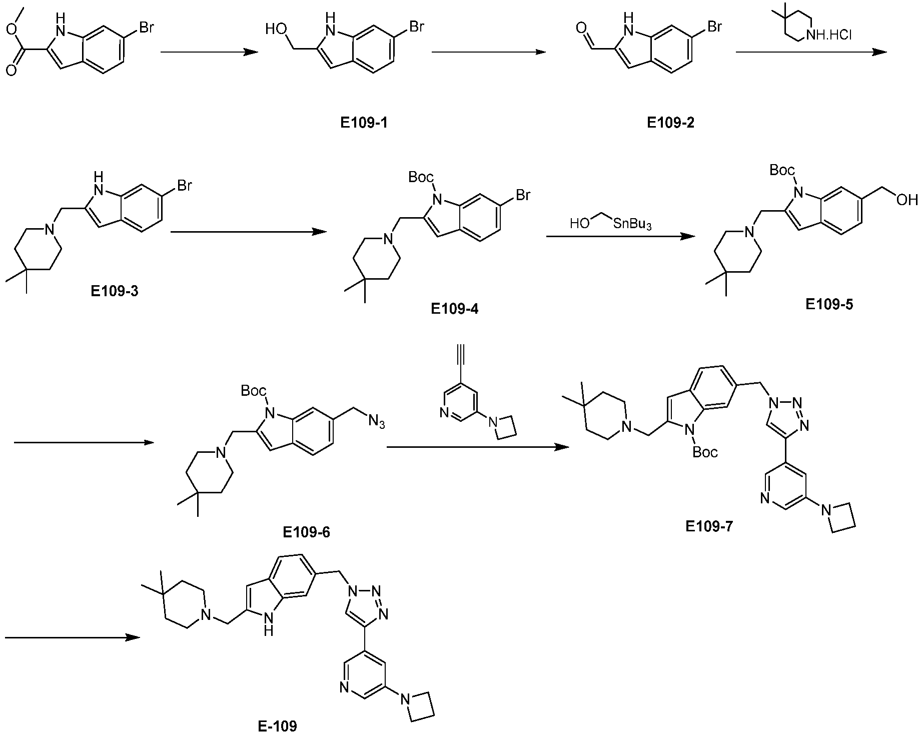

### E109-1: (6-bromo-1H-indole-2-yl)methanol

A brown solid intermediate **E109-1** (17.0 g, crude product) was prepared from methyl 6-bromo-1H-indole-2-carboxylate using the method described in intermediate **E54-1.**

LC-MS (ESI): m/z 225.9 [M+H]⁺.

### E109-2: 6-bromo-1H-indole-2-formaldehyde

A yellow solid intermediate **E109-2** (5.80 g, 34.4%) was prepared from intermediate **E109-1** using the method described in intermediate **E54-2.**

LC-MS (ESI): m/z 224.0 [M+H]⁺.

### E109-3: 6-bromo-2-((4,4-dimethylpiperidin-1-yl)methyl)-1H-indole

A pink solid intermediate **E109-3** (3.10 g, crude product) was prepared from intermediate **E109-2** using the method described in intermediate **E30-1.**

LC-MS (ESI): m/z 321.1 [M+H]⁺.

### E109-4: Tert-butyl 6-bromo-2-((4,4-dimethylpiperidin-1-yl)methyl)-1H-indole-1-carboxylate

At 0°C, Boc₂O (10.6 g, 48.5 mmol, 11.1 mL, 4.00 eq) and LiHMDS (1.00 M, 36.4 mL, 3.00 eq) were added to a solution of intermediate **E109-3** (3.90 g, 12.1 mmol, 1.00 eq) in THF (40.0 mL), and the resulting mixture was stirred at 70°C for 16 h under nitrogen protection. The mixture was quenched with water (100 mL) and extracted with ethyl acetate (30.0 mL * 3). The combined organic layers were washed with saline (30.00 mL), dried over anhydrous sodium sulfate, filtered and concentrated. The residue was purified by silica gel column chromatography and eluted with DCM: MeOH=50/1 to 10:1 to obtain a red oily intermediate **E109-4** (4.50 g, 87.9%).

LC-MS (ESI): m/z 421.1 [M+H]⁺.

### E109-5: Tert-butyl 2-((4,4-dimethylpiperidin-1-yl)methyl)-6-(hydroxymethyl)-1H-indole-1-carboxylate

A yellow solid intermediate **E109-5** (430 mg, 41.3%) was prepared from intermediate **E109-4** using the method described in intermediate **8-b.**

LC-MS (ESI): m/z 373.3 [M+H]⁺.

### E109-6: Tert-butyl 6-(azidomethyl)-2-((4,4-dimethylpiperidin-1-yl)methyl)-1H-indole-1-carboxylate

A colorless oily intermediate **E109-6** (0.30 g, 53.2%) was prepared from intermediate **E109-5** using the method described in intermediate **I-7.**

LC-MS (ESI): m/z 398.3 [M+H]⁺.

### E109-7: Tert-butyl 6-((4-(5-(azetidin-1-yl)pyridin-3-yl)-1H-1,2,3-triazol-1-yl)methyl)-2-((4,4-dimethylpiperidin-1-yl)methyl)-1H-indole-1-carboxylate

A yellow solid intermediate **E109-7** (0.05 g, 14.8%) was prepared from intermediate **E109-6** using the method described in intermediate **4-d.**

LC-MS (ESI): m/z 556.3 [M+H]⁺.

### E-109: 6-((4-(5-(azetidine-1-yl)pyridin-3-yl)-1H-1,2,3-triazol-1-yl)methyl)-2-((4,4-dimethylpiperidin-1-yl)methyl)-1H-indole

A yellow solid compound **E-109** (8.10 mg, 19.0%) was prepared from intermediate **E109-7** using the method described in intermediate **E-94.**

LC-MS (ESI): m/z 456.3 [M+H]⁺. ¹H NMR (400 MHz, DMSO-*d*₆): *δ* 11.06 (s, 1H), 8.66 (s, 1H), 8.37 (s, 1H), 7.73 (s, 1H), 7.43 (d, *J =* 8.2 Hz, 1H), 7.31 (s, 1H), 7.19-7.16 (m, 1H), 6.97 (dd, *J =* 1.5, 8.1 Hz, 1H), 6.25 (d, *J =* 1.1 Hz, 1H), 5.67 (s, 2H), 3.90 (t, *J =* 7.3 Hz, 4H), 3.57 (s, 2H), 2.39-2.29 (m, 6H), 1.31 (t, *J =* 5.4 Hz, 4H), 0.87 (s, 6H).

### Example 110: Synthesis of E-110: 1-cyclobutyl-N-((2-((4-(3-(pyrrolidin-1-yl)phenyl)-1H-1,2,3-triazol-1-yl)methyl)imidazo[1,2-a]pyridin-6-yl)methyl)methylamine

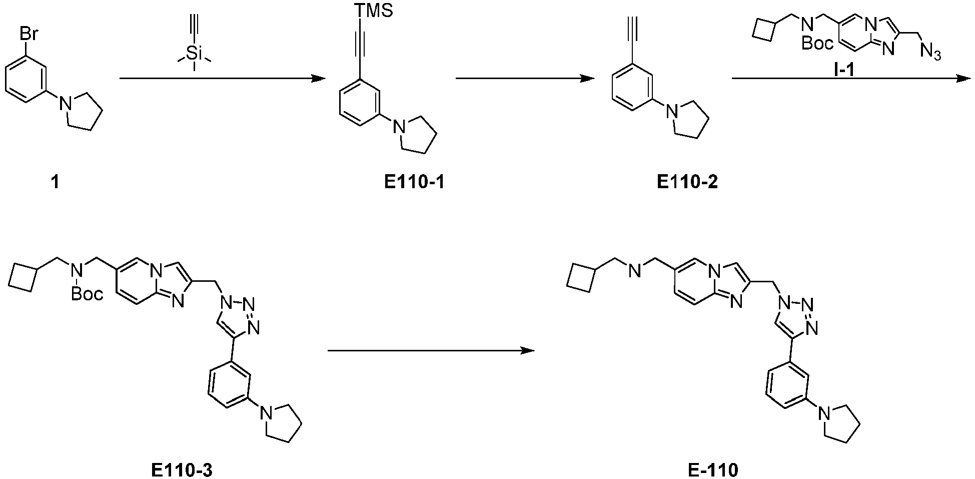

### E110-1: 1-(3-((trimethylsilyl)ethynyl)phenyl)pyrrolidine

A yellow solid intermediate **E110-1** (0.18 g, 66.1%) was prepared from 1-(3-bromophenyl)pyrrolidine using the method described in intermediate **E53-6.**

LC-MS (ESI): m/z 244.1 [M+H]⁺.

### E110-2: 1-(3-ethynylphenyl)pyrrolidine

A brown solid intermediate **E110-2** (0.06 g, 60.0%) was prepared from intermediate **E110-1** using the method described in intermediate I**-2.**

LC-MS (ESI): m/z 172.3 [M+H]⁺.

### E110-3: Tert-butyl (2-((4-(3-(pyrrolidin-1-yl)phenyl)-1H-1,2,3-triazol-1-yl)methyl)imidazo[1,2-a]pyridin-6-yl)methyl)carbamate

A brown solid intermediate **E110-3** (0.15 g, 79.0%) was prepared from intermediate **E110-2** using the method described in intermediate **4-d.**

LC-MS (ESI): m/z 542.4 [M+H]⁺.

### E-110: 1-cyclobutyl-N-((2-((4-(3-(pyrrolidin-1-yl)phenyl)-1H-1,2,3-triazol-1-yl)methyl)imidazo[1,2-a]pyridin-6-yl)methyl)methylamine

A yellow solid compound **E-110** (96.4 mg, 72.8%) was prepared from intermediate **E110-3** using the method described in intermediate **E-1.**

LC-MS (ESI): m/z 442.4 [M+H]⁺. ¹H NMR (400 MHz, DMSO-*d*₆): *δ* 9.76 (br s, 2 H), 9.11 (s, 1 H), 8.80 (s, 1 H), 8.56 (s, 1 H), 8.24 (d, *J* = 9.30 Hz, 1 H), 8.01 (d, *J* = 9.42 Hz, 1 H), 7.24 - 7.32 (m, 1 H), 7.19 (br s, 2 H), 6.71 (d, *J =* 2.98 Hz, 1 H), 6.03 (s, 2 H), 4.25 (br t, *J* = 5.01 Hz, 2 H), 3.33 (br s, 4 H), 2.85 - 3.03 (m, 2 H), 2.64 - 2.79 (m, 1 H), 1.92 - 2.12 (m, 6 H), 1.69 - 1.90 (m, 4 H).

### Example 111: Synthesis of E-111: 2-((4,4-dimethylpiperidin-1-yl)methyl)-6-((4-(5-(pyrrolidin-1-yl)pyridin-3-yl)-1H-1,2,3-triazole-1-yl)methyl)-1H-indole

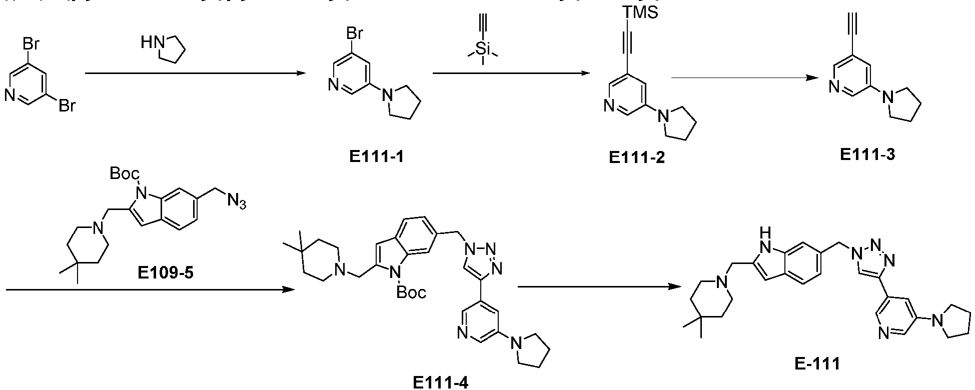

### E111-1: 3-bromo-5-(pyrrolidin-1-yl)pyridine

A yellow solid intermediate **E111-1** (7.70 g, 80.3%) was prepared from 3,5-dibromopyridine using the method described in intermediate **E15-1.**

LC-MS (ESI): m/z 227.0 [M+H]⁺.

### E111-2: 3-(pyrrolidin-1-yl)-5-((trimethylsilyl)ethynyl)pyridine

A brown solid intermediate **E111-2** (6.80 g, 83.1%) was prepared from intermediate **E111-1** using the method described in intermediate **E53-6.**

LC-MS (ESI): m/z 245.3 [M+H]⁺.

### E111-3: 3-ethynyl-5-(pyrrolidin-1-yl)pyridine

A white solid intermediate **E111-3** (4.00 g, 83.5%) was prepared from intermediate **E111-2** using the method described in intermediate **I-2.**

LC-MS (ESI): m/z 173.3 [M+H]⁺.

### E111-4: Tert-butyl 2-((4,4-dimethylpiperidin-1-yl)methyl)-6-((4-(5-(pyrrolidin-1-yl)pyridin-3-yl)-1H-1,2,3-triazol-1-yl)methyl)-1H-indole-1-carboxylate

A brown oily intermediate **E111-4** (0.03 g, 30.2%) was prepared from intermediate **E111-3** using the method described in intermediate **4-d.**

LC-MS (ESI): m/z 570.4 [M+H]⁺.

### E-111: 2-((4,4-dimethylpiperidin-1-yl)methyl)-6-((4-(5-(pyrrolidin-1-yl)pyridin-3-yl)-1H-1,2,3-triazole-1-yl)methyl)-1H-indole

A yellow solid compound **E-111** (7.40 mg, 26.2%) was prepared from intermediate **E111-4** using the method described in intermediate **E-1.**

LC-MS (ESI): m/z 470.3 [M+H]⁺ . ¹H NMR (400 MHz, DMSO-*d*₆): *δ* 11.55 (s, 1 H), 10.56 (d, *J =* 1.38 Hz, 1 H), 8.93 (s, 1 H), 8.44 (s, 1 H), 8.00 (d, *J =* 2.50 Hz, 1 H), 7.82 (s, 1 H), 7.60 (d, *J =* 8.25 Hz, 1 H), 7.49 (s, 1 H), 7.08 (dd, *J =* 8.19, 1.06 Hz, 1 H), 6.65 (s, 1 H), 5.78 (s, 2 H), 4.47 (d, *J =* 4.63 Hz, 2 H), 3.39 (t, *J =* 6.38 Hz, 4 H), 3.15 - 3.20 (m, 2 H), 3.01 - 3.09 (m, 2 H), 1.98 - 2.03 (m, 4 H), 1.62 - 1.73 (m, 2 H), 1.50 (d, *J =* 14.13 Hz, 2 H), 0.95 (d, *J* = 11.38 Hz, 6 H).

### Example 112: Synthesis of E-112: 1-(6-((4-(5-(azetidine-1-yl)pyridin-3-yl)-1H-1,2,3-triazol-1-yl)methyl)-1H-indole-2-yl)-N-(cyclobutylmethyl)methylamine

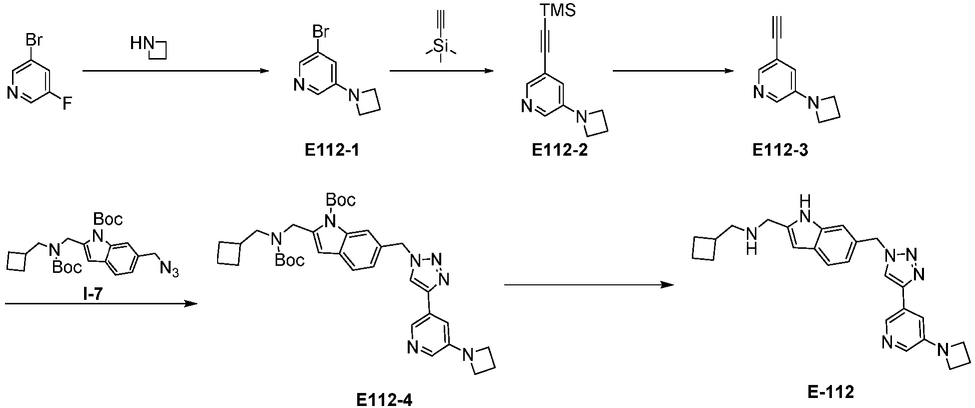

### E112-1: 3-(azetidine-1-yl)-5-bromopyridine

A white solid intermediate **E112-1** (1.00 g, 75.1%) was prepared from 3-bromo-5-fluoropyridine using the method described in intermediate **E16-1.**

LC-MS (ESI): m/z 212.9 [M+H]⁺.

### E112-2: 3-(azetidin-1-yl)-5-((trimethylsilyl)ethynyl)pyridine

A brown solid intermediate **E112-2** (0.30 g, 92.5%) was prepared from intermediate **E112-1** using the method described in intermediate **2-b.**

LC-MS (ESI): m/z 231.2 [M+H]⁺.

### E112-3: 3-(azetidin-1-yl)-5-ethynylpyridine

A white solid intermediate **E112-3** (100.0 mg, 67.7%) was prepared from intermediate **E112-2** using the method described in intermediate **I-2.**

LC-MS (ESI): m/z 159.1 [M+H]⁺.

### E112-4: Tert-butyl 6-((4-(5-(azetidin-1-yl)pyridin-3-yl)-1H-1,2,3-triazol-1-yl)methyl)-2-(((tert-butoxycarbonyl)(cyclobutylmethyl)amino)methyl)-1H-indole-1-carboxylate

A white solid intermediate **E112-4** (0.10 g, 84.0%) was prepared from intermediate **E112-3** using the method described in intermediate **4-d.**

LC-MS (ESI): m/z 628.3 [M+H]⁺.

### E-112: 1-(6-((4-(5-(azetidine-1-yl)pyridin-3-yl)-1H-1,2,3-triazol-1-yl)methyl)-1H-indole-2-yl)-N-(cyclobutylmethyl)methylamine

A white solid compound **E-112** (12.0 mg, 52.9%) was prepared from intermediate **E112-4** using the method described in intermediate **E-1.**

LC-MS (ESI): m/z 428.2 [M+H]⁺. ¹H NMR (400 MHz, DMSO-*d*₆): *δ* 11.03 (s, 1H), 8.69 (s, 1H), 8.39 (d, *J* = 1.7 Hz, 1H), 7.75 (d, *J =* 2.7 Hz, 1H), 7.45 (d, *J =* 8.1 Hz, 1H), 7.34 (s, 1H), 7.20 (d, *J =* 2.2 Hz, 1H), 6.99 (d, *J =* 8.2 Hz, 1H), 6.25 (s, 1H), 5.69 (s, 2H), 3.92 (t, *J =* 7.2 Hz, 4H), 3.80 (s, 2H), 2.49-2.29 (m, 4H), 2.06-1.90 (m, 3H), 1.89-1.73 (m, 2H), 1.70-1.53 (m, 2H).

### Example 113: Synthesis of E-113: 1-cyclobutyl-N-((2-((4-(5-(2,5-dihydro-1H-pyrrol-1-yl)pyridin-3-yl)-1H-1,2,3-triazol-1-yl)methyl)imidazo[1,2-a]pyridin-6-yl)methyl)methylamine

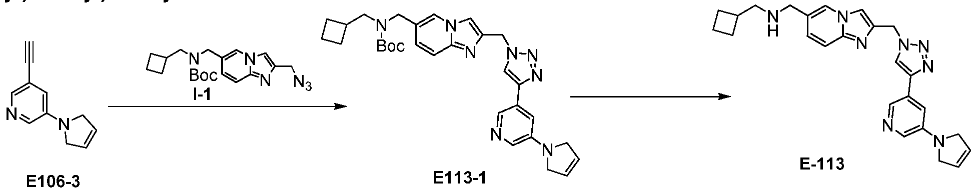

### E113-1: Tert-butyl (cyclobutylmethyl)((2-((4-(5-(2,5-dihydro-1H-pyrrol-1-yl)pyridin-3-yl)-1H-1,2,3-triazol-1-yl)methyl)imidazo[1,2-a]pyridin-6-yl)methyl)carbamate

A yellow solid intermediate **E113-1** (0.03 g, 28.9%) was prepared from intermediate **E106-3** using the method described in intermediate **4-d.**

LC-MS (ESI): m/z 541.2 [M+H]⁺.

### E-113: 1-cyclobutyl-N-((2-((4-(5-(2,5-dihydro-1H-pyrrol-1-yl)pyridin-3-yl)-1H-1,2,3-triazol-1-yl)methyl)imidazo[1,2-a]pyridin-6-yl)methyl)methylamine

A yellow solid compound **E-113** (10.7 mg, 39.4%) was prepared from intermediate **E113-1** using the method described in compound **E-1.**

LC-MS (ESI): m/z 441.2 [M+H]⁺ . ¹H NMR (400 MHz, DMSO-*d*₆): *δ* 9.42 (br s, 2H), 9.07 (s, 1H), 8.88 (s, 1H), 8.54 (s, 1H), 8.32 (s, 1H), 8.06 (d, *J =* 2.4 Hz, 1H), 7.94 (s, 1H), 7.80-7.73 (m, 2H), 6.10 (s, 2H), 5.95 (s, 2H), 4.27 (s, 4H), 4.19-4.16 (m, 2H), 2.97-2.92 (m, 2H), 2.72-2.68 (m, 1H), 2.12-2.02 (m, 2H), 1.89-1.76 (m, 4H).

### Example 114: Synthesis of E-114: 1-(2-((4-(5-(1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

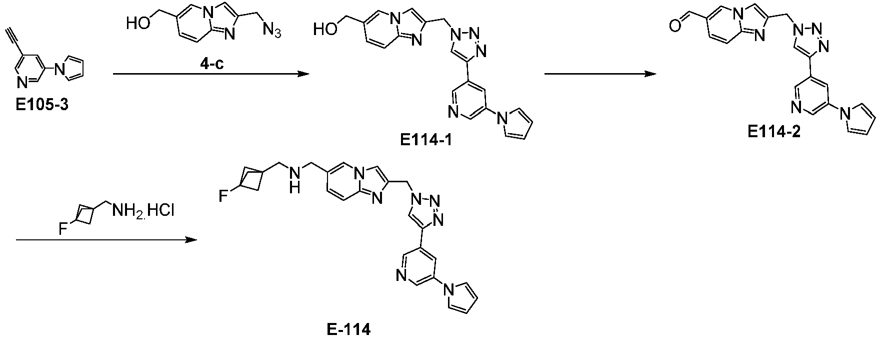

### E114-1: (2-((4-(5-(1H-pyrrol-1-yl)pyridin-3-yl)-1H-1,2,3-triazol-1-yl)methyl)imidazo[1,2-a]pyridin-6-yl)methanol

Yellow solid **E114-1** (180 mg, crude product) was prepared from **E105-3** and **4-c** using the method described in **4-d.**

LC-MS: [M+H]⁺= 372.2.

### E114-2: 2-((4-(5-(1H-pyrrol-1-yl)pyridin-3-yl)-1H-1,2,3-triazol-1-yl)methyl)imidazo[1,2-a]pyridine-6-formaldehyde

White solid **E114-2** (152 mg, crude product) was prepared from **E114-1** using the method described in **E54-2.**

LC-MS: [M+H]⁺= 370.2.

### E-114: 1-(2-((4-(5-(1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

Yellow solid **E-114** (32.5 mg, 42.1%) was prepared from **E114-2** using the method described in **E6-1.**

LC-MS: [M+H]⁺= 469.0. ¹H NMR (400 MHz, DMSO-*d*₆): δ 8.97 (s, 1H), 8.88-8.85 (m, 1H), 8.83 (s, 1H), 8.42 (d, *J =* 14.6 Hz, 2H), 8.02-7.99 (m, 1H), 7.56 (s, 2H), 7.48 (d, *J =* 9.4 Hz, 1H), 7.27 (d, *J =* 8.6 Hz, 1H), 6.34 (s, 2H), 5.79 (s, 2H), 3.69 (br s, 2H), 2.75 (br s, 2H), 1.94 (d, *J* = 2.6 Hz, 6H).

### Example 116: Synthesis of E-116: 3-(1-((6-(((cyclobutylmethyl)amino)methyl)imidazo[1,2-a]pyridin-2-yl)methyl)-1H-1,2,3-triazole-4-yl)-5-nitropyridin-4-amine

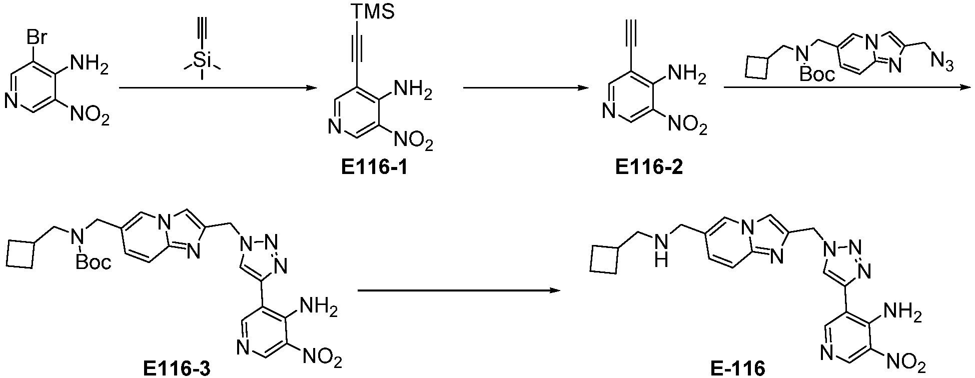

### E116-1: 3-nitro-5-((trimethylsilyl)ethynyl)pyridin-4-amine

A black solid intermediate **E116-1** (8.50 g, 78.8%) was prepared from 3-bromo-5-nitropyridine-4-amine using the method described in intermediate **2-b.**

LC-MS (ESI): m/z 236.1 [M+H]⁺.

### E116-2: 3-ethynyl-5-nitropyridine-4-amine

A black solid intermediate **E116-2** (5.20 g, 88.2%) was prepared from intermediate **E116-1** using the method described in intermediate **I-2.**

LC-MS (ESI): m/z 164.1 [M+H]⁺.

### E116-3: Tert-butyl (2-((4-(4-amino-5-nitropyridin-3-yl)-1H-1,2,3-triazol-1-yl)methyl)imidazo[1,2-a]pyridin-6-yl)methyl)(cyclobutylmethyl)carbamate

A white solid intermediate **E116-3** (1.30 g, 90.3%) was prepared from intermediate **E116-2** using the method described in intermediate **4-d.**

LC-MS (ESI): m/z 534.2 [M+H]⁺.

### E-116: 3-(1-((6-(((cyclobutylmethyl)amino)methyl)imidazo[1,2-a]pyridin-2-yl)methyl)-1H-1,2,3-triazole-4-yl)-5-nitropyridin-4-amine

A yellow solid compound **E-116** (40.2 mg, 22.8%) was prepared from intermediate **E116-3** using the method described in compound **E-1.**

LC-MS (ESI): m/z 434.1 [M+H]⁺ . ¹H NMR (400 MHz, DMSO-*d*₆): *δ* 9.51 (br s, 2H), 9.23 (s, 1H), 9.12 (s, 1H), 8.95 (s, 1H), 8.84 (s, 1H), 8.43 (s, 1H), 7.9-7.89 (m, 1H), 7.87-7.82 (m, 1H), 6.04 (s, 2H), 4.20 (br s, 2H), 3.04-2.84 (m, 2H), 2.77-2.59 (m, 1H), 2.12-1.98 (m, 2H), 1.90-1.69 (m, 4H).

### Example 119: Synthesis of E-119: N-((2-((4,4-dimethylpiperidin-1-yl)methyl)-1H-indole-6-yl)methyl)-5-(pyrrolidin-1-yl)nicotinamide

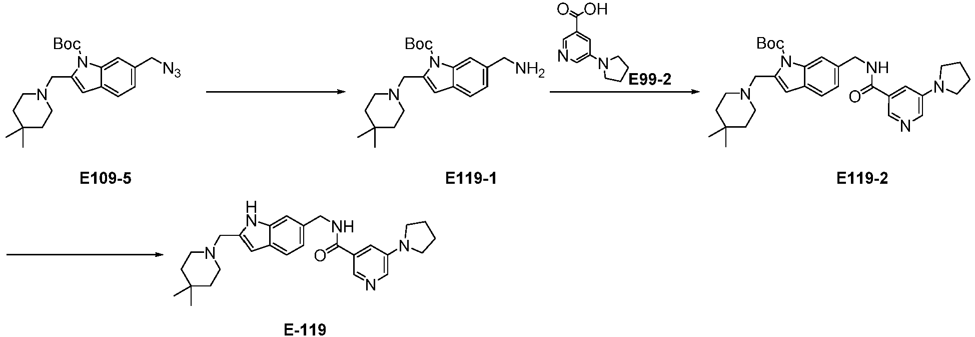

### E119-1: Tert-butyl 6-(aminomethyl)-2-((4,4-dimethylpiperidin-1-yl)methyl)-1H-indole-1-carboxylate

A white solid intermediate **E119-1** (0.07 g, 37.5%) was prepared from intermediate **E109-5** using the method described in intermediate **I-10.**

LC-MS (ESI): m/z 372.4 [M+H]⁺.

### E119-2: Tert-butyl 2-((4,4-dimethylpiperidin-1-yl)methyl)-6-((5-(pyrrolidin-1-yl)nicotinamido)methyl)-1H-indole-1-carboxylate

A white solid intermediate **E119-2** (0.05 g, 48.6%) was prepared from intermediate **E119-1** using the method described in intermediate **E101-2.**

LC-MS (ESI): m/z 546.1 [M+H]⁺.

### E-119: N-((2-((4,4-dimethylpiperidin-1-yl)methyl)-1H-indole-6-yl)methyl)-5-(pyrrolidin-1-yl)nicotinamide

A white solid compound **E-119** (12.7 mg, 30.8%) was prepared from intermediate **E119-2** using the method described in compound **E-1.**

LC-MS (ESI): m/z 446.3 [M+H]⁺. ¹H NMR (400 MHz, DMSO-*d*₆): *δ* 10.88 (s, 1H), 9.04 (t, *J* = 5.9 Hz, 1H), 8.31 (d, *J =* 1.6 Hz, 1H), 8.03 (d, *J =* 2.8 Hz, 1H), 7.37 (d, *J =* 8.0 Hz, 1H), 7.30-7.26 (m, 2H), 6.93 (dd, *J =* 1.3, 8.0 Hz, 1H), 6.20 (s, 1H), 4.53 (d, *J =* 5.9 Hz, 2H), 3.56 (s, 2H), 3.30-3.27 (m, 4H), 2.36-2.32 (m, 4H), 1.99-1.94 (m, 4H), 1.32 (t, *J* = 5.6 Hz, 4H), 0.87 (s, 6H).

### Example 120: Synthesis of E-120: 5-(1-((6-(((cyclobutylmethyl)amino)methyl)imidazo[1,2-a]pyridin-2-yl)methyl)-1H-1,2,3-triazole-4-yl)pyridine-3,4-diamine

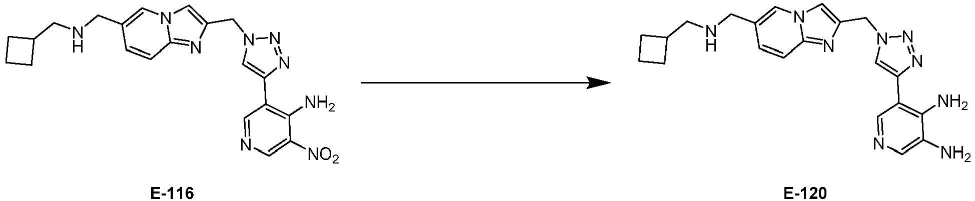

### E-120: 5-(1-((6-(((cyclobutylmethyl)amino)methyl)imidazo[1,2-a]pyridin-2-yl)methyl)-1H-1,2,3-triazole-4-yl)pyridine-3,4-diamine

A white solid compound **E-120** (14.9 mg, 38.2%) was prepared from compound **E-116** using the method described in intermediate **E3-2.**

LC-MS (ESI): m/z 404.2 [M+H]⁺ . ¹H NMR (400 MHz, DMSO-*d*₆): *δ* 8.64 (s, 1H), 8.45 (s, 1H), 8.21 (br s, 1H), 8.05- 7.91 (m, 2H), 7.61 (s, 1H), 7.47 (d, *J =* 9.3 Hz, 1H), 7.27 (d, *J* = 9.4 Hz, 1H), 6.53 (br s, 2H), 5.75 (s, 2H), 3.71 (s, 2H), 2.55 (d, *J =* 6.8 Hz, 2H), 2.46-2.37 (m, 1H), 2.03-1.92 (m, 2H), 1.87-1.73 (m, 2H), 1.69-1.56 (m, 2H).

### Example 121: Synthesis of E-121: 1-(3-fluorobicyclo[1.1.1]pentan-1-yl)-N-((6-((4-(5-(pyrrolidin-1-yl)pyridin-3-yl)-1H-1,2,3-triazol-1-yl)methyl)-1H-indole-2-yl)methyl)methylamine

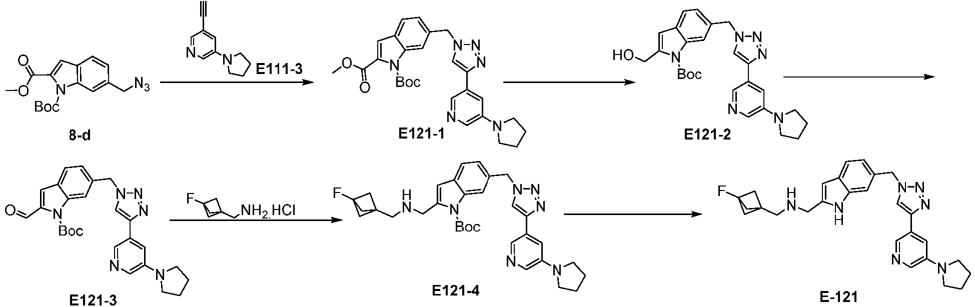

### E121-1: 1-(tert-butyl)2-methyl 6-((4-(5-(pyrrolidin-1-yl)pyridin-3-yl)-1H-1,2,3-triazol-1-yl)methyl)-1H-indol-1,2-dicarboxylate

A white solid intermediate **E121-1** (0.95 g, 56.4%) was prepared from intermediate **8-d** using the method described in intermediate **4-d.**

LC-MS (ESI): m/z 503.3 [M+H]⁺.

### E121-2: Tert-butyl 2-(hydroxymethyl)-6-((4-(5-(pyrrolidin-1-yl)pyridin-3-yl)-1H-1,2,3-triazol-1-yl)methyl)-1H-indole-1-carboxylate

A white solid intermediate **E121-2** (95.0 mg g, 44.6%) was prepared from intermediate **E121-1** using the method described in intermediate **4-b.**

LC-MS (ESI): m/z 475.1 [M+H]⁺.

### E121-3: Tert-butyl 2-formyl-6-((4-(5-(pyrrolidin-1-yl)pyridin-3-yl)-1H-1,2,3-triazol-1-yl)methyl)-1H-indole-1-carboxylate

A white solid intermediate **E121-3** (0.07 g, 57.8%) was prepared from intermediate **E121-2** using the method described in intermediate **E54-2.**

LC-MS (ESI): m/z 473.1 [M+H]⁺.

### E121-4: Tert-butyl 2-((((3-fluorobicyclo[1.1.1]pentan-1-yl)methyl)amino)-6-((4-(5-(pyrrolidin-1-yl)pyridin-3-yl)-1H-1,2,3-triazol-1-yl)methyl)-1H-indole-1-carboxylate

A yellow solid intermediate **E121-4** (20.0 mg, 25.6%) was prepared from intermediate **E121-3** using the method described in intermediate **E6-1.**

LC-MS (ESI): m/z 572.2 [M+H]⁺.

### E-121: 1-(3-fluorobicyclo[1.1.1]pentan-1-yl)-N-((6-((4-(5-(pyrrolidin-1-yl)pyridin-3-yl)-1H-1,2,3-triazol-1-yl)methyl)-1H-indole-2-yl)methyl)methylamine

A yellow solid compound **E-121** (4.00 mg, 22.5%) was prepared from intermediate **E121-4** using the method described in compound **E-3.**

LC-MS (ESI): m/z 472.2 [M+H]⁺. ¹H NMR (400 MHz, DMSO-*d*₆): *δ* 11.64 (s, 1H), 9.63 (br s, 2H), 8.97 (s, 1H), 8.45 (s, 1H), 8.01 (d, *J* = 2.5 Hz, 1H), 7.88 (s, 1H), 7.57 (d, *J =* 8.3 Hz, 1H), 7.48 (s, 1H), 7.07 (dd, *J =* 1.3, 8.3 Hz, 1H), 6.62 (s, 1H), 5.77 (s, 2H), 4.32 (br s, 2H), 3.44 (br s, 2H), 3.24 (br s, 4H), 2.11 (d, *J =* 2.5 Hz, 6H), 2.00 (t, *J =* 6.6 Hz, 4H).

### Example 122: Synthesis of E-122: 1-(3-fluorobicyclo[1.1.1]pentan-1-yl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine

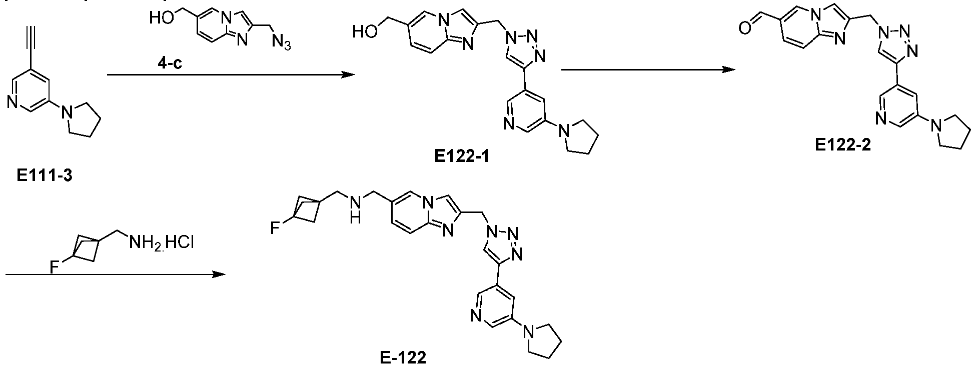

### E122-1: (2-((4-(5-(pyrrolidin-1-yl)pyridin-3-yl)-1H-1,2,3-triazol-1-yl)methyl)imidazo[1,2-a]pyridin-6-yl)methanol

A brown solid intermediate **E122-1** (240.0 mg, crude product) was prepared from intermediate **E111-3** using the method described in intermediate **4-d.**

LC-MS (ESI): m/z 376.0 [M+H]⁺.

### E122-2: 2-((4-(5-(pyrrolidin-1-yl)pyridin-3-yl)-1H-1,2,3-triazol-1-yl)methyl)imidazo[1,2-a]pyridine-6-formaldehyde

A yellow solid intermediate **E122-2** (0.04 g, 20.1%) was prepared from intermediate **E122-1** using the method described in intermediate **E54-2.**

LC-MS (ESI): m/z 374.3 [M+H]⁺.

### E-122: 1-(3-fluorobicyclo[1.1.1]pentan-1-yl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine

A pink solid compound **E-122** (11.3 mg, 14.8%) was prepared from intermediate **E122-2** using the method described in intermediate **E6-1.**

LC-MS (ESI): m/z 473.2 [M+H]⁺ . ¹H NMR (400 MHz, DMSO-*d*₆): *δ* 8.67 (s, 1H), 8.42 (s, 1H), 8.30 (d, *J =* 1.4 Hz, 1H), 7.95 (s, 1H), 7.88 (d, *J =* 2.6 Hz, 1H), 7.46 (d, *J =* 9.1 Hz, 1H), 7.29 (d, *J =* 1.6 Hz, 1H), 7.27-7.23 (m, 1H), 5.73 (s, 2H), 3.67 (s, 2H), 3.31-3.28 (m, 4H), 2.74 (s, 2H), 1.97 (t, *J* = 6.4 Hz, 4H), 1.93 (d, *J =* 2.8 Hz, 6H).

### Example 123: Synthesis of E-123: N-((6-((4,4-dimethylpiperidin-1-yl)methyl)imidazo[1,2-a]pyridin-2-yl)methyl)-5-(pyrrolidin-1-yl)nicotinamide

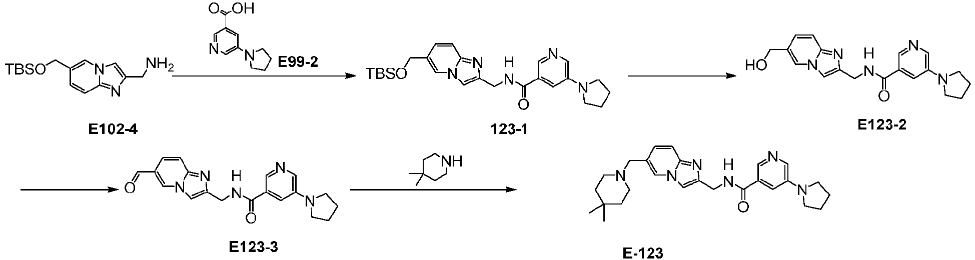

### E123-1: N-((6-(((tert-butyldimethylsilyl)oxy)methyl)imidazo[1,2-a]pyridin-2-yl)methyl)-5-(pyrrolidin-1-yl)nicotinamide

A brown solid intermediate **E123-1** (0.16 g, 33.4%) was prepared from intermediates **E102-4** and **E99-2** using the method described in intermediate **E101-2.**

LC-MS (ESI): m/z 466.0 [M+H]⁺.

### E123-2: N-((6-(hydroxymethyl)imidazo[1,2-a]pyridin-2-yl)methyl)-5-(pyrrolidin-1-yl)nicotinamide

A white solid intermediate **E123-2** (0.07 g, 58.0%) was prepared from intermediate **E123-1** using the method described in intermediate **E102-6.**

LC-MS (ESI): m/z 352.0 [M+H]⁺.

### E123-3: n-((6-formylimidazole[1,2-a]pyridin-2-yl)methyl)-5-(pyrrolidin-1-yl)nicotinamide

A white solid intermediate **E123-3** (0.03 g, 43.1%) was prepared from intermediate **E123-2** using the method described in intermediate I**-4.**

LC-MS (ESI): m/z 350.0 [M+H]⁺.

### E-123: N-((6-((4,4-dimethylpiperidin-1-yl)methyl)imidazo[1,2-a]pyridin-2-yl)methyl)-5-(pyrrolidin-1-yl)nicotinamide

A white solid compound **E-123** (16.4 mg, 30.7%) was prepared from intermediate **E123-3** using the method described in intermediate **E30-1.**

LC-MS (ESI): m/z 447.2 [M+H]⁺ . ¹H NMR (400 MHz, DMSO-*d*₆): *δ* 9.09 (t, *J* = 5.7 Hz, 1H), 8.37 (s, 1H), 8.31 (d, *J =* 1.6 Hz, 1H), 8.04 (d, *J* = 2.8 Hz, 1H), 7.76 (s, 1H), 7.42 (d, *J =* 9.3 Hz, 1H), 7.31-7.28 (m, 1H), 7.15 (dd, *J* = 1.6, 9.2 Hz, 1H), 4.55 (d, *J =* 5.6 Hz, 2H), 3.42 (s, 2H), 3.31-3.28 (m, 4H), 2.33 (br s, 4H), 2.00-1.93 (m, 4H), 1.31 (t, *J =* 5.5 Hz, 4H), 0.88 (s, 6H).

### Example 124: Synthesis of E-124: N-((6-(((cyclobutylmethyl)amino)methyl)imidazo[1,2-a]pyridin-2-yl)methyl)-5-(piperidin-1-yl)nicotinamide

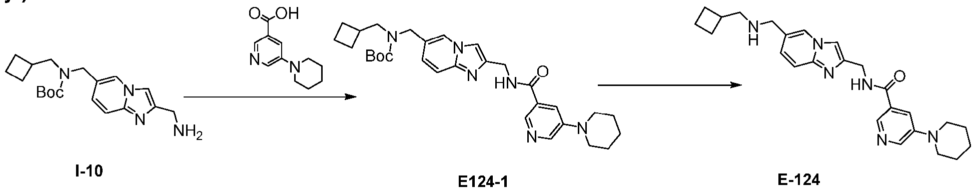

### E124-1: Tert-butyl (2-((5-(piperidin-1-yl)nicotinamido)methyl)imidazo[1,2-a]pyridin-6-yl)methyl)carbamate

A white solid intermediate **E124-1** (0.08 g, 49.0%) was prepared from intermediate I**-10** using the method described in intermediate **E101-2.**

**LC-MS (ESI):** m/z 533.1 [M+H]⁺.

### E-124: N-((6-(((cyclobutylmethyl)amino)methyl)imidazo[1,2-a]pyridin-2-yl)methyl)-5-(piperidin-1-yl)nicotinamide

A yellow solid compound **E-124** (33.8 mg, 48.0%) was prepared from intermediate **E124-1** using the method described in compound **E-3.**

LC-MS (ESI): m/z 433.2 [M+H]⁺. ¹H NMR (400 MHz, DMSO-*d*₆): *δ* 10.06 (t, *J =* 5.3 Hz, 1H), 9.65 (br s, 2H), 9.04 (s, 1H), 8.54 (d, *J =* 2.8 Hz, 1H), 8.52 (s, 1H), 8.43 (s, 1H), 8.35 (br s, 1H), 8.18 (d, *J =* 9.3 Hz, 1H), 8.01 (d, *J =* 9.3 Hz, 1H), 4.77 (d, *J =* 5.4 Hz, 2H), 4.25 (t, *J* = 5.3 Hz, 2H), 3.46 (br s, 4H), 3.00-2.91 (m, 2H), 2.77-2.66 (m, 1H), 2.09-2.00 (m, 2H), 1.79 (dd, *J* = 5.0, 8.4 Hz, 4H), 1.62 (br s, 6H).

### Example 125: Synthesis of E-125: N-((6-(((cyclobutylmethyl)amino)methyl)imidazo[1,2-a]pyridin-2-yl)methyl)-5-fluoronicotinamide

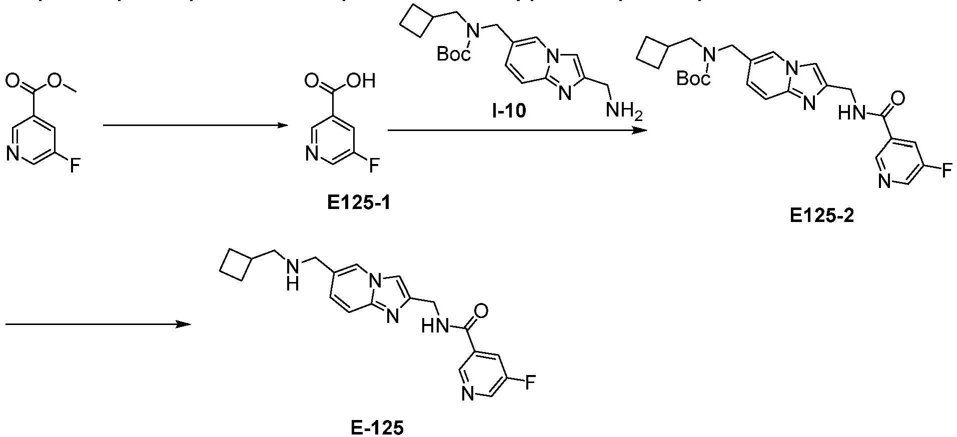

### E125-1: 5-fluoronicotinic acid

A white solid intermediate **E125-1** (1.50 g, crude product) was prepared from methyl 5-fluoricotinate using the method described in intermediate **E7-2.**

LC-MS (ESI): m/z 142.1 [M+H]⁺.

### E125-2: Tert-butyl (cyclobutylmethyl)((2-((5-fluoronicotinamido)methyl)imidazo[1,2-a]pyridin-6-yl)methyl)carbamate

A yellow solid intermediate **E125-2** (0.16 g, 54.3%) was prepared from intermediate **E125-1** using the method described in intermediate **E101-2.**

LC-MS (ESI): m/z 468.0 [M+H]⁺.

### E-125: N-((6-(((cyclobutylmethyl)amino)methyl)imidazo[1,2-a]pyridin-2-yl)methyl)-5-fluoronicotinamide

A yellow solid compound **E-125** (5.4 mg, 17.2%) was prepared from intermediate **E125-2** using the method described in compound **E-3.**

LC-MS (ESI): m/z 367.9 [M+H]⁺ . ¹H NMR (400 MHz, DMSO-*d*₆): *δ* 9.32 (t, *J* = 5.6 Hz, 1H), 8.94 (s, 1H), 8.78-8.73 (m, 1H), 8.75 (d, *J =* 2.6 Hz, 1H), 8.35 (s, 1H), 8.17-8.09 (m, 1H), 7.81 (s, 1H), 7.43 (d, *J =* 9.1 Hz, 1H), 7.21 (d, *J =* 9.3 Hz, 1H), 4.59 (d, *J =* 5.6 Hz, 2H), 3.64 (s, 2H), 2.49-2.47 (m, 2H), 2.39 (td, *J =* 7.6, 14.8 Hz, 1H), 2.02-1.92 (m, 2H), 1.86-1.73 (m, 2H), 1.66-1.56 (m, 2H).

### Example 127: Synthesis of E-127: 1-(6-((4-(5-(1H-pyrazol-1-yl)pyridin-3-yl)-1H-1,2,3-triazol-1-yl)methyl)-1H-indole-2-yl)-N-(cyclobutylmethyl)methylamine

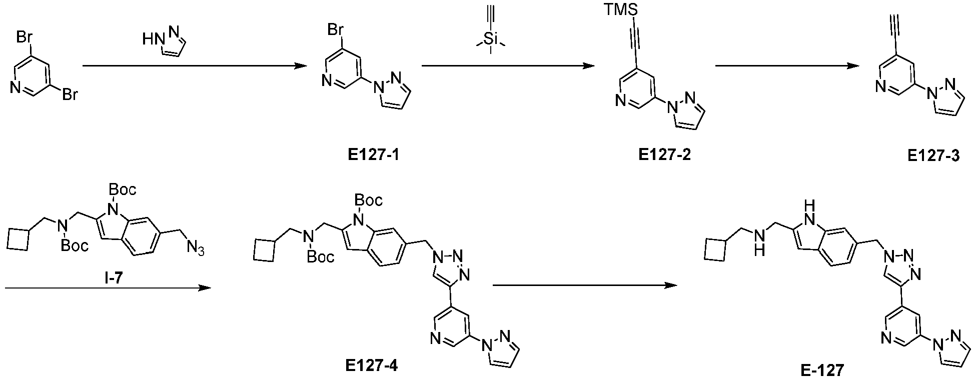

### E127-1: 3-bromo-5-(1H-pyrazol-1-yl)pyridine

K₂CO₃ (2.92 g, 21.1 mmol, 2.50 eq), Cul (161 mg, 844 µmol, 0.10 eq), and 1,10-phenanthroline (304 mg, 1.69 mmol, 0.20 eq) were added to a solution of 3,5-dibromopyridine (2.00 g, 8.44 mmol, 1.00 eq) and 1H-pyrazole (632 mg, 9.29 mmol, 1.10 eq) in DMF (40.0 mL), and the resulting mixture was stirred at 120°C for 16 h under nitrogen protection. The mixture was quenched with water (200 mL) and extracted with DCM (50.0 mL * 3). The combined organic layers were dried over anhydrous sodium sulfate, filtered and concentrated. The residue was purified by preparative thin-layer chromatography and eluted with EtOAc/petroleum ether (1:10) to obtain a yellow solid intermediate **E127-1** (0.50 g, 26.4%).

LC-MS (ESI): m/z 224.0 [M+H]⁺.

### E127-2: 3-(1H-pyrazol-1-yl)-5-((trimethylsilyl)ethynyl)pyridine

A yellow solid intermediate **E127-2** (0.45 g, 83.6%) was prepared from intermediate **E127-1** using the method described in intermediate **2-b.**

LC-MS (ESI): m/z 242.1 [M+H]⁺.

### E127-3: 3-ethynyl-5-(1H-pyrazol-1-yl)pyridine

A yellow solid intermediate **E127-3** (0.23 g, 92.3%) was prepared from intermediate **E127-2** using the method described in intermediate **I-2.**

LC-MS (ESI): m/z 170.1 [M+H]⁺.

### E127-4: Tert-butyl 6-((4-(5-(1H-pyrazol-1-yl)pyridin-3-yl)-1H-1,2,3-triazol-1-yl)methyl)-2-(((tert-butoxycarbonyl)(cyclobutylmethyl)amino)methyl)-1H-indole-1-carboxylate

A brown solid intermediate **E127-4** (0.06 g, 88.2%) was prepared from intermediate **E127-3** using the method described in intermediate **4-d.**

LC-MS (ESI): m/z 639.3 [M+H]⁺.

### E-127: 1-(6-((4-(5-(1H-pyrazol-1-yl)pyridin-3-yl)-1H-1,2,3-triazol-1-yl)methyl)-1H-indole-2-yl)-N-(cyclobutylmethyl)methylamine

A white solid compound **E-127** (17.9 mg, 40.1%) was prepared from intermediate **E127-4** using the method described in compound **E-1.**

LC-MS (ESI): m/z 438.9 [M+H]⁺ . ¹H NMR (400 MHz, DMSO-*d*₆): *δ* 11.62 (br s, 1H), 9.43 (br s, 2H), 9.12 (d, *J =* 2.4 Hz, 1H), 9.03 (d, *J =* 1.8 Hz, 1H), 8.93 (s, 1H), 8.77 (d, *J* = 1.9 Hz, 1H), 8.73 (d, *J* = 2.5 Hz, 1H), 7.87 (d, *J =* 1.4 Hz, 1H), 7.57 (d, *J =* 8.1 Hz, 1H), 7.49 (s, 1H), 7.08 (dd, *J =* 1.3, 8.1 Hz, 1H), 6.66-6.63 (m, 1H), 6.61 (s, 1H), 5.77 (s, 2H), 4.27 (t, *J =* 5.2 Hz, 2H), 2.96-2.87 (m, 2H), 2.66 (td, *J =* 7.5, 14.6 Hz, 1H), 2.09-1.98 (m, 2H), 1.87-1.72 (m, 4H).

### Example 129: Synthesis of E-129: N-((6-(((cyclobutylmethyl)amino)methyl)imidazo[1,2-a]pyridin-2-yl)methyl)-N-methyl-5-(pyrrolidin-1-yl)nicotinamide hydrochloride

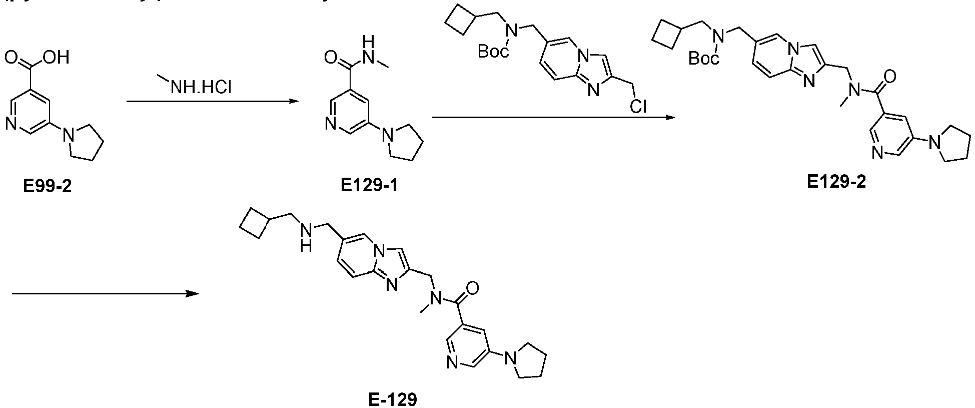

### E129-1: Methyl-5-(pyrrolidin-1-yl)nicotinamide

A white solid intermediate **E129-1** (0.06 g, 54.8%) was prepared from intermediate **E99-2** using the method described in intermediate **E101-2.**

LC-MS (ESI): m/z 206.2 [M+H]⁺.

### E129-2: Tert-butyl (2-((N-methyl-5-(pyrrolidin-1-yl)nicotinamido)methyl)imidazo[1,2-a]pyridin-6-yl)methyl)carbamate

A yellow solid intermediate **E129-2** (0.10 g, 91.5%) was prepared from intermediate **E129-1** using the method described in intermediate **7-a.**

LC-MS (ESI): m/z 533.0 [M+H]⁺.

### E-129: N-((6-(((cyclobutylmethyl)amino)methyl)imidazo[1,2-a]pyridin-2-yl)methyl)-N-methyl-5-(pyrrolidin-1-yl)nicotinamide hydrochloride

A yellow solid compound **E-129** (14.9 mg, 32.8%) was prepared from intermediate **E129-2** using the method described in compound **E-3.**

LC-MS (ESI): m/z 433.2 [M+H]⁺ . ¹H NMR (400 MHz, DMSO-*d*₆): *δ* 9.71-9.49 (m, 2H), 9.14-8.91 (m, 1H), 8.58-8.36 (m, 1H), 8.30-7.90 (m, 4H), 7.88-7.72 (m, 1H), 5.01-4.67 (m, 2H), 4.27 (br s, 2H), 3.39 (br s, 4H), 3.04 (s, 2H), 3.02-2.93 (m, 3H), 2.78-2.68 (m, 1H), 2.11-1.94 (m, 6H), 1.80 (dd, *J =* 5.3, 8.4 Hz, 4H).

### Example 130: Synthesis of E-130: 1-cyclobutyl-N-((2-((4-(4-methyl-5-(pyrrolidin-1-yl)pyridin-3-yl)-1H-1,2,3-triazol-1-yl)methyl)imidazo[1,2-a]pyridin-6-yl)methyl)methylamine

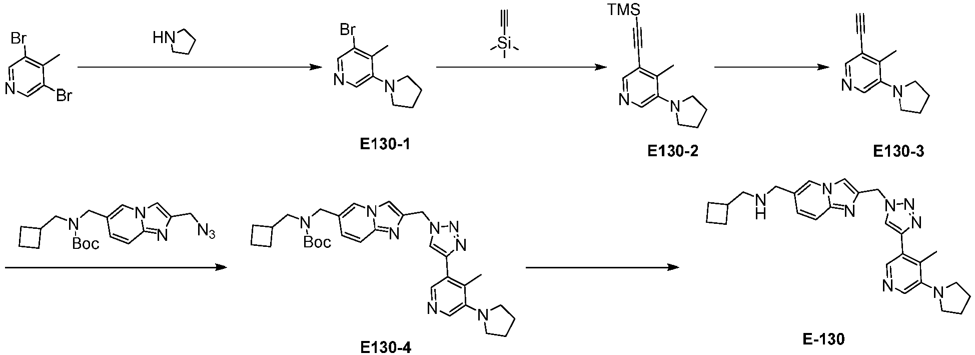

### E130-1: 3-bromo-4-methyl-5-(pyrrolidin-1-yl)pyridine

T-BuONa (3.45 g, 35.9 mmol, 2.00 eq), BINAP (558 mg, 897 µmol, 0.0500 eq), and Pd₂(dba)₃ (657 mg, 717 µmol, 0.04 eq) were added to a solution of 3,5-dibromo-4-methylpyridine (4.50 g, 17.9 mmol, 1.00 eq) and pyrrolidine (1.28 g, 17.9 mmol, 1.50 mL, 1.00 eq) in dioxane (45.0 mL), and the resulting mixture was stirred at 25°C for 16 h. The mixture was concentrated, and the residue was purified by column chromatography (petroleum ether/ethyl acetate=30/1 to 10/1) to obtain a yellow solid intermediate **E130-1** (2.00 g, 46.2%).

¹H NMR (400 MHz, chloroform-*d*): *δ* 8.20-8.15 (m, 1H), 8.03-7.97 (m, 1H), 3.15 ( t, *J* = 6.44 Hz, 4H), 2.35-2.27 (m, 3H), 1.94-1.84 (m, 4H).

### E130-2: 4-methyl-3-(pyrrolidin-1-yl)-5-((trimethylsilyl)ethynyl)pyridine

A brown oily intermediate **E130-2** (0.60 g, 56.0%) was prepared from intermediate **E130-1** using the method described in intermediate **E53-6.**

LC-MS (ESI): m/z 259.1 [M+H]⁺.

### E130-3: 3-ethynyl-4-methyl-5-(pyrrolidin-1-yl)pyridine

A yellow solid intermediate **E130-3** (0.05 g, 69.4%) was prepared from intermediate **E130-2** using the method described in intermediate **I-2.**

¹H NMR (400 MHz, chloroform-*d*): *δ* 8.43-7.91 (m, 2H), 3.29-3.25 (m, 1H), 3.17 (t, *J* = 6.50 Hz, 4 H), 2.39-2.32 (m, 3H), 1.94-1.84 (m, 4H).

### E130-4: Tert-butyl (2-((4-(4-methyl-5-(pyrrolidin-1-yl)pyridin-3-yl)-1H-1,2,3-triazol-1-yl)methyl)imidazo[1,2-a]pyridin-6-yl)methyl)carbamate

A yellow solid intermediate **E130-4** (0.10 g, 66.5%) was prepared from intermediate **E130-3** using the method described in intermediate **4-d.**

LC-MS (ESI): m/z 557.3 [M+H]⁺.

### E-130: 1-cyclobutyl-N-((2-((4-(4-methyl-5-(pyrrolidin-1-yl)pyridin-3-yl)-1H-1,2,3-triazol-1-yl)methyl)imidazo[1,2-a]pyridin-6-yl)methyl)methylamine

A yellow solid compound **E-130** (54.1 mg, 59.3%) was prepared from intermediate **E130-4** using the method described in compound **E-1.**

LC-MS (ESI): m/z 457.2 [M+H]⁺ . ¹H NMR (400 MHz, DMSO-*d*₆): *δ* 9.51-9.35 (m, 2H), 8.92-8.87 (m, 1H), 8.87-8.83 (m, 1H), 8.55-8.52 (m, 1H), 8.37-8.32 (m, 1H), 8.20-8.16 (m, 1H), 7.90-7.72 (m, 2H), 6.01-5.95 (m, 2H), 4.19 (t, *J =* 5.1 Hz, 2H), 3.46-3.36 (m, 4H), 3.01-2.91 (m, 2H), 2.71-2.65 (m, 1H), 2.56 (s, 3H), 2.10-2.01 (m, 2H), 2.01-1.91 (m, 4H), 1.89-1.73 (m, 4H).

### Example 131: Synthesis of E-131: 1-cyclobutyl-N-((2-((4-(5-(3,3-difluoropyrrolidin-1-yl)pyridin-3-yl)-1H-1,2,3-triazol-1-yl)methyl)imidazo[1,2-a]pyridin-6-yl)methyl)methylamine

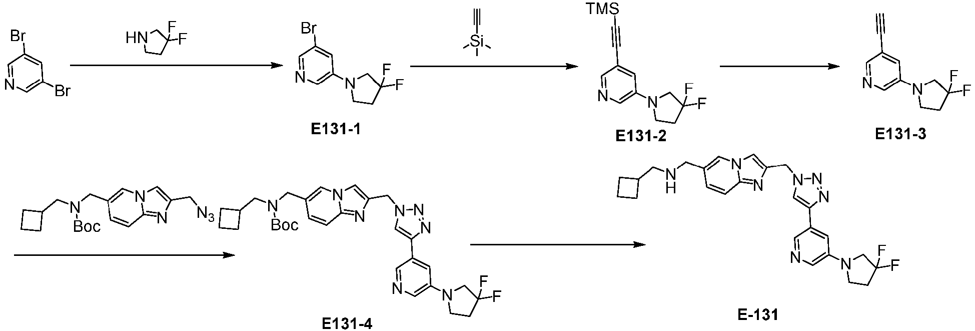

### E131-1: 3-bromo-5-(3,3-difluoropyrrolidin-1-yl)pyridine

A brown solid intermediate **E131-1** (0.17 g, 76.5%) was prepared from 3,5-dibromopyridine using the method described in intermediate **E130-1.**

LC-MS (ESI): m/z 262.9 [M+H]⁺.

### E131-2: 3-(3,3-difluoropyrrolidin-1-yl)-5-((trimethylsilyl)ethynyl)pyridine

A brown solid intermediate **E131-2** (0.15 g, 88.0%) was prepared from intermediate **E131-1** using the method described in intermediate **E53-6.**

LC-MS (ESI): m/z 281.1 [M+H]⁺.

### E131-3: 3-(3,3-difluoropyrrolidin-1-yl)-5-ethynylpyridine

A black liquid intermediate **E131-3** (0.04 g, crude product) was prepared as from intermediate **E131-2** using the method described in intermediate **I-2.**

LC-MS (ESI): m/z 209.0 [M+H]⁺.

### E131-4: Tert-butyl (2-((4-(5-(3,3-difluoropyrrolidin-1-yl)pyridin-3-yl)-1H-1,2,3-triazol-1-yl)methyl)imidazo[1,2-a]pyridin-6-yl)methyl)carbamate

A white solid intermediate **E131-4** (0.10 g, 89.9%) was prepared from intermediate **E131-3** using the method described in intermediate **4-d.**

LC-MS (ESI): m/z 579.0 [M+H]⁺.

### E-131: 1-cyclobutyl-N-((2-((4-(5-(3,3-difluoropyrrolid-1-yl)pyridin-3-yl)-1H-1,2,3-triazol-1-yl)methyl)imidazo[1,2-a]pyridin-6-yl)methyl)methylamine

A white solid compound **E-131** (29.8 mg, 33.3%) was prepared from intermediate **E131-4** using the method described in compound **E-1.**

LC-MS (ESI): m/z 479.2 [M+H]⁺ . ¹H NMR (400 MHz, DMSO-*d*₆): *δ* 9.40 (br s, 2H), 9.05 (s, 1H), 8.89 (s, 1H), 8.60 (s, 1H), 8.35 (s, 1H), 8.14 (d, *J =* 2.5 Hz, 1H), 7.98 (s, 1H), 7.84-7.76 (m, 2H), 5.96 (s, 2H), 4.18 (t, *J* = 5.3 Hz, 2H), 3.95 (s, 2H), 3.70 (br s, 2H), 2.97-2.92 (m, 2H), 2.73-2.64 (m, 2H), 2.61 (d, *J =* 7.0 Hz, 1H), 2.08-2.01 (m, 2H), 1.87-1.74 (m, 4H).

### Example 132: Synthesis of E-132: 5-(azetidine-1-yl)-N-((6-(((cyclobutylmethyl)amino)methyl)imidazo[1,2-a]pyridin-2-yl)methyl)nicotinamide

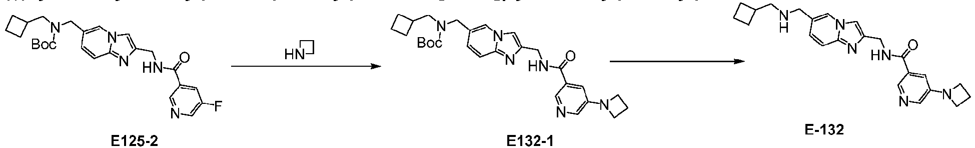

### E132-1: Tert-butyl ((2-((5-(azetidine-1-yl)nicotinamido)methyl)imidazo[1,2-a]pyridin-6-yl)methyl)(cyclobutylmethyl)carbamate

A yellow solid intermediate **E132-1** (0.05 g, 42.5%) was prepared from intermediate **E125-2** using the method described in intermediate **E16-1.**

LC-MS (ESI): m/z 505.0 [M+H]⁺.

### E-132: 5-(azetidine-1-yl)-N-((6-(((cyclobutylmethyl)amino)methyl)imidazo[1,2-a]pyridin-2-yl)methyl)nicotinamide

A yellow solid compound **E-132** (4.00 mg, 9.53%) was prepared from intermediate **E132-1** using the method described in compound **E-94.**

LC-MS (ESI): m/z 405.2 [M+H]⁺ .¹H NMR (400 MHz, DMSO-*d*₆): *δ* 9.09 (t, *J* = 5.8 Hz, 1H), 8.41-8.34 (m, 2H), 7.90 (d, *J =* 2.8 Hz, 1H), 7.76 (s, 1H), 7.42 (d, *J =* 9.3 Hz, 1H), 7.25-7.18 (m, 2H), 4.55 (d, *J =* 5.8 Hz, 2H), 3.91 (t, *J =* 7.3 Hz, 4H), 3.64 (s, 2H), 2.49 (br s, 2H), 2.42-2.31 (m, 3H), 2.02-1.92 (m, 2H), 1.86-1.72 (m, 2H), 1.67-1.56 (m, 2H).

### Example 133: Synthesis of E-133: 1-(6-((4-(5-(1H-imidazol-1-yl)pyridin-3-yl)-1H-1,2,3-triazol-1-yl)methyl)-1H-indole-2-yl)-N-(cyclobutylmethyl)methylamine

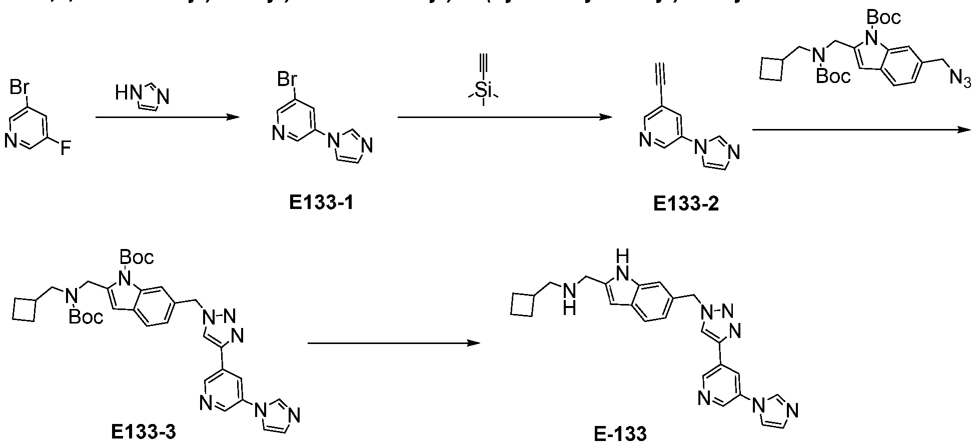

### E133-1: 3-bromo-5-(1H-imidazol-1-yl)pyridine

A yellow solid intermediate **E133-1** (0.15 g, 58.9%) was prepared from 3-bromo-5-fluoropyridine using the method described in intermediate **E16-1.**

LC-MS (ESI): m/z 223.9 [M+H]⁺.

### E133-2: 3-ethynyl-5-(1H-imidazol-1-yl)pyridine

A yellow solid intermediate **E133-2** (0.03 g, 22.5%) was prepared from intermediate **E133-1** using the method described in intermediate **2-b.**

LC-MS (ESI): m/z 170.1 [M+H]⁺.

### E133-3: Tert-butyl 6-((4-(5-(1H-imidazol-1-yl)pyridin-3-yl)-1H-1,2,3-triazol-1-yl)methyl)-2-((tert-butoxycarbonyl)(cyclobutylmethyl)amino)methyl)-1H-indole-1-carboxylate

A brown solid intermediate **E133-3** (0.02 g, 56.1%) was prepared from intermediate **E133-2** using the method described in intermediate **4-d.**

LC-MS (ESI): m/z 639.5 [M+H]⁺.

### E-133: 1-(6-((4-(5-(1H-imidazol-1-yl)pyridin-3-yl)-1H-1,2,3-triazol-1-yl)methyl)-1H-indole-2-yl)-N-(cyclobutylmethyl)methylamine

A white solid compound **E-133** (3.20 mg, 20.9%) was prepared from intermediate **E133-3** using the method described in compound **E-1.**

LC-MS (ESI): m/z 439.1 [M+H]⁺. ¹H NMR (400 MHz, DMSO-*d*₆): *δ* 11.63 (s, 1H), 9.83 (s, 1H), 9.42 (br s, 2H), 9.22 (d, *J =* 1.6 Hz, 1H), 9.01 (d, *J =* 2.5 Hz, 1H), 8.85 (s, 1H), 8.72 (t, *J* = 2.1 Hz, 1H), 8.41 (t, *J =* 1.6 Hz, 1H), 7.96 (s, 1H), 7.57 (d, *J* = 8.1 Hz, 1H), 7.48 (s, 1H), 7.07 (dd, *J =* 1.3, 8.2 Hz, 1H), 6.61 (s, 1H), 5.79 (s, 2H), 4.27 (t, J = 5.2 Hz, 2H), 2.96-2.89 (m, 2H), 2.68-2.64 (m, 1H), 2.08-2.00 (m, 2H), 1.87-1.71 (m, 4H).

### Example 134: Synthesis of E-134: 6-(piperidin-1-yl methyl)-2-((4-(5-(pyrrolidin-1-yl)pyridin-3-yl)-1H-1,2,3-triazol-1-yl)methyl)imidazo[1,2-a]pyridine

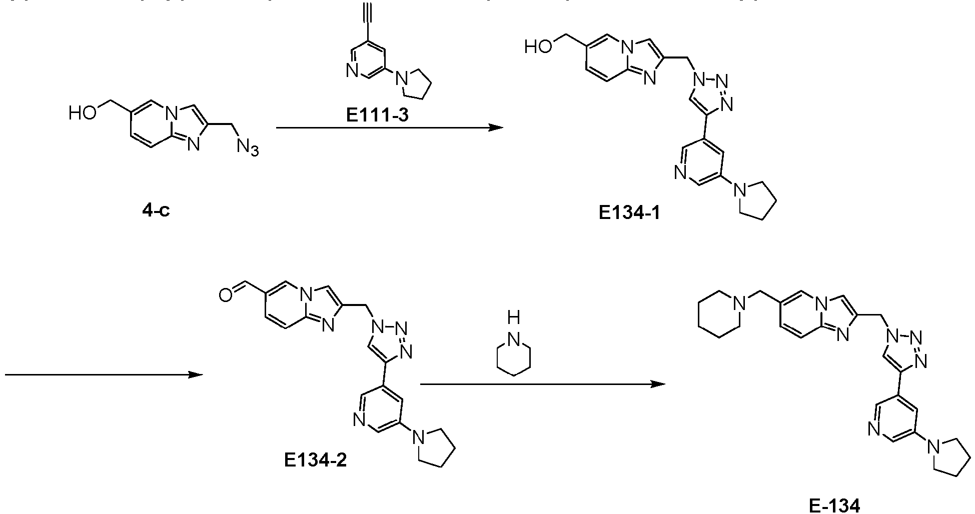

### E134-1: (2-((4-(5-(pyrrolidin-1-yl)pyridin-3-yl)-1H-1,2,3-triazol-1-yl)methyl)imidazo[1,2-a]pyridin-6-yl)methanol

A brown solid Intermediate **E134-1** (0.4 g, crude product) was prepared from intermediate **E111-3** using the method described in intermediate **4-d.**

LC-MS (ESI): m/z 376.0 [M+H]⁺.

### E134-2: 2-((4-(5-(pyrrolidin-1-yl)pyridin-3-yl)-1H-1,2,3-triazol-1-yl)methyl)imidazo[1,2-a]pyridin-6-formaldehyde

A yellow solid intermediate **E134-2** (0.07 g, 35.2%) was prepared from intermediate **E134-1** using the method described in intermediate **I-4.**

LC-MS (ESI): m/z 374.0 [M+H]⁺.

### E-134: 6-(piperidin-1-yl methyl)-2-((4-(5-(pyrrolidin-1-yl)pyridin-3-yl)-1H-1,2,3-triazol-1-yl)methyl)imidazo[1,2-a]pyridine

A yellow solid compound **E-134** (6.40 mg, 17.4%) was prepared from intermediate **E134-2** using the method described in intermediate **E30-1.**

LC-MS (ESI): m/z 443.2 [M+H]⁺ . ¹H NMR (400 MHz, DMSO-*d*₆): *δ* 8.67 (s, 1H), 8.43 (s, 1H), 8.31 (d, *J =* 1.8 Hz, 1H), 7.95 (s, 1H), 7.88 (d, *J =* 2.8 Hz, 1H), 7.46 (d, *J =* 9.3 Hz, 1H), 7.32-7.27 (m, 1H), 7.21 (dd, *J* = 1.6, 9.3 Hz, 1H), 5.73 (s, 2H), 3.39 (s, 2H), 3.31-3.27 (m, 4H), 2.33 (d, *J =* 1.8 Hz, 4H), 2.01-1.94 (m, 4H), 1.48 (td, *J =* 5.3, 10.5 Hz, 4H), 1.38 (d, *J =* 5.4 Hz, 2H).

### Example 136: Synthesis of E-136: 1-(6-((4-(5-(4H-1,2,4-triazol-4-yl)pyridin-3-yl)-1H-1,2,3-triazol-1-yl)methyl)-1H-indole-2-yl)-N-(cyclobutylmethyl)methylamine

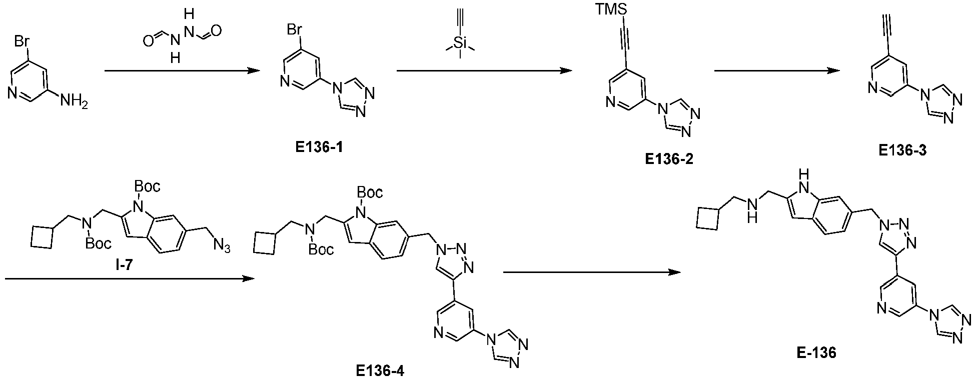

### E136-1: 3-bromo-5-(4H-1,2,4-triazol-4-yl)pyridine

A solution of 5-bromopyridine-3-amine (1.00 g, 5.78 mmol, 1.00 eq) and N'-formylhydrazide (509 mg, 5.78 mmol, 1.00 eq) was added into a sealed tube. The resulting mixture was stirred at 140°C for 16 h under nitrogen protection. The mixture was concentrated, and the residue was purified by column chromatography (petroleum ether/ethyl acetate=20/1 to 0/1) to obtain a brown solid intermediate **E136-1** (300 mg, 23.1%).

LC-MS (ESI): m/z 226.9 [M+H]⁺.

### E136-2: 3-(4H-1,2,4-triazol-4-yl)-5-((trimethylsilyl)ethynyl)pyridine

A brown oily intermediate **E136-2** (0.08 g, 74.3%) was prepared from intermediate **E136-1** using the method described in intermediate **2-b.**

LC-MS (ESI): m/z 243.2 [M+H]⁺.

### E136-3: 3-ethynyl-5-(4H-1,2,4-triazol-4-yl)pyridine

A black solution intermediate **E136-3** (35.00 mg, crude product) was prepared from intermediate **E136-2** using the method described in intermediate **I-2.**

LC-MS (ESI): m/z 171.2 [M+H]⁺.

### E136-4: Tert-butyl 6-((4-(5-(4H-1,2,4-triazol-4-yl)pyridin-3-yl)-1H-1,2,3-triazol-1-yl)methyl)-2-(((tert-butoxycarbonyl)(cyclobutylmethyl)amino)methyl)-1H-indole-1-carboxylate

A brown solid intermediate **E136-4** (50 mg, 38.0%) was prepared from intermediate **E136-3** using the method described in intermediate **4-d.**

LC-MS (ESI): m/z 640.4 [M+H]⁺.

### E-136: 1-(6-((4-(5-(4H-1,2,4-triazol-4-yl)pyridin-3-yl)-1H-1,2,3-triazol-1-yl)methyl)-1H-indole-2-yl)-N-(cyclobutylmethyl)methylamine

A yellow solid compound **E-136** (9.30 mg, 25.0%) was prepared from intermediate **E136-4** using the method described in compound **E-1.**

LC-MS (ESI): m/z 440.2 [M+H]⁺ . ¹H NMR (400 MHz, DMSO-*d*₆): *δ* 11.53 (s, 1H), 9.33 (s, 2H), 9.28 (br s, 2H), 9.15-9.08 (m, 1H), 8.95 (d, *J =* 2.4 Hz, 1H), 8.80 (s, 1H), 8.60 (t, *J* = 2.0 Hz, 1H), 7.58 (d, *J =* 8.3 Hz, 1H), 7.48 (s, 1H), 7.09-7.05 (m, 1H), 6.61 (s, 1H), 5.78 (s, 2H), 4.27 (t, *J =* 5.0 Hz, 2H), 2.98-2.90 (m, 2H), 2.68-2.62 (m, 1H), 2.07-2.00 (m, 2H), 1.86-1.73 (m, 4H).

### Example 139: Synthesis of E-139: 1-(2-((4-(1H-imidazo[4,5-c]pyridin-7-yl)-1H-1,2,3-triazol-1-yl)methyl)imidazo[1,2-a]pyridin-6-yl)-N-(cyclobutylmethyl)methylamine

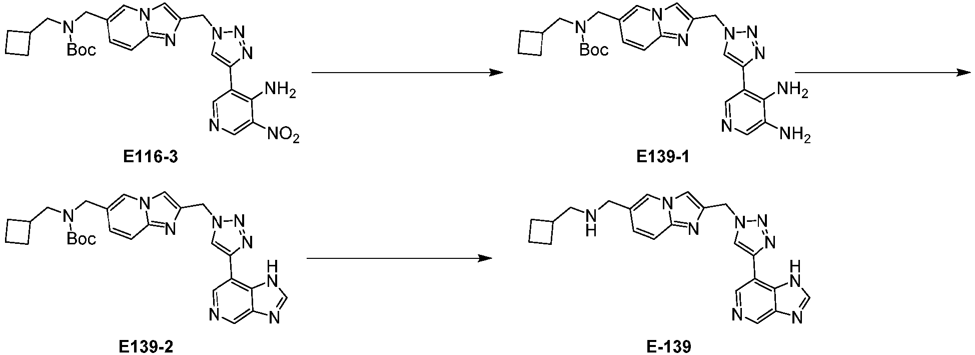

### E139-1: Tert-butyl (2-((4-(4,5-diaminopyridin-3-yl)-1H-1,2,3-triazol-1-yl)methyl)imidazo[1,2-a]pyridin-6-yl)methyl)carbamate

A brown solid intermediate **E139-1** (0.15 g, crude product) was prepared from intermediate **E116-3** using the method described in intermediate **E3-2.**

LC-MS (ESI): m/z 504.5 [M+H]⁺.

### E139-2: Tert-butyl (2-((4-(1H-imidazo[4,5-c]pyridin-7-yl)-1H-1,2,3-triazol-1-yl)methyl)imidazo[1,2-a]pyridin-6-yl)methyl)(cyclobutylmethyl)carbamate

Intermediate **E139-1** (100 mg, 199 µmol, 1.00 eq) and diethoxyethoxyethane (1.00 mL) were stirred at 140°C for 40 h. The mixture was diluted with water (10.0 mL) and extracted with ethyl acetate (3.00 mL * 3). The combined organic layers were washed with saline (2.00 mL), dried over anhydrous sodium sulfate, and concentrated. The residue was purified by preparative thin-layer chromatography (ethyl acetate: methanol=10:1) to obtain a colorless oily intermediate **E139-2** (50.0 mg, 49.0%).

LC-MS (ESI): m/z 514.5 [M+H]⁺.

### E-139: 1-(2-((4-(1H-imidazo[4,5-c]pyridin-7-yl)-1H-1,2,3-triazol-1-yl)methyl)imidazo[1,2-a]pyridin-6-yl)-N-(cyclobutylmethyl)methylamine

A white solid compound **E-139** (12.9 mg, 25.0%) was prepared from intermediate **E139-2** using the method described in compound **E-1.**

LC-MS (ESI): m/z 414.1 [M+H]⁺ .¹H NMR (400 MHz, DMSO-*d*₆): *δ* 9.39 (br s, 1H), 9.13 (s, 2H), 8.95 (br s, 1H), 8.87 (br s, 2H), 8.71 (s, 1H), 8.21 (s, 1H), 7.65 (d, *J =* 9.4 Hz, 1H), 7.43 (dd, *J =* 1.4, 9.4 Hz, 1H), 5.95 (s, 2H), 4.16 (br s, 2H), 2.98 (d, *J =* 3.3 Hz, 2H), 2.60 (td, *J* = 7.5, 15.2 Hz, 1H), 2.11-2.00 (m, 2H), 1.91-1.71 (m, 4H).

### Example 140: Synthesis of E-140: 6-((4-(5-(2,5-dihydro-1H-pyrrol-1-yl)pyridin-3-yl)-1H-1,2,3-triazol-1-yl)methyl)-2-((4,4-dimethylpiperidin-1-yl)methyl)-1H-indole

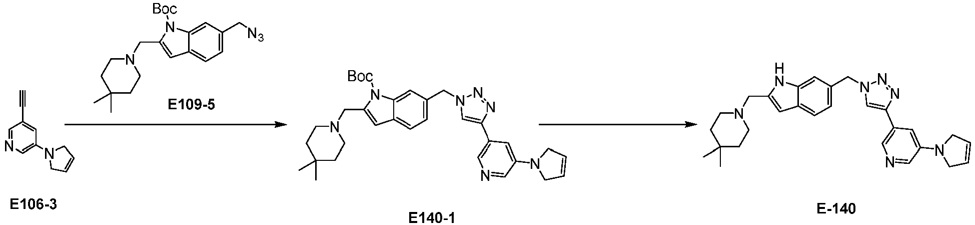

### E140-1: Tert-butyl 6-((4-(5-(2,5-dihydro-1H-pyrrol-1-yl)pyridin-3-yl)-1H-1,2,3-triazol-1-yl)methyl)-2-((4,4-dimethylpiperidin-1-yl)methyl)-1H-indole-1-carboxylate

A yellow solid intermediate **E140-1** (0.02 g, 24.1%) was prepared from intermediate **E106-3** using the method described in intermediate **4-d.**

LC-MS (ESI): m/z 568.4 [M+H]⁺.

### E-140: 6-((4-(5-(2,5-dihydro-1H-pyrrol-1-yl)pyridin-3-yl)-1H-1,2,3-triazol-1-yl)methyl)-2-((4,4-dimethylpiperidin-1-yl)methyl)-1H-indole

A white solid compound **E-140** (4.00 mg, 21.9%) was prepared from intermediate **E140-1** using the method described in compound **E-1.**

LC-MS (ESI): m/z 468.4 [M+H]⁺. ¹H NMR (400 MHz, DMSO-*d*₆): *δ* 11.06 (s, 1H), 8.68 (s, 1H), 8.34 (d, *J =* 1.6 Hz, 1H), 7.88 (d, *J =* 2.8 Hz, 1H), 7.44 (d, *J =* 8.1 Hz, 1H), 7.35-7.26 (m, 2H), 6.99 (dd, *J =* 1.4, 8.1 Hz, 1H), 6.25 (d, *J =* 1.1 Hz, 1H), 6.06 (s, 2H), 5.68 (s, 2H), 4.14 (s, 4H), 3.58 (s, 2H), 2.34 (dd, *J* = 1.6, 4.5 Hz, 4H), 1.32 (br s, 4H), 0.88 (s, 6H).

### Example 141: Synthesis of E-141: 2-((4-(5-(2,5-dihydro-1H-pyrrol-1-yl)pyridin-3-yl)-1H-1,2,3-triazol-1-yl)methyl)-6-((4,4-dimethylpiperidin-1-yl)methyl)imidazo[1,2-a]pyridine

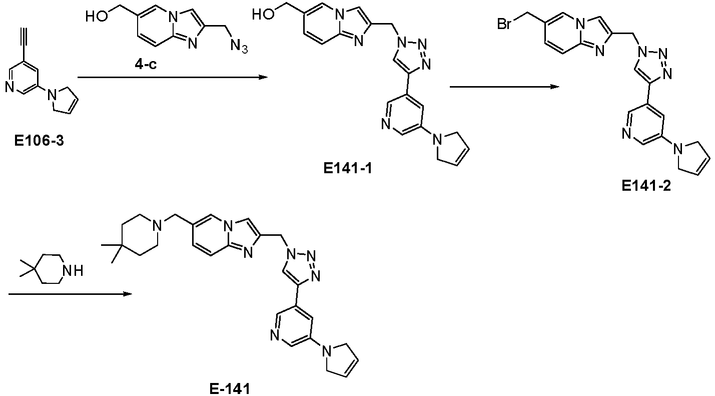

### E141-1: (2-((4-(5-(2,5-dihydro-1H-pyrrol-1-yl)pyridin-3-yl)-1H-1,2,3-triazol-1-yl)methyl)imidazo[1,2-a]pyridin-6-yl)methanol

A brown solid intermediate **E141-1** (0.13 g, crude product) was prepared from intermediate **E106-3** using the method described in intermediate **4-d.**

LC-MS (ESI): m/z 374.2 [M+H]⁺.

### E141-2: 6-(bromomethyl)-2-((4-(5-(2,5-dihydro-1H-pyrrol-1-yl)pyridin-3-yl)-1H-1,2,3-triazol-1-yl)methyl)imidazo[1,2-a]pyridine

PBr₃ (32.6 mg, 121 µmol, 0.50 eq) was added to a solution of intermediate **E141-**1 (90.0 mg, 241 µmol, 1.00 eq) in THF (0.90 mL) at 0°C. The resulting mixture was stirred at 25°C for 16 h. The mixture was concentrated, and the residue was purified by preparative thin-layer chromatography (DCM: MeOH=10:1) to obtain a white solid intermediate **E141-2** (80.0 mg, 76.1%).

LC-MS (ESI): m/z 437.9 [M+H]⁺.

### E-141: 2-((4-(5-(2,5-dihydro-1H-pyrrol-1-yl)pyridin-3-yl)-1H-1,2,3-triazol-1-yl)methyl)-6-((4,4-dimethylpiperidin-1-yl)methyl)imidazo[1,2-a]pyridine

A white solid compound **E-141** (1.90 mg, 2.21%) was prepared from intermediate **E141-2** using the method described in intermediate **E16-1.**

LC-MS (ESI): m/z 469.3 [M+H]⁺ . ¹H NMR (400 MHz, DMSO-*d*₆): *δ* 8.69 (s, 1H), 8.44 (s, 1H), 8.36 (s, 1H), 7.96 (s, 1H), 7.89 (d, *J =* 2.1 Hz, 1H), 7.46 (d, *J =* 9.3 Hz, 1H), 7.30 (br s, 1H), 7.21 (d, *J =* 9.4 Hz, 1H), 6.07 (s, 2H), 5.75 (s, 2H), 4.15 (s, 4H), 3.47-3.40 (m, 2H), 2.39-2.27 (m, 4H), 1.31 (t, *J =* 5.3 Hz, 4H), 0.89 (s, 6H).

### Example 143: Synthesis of E-143: 5-(1-((6-((((3-fluorobicyclo[1.1.1]pentan-1-yl)methyl)amino)methyl)imidazo[1,2-a]pyridin-2-yl)methyl)-1H-1,2,3-triazole-4-yl)-N,n-dimethylpyridin-3-amine

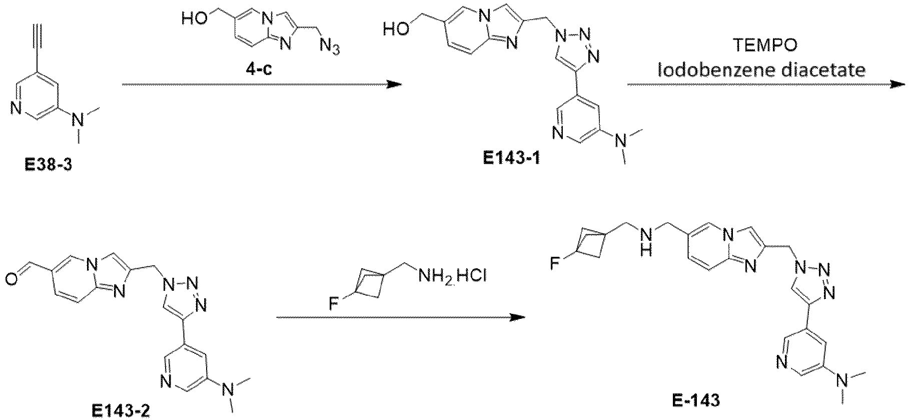

### E143-1: (2-((4-(5-(dimethylamino)pyridin-3-yl)-1H-1,2,3-triazol-1-yl)methyl)imidazo[1,2-a]pyridin-6-yl)methanol

A yellow solid intermediate **E143-1** (0.32 g, crude product) was prepared from intermediate **E38-3** using the method described in intermediate **4-d.**

LC-MS (ESI): m/z 350.1 [M+H]⁺.

### E143-2: 2-((4-(5-(dimethylamino)pyridin-3-yl)-1H-1,2,3-triazol-1-yl)methyl)imidazo[1,2-a]pyridine-6-formaldehyde

lodobenzene diacetate (111 mg, 343 µmol, 1.20 eq) and TEMPO (22.5 mg, 143 µmol, 0.50 eq) were added to a solution of intermediate **E143-1** (100 mg, 286 µmol, 1.00 eq) in DCM (2.00 mL), and the resulting mixture was stirred at 25°C for 3 h under nitrogen protection. The mixture was quenched with saturated sodium bicarbonate (5.00 mL), washed with water (5 mL), and concentrated to obtain a yellow solid intermediate **E143-2** (50 mg, crude product).

LC-MS (ESI): m/z 348.1 [M+H]⁺.

### E-143: 5-(1-((6-((((3-fluorobicyclo[1.1.1]pentan-1-yl)methyl)amino)methyl)imidazo[1,2-a]pyridin-2-yl)methyl)-1H-1,2,3-triazole-4-yl)-N,n-dimethylpyridin-3-amine

A yellow solid compound **E-143** (8.90 mg, 12.8%) was prepared from intermediate **E143-2** using the method described in intermediate **E6-1.**

LC-MS (ESI): m/z 447.3 [M+H]⁺ .¹H NMR (400 MHz, DMSO-*d*₆): *δ* 9.60-9.47 (m, 2H), 9.06 (s, 1H), 8.85 (s, 1H), 8.52 (s, 1H), 8.30 (s, 1H), 8.17 (d, *J =* 2.9 Hz, 1H), 8.06 (s, 1H), 7.73 (br s, 2H), 5.92 (s, 2H), 4.20 (d, *J =* 5.0 Hz, 2H), 3.29-3.25 (m, 2H), 3.11 (s, 6H), 2.12 (d, *J* = 2.5 Hz, 6H).

### Test Example 1

### Treatment of MOLM-13 cells with METTL3 inhibitor

MOLM-13 cell suspension was spread in a 96-well cell culture plate (tissue culture medium RPMI 1640+10% FBS), with a final concentration of 50000/mL and a volume of 100 µL per well. After overnight culture in a cell incubator, an METTL3 inhibitor was dissolved in DMSO, with an initial concentration of 50 mM, and subjected to triple gradient dilution in a 384-well storage plate. The inhibitor in the storage plate was pumped into a 96-well cell culture plate with an Echo instrument. The concentration of the compound ranged from 50 µM to 0.01 µM, and the volume was 100 nL/well. The final DMSO concentration was 0.1%, diluted by triple gradient, with 9 concentration gradients and diplopore. The cells were incubated in a cell incubator at 37°C for 72 h together with the compound. 100 µL/well of Cell Titer-Glo Luminescent Cell Viability Assay reagent was added and mixed well, and the obtained mixture was incubated at room temperature for 10 min and read with Envision. The sample treated with a 50 µM positive compound was used as positive control, and the sample treated with 100 nL DMSO was used as negative control. Calculation formula: (sample reading-positive control reading)/(negative control reading-positive control reading)*100%. IC₅₀ was calculated.

### HTRF experiment

The prokaryotic system expression plasmid pGEX-6P-1-YTHDC1 345-509AA (GST-YTHDC1 reader domain), abbreviated as YTHDC1, is the recognition protein of m6A. Mammalian expression system 293T cells co-transited plasmids pcDNA3.1-METTL3-3XFLAG (F119410) and pcDNA3.1-METTL14-3XFLAG (F102012), and co-expressed Flag-METTL3 and Flag-METTL14. After 48 hours, the cells were collected and the protein was purified. HTRF detection was divided into two parts, i.e., a reaction system and a detection system. First, the compound was pumped into a 384-well reaction plate using Echo, and then samples were added in two steps according to the reaction system and detection system. Reaction system: 10 µL of 100 nM FLAG-METTL3/14, 5 µL of 250 nM biotin-RNA, and 5 µL of 2.5 mM SAM. Reaction buffer: 20 mM Tris-HCL with pH of 7.5, 0.01% Triton X-100, 1 mM DTT, 0.02% BSA, and 0.01% Rnase inhibitor, which were in a total of 20 µL, mixed well and reacted at 37°C for 3 h. Detection system: 20 nM YTHDC1, 0.85 nM GST-Eu, and 20 µL of 31.3 nM SA-XL665. Detection buffer: 100 mM HEPES, 300 mM NaCl, and 200 mM KF with pH of 7.5, which were mixed well and reacted at room temperature for 1 h, reading with Envision. DMSO was used as a positive control instead of the compound, and samples treated with buffers instead of METTL3 and METTL14 were used as negative controls. Calculation formula: (sample reading-negative control reading)/(positive control reading-negative control reading)*100%. IC₅₀ was calculated.

The IC₅₀ values of some of the compounds provided by the present invention are shown in Table 1 below:

**Table 1**

| **Compound** | **METTL3_14 IC₅₀ (nM)** | **MOLM13 IC₅₀ (µM)** |
|---|---|---|
| E-1 | 0.75 | 0.13 |
| E-2 | 1.24 | 0.28 |
| E-3 | 3.24 | 4.73 |
| E-4 | 1.69 | 0.60 |
| E-5 | 1.38 | 0.30 |
| E-6 | 7.53 | 0.26 |
| E-7 | 3.70 | 1.33 |
| E-8 | 2.50 | 0.69 |
| E-9 | 0.90 | 0.49 |
| E-10 | 17.90 | 2.40 |
| E-11 | 10.30 | 2.60 |
| E-12 | 22.11 | 4.13 |
| E-13 | 43.80 | 20.64 |
| E-14 | 0.30 | 0.07 |
| E-15 | 6.32 | 1.87 |
| E-16 | 19.30 | >50 |
| E-17 | 45.50 | 0.92 |
| E-18 | 1.42 | 0.47 |
| E-19 | 45.90 | >50 |
| E-20 | 156.10 | >50 |
| E-21 | 24.58 | 7.95 |
| E-22 | 1.21 | 0.43 |
| E-23 | 0.69 | 0.17 |
| E-24 | 1.40 | 1.13 |
| E-25 | 20.18 | 3.98 |
| E-26 | 0.61 | 0.08 |
| E-27 | 0.80 | 0.11 |
| E-28 | 1.72 | 0.16 |
| E-29 | 1.71 | 0.25 |
| E-30 | 0.56 | 0.09 |
| E-31 | 2.20 | 0.39 |
| E-32 | 4.27 | 2.63 |
| E-33 | 1.17 | 0.53 |
| E-34 | 2.38 | 1.24 |
| E-35 | 1.79 | 0.85 |
| E-36 | 8.25 | 1.46 |
| E-37 | 68.95 | 16.93 |
| E-38 | 10.30 | 2.32 |
| E-39 | 0.75 | 0.13 |
| E-40 | 2.30 | 0.89 |
| E-41 | 2.50 | 1.02 |
| E-42 | 3.70 | 11.33 |
| E-43 | 1.40 | 0.71 |
| E-44 | 1.65 | 0.59 |
| E-45 | 1.37 | 1.70 |
| E-46 | 4.50 | 9.12 |
| E-47 | 1.23 | 0.55 |
| E-48 | 1.89 | 0.51 |
| E-49 | 0.37 | 0.17 |
| E-50 | 0.29 | 0.40 |
| E-51 | 0.25 | 0.23 |
| E-52 | 2.85 | 0.59 |
| E-53 | 2.25 | 0.71 |
| E-54 | 41.15 | 7.44 |
| E-55 | 0.40 | 0.19 |
| E-56 | 0.32 | 0.17 |
| E-57 | 0.10 | 0.03 |
| E-58 | 1.18 | 0.53 |
| E-59 | 0.59 | 0.23 |
| E-60 | 1.39 | 0.23 |
| E-61 | 1.64 | 0.85 |
| E-62 | 1248.94 | 65.30 |
| E-63 | 129.42 | 24.70 |
| E-64 | 1.06 | 0.77 |
| E-65 | 0.33 | 0.09 |
| E-66 | 24.26 | 4.45 |
| E-67 | 47.14 | 7.35 |
| E-68 | 25.45 | 3.87 |
| E-69 | 1.02 | 0.47 |
| E-70 | 34.95 | 7.41 |
| E-71 | 17.67 | 6.40 |
| E-72 | 157.60 | 2.74 |
| E-73 | 31.66 | 61.85 |
| E-74 | 3.83 | 3.24 |
| E-75 | 5.41 | 4.17 |
| E-76 | 13.23 | 6.46 |
| E-77 | 1.84 | 0.39 |
| E-78 | 4.13 | 4.09 |
| E-79 | 0.54 | 0.24 |
| E-80 | 6.61 | 2.99 |
| E-81 | 27.44 | 8.39 |
| E-82 | 6.31 | 0.89 |
| E-83 | 6.06 | 1.26 |
| E-84 | 0.24 | 0.46 |
| E-85 | 11.15 | 5.68 |
| E-86 | 0.16 | 0.07 |
| E-87 | 0.19 | 0.08 |
| E-88 | 0.40 | 0.27 |
| E-89 | 39.32 | 15.39 |
| E-90 | 3.83 | 1.92 |
| E-91 | 4.92 | 1.53 |
| E-92 | 12.11 | 3.15 |
| E-93 | 3.50 | 2.55 |
| E-94 | 9.01 | 1.72 |
| E-95 | 2.71 | 0.38 |
| E-96 | 12.24 | 10.28 |
| E-97 | 3.94 | 1.72 |
| E-98 | 3.99 | 1.86 |
| E-99 | 2.33 | 0.93 |
| E-100 | 0.12 | 0.49 |
| E-101 | 0.25 | 0.41 |
| E-102 | 11.23 | 12.73 |
| E-103 | 1.09 | 1.83 |
| E-104 | 0.41 | 0.42 |
| E-105 | 0.29 | 1.53 |
| E-106 | 0.48 | 1.15 |
| E-107 | 912.52 | 62.27 |
| E-108 | 841.64 | 3.35 |
| E-109 | 0.50 | 0.46 |
| E-110 | 712.47 | 17.46 |
| E-111 | 0.24 | 0.13 |
| E-112 | 1.25 | 1.01 |
| E-113 | 0.74 | 0.45 |
| E-114 | 0.31 | 0.48 |
| E-116 | 6641.68 | >10 |
| E-119 | 0.29 | 0.34 |
| E-120 | 8448.39 | 54.27 |
| E-121 | 0.20 | 0.13 |
| E-122 | 0.16 | 0.05 |
| E-123 | 1.68 | 0.94 |
| E-124 | 71.20 | 20.92 |
| E-125 | 5.78 | >50 |
| E-127 | 4.15 | 2.58 |
| E-129 | 19.56 | 84.81 |
| E-130 | 101.10 | 7.19 |
| E-131 | 3.66 | 0.80 |
| E-132 | 8.78 | 3.02 |
| E-133 | 20.77 | 8.85 |
| E-134 | 3.80 | 1.47 |
| E-136 | 358.96 | >50 |
| E-139 | 216.90 | 50.17 |
| E-140 | 0.17 | 0.31 |
| E-141 | 0.36 | 0.25 |
| E-143 | 0.77 | 0.35 |
| | | |

The present invention also tested the activity of the following compounds under the same conditions as described above, and the results are shown in Table 2:

**Table 2**

| Compound structure | METTL3/14 (nM) | MOLM 13 (µM) |
|---|---|---|
| 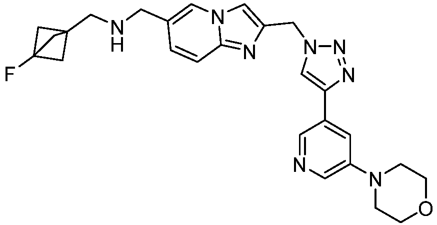 **TC006-RC019** | 7.36 | 3.13 |
| 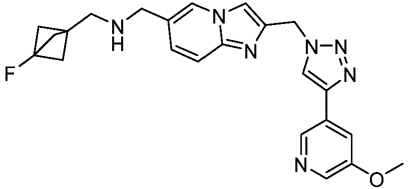 **TC006-RC020** | 5.8 | 1.82 |

From the above results, it can be seen that some of the compounds provided by the present invention have significantly higher activity as METTL3 inhibitors than the compounds listed in Table 2.

### 2.1 Preparation of intermediates corresponding to formula I' and formula I"

### Intermediate I-1: (2-(azidomethyl)imidazo[1,2-a]pyridin-6-yl)methanol

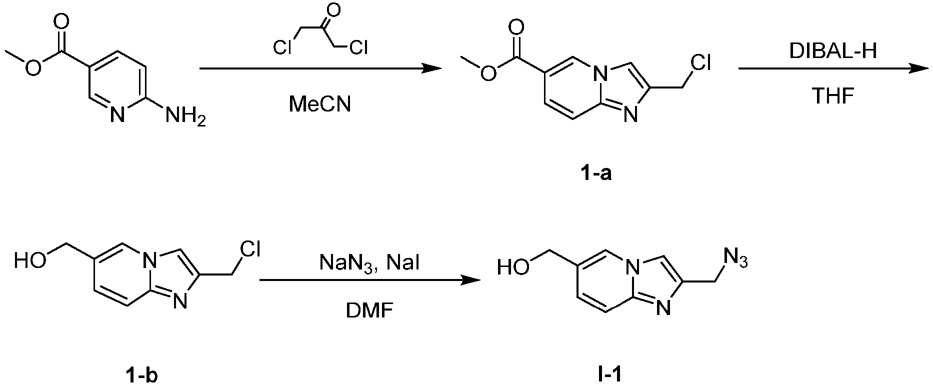

### Intermediate 1-a: Methyl 2-(chloromethyl)imidazo[1,2-a]pyridine-6-carboxylate

1,3-dichloropropan-2-one (147 g, 1.16 mol, 144 mL) was added to a solution of methyl 6-aminonicotinate (90.0 g, 591 mmol) in acetonitrile (1.47 L) at 25°C. The resulting mixture was stirred at 80°C for 16 h. After cooling to room temperature, the reaction was quenched with a sodium bicarbonate aqueous solution (900 mL). The mixture was extracted with dichloromethane (900 mL * 3), and the combined organic layers were washed with a saturated saline solution, dried over anhydrous sodium sulfate, filtered, and concentrated under reduced pressure to obtain a crude product. The crude product was purified by column chromatography (V_{petroleum ether/ethyl acetate}=20/1 to 2/1) to obtain yellow solid **1-a** (70.0 g, 52.7%). ¹H NMR (400 MHz, CDCl₃): *δ* 8.89 - 8.91 (m, 1 H) 7.76 - 7.81 (m, 1 H) 7.71 - 7.74 (m, 1 H) 7.59-7.63 (m, 1 H) 4.77 - 4.82 (m, 2 H) 3.98 - 4.00 (m, 3 H).

### Intermediate 1-b: (2-(chloromethyl)imidazo[1,2-a]pyridin-6-yl)methanol

Diisobutylaluminum hydride (1.00 M, 445 mL) was added to a solution of **1-a** (40.0 g, 178 mmol) in tetrahydrofuran (1.2 L) at 0°C under nitrogen protection. The resulting mixture was stirred at 0°C for 2 h under nitrogen protection. The reaction was quenched with methanol (2 L). The mixture was extracted with dichloromethane (4 L), and the combined organic layers were washed with a saturated saline solution, dried over anhydrous sodium sulfate, filtered, and concentrated under reduced pressure to obtain a crude product. The crude product was purified by column chromatography (V_{petroleum ether/ethyl acetate}=3/1) to obtain yellow solid **1-b** (10.0 g, 25.7%). **LC-MS (ESI):** m/z 197.6 [M+H]⁺.

### Intermediate I-1: (2-(azidomethyl)imidazo[1,2-a]pyridin-6-yl)methanol

Sodium iodide (609 mg, 4.07 mmol) and sodium azide (5.33 g, 82.0 mmol) were added to a solution of **1-b** (8.00 g, 40.7 mmol) in N,N-dimethylformamide (80 mL) at 25°C. The resulting mixture was stirred at 25°C for 3 h. The reaction solution was diluted with a sodium bicarbonate aqueous solution (250 mL) and extracted with ethyl acetate (80 mL * 6). The combined organic layers were washed with a saturated saline solution (400 mL), dried over anhydrous sodium sulfate, filtered, and concentrate under reduced pressure to obtain a yellow solid crude product **I-1** (6.40 g, 77.4%). ¹H NMR (400 MHz, DMSO-*d*₆) δ 8.41 - 8.47 (m, 1 H) 7.96 (s, 1 H) 7.50 (br d, J = 9.41 Hz, 1 H) 7.22 (br d, J = 9.17 Hz, 1 H) 5.36 (br s, 1 H) 4.48 (br s, 4 H).

### Intermediate I-2: 2-((4-(5-(pyrrolidin-1-yl)pyridin-3-yl)-1H-1,2,3-triazol-1-yl)methyl)imidazo[1,2-a]pyridine-6-formaldehyde

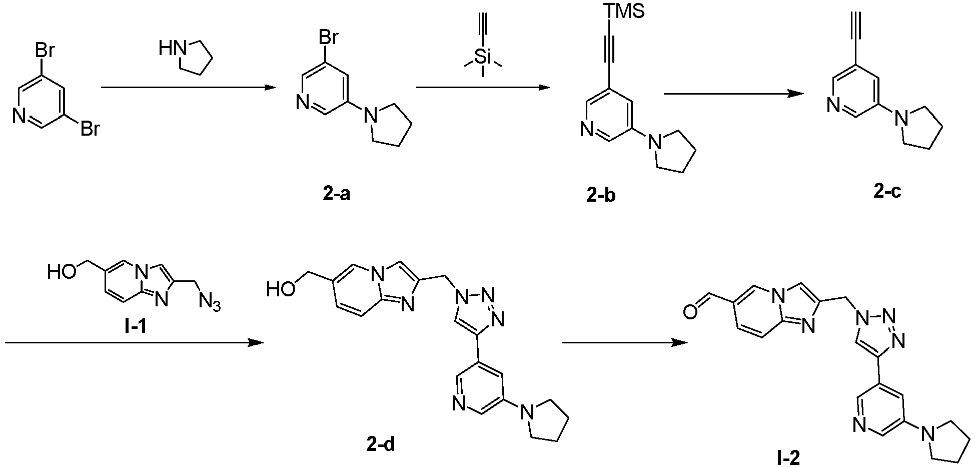

### Intermediate 2-a: 3-bromo-5-(pyrrolidin-1-yl)pyridine

Under nitrogen protection at 25°C, sodium tert-butoxide (97.4 g, 1.01 mol), 1,1'-dinaphthalene-2,2'-bis-diphenylphosphine (12.6 g, 20.3 mmol), and tris(dibenzylidene BASE acetone)dipalladium (0) (6.18 g, 6.75 mmol) were added to a toluene solution (800 mL) of 3,5-dibromopyridine (80.0 g, 338 mmol) and tetrahydropyrrole (25.2 g, 355 mmol, 29.6 mL). The resulting mixture was stirred at 100°C for 2 h under nitrogen protection. After cooling to room temperature, the mixture was extracted with ethyl acetate (300 mL * 4), and the combined organic layers were washed with a saturated saline solution, dried over anhydrous sodium sulfate, filtered, and concentrated under reduced pressure to obtain a crude product. The crude product was purified by column chromatography (V_{petroleum ether/ethyl acetate}=100/1 to 5/1) to obtain yellow solid **2-a** (47.0 g, 61.3%). ¹H NMR: (400 MHz, DMSO-*d₆*): *δ* 7.90 (d, *J =* 2.4 Hz, 1H), 7.85 (d, *J =* 1.6 Hz, 1H), 7.06 (t, *J =* 2.1 Hz, 1H), 3.25 (t, *J =* 6.6 Hz, 4H), 1.94 (td, *J =* 3.4, 6.4 Hz, 4H).

### Intermediate 2-b: 3-(pyrrolidin-1-yl)-5-((trimethylsilyl)ethynyl)pyridine

Under nitrogen protection at 25°C, copper iodide (4.91 g, 25.8 mmol) and Pd(dppf)Cl₂ (18.9 g, 25.8 mmol) were added to a triethylamine solution (586 mL) of **2-a** (58.6 g, 258 mmol) and ethynyl (trimethyl) silane (253 g, 2.58 mol, 357 mL). The resulting mixture was stirred at 80°C for 16 h under nitrogen protection. After cooling to room temperature, the mixture was extracted with ethyl acetate (1 L * 3), and the combined organic layers were washed with a saturated saline solution, dried over anhydrous sodium sulfate, filtered, and concentrated under reduced pressure to obtain a crude product. The crude product was purified by medium pressure preparative chromatography (V_{petroleum ether/ethyl acetate}=1:1) to obtain brown solid **2-b** (56.0 g, 88.80%). **¹H NMR:** (400 MHz, DMSO-*d₆*): *δ* 7.92 (d, *J* = 2.8 Hz, 1H), 7.85 (d, *J =* 1.4 Hz, 1H), 6.87 (d, *J =* 1.9 Hz, 1H), 3.25 (br s, 4H), 1.94 (br s, 4H), 0.29 - 0.15 (m, 9H).

### Intermediate 2-c: 3-ethynyl-5-(pyrrolidin-1-yl)pyridine

Potassium carbonate (63.3 g, 458 mmol) was added to a solution of **2-b** (56.0 g, 229 mmol) in N,N-dimethylformamide (560 mL) at 25°C. The resulting mixture was stirred at 25°C for 0.5 h under nitrogen protection. The mixture was extracted with ethyl acetate (500 mL * 4), and the combined organic layers were washed with a saturated saline solution, dried over anhydrous sodium sulfate, filtered, and concentrated under reduced pressure to obtain a crude product. The crude product was purified by medium pressure preparative chromatography (V_{petroleum ether/ethyl acetate}=100/1 to 1/1) to obtain brown solid **2-c** (27.6 g, 69.90%).

**¹H NMR:** (400 MHz, DMSO-*d₆*): *δ* 7.93 (d, *J* = 2.9 Hz, 1H), 7.88 (d, *J =* 1.3 Hz, 1H), 6.92 (br s, 1H), 4.28 (s, 1H), 3.25 (t, *J =* 6.4 Hz, 4H), 1.95 (td, *J =* 3.4, 6.3 Hz, 4H).

### Intermediate 2-d: (2-((4-(5-(pyrrolidin-1-yl)pyridin-3-yl)-1H-1,2,3-triazol-1-yl)methyl)imidazo[1,2-a]pyridin-6-yl)methanol

Sodium ascorbate (33.9 g, 171 mmol) and copper sulfate (4.97 g, 31.1 mmol, 4.78 mL) were added to a solution of **I-1** (36.0 g, 156 mmol) and **2-c** (26.8 g, 156 mmol) in N,N-dimethylformamide (268 mL) and water (78 mL) at 25°C. The resulting mixture was stirred at 25°C for 2 h under nitrogen protection. The reaction solution was diluted with water (1.4 L) and dichloromethane/methanol (3.00 L, v/v=10/1), and the combined organic layers were dried over anhydrous sodium sulfate, and then filtered and concentrated under reduced pressure to obtain a yellow solid crude product **2-d** (66.0 g). **LC-MS:** [M+H]⁺=376.3.

### Intermediate I-2: 2-((4-(5-(pyrrolidin-1-yl)pyridin-3-yl)-1H-1,2,3-triazol-1-yl)methyl)imidazo[1,2-a]pyridine-6-formaldehyde

lodobenzene diacetate (124 g, 384 mmol) and manganese dioxide (25.1 g, 160 mmol) were added to a solution of **2-d** (60.0 g, 160 mmol) in dichloromethane (0.6 L) at 25°C. The resulting mixture was stirred at 80°C for 2 h. After cooling to room temperature, the mixture was filtered and concentrated under reduced pressure to obtain a crude product. The crude product was purified by beating with ethyl acetate (1 L) to obtain brown solid **I-2** (43.5 g, 73.0%).

**¹H NMR:** (400 MHz, DMSO-*d₆*): *δ* 9.93 (s, 1H), 9.34 (s, 1H), 8.73-8.68 (m, 1H), 8.31 (s, 1H), 8.21 (s, 1H), 7.89 (d, *J =* 2.4 Hz, 1H), 7.65-7.58 (m, 2H), 7.29 (d, *J =* 1.6 Hz, 1H), 5.81 (s, 2H), 3.30 (br s, 4H), 1.97 (t, *J* = 6.5 Hz, 4H).

### Intermediate I-3: (3-fluoro-1-bicyclo[1.1.1]pentyl)methylamine

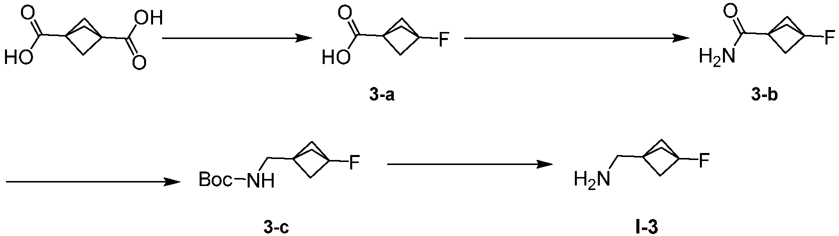

### Intermediate 3-a: 3-fluorobicyclo[1.1.1]pentane-1-carboxylic acid

Silver nitrate (2.18 g, 12.8 mmol) was added to an aqueous solution (400 mL) of bicyclo[1.1.1]pentane-1,3-dicarboxylic acid (20.0 g, 128 mmol) and a selective fluorine reagent (113 g, 320 mmol, 29.6 mL) at 25°C. The resulting mixture was stirred at 65°C for 16 h. The mixture was extracted with methyl tert-butyl ether (500 mL * 3), and the combined organic layers were washed with a saturated saline solution, dried over anhydrous sodium sulfate, filtered, and concentrated under reduced pressure to obtain a color-changed solid crude product **3-a** (40.0 g, 60.0%).

**¹H NMR:** (400 MHz, DMSO-*d₆*): *δ* 2.34 (d, J = 2.6 Hz, 6H).

### Intermediate 3-b: 3-fluorobicyclo[1.1.1]pentane-1-carboxamide

Oxalyl chloride (117 g, 922 mmol, 80.7 mL) was added dropwise to a solution of **3-a** (40.00 g, 307 mmol) and N,N-dimethylformamide (2.25 g, 30.7 mmol) in dichloromethane (400 mL) at 0°C. The resulting mixture was stirred at 0°C for 1 h. 450 mL of ammonia water was added dropwise at 0°C. The mixture was stirred at 25°C for 1 h. The reaction solution was concentrated to obtain a crude product. The crude product was purified by column chromatography (V_{petroleum ether/ethyl acetate}=100/1 to dichloromethane/methanol=1:1) to obtain white solid **3-b** (23.7 g, 60.6%).

**¹H NMR:** (400 MHz, DMSO-*d₆*): *δ* 7.41 (br s, 1H), 7.11 (br s, 1H), 2.23 (d, J=2.6 Hz, 6H).

### Intermediate 3-c: Tert-butyl N-[(3-fluoro-1-bicyclo[1.1.1]pentyl)methyl]carbamate

Lithium aluminum tetrahydroxide (16.9 g, 444 mmol) was added dropwise to a tetrahydrofuran solution (574 mL) of **3-b** (28.7 g, 222 mmol) at 25°C. The resulting mixture was stirred at 25°C for 2 h. Thin layer chromatography on silica gel plates (V_{dichloromethane/methanol}=10:1, Rf=0.03) showed that most of the raw materials had been consumed. Glauber's salt (114 g) was added at 0°C. The mixture was stirred at 25°C for 1 h. Di-tert-butyl dicarbonate ester (58.2 g, 266 mmol) was added at 25°C and stirred for 13 h. The resulting mixture was extracted with ethyl acetate (500 mL * 3), and the combined organic layers were washed with a saturated saline solution, dried over anhydrous sodium sulfate, filtered, and concentrate under reduced pressure to obtain a crude product. The crude product was purified by column chromatography (V_{petroleum ether/ethyl acetate}=100/1 to 0/1) to obtain white solid **3-c** (28.0 g, 58.5%). **¹H NMR:** (400 MHz, DMSO-*d₆*): *δ* 4.44 (br s, 1H), 3.33 (br s, 2H), 1.90 (d, *J =* 2.6 Hz, 6H), 1.37 (s, 9H).

### Intermediate I-3: (3-fluoro-1-bicyclo[1.1.1]pentyl)methylamine

Compound **3-c** (28 g, 130 mmol) was dissolved in hydrochloric acid/1,4-dioxane (140 mL, 4 N), and the solution was stirred at room temperature for 2 h and then concentrated under reduced pressure. The crude product was purified by preparative high-performance liquid chromatography to obtain a white solid compound **I-3** (17.5 g, 88.7%).

**¹H NMR:** (400 MHz, DMSO-*d₆*): *δ* 8.37-8.11 (m, 1H), 3.13 (s, 2H), 2.07 (d, *J* = 2.6 Hz, 6H).

### Intermediate I-4: Tert-butyl N-[[2-(azidomethyl)imidazo[1,2-a]pyridin-6-yl]methyl]-N-[(3-fluoro-1-bicyclo[1.1.1]pentyl)methyl]carbamate

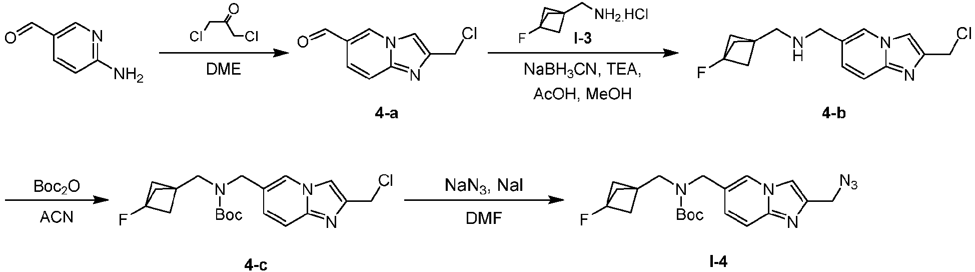

### Intermediate 4-a: 2-(chloromethyl)imidazo[1,2-a]pyridine-6-formaldehyde

A white solid compound **4-a** (2.5 g, 31.4%) was synthesized from a compound 6-aminopyridine-3-formaldehyde using the same method as compound **1-a.**

**¹H NMR:** (400 MHz, DMSO-*d₆*): *δ* 9.93 (s, 1H), 9.35-9.26 (m, 1H), 8.20 (s, 1H), 7.66-7.57 (m, 2H), 4.89 (s, 2H).

### Intermediate 4-b: 1-[2-(chloromethyl)imidazo[1,2-a]pyridin-6-yl]-N-[(3-fluoro-1-bicyclo[1.1.1]pentyl)methyl]methylamine

A yellow oily compound **4-b** (3.2 g, crude product) was synthesized from compound **4-a** using the same method as compound **E'-2.**

### Intermediate 4-c: Tert-butyl N-[[2-(chloromethyl)imidazole[1,2-a]pyridin-6-yl]methyl]-N-[(3-fluoro-1-bicyclo[1.1.1]pentyl)methyl]carbamate

Di-tert-butyl dicarbonate (2.52 g, 11.54 mmol) was added to an acetonitrile solution (45.2 mL) of **4-b** (2.26 g, 7.69 mmol) at 25°C. The resulting mixture was stirred at 25°C for 1 h. The mixture was extracted with ethyl acetate (10 mL * 5), and the combined organic layers were washed with a saturated saline solution, dried over anhydrous sodium sulfate, filtered, and concentrated under reduced pressure to obtain a crude product. The crude product was purified by column chromatography (V_{petroleum ether/ethyl acetate}=20/1 to 0/1) to obtain white solid **4-c** (560mg, **16.4%).¹H NMR:** (400 MHz, DMSO-*d₆*): *δ* 8.41 (br s, 1H), 7.99 (s, 1H), 7.51 (d, *J =* 9.3 Hz, 1H), 7.16 (d, *J =* 9.1 Hz, 1H), 4.83 (s, 2H), 4.32 (s, 2H), 3.57-3.42 (m, 2H), 1.91 (d, *J* = 2.6 Hz, 6H), 1.42 (br s, 9H).

### Intermediate I-4: Tert-butyl N-[[2-(azidomethyl)imidazo[1,2-a]pyridin-6-yl]methyl]-N-[(3-fluoro-1-bicyclo[1.1.1]pentyl)methyl]carbamate

A red oily compound **4-a** (360 mg, crude product) was synthesized from compound **4-c** using the same method as compound **I-1.**

**¹H NMR:** (400 MHz, DMSO-*d₆*): *δ* 8.44 (br s, 1H), 7.96 (d, *J* = 5.9 Hz, 2H), 7.54 (d, *J =* 9.3 Hz, 1H), 7.17 (dd, *J*= 1.6, 9.3 Hz, 1H), 4.49 (s, 2H), 4.33 (s, 2H), 3.57-3.44 (m, 2H), 1.92 (d, *J =* 2.6 Hz, 6H), 1.42 (br s, 9H).

### 2.2 Preparation process and activity testing of compounds corresponding to formula I' and formula I"

### Example 1 Compound E'-1: 1-(3-fluorobicyclo[1.1.1]pentan-1-yl)-N-((2-((1-(5-(pyrrolidin-1-yl)pyridin-3-yl)-1H-1,2,3-triazol-4-yl)methyl)imidazo[1,2-a]pyridin-6-yl)methyl)methylamine

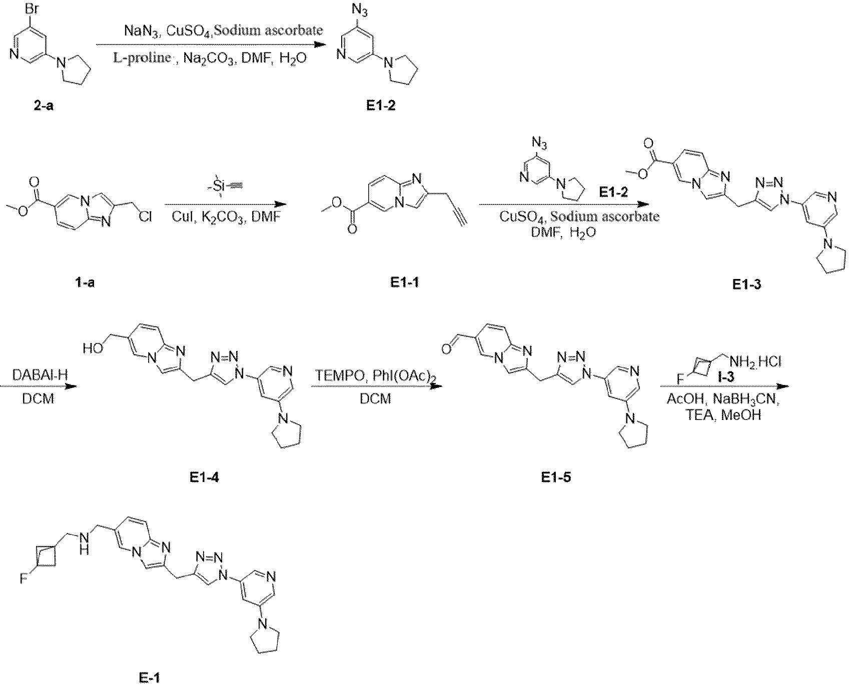

### Compound E1-1: Methyl 2-(2-alkynyl-1-propyl)imidazo[1,2-a]pyridine-6-carboxylate

Cuprous iodide (254 mg, 1.34 mmol) and potassium carbonate (5.54 g, 40.1 mmol) were added to a solution of **1-a** (3.00 g, 13.4 mmol) in N,N-dimethylformamide (30 mL) at 25°C. The resulting mixture was stirred at 25s°C for 15 min under nitrogen protection, and then acetylene(trimethyl)silane (13.1 g, 134 mmol, 18.5 mL) was added. The mixture was stirred at 70°C for 16 h under nitrogen protection. The reaction solution was diluted with water (150 mL) and extracted with dichloromethane (50 mL * 3). The combined organic layers were dried over with anhydrous sodium sulfate, filtered and concentrated under reduced pressure to obtain a crude product. The crude product was purified by column chromatography (V_{petroleum ether/ethyl acetate}=50/1 to 2/1) to obtain yellow solid **E1-1** (40.0 mg, 1.40%). **LC-MS:** [M+H]+=215.1.

### Compound E1-2: 3-azido-5-(pyrrolidin-1-yl)pyridine

Sodium ascorbate (872 mg, 4.40 mmol) and copper sulfate (281 mg, 1.76 mmol) were added to a solution of **2-a** (2.00 g, 8.81 mmol), L-proline (203 mg, 1.76 mmol), sodium carbonate (187 mg, 1.76 mmol), and sodium azide (859 mg, 13.2 mmol) in N,N-dimethylformamide (30 mL) and water (10 mL) at 25°C. The resulting mixture was stirred at 85°C for 16 h under nitrogen protection. After cooling to room temperature, the reaction solution was diluted with water (160 mL) and ammonia water (40 mL) and extracted with ethyl acetate (50 mL * 5). The combined organic layers were dried over anhydrous sodium sulfate, filtered, and concentrated under reduced pressure to obtain a brown liquid crude product **E1-2** (0.60 g).

### Compound E1-3: Methyl 2-((1-(5-(pyrrolidin-1-yl)pyridin-3-yl)-1H-1,2,3-triazol-4-yl)methyl)imidazo[1,2-a]pyridine-6-carboxylate

A brown solid compound **E1-3** (25.0 mg, 33.2%) was synthesized from compound **E1-2** using the same method as compound **2-d. LC-MS:** [M+H]⁺=404.1.

### Compound E1-4: (2-((1-(5-(pyrrolidin-1-yl)pyridin-3-yl)-1H-1,2,3-triazol-4-yl)methyl)imidazo[1,2-a]pyridin-6-yl)methanol

A brown solid compound **E1-4** (20.0 mg, crude product) was synthesized from diisobutylaluminum hydride using the same method as compound **1-b. LC-MS:** [M+H]⁺=376.1.

### Compound E1-5: 2-((1-(5-(pyrrolidin-1-yl)pyridin-3-yl)-1H-1,2,3-triazol-4-yl)methyl)imidazo[1,2-a]pyridine-6-formaldehyde

lodobenzene diacetate (41.2 mg, 128 µmol) and 2,2,6,6-tetramethylpiperidine oxide (8.38 mg, 53.3 µmol) were added to a solution of **E1-4** (20.0 mg, 53.3 µmol) in dichloromethane (0.6 mL) at 25°C. The resulting mixture was stirred at 25°C for 1 h under nitrogen protection. The reaction was quenched with a saturated sodium bicarbonate aqueous solution (3 mL) and extracted with dichloromethane/methanol (10.0 mL, v/v=10/1). The combined organic layers were dried over anhydrous sodium sulfate, filtered, and concentrated under reduced pressure to obtain a crude product. The crude product was purified by preparative thin-layer liquid chromatography (V_{dichloromethane/methanol}=10/1) to obtain yellow solid **E1-**5 (12 mg, 60.3%). **LC-MS:** [M+H]⁺=374.1.

### Compound E'-1: 1-(3-fluorobicyclo[1.1.1]pentan-1-yl)-N-((2-((1-(5-(pyrrolidin-1-yl)pyridin-3-yl)-1H-1,2,3-triazol-4-yl)methyl)imidazo[1,2-a]pyridin-6-yl)methyl)methylamine

Triethylamine (3.25 mg, 32.1 µmol, 1.47 µL) was added to a solution of **E1-5** (12.0 mg, 32.1 µmol) and **I-3** (4.87 mg, 32.1 µmol) in methanol (0.36 mL) at 25°C. The resulting mixture was stirred at 25°C for 5 min, and then acetic acid (3.86 mg, 64.3 µmol, 3.68 µL) was added. After stirring at 30°C for 30 min, sodium cyanoborohydride (4.04 mg, 64.3 µmol) was added. The mixture was stirred at 50°C for 1 h. After cooling at room temperature, the reaction was quenched with water (0.2 mL), and concentration was performed under reduced pressure to obtain a crude product. The crude product was purified by preparative high-performance liquid chromatography to obtain yellow solid **E'-1** (6.5 mg, 42.3%). **LC-MS:** [M+H]⁺=473.1. **¹H NMR:** (400 MHz, DMSO-*d₆*): *δ* 8.68 (s, 1H), 8.36-8.30 (m, 2H), 8.00 (d, *J* = 2.5 Hz, 1H), 7.71 (s, 1H), 7.41 (d, *J* = 9.1 Hz, 1H), 7.28 (t, *J =* 2.2 Hz, 1H), 7.19 (dd, *J =* 1.4, 9.3 Hz, 1H), 4.18 (s, 2H), 3.67 (s, 2H), 3.32-3.30 (m, 4H), 2.74 (s, 2H), 1.98 (t, *J =* 6.5 Hz, 4H), 1.93 (d, *J =* 2.8 Hz, 6H).

### Example 2 Compound E'-2: 1-(3-fluorobicyclo[1.1.1]pentan-1-yl)-N-((2-((1-(5-(pyrrolidin-1-yl)pyridin-3-yl)-1H-1,2,3-triazol-4-yl)methyl)imidazo[1,2-a]pyridin-6-yl)methyl)methylamine

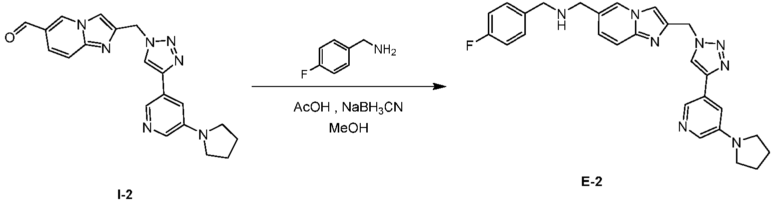

Acetic acid (16.1 mg, 268 µmol, 15.3 µL) was added to a solution of **I-2** (50.0 mg, 134 µmol) and (4-fluorophenyl)methylamine (20.1 mg, 161 µmol, 18.25 µL) in methanol (1.5 mL) at 25°C. The resulting mixture was stirred at 30°C for 0.5 h, and then sodium cyanoborohydride (16.8 mg, 268 µmol) was added. The mixture was stirred at 30°C for 1 h. After cooling at room temperature, the reaction was quenched with water (0.3 mL), and concentration was performed under reduced pressure to obtain a crude product. The crude product was purified by preparative high-performance liquid chromatography to obtain yellow solid **E'-2** (12.1 mg, 18.7%). **LC-MS:** [M+H]⁺=483.1. **¹H NMR:** (400 MHz, DMSO-*d₆*): *δ* 8.68 (s, 1H), 8.44 (s, 1H), 8.31 (d, *J* = 1.4 Hz, 1H), 7.96 (s, 1H), 7.88 (d, *J =* 2.8 Hz, 1H), 7.47 (d, *J =* 9.4 Hz, 1H), 7.38 (dd, *J= 5.8,* 8.4 Hz, 2H), 7.30-7.27 (m, 2H), 7.12 (t, *J =* 8.9 Hz, 2H), 5.73 (s, 2H), 3.65 (d, *J =* 4.3 Hz, 4H), 3.31-3.27 (m, 4H), 2.00-1.94 (m, 4H).

### Synthesis of compounds E'-2-1 to E'-2-13:

The compounds in Table 2 were prepared from intermediate I-2 and the appropriate amino intermediates using methods similar to compound E'-2.

**Table 2**

| **Comp ound** | **Name** | **Structure** | **LCMS [M+H]⁺** | **Source of raw material amino interme diate** |
|---|---|---|---|---|
| E'-2-1 | 3-(((2-((4-(5-(pyrrolidin-1-yl)pyridin-3-yl)-1H-1,2,3-triazol-1-yl)methyl)imidazo[1,2-a]pyridin-6-yl)methyl)amino)methyl)b icyclo[1.1.1]pentane-1-carbonitrile | 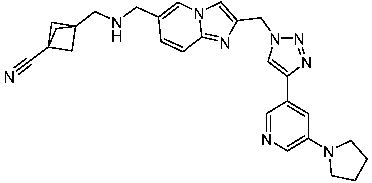 | 480.1 | Commer cially available |
| E'-2-2 | n-benzyl-1-(2-((4-(5-(pyrrolidin-1-yl)pyridin-3-yl)-1H-1,2,3-triazol-1-yl)methyl)imidazo[1,2-a]pyridin-6-yl)methylamine | 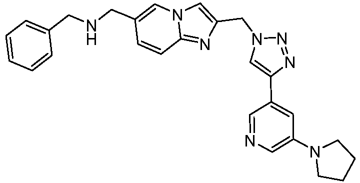 | 465.2 | Commer cially available |
| E'-2-3 | 2,2-dimethyl-N-((2-((4-(5-(pyrrolidin-1-yl)pyridin-3-yl)-1H-1,2,3-triazol-1-yl)methyl)imidazo[1,2-a]pyridin-6-yl)methyl)propan-1-amine | 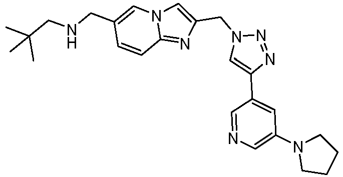 | 445.3 | Commer cially available |
| E'-2-4 | 1-[[2-[[4-(5-pyrrolidin-1-yl-3-pyridyl)triazol-1-yl]methyl]imidazo[1,2-a]pyridin-6-yl]methylamino]methyl]cy clobutanol | 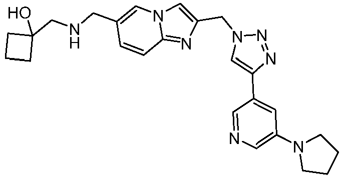 | 459.3 | Commer cially available |
| E'-2-5 | 6-(6-azaspiro[3.4]oct-6-yl methyl)-2-[[4-(5-pyrrolid-1-yl-3-pyridyl)triazol-1-yl]methyl]imidazo[1,2-a]pyridine | 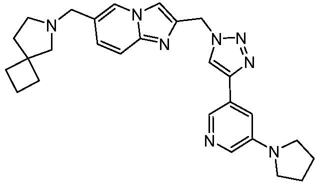 | 469.2 | Commer cially available |
| E'-2-6 | N-[(3,3-difluorocyclobutyl)methyl] -1-[2-[[4-(5-pyrrolidin-1-yl-3-pyridyl)triazol-1-yl]methyl]imidazo[1,2-a]pyridin-6-yl]methylamine | 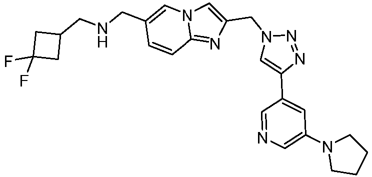 | 479.2 | Commer cially available |
| E'-2-7 | 1-[[2-[[4-(5-pyrrolidin-1-yl-3-pyridyl)triazol-1-yl]methyl]imidazo[1,2-a]pyridin-6-yl]methylamino]methyl]cy clohexanol | 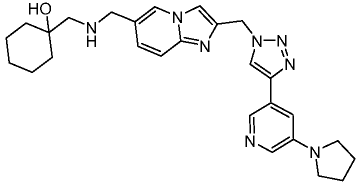 | 487.2 | Commer cially available |
| E'-2-8 | N-(cyclohexylmethyl)-1-[2-[[4-(5-pyrrolidin-1-yl-3-pyridyl)triazol-1-yl]methyl]imidazo[1,2-a]pyridin-6-yl]methylamine | 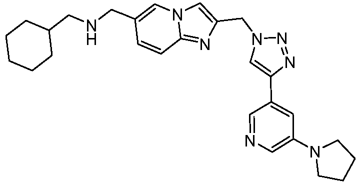 | 471.3 | Commer cially available |
| E'-2-9 | n-(4-chlorobenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 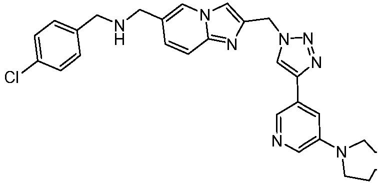 | 499.2 | Commer cially available |
| E'-2-10 | n-(4-fluoro-3-methylbenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 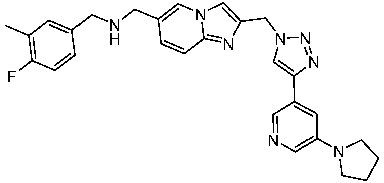 | 497.2 | Commer cially available |
| E'-2-11 | 1-(2-((4-(5-(pyrrolidin-1-yl)pyridin-3-yl)-1H-1,2,3-triazol-1-yl)methyl)imidazo[1,2-a]pyridin-6-yl)-N-(4-(trifluoromethyl)benzyl)m ethylamine | 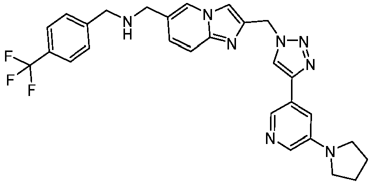 | 533.2 | Commer cially available |
| E'-2-12 | 4-(((2-((4-(5-(pyrrolidin-1-yl)pyridin-3-yl)-1H-1,2,3-triazol-1-yl)methyl)imidazo[1,2-a]pyridin-6-yl)methyl)amino)benzonit rile | 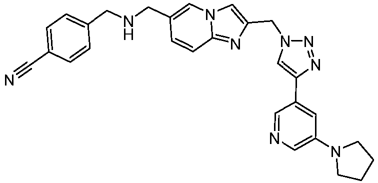 | 490.2 | Commer cially available |
| E'-2-13 | N-(3,4-difluorobenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 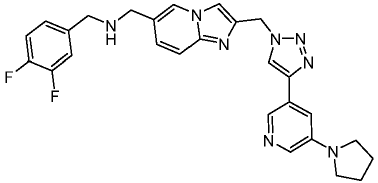 | 501.2 | Commer cially available |
| E'-2-14 | n-(4-fluoro-2-methylbenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 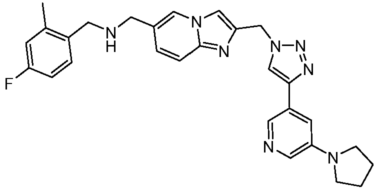 | 497.2 | Commer cially available |
| E'-2-15 | 1-(4-fluorophenyl)-N-((2-((4-(5-(pyrrolidin-1-yl)pyridin-3-yl)-1H-1,2,3-triazol-1-yl)methyl)imidazo[1,2-a]pyridin-6-yl)methyl)ethyl-1-amine | 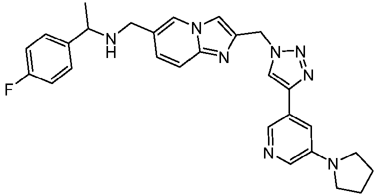 | 497.3 | Commer cially available |
| E'-2-16 | 1-(pyridin-4-yl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine | 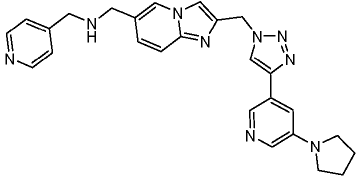 | 466.2 | Commer cially available |
| E'-2-17 | 2-(4-fluorophenyl)-N-((2-((4-(5-(pyrrolidin-1-yl)pyridin-3-yl)-1H-1,2,3-triazol-1-yl)methyl)imidazo[1,2-a]pyridin-6-yl)methyl)ethyl-1-amine | 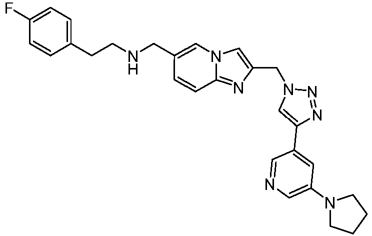 | 497.2 | Commer cially available |
| E'-2-18 | N-(2,4-difluorobenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 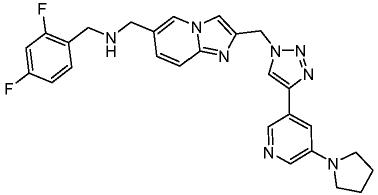 | 501.2 | Commer cially available |
| E'-2-19 | n-(2-fluorobenzyl)-1-(2-((4-(5-(pyrrolidin-1-yl)pyridin-3-yl)-1H-1,2,3-triazol-1-yl)methyl)imidazo[1,2-a]pyridin-6-yl)methylamine | 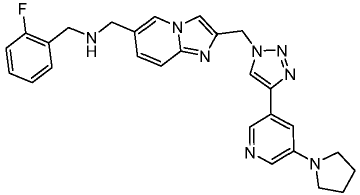 | 483.2 | Commer cially available |
| E'-2-20 | 6-((4,4-difluoropiperidin-1-yl)methyl)-2-((4-(5-(pyrrolidin-1-yl)pyridin-3-yl)-1H-1,2,3-triazol-1-yl)methyl)imidazo[1,2-a]pyridine | 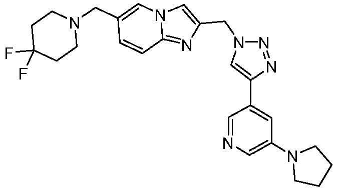 | 479.2 | Commer cially available |
| E'-2-21 | 2-[[2-[[4-(5-pyrrolidin-1-yl-3-pyridyl)triazol-1-yl]methyl]imidazo[1,2-a]pyridin-6-yl]methylamino]acetonitril e | 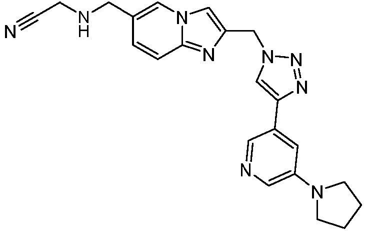 | 414.2 | Commer cially available |
| E'-2-22 | n-[[2-[[4-(5-pyrrolidin-1-yl-3-pyridyl)triazol-1-yl]methyl]imidazo[1,2-a]pyridin-6-yl]methyl]propyl-2-alkyne-1-amine | 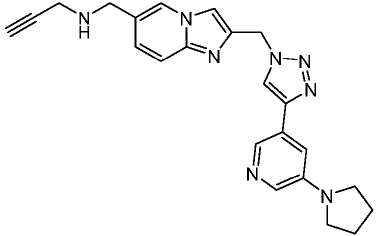 | 413.2 | Commer cially available |
| E'-2-23 | n-[(1-fluorocyclopropyl)methyl] -1-[2-[[4-(5-pyrrolid-1-yl-3-pyridyl)triazol-1-yl]methyl]imidazo[1,2-a]pyridin-6-yl]methylamine | 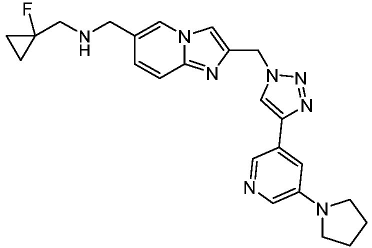 | 447.2 | Commer cially available |
| E'-2-24 | 1-(1-methylcyclopropyl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine | 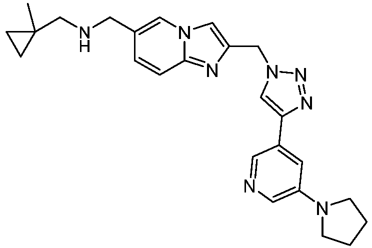 | 443.4 | Commer cially available |
| E'-2-25 | N-(cyclopropylmethyl)-1-[2-[[4-(5-pyrrolidin-1-yl-3-pyridyl)triazol-1-yl]methyl]imidazo[1,2-a]pyridin-6-yl]methylamine | | 429.2 | Commer cially available |
| E'-2-26 | n-[[2-[[4-(5-pyrrolidin-1-yl-3-pyridyl)triazol-1-yl]methyl]imidazo[1,2-a]pyridin-6-yl]methyl]butyl-2-alkyne-1-amine | | 427.3 | Commer cially available |

### Example 3 Compound E'-3: 1-(5-(1-((6-(((3-fluorobicyclo[1.1.1]pentan-1-yl)methyl)amino)methyl)imidazo[1,2-a]pyridin-2-yl)methyl)-1H-1,2,3-triazol-4-yl)pyridin-3-yl)pyrrolidin-2-one

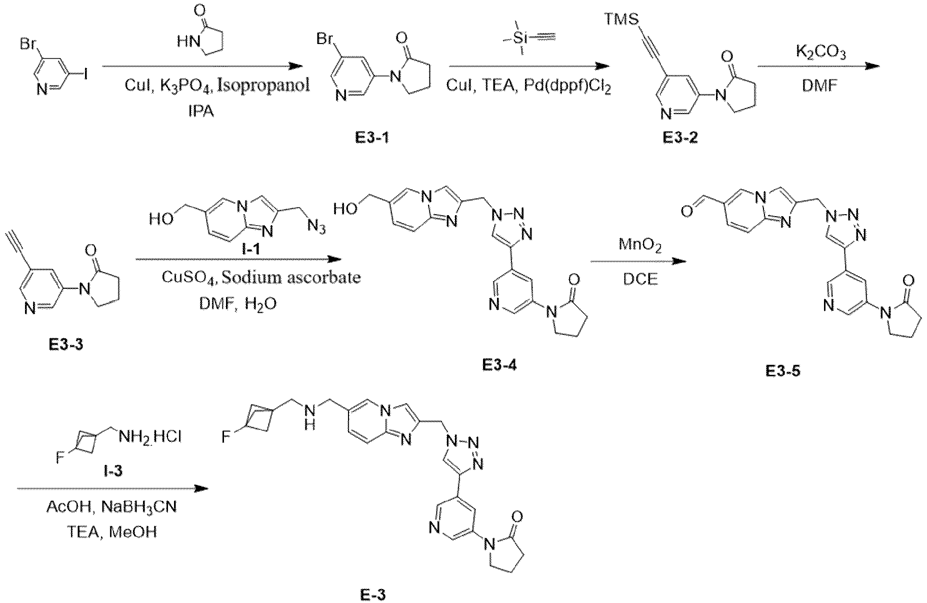

### Compound E3-1: 1-(5-bromopyridin-3-yl)pyrrolidin-2-one

Cuprous iodide (67.1 mg, 352 µmol), ethylene glycol (43.7 mg, 704 µmol), and potassium phosphonate (5.54 g, 40.1 mmol) were added to a solution of 3-bromo-5-iodopyridine (1.00 g, 3.52 mmol) and pyrrolidin-2-one (899 mg, 10.6 mmol) in isopropanol (10 mL) at 25°C. The resulting mixture was stirred at 110°C for 12 h under nitrogen protection. The reaction solution was diluted with water (30 mL) and extracted with ethyl acetate (10 mL * 3). The combined organic layers were dried over anhydrous sodium sulfate, filtered and concentrated under reduced pressure to obtain a crude product. The crude product was purified by column chromatography (V_{petroleum ether/ethyl acetate}=100/1 to 2/1) to obtain yellow solid **E3-1** (500.0 mg, 58.9%). **LC-MS:** [M+H]+=241.0.

### Compound E3-2: 1-(5-((trimethylsilyl)ethynyl)pyridin-3-yl)pyrrolidin-2-one

A brown solid compound **E3-2** (90.0 mg, 22.7%) was synthesized from **E3-1** using the same method as compound **2-b. LC-MS:** [M+H]⁺=259.1.

### Compound E3-3: 1-(5-ethynylpyridin-3-yl)pyrrolidin-2-one

A yellow solid compound **E3-3** (60.0 mg, crude product) was synthesized from **E3-2** using the same method as compound **2-c. LC-MS:** [M+H]⁺=187.2.

### Compound E3-4: 1-(5-(1-((6-(hydroxymethyl)imidazo[1,2-a]pyridin-2-yl)methyl)-1H-1,2,3-triazol-4-yl)pyridin-3-yl)pyrrolidin-2-one

A yellow solid compound **E3-4** (150.0 mg, crude product) was synthesized from **E3-3** using the same method as compound **2-d. LC-MS:** [M+H]⁺=390.1.

### Compound E3-5: 2-((4-(5-(2-oxopyrrolidin-1-yl)pyridin-3-yl)-1H-1,2,3-triazol-1-yl)methyl)imidazo[1,2-a]pyridine-6-formaldehyde

A brown solid compound **E3-5** (20.0 mg, 26.8%) was synthesized from **E3-4** using the same method as compound **I-2. LC-MS:** [M+H]⁺=388.2.

### Compound E'-3: 1-(5-(1-((6-(((3-fluorobicyclo[1.1.1]pentan-1-yl)methyl)amino)methyl)imidazo[1,2-a]pyridin-2-yl)methyl)-1H-1,2,3-triazol-4-yl)pyridin-3-yl)pyrrolidin-2-one

A yellow oily compound **E'-3** (6.6 mg, 26.3%) was synthesized from **E3-5** by preparative high-performance liquid chromatography purification using the same method as compound **E'-2. LC-MS:** [M+H]⁺=487.2. **¹H NMR:** (400 MHz, DMSO-*d₆*): *δ* 8.82 (d, *J =* 13.1 Hz, 2H), 8.75 (s, 1H), 8.52 (s, 1H), 8.42 (s, 1H), 7.97 (s, 1H), 7.47 (d, *J =* 9.3 Hz, 1H), 7.26 (d, *J =* 9.3 Hz, 1H), 5.76 (s, 2H), 3.92 (t, *J =* 7.0 Hz, 2H), 3.68 (br s, 2H), 2.75 (br s, 2H), 2.56-2.52 (m, 2H), 2.15-2.07 (m, 2H), 1.93 (d, *J* = 2.5 Hz, 6H).

### Example 4 Compound E'-4: 1-[2-[[4-[5-[(3R,4R)-3,4-difluoropyrrolidin-1-yl]-3-pyridyl]triazol-1-yl]methyl]imidazo[1,2-a]pyridin-6-yl]-N-[(3-fluoro-1-bicyclo[1.1.1]pentyl)methyl]methylamine

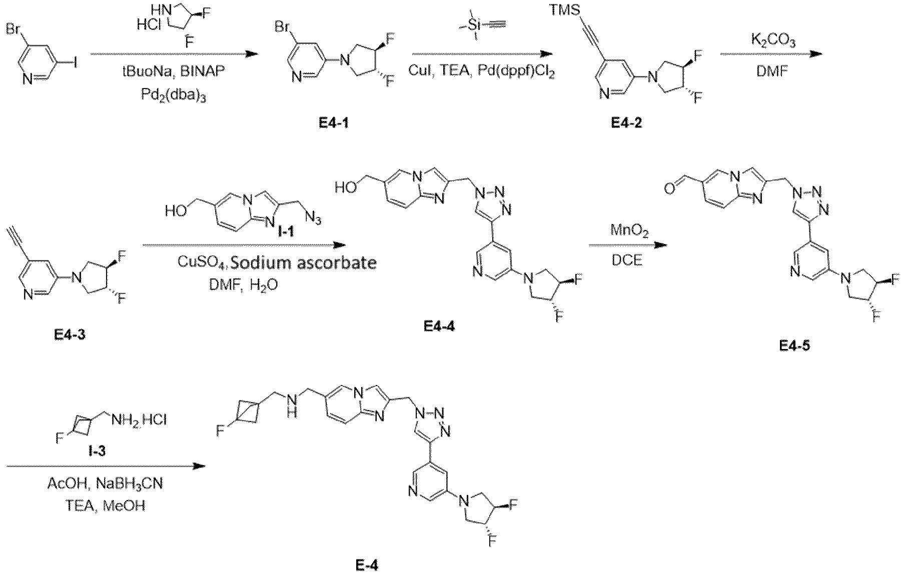

### Compound E4-1: 3-bromo-5-[(3R,4R)-3,4-difluoropyrrolidin-1-yl]pyridine

A yellow solid compound **E4-1** (150.0 mg, 32.4%) was synthesized from 3-bromo-5-iodo-pyridine using the same method as compound **2-a. LC-MS:** [M+H]⁺=265.0.

### Compound E4-2: 2-[5-[(3R,4R)-3,4-difluoropyrrolidin-1-yl]-3-pyridyl]ethynyl-trimethylsilane

A brown solid compound **E4-2** (100.0 mg, 72.7%) was synthesized from **E4-1** using the same method as compound **2-b. LC-MS:** [M+H]⁺= 281.1.

### Compound E4-3: 3-[(3R,4R)-3,4-difluoropyrrolidin-1-yl]-5-ethynyl-pyridine

A yellow solid compound **E4-3** (74.0 mg, crude product) was synthesized from **E4-2** using the same method as compound **2-c.**

### Compound E4-4: (2-((4-(5-((3R,4R)-3,4-difluoropyrrolidin-1-yl)pyridin-3-yl)-1H-1,2,3-triazol-1-yl)methyl)imidazo[1,2-a]pyridin-6-yl)methanol

A yellow solid compound **E4-4** (140.0 mg, crude product) was synthesized from **E4-3** using the same method as compound **2-d. LC-MS:** [M+H]⁺=412.4.

### Compound E4-5: 2-[[4-[5-[(3R,4R)-3,4-difluoropyrrolidin-1-yl]-3-pyridyl]triazol-1-yl]methyl]imidazo[1,2-a]pyridine-6-formaldehyde

A yellow solid compound **E4-5** (60.0 mg, 46.4%) was synthesized from **E4-4** using the same method as compound **I-2. LC-MS:** [M+H]⁺=410.1.

### Compound E'-4: 1-[2-[[4-[5-[(3R,4R)-3,4-difluoropyrrolidin-1-yl]-3-pyridyl]triazol-1-yl]methyl]imidazo[1,2-a]pyridin-6-yl]-N-[(3-fluoro-1-bicyclo[1.1.1]pentyl)methyl]methylamine

A yellow oily compound **E'-4** (22.9 mg, 36.9%) was synthesized from **E4-5** by preparative high-performance liquid chromatography purification using the same method as compound **E'-2. LC-MS:** [M+H]⁺= 509.2. **¹H NMR:** (400 MHz, DMSO-*d₆*): *δ* 8.69 (s, 1H), 8.42 (d, *J =* 1.3 Hz, 2H), 8.00-7.94 (m, 2H), 7.46 (d, *J =* 9.3 Hz, 1H), 7.41-7.39 (m, 1H), 7.26 (dd, *J =* 1.4, 9.3 Hz, 1H), 5.74 (s, 2H), 5.61-5.53 (m, 1H), 5.49-5.44 (m, 1H), 3.81-3.72 (m, 2H), 3.67 (s, 4H), 2.74 (s, 2H), 1.93 (d, *J =* 2.6 Hz, 6H).

### Synthesis of Compounds E'-4-1 to E'-4-3

The compounds in Table 3 were synthesized and characterized in the same manner as compound E'-4.

**Table 3**

| **Compo und** | **Name** | **Structure** | **LCMS [M+H]⁺** |
|---|---|---|---|
| E'-4-1 | 1-[2-[[4-[5-[(3S,4R)-3,4-difluoropyrrolidin-1-yl]-3-pyridyl]triazol-1-yl]methyl]imidazo[1,2-a]pyridin-6-yl]-N-[(3-fluoro-1-bicyclo[1.1.1]pentyl)methyl]met hylamine | 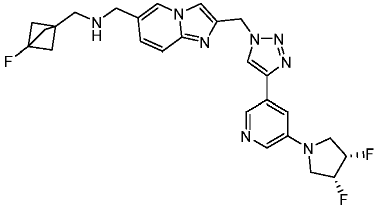 | 509.1 |
| E'-4-2 | 1-[2-[[4-[5-(7-azabicyclo[2.2.1]hept-7-yl)-3-pyridyl]triazol-1-yl]methyl]imidazo[1,2-a]pyridin-6-yl]-N-[(3-fluoro-1-bicyclo[1.1.1]pentyl)methyl]met hylamine | 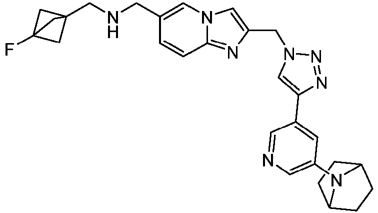 | 499.4 |
| E'-4-3 | n-[(3-fluoro-1-bicyclo[1.1.1]pentyl)methyl]-1-[2-[[4-(5-isoxazolidine-2-yl-3-pyridyl)triazol-1-yl]methyl]imidazo[1,2-a]pyridin-6-yl]methylamine | 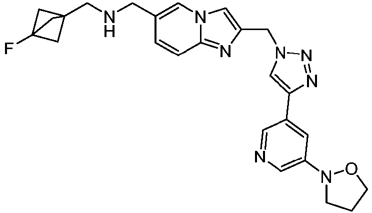 | 475.2 |
| E'-4-4 | 1-(2-((4-(5-(6-azaspiro[3.4]oct-6-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 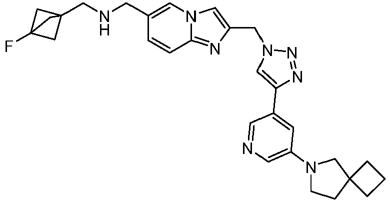 | 513.2 |
| E'-4-5 | 1-[2-[[4-[5-(6,6-difluoro-3-azabicyclo[3.1.0]hex-3-yl)-3-pyridyl]triazol-1-yl]methyl]imidazo[1,2-a]pyridin-6-yl]-N-[(3-fluoro-1-bicyclo[1.1.1]pentyl)methyl]met hylamine | 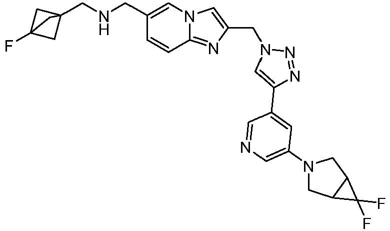 | 521.3 |
| E'-4-6 | 1-[2-[[4-[5-(5-azaspiro[2.4]hept-5-yl)-3-pyridyl]triazol-1-yl]methyl]imidazo[1,2-a]pyridin-6-yl]-N-[(3-fluoro-1-bicyclo[1.1.1]pentyl)methyl]met hylamine | 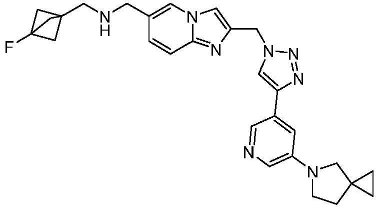 | 499.2 |
| E'-4-7 | 1-[5-[1-[[6-[[(3-fluoro-1-bicyclo[1.1.1]pentyl)methylamin o]methyl]imidazo[1,2-a]pyridin-2-yl]methyl]triazol-4-yl]-3-pyridyl]pyrrolidine-3-ol | 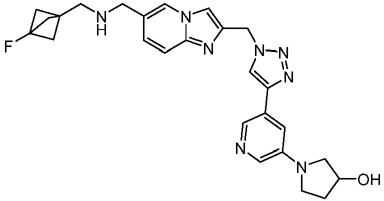 | 489.4 |
| E'-4-8 | 1-(2-((4-(5-(2-oxo-6-azaspiro[3.4]oct-6-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 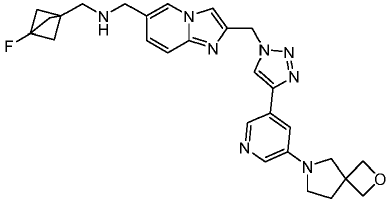 | 515.3 |
| E'-4-9 | 1-(3-fluorobicyclo[1.1.1]pentan-1-yl)-N-((2-((4-(5-(3-(trifluoromethyl)pyrrolidin-1-yl)pyridin-3-yl)-1H-1,2,3-triazol-1-yl)methyl)imidazo[1,2-a]pyridin-6-yl)methyl)methylamine | 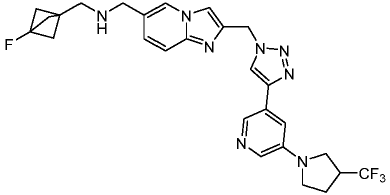 | 541.2 |
| E'-4-10 | 1-(3-fluorobicyclo[1.1.1]pentan-1-yl)-N-((2-((4-(5-(hexahydrocyclopentane [c]pyrrole-2(1H)-yl)pyridin-3-yl)-1H-1,2,3-triazole-1-yl)methyl)imidazo[1,2-a]pyridin-6-yl)methyl)methylamine | 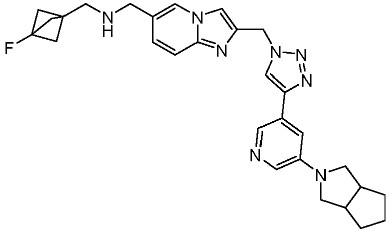 | 513.2 |
| E'-4-11 | 1-(2-((4-(5-(2-azabicyclo[3.1.0]hex-2-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 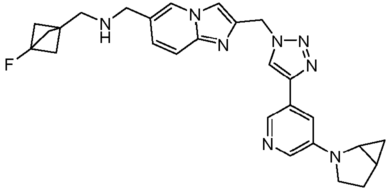 | 485.3 |
| E'-4-12 | 1-(3-fluorobicyclo[1.1.1]pentan-1-yl)-N-((2-((4-(5-(3,3,4,4-tetrafluoropyrrolidin-1-yl)pyridin-3-yl)-1H-1,2,3-triazol-1-yl)methyl)imidazo[1,2-a]pyridin-6-yl)methyl)methylamine | 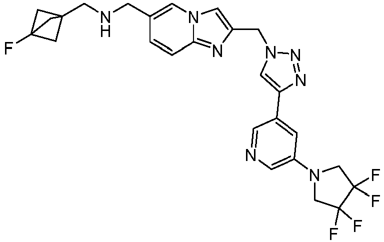 | 545.3 |

### Example 5 Compound E'-5: n-[(3-fluoro-1-bicyclo[1.1.1]pentyl)methyl]-1-[2-[[4-(5-pyrazol-1-yl-3-pyridyl)triazol-1-yl]methyl]imidazo[1,2-a]pyridin-6-yl]methylamine

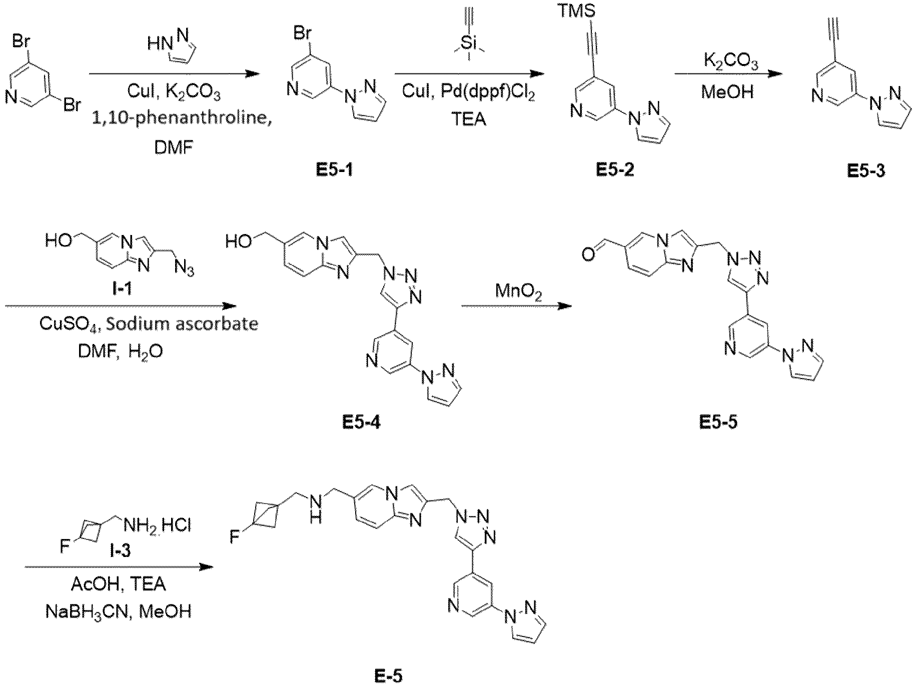

### Compound E5-1: 3-bromo-5-pyrazol-1-yl-pyridine

Cuprous iodide (161 mg, 844 µmol), 1,10-phenanthroline (304 mg, 1.6 mmol), and potassium carbonate (2.92 g, 21.1 mmol) were added to a solution of 3,5-dibromopyridine (2.00 g, 8.44 mmol) and 1H-pyrazole (632 mg, 9.29 mmol) in N,N-dimethylformamide (40 mL) at 25°C. The resulting mixture was stirred at 120°C for 16 h under nitrogen protection. The reaction solution was diluted with water (200 mL) and extracted with dichloromethane (50 mL * 3). The combined organic layers were dried over anhydrous sodium sulfate, filtered and concentrated under reduced pressure to obtain a crude product. The crude product was purified by column chromatography (V_{petroleum ether/ethyl acetate}=50/1 to 10/1) to obtain yellow solid **E5-1** (500.0 mg, 26.4%). **LC-MS:** [M+H]⁺=224.0.

### Compound E5-2: Trimethyl-[2-(5-pyrazol-1-yl-3-pyridyl)ethynyl]silane

A yellow solid compound **E5-2** (450.0 mg, 83.6%) was synthesized from **E5-1** using the same method as compound **2-b. LC-MS:** [M+H]⁺=242.1.

### Compound E5-3: 3-ethynyl-5-pyrazol-1-yl-pyridine

A yellow solid compound **E5-3** (230.0 mg, 92.3%) was synthesized from **E5-2** using the same method as compound **2-c. LC-MS:** [M+H]⁺= 170.1.

### Compound E5-4: (2-((4-(5-(1H-pyrazol-1-yl)pyridin-3-yl)-1H-1,2,3-triazol-1-yl)methyl)imidazo[1,2-a]pyridin-6-yl)methanol

A yellow solid compound **E5-4** (200.0 mg, crude product) was synthesized from **E5-3** using the same method as compound **2-d. LC-MS:** [M+H]⁺=372.4.

### Compound E5-5: 2-((4-(5-(1H-pyrazol-1-yl)pyridin-3-yl)-1H-1,2,3-triazol-1-yl)methyl)imidazo[1,2-a]pyridine-6-formaldehyde

A yellow solid compound **E5-5** (60.0 mg, 46.4%) was synthesized from **E5-4** using the same method as compound **I-2. LC-MS:** [M+H]⁺=370.4.

### Compound E'-5: n-[(3-fluoro-1-bicyclo[1.1.1]pentyl)methyl]-1-[2-[[4-(5-pyrazol-1-yl-3-pyridyl)triazol-1-yl]methyl]imidazo[1,2-a]pyridin-6-yl]methylamine

A yellow solid compound **E'-5** (3.50 mg, 9.09%) was synthesized from **E5-5** by preparative high-performance liquid chromatography purification using the same method as compound **E'-2. LC-MS:** [M+H]⁺= 470.2. **¹H NMR:** (400 MHz, DMSO-*d₆*): *δ* 9.07 (d, *J =* 2.4 Hz, 1H), 9.01 (d, *J =* 1.6 Hz, 1H), 8.84 (s, 1H), 8.69 (d, *J =* 2.4 Hz, 1H), 8.66 (t, *J =* 2.1 Hz, 1H), 8.43 (s, 1H), 7.99 (s, 1H), 7.85 (d, *J* = 1.4 Hz, 1H), 7.47 (d, *J =* 9.3 Hz, 1H), 7.26 (dd, *J =* 1.5, 9.3 Hz, 1H), 6.63 (dd, *J =* 1.8, 2.4 Hz, 1H), 5.78 (s, 2H), 3.68 (s, 2H), 2.74 (s, 2H), 1.93 (d, *J =* 2.8 Hz, 6H).

### Synthesis of Compounds E'-5-1

The compound in Table 4 was synthesized and characterized in the same manner as compound E'-5.

**Table 4**

| **Compo und** | **Name** | **Structure** | **LCMS [M+H]⁺** |
|---|---|---|---|
| E'-5-1 | n-[(3-fluoro-1-bicyclo[1.1.1]pentyl)methyl]-1-[2-[[4-[5-(1,2,4-triazol-1-yl)-3-pyridyl]triazol-1-yl]methyl]imidazo[1,2-a]pyridin-6-yl]methylamine | 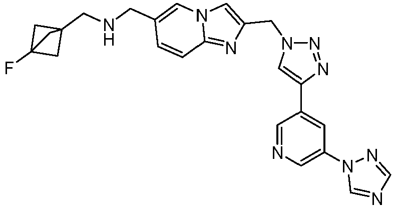 | 471.2 |

### Example 6 Compound E'-6: n-[(3-fluoro-1-bicyclo[1.1.1]pentyl)methyl]-1-[2-[[4-[5-(3-methylpyrrolidin-1-yl)-3-pyridyl]triazol-1-yl]methyl]imidazo[1,2-a]pyridin-6-yl]methylamine

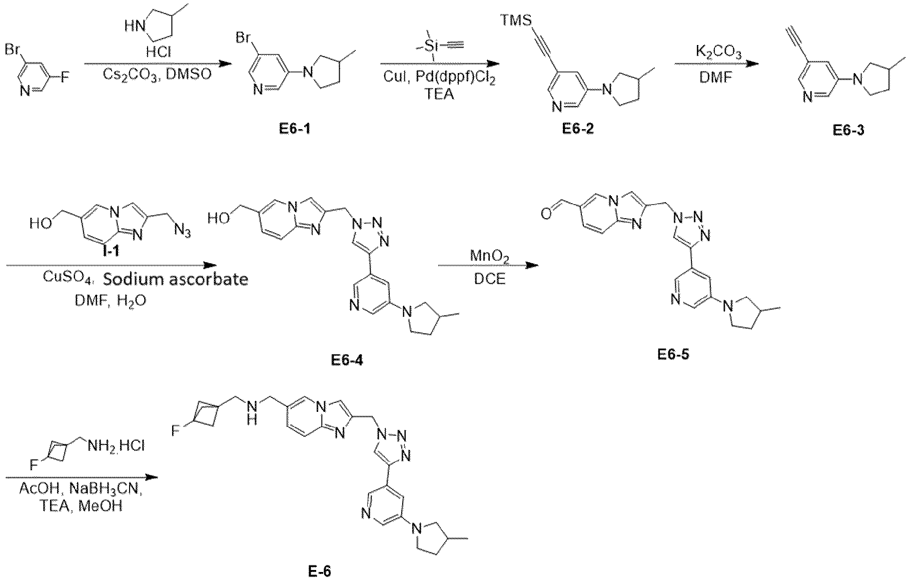

### Compound E6-1: 3-bromo-5-(3-methylpyrrolidin-1-yl)pyridine

Cesium carbonate (2.78 g, 8.52 mmol) was added to a solution of 3-bromo-5-fluoro-pyridine (0.5 g, 2.84 mmol) and 3-methylpyrrolidine (346 mg, 2.84 mmol) in dimethyl sulfoxide (10 mL) at 25°C. The resulting mixture was stirred at 120°C for 16 h. The reaction solution was diluted with water (50 mL) and extracted with ethyl acetate (20 mL * 3). The combined organic layers were dried with anhydrous sodium sulfate, filtered and concentrated under reduced pressure to obtain a crude product. The crude product was purified by column chromatography (petroleum ether/ethyl acetate=100/1 to 2/1) to obtain yellow solid **E6-1** (580.0 mg, 84.7%). **LC-MS:** [M+H]⁺= 241.2.

### Compound E6-2: Trimethyl-[2-[5-(3-methylpyrrolidin-1-yl)-3-pyridyl]ethynyl]silane

A yellow solid compound **E6-2** (240.0 mg, 74.3%) was synthesized from **E6-1** using the same method as compound **2-b. LC-MS:** [M+H]⁺=259.2.

### Compound E6-3: 3-ethynyl-5-(3-methylpyrrolidin-1-yl)pyridine

A yellow solid compound **E6-3** (60.0 mg, crude product) was synthesized from **E6-2** using the same method as compound **2-c.**

### Compound E6-4: [2-[[4-[5-(3-methylpyrrolidin-1-yl)-3-pyridyl]triazol-1-yl]methyl]imidazo[1,2-a]pyridin-6-yl]methanol

A yellow solid compound **E6-4** (120.0 mg, crude product) was synthesized from **E6-3** using the same method as compound **2-d.**

**LC-MS:** [M+H]⁺=390.2.

### Compound E6-5: 2-[[4-[5-(3-methylpyrrolidin-1-yl)-3-pyridyl]triazol-1-yl]methyl]imidazo[1,2-a]pyridine-6-formaldehyde

A yellow solid compound **E6-5** (25.0 mg, 20.9%) was synthesized from **E6-4** using the same method as compound **I-2. LC-MS:** [M+H]⁺=388.2.

### Compound E'-6: n-[(3-fluoro-1-bicyclo[1.1.1]pentyl)methyl]-1-[2-[[4-[5-(3-methylpyrrolidin-1-yl)-3-pyridyl]triazol-1-yl]methyl]imidazo[1,2-a]pyridin-6-yl]methylamine

A yellow solid compound **E'-6** (14.5 mg, 45.8%) was synthesized from **E6-5** by preparative high-performance liquid chromatography purification using the same method as compound **E'-2. LC-MS:** [M+H]⁺= 487.2. **¹H NMR:** (400 MHz, DMSO-*d₆*): *δ* 8.67 (s, 1H), 8.42 (s, 1H), 8.30 (d, *J =* 1.5 Hz, 1H), 7.95 (s, 1H), 7.85 (d, *J =* 2.8 Hz, 1H), 7.46 (d, *J =* 9.3 Hz, 1H), 7.29-7.23 (m, 2H), 5.73 (s, 2H), 3.67 (s, 2H), 3.48 (t, *J ₌* 8.3 Hz, 1H), 3.43-3.37 (m, 1H), 3.31-3.26 (m, 1H), 2.92-2.81 (m, 1H), 2.74 (s, 2H), 2.41-2.32 (m, 1H), 2.16-2.08 (m, 1H), 1.93 (d, *J* ₌ 2.8 Hz, 6H), 1.60 (qd, *J ₌* 8.3, 12.0 Hz, 1H), 1.09 (d, *J =* 6.6 Hz, 3H).

### Synthesis of Compounds E'-6-1 to E'-6-7

The compounds in Table 5 were synthesized and characterized in the same manner as compound E'-6.

**Table 5**

| **Compou nd** | **Name** | **Structure** | **LCM S [M+H ]⁺** |
|---|---|---|---|
| E'-6-1 | 1-(2-((4-(5-(3-azabicyclo[3.1.0]hex-3-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 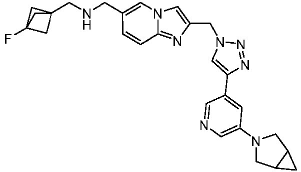 | 485.1 |
| E'-6-2 | 1-(3-fluorobicyclo[1.1.1]pentan-1-yl)-N-((2-((4-(5-(piperidin-1-yl)pyridin-3-yl)-1H-1,2,3-triazol-1-yl)methyl)imidazo[1,2-a]pyridin-6-yl)methyl)methylamine | 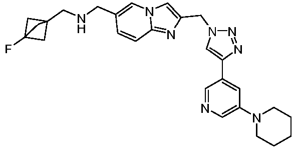 | 487.1 |
| E'-6-3 | 1-(2-((4-(5-(2-azabicyclo[2.1.1]hex-2-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 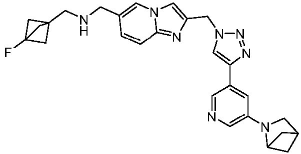 | 485.1 |
| E'-6-4 | n-[(3-fluoro-1-bicyclo[1.1.1jpentyl)methyl]-1-[2-[[4-[5-(2-methylpyrrolidin-1-yl)-3-pyridyl]triazol-1-yl]methyl]imidazo[1,2-a]pyridin-6-yl]methylamine | 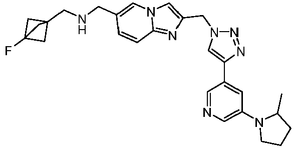 | 487.3 |
| E'-6-5 | 1-(2-((4-(5-(1H-imidazol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 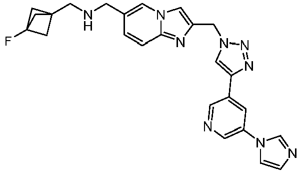 | 470.2 |
| E'-6-6 | 1-(2-((4-(5-(1,4-dioxa-7-azaspiro[4.4]nonan-7-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 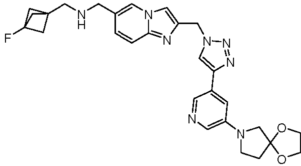 | 531.1 |
| E'-6-7 | tert-butyl-N-[(3-fluoro-1-bicyclo[1.1.1]pentyl)methyl]-N-[[2-[[4-[5-(2,2,3,3,4,4,5,5-octadeuteropyrrolidin-1-yl)-3-pyridyl]triazol-1-yl]methyl]imidazo[1,2-a]pyridin-6-yl]methyl]carbamate | 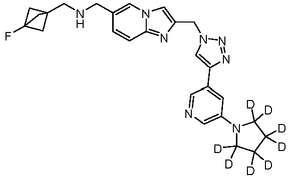 | 481.5 |
| E'-6-8 | 1-[2-[[4-[5-(3,3-dimethylpyrrolidin-1-yl)-3-pyridyl]triazol-1-yl]methyl]imidazo[1,2-a]pyridin-6-yl]-N-[(3-fluoro-1-bicyclic[1.1.1]pentyl)methyl]methylamine | 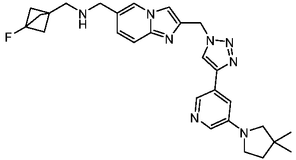 | 501.3 |
| E'-6-9 | 1-[2-[[4-[5-(3-azabicyclo[3.2.0]hept-3-yl)-3-pyridyl]triazol-1-yl]methyl]imidazo[1,2-a]pyridin-6-yl]-N-[(3-fluoro-1-bicyclo[1.1.1]pentyl)methyl]methylamine | 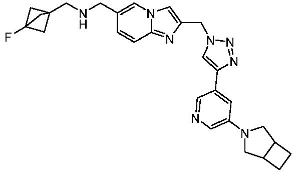 | 499.2 |
| E'-6-10 | n-[(3-fluoro-1-bicyclo[1.1.1]pentyl)methyl]-1-[2-[[4-[5-(3-methylpyrrol-1-yl)-3-pyridyl]triazol-1-yl]methyl]imidazo[1,2-a]pyridin-6-yl]methylamine | 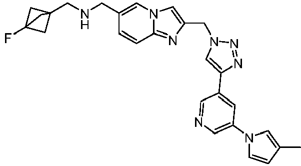 | 483.2 |
| E'-6-11 | 1-[5-[1-[[6-[(3-fluoro-1-bicyclo[1.1.1]pentyl)methylamino]methyl]i midazo[1,2-a]pyridin-2-ylmethyl]triazol-4-yl]-3-pyridyl]pyrrolidine-3-carbonitrile | 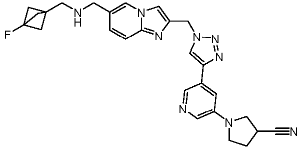 | 498.2 |
| E'-6-12 | 1-(2-((4-(5-(3,4-difluoro-1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 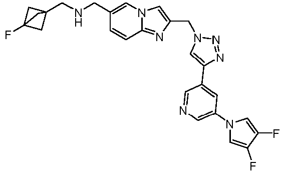 | 505.1 |
| E'-6-13 | 1-(2-((4-(5-(3,4-dimethyl-1H-pyrrol-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine | 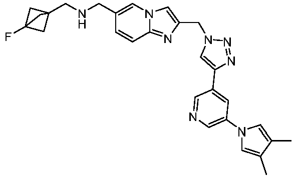 | 497.2 |

### Example 7 Compound E'-7: n-[(4-fluoro-1-bicyclo[2.2.2]octyl)methyl]-1-[2-[[4-(5-pyrrolidin-1-yl-3-pyridyl)triazol-1-yl]methyl]imidazo[1,2-a]pyridin-6-yl]methylamine

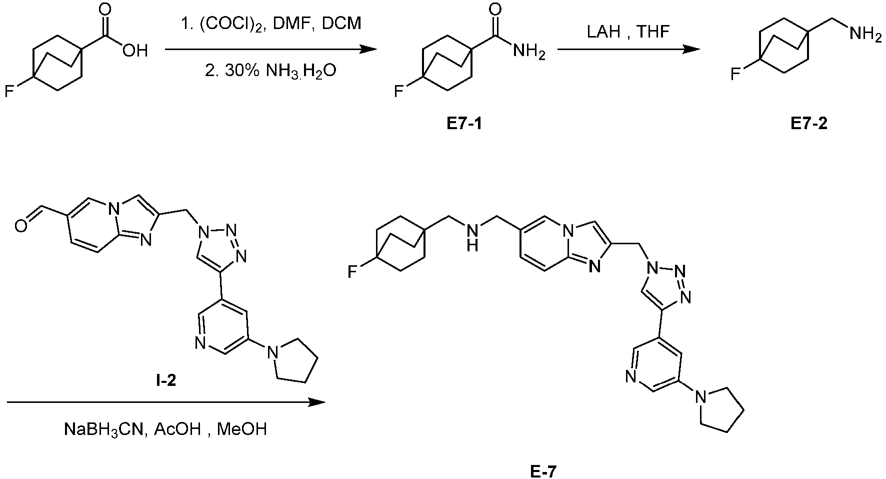

### Compound E7-1: 4-fluorobicyclo[2.2.2]octan-1-carboxamide

Under nitrogen protection at 0°C, oxalyl chloride (442 mg, 3.48 mmol) was added dropwise to a solution of 4-fluorobicyclo[2.2.2]octane-1-carboxylic acid (0.20 g, 1.16 mmol) and N,N-dimethylformamide (8.49 mg, 116 µmol) in dichloromethane (2 mL). The resulting mixture was stirred at 0°C for 1 h under nitrogen protection. After being concentrated under reduced pressure, the reaction solution was diluted with dichloromethane (2 mL), then ammonia water (2 mL) was added at 0°C, and the mixture was stirred at 25°C for 1 h. The reaction solution was concentrated under reduced pressure to obtain a crude product. The crude product was purified by column chromatography (V_{dichloromethane/methanol}=20/1) to obtain white solid **E7-1** (160.0 mg, 80.5%). **¹H NMR:** (400 MHz, DMSO-*d₆*): *δ* 7.10-6.59 (m, 2H), 1.93-1.62 (m, 12H).

### Compound E7-2: (4-fluoro-1-bicyclo[2.2.2]octyl)methylamine

Lithium aluminum tetrahydroxide (70.9 mg, 1.87 mmol) was added dropwise to a solution of 4-fluorobicyclo[2.2.2]octane-1-carboxamide (0.16 g, 935 µmol) in tetrahydrofuran (3.2 mL) at 25°C under nitrogen protection. The resulting mixture was stirred at 25°C for 2 h under nitrogen protection. At 0°C, glauber's salt (0.20 g) was slowly added to the reaction solution and stirred at 25°C for 1 h under nitrogen protection. The reaction solution was filtered and concentrated under reduced pressure to obtain a colorless oily liquid crude product **E7-2** (0.14 g). **¹H NMR:** (400 MHz, DMSO-*d₆*): *δ* 2.20 (s, 2H), 1.74-1.67 (m, 6H), 1.54-1.43 (m, 6H).

### Compound E'-7: n-[(4-fluoro-1-bicyclo[2.2.2]octyl)methyl]-1-[2-[[4-(5-pyrrolidin-1-yl-3-pyridyl)triazol-1-yl]methyl]imidazo[1,2-a]pyridin-6-yl]methylamine

A yellow solid compound **E'-7** (7.70 mg, 18.6%) was synthesized from **E7-2** by preparative high-performance liquid chromatography purification using the same method as compound **E'-2. LC-MS:** [M+H]⁺= 515.3. **¹H NMR:** (400 MHz, DMSO-*d₆*): *δ* 8.68 (s, 1H), 8.39 (s, 1H), 8.31 (s, 1H), 7.95 (s, 1H), 7.89 (br s, 1H), 7.45 (d, *J ₌* 9.3 Hz, 1H), 7.32-7.28 (m, 1H), 7.25 (dd, *J ₌* 1.6, 9.3 Hz, 1H), 5.73 (s, 2H), 3.62 (s, 2H), 3.30 (br s, 4H), 2.13 (s, 2H), 2.00-1.95 (m, 4H), 1.73-1.65 (m, 6H), 1.59-1.49 (m, 6H).

### Example 8 Compound E'-8: 1-(5-(1-((6-(((3-fluorobicyclo[1.1.1]pentan-1-yl)methyl)amino)methyl)imidazo[1,2-a]pyridin-2-yl)methyl)-1H-1,2,3-triazol-4-yl)pyridin-3-yl)pyrrolidin-3-one

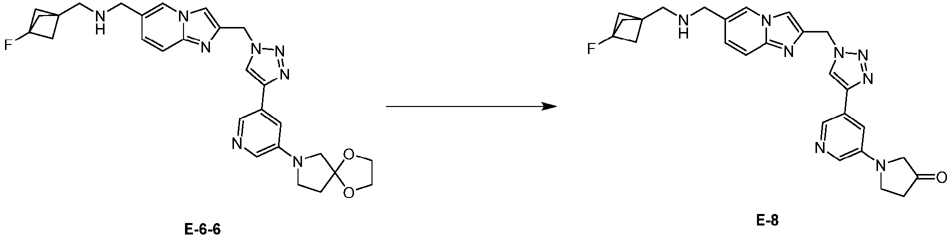

A yellow solid compound **E'-8** (1.6 mg, 13.6%) was synthesized from **E'-6-6** by preparative high-performance liquid chromatography purification using the same method as compound **I-3. LC-MS:** [M+H]⁺= 487.2. **¹H NMR:** (400 MHz, DMSO-*d₆*): *δ* 8.72-8.72 (m, 1H), 8.72 (s, 1H), 8.47 (br s, 1H), 8.42 (br s, 1H), 8.03 (br s, 1H), 7.97 (s, 1H), 7.46 (br s, 2H), 7.28 (s, 1H), 5.75 (br s, 2H), 3.78 (br s, 2H), 3.72-3.71 (m, 2H), 3.68 (br s, 2H), 2.74 (br s, 2H), 2.72-2.70 (m, 2H), 1.93 (br s, 6H).

### Example 9 Compound E'-9: 1-[2-[[4-[5-(aziridin-1-yl)-3-pyridyl]triazol-1-yl]methyl]imidazo[1,2-a]pyridin-6-yl]-N-[(3-fluoro-1-bicyclo[1.1.1]pentyl)methyl]methylamine

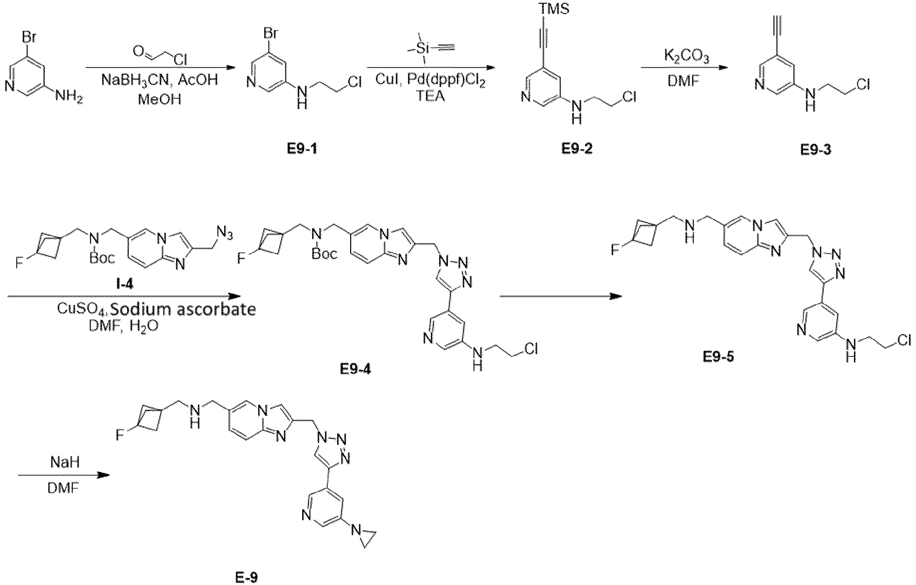

### Compound E9-1: 5-bromo-N-(2-chloroethyl)pyridine-3-amine

A yellow solid compound **E9-1** (0.75 g, 55.1%) was synthesized from 5-bromopyridine-3-amine using the same method as compound **E'-2. LC-MS:** [M+H]⁺=236.9.

### Compound E9-2: n-(2-chloroethyl)-5-(2-trimethylsilylethynyl)pyridine-3-amine

A brown solid compound **E9-2** (0.15 g, 33.5%) was synthesized from **E9-1** using the same method as compound **2-b. LC-MS:** [M+H]⁺=253.1.

### Compound E9-3: n-(2-chloroethyl)-5-ethynyl-pyridine-3-amine

A yellow solid compound **E9-3** (15 mg, crude product) was synthesized from **E9-**2 using the same method as compound **2-c. LC-MS:** [M+H]⁺=181.2.

### Compound E9-4: Tert-butyl N-[[2-[[4-[5-(2-chloroethylamino)-3-pyridyl]triazol-1-yl]methyl]imidazo[1,2-a]pyridin-6-yl]methyl]-N-[(3-fluoro-1-bicyclo[1.1.1]pentyl)methyl]carbamate

A yellow solid compound **E9-4** (70 mg, crude product) was synthesized from **E9-**3 using the same method as compound **2-d. LC-MS:** [M+H]⁺=581.3.

### Compound E9-5: n-(2-chloroethyl)-5-[1-[[6-[(3-fluoro-1-bicyclic[1.1.1]pentyl)methylamino]methyl]imidazo[1,2-a]pyridin-2-yl]methyl]triazol-4-yl]pyridin-3-amine

A yellow solid compound **E9-5** (120 mg, crude product) was synthesized from **E9-4** using the same method as compound **I-3. LC-MS:** [M+H]⁺=481.2.

### Compound E'-9: 1-[2-[[4-[5-(aziridin-1-yl)-3-pyridyl]triazol-1-yl]methyl]imidazo[1,2-a]pyridin-6-yl]-N-[(3-fluoro-1-bicyclo[1.1.1]pentyl)methyl]methylamine

Sodium hydride (19.3 mg, 483 µmol) was added to an N,N-dimethylformamide solution (0.50 mL) of **E9-5** (50 mg, 96.6 µmol) at 0°C. The resulting mixture was stirred at 25°C for 2 h. Water (0.5mL) was added at room temperature to quench the reaction solution, and the reaction solution was concentrated under reduced pressure to obtain solid. The solid was purified by preparative high-performance liquid chromatography to obtain a yellow solid compound **E'-9** (6.0 mg, 13.8%). **LC-MS:** [M+H]⁺= 445.2. **¹H NMR:** (400 MHz, DMSO-*d₆*): *δ* 8.70 (s, 1H), 8.65 (d, *J ₌* 1.9 Hz, 1H), 8.42 (s, 1H), 8.24 (d, *J* ₌ 2.5 Hz, 1H), 7.96 (s, 1H), 7.79 (t, *J* ₌ 2.2 Hz, 1H), 7.46 (d, *J ₌* 9.3 Hz, 1H), 7.26 (dd, *J* ₌ 1.6, 9.3 Hz, 1H), 5.74 (s, 2H), 3.68 (s, 2H), 2.74 (s, 2H), 2.16 (s, 4H), 1.93 (d, *J* ₌ 2.6 Hz, 6H).

### Example 10 Compound E'-10: n-[(3-chloro-1-bicyclo[1.1.1]pentyl)methyl]-1-[2-[[4-(5-pyrrolidin-1-yl-3-pyridyl)triazol-1-yl]methyl]imidazo[1,2-a]pyridin-6-yl]methylamine

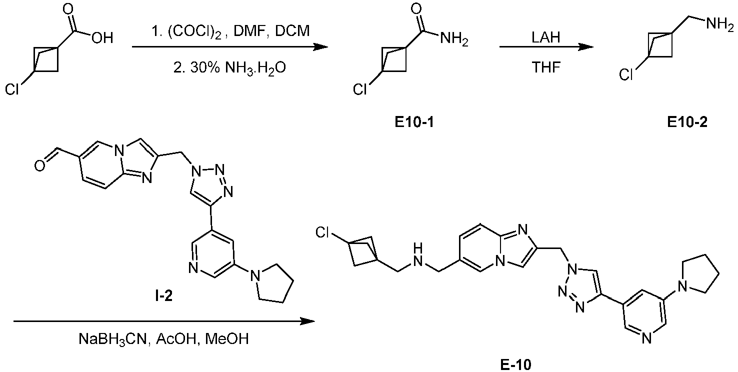

### Compound E10-1: 3-chlorobicyclo[1.1.1]pentane-1-carboxamide

A white solid compound **E10-1** (0.36 g, 72.5%) was synthesized from 3-chlorobicyclo[1.1.1]pentane-1-carboxylic acid using the same method as compound 3-b. **¹H NMR:** (400 MHz, DMSO-*d₆*): *δ* 7.39 (br s, 1H), 7.11 (br s, 1H), 2.31-2.31 (m, 1H), 2.31 (s, 6H).

### Compound E10-2: (3-chloro-1-bicyclo[1.1.1]pentyl)methylamine

Lithium aluminum tetrahydroxide (188 mg, 4.95 mmol) was added dropwise to a tetrahydrofuran solution (7.20 mL) of **E10-1** (0.36 g, 2.47 mmol) at 25°C. The resulting mixture was stirred at 25°C for 2 h. Glauber's salt (0.6 g) was added at 0°C. The mixture was stirred at 25°C for 1 h. The reaction solution was extracted with dichloromethane (30 mL * 3), and the combined organic layers were washed with a saturated saline solution, dried over anhydrous sodium sulfate, filtered, and concentrated under reduced pressure to obtain a white solid crude product **E10-2** (70 mg). **¹H NMR:** (400 MHz, DMSO-*d₆*): *δ* 7.38 (br s, 1H), 7.09 (br s, 1H), 2.31 (s, 6H), 2.00 (s, 2H).

### Compound E'-10: n-[(3-chloro-1-bicyclo[1.1.1]pentyl)methyl]-1-[2-[[4-(5-pyrrolidin-1-yl-3-pyridyl)triazol-1-yl]methyl]imidazo[1,2-a]pyridin-6-yl]methylamine

A light yellow solid compound **E'-10** (3.10 mg, 7.69%) was synthesized from **E10-2** by preparative high-performance liquid chromatography purification using the same method as compound **E'-2. LC-MS:** [M+H]⁺= 489.2. **¹H NMR:** (400 MHz, DMSO-*d₆*): *δ* 8.67 (s, 1H), 8.41 (s, 1H), 8.31 (s, 1H), 7.95 (s, 1H), 7.89 (d, *J* = 2.4 Hz, 1H), 7.46 (d, *J =* 9.3 Hz, 1H), 7.30-7.27 (m, 1H), 7.25 (dd, *J ₌* 1.5, 9.3 Hz, 1H), 5.73 (s, 2H), 3.66 (s, 2H), 3.29 (br s, 4H), 2.65 (s, 2H), 2.05 (s, 6H), 2.00-1.95 (m, 4H).

### Synthesis of Compounds E'-10-1 to E'-10-5

The compounds in Table 6 were synthesized and characterized in the same manner as compound E'-10.

**Table 6**

| **Compou nd** | **Name** | **Structure** | **LCMS [M+H]⁺** |
|---|---|---|---|
| E'-10-1 | 1-(3-methylcyclo[1.1.1]pentan-1-yl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine | 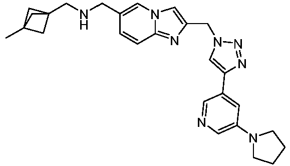 | 469.3 |
| E'-10-2 | 1-(bicyclo[1.1.1]pentan-1-yl)-N-((2-((4-(5-(pyrrolidin-1-yl)pyridin-3-yl)-1H-1,2,3-triazol-1-yl)methyl)imidazo[1,2-a]pyridin-6-yl)methyl)methanamine | 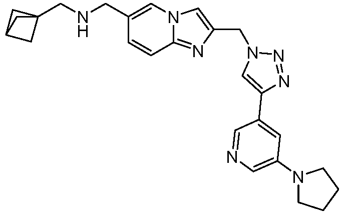 | 455.3 |
| E'-10-3 | 1-(2-((4-(5-(pyrrolidin-1-yl)pyridin-3-yl)-1H-1,2,3-triazol-1-yl)methyl)imidazo[1,2-a]pyridin-6-yl)-N-((3-(trifluoromethyl)bicyclo[1.1.1] pentan-1-yl)methyl)methylamine | | 523.2 |
| E'-10-4 | n-[(4-fluoro-1-yl)methyl]-1-[2-[[4-(5-pyrrolidin-1-yl-3-pyridyl)triazol-1-yl]methyl]imidazo[1,2-a]pyridin-6-yl]methylamine | | 509.3 |
| E'-10-5 | n-[(3-isopropyl-1-bicyclo[1.1.1]pentyl)methyl]-1-[2-[[4-(5-pyrrolidin-1-yl-3-pyridyl)triazol-1-yl]methyl]imidazo[1,2-a]pyridin-6-yl]methylamine | | 497.3 |

### Example 11 Compound E'-11: n-[(3-fluoro-1-bicyclo[1.1.1]pentyl)methyl]-1-[2-[[4-[5-(1,2,4-triazol-4-yl)-3-pyridyl]triazol-1-yl]methyl]imidazo[1,2-a]pyridin-6-yl]methylamine

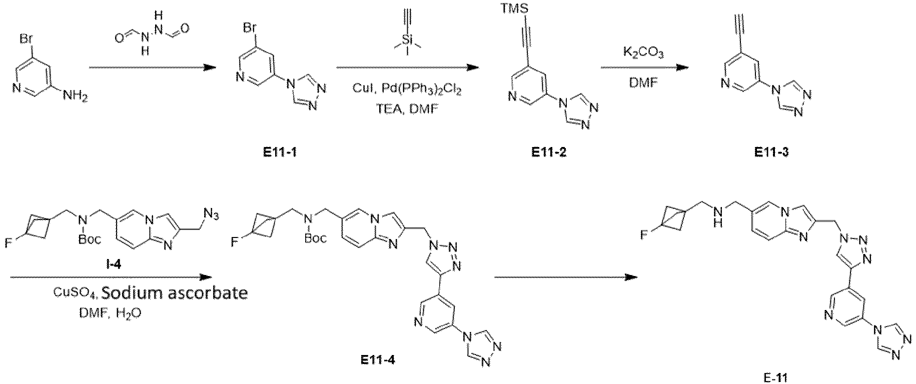

### Compound E11-1: 3-bromo-5-(4H-1,2,4-triazol-4-yl)pyridine

5-bromopyridine-3-amine (1.0 g, 5.78 mmol) and diformylhydrazine (509 mg, 5.78 mmol) were added into a sealed tube at room temperature. The mixture was stirred at 140°C for 16 h under nitrogen protection. After cooling to room temperature, the reaction solution was concentrated under reduced pressure to obtain a crude product. The crude product was purified by column chromatography (V_{petroleum ether/ethyl acetate}=20/1 to 0/1) to obtain brown solid **E11-1** (300.0 mg, 23.1%). **LC-MS:** [M+H]⁺=226.9.

### Compound 11-2: Trimethyl-[2-[5-(1,2,4-triazol-4-yl)-3-pyridyl]ethynyl]silane

A brown oily compound **E11-2** (0.08 g, 74.3%) was synthesized from **E11-1** using the same method as compound **2-b. LC-MS:** [M+H]⁺=243.2.

### Compound E11-3: 3-ethynyl-5-(1,2,4-triazole-4-yl)pyridine

A yellow solid compound **E11-3** (35 mg, crude product) was synthesized from **E11-2** using the same method as compound **2-c. LC-MS:** [M+H]⁺=171.2.

### Compound E11-4: Tert-butyl ((2-((4-(5-(4H-1,2,4-triazol-4-yl)pyridin-3-yl)-1H-1,2,3-triazol-1-yl)methyl)imidazo[1,2-a]pyridin-6-yl)methyl)((3-fluorobicyclo[1.1.1]pentan-1-yl)methyl)carbamate

A yellow solid compound **E11-4** (50 mg, crude product) was synthesized from **E11-3** using the same method as compound **2-d. LC-MS:** [M+H]⁺=571.3.

### E'-11: n-[(3-fluoro-1-bicyclo[1.1.1]pentyl)methyl]-1-[2-[[4-[5-(1,2,4-triazol-4-yl)-3-pyridyl]triazol-1-yl]methyl]imidazo[1,2-a]pyridin-6-yl]methylamine

A gray solid compound **E'-11** (2 mg, 1.97%) was synthesized from **E11-4** by preparative high-performance liquid chromatography purification using the same method as compound **I-3. LC-MS:** [M+H]⁺= 471.2. **¹H NMR:** (400 MHz, DMSO-*d₆*): δ 9.26 (s, 2H), 9.13 (d, *J* = 1.8 Hz, 1H), 8.94 (d, *J =* 2.5 Hz, 1H), 8.77 (s, 1H), 8.59 (t, *J =* 2.1 Hz, 1H), 8.43 (s, 1H), 8.01 (s, 1H), 7.47 (d, *J =* 9.3 Hz, 1H), 7.27 (dd, *J =* 1.5, 9.3 Hz, 1H), 5.80 (s, 2H), 3.68 (br s, 2H), 2.74 (br s, 2H), 1.93 (d, *J =* 2.8 Hz, 6H).

### Example 12 Compound E'-12: [6-[[(3-fluoro-1-bicyclo[1.1.1]pentyl)methylamino]methyl]imidazo[1,2-a]pyridin-2-yl]methyl 5-pyrrolidin-1-yl pyridin-3-carboxylate

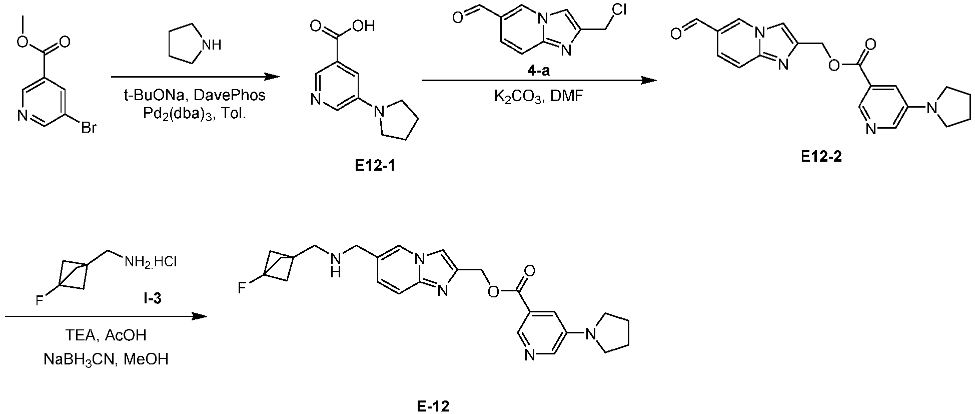

### Compound E12-1: 5-(pyrrolidin-1-yl)nicotinic acid

Sodium tert-butoxide (4.45 g, 46.3 mmol), 2-dicyclohexylphosphino-2'-(N,N-dimethylamine)-biphenyl (911 mg, 2.31 mmol), and tris(dibenzylidene BASE acetone)dipalladium (0) (2.12 g, 2.31 mmol) were added to a toluene solution (50 mL) of 5-bromopyridine-3-carboxylate (5.0 g, 23.1 mmol) and tetrahydropyrrole (32.9 g, 463 mmol, 29.6 mL) at 25°C under nitrogen protection. The resulting mixture was stirred at 80°C for 16 h under nitrogen protection. After cooling to room temperature, the mixture was extracted with ethyl acetate (30 mL * 2), and the combined organic layers were washed with a saturated saline solution, dried over anhydrous sodium sulfate, filtered, and concentrated under reduced pressure to obtain a crude product. The crude product was purified by column chromatography (Vₚₑₜᵣₒₗₑᵤₘ ether/ethyl _{acetate}=20/1 to 0/1) to obtain yellow solid **E12-1** (4.0 g, 70.5%). **LC-MS:** [M+H]⁺=193.

### Compound E12-2: (6-formyl imidazo[1,2-a]pyridin-2-yl)methyl 5-pyrrolidin-1-yl pyridine-3-carboxylate

A yellow solid compound **E12-2** (20 mg, 11.1%) was synthesized from **E12-1** using the same method as compound **2-c.**

**LC-MS:** [M+H]⁺= 351.1.

### Compound E'-12: [6-[[(3-fluoro-1-bicyclo[1.1.1]pentyl)methylamino]methyl]imidazo[1,2-a]pyridin-2-yl]methyl 5-pyrrolidin-1-yl pyridin-3-carboxylate

A yellow solid compound **E'-12** (7.30 mg, 36.8%) was synthesized from **E12-2** by preparative high-performance liquid chromatography purification using the same method as compound **E'-2. LC-MS:** [M+H]⁺= 450.2. **¹H NMR** (400 MHz, DMSO-*d₆*): *δ* 8.41 (s, 1H), 8.36 (d, *J ₌* 1.6 Hz, 1H), 8.16 (d, *J ₌* 3.0 Hz, 1H), 8.01 (s, 1H), 7.48 (d, *J ₌* 9.3 Hz, 1H), 7.29-7.24 (m, 2H), 5.45 (s, 2H), 3.69 (s, 2H), 3.31-3.25 (m, 4H), 2.76 (s, 2H), 1.99-1.95 (m, 4H), 1.94 (d, *J ₌* 2.8 Hz, 6H).

### Example 13 Compound E'-13: 1-(2-((4-(5-(azetidine-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

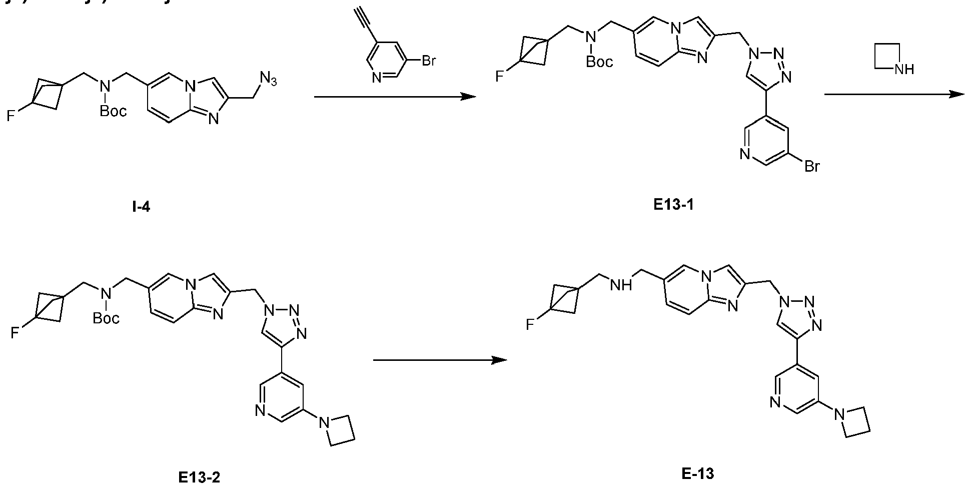

### Compound E13-1: Tert-butyl ((2-((4-(5-bromopyridin-3-yl)-1H-1,2,3-triazol-1-yl)methyl)imidazo[1,2-a]pyridin-6-yl)methyl)((3-fluorobicyclo[1.1.1]pentan-1-yl)methyl)carbamate

A colorless oily compound **E13-1** (40 mg, 44.4%) was synthesized from **I-4** using the same method as compound **2-d.**

**LC-MS:** [M+H]⁺=583.9.

### Compound E13-2: Tert-butyl ((2-((4-(5-(azetidine-1-yl)pyridin-3-yl)-1H-1,2,3-triazol-1-yl)methyl)imidazo[1,2-a]pyridin-6-yl)methyl)((3-fluorobicyclo[1.1.1]pentan-1-yl)methyl)carbamate

A yellow solid compound **E13-2** (18 mg, 62.6%) was synthesized from **E13-1** using the same method as compound **E12-2.**

**LC-MS:** [M+H]⁺=559.4.

### Compound E'-13: 1-(2-((4-(5-(azetidine-1-yl)pyridin-3-yl)-1H-1,2,3-triazol-1-yl)methyl)imidazo[1,2-a]pyridin-6-yl)-N-((3-fluorobicyclo[1.1.1]pentan-1-yl)methyl)methylamine

A yellow solid compound **E'-13** (1.40 mg, 10.9%) was synthesized from **E13-2** by preparative high-performance liquid chromatography purification using the same method as compound **I-3.**

**LC-MS:** [M+H]⁺= 459.2. **¹H NMR** (400 MHz, DMSO-*d₆*): *δ* 8.67 (s, 1H), 8.42 (s, 1H), 8.38 (d, *J =* 1.6 Hz, 1H), 7.95 (s, 1H), 7.74 (d, *J =* 2.6 Hz, 1H), 7.46 (d, *J =* 9.3 Hz, 1H), 7.26 (dd, *J* ₌ 1.6, 9.3 Hz, 1H), 7.21-7.19 (m, 1H), 5.73 (s, 2H), 3.91 (t, *J =* 7.2 Hz, 4H), 3.67 (s, 2H), 2.74 (s, 2H), 2.35 (d, *J ₌* 7.3 Hz, 2H), 1.93 (d, *J ₌* 2.8 Hz, 6H).

### Example 14 Compound E'-14: n-[[6-[[(3-fluoro-1-bicyclo[1.1.1]pentyl)methylamino]methyl]imidazo[1,2-a]pyridin-2-yl]methyl]-5-pyrrolidin-1-yl-pyridazin-3-carboxamide

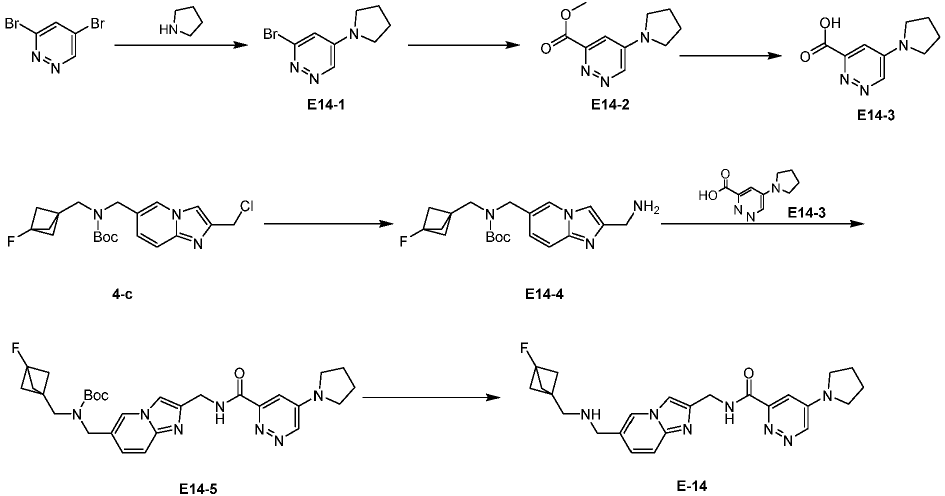

### Compound E14-1: 3-bromo-5-pyrrolidin-1-yl-pyridazine

Pyrrolidine (2.27 g, 32.0 mmol) was added to a tetrahydrofuran solution (24 mL) of 3,5-dibromopyridazine (800.0 mg, 3.36 nmol) at 25°C. The resulting mixture was stirred at 25°C for 16 h under nitrogen protection. The reaction solution was concentrated under reduced pressure to obtain a crude product. The crude product was purified by column chromatography (Vₚₑₜᵣₒₗₑᵤₘ ether/ethyl _{acetate}=50/1 to 1/2) to obtain yellow solid **E14-1** (630 mg, 82.1%).

**LC-MS:** [M+H]⁺=227.9.

### Compound E14-2: Methyl 5-pyrrolidin-1-yl pyridazine-3-carboxylate

Triethylamine (1.40 g, 13.8 mmol) and 1,1-bis(diphenylphosphino)ferrocene palladium (II) dichloride (202 mg, 276 µmol) were added to a methanol solution (31.5 mL) of **E14-1** (0.63 g, 2.76 mmol) at 25°C under nitrogen protection. The resulting mixture was stirred at 80°C for 16 h in presence of carbon monoxide. After cooling to room temperature, the reaction solution was filtered and concentrated under reduced pressure to obtain a crude product. The crude product was purified by column chromatography (V_{ethyl acetate/methanol}=50/1 to 10/1) to obtain brown solid **E14-2** (0.39 g, 68.1%). **LC-MS:** [M+H]⁺=208.

### Compound E14-3: 5-pyrrolidin-1-yl pyridazin-3-carboxylic acid

Lithium hydroxide (34.7 mg, 1.45 mmol) was added to a methanol solution (2 mL) and water (0.5 mL) of **E14-2** (0.1 g, 482.56 µmol) at 25°C. The resulting mixture was stirred at 25°C for 2 h. The pH was adjusted to 4 with 2N hydrochloric acid, the reaction solution was extracted with ethyl acetate (2 mL * 3), and the combined organic layers were washed with a saturated saline solution, dried over anhydrous sodium sulfate, filtered, and concentrated under reduced pressure to obtain brown solid **E14-3** (120 mg, crude product).

**LC-MS:** [M+H]⁺=194.

### Compound E14-4: Tert-butyl N-[[2-(aminomethyl)imidazo[1,2-a]pyridin-6-yl]methyl]-N-[(3-fluoro-1 bicyclo[1.1.1]pentyl)methyl]carbamate

An ammonia/methanol solution (7 M, 10 mL) was added to **4-c** (0.32 g, 812 µmol) at 25°C. The resulting mixture was stirred in an autoclave at 50°C for 16 h. After cooling to room temperature, the reaction solution was concentrated under reduced pressure to obtain a crude product. The crude product was purified by column chromatography (V_{dichloromethane/methanol}=5/1) to obtain yellow solid **E14-4** (0.19 g, 62.5%). **LC-MS:** [M+H]⁺= 375.3.

### Compound E14-5: Tert-butyl N-[(3-fluoro-1-bicyclo[1.1.1]pentyl)methyl]-N-[[2-[[(5-pyrrolidin-1-yl pyridazin-3-carbonyl)amino]methyl]imidazo[1,2-a]pyridin-6-yl]methyl]carbamate

Chloro-N,N,N',N'-tetramethylformamidinium hexafluorophosphate (31.5 mg, 112 µmol) and N-methylimidazole (38.4 mg, 467 µmol, 37.3 µL) were added to a solution of **E14-4** (35 mg, 93.5 µmol) and **E14-3** (27.1 mg, 140 µmol) in N,N-dimethylformamide (1.05 mL) at 25°C. The resulting mixture was stirred at 25°C for 16 h. The reaction solution was quenched with water (5 mL) and extracted with ethyl acetate (2 mL * 4). The combined organic layers were washed with a saturated saline solution, dried over anhydrous sodium sulfate, filtered, and concentrated under reduced pressure to obtain a crude product. The crude product was purified by column chromatography (V_{dichloromethane/methanol}=20/1) to obtain pale yellow oily liquid **E14-5** (25.0 mg, 48.7%).

**LC-MS:** [M+H]⁺= 550.4.

### Compound E'-14: n-[[6-[[(3-fluoro-1-bicyclo[1.1.1]pentyl)methylamino]methyl]imidazo[1,2-a]pyridin-2-yl]methyl]-5-pyrrolidin-1-yl-pyridazin-3-carboxamide

A yellow solid compound **E'-14** (10 mg, 48.8%) was synthesized from **E14-5** by preparative high-performance liquid chromatography purification using the same method as compound **I-3.**

**LC-MS:** [M+H]⁺= 450.4. **¹H NMR** (400 MHz, DMSO-*d₆*): *δ* 9.03 (s, 1H), 8.68 (d, *J* = 1.9 Hz, 1H), 8.37 (s, 1H), 8.10 (d, *J ₌* 9.1 Hz, 1H), 7.99 (d, *J ₌* 9.3 Hz, 1H), 7.74 (br s, 1H), 4.94 (s, 2H), 4.45 (s, 2H), 3.82-3.71 (m, 4H), 3.51 (s, 2H), 2.19 (d, *J =* 2.1 Hz, 10H).

### Example 15 Compound E'-15: n-[(3-fluoro-1-bicyclo[1.1.1]pentyl)methyl]-1-[2-[[3-(5-pyrrolidin-1-yl-3-pyridyl)-1,2,4-triazol-1-yl]methyl]imidazo[1,2-a]pyridin-6-yl]methylamine

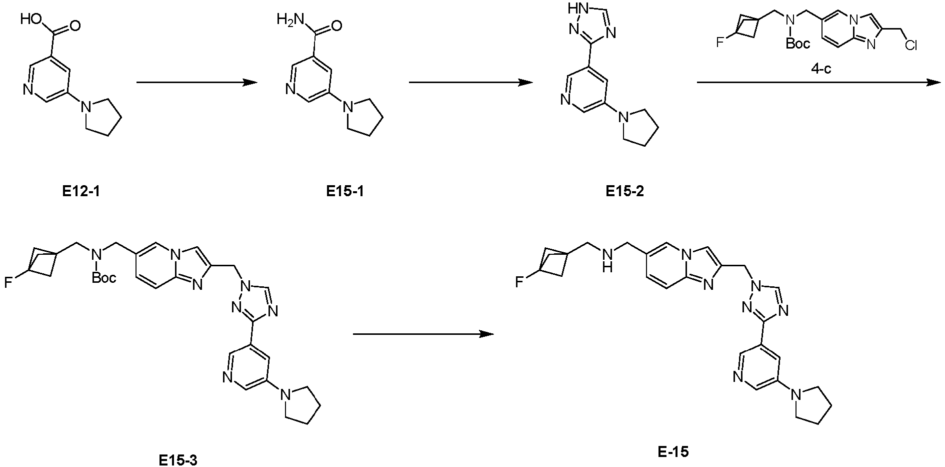

### Compound E15-1: 5-(pyrrolidin-1-yl)nicotinamide

1-(3-dimethylaminopropyl)-3-ethylcarbodiimide hydrochloride (299 mg, 1.56 mmol), 1-hydroxybenzotriazole (210 mg, 1.56 mmol), N,N-diisopropylethylamine (403 mg, 3.12 mmol, 543 µL), and ammonium chloride (111 mg, 2.08 mmol) were added to a solution of **E12-1** (0.20 g, 1.04 mmol) in N,N-dimethylformamide (10.0 mL) at 25°C. The resulting mixture was stirred at 25°C for 12 h. The reaction solution was diluted with water (50 mL) and extracted with ethyl acetate (15 mL * 6). The combined organic layers were washed with a saturated saline solution, dried over anhydrous sodium sulfate, filtered, and concentrated under reduced pressure to obtain a crude product. The crude product was purified by column chromatography (Vdichloromethane/methanol=20/1) to obtain white solid **E15-1** (120.0 mg, 60.3%).

**LC-MS:** [M+H]⁺=192.1.

### Compound E15-2: 3-(pyrrolidin-1-yl)-5-(1H-1,2,4-triazole-3-yl)pyridine

A solution of **E15-1** (120 mg, 627 µmol) in N,N-dimethylformamide dimethyl acetal (4.80 mL) was stirred at 105°C for 2 h. After the reaction solution was concentrated under reduced pressure, acetic acid (4.80 mL) and hydrazine hydrate (219 mg, 4.39 mmol, 213 µL) were added. The resulting mixture was stirred at 100°C for 1 h. The reaction solution was cooled to room temperature, concentrated under reduced pressure, diluted with water (2 mL) and extracted with ethyl acetate (1 mL * 3). The combined organic layers were washed with a saturated saline solution, dried over anhydrous sodium sulfate, filtered, and concentrated under reduced pressure to obtain a crude product. The crude product was purified by column chromatography (V_{dichloromethane/methanol}=20/1) to obtain yellow solid **E15-2** (100.0 mg, 74.0%).

**LC-MS:** [M+H]⁺=216.2.

### Compound E15-3: Tert-butyl ((3-fluorobicyclo[1.1.1]pentan-1-yl)methyl)((2-((3-(5-(pyrrolidin-1-yl)pyridin-3-yl)-1H-1,2,4-triazole-1-yl)methyl)imidazo[1,2-a]pyridin-6-yl)methyl)carbamate

A white solid compound **E15-3** (20.0 mg, 30.0%) was synthesized from **E15-2** using the same method as compound **E6-1. LC-MS:** [M+H]⁺=573.2.

### Compound E'-15: n-[(3-fluoro-1-bicyclo[1.1.1]pentyl)methyl]-1-[2-[[3-(5-pyrrolidin-1-yl-3-pyridyl)-1,2,4-triazol-1-yl]methyl]imidazo[1,2-a]pyridin-6-yl]methylamine

A yellow solid compound **E'-15** (10.7 mg, 57.7%) was synthesized from **E15-3** by preparative high-performance liquid chromatography purification using the same method as compound **I-3.**

**LC-MS:** [M+H]⁺= 473.3. **¹H NMR:** (400 MHz, DMSO-*d₆*): *δ* 10.00 (br s, 1H), 9.12 (s, 1H), 9.03 (s, 1H), 8.55 (s, 1H), 8.40 (s, 1H), 8.21 (d, *J =* 9.3 Hz, 1H), 8.15 (d, *J =* 1.9 Hz, 1H), 7.99 (d, *J* ₌ 9.3 Hz, 1H), 7.90 (br s, 1H), 5.93 (s, 2H), 4.29 (br s, 2H), 3.42 (br s, 4H), 3.27 (br s, 2H), 2.13 (d, *J ₌* 2.0 Hz, 6H), 2.01 (br s, 4H).

### Example 16 Compound E'-16: N-[1-[6-[(cyclobutylmethylamino)methyl]imidazo[1,2-a]pyridin-2-yl]propyl]-5-pyrrolidin-1-yl-pyridine-3-carboxamide

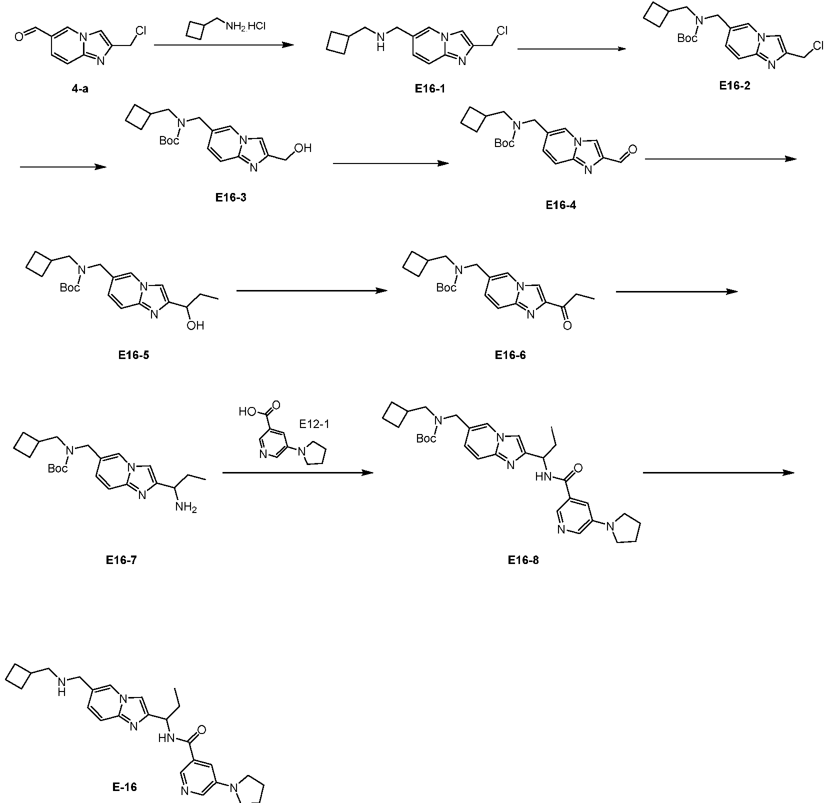

### Compound E16-1: 1-(2-(chloromethyl)imidazo[1,2-a]pyridin-6-yl)-N-(cyclobutylmethyl)methylamine

A white solid compound **E16-1** (6.5 g, 75.3%) was synthesized from **4-a** using the same method as compound **E'-2.**

**LC-MS:** [M+H]⁺=264.1.

### Compound E16-2: Tert-butyl ((2-(chloromethyl)imidazo[1,2-a]pyridin-6-yl)methyl)(cyclobutylmethyl)carbamate

A pale yellow solid compound **E16-2** (7.32 g, 81.7%) was synthesized from **E16-**1 using the same method as compound **4-c.**

**LC-MS:** [M+H]⁺=364.1.

### Compound E16-3: Tert-butyl (cyclobutylmethyl)((2-(hydroxymethyl)imidazo[1,2-a]pyridin-6-yl)methyl)carbamate

Sodium bicarbonate (1.39 g, 16.5 mmol, 641 µL, 3.00 eq) was added to a mixed solution of **E16-2** (2.00 g, 5.50 mmol, 1.00 eq) in dimethyl sulfoxide (20.0 mL) and water (20.0 mL), and the resulting mixture was stirred at 120°C for 1 h. The reaction mixture was cooled to 20°C, diluted with water (100 mL), and extracted with DCM (30.0 mL × 3). The combined organic layers were dried over anhydrous sodium sulfate, filtered, and concentrated. The crude product was purified by silica gel column chromatography (V_{ethyl acetate: methanol}=50/1 to 10/1) to obtain yellow solid **E16-3** (1.20 g, 63.2%).

**LC-MS:** [M+H]⁺=346.1.

### Compound E16-4: Tert-butyl (cyclobutylmethyl)((2-formylimidazo[1,2-a]pyridin-6-yl)methyl)carbamate

A pale yellow solid compound **E16-4** (1.10 g, 92.4%) was synthesized from **E16-3** using the same method as compound **I-2.**

**LC-MS:** [M+H]⁺=344.2.

### Compound E16-5: Tert-butyl (cyclobutylmethyl)((2-(1-hydroxypropyl)imidazo[1,2-a]pyridin-6-yl)methyl)carbamate

Magnesium bromide(ethyl) (3 M, 388 µL) was added dropwise to a solution of **E16-4** (200 mg, 582 µmol) in tetrahydrofuran (4.0 mL) under nitrogen protection at -60°C, and the resulting mixture was stirred at 0°C for 1 h. The reaction solution was quenched with a saturated ammonium chloride solution (5.00 mL) at 0°C, diluted with water (3.00 mL), and extracted with dichloromethane (2.0 mL × 3). The combined organic layers were dried over anhydrous sodium sulfate, filtered, and concentrated. The crude product was purified by silica gel column chromatography (ethyl acetate) to obtain yellow solid **E16-5** (0.11 g, 50.6%).

**LC-MS:** [M+H]⁺=374.2.

### Compound E16-6: Tert-butyl N-(cyclobutylmethyl)-N-[(2-propionimidylazo)[1,2-a]pyridin-6-yl)methyl]carbamate

A yellow solid compound **E16-6** (60.0 mg, 54.8%) was synthesized from **E16-5** using the same method as compound **I-2.**

**LC-MS:** [M+H]⁺=372.2.

### Compound E16-7: Tert-butyl ((2-(1-aminopropyl)imidazo[1,2-a]pyridin-6-yl)methyl)(cyclobutylmethyl)carbamate

Ammonium acetate (125 mg, 1.62 mmol) was added to a solution of **E16-6** (60 mg, 161 µmol) in methanol (1.80 mL) at 30°C. After stirring at 30°C for 0.5 h, sodium cyanoborohydride (20.3 mg, 323 µmol) was added. The resulting mixture was stirred at 50°C for 15.5 h. After the reaction solution was concentrated under reduced pressure, water (5.20 mL) was added and the pH was adjusted to 8 with a saturated sodium bicarbonate solution. The product was extracted with ethyl acetate (2 mL * 3), and the combined organic layers were washed with a saturated saline solution, dried over anhydrous sodium sulfate, filtered, and concentrated under reduced pressure to obtain yellow solid **E16-7** (100.0 mg, crude product). **LC-MS:** [M+H]⁺=373.2.

### Compound E16-8: Tert-butyl (cyclobutylmethyl)((2-(1-(5-(pyrrolidin-1-yl)nicotinyl)propyl)imidazo[1,2-a]pyridin-6-yl)methyl)carbamate

1-(3-dimethylaminopropyl)-3-ethylcarbodiimide hydrochloride (54.0 mg, 282 µmol) was added to a solution of **E12-1** (39.7 mg, 207 µmol) and **E16-7** (70.0 mg, 188 µmol) in pyridine (0.70 mL) while stirring. The resulting mixture was stirred at 25°C for 2 h under nitrogen protection. The mixture was quenched with water (3.00 mL) and extracted with dichloromethane (1.00 mL * 3). The combined organic layers were washed with a saturated saline solution (1.00 mL), dried over anhydrous sodium sulfate, filtered, and concentrated. The crude product was purified by silica gel pre-thin-layer chromatography (DCM: MeOH=10:1) and then eluted to obtain white solid **E16-8** (26.0 mg, 25.3%).

**LC-MS:** [M+H]⁺=547.2.

### Compound E'-16: N-[1-[6-[(cyclobutylmethylamino)methyl]imidazo[1,2-a]pyridin-2-yl]propyl]-5-pyrrolidin-1-yl-pyridine-3-carboxamide

A yellow solid compound **E'-16** (7.50 mg, 29.9%) was synthesized from **E16-8** by preparative high-performance liquid chromatography purification using the same method as compound **I-3.**

**LC-MS:** [M+H]⁺= 447.4. **¹H NMR:** (400 MHz, DMSO-*d₆*): δ 9.80 (d, *J ₌* 7.8 Hz, 1H), 9.65 (br s, 2H), 9.04 (s, 1H), 8.51 (d, *J ₌* 14.8 Hz, 2H), 8.20-8.14 (m, 2H), 8.06-7.97 (m, 2H), 5.35-5.28 (m, 1H), 4.25 (t, *J ₌* 5.0 Hz, 2H), 3.43 (br s, 4H), 2.98-2.92 (m, 2H), 2.70 (dd, *J* ₌ 6.9, 14.3 Hz, 1H), 2.16-1.97 (m, 8H), 1.86-1.75 (m, 4H), 1.00 (t, *J ₌* 7.3 Hz, 3H).

The compounds in Table 7 were synthesized and characterized in the same manner as compound E'-16.

**Table 7**

| **Com poun d** | **Name** | **Structure** | **LCMS [M+H]⁺** |
|---|---|---|---|
| E'-16-1 | n-[cyano-[6-[(cyclobutylmethylamino)methyl]imi dazo[1,2-a]pyridin-2-yl]methyl]-5-pyrrolidin-1-yl-pyridin-3-carboxamide | 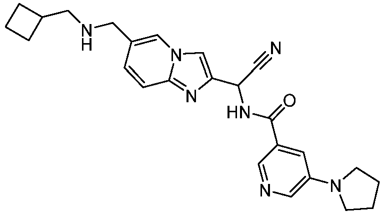 | 444.4 |
| E'-16-2 | n-(1-(6-(((3-fluorobicyclo[1.1.1]pentan-1-yl)methyl)amino)methyl)imidazo[1,2 -a]pyridin-2-yl)ethyl)-5-(pyrrolidin-1-yl)nicotinamide | 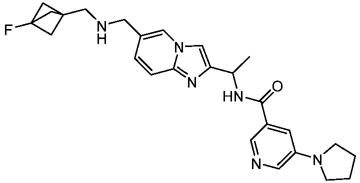 | 463.3 |
| E'-16-2A | | 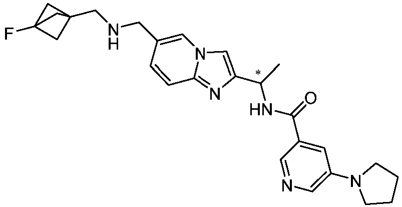 Monomer, SFC retention time Rt=2.083 min, resolution conditions: chromatographic column: CHIRALPAKID3; stationary phase A: MtBE (0.1% DEA):(MeOH:DCM=1:1)=60:40; flow rate: 1.0 mL/min | 463.3 |
| E'-16-2B | | 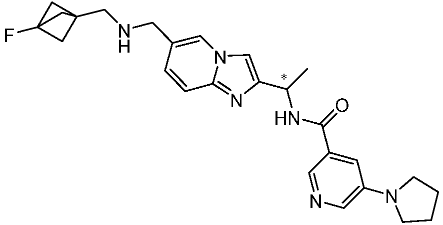 Monomer, SFC retention time Rt=2.436 min, resolution conditions: chromatographic column: CHIRALPAKID3; stationary phase A: MtBE (0.1% DEA):(MeOH:DCM=1:1)=60:40; flow rate: 1.0 mL/min | 463.3 |
| E'-16-3 | 5-(dimethylamino)-N-(1-(6-((((3-fluorobicyclo[1.1.1]pent an-1-yl)methyl)amino)methyl )imidazo[1,2-a]pyridin-2-yl)ethyl)nicotinamide | 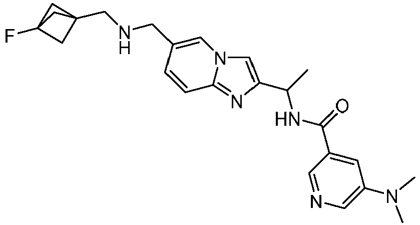 | 437.3 |
| E'-16-3A | | 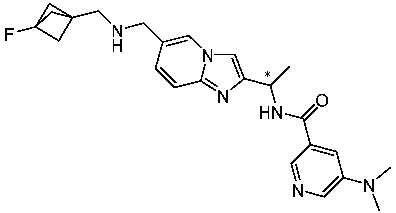 Monomer, SFC retention time Rt=1.683 min, resolution conditions: chromatographic column: (S, S)Whelk01; stationary phase A: (Hex:DCM=1:1) (0.1% DEA):EtOH=60:40; flow rate: 1 mL/min | 437.3 |
| E'-16-3B | | 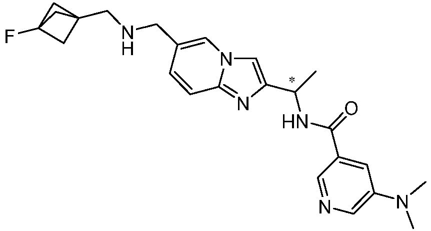 Monomer, SFC retention time Rt=2.586 min, resolution conditions: chromatographic column: (S, S)Whelk01; stationary phase A: (Hex:DCM=1:1) (0.1% DEA):EtOH=60:40; flow rate: 1 mL/min | 437.3 |
| E'-16-4 | N-(1-(6-(((cyclobutylmethyl)ami no)methyl)imidazo[1,2-a]pyridin-2-yl)ethyl)-5-(pyrrolidin-1-yl)nicotinamide | 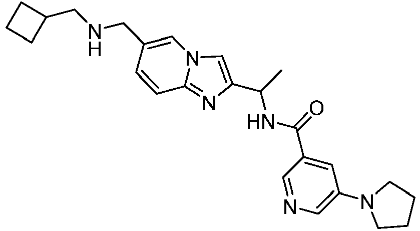 | 433.2 |
| E'-16-5 | N-(1-(6-(((cyclobutylmethyl)ami no)methyl)imidazo[1,2-a]pyridin-2-yl)ethyl)-5-(dimethylamino)nicotin amide | 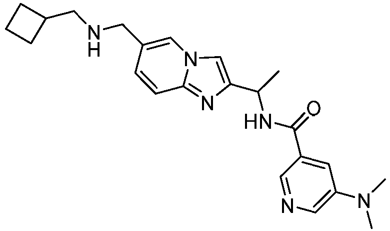 | 407.2 |
| E'-16-6 | n-(1-(6-((((3-fluorobicyclo[1.1.1]pent an-1-yl)methyl)amino)methyl )imidazo[1,2-a]pyridin-2-yl)propyl)-5-(pyrrolidin-1-yl)nicotinamide | 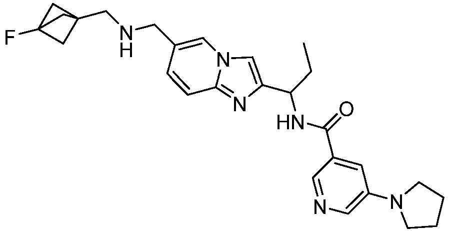 | 477.3 |
| E'-16-6A | | 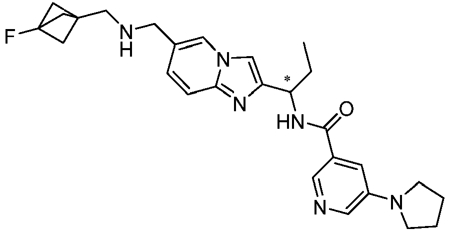 Monomer, SFC resolution | 477.3 |
| E'-16-6B | | 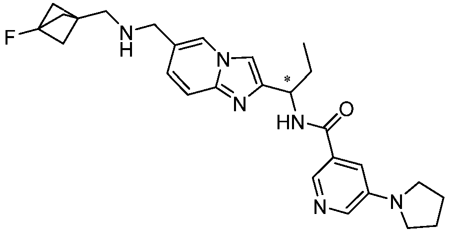 Monomer, SFC resolution | 477.3 |
| E'-16-7 | 5-(dimethylamino)-N-(1-(6-((((3-fluorobicyclo[1.1.1]pent an-1-yl)methyl)amino)methyl )imidazo[1,2-a]pyridin-2-yl)propyl)nicotinamide | 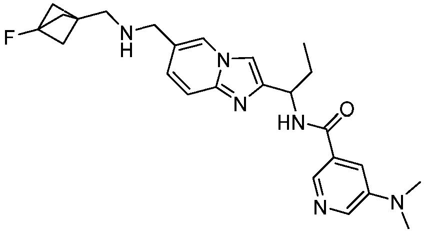 | 451.3 |
| E'-16-7A | | 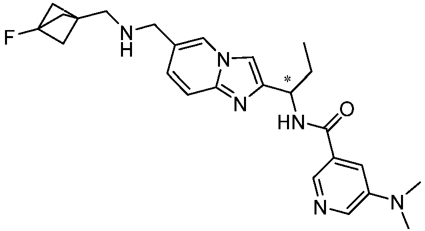 Monomer, SFC resolution | 451.3 |
| E'-16-7B | | 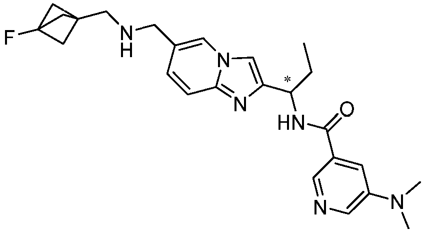 Monomer, SFC resolution | 451.3 |

### Example 17 Compound E'-17: 2-fluoro-N-[[6-[[(3-fluoro-1-bicyclo[1.1.1]pentyl)methylamino]methyl]imidazo[1,2-a]pyridin-2-yl]methyl]-5-pyrrolidin-1-yl-pyridine-3-carboxamide

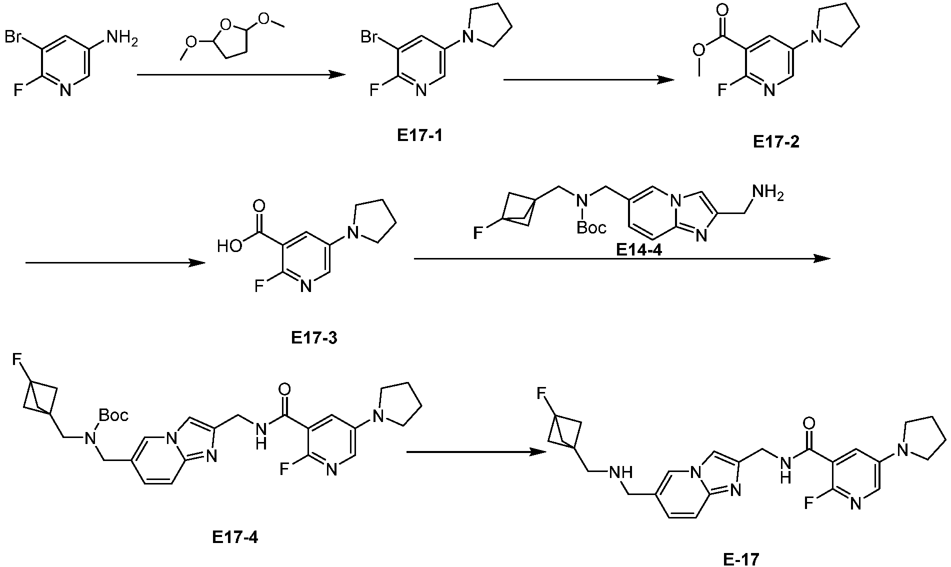

### Compound E17-1: 3-bromo-2-fluoro-5-pyrrolidin-1-yl-pyridine

Sulfuric acid (4.11 g, 41.9 mmol, 2.23 mL) and water (18.1 g, 1.00 mol, 18.1 mL) were added to a solution of 5-bromo-6-fluoro-pyridin-3-amine (2.00 g, 10.5 mmol) in THF (40.00 mL) and MeOH (40.00 mL). Then, 2,5-dimethoxy-tetrahydrofuran (4.15 g, 31.4 mmol, 4.06 mL) and NaBH₄ (1.19 g, 31.5 mmol) were added in batches at 0°C and stirred at 25°C for 16 h. The reaction solution was quenched with water (80.00 mL) at 0°C, and the pH was adjusted to about 8 using a saturated sodium bicarbonate aqueous solution. The product was extracted with ethyl acetate (40.00 mL × 3), and the combined organic layers were washed with a saturated saline solution (30.00 mL), dried over anhydrous sodium sulfate, filtered and concentrated. The crude product was purified by preparative thin-layer chromatography (V_{petroleum ether:ethyl acetate}=20:1) to obtain white solid **E17-1** (0.93 g, 36.4%). LC-MS: [M+H]+=245.

### Compound E17-2: Methyl 2-fluoro-5-pyrrolidin-1-yl-pyridin-3-carboxylate

A brown solid compound **E17-2** (600.0 mg, 79.0%) was synthesized from **E17-1** using the same method as compound **E14-2.**

**LC-MS:** [M+H]⁺=225.

### Compound E17-3: 2-fluoro-5-pyrrolidin-1-yl-pyridin-3-carboxylic acid

A green solid compound **E17-3** (200.0 mg, 71.1%) was synthesized from **E17-2** using the same method as compound **E14-3.**

**LC-MS:** [M+H]⁺=211.

### Compound E17-4: Tert-butyl N-[(3-fluoro-1-bicyclo[1.1.1]pentyl)methyl]-N-[[2-[[(2-fluoro-5-pyrrolidin-1-yl-pyridin-3-carbonyl)amino]methyl]imidazo[1,2-a]pyridin-6-yl]methyl]carbamate

A brown solid compound **E17-4** (25.0 mg, 55.1%) was synthesized from **E17-3** using the same method as compound **E14-5.**

**LC-MS:** [M+H]⁺=567.4.

### Compound E'-17: 2-fluoro-N-[[6-[[(3-fluoro-1-bicyclo[1.1.1]pentyl)methylamino]methyl]imidazo[1,2-a]pyridin-2-yl]methyl]-5-pyrrolidin-1-yl-pyridine-3-carboxamide

A yellow oily compound **E'-17** (12.90 mg, 55.8%) was synthesized from **E17-4** by preparative high-performance liquid chromatography purification using the same method as compound **I-3.**

**LC-MS:** [M+H]⁺= 467.4. **¹H NMR:** (400 MHz, DMSO-*d₆*):5 9.69 (br s, 2H), 9.21-9.13 (m, 1H), 9.05-8.96 (m, 1H), 8.36 (s, 1H), 8.17 (d, *J ₌* 9.4 Hz, 1H), 8.02 (d, *J =* 9.4 Hz, 1H), 7.61-7.55 (m, 1H), 7.37 (dd, *J =* 3.1, 8.0 Hz, 1H), 4.73 (d, *J =* 5.5 Hz, 2H), 4.32-4.28 (m, 2H), 3.32-3.27 (m, 4H), 3.27-3.24 (m, 2H), 2.13 (d, *J* ₌ 2.5 Hz, 6H), 2.01-1.94 (m, 4H).

### Example 18 Compound E'-18: n-[(3-fluoro-1-bicyclo[1.1.1]pentyl)methyl]-1-[2-[[4-[5-[2-(2-methoxyethoxy)ethoxy]-3-pyridyl]triazol-1-yl]methyl]imidazo[1,2-a]pyridin-6-yl]methylamine

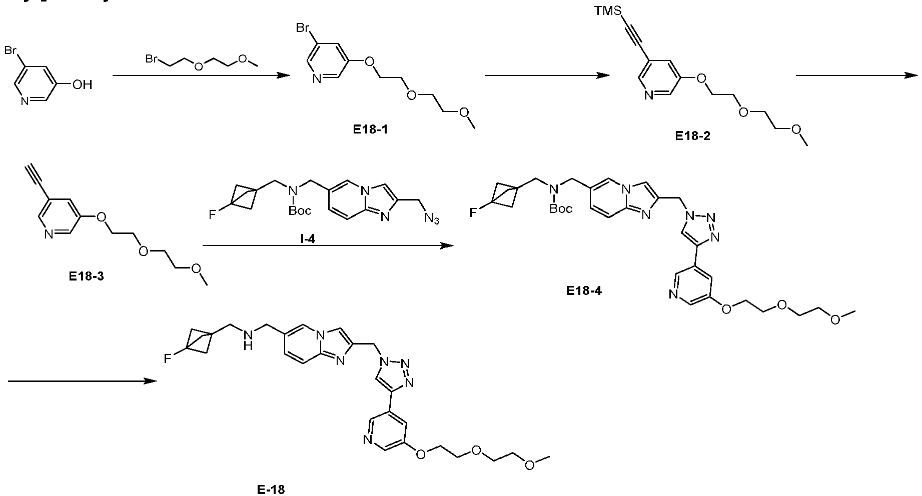

### Compound E18-1: 3-bromo-5-[2-(2-methoxyethoxy)ethoxy]pyridine

A yellow oily compound **E18-1** (1.14 g, 71.8%) was synthesized from 5-bromopyridine-3-ol (1.00 g, 5.75 mmol) using the same method as compound **2-c. LC-MS:** [M+H]⁺=278.

### Compound E18-2: 2-[5-[2-(2-methoxyethoxy)ethoxy]-3-pyridyl]ethynyl-trimethylsilane

A yellow oily compound **E18-2** (190.0 mg, 89.4%) was synthesized from **E18-1** using the same method as compound **2-b.**

**LC-MS:** [M+H]⁺=294.

### Compound E18-3: 3-ethynyl-5-[2-(2-methoxyethoxy)ethoxy]pyridine

A yellow oily compound **E18-3** (190.0 mg, crude product) was synthesized from **E18-2** using the same method as compound **2-c.**

**LC-MS:** [M+H]⁺=222.

### Compound E18-4: Tert-butyl ((3-fluorobicyclo[1.1.1]pentan-1-yl)methyl)((2-((4-(5-(2-(2-methoxyethoxy)ethoxy)pyridin-3-yl)-1H-1,2,3-triazol-1-yl)methyl)imidazo[1,2-a]pyridin-6-yl)methyl)carbamate

A yellow oily compound **E18-4** (190.0 mg, 89.4%) was synthesized from **E18-3** using the same method as compound **2-d.**

**LC-MS:** [M+H]⁺=622.3.

### Compound E'-18: n-[(3-fluoro-1-bicyclo[1.1.1]pentyl)methyl]-1-[2-[[4-[5-[2-(2-methoxyethoxy)ethoxy]-3-pyridyl]triazol-1-yl]methyl]imidazo[1,2-a]pyridin-6-yl]methylamine

A yellow oily compound **E'-18** (16.20 mg, 43.5%) was synthesized from **E18-4** by preparative high-performance liquid chromatography purification using the same method as compound **I-3.**

**LC-MS:** [M+H]⁺= 522.3. **¹H NMR:** (400 MHz, DMSO-*d₆*):*δ* 8.73 (s, 1H), 8.68 (d, *J* = 1.5 Hz, 1H), 8.42 (s, 1H), 8.24 (d, *J ₌* 2.8 Hz, 1H), 7.96 (s, 1H), 7.83-7.79 (m, 1H), 7.46 (d, *J=* 9.3 Hz, 1H), 7.26 (dd, *J ₌* 1.4, 9.3 Hz, 1H), 5.75 (s, 2H), 4.25-4.21 (m, 2H), 3.78-3.75 (m, 2H), 3.67 (s, 2H), 3.59 (dd, *J* ₌ 3.8, 5.6 Hz, 2H), 3.45 (dd, *J* ₌ 3.8, 5.6 Hz, 2H), 3.23 (s, 3H), 2.74 (s, 2H), 1.93 (d, *J =* 2.8 Hz, 6H).

### Example 19 Compound E'-19: n-[(3-fluoro-1-bicyclo[1.1.1]pentyl)methyl]-1-[2-[[4-(5-pyrrolidin-1-yl-3-pyridyl)imidazol-1-yl]methyl]imidazo[1,2-a]pyridin-6-yl]methylamine

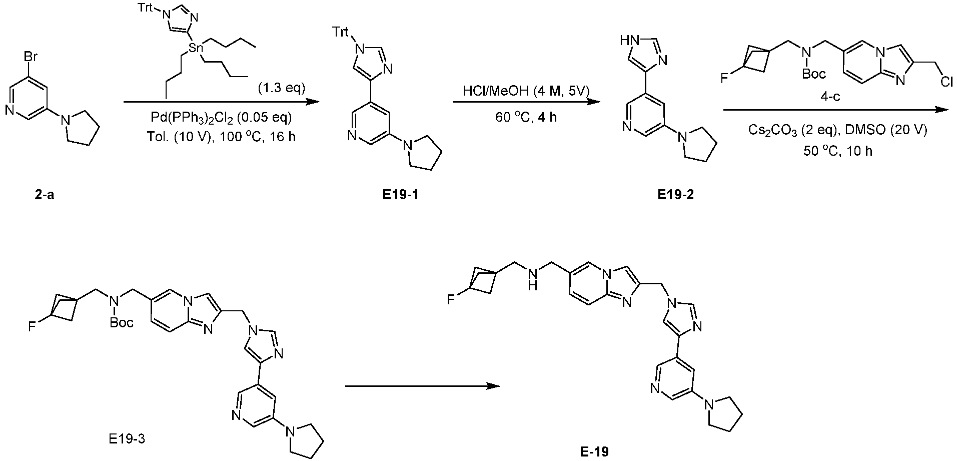

### Compound E19-1: 3-(pyrrolidin-1-yl)-5-(1-triphenylmethyl-1H-imidazol-4-yl)pyridine

Palladium bis(triphenylphosphine) dichloride (46.4 mg, 66.1 µmol) was added to a solution of **2-a** (300 mg, 1.32 mmol) and tributyl-(1-triphenylmethylimidazol-4-yl)stannane (1.03 g, 1.72 mmol) in toluene (3.00 mL) at 25°C. The resulting mixture was stirred at 100°C for 16 h under nitrogen protection. The reaction solution was cooled to room temperature, concentrated under reduced pressure, diluted with water (10 mL) and extracted with ethyl acetate (5 mL * 3). The combined organic layers were washed with a saturated saline solution, dried over anhydrous sodium sulfate, filtered, and concentrated under reduced pressure to obtain a crude product. The crude product was purified by preparative thin-layer chromatography (ethyl acetate) to obtain yellow solid **E19-1** (200.0 mg, 29.8%).

**LC-MS:** [M+H]⁺=457.3.

### Compound E19-2: 3-(1H-imidazol-4-yl)-5-(pyrrolidin-1-yl)pyridine

A solution of **E19-1**(180 mg, 394 µmol) in hydrochloric acid/methanol (4 M, 1.00 mL) was stirred at 60°C for 4 h. The reaction solution was cooled to room temperature and concentrated under pressure to obtain a brown solid **E19-2** (400 mg, crude product).

**LC-MS:** [M+H]⁺=215.2.

### Compound E19-3: Tert-butyl ((3-fluorobicyclo[1.1.1]pentan-1-yl)methyl)((2-((4-(5-(pyrrolidin-1-yl)pyridin-3-yl)-1H-imidazol-1-yl)methyl)imidazo[1,2-a]pyridin-6-yl)methyl)carbamate

A yellow oily compound **E19-3** (190.0 mg, crude product) was synthesized from **E19-2** using the same method as compound **E6-1.**

**LC-MS:** [M+H]⁺=572.2.

### Compound E'-19: n-[(3-fluoro-1-bicyclo[1.1.1]pentyl)methyl]-1-[2-[[4-(5-pyrrolidin-1-yl-3-pyridyl)imidazol-1-yl]methyl]imidazo[1,2-a]pyridin-6-yl]methylamine

A yellow oily compound **E'-19** (13.30 mg, 25.5%) was synthesized from **E19-3** by preparative high-performance liquid chromatography purification using the same method as compound **I-3.**

**LC-MS:** [M+H]⁺= 472.3. **¹H NMR:** (400 MHz, DMSO-*d₆*): *δ* 8.41 (s, 1H), 8.22 (d, *J* = 1.6 Hz, 1H), 7.88-7.79 (m, 2H), 7.79-7.74 (m, 2H), 7.46 (d, *J ₌* 9.3 Hz, 1H), 7.25 (dd, *J* ₌ 1.6, 9.3 Hz, 1H), 7.21-7.18 (m, 1H), 5.31 (s, 2H), 3.67 (s, 2H), 3.28 (t, *J ₌* 6.6 Hz, 4H), 2.74 (s, 2H), 1.99-1.95 (m, 4H), 1.93 (d, *J* ₌ 2.8 Hz, 6H).

### Example 20 Compound E'-20: n-[(3-fluoro-1-bicyclo[1.1.1]pentyl)methyl]-1-[2-[[5-(5-pyrrolidin-1-yl-3-pyridyl)-1,3,4-thiadiazol-2-yl]methyl]imidazo[1,2-a]pyridin-6-yl]methylamine

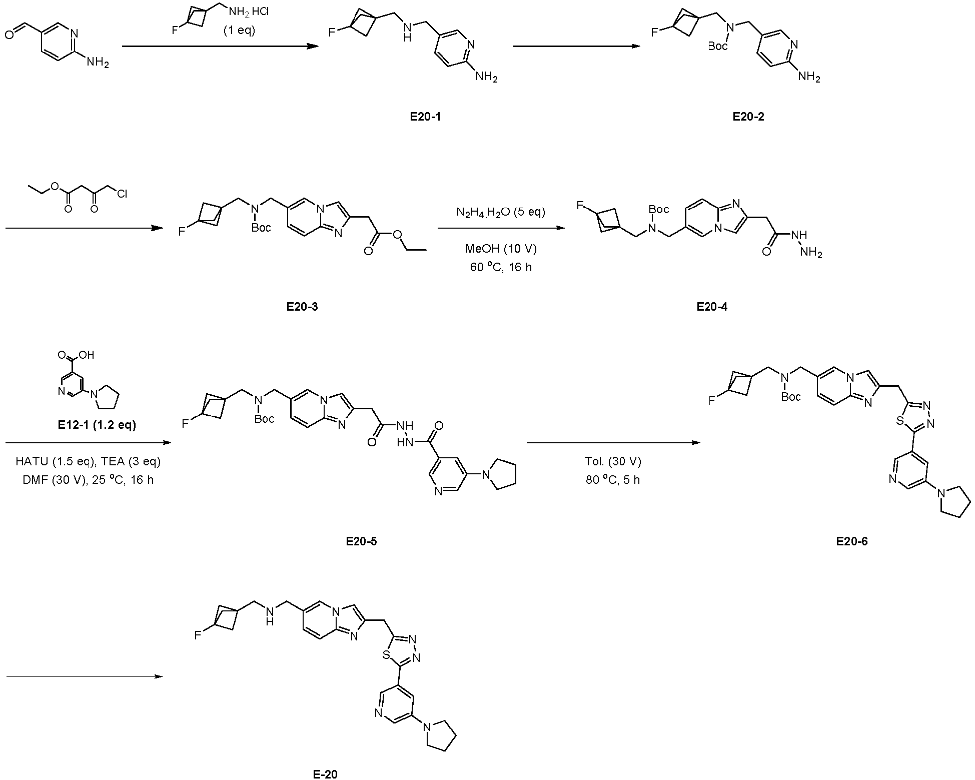

### Compound E20-1: 5-((((3-fluorobicyclo[1.1.1]pentan-1-yl)methyl)amino)methyl)pyridine-2-amine

A white solid compound **E20-1** (700.0 mg, crude product) was synthesized from 6-aminonicotinaldehyde using the same method as compound **E'-2. LC-MS:** [M+H]⁺=222.1.

### Compound E20-2: Tert-butyl N-[(6-amino-3-pyridyl)methyl]-N-[(3-fluoro-1-bicyclo[1.1.1]pentyl)methyl]carbamate

A colorless oily compound **E20-2** (400.0 mg, 39.3%) was synthesized from **E20-1** using the same method as compound **4-c.**

**LC-MS:** [M+H]⁺=322.1.

### Compound E20-3: Ethyl 2-[6-[[tert-butoxycarbonyl-[(3-fluoro-1-bicyclo[1.1.1]pentyl)methyl]amino]methyl]imidazo[1,2-a]pyridin-2-yl]acetate

A yellow oily compound **E20-3** (400.0 mg, 74.5%) was synthesized from **E20-2** using the same method as compound **1-a.**

**LC-MS:** [M+H]⁺=432.2.

### Compound E20-4: Tert-butyl N-[(3-fluoro-1-bicyclo[1.1.1]pentyl)methyl]-N-[[2-(2-hydrazino-2-oxo-ethyl)imidazo[1,2-a]pyridin-6-yl]methyl]carbamate

Hydrazine hydrate (118 mg, 2.32 mmol, 115 µL) was added to a solution of **E20-**3 (200 mg, 464 µmol) in methanol (2.00 mL) at 25°C. The resulting mixture was stirred at 60°C for 16 h. The reaction solution was cooled to room temperature and concentrated under reduced pressure to obtain yellow solid **E20-4** (190.0 mg, crude product).

**LC-MS:** [M+H]⁺=418.2.

### Compound E20-5: Tert-butyl N-[(3-fluoro-1-bicyclo[1.1.1]pentyl)methyl]-N-[[2-[2-oxo-2-[2-(5-pyrrolidin-1-yl pyridin-3-carbonyl)hydrazino]ethyl]imidazo[1,2-a]pyridin-6-yl]methyl]carbamate

Under nitrogen protection at 25°C, tert-butyl (cyclobutylmethyl)((2-(2-hydrazino-2-oxoethyl)imidazo[1,2-a]pyridin-6-yl)methyl)carbamate (71.9 mg, 179 µmol) and triethylamine (36.4 mg, 359 µmol) were added to a solution of **E20-4** (50.0 mg, 119 µmol) and **E12-1** (27.6 mg, 144 µmol) in N,N-dimethylformamide (1.5 mL). The resulting mixture was stirred at 25°C for 16 h under nitrogen protection. The reaction solution was quenched with a saturated sodium bicarbonate solution (20 mL) and extracted with EtOAc (4 x 5 mL). The combined organic layers were washed with a saturated saline solution (5 mL), dried over anhydrous sodium sulfate, filtered, and concentrated under reduced pressure. The crude product was purified by preparative high-performance liquid chromatography to obtain yellow solid **E20-5** (40 mg, 56.5%).

LC-MS: [M+H]⁺=592.2.

### Compound E20-6: Tert-butyl N-[(3-fluoro-1-bicyclo[1.1.1]pentyl)methyl]-N-[[2-[[5-(5-pyrrolidin-1-yl-3-pyridyl)-1,3,4-thiadiazol-2-yl]methyl]imidazo[1,2-a]pyridin-6-yl]methyl]carbamate

A Lawesson reagent (32.4 mg, 76.1 µmol) was added to a solution of **E20-5** (30 mg, 50.7 µmol) in toluene (0.09 mL) at 25°C. The resulting mixture was stirred at 80°C for 5 h under nitrogen protection. The reaction solution was concentrated under reduced pressure, and the crude product was purified by preparative high-performance liquid chromatography to obtain yellow solid **E20-6** (20 mg, 63.0%).

LC-MS: [M+H]⁺=590.3.

### Compound E'-20: n-[(3-fluoro-1-bicyclo[1.1.1]pentyl)methyl]-1-[2-[[5-(5-pyrrolidin-1-yl-3-pyridyl)-1,3,4-thiadiazol-2-yl]methyl]imidazo[1,2-a]pyridin-6-yl]methylamine

A white solid compound **E'-20** (9.10 mg, 48.5%) was synthesized from **E20-6 by** preparative high-performance liquid chromatography purification using the same method as compound **I-3.**

**LC-MS:** [M+H]⁺= 490.4. **¹H NMR:** (400 MHz, DMSO-*d₆*): *δ* 9.78 (br s, 2H), 9.03 (s, 1H), 8.46 (s, 1H), 8.41 (s, 1H), 8.17 (d, *J* = 2.5 Hz, 1H), 8.10 (d, *J* ₌ 9.0 Hz, 1H), 7.96 (d, *J =* 9.3 Hz, 1H), 7.67 (br s, 1H), 4.94 (s, 2H), 4.28 (br s, 2H), 3.39 (d, *J ₌* 7.4 Hz, 4H), 3.29 (d, *J=* 4.8 Hz, 2H), 2.13 (d, *J =* 2.4 Hz, 6H), 2.00 (t, *J ₌* 6.5 Hz, 4H).

### Example 21: Synthesis of E'-122: 1-(3-fluorobicyclo[1.1.1]pentan-1-yl)-N-((2-((4-(5-(pyrrolidin-1-yl)pyridin-3-yl)-1H-1,2,3-triazol-1-yl)methyl)imidazo[1,2-a]pyridin-6-yl)methyl)methylamine

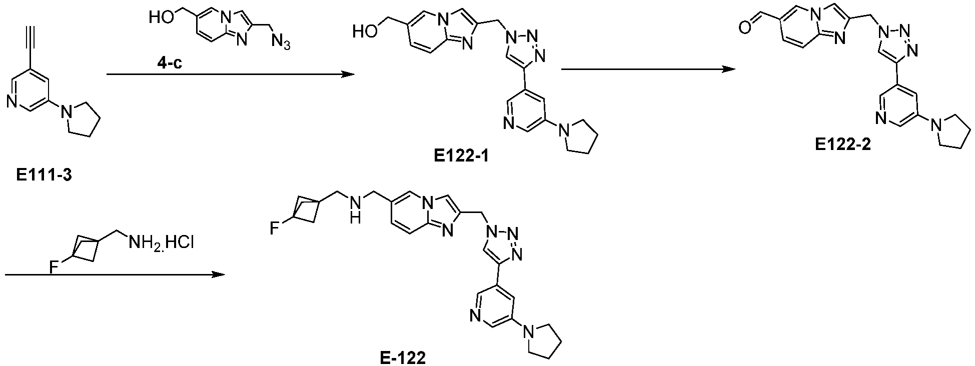

A pink solid compound **E'-122** (11.3 mg, 14.8%) was prepared by referring to the method described in Example 3.

LC-MS (ESI): m/z 473.2 [M+H]⁺ . ¹H NMR (400 MHz, DMSO-*d₆*): *δ* 8.67 (s, 1H), 8.42 (s, 1H), 8.30 (d, *J =* 1.4 Hz, 1H), 7.95 (s, 1H), 7.88 (d, *J =* 2.6 Hz, 1H), 7.46 (d, *J =* 9.1 Hz, 1H), 7.29 (d, *J =* 1.6 Hz, 1H), 7.27-7.23 (m, 1H), 5.73 (s, 2H), 3.67 (s, 2H), 3.31-3.28 (m, 4H), 2.74 (s, 2H), 1.97 (t, *J ₌* 6.4 Hz, 4H), 1.93 (d, *J =* 2.8 Hz, 6H).

### Test Example 2

### Treatment of MOLM-13 cells with METTL3 inhibitor

MOLM-13 cell suspension was spread in a 96-well cell culture plate (tissue culture medium RPMI 1640+10% FBS), with a final concentration of 50000/mL and a volume of 100 µL per well. After overnight culture in a cell incubator, an METTL3 inhibitor was dissolved in DMSO, with an initial concentration of 50 mM, and subjected to triple gradient dilution in a 384-well storage plate. The inhibitor in the storage plate was pumped into a 96-well cell culture plate with an Echo instrument. The concentration of the compound ranged from 50 µM to 0.01 µM, and the volume was 100 nL/well. The final DMSO concentration was 0.1%, diluted by triple gradient, with 9 concentration gradients and diplopore. The cells were incubated in a cell incubator at 37°C for 72 h together with the compound. 100 µL/well of Cell Titer-Glo Luminescent Cell Viability Assay reagent was added and mixed well, and the obtained mixture was incubated at room temperature for 10 min and read with Envision. The sample treated with a 50 µM positive compound was used as positive control, and the sample treated with 100 nL DMSO was used as negative control. Calculation formula: (sample reading-positive control reading)/(negative control reading-positive control reading)*100%. IC₅₀ was calculated.

The IC₅₀ values of some of the compounds provided by the present invention are shown in Table A below:

**Table A**

| **Compound** | **MOLM13 IC₅₀ (µM)** | **Compound** | **MOLM13 IC₅₀ (µM)** |
|---|---|---|---|
| E'-1 | 0.0756 | E'-4-11 | 0.5984 |
| E'-2 | 0.5105 | E'-4-12 | 3.0591 |
| E'-2-1 | 0.4268 | E'-5 | 2.9976 |
| E'-2-2 | 0.6113 | E'-5-1 | 26.7873 |
| E'-2-3 | 0.7571 | E'-6 | 0.2234 |
| E'-2-4 | 0.9755 | E'-6-1 | 0.0607 |
| E'-2-5 | 0.1355 | E'-6-2 | 0.8509 |
| E'-2-6 | 0.6238 | E'-6-3 | 0.2807 |
| E'-2-7 | 1.0546 | E'-6-4 | 0.3850 |
| E'-2-8 | 0.6050 | E'-6-5 | 8.1411 |
| E'-2-9 | 2.9446 | E'-6-6 | 1.2289 |
| E'-2-10 | 2.6972 | E'-6-7 | 0.0702 |
| E'-2-11 | 8.6017 | E'-6-8 | 0.7745 |
| E'-2-12 | 3.7840 | E'-6-9 | 0.1963 |
| E'-2-13 | 3.4291 | E'-6-10 | 5.2705 |
| E'-2-14 | 1.9217 | E'-6-11 | 0.4705 |
| E'-2-15 | 2.2262 | E'-6-12 | 5.1350 |
| E'-2-16 | 3.2284 | E'-6-13 | 21.0744 |
| E'-2-17 | 4.4289 | E'-7 | 0.8073 |
| E'-2-18 | 5.2586 | E'-8 | 1.2323 |
| E'-2-19 | 4.5240 | E'-9 | 3.6926 |
| E'-2-20 | 3.7400 | E'-10 | 0.7680 |
| E'-2-21 | 15.3695 | E'-10-1 | 0.1955 |
| E'-2-22 | 14.3591 | E'-10-2 | 0.0888 |
| E'-2-23 | 5.1427 | E'-10-3 | 5.4195 |
| E'-2-24 | 1.3842 | E'-10-4 | 0.2015 |
| E'-2-25 | 2.1633 | E'-10-5 | 6.3284 |
| E'-2-26 | 5.0751 | E'-11 | >50.0 |
| E'-3 | 10.0702 | E'-12 | 1.0837 |
| E'-4 | 0.1824 | E'-13 | 0.5974 |
| E'-4-1 | 0.1447 | E'-14 | 56.3887 |
| E'-4-2 | 0.7546 | E'-15 | 0.3763 |
| E'-4-3 | 3.8553 | E'-16 | 1.4999 |
| E'-4-4 | 2.6910 | E'-16-1 | 1.4028 |
| E'-4-5 | 0.3921 | E'-16-2 | 0.3060 |
| E'-4-6 | 2.0417 | E'-17 | 4.5582 |
| E'-4-7 | 0.7904 | E'-18 | 14.2300 |
| E'-4-8 | 1.2074 | E'-19 | 0.1059 |
| E'-4-9 | 0.9036 | E'-20 | 0.0473 |
| E'-4-10 | 1.2277 | E'-16-2A | 0.0949 |
| E'-16-3 | 1.3751 | E'-16-2B | 11.3854 |
| E'-16-3A | 69.9611 | E'-16-6A | 37.6154 |
| E'-16-3B | 0.5352 | E'-16-6B | 0.0582 |
| E'-16-4 | 1.7185 | E'-16-7 | 0.7372 |
| E'-16-5 | 4.1281 | E'-16-7A | 0.3473 |
| E'-16-6 | 0.1291 | E'-16-7B | 17.0116 |

The present invention also tested the activity of the following compound under the same conditions as described above, and the result is shown in Table B below:

**Table B**

| Compound structure | MOLM 13 IC₅₀ (µM) |
|---|---|
| 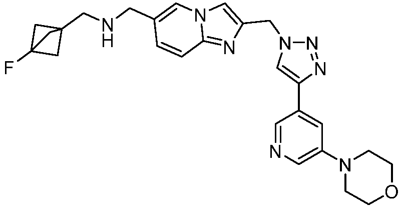 TC006-RC019 | 3.13 |

From the above results, it can be seen that some compounds provided by the present invention have significantly higher activity as METTL3 inhibitors than the compounds listed in Table B.

The embodiments of the present invention have been described above. However, the present invention is not limited to the above embodiments. Any modification, equivalent replacement, improvement and the like made within the spirit and principle of the present invention shall be included in the protection scope of the present invention.

## Claims

1. A compound shown in formula I, formula I' or formula I", and tautomers, stereoisomers, or pharmaceutically acceptable salts of the three compounds: 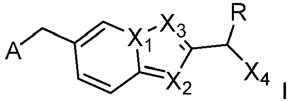
wherein A is selected from the following groups unsubstituted or optionally substituted by one, two or more Ra: -NH-(C₁₋₁₂alkyl)-C₃₋₂₀cycloalkyl, 3-20 membered heterocyclic groups, -3-20 membered heterocyclic groups-3-20 membered heterocyclic groups, -5-20 membered heteroaryl-5-20 membered heteroaryl, -NH-(C₁₋₁₂alkyl)-3-20 membered heterocyclic groups, C₃₋₂₀cycloalkyl or -N(Z)CO-5-20 membered heteroaryl;
X₁, X₂, and X₃ are the same or different, independently selected from C, O, or N, provided that X₁, X₂, and X₃ are not simultaneously heteroatoms;
X₄ is selected from the following groups unsubstituted or optionally substituted by one, two or more Rb: -5-20 membered heteroaryl-5-20 membered heteroaryl, -5-20 membered heteroaryl-C₆₋₂₀aryl, -N(Z)CO-5-20 membered heteroaryl, -CON(Z)-5-20 membered heteroaryl, - 5-20 membered heteroaryl-CO-C₁₋₁₂alkyl, -NH-(C₁₋₁₂alkyl)-C₃₋₂₀cycloalkyl, 3-20 membered heterocyclic groups or -COO-5-20 membered heteroaryl;
R is selected from H or C₁₋₁₂alkyl;
Ra is selected from nitro, amino, halogen, -CH₂N(C₁₋₁₂alkyl)₂, -N(C₁₋₁₂alkyl)₂, -O-(C₁₋₁₂alkyl-O)₂-C₁₋₁₂alkyl, 3-20 membered heterocyclic groups, C₁₋₁₂alkyl substituted 3-20 membered heterocyclic groups, -Se-C₁₋₁₂alkyl, -S-C₁₋₁₂alkyl, 5-20 membered heteroaryl, C₁₋₁₂alkoxy or C₁₋₁₂alkyl;
Rb is selected from H, halogen, NO₂, -CHO, OH, NH₂, =O, and the following groups unsubstituted or optionally substituted by one, two or more Rd: -S-C₁₋₁₂alkyl, -NH-C₁₋₁₂alkyl, - N(C₁₋₁₂alkyl)₂, 3-20 membered heterocyclic groups, C₁₋₁₂alkoxy, -O-(C₁₋₁₂alkyl-O)₂-C₁₋₁₂alkyl, - S(O)₂-C₁₋₁₂alkyl, -Se-C₁₋₁₂alkyl, -N(C₁₋₁₂alkyl-OH)₂, -N(C₁₋₁₂alkyl-Cl)₂, C₁₋₁₂alkyl, -NHC(O)C₁₋₁₂alkyl, -N(Z)CO-5-20 membered heteroaryl, -OC(O)C₁₋₁₂alkyl, -N(CD₃)₂, and 5-20 membered heteroaryl;
Z is selected from H or C₁₋₁₂alkyl;
Rd is selected from halogen, cyano, C₁₋₁₂alkyl, -S-C₁₋₁₂alkyl, -NH-C₁₋₁₂alkyl, -N(C₁₋₁₂alkyl)₂; 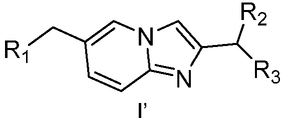
in formula I', R₁ is selected from the following groups: -NH-(C₁₋₁₂alkyl)-bicyclo[1.1.1]pentane-(Ra)n, -NH-(C₁₋₁₂alkyl)-C₆₋₂₀aryl-(Ra)n, -NH-(C₁₋₁₂alkyl)-(Ra)n, -NH-(C₁₋₁₂alkyl)-C₃₋₂₀cycloalkyl-(Ra)n, -NH-(C₁₋₁₂alkyl)-3-20 membered heterocyclic groups-(Ra)n, -3-20 membered heterocyclic groups-(Ra)n, -NH-(C₁₋₁₂alkyl)-5-20 membered heteroaryl-(Ra)n, -NH-(C₁₋₁₂alkyl)-bicyclo[2.2.2]octane-(Ra)n, and -NH-(C₁₋₁₂alkyl)-cubane-(Ra)n;
Ra is selected from H, OH, halogen, halogenated C₁₋₁₂alkyl, C₁₋₁₂alkyl, CN or C₂₋₁₂alkynyl;
n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₁₂alkyl, or CN;
R₃ is selected from -5-20 membered heteroaryl-Rb, -OC(O)-5-20 membered heteroaryl-Rb or -NC(O)-5-20 membered heteroaryl-Rb;
Rb is selected from 5-20 membered heteroaryl-Rc, 3-20 membered heterocyclic groups or halogen;
m is 1, 2 or 3;
Rc is selected from -3-20 membered heterocyclic groups-Rd, -5-20 membered heteroaryl-Rd, -O(C₁₋₁₂alkyl-O)m-C₁₋₁₂alkyl;
Rd is selected from H, deuterium, halogen OH, CN, halogenated C₁₋₁₂alkyl, C₁₋₁₂alkyl or =O; 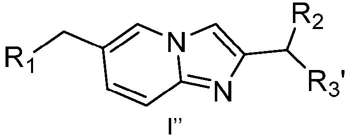
in formula I", R₁ is selected from the following groups: -NH-(C₁₋₁₂alkyl)-bicyclo[1.1.1]pentane-(Ra)n, -NH-(C₁₋₁₂alkyl)-C₆₋₂₀aryl-(Ra)n, -NH-(C₁₋₁₂alkyl)-(Ra)n, -NH-(C₁₋₁₂alkyl)-C₃₋₂₀cycloalkyl-(Ra)n, -NH-(C₁₋₁₂alkyl)-3-20 membered heterocyclic groups-(Ra)n, -3-20 membered heterocyclic groups-(Ra)n, -NH-(C₁₋₁₂alkyl)-5-20 membered heteroaryl-(Ra)n, -NH-(C₁₋₁₂alkyl)-bicyclo[2.2.2]octane-(Ra)n, and -NH-(C₁₋₁₂alkyl)-cubane-(Ra)n;
Ra is selected from H, OH, halogen, halogenated C₁₋₁₂alkyl, C₁₋₁₂alkyl, CN or C₂₋₁₂alkynyl; n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₁₂alkyl, or CN; and
R₃ is selected from -OC(O)-5-12 membered heteroaryl-Rm or -NC(O)-5-12 membered heteroaryl-Rm, and Rm is selected from -NH-(C₁₋₁₂alkyl), -N(C₁₋₁₂alkyl)₂ or 3-12 membered heterocyclic groups.

2. The compound according to claim 1, **characterized in that** in formula I, A is selected from the following groups unsubstituted or optionally substituted by one, two or more Ra: -NH-(C₁₋₆alkyl)-C₃₋₁₂cycloalkyl, 3-12 membered heterocyclic groups, -3-12 membered heterocyclic groups-3-12 membered heterocyclic groups, -5-12 membered heteroaryl-5-12 membered heteroaryl, -NH-(C₁₋₆alkyl)-3-12 membered heterocyclic groups, C₃₋₁₂cycloalkyl or -N(Z)CO-5-12 membered heteroaryl;
X₁, X₂, and X₃ are the same or different, independently selected from C, O, or N, provided that X₁, X₂, and X₃ are not simultaneously heteroatoms;
X₄ is selected from the following groups unsubstituted or optionally substituted by one, two or more Rb: -5-12 membered heteroaryl-5-12 membered heteroaryl, -5-12 membered heteroaryl-C₆₋₁₂ aryl, -N(Z)CO-5-12 membered heteroaryl, -CON(Z)-5-12 membered heteroaryl, -5-12 membered heteroaryl-CO-C₁₋₆alkyl, -NH-(C₁₋₃alkyl)-C₃₋₁₂cycloalkyl, 3-12 membered heterocyclic groups or -COO-5-12 membered heteroaryl;
R is selected from H or C₁₋₆alkyl;
Ra is selected from NO₂, NH₂, halogen, -CH₂N(C₁₋₃alkyl)₂, -N(C₁₋₆alkyl)₂, -O-(C₁₋₆alkyl-O)₂-C₁₋₆alkyl, 3-12 membered heterocyclic groups, C₁₋₆alkyl substituted 3-12 membered heterocyclic groups, -Se-C₁₋₆alkyl, -S-C₁₋₆alkyl, 5-12 membered heteroaryl, C₁₋₆alkoxy or C₁₋₆alkyl;
Rb is selected from H, halogen, NO₂, -CHO, OH, NH₂, =O, and the following groups unsubstituted or optionally substituted by one, two or more Rd: -S-C₁₋₆alkyl, -NH-C₁₋₆alkyl, -N(C₁₋₆alkyl)₂, 3-12 membered heterocyclic groups, C₁₋₆alkoxy, -O-(C₁₋₆alkyl-O)₂-C₁₋₆alkyl, -S(O)₂-C₁₋₆alkyl, -Se-C₁₋₆alkyl, -N(C₁₋₆alkyl-OH)₂, -N(C₁₋₆alkyl-Cl)₂, C₁₋₆alkyl, -NHC(O)C₁₋₆alkyl, -N(Z)CO-5-12 membered heteroaryl, -OC(O)C₁₋₆alkyl, -N(CD₃)₂, and 5-12 membered heteroaryl;
Z is selected from H or C₁₋₆alkyl;
Rd is selected from halogen, CN, C₁₋₆alkyl, -S-C₁₋₆alkyl, -NH-C₁₋₆alkyl, -N(C₁₋₆alkyl)₂;
preferably, in formula I', R₁ is selected from the following groups: -NH-(C₁₋₃alkyl)-bicyclo[1.1.1]pentane-(Ra)n, -NH-(C₁₋₃alkyl)-C₆₋₁₂ aryl-(Ra)n, -NH-(C₁₋₃alkyl)-(Ra)n, -NH-(C₁₋₃alkyl)-C₃₋₁₂cycloalkyl-(Ra)n, -NH-(C₁₋₃alkyl)-3-12 membered heterocyclic groups-(Ra)n, -3-12 membered heterocyclic groups-(Ra)n, -NH-(C₁₋₃alkyl)-5-12 membered heteroaryl-(Ra)n, -NH-(C₁₋₃alkyl)-bicyclo[2.2.2]octane-(Ra)n, and -NH-(C₁₋₃alkyl)-cubane-(Ra)n;
Ra is selected from H, OH, halogen, halogenated C₁₋₃alkyl, C₁₋₃alkyl, CN or C₂₋₆alkynyl;
n is the number of Ra substituents, selected from 1, 2, 3, 4 or 5;
Ra in -NH-(C₁₋₃alkyl)-C₃₋₁₂cycloalkyl-(Ra)n is optionally substituted on C₃₋₁₂cycloalkyl or on carbon atoms where C₃₋₁₂cycloalkyl are attached to C₁₋₃alkyl;
R₂ is selected from H, C₁₋₃alkyl, or CN;
R₃ is selected from -5-12 membered heteroaryl-Rb, -OC(O)-5-12 membered heteroaryl-Rb or -NC(O)-5-12 membered heteroaryl-Rb;
Rb is selected from 5-12 membered heteroaryl-Rc, 3-12 membered heterocyclic groups or halogen;
m is 1, 2 or 3;
Rc is selected from -3-20 membered heterocyclic groups-Rd, -5-12 membered heteroaryl-Rd, or -O(C₁₋₃alkyl-O)m-C₁₋₃alkyl;
Rd is selected from H, deuterium, halogen OH, CN, halogenated C₁₋₃alkyl, C₁₋₃alkyl or =O; and
preferably, in formula I", R₁ is selected from 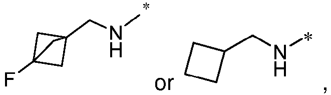
R₂ is selected from C₁₋₆alkyl, R₃ is selected from -OC(O)-5-6 membered heteroaryl-Rm or -NC(O)-5-6 membered heteroaryl-Rm, and Rm is selected from -NH-(C₁₋₃alkyl), -N(C₁₋₃alkyl)₂ or 3-6 membered heterocyclic groups.

3. The compound according to claim 1 or 2, **characterized in that** in formula I, A is selected from 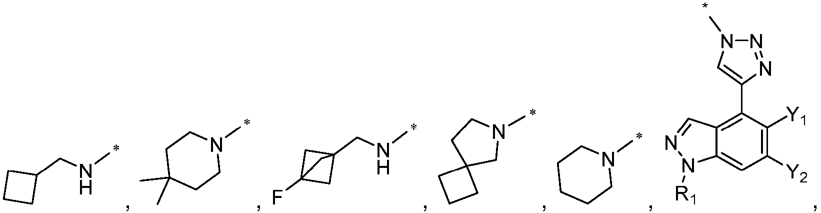 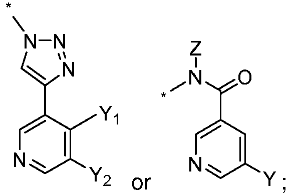
X₄ is selected from 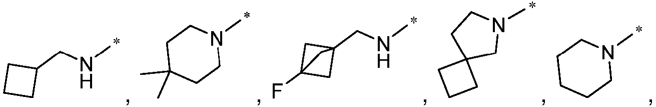 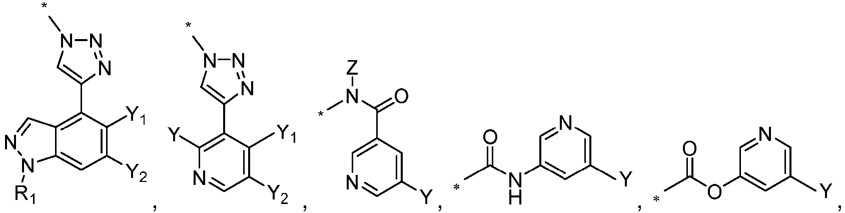 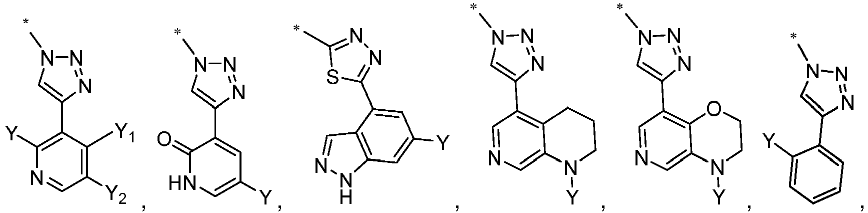 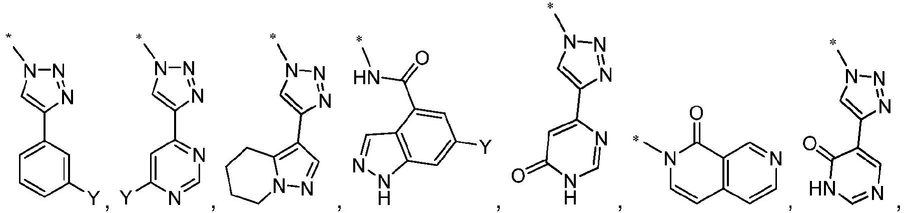 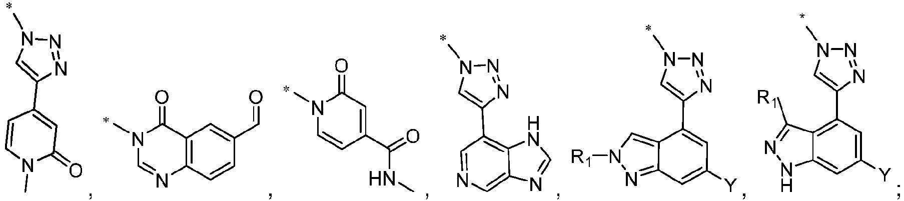
Y, Y₁ and Y₂ are the same or different, independently selected from H, NH₂, CN, CHO, OH, =O, -S-C₁₋₆alkyl, **I,** NH₂, -NH-C₁₋₆alkyl, -N(C₁₋₆alkyl)₂, 3-6 membered heterocyclic groups, Cl, Br, C₁₋₆alkoxy, -O-(C₁₋₆alkyl-O)₂-C₁₋₆alkyl, -S(O)₂-C₁₋₆alkyl, -Se-C₁₋₆alkyl, -N(C₁₋₆alkyl-OH)₂, -N(C₁₋₆alkyl-Cl)₂, C₁₋₆alkyl, -NHC(O)C₁₋₆alkyl, -OC(O)C₁₋₆alkyl, -CHO, OH, -N(CD₃)₂, 5-6 membered heteroaryl, NO₂, F; the 3-6 membered heterocyclic groups or 5-6 membered heteroaryl is optionally substituted with one, two, or three groups selected from F, Cl, Br, and I;
R₁ and R₂ are the same or different, independently selected from H, F or C₁₋₆alkyl;
Z is the same or different, independently selected from H or C₁₋₆alkyl;
preferably, in formula I', R₁ is selected from the following groups: 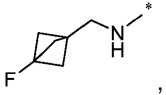 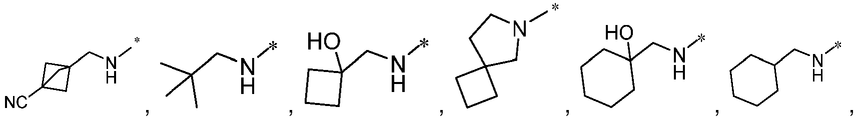 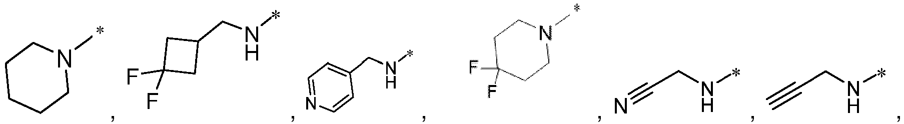 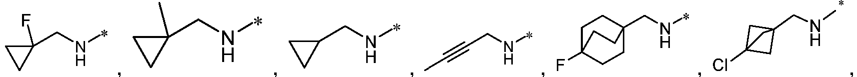 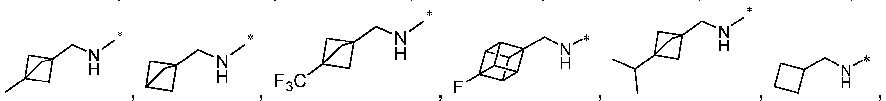 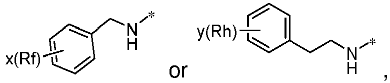
wherein Rf and Rh are the same or different, independently selected from H, F, Cl, methyl, trifluoromethyl, or CN; m and n are the same or different, independently selected from 1, 2, 3, 4, or 5;
in formula I', 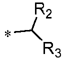
is selected from the following groups: 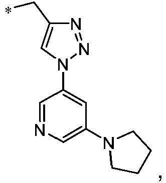 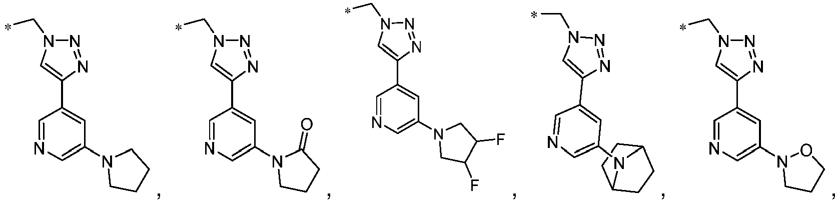 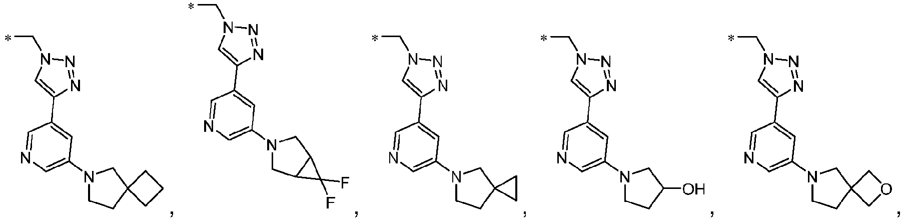 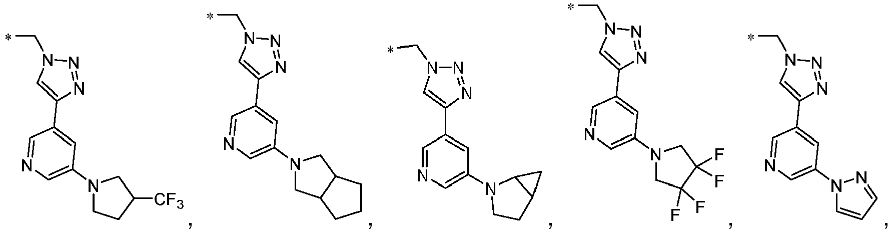 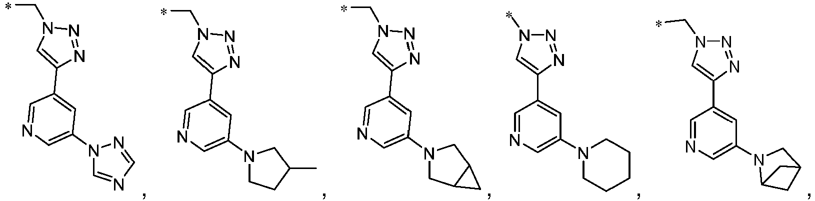 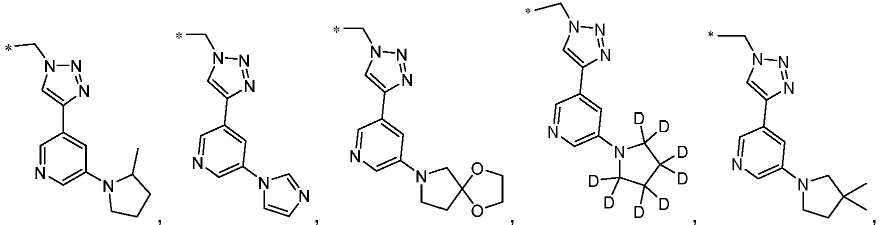 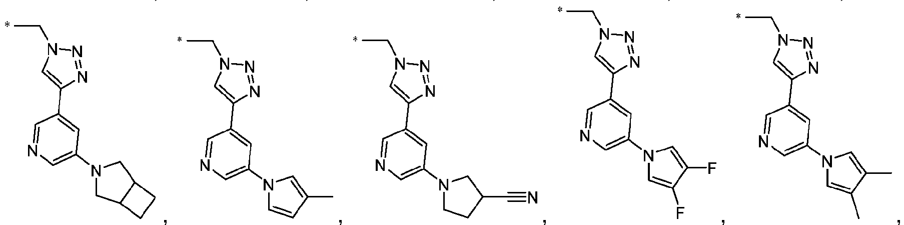 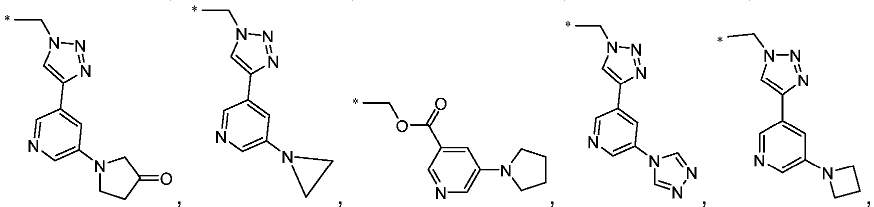 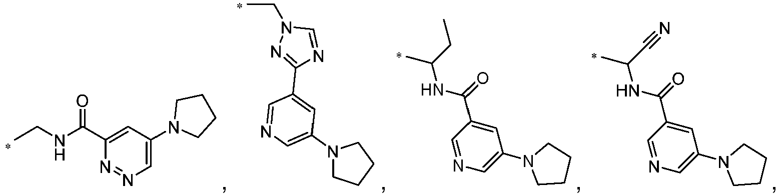 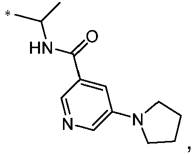 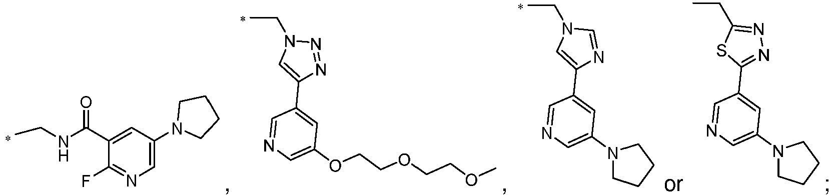
preferably, in formula I", R₁ is selected from 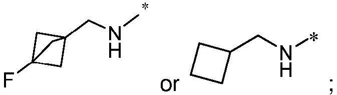 R2 is selected from C₁₋₃alkyl; R₃ is selected from

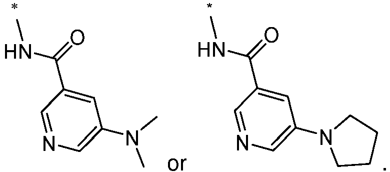

4. The compound according to any one of claims 1-3, **characterized in that** the compound shown in formula I is selected from the following structures: 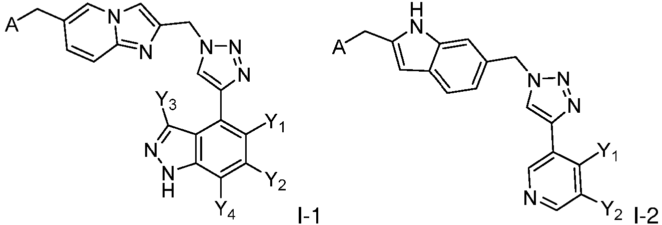 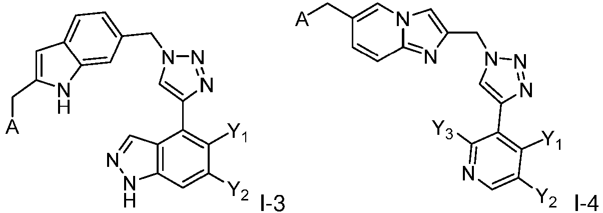 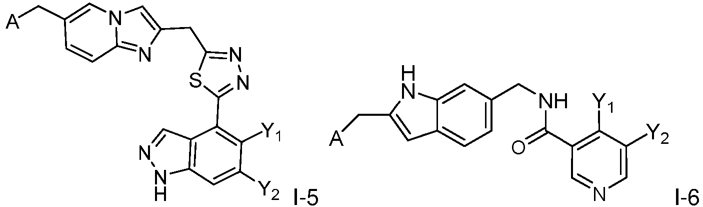 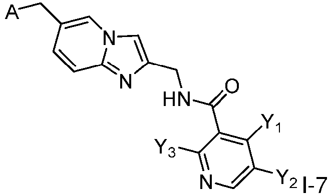
wherein A is selected from 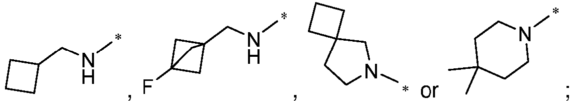 Y₁, Y₂, Y₃, and Y₄ are the same or different, independently selected from H, I, Br, Cl, F, NH₂, 3-6 membered heterocyclic groups, -Se-C₁₋₆alkyl, -S-C₁₋₆alkyl, -N(C₁₋₆alkyl)₂, -NH-C₁₋₆alkyl, -N(C₁₋₃alkyl-Cl)₂, difluoro 3-6 membered heterocyclic groups (e.g., 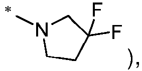 fluoro 3-6 membered heterocyclic groups (e.g., 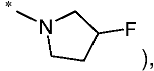 5-6 membered heteroaryl, C₁₋₆alkyl substituted 3-6 membered heterocyclic groups (e.g., 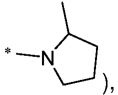 C₁₋₆alkyl or C₁₋₆alkoxy;
preferably, the compound shown in formula I' is selected from the following structures: 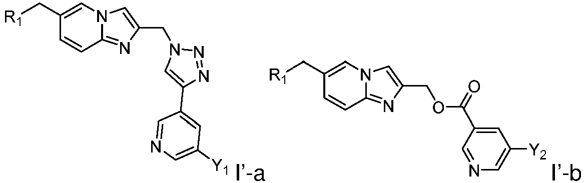 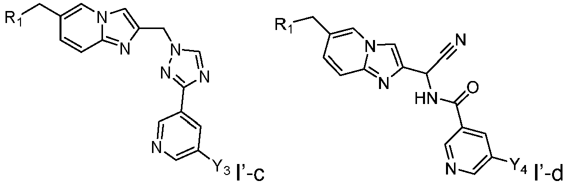 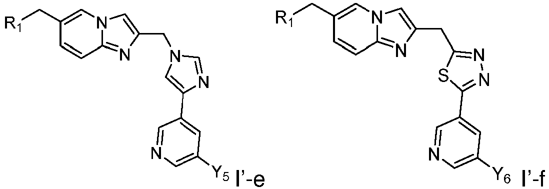 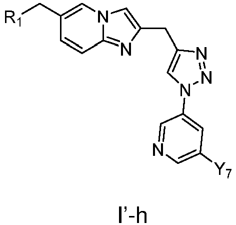
wherein A is selected from 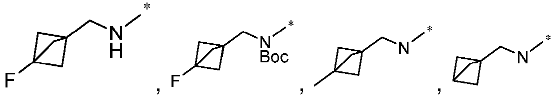
or 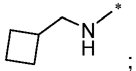
Y₁, Y₂, Y₃, Y₄, Y₅, Y₆, and Y₇ are the same or different, independently selected from 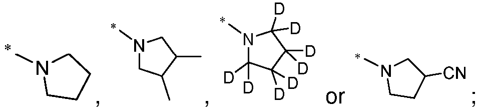 and
preferably, in formula I", R₁ is selected from 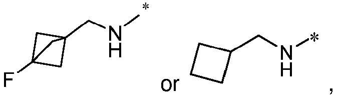 and R₂ is selected from methyl or ethyl.

5. The compound according to any one of claims 1-4, **characterized in that** the compound shown in formula I is selected from the following: 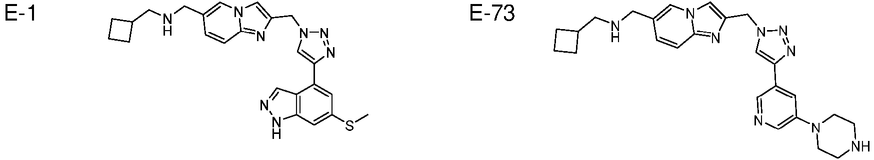 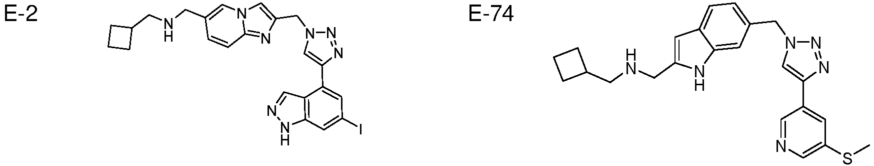 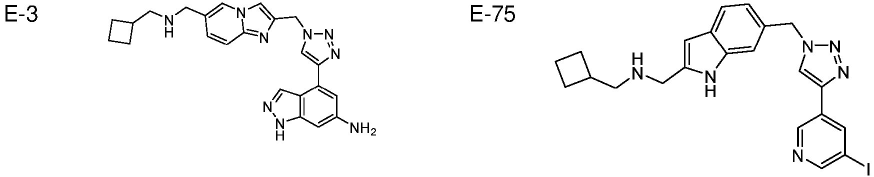 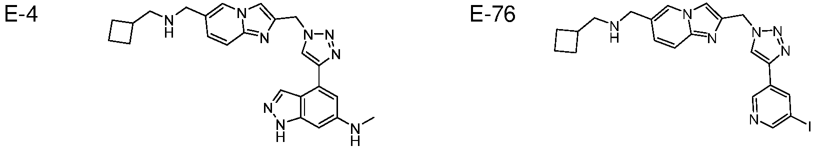 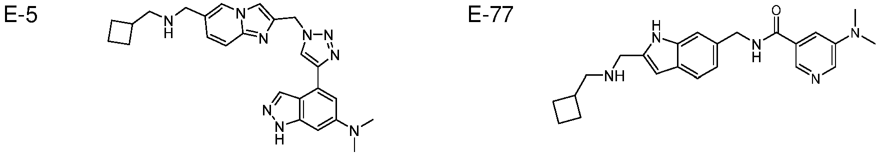 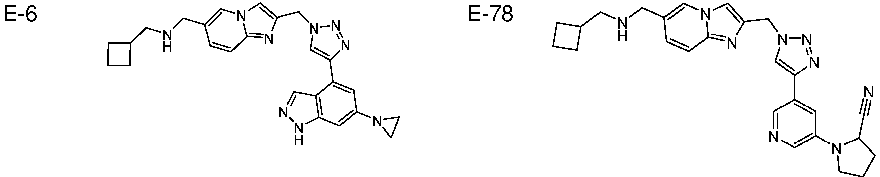 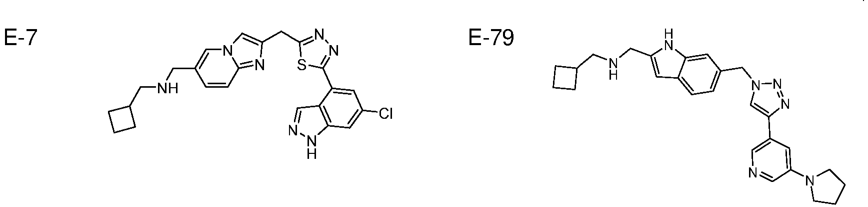 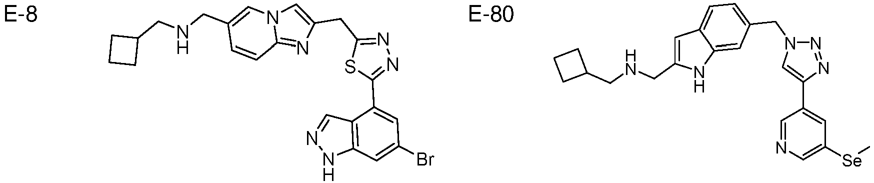 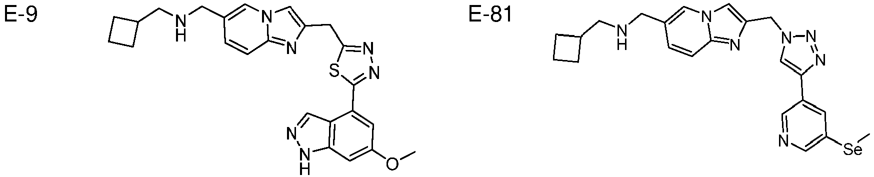 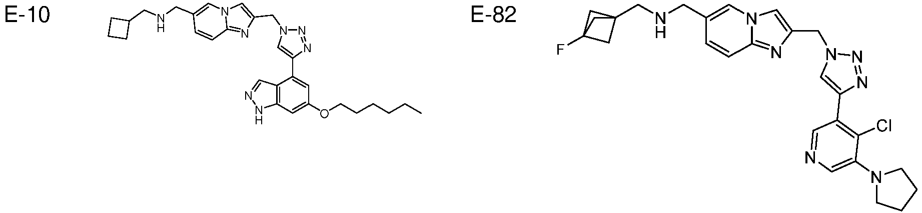 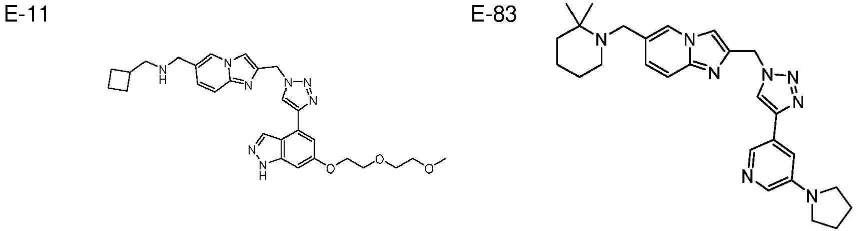 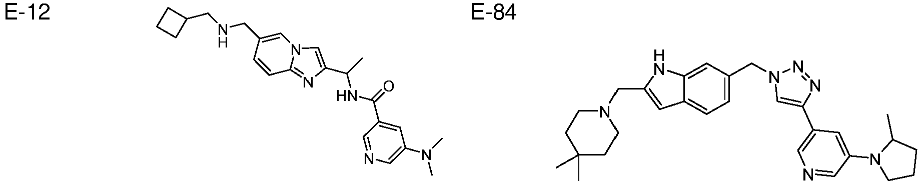 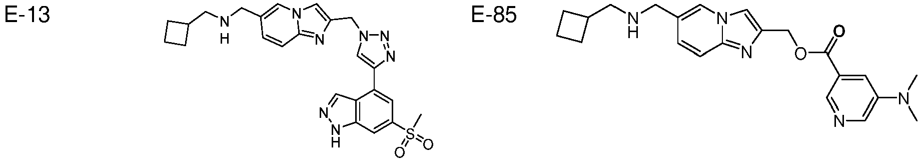 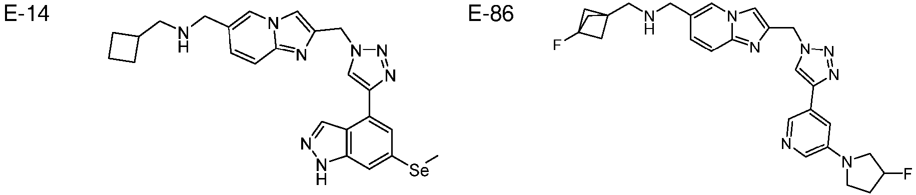 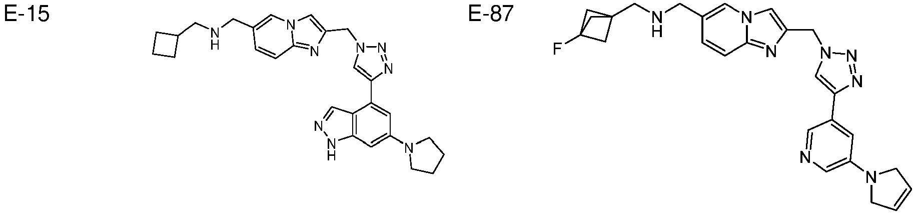 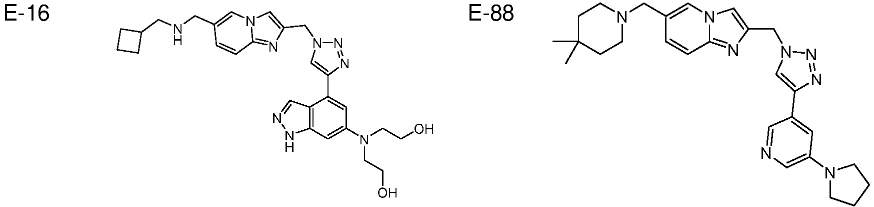 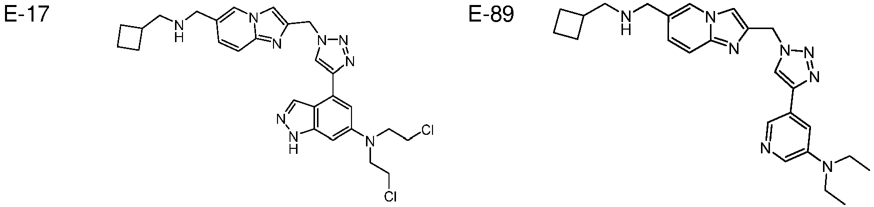 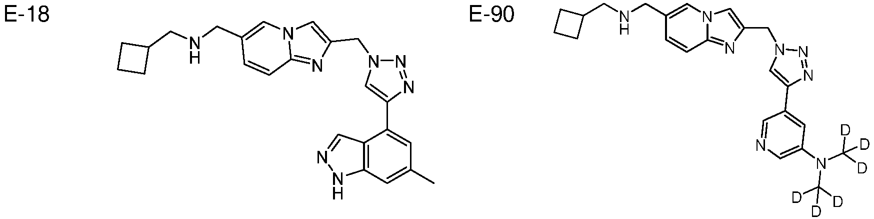 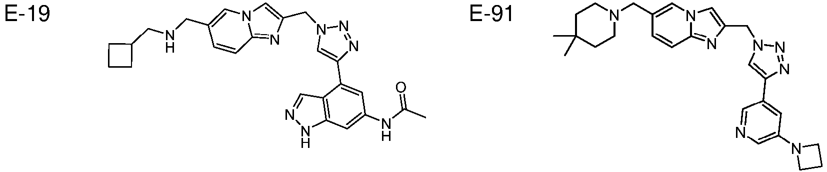 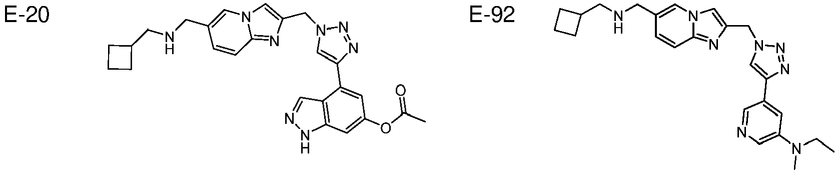 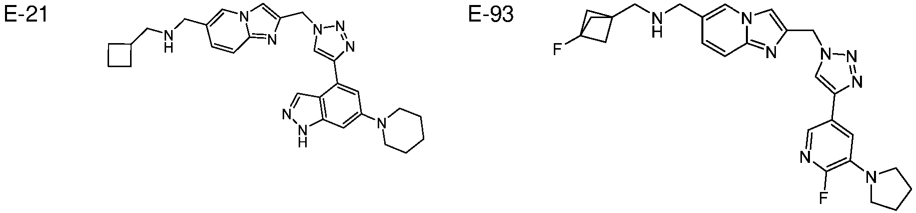 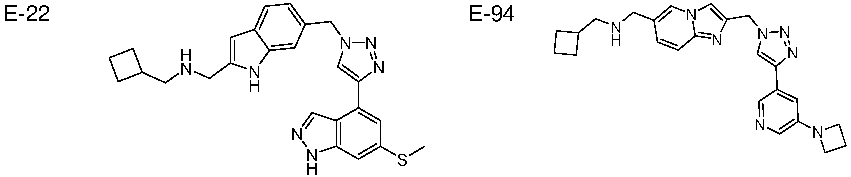 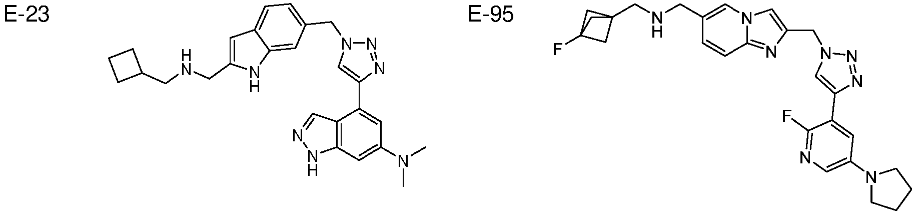 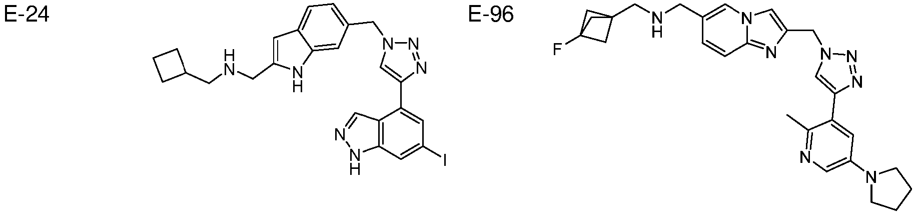 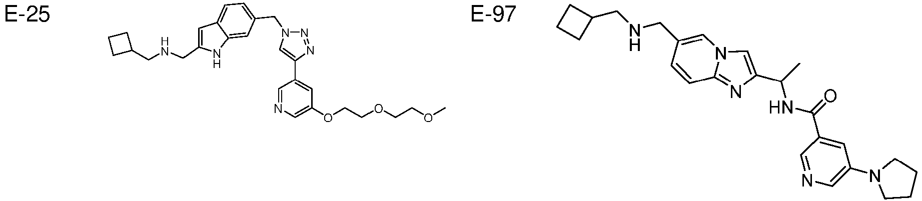 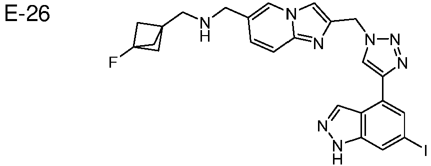 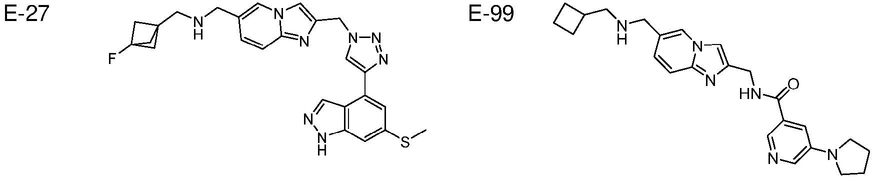 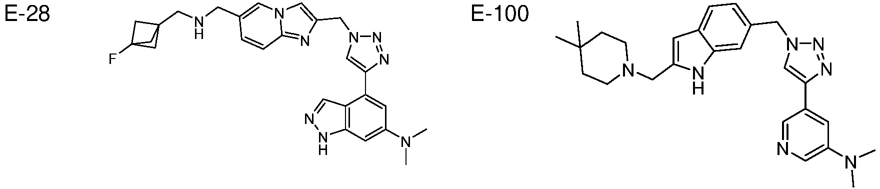 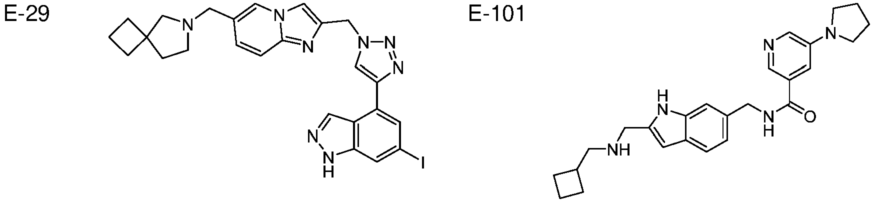 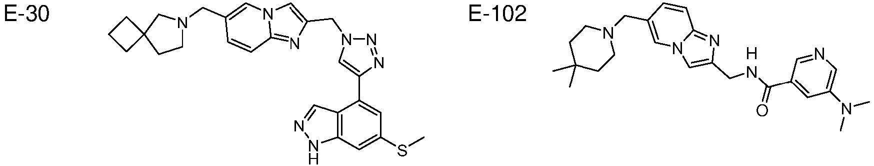 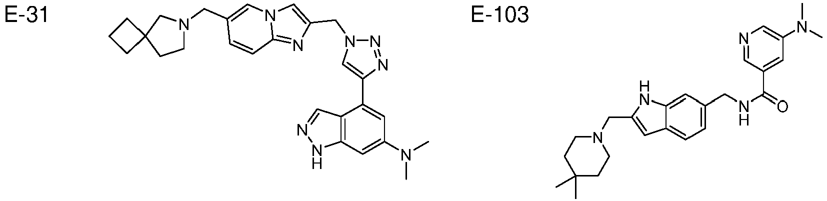 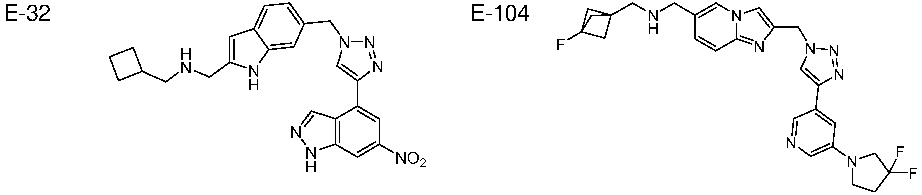 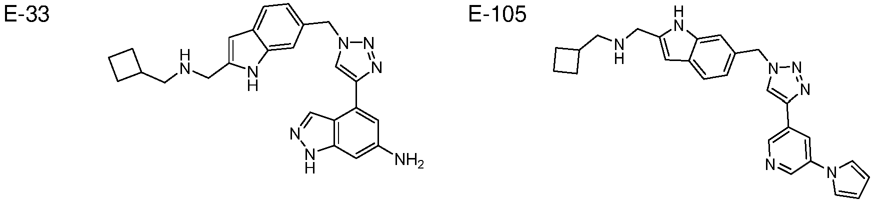 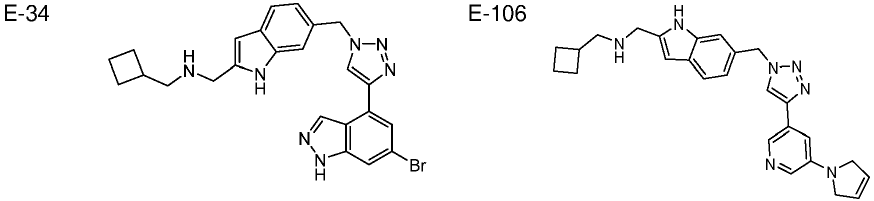 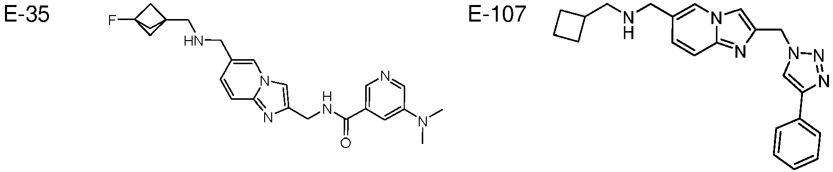 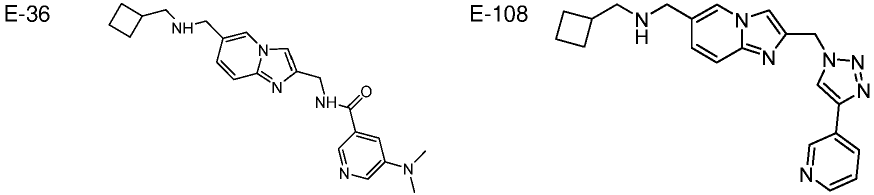 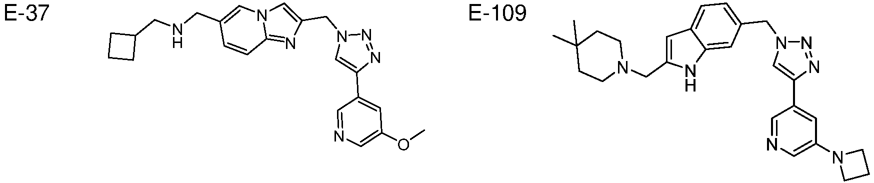 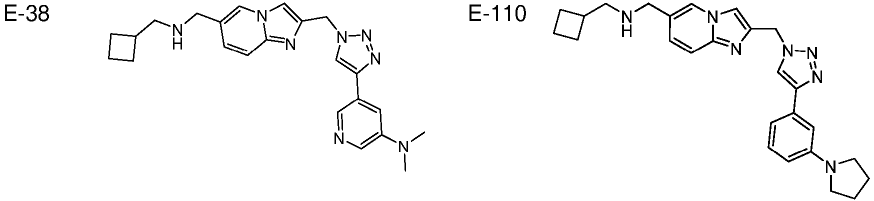 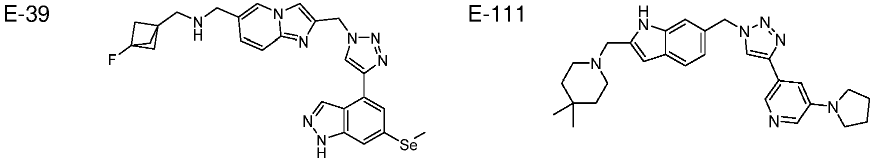 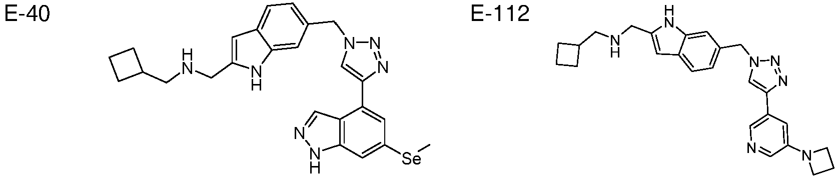 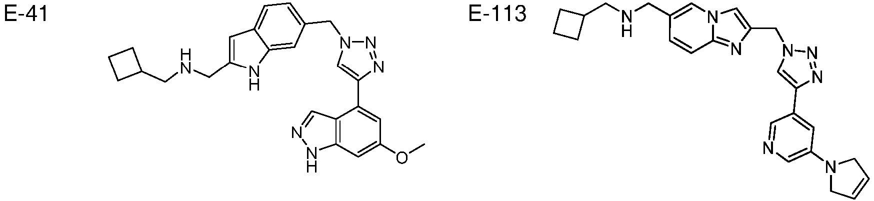 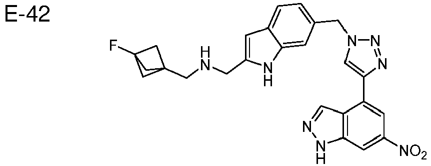 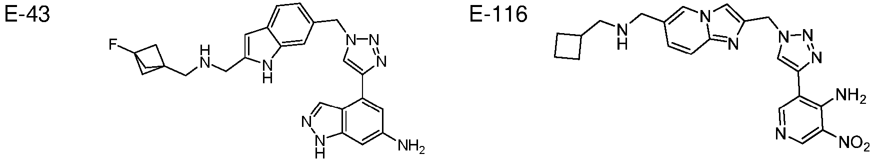 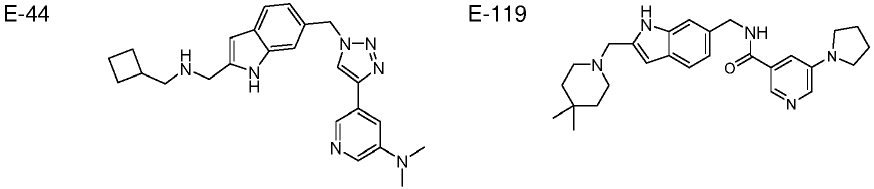 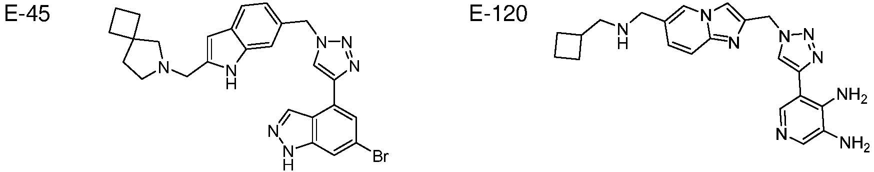 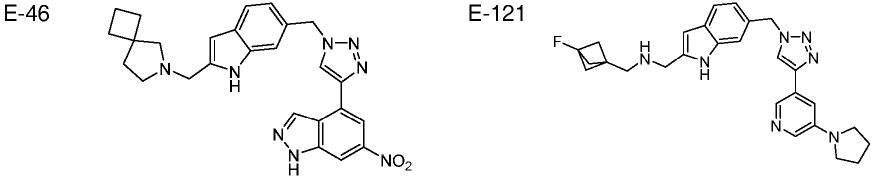 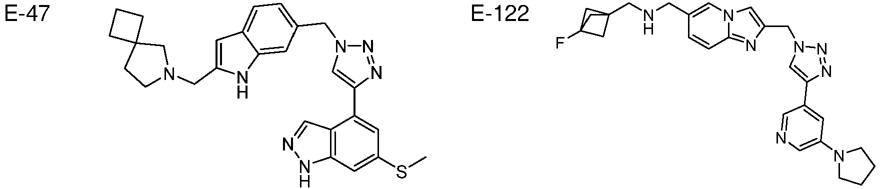 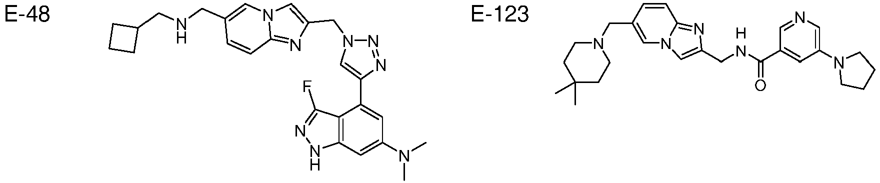 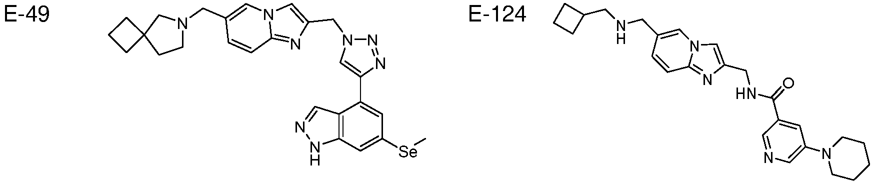 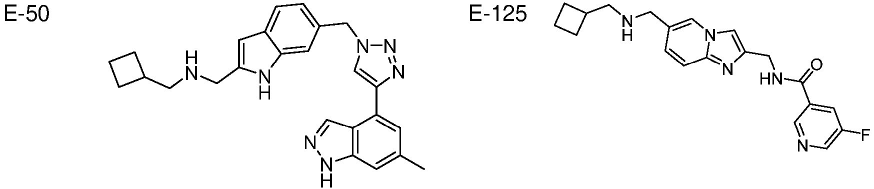 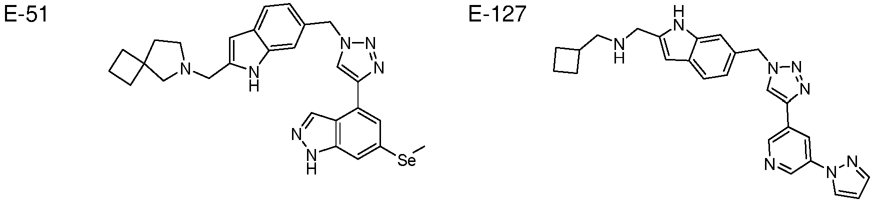 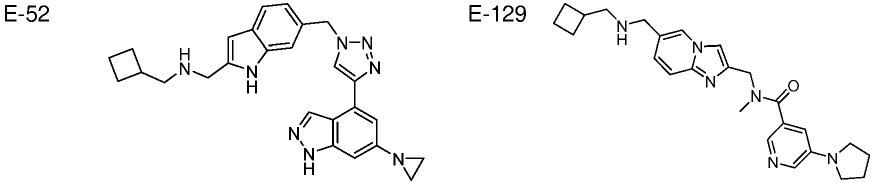 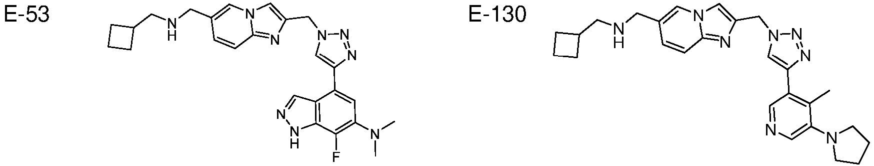 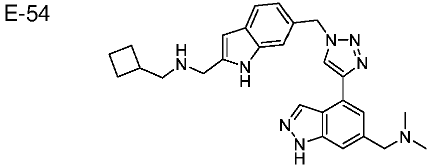 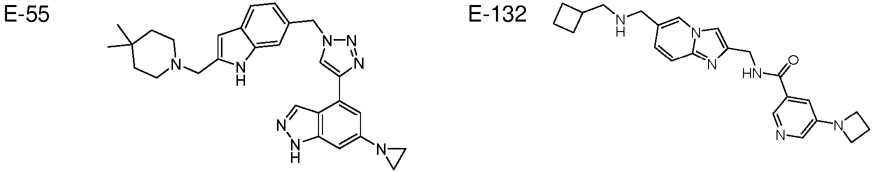 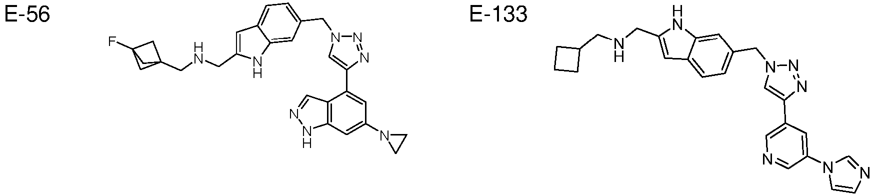 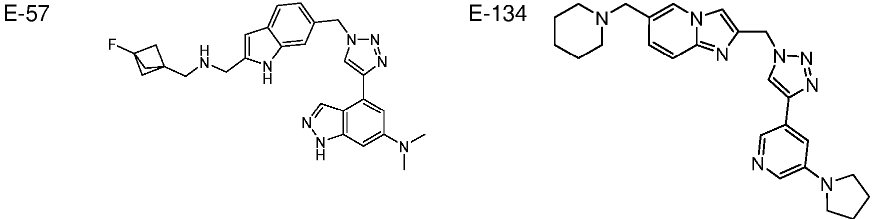 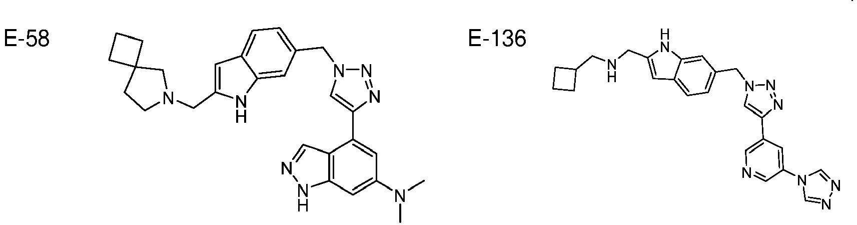 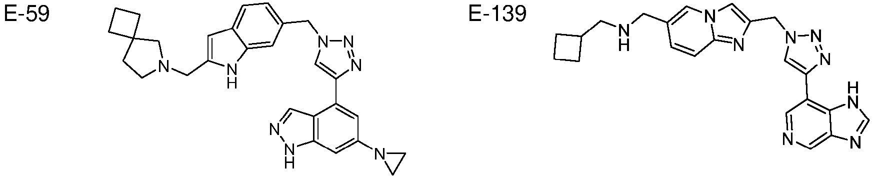 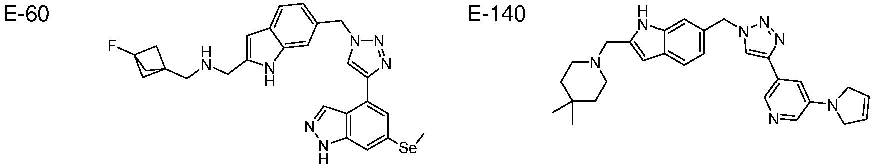 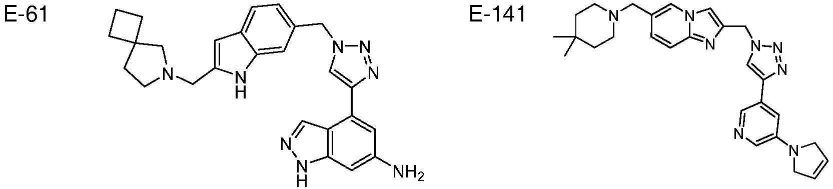 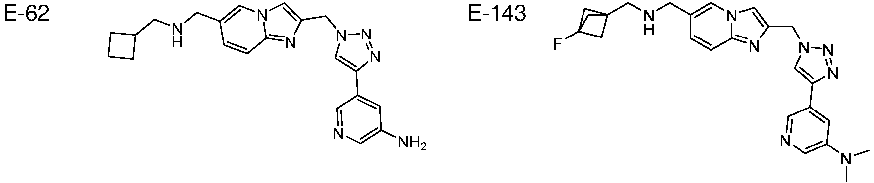 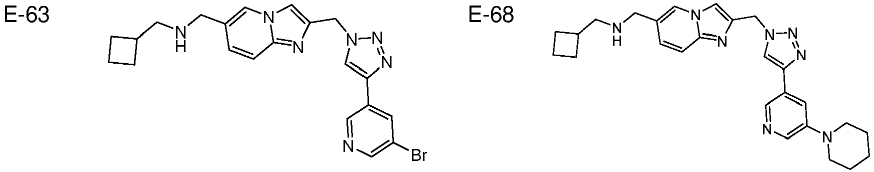 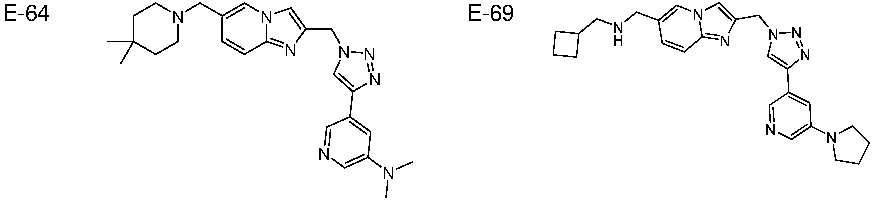 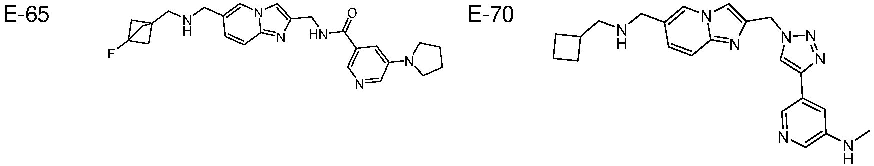 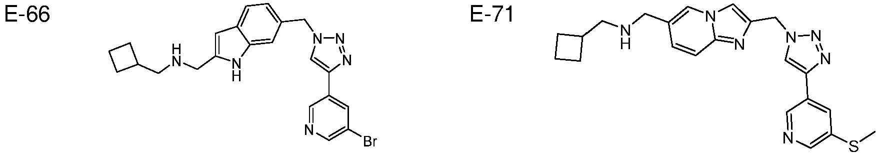 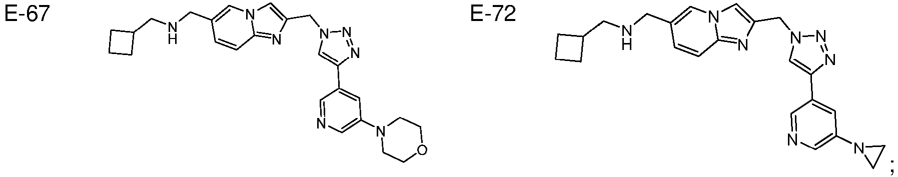
preferably, the compound shown in formula I is selected from TFA salts or HCl salts of the following compounds: 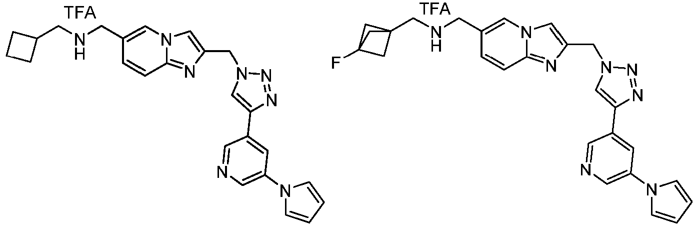 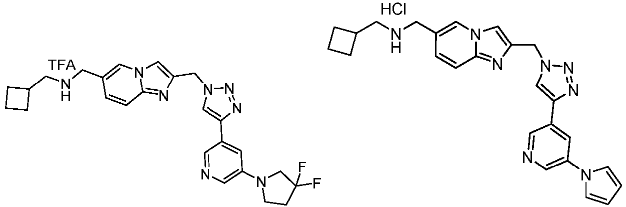 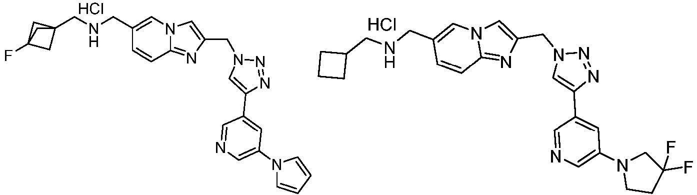
preferably, the compound shown in formula I' is selected from the following: 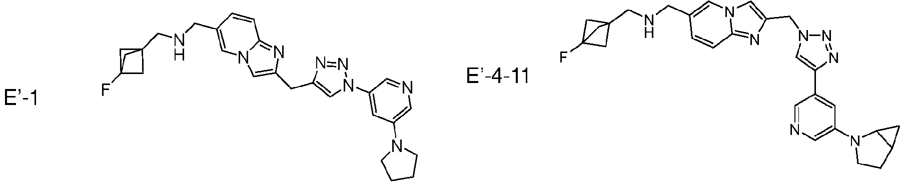 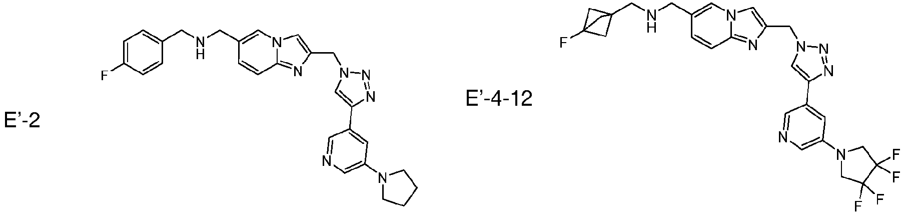 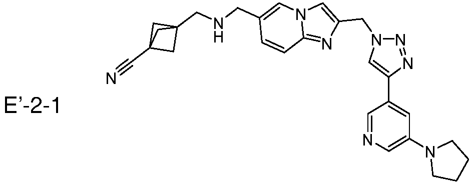 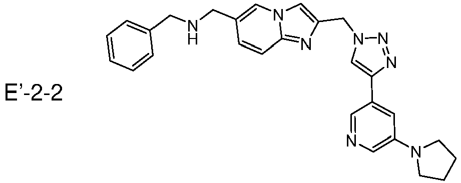 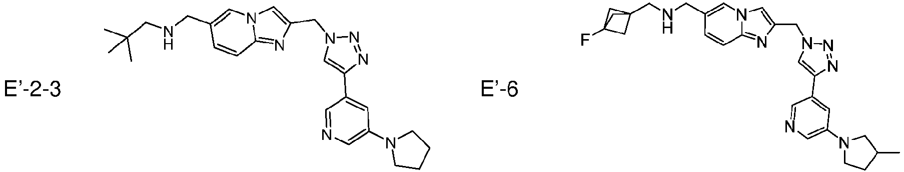 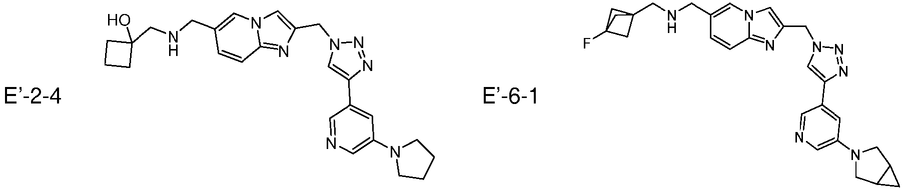 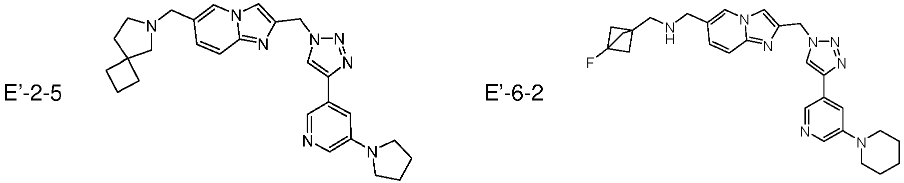 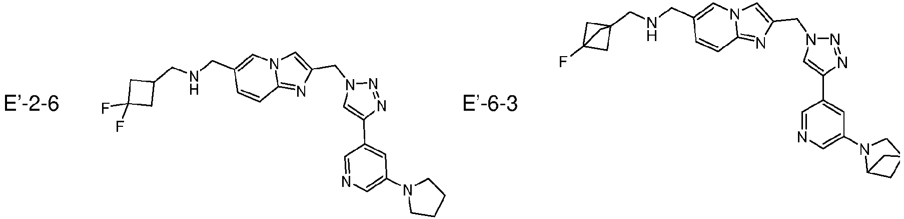 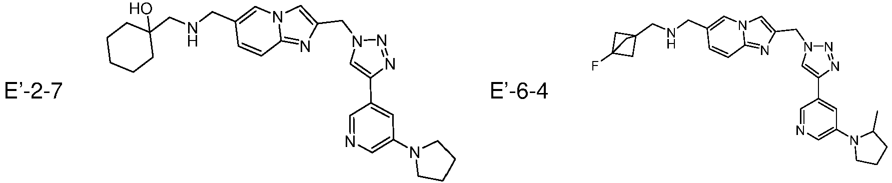 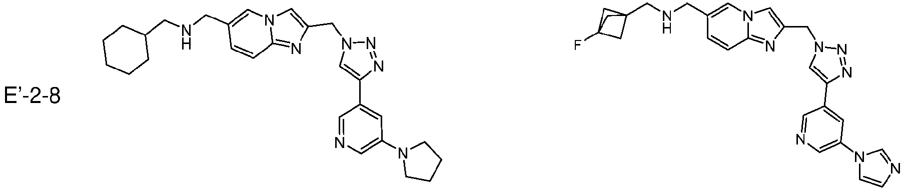 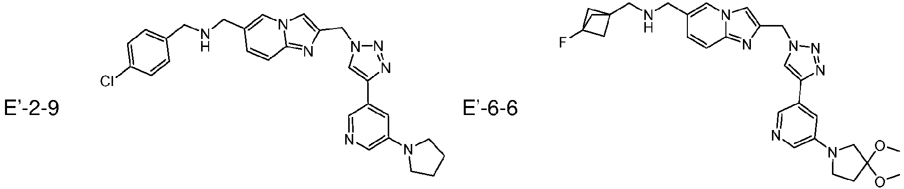 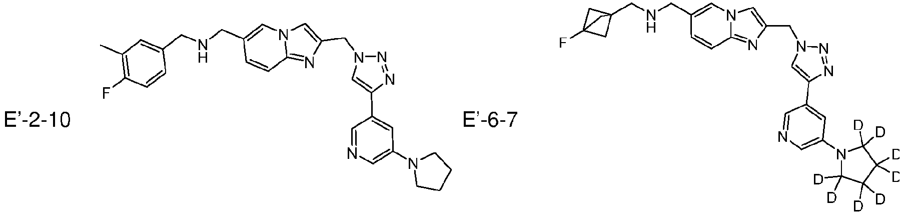 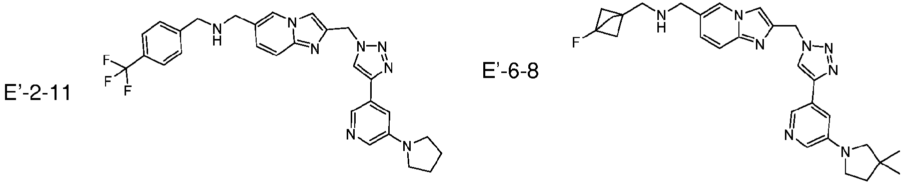 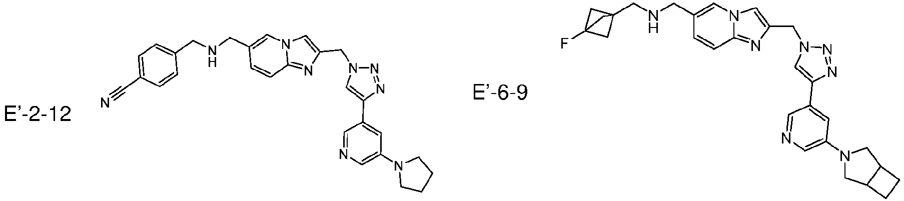 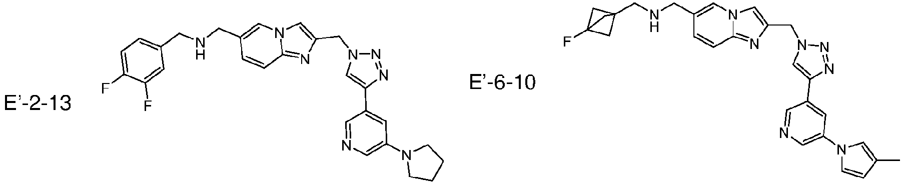 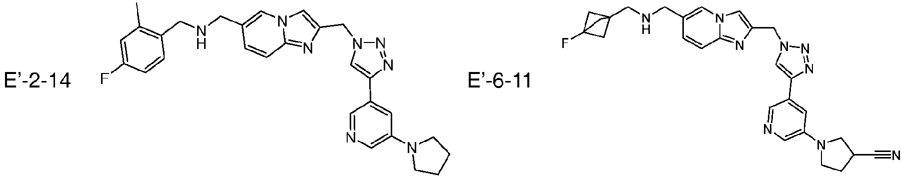 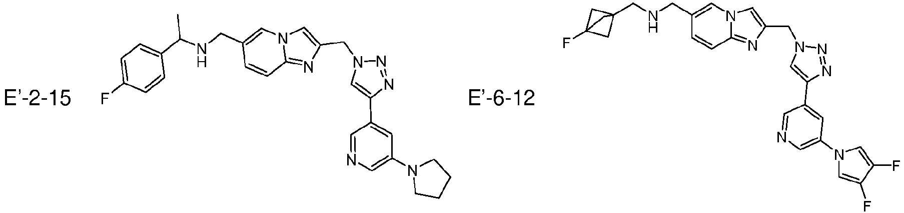 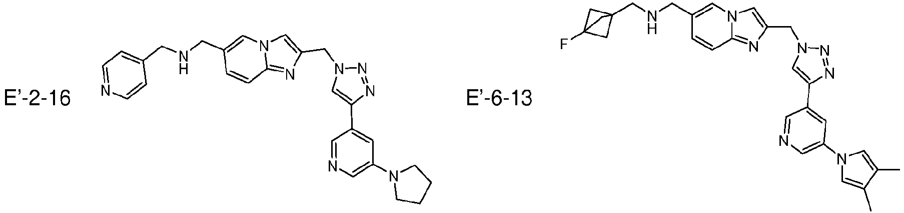 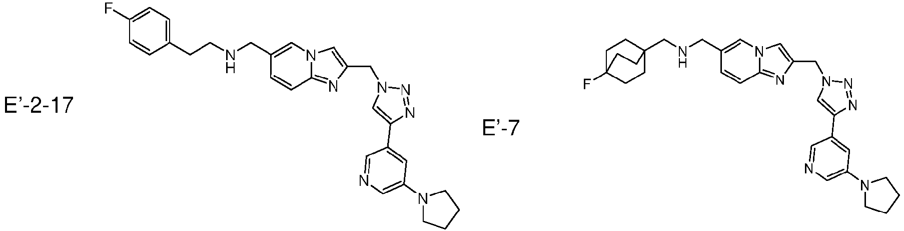 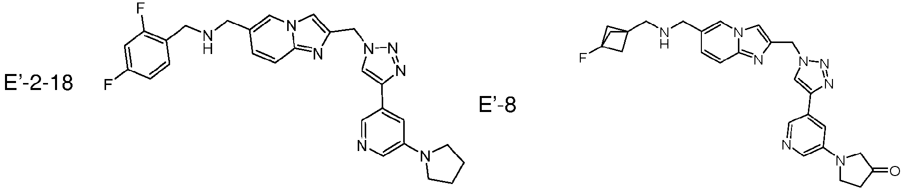 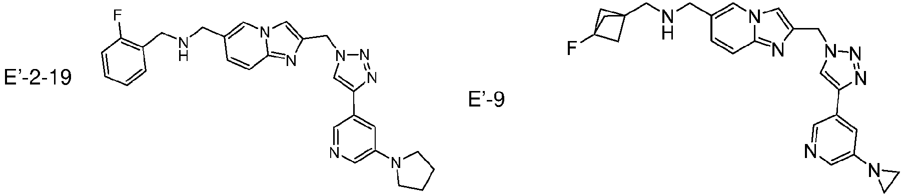 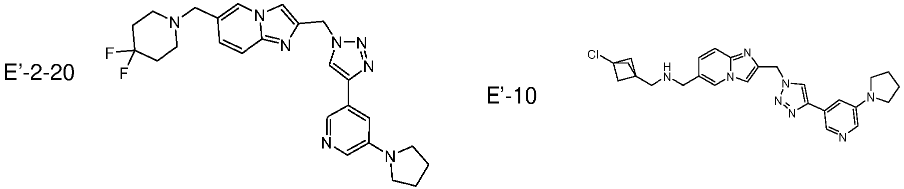 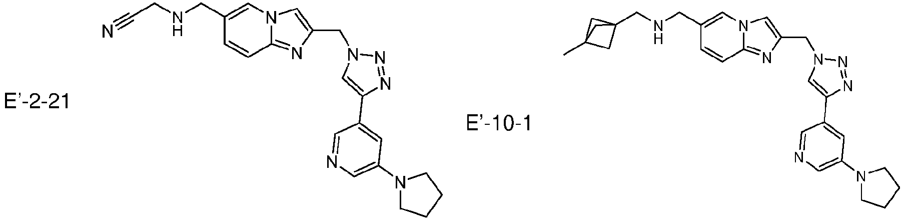 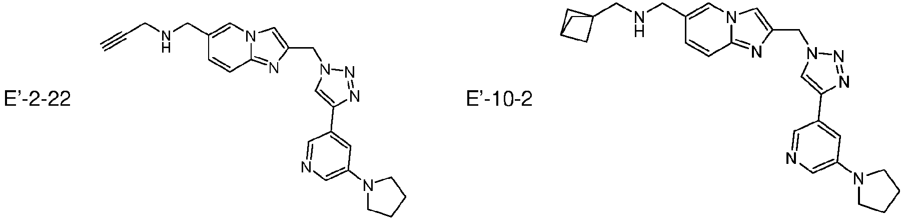 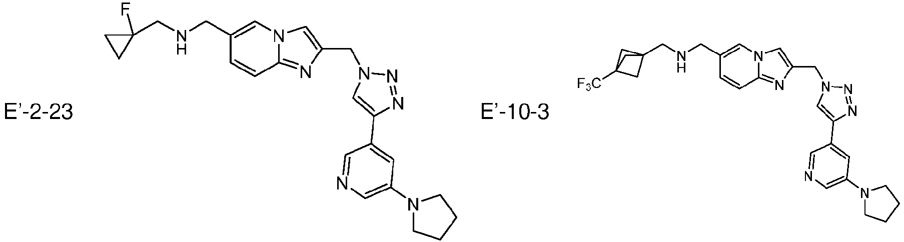 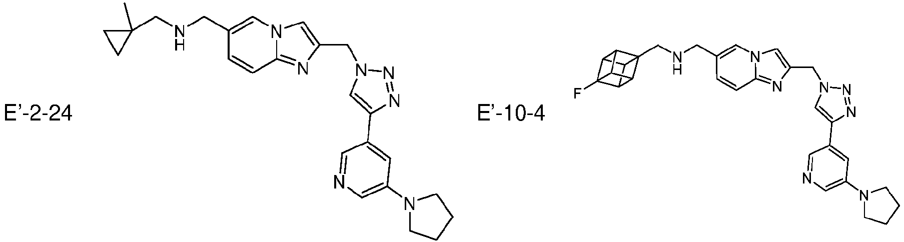 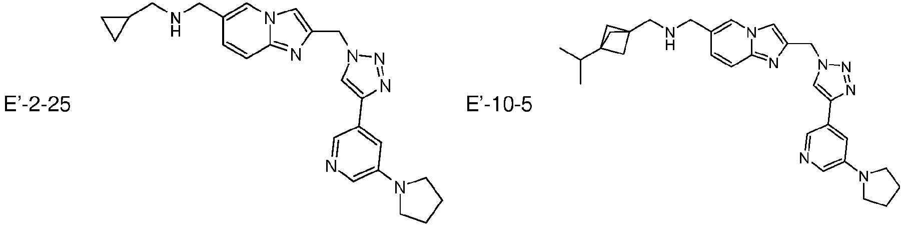 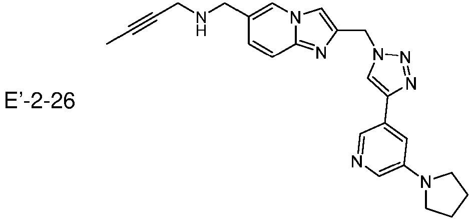 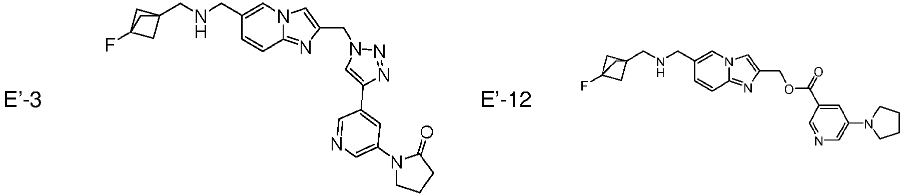 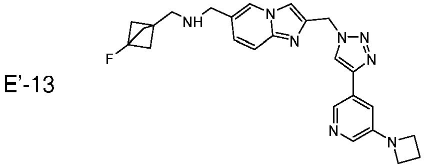 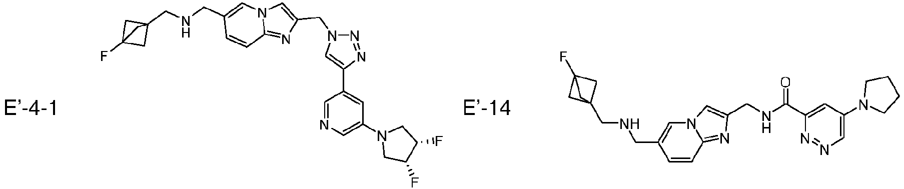 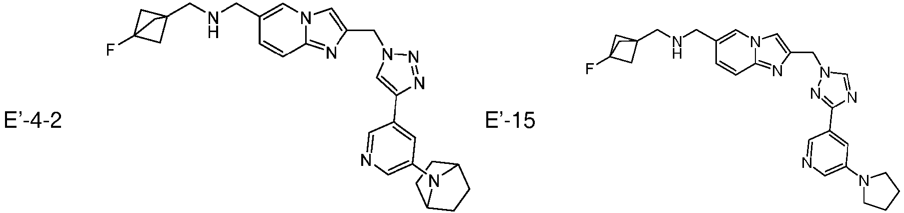 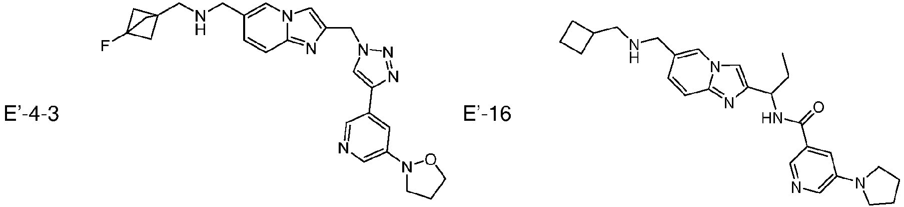 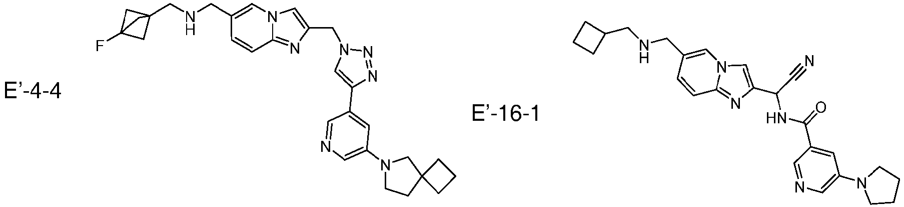 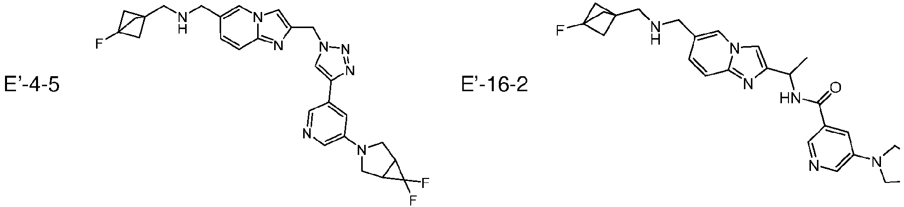 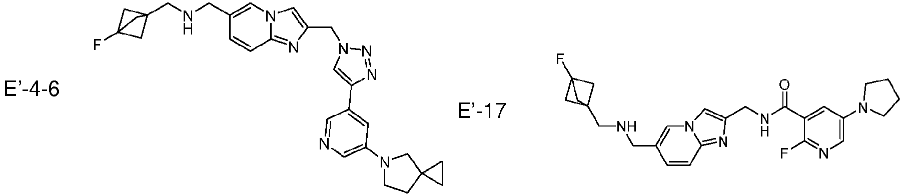 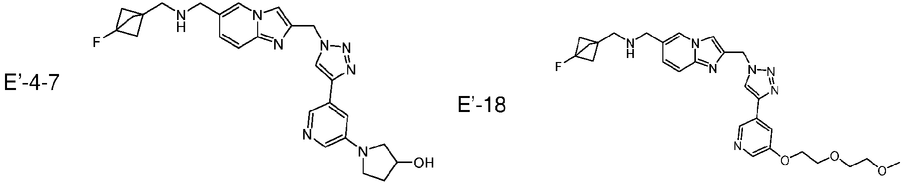 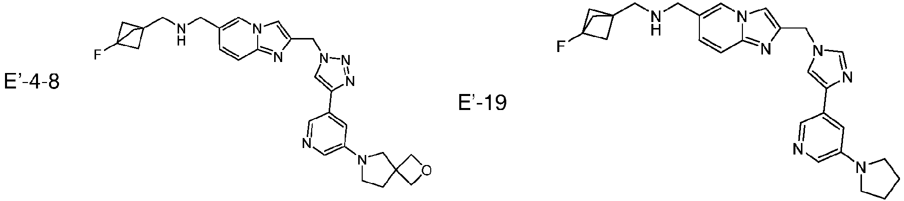 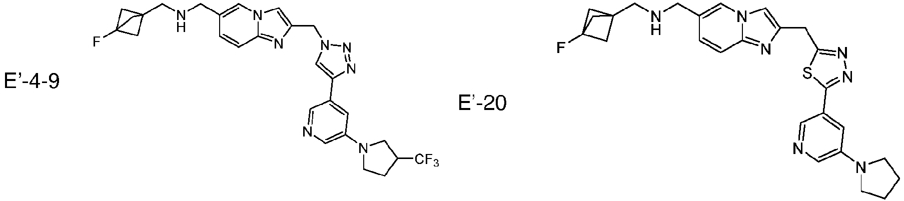 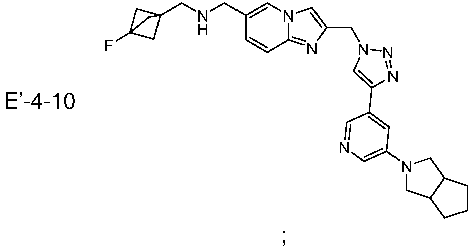
preferably, the compound shown in formula I' is selected from TFA salts or HCl salts of the following compounds: 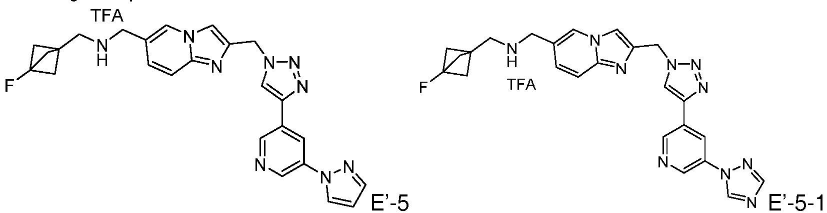 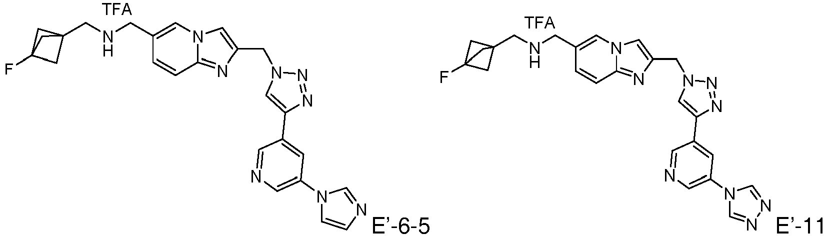 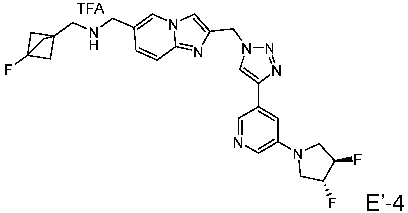 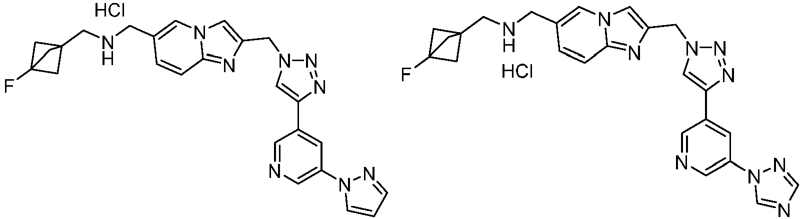 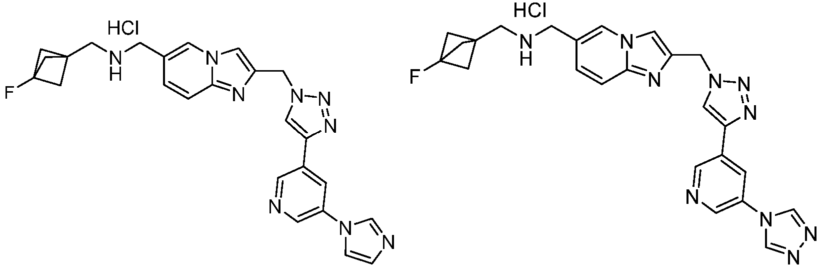 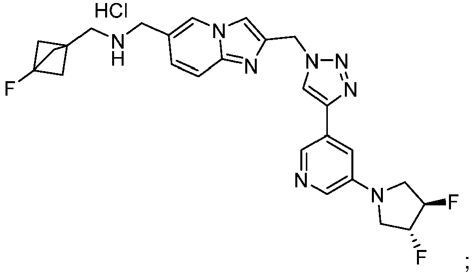
preferably, the compound shown in formula I" is selected from the following:

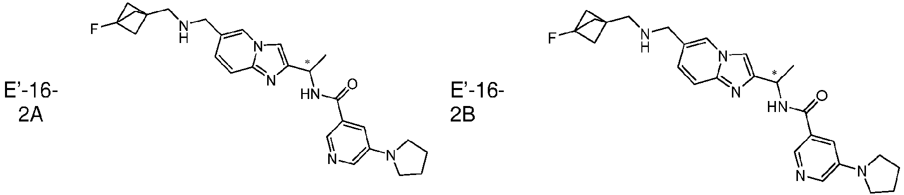

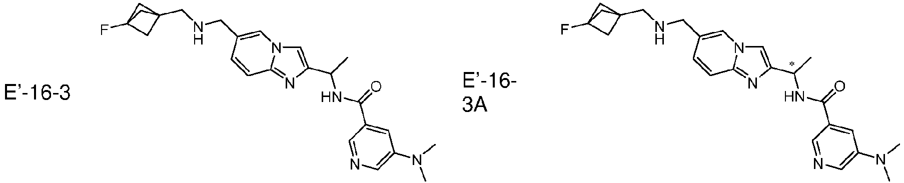

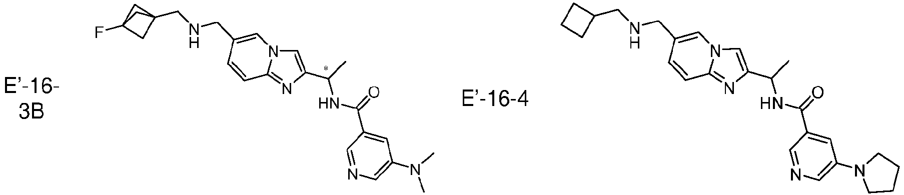

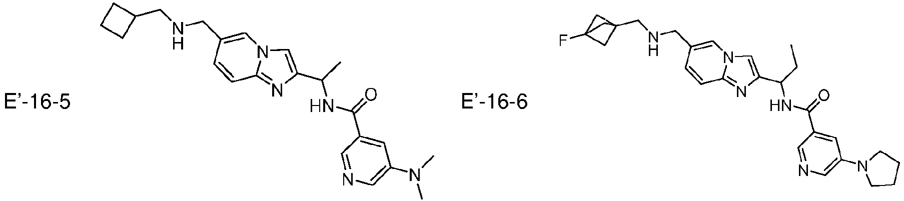

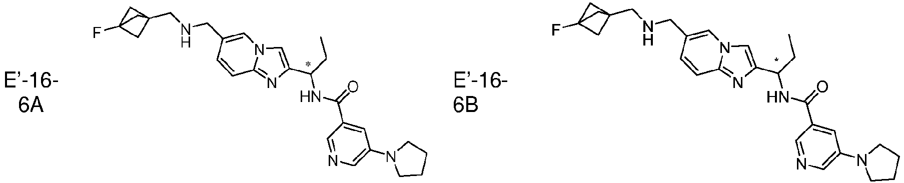

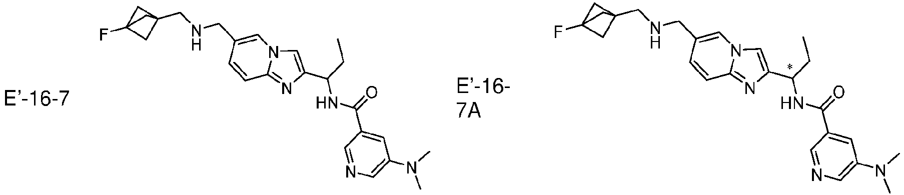

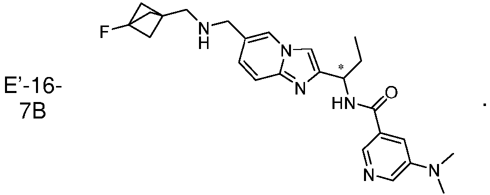

6. The compound according to any one of claims 1-5, **characterized in that** the pharmaceutically acceptable salts are HCl or TFA salts.

7. Use of at least one of the compound shown in formula I, formula I' or formula I", and the tautomers, stereoisomers, or pharmaceutically acceptable salts of the three compounds according to any one of claims 1-6 in preparation of METTL3 inhibitors.

8. The use according to claim 7, **characterized in that** diseases, symptoms or conditions prevented, alleviated and/or treated by the METTL3 inhibitors comprise solid tumors, leukemia, autoimmune diseases, neurological diseases, inflammatory diseases or infectious diseases; and
preferably, the diseases, symptoms or conditions prevented, alleviated and/or treated by the METTL3 inhibitors comprise hematological malignant tumors, AML leukemia, chronic myeloid leukemia, solid tumors or diseases caused by viruses.

9. A pharmaceutical composition, **characterized by** comprising at least one of the compound shown in formula I, formula I' or formula I", and the tautomers, stereoisomers, or pharmaceutically acceptable salts of the three compounds according to any one of claims 1-6.

10. The pharmaceutical composition according to claim 9, **characterized in that** the pharmaceutical composition is used for treating, alleviating, and/or preventing diseases, symptoms or conditions related to METTL3 dysfunction.
